(19) Europäisches Patentamt
European Patent Office
Office européen des brevets

(11) EP 4 205 769 A1

(12) EUROPEAN PATENT APPLICATION

(43) Date of publication:
**05.07.2023 Bulletin 2023/27**

(21) Application number: **22182384.2**

(22) Date of filing: **27.09.2019**

(51) International Patent Classification (IPC):
***A61K 51/10*** *(2006.01)* ***C07K 16/28*** *(2006.01)*
***G01N 33/566*** *(2006.01)* ***G01N 33/68*** *(2006.01)*

(52) Cooperative Patent Classification (CPC):
**A61K 51/1069; A61K 51/1093; A61K 51/1096; G01N 33/566; G01N 33/6872**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.09.2018 US 201862738938 P**
**29.03.2019 US 201962826606 P**

(62) Document number(s) of the earlier application(s) in accordance with Art. 76 EPC:
**19866804.8 / 3 856 786**

(71) Applicant: **Imaginab, Inc.**
**Inglewood, CA 90301 (US)**

(72) Inventors:
- **WILSON, Ian, Andrew**
  **California, 90301 (US)**
- **BEHERA, Deepak**
  **California, 90301 (US)**
- **LE, William, Huy**
  **California, 90301‘ (US)**

(74) Representative: **Lawrie IP Limited**
**310 St. Vincent Street**
**Glasgow G2 5RG (GB)**

Remarks:
•The complete document including Reference Table(s) and the Sequence Listing(s) can be downloaded from the EPO website
•This application was filed on 30-06-2022 as a divisional application to the application mentioned under INID code 62.
•Claims filed after the date of filing of the application (Rule 68(4) EPC).

# (54) CD8 IMAGING CONSTRUCTS AND METHODS OF USE THEREOF

(57) Antigen binding constructs that bind to CD8, as well as formulations and methods of using them, are described herein.

## FIGURE 1A

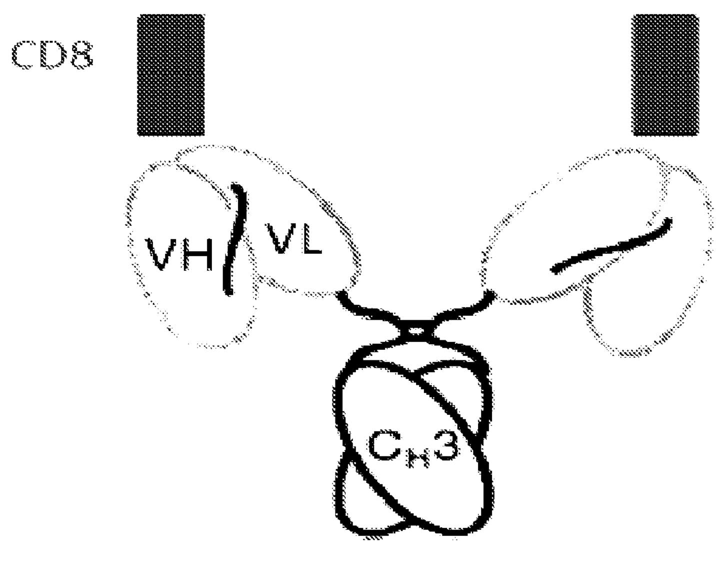




Processed by Luminess, 75001 PARIS (FR)

## Description

### PRIORITY

The present application is a PCT and claims priority to U.S. App. No. 62/738,938, filed September 28, 2018 and U.S. App. No. 62/826,606, filed March 29, 2019, the entireties of both of which are hereby incorporated by reference.

### SEQUENCE LISTING

The present application is being filed along with a Sequence Listing in electronic format. The Sequence Listing is provided as a file entitled IGNAB047WOSEQLIST.txt, created September 27, 2019, which is 126,124 bytes in size. The information in the electronic format of the Sequence Listing is incorporated herein by reference in its entirety.

### BACKGROUND

#### Field

The technology generally relates to imaging and treatment aspects related to CD8.

#### Description of the Related Art

CD8 (cluster of differentiation) is a transmembrane glycoprotein which is a specific marker for a subclass of T-cells (which includes cytotoxic T-cells). CD8 assembles as either a heterodimer of the CD8 alpha and CD8 beta subunits or a CD8 alpha homodimer. The assembled dimeric CD8 complex acts as a co-receptor together with the T-cell receptor (TCR) to recognize antigen presentation by MHC class I cells. CD8 plays a role in the development of T-cells and activation of mature T-cells. Changes in T-cell localization can reflect the progression of an immune response and can occur over time.

A variety of CD8 directed antigen binding constructs exist, including those, discussed in U.S. Pat. Pub. No. 20160024209.

### SUMMARY

In some embodiments, a method of monitoring CD8 in vivo is provided. The method comprises providing a CD8 minibody to a subject, wherein the CD8 minibody binds to a CD8 as shown in FIG. 1C, and wherein the minibody is labeled with a detectable marker; and monitoring a distribution of the CD8 minibody in the subject within 6-36 hours of administering the CD8 minibody to the subject.

In some embodiments, a method of monitoring CD8 in vivo comprises providing a CD8 minibody to a subject, wherein the CD8 minibody binds to aCD8 as shown in FIG. 1C, and wherein the minibody is labeled with a detectable marker; and monitoring a distribution of the CD8 minibody in the subject, wherein the monitoring can detect a tissue infiltrated with 500 or less CD8 bearing cells per $mm^2$ within the subject, and wherein monitoring is achieved via PET.

In some embodiments, a method of visualizing CD8 positive cells comprises providing a CD8 minibody to a subject, wherein the CD8 minibody is labeled with a detectable marker, wherein the CD8 minibody is humanized in sequence and binds to human CD8; and monitoring a distribution of the CD8 minibody in the subject within 6-36 hours of administering the CD8 minibody to the subject, wherein monitoring can detect a tissue infiltrated with 500 or less CD8 positive cells per $mm^2$ within the subject, and wherein monitoring is achieved via PET.

In some embodiments, the cell count (e.g., 500 or less) is per 10 micron or 30 micron depth.

In some embodiments, a method of visualizing cells in a human comprises providing a means of binding human CD8 positive cells to a subject, wherein the means comprises a detectable label; and monitoring and determining a first distribution of the means of binding human CD8 positive cells in the subject within 6-36 hours of administering the means of binding human CD8 positive cells to the subject.

In some embodiments, a method of administering a minibody to a subject comprises providing to a subject a labeled minibody that binds to human CD8, wherein 3 mCi +20% of radiation is provided to the subject via the labeled minibody and wherein an amount between 0.2 and 10mg of total protein is provided to the subject.

In some embodiments, a method of administering a minibody to a subject comprises providing to a subject a labeled minibody that binds to human CD8, wherein 0.75-1.5 +/- 20% mCi of radiation is provided to the subject via the labeled minibody and wherein an amount between 0.2 and 10mg of total protein is provided to the subject.

In some embodiments, a method of treating a patient comprises administering to a human patient diagnosed with a cancer a dose of an antigen-binding construct that binds to human CD8, wherein the dose comprises: a $^{89}Zr$-

labeled antigen-binding construct providing a radiation activity of about 3 mCi; and about 10 mg or less of the antigen-binding construct. The method further comprises detecting the $^{89}$Zr-labeled antigen-binding construct in the patient at a first time point after administering the dose, to generate a first patient image corresponding to the first time point, wherein the first time point is about 6 hours to 36 hours after administering; determining a first abundance and/or distribution of CD8 cells in one or more tissues and/or neoplasia in the patient based on the first patient image; and administering to the patient a first treatment for the cancer based on the first abundance and/or distribution of CD8 cells in the one or more tissues.

In some embodiments, a method of treating a tumor comprises administering a minibody to a subject such that the minibody binds to a tumor within the subject to form a labeled tumor, wherein the minibody binds to human CD8, and wherein the minibody is linked to a detectable label. If the minibody binds in a biased manner to a surface of any tumor in forming the labeled tumor, then administering a treatment selected from the group consisting of an immune checkpoint inhibitor. If the minibody binds throughout all tumors in forming the labeled tumor, then not administering an immune checkpoint inhibitor.

In some embodiments, a method of treating a subject, comprises administering to a patient that has a neoplasia a human minibody that binds to human CD8; monitoring a distribution of the human minibody to determine a first tumor-infiltrating lymphocyte ("TIL") status within the neoplasia; and treating the patient based upon at least the first TIL status of the neoplasia.

In some embodiments, a method of analyzing CD8 distribution in a subject comprises providing an image of a distribution of a detectable marker via a first PET image, wherein the detectable marker is linked to a CD8 minibody, wherein the CD8 minibody binds to human CD8, and wherein the CD8 minibody does not provoke an immune response in the subject; providing an image of a distribution of a FDG marker via a second PET image of the subject; creating a third PET image that comprises an overlay of the first PET image onto the second PET image; and identifying a tumor as TIL, based upon the third PET image.

In some embodiments, a method of treating a subject comprises administering to a patient that has a neoplasia an antigen-binding construct that binds to human CD8; monitoring a distribution of the antigen-binding construct to determine the tumor-infiltrating lymphocyte ("TIL") status within the neoplasia; and administering an immunotherapy ("IOT") to the patient if the TIL status of at least one neoplasia is negative. Optionally continuing this until the status becomes TIL positive.

In some embodiments, a method of treating a subject, comprises administering to a patient that has a neoplasia an antigen-binding construct that binds to human CD8; monitoring a distribution of the antigen-binding construct to determine the tumor-infiltrating lymphocyte ("TIL") status within the neoplasia; and administering an alternative immunotherapy ("IOT") to the patient if the TIL status of the neoplasia is not positive. Optionally continuing this until the status becomes TIL positive.

In some embodiments, a method of characterizing a tumor comprises applying an antigen-binding construct that binds to human CD8 to a human subject, wherein the antigen-binding construct comprises a detectable marker; monitoring a distribution of the detectable marker; generating an image based on the distribution of the detectable marker; and displaying the image to a user in a manner to allow the user to characterize the tumor.

In some embodiments, a method of generating an image comprises applying an antigen-binding construct that binds to CD8, wherein the antigen-binding construct comprises a detectable marker; detecting at least one detectable marker within a location within the tumor and assigning a positive pixel for each detectable marker detected; and generating an image based on a distribution of each positive pixel assigned, wherein the image is stored in a non-transitory computer readable media.

In some embodiments, a method of positron emission tomography ("PET") comprises administering a tracer that binds to CD8 to a subject; providing a scintillator; using the scintillator to detect a pair of photons created by the tracer; and using detection of the pair of photons to localize a source of the tracer via a list of coincidence events that are processed via a processor that is configured to take an output from the scintillator and convert it to the list of coincidence events, wherein between about 300 and about 500 CD8 positive cells can be detected per $mm^3$ of a tissue or neoplasia within the subject.

In some embodiments, a method of manufacturing a diagnostic composition comprises conjugating a minibody to desferrioxamine to form a Df-minibody; radiolabeling the Df-minibody with $^{89}$Zr to form radiolabeled minibody; purifying the radiolabeled minibody; and mixing the radiolabeled minibody with a cold minibody to form a diagnostic composition, wherein the minibody and the cold minibody bind to a same epitope on CD8.

In some embodiments, a diagnostic composition comprises 3.0±20% mCi of a 89Zr-Df-labeled minibody; 20 mM Histidine; 5% sucrose; 51-62 mM Sodium Chloride; 141-194 Arginine; and 2-20 mM Glutamic acid.

In some embodiments, a diagnostic composition comprises a labeled minibody comprising the structure of:

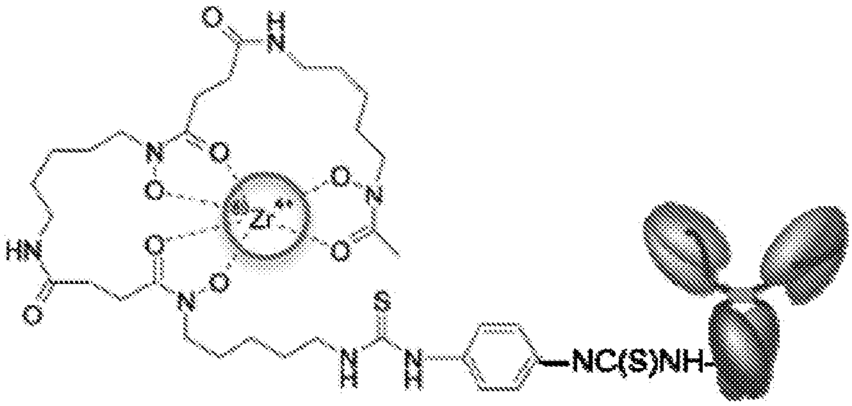

wherein the minbody comprises a heavy chain variable region within SEQ ID NO: 1, 3, 16, or 147 and a light chain variable region within SEQ ID NO: 7, 9, 15 or 147, and wherein the composition provides at least 3 mCi of radiation.

In some embodiments, a diagnostic composition comprises a labeled minibody comprising the structure of:

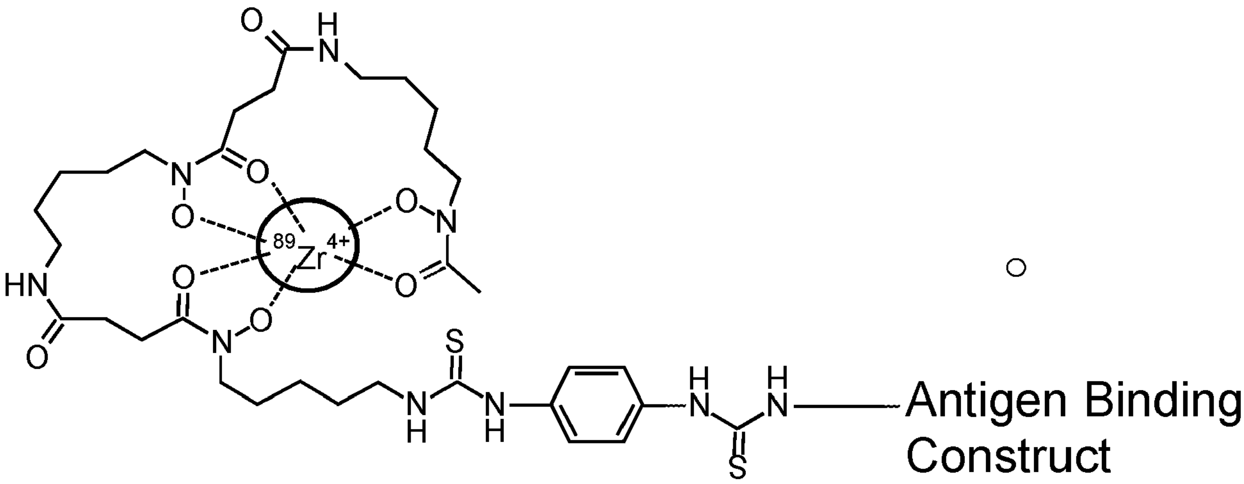

,

wherein the minbody comprises a heavy chain variable region within SEQ ID NO: 1, 3, 16, or 147 and a light chain variable region within SEQ ID NO: 7, 9, 15, or 147, and wherein the composition provides at least 3 mCi of radiation.

In some embodiments, a composition comprises a first minibody that binds to CD8 that comprises an active marker; and a second minibody that binds to CD8, wherein the second minbody comprises an inactive marker or lacks an active marker.

In some embodiments, a diagnostic composition comprises a first CD8 minibody that is conjugated to a radiolabel; and a second CD8 minibody that is not conjugated to the radiolabel, wherein the first and second CD8 minibodies have a same sequence, wherein the composition provides about 3 mCi of radiation, and wherein the composition provides 1.5-10 mg of total mass protein.

In some embodiments, a method of imaging a subject comprises administering a first dose of any one of the compositions provided above; imaging the subject to obtain a first image; administering a second dose of any one of the compositions provided above; and imaging the subject to obtain a second image; and comparing the first image to the second image.

In some embodiments, a method of determining a standard uptake value comprises applying an antigen binding construct to a subject, wherein the antigen binding construct comprises a radioactive probe; determining r, where r is the radioactivity concentration (kBq/ml) measured by a PET scanner within a ROI of radiation from the antigen binding construct; determining a', wherein a' is the decay-corrected amount of the injected radiolableeld tracer (kBq); determining w, the weight of the patient; and determining SUV as being the result of r(a'/W).

In some embodiments, a method of analyzing an image comprises providing an image; defining on the image a first region of interest (ROI) by marking the image; determining a signal intensity for a data point within the first ROI;

determining a maximum signal intensity within the first ROI; determining a mean value of the signal intensities within the first ROI; summing together each signal intensity within the first ROI to obtain a first summed signal level for the first ROI, wherein the first ROI represents data for an amount of a detectable marker associated with an antigen binding construct that has been administered to a subject.

In some embodiments, a method of administering an antigen binding construct to a subject is provided. The method comprises providing to a subject a labeled antigen binding construct that binds to human CD8. The label comprises $^{89}$Zr, the label provides 0.75 (or 0.5) - 3 mCi ±20% of radiation at injection, and an amount between 0.2 and 10mg of total antigen binding construct is provided to the subject.

In some embodiments, a method of treating a patient is provided. The method comprises: a) administering to a human patient diagnosed with a cancer a dose of an antigen-binding construct that binds to human CD8, wherein the dose comprises: a $^{89}$Zr-labeled antigen-binding construct providing a radiation activity of about 0.75 (or 0.5) to 3.6 mCi; and about 10 mg or less of the antigen-binding construct; b) detecting the $^{89}$Zr-labeled antigen-binding construct in the patient at a first time point after administering the dose, to generate a first patient image corresponding to the first time point, wherein the first time point is about 6 hours to 36 hours after administering; c) determining a first abundance and/or distribution of CD8 cells in one or more tissues and/or neoplasia in the patient based on the first patient image; d) administering to the patient a first treatment for the cancer; e) after administering the first treatment, administering to the human patient diagnosed with the cancer a second dose of the antigen-binding construct; f) determining a second abundance and/or distribution of CD8 cells in the one or more tissues and/or neoplasia in the patient based on a second patient image; and g) comparing the first patient image to the second patient image and administering to the patient a second treatment for the cancer based on a comparison of the first and second patient images.

In some embodiments, a method of treating a patient is provided. The method comprises: a) administering to a human patient diagnosed with a cancer a dose of a CD8 PET tracer, wherein the dose comprises: a CD8 PET tracer that provides a radiation activity of about 0.75 (or 0.5) to 3.6 mCi; and between about 5 micrograms and 50 mg of the CD8 PET tracer (or between about 20 micrograms and 10 mg); b) detecting the CD8 PET tracer in the patient at a first time point after administering the dose, to generate a first patient image corresponding to the first time point, wherein the first time point is about 1 hour to 36 hours after administering; c) determining a first abundance and/or distribution of the CD8 PET tracer in one or more tissues and/or neoplasia in the patient based on the first patient image; d) administering to the patient a first treatment for the cancer; e) after administering the first treatment, administering to the human patient diagnosed with the cancer a second dose of the CD8 PET tracer; f) determining a second abundance and/or distribution of the CD8 PET tracer in the one or more tissues and/or neoplasia in the patient based on a second patient image; and g) comparing the first patient image to the second patient image and administering to the patient a second treatment for the cancer based on a comparison of the first and second patient images.

In some embodiments, the method comprises administering to a patient that has a neoplasia and that is being treated with an immunotherapy ("IOT") an antigen-binding construct that binds to human CD8; wherein the antigen binding construct comprises a detectable marker, wherein the antigen binding construct does not provoke an immune response in the subject, wherein the detectable marker comprises $^{89}$Zr, wherein the detectable marker provides 0.5-3 mCi ±20% of radiation at injection, and wherein an amount between 0.2 and 10mg of total antigen binding construct is provided to the patient; monitoring a distribution of the antigen-binding construct to determine the tumor-infiltrating lymphocyte ("TIL") status within the neoplasia; and administering an alternative IOT to the patient if the TIL status of the neoplasia is not positive, and repeating the foregoing method until the TIL status of the neoplasia becomes positive.

In some embodiments, a method of treating a human subject having cancer is provided. The method comprises: i) providing a first image of a CD8 cells within a tumor in a subject using a CD8 antigen binding construct; ii) administering a therapy including a candidate therapeutic to the subject; iii) after administering the therapy including the candidate therapeutic, providing a second image of the CD8 cells within the tumor in the subject using the CD8 antigen binding construct; and iv) comparing the first and second images to determine if: a) the tumor demonstrates increased CD8 infiltration or b) the tumor demonstrates the same or decreased CD8 infiltration, wherein, if a), then instructing the subject to continue the therapy.

In some embodiments, a method of treating a human subject having cancer is provided. The method comprises i) providing a first image of CD8 cells within a tumor in a subject using a CD8 PET tracer; ii) administering a therapy including a candidate therapeutic to the subject; iii) after administering the therapy, providing a second image of the CD8 cells within the tumor in the subject using the CD8 PET tracer; and iv) comparing the first and second images to determine if: a) the tumor demonstrates increased CD8 infiltration or b) the tumor demonstrates the same or decreased CD8 infiltration, wherein, if a), then instructing the subject to continue the therapy.

In some embodiments, a method of treating a human subject is provided. The method comprises: a) administering a candidate therapeutic to a human subject; b) determining if a size of a tumor in the human subject has increased or decreased in response to the candidate therapeutic; and c) if the size has increased following the candidate therapeutic, then using a CD8 antigen binding construct to determine if an amount of CD8 present within the tumor has increased or decreased in response to the candidate therapeutic. An increase in tumor size without an increase in the amount of

CD8 indicates tumor progression, whereas an increase in tumor size with an increase in the amount of CD8 indicates tumor pseudoprogression, wherein a treatment is continued if the patient is experiencing tumor pseudoprogression, and wherein a treatment is changed if the patient is experiencing tumor progression.

In some embodiments, a method of administering a label to a human subject is provided. The method comprises: administering $^{18}F$ to a subject; within about 6 hours, conducting a PET scan of the subject for a $^{18}F$ distribution of the subject; administering a CD8 PET tracer to the subject, wherein the CD8 PET tracer comprises $^{89}Zr$; and within about 36 hours of administering the CD8 PET tracer, conducting a PET scan on the subject for a distribution of $^{89}Zr$. In some embodiments, the 18F is any a PET isotope with a half-life of less than 3 hours

In some embodiments, a diagnostic composition comprises: 3.0±20% mCi of a 89Zr-Df-labeled antigen binding construct; 20 mM Histidine; 5% sucrose; 51-62 mM Sodium Chloride; 141-194 Arginine; and 2-20 mM Glutamic acid.

In some embodiments, a formulation for a CD8 composition comprises: a CD8 antigen binding construct, wherein the CD8 antigen binding construct is less than 105 kDa in size; and a $^{89}Zr$ radiolabel associated with the CD8 antigen binding construct, wherein the radiolabel provides more than 0.5 but less than 3.6 (or 3.0) mCi of radiation for the formulation, wherein the formulation is configured for administration to a human.

## BRIEF DESCRIPTION OF THE DRAWINGS

**FIG. 1A** illustrates some embodiments of a schematic of a minibody having bivalent binding to CD8.
**FIG. 1B** illustrates some embodiments of a schematic of a minibody.
**FIG. 1C** provides an example of a CD8 alpha.
**FIG. 2A** illustrates some embodiments of an alignment of the OKT8 Variable Heavy ($V_H$) region against a human antibody (4D5v8) and a humanized $V_H$ region of the invention (IAB-huCD8 construct). Some embodiments of the CDR regions (Chothia) are indicated by the boxed region).
**FIG. 2B** illustrates some embodiments of an alignment of the OKT8 Variable Light ($V_L$) region against a humanized $V_L$ region and the IAB-huCD8 construct. Some embodiments of the CDR regions (Chothia) are indicated by the boxed region).
**FIG. 3A** illustrates some embodiments of a schematic of a cys-diabody showing bivalent binding to an antigen.
**FIG. 3B** illustrates a schematic of a cys-diabody showing bivalent binding to an antigen.
**FIG. 4** illustrates some embodiments of a chimeric IAB-huCD8 minibody $V_L$-$V_H$ sequence.
**FIG. 5** illustrates some embodiments of a chimeric IAB-huCD8 minibody $V_H$-$V_L$ sequence.
**FIG. 6** illustrates some embodiments of a humanized IAB-huCD8 minibody $V_L$-$V_H$ sequence.
**FIG. 7** illustrates some embodiments of a humanized IAB-huCD8 minibody $V_H$-$V_L$ sequence.
**FIG. 8** illustrates some embodiments of a humanized IAB-huCD8 cys-diabody $V_L$-5-$V_H$ sequence.
**FIG. 9** illustrates some embodiments of a humanized IAB-huCD8 cys-diabody $V_H$-5-$V_L$ sequence.
**FIG. 10** illustrates some embodiments of a humanized IAB-huCD8 cys-diabody $V_L$-8-$V_H$ sequence.
**FIG. 11** illustrates some embodiments of a humanized IAB-huCD8 cys-diabody $V_H$-8-$V_L$ sequence.
**FIG. 12A** depicts some embodiments of sequences for cys-diabodies.
**FIG. 12B** depicts some embodiments of sequences for minibodies.
**FIG. 12C** depicts some embodiments of sequences for $V_L$.
**FIG. 12D** depicts some embodiments of sequences for hu$V_L$.
**FIG. 12E** depicts some embodiments of sequences for $V_H$.
**FIG. 12F** depicts some embodiments of sequences for hu$V_H$ (version "a" from Version 1).
**FIG. 12G** depicts some embodiments of sequences for hu$V_H$ (version "b" from Version 1).
**FIG. 12H** depicts some embodiments of sequences for hu$V_H$ (version "c" from Version 2).
**FIG. 121** depicts some embodiments of sequences for hu$V_H$ (version "c" from Version 2).
**FIG. 13** illustrates some embodiments of a vector map for pcDNA™ 3.1/myc-His(-) Versions A, B, C.
**FIG. 14** illustrates some embodiments of a method of detecting a presence or absence of a target.
**FIG. 15** shows protein sequence information for some embodiments of IAB22M γ2 EH1.
**FIG. 16** shows protein sequence information for some embodiments of IAB22M γ2 EH2 variant.
**FIG. 17A** shows protein sequence information for some embodiments of IAB22M γ1 EH1.
**FIG. 17B** shows protein sequence information for some embodiments of IAB22M γ2 NH1.
**FIG. 17C** shows protein sequence information for some embodiments of IAB22M γ2 NH2.
**FIG. 18** is an illustration of some embodiments of hinge variants listed in Table 3 (γ1 EH1 top, γ1 EH3 middle, γ1 EH4 bottom).
**FIG. 19A** shows protein sequence information for some embodiments of IAB22M γ1 EH3.
**FIG. 19B** shows protein sequence information for some embodiments of IAB22M γ1 EH5.

**FIG. 19C** shows protein sequence information for some embodiments of IAB22M γ3/γ1 EH6.

**FIG. 19D** shows protein sequence information for some embodiments of IAB22M γ3/γ1 EH7.

**FIG. 19E** shows protein sequence information for some embodiments of IAB22M γ3/γ1 EH8.

**FIG. 20** shows protein sequence information for some embodiments IAB22M γ1 EH2.

**FIG. 21** shows protein sequence information of VL and VH domains of Mbs with different antigen-specificities.

**FIG. 22** shows protein sequence information of various embodiments of linker sequences.

**FIG. 23** shows protein sequence information of various embodiments of hinge regions.

**FIG. 24** shows protein sequence information of various embodiments of $C_H3$ domains.

**FIG. 25** shows an alignment of protein sequences of an embodiment each of IAB22M γ1 EH1(M1) and IAB22M γ1 EH3(M1). Sequence differences are shown in boxes.

**FIG. 26** shows the DNA and translated protein sequence of an embodiment of IAB22M γ1 EH3(M1). In boxes are shown the signal, CDR, linker and hinge sequences.

**FIG. 27** shows the DNA and translated protein sequence of an embodiment of IAB22M γ1 EH5(M1).

**FIG. 28** shows the DNA and translated protein sequence of an embodiment of IAB22M γ1 EH7(M1).

**FIG. 29** shows the DNA and translated protein sequence of an embodiment of IAB22M γ1 EH8(M1).

**FIG. 30** shows the DNA and translated protein sequence of an embodiment of IAB22M γ2 EH2(M1).

**FIG. 31** shows the DNA and translated protein sequence of an embodiment of IAB22M γ2 EH2(M1) with VH-K67R polymorphism.

**FIG. 32** shows the protein sequence of an embodiment of IAB22M VH domain.

**FIG. 33** shows the protein sequence of an embodiment of T-cell surface glycoprotein CD8 alpha chain from *Homo sapiens.*

**FIG. 34** shows the protein sequence of an embodiment of T-cell surface glycoprotein CD8 beta chain from *Homo sapiens.*

**FIG. 35** shows an illustration of an engineered minibody (Mb).

**FIG. 36** shows an illustration of various embodiments of Mb hinges based on human IgG1 (γ1 EH1 top and γ1 EH2 bottom).

**FIG. 37** shows an illustration of various embodiments of Mb hinges based on human IgG2 (γ2 EH1 top and γ2 EH2 bottom).

**FIG. 38** shows an illustration of various embodiments of additional Mb hinges based on human IgG2 (γ2 NH1 top and γ2 NH2 bottom).

**FIG. 39** depicts some embodiments of a CD8 minibody.

**FIG. 40** is a schematic diagram showing the manufacture of $^{89}$Zr-Df-IAB22M2C, according some embodiments of the present disclosure.

**FIG. 41** shows a flow diagram depicting unit steps and respective testing during the manufacture of $^{89}$Zr-Df-IAB22M2C, according to some embodiments of the present disclosure.

**FIGs. 42A** and **42B** show results of a competition FACS assay with AlexaFluor-488 pre-labeled IAB22M2C on HPB-ALL (FIG. 3A) and purified human T cells (FIG. 3B).

**FIG. 43** shows results from T cell proliferation assays using human PBMC.

**FIG. 44** shows results from human PBMC cytokine release assays.

**FIG. 45** shows results of T cell survival (FIG. 6A) and T cell proliferation (FIG. 6B) assays using human PBMC.

**FIG. 46** shows Table 3, showing predicted pharmacokinetic parameters for an intravenous 20 μg/kg dose of $^{89}$Zr-Df-IAB22M2C in humans.

**FIG. 47** shows results of FACS analysis of T-cell populations in the blood of NSG mice engrafted with human CD34 stem cells at baseline.

**FIG. 48** shows results of FACS analysis of T-cell populations in the blood of NSG mice engrafted with human CD34 stem cells at 24 hours.

**FIG. 49** shows results of LegendPlex™ Th1 bead assay performed on serum samples from mice engrafted with human CD34 progenitor cells.

**FIG. 50-FIG. 54** depict PET results for 89ZR-Df-IAB22M2C2.

**FIG. 55** is a flow diagram of a schedule of use of some antigen binding constructs.

**FIG. 56** is a flow diagram of some embodiments of various methods provided herein for screening and patient selection. Figure legend is shown in 5624 in FIG. 56.

**FIG. 57** is a flow diagram of some embodiments of various methods provided herein for treatment initiation and monitoring effectiveness. Figure legend is shown in 5719 in FIG. 57.

**FIG. 58** is a flow diagram of some embodiments of various methods provided herein for treatment minotoring for toxicity. Figure legend is shown in 5824 in FIG. 58.

**FIG. 59** is a flow diagram of some embodiments of various methods provided herein for screening and patient selection for product concept use cases. Figure legend is shown in 5920 in FIG. 59.

**FIG. 60** is a flow diagram of some embodiments of various methods provided herein for treatment initiation and monitoring effectiveness for product use cases. Figure legend is shown in 6019 in FIG. 60.

**FIG. 61** is a flow diagram of some embodiments of various methods provided herein for treatment minotoring for toxicity for product use cases. Figure legend is shown in 6125 in FIG. 61.

**FIG. 62A** is a graph depicting the central read design outlined in Example 85.

**FIGS. 62B-62H** show the time activity curves (TACs) for reference tissues as determined from the SUVmean of each subject enrolled in Stage I. For FIGs. 62B-62J, 62L, and 62M, the order of the legend beneath the chart displays the order of the bars in the graph.

**FIGS. 62I and 62J** are bar charts at stage I and II at 0.5 mg and 1.5 mg.

**FIG. 62K** is a graph showing the frequency of nodal uptake.

**FIGs. 62L** and M are plots of the distribution of SUVmax and SUVpeak uptake values at each of the average time points of the target tumor lesions.

**FIG. 62N** generally depicts a PET scan showing the ability of an antigen binding construct, labeled with $^{89}Zr$, to bind to a target (CD8), within 6 hours or 6 days of administration.

**FIG. 63A** is a chart depicting steps to provide quantitative results for correlation of radiotracer uptake with CD8+ T cell counts as determined by IHC analysis.

**FIGS. 63B** and **63C** are graphs demonstrating the linear relationship between SUV based measurements (SUVmax and SUVpeak) and Average CD8+ T cell count of biopsy samples at baseline (a) and on-treatment (b) including the regression coefficients ($r^2$).

**FIG. 63D** is a graph demonstrating the linear relationship including regression coefficients ($r^2$) when both baseline and on-treatment results are plotted together for SUVmax (a) and SUVpeak(b).

**FIG. 64A** depicts PET images from two subjects over several different points in time.

**FIGs. 64B** and **64C** depict a combination of FDG PET with CD8 PET, to characterize the tumor micro environment more completely.

**FIG. 64D** is a responder image (PET/CT).

**FIG. 64E** is a PET scan and a fused PET/CT scan showing the results of using the minibody construct for testing the effectiveness of various treatments.

**FIG. 64F** is a non-responder image (PET and PET/CT scan).

## DETAILED DESCRIPTION OF VARIOUS EMBODIMENTS

The present disclosure provides methods to visualize CD8 molecules, and thus, CD8+ T cells *in vivo* via noninvasive methods. In some embodiments, a radiolabeled antigen-binding construct of the present disclosure, such as $^{89}Zr$-Df-IAB22M2C, is a PET imaging agent for the detection of CD8+ T cells in a human patient *in vivo.* In some embodiments, the methods of the present disclosure can provide a better understanding of local and systemic immune responses to immunotherapy, select a superior immunotherapy or cancer therapy in general for a subject, and/or result in novel immunotherapy agents for treatment of cancer."

Provided herein are various methods and compositions relating to monitoring, diagnosing, and treating various cancers that relate to CD8 expression, including various compositions therefor and additional alternative uses thereof. Some embodiments provided herein allow for one to monitor the distribution of CD8 molecules and/or CD8 bearing cells, such as CD8+ T cells, within a subject. In some embodiments, this is achieved, at least in part, with antigen binding constructs, such as minibodies, that are labeled, and that bind to human CD8.

In some embodiments, the properties of the antigen binding construct allow for a superior timing of distribution of the antigen binding construct (which can be linked or associated with a detectable marker), with same day imaging be available, or with single dose multiple day imaging being available.

Some embodiments provided herein allow for greater characterization of tumor or neoplasias within a human subject. For example, the processes can allow for a determination of how, or how effectively, various CD8 bearing cells infiltrate a tumor or neoplasia. Based upon this process, one can then determine the correct selection of therapy for a subject, by, for example, providing alternative treatments to see which is most effective in changing the status of the tumor or neoplasia, from one status to an another status. For example, by being able to observe the immune phenotype, one can determine what the subject's current immune phenotype is (e.g., immune desert, immune excluded, or TIL positive (or inflamed)), and then determine if that status should be changed, what therapy should be use to change the status, and what subsequent therapy should be used to treat the patient, given the new status.

The skilled person will understand that "characterization of a tumor" as employed in the invention herein may be represented with diverse words and phrases in addition to "immune desert, immune excluded, or TIL positive (or inflamed)". Some practicioners will use the "hot", "cold" or "intermediate" scale to describe observed results. Other characterizations may provide more detailed histologic information about CD8 bearing lymphocytes such as the location (invasive margin, tumor surface, tumour core); or the density (e.g. immune density and quantity) of the tumor. Other

characterizations may distinguish whether a "hot" tumor is homogeneously infiltrated or heterogeneously infiltrated. Some practicioners may prefer a more comprehensive four- category classification of tumours: hot, altered-excluded, altered- immunosuppressed and cold All such words and phrases are acceptable for describing the observed results of the invention and use of any one phrase herein is not intended to exclude the use of any other appropriate description of the observation.

In addition, various methods for analyzing and characterizing the resulting information from the use of these CD8 antigen binding constructs are also provided, allowing one to, for example, provide a correct therapy for a subject.

As provided herein, using these CD8 antigen binding constructs, methods, and formulations can be useful to determine the CD8 status of a neoplasia. This information can then be employed for various methods, for example, to determine if, generally, an immunotherapy is a good match for a subject (e.g., is the neoplasia TIL positive). Alternatively, this information can be used for screening various therapies in a subject, to determine if a particular immunotherapy is effective in a subject (e.g., the neoplasia is initially immune excluded or an immune desert, but changes to TIL positive (or inflamed) when the subject receives the immunotherapy. Generally speaking, any of the methods provided (for monitoring or treating, etc.) can be used for either arrangement. Thus, the disclosure of one in a particular situation or embodiment is also intended to disclose the other (in the corresponding particular situation or embodiment). It is merely that the subsequent step (e.g., i) administering an immunotherapy to a subject who is TIL positive vs. ii) administering an immunotherapy (or new immunotherapy) to a subject to cause their tumor phenotype to change to TIL positive will vary.)

The following section provides a brief listing of various definitions for various terms as well as various embodiments for some of the terms. Following this section are detailed descriptions of various methods, and additional detail regarding various options and embodiments contemplated for the inventions provided herein. Following that section are a series of examples.

***Definitions and various embodiments***

Unless defined, the plain and ordinary meaning of terms as understood by one of ordinary skill in the art apply.

"Treating" or "treatment" of a condition may refer to preventing the condition, slowing the onset and/or rate of development of the condition, reducing the risk of developing the condition, preventing and/or delaying the development of symptoms associated with the condition, reducing or ending symptoms associated with the condition, generating a complete or partial regression of the condition, or some combination thereof. The term "prevent" does not require the absolute prohibition of the disorder or disease. Treatment includes altering the immune phenotype of the tumor or neoplasia in the subject (from desert to excluded to TIL positive) as well as the subsequent therapeutic application for the tumor for that particular phenotype.

A "therapeutically effective amount" or a "therapeutically effective dose" is an amount that produces a desired therapeutic effect in a subject, such as preventing, treating a target condition, delaying the onset of the disorder and/or symptoms, and/or alleviating symptoms associated with the condition. This amount will vary depending upon a variety of factors, including but not limited to the characteristics of the therapeutic compound (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and/or the route of administration. One skilled in the clinical and pharmacological arts is able to determine a therapeutically effective amount through routine experimentation, for example by monitoring a subject's response to administration of a compound and adjusting the dosage accordingly, given the present disclosure. For additional guidance, see Remington: The Science and Practice of Pharmacy 21.sup.st Edition, Univ. of Sciences in Philadelphia (USIP), Lippincott Williams & Wilkins, Philadelphia, Pa., 2005.

The term "CD8 PET tracer" denotes any molecule that can associate or selectively bind to CD8 and associate a marker or label with the CD8 molecule. This includes aspects such as antigen binding constructs, antibodies, minibodies, diabodies, cys-diabodies, nanobodies, etc. Further included within the scope of CD8 PET tracer are small peptides and molecules that selectively bind to CD8, and to which a PET marker or PET detectable label can be associated (e.g., linked or covalently bonded to). Further examples of CD8 PET tracers include CD8 specific capture agents, such as those disclosed in WO2017/176769, the entirety of which is incorporated herein by reference with respect to such CD-8 specific capture agents. In some embodiments, any of the methods provided herein can employ a CD8 capture agent (or just the "ligands") as provided in WO2017/176769, including the capture agent of any of the following:

(1) HGRGH-Linker-wplrf, targeted against Epitope 2C (AAEGLDTQR) and Epitope IN (SQFRVSPLD).
(2) HGRGH-Linker-AKYRG, targeted against Epitope 2C (AAEGLDTQR) and Epitope IN (SQFRVSPLD).
(3) Ghtwp-Linker-hGrGh, targeted against Epitope 2N (FLLYLSQNKP) and Epitope 2C (AAEGLDTQR).
(4) PWTHG-Linker-AKYRG, targeted against Epitope 2N (FLLYLSQNKP) and Epitope IN (SQFRVSPLD).

In some embodiments, the molecule that binds to CD8 consists or comprises one or more of:

1) A sequence that is 80-100% identical to at least one of: a . HGSYG; b. KRLGA; c . AKYRG; d . hallw; e . lrGyw; f . vashf; g - nGnvh; h . wplrf; i . rwfnv; j havwh; k. wvplw; i. ffrly; and m. wyyGf; or

2)A sequence 80-100% identical to an amino acid sequence selected from the group consisting of: a AGDSW; b. HVRHG; c . HGRGH; d . THPTT; e . FAGYH; f . WTEHG; g . PWTHG; h . TNDFD; i. LFPFD; j . slrfG; k . yfrGs; 1. wnwvG; m. vawlG; n . fhvhG; o . wvsnv; p . wsvnv; q . lnshG; r. yGGvr; s . nsvhG; t . ttvhG; u . fdvGh; v. rhGwk; w. Ghtwp; and x . hGrGh.

In some embodiments, methods for screening and/or making capture agents can also be found in International Publication Nos. WO 2012/106671, WO 2013/033561, WO 2013/009869 and WO 2014/074907, each of which is incorporated by reference, herein, in their entireties. In some embodiments, the CD8 PET tracer is less than 200kDA, 170 kDa, 150kDa, 120 kDa, 105 kDa, 100 kDa, 80kDa, 50 kDa, 30 kDa, 10 kDa, 5 kDa, or 2 kDa.

The term "antigen binding construct" includes all varieties of antibodies, including binding fragments thereof. Further included are constructs that include 1, 2, 3, 4, 5, and/or 6 CDRs. In some embodiments, these CDRs can be distributed between their appropriate framework regions in a traditional antibody. In some embodiments, the CDRs can be contained within a heavy and/or light chain variable region. In some embodiments, the CDRs can be within a heavy chain and/or a light chain. In some embodiments, the CDRs can be within a single peptide chain. In some embodiments, the CDRs can be within two or more peptides that are covalently linked together. In some embodiments, they can be covalently linked together by a disulfide bond. In some embodiments, they can be linked via a linking molecule or moiety. In some embodiments, the antigen binding proteins are non-covalent, such as a diabody and a monovalent scFv. Unless otherwise denoted herein, the antigen binding constructs described herein bind to the noted target molecule. The term also includes minibodies.

The term "CD8 antigen binding construct" refers to an antigen binding construct that binds to CD8. This includes minibody, cys-diabody, ScFv, etc. The term also includes full-length antibody unless excluded expressly or by implication of context. CD8 means "human CD8" which means human CD8 alpha chain: HGNC:HGNC:1706 Ensembl:ENSG00000153563 MIM:186910; human CD8 beta chain HGNC:HGNC:1707 Ensembl:ENSG00000172116 MIM:186730. In some embodiments, the CD8 antigen binding construct is single chain. In some embodiments, the CD8 antigen binding construct is linked-multi-chain. In some embodiments, the CD8 antigen binding construct is humanized. In some embodiments, the CD8 antigen binding construct demonstrates no or low immunogenicity when administered (repeatedly) to a human. In some embodiments, the CD8 antigen binding construct is humanized and demonstrates no or low immunogenicity when administered (repeatedly) to a human. In some embodiments, the CD8 antigen binding construct includes a detectable label unless otherwise indicated.

The terms "cell proliferative disorder" and "proliferative disorder" refer to disorders that are associated with some degree of abnormal cell proliferation. In one embodiment, the cell proliferative disorder is cancer.

"Tumor," as used herein, refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein. The term "neoplasia" encompasses the term tumor.

"Tumor" means solid tumor unless indicated otherwise; includes neoplasia and any aberrant cellular growth of human cells in a subject (but does not include infection by foreign organism).

"Surface of tumor" or "tumor surface" means the outer perimeter of the tumor mass which is in contact with normal (e.g. non-tumor and non-tumor induced) cells of the subject. It is sometimes referred to interchangeably as the "tumor margin" or "tumor border". At a cellular level it may range from a few cells to a few hundred cells in thickness and may be unevenly integrated with surrounding normal cells.

The terms "cancer" and "cancerous" refer to or describe the physiological condition in mammals that is typically characterized by unregulated cell growth. Examples of cancer include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain, as well as head and neck cancer, and associated metastases. The term cancer includes adult and pediatric solid cancers. In some embodiments, the cancer can be a solid tumor.

The term "target" or "target molecule" denotes the CD8 protein. Examples of CD8 proteins are known in the art, and include, for example the CD8 protein of SEQ ID NO: 24, FIG. 1C, or FIG. 33, or FIG. 34. The term CD8, with respect to binding of, for example, an antigen binding contruct binding to CD8, denotes the alpha chain of CD8, the beta chain of CD8, and both the alpha and the beta chain of CD8.

The term "antibody" includes, but is not limited to, genetically engineered or otherwise modified forms of immunoglobulins, such as intrabodies, chimeric antibodies, fully human antibodies, humanized antibodies, antibody fragments, and heteroconjugate antibodies (e.g., bispecific antibodies, diabodies, triabodies, tetrabodies, etc.). The term "antibody" includes cys-diabodies and minibodies. Thus, each and every embodiment provided herein in regard to "antibodies" is also envisioned as cys-diabody and/or minibody embodiments, unless explicitly denoted otherwise. The term "antibody" includes a polypeptide of the immunoglobulin family or a polypeptide comprising fragments of an immunoglobulin that is capable of noncovalently, reversibly, and in a specific manner binding a corresponding antigen. An exemplary antibody structural unit comprises a tetramer. In some embodiments, a full length antibody can be composed of two identical pairs of polypeptide chains, each pair having one "light" and one "heavy" chain (, connected through a disulfide bond. The recognized immunoglobulin genes include the kappa, lambda, alpha, gamma, delta, epsilon, and mu constant region genes, as well as the myriad immunoglobulin variable region genes. For full length chains, the light chains are classified as either kappa or lambda. For full length chains, the heavy chains are classified as gamma, mu, alpha, delta, or epsilon, which in turn define the immunoglobulin classes, IgG, IgM, IgA, IgD, and IgE, respectively. The N-terminus of each chain defines a variable region of about 100 to 110 or more amino acids primarily responsible for antigen recognition. The terms variable light chain ($V_L$) and variable heavy chain ($V_H$) refer to these regions of light and heavy chains respectively. As used in this application, an "antibody" encompasses all variations of antibody and fragments thereof. Thus, within the scope of this concept are full length antibodies, chimeric antibodies, humanized antibodies, single chain antibodies (scFv), Fab, Fab', and multimeric versions of these fragments (e.g., $F(ab')_2$) with the same binding specificity. In some embodiments, the antibody binds specifically to a desired target.

"Complementarity-determining domains" or "complementarity-determining regions ("CDRs") interchangeably refer to the hypervariable regions of $V_L$ and $V_H$. The CDRs are the target protein-binding site of the antibody chains that harbors specificity for such target protein. In some embodiments, there are three CDRs (CDR1-3, numbered sequentially from the N-terminus) in each $V_L$ and/or $V_H$, constituting about 15-20% of the variable domains. The CDRs are structurally complementary to the epitope of the target protein and are thus directly responsible for the binding specificity. The remaining stretches of the $V_L$ or $V_H$, the so-called framework regions (FRs), exhibit less variation in amino acid sequence (Kuby, Immunology, 4th ed., Chapter 4. W.H. Freeman & Co., New York, 2000).

The positions of the CDRs and framework regions can be determined using various well known definitions in the art, e.g., Kabat (Wu, T. T., E. A. Kabat. 1970. An analysis of the sequences of the variable regions of Bence Jones proteins and myeloma light chains and their implications for antibody complementarity. J. Exp. Med. 132: 211-250; Kabat, E. A., Wu, T. T., Perry, H., Gottesman, K., and Foeller, C. (1991) Sequences of Proteins of Immunological Interest, 5th ed., NIH Publication No. 91-3242, Bethesda, MD), Chothia (Chothia and Lesk, J. Mol. Biol., 196:901-917 (1987); Chothia et al., Nature, 342:877-883 (1989); Chothia et al., J. Mol. Biol., 227:799-817 (1992); Al-Lazikani et al., J. Mol. Biol., 273:927-748 (1997)), ImMunoGeneTics database (IMGT) (on the worldwide web at imgt.org/) Giudicelli, V., Duroux, P., Ginestoux, C., Folch, G., Jabado-Michaloud, J., Chaume, D. and Lefranc, M.-P. IMGT/LIGM-DB, the IMGT® comprehensive database of immunoglobulin and T cell receptor nucleotide sequences Nucl. Acids Res., 34, D781-D784 (2006), PMID: 16381979; Lefranc, M.-P., Pommié, C., Ruiz, M., Giudicelli, V., Foulquier, E., Truong, L., Thouvenin-Contet, V. and Lefranc, G., IMGT unique numbering for immunoglobulin and T cell receptor variable domains and Ig superfamily V-like domains Dev. Comp. Immunol., 27, 55-77 (2003). PMID: 12477501; Brochet, X., Lefranc, M.-P. and Giudicelli, V. IMGT/V-QUEST: the highly customized and integrated system for IG and TR standardized V-J and V-D-J sequence analysis Nucl. Acids Res, 36, W503-508 (2008); AbM (Martin et al., Proc. Natl. Acad. Sci. USA, 86:9268-9272 (1989); the contact definition (MacCallum et al., J. Mol. Biol., 262:732-745 (1996)), and/or the automatic modeling and analysis tool Honegger A, Plückthun A. (world wide web at bioc dot uzh dot ch/antibody/Numbering/index dot html).

The term "binding specificity determinant" or "BSD" interchangeably refer to the minimum contiguous or non-contiguous amino acid sequence within a complementarity determining region necessary for determining the binding specificity of an antibody. A minimum binding specificity determinant can be within one or more CDR sequences. In some embodiments, the minimum binding specificity determinants reside within (i.e., are determined solely by) a portion or the full-length of the CDR3 sequences of the heavy and light chains of the antibody. In some embodiments, CDR3 of the heavy chain variable region is sufficient for the antigen binding construct specificity.

The terms "binds in a biased manner" and "binds in a non-biased manner" with respect to the surface of a tumor, refers to an image of a tumor where the detectable label is observed to bind either substantially to the tumor surface (with relatively reduced or absent binding in the interior of the tumor) (="biased") or where the detectable label is not significantly associated with the surface of the tumor (="non-biased") and for example may be dispersed evenly or unevenly throughout the interior of the tumor or absent from the tumor altogether. "Biased" includes binding selectively to any tumor margin without substantially perfusing the volume of the tumor.

The term "Region of interest" or "ROI" means, in or on an image of CD8 distribution in a human subject, a sub-area of the image that is selected by a human operator, optionally assisted by an automated or semi-automated imaging processing method, which narrowly circumscribes a region of the image which identifies a tumor, or is expected to contain a tumor based on other diagnostic methods (e.g. FDG-PET, CT scan, MRI, biopsy, visual inspection, etc.).

The term "Pseudoprogression" and "Pseudo-progression" are used interchangeably in the academic literature and herein. Hyper-progression is the rapid increase in disease progression following initiation of immunotherapy. In some embodiments, any of the pseudo-progression methods provided herein can be applied to hyper-progression as well.

The term "Active marker" relative to a composition comprising a CD8 antigen binding construct, is interchangeable with "detectable label" and means a chemical moiety or a compound that may be detected either by emission based on interrogation or by spontaneous emission, using a specified method of detection which recognizes the emission, such markers including fluorescence imaging components or radiolabels. An "inactive marker" refers to a compound or chemical moiety on the construct that can no longer be detected using the specified method of detection either due to chemical decomposition or to previous spontaneous emission that has rendered the compound or chemical moiety no longer detectable in such method of detection.

The term "distribution", in the context of monitoring, detecting, comparing, or observing a distribution of CD8 antigen binding construct associated with $^{89}Zr$ which has been administered to a subject, means a visual image of the biodistribution of a detected label associated with a CD8 antigen binding construct in relation to a whole body or partial body scan of the subject, which image may be represented as a flat image (2-dimensional) or as computer assisted three-dimensional representation (including a hologram), and is in a format useful to the operator or clinician to observe distribution of the CD8 antigen binding construct at the individual tissue level, and the individual tumor level. In advanced forms of imaging, a "distribution" may not be a visual image of a whole or partial body scan but may instead be a report of a computerized assessment of the absence or presence of a tumor in the subject, and its TIL status, "comparing a distribution of $^{18}F$ to a distribution of $^{89}Zr$" requires aligning the scan of the subject in each distribution so that individual tissues and tumors can be compared.

Response Evaluation Criteria in Solid Tumors ("RECIST"). For computed tomography (CT) anatomic imaging of tumor response, the most common standard is RECIST. This evaluates the size of the tumor to note changes - growth is an indication a treatment is not working, and a decrease in tumor size shows a successful treatment.

PET Response Criteria in Solid Tumors ("PERCIST"). In nuclear imaging of metabolic tumor response assessment with 18F-FDG <u>positron emission tomography</u> (PET), the standard is the PET Response Criteria in Solid Tumors (PERCIST).

An "antibody variable light chain" or an "antibody variable heavy chain" as used herein refers to a polypeptide comprising the $V_L$ or $V_H$, respectively. The endogenous $V_L$ is encoded by the gene segments V (variable) and J (junctional), and the endogenous $V_H$ by V, D (diversity), and J. Each of $V_L$ or $V_H$ includes the CDRs as well as the framework regions. In this application, antibody variable light chains and/or antibody variable heavy chains may, from time to time, be collectively referred to as "antibody chains." These terms encompass antibody chains containing mutations that do not disrupt the basic structure of $V_L$ or $V_H$, as one skilled in the art will readily recognize. In some embodiments, full length heavy and/or light chains are contemplated. In some embodiments, only the variable region of the heavy and/or light chains are contemplated as being present.

Antibodies can exist as intact immunoglobulins or as a number of fragments produced by digestion with various peptidases. Thus, for example, pepsin digests an antibody below the disulfide linkages in the hinge region to produce $F(ab)'_2$, a dimer of Fab' which itself is a light chain ($V_L$-$C_L$) joined to $V_H$-$C_H1$ by a disulfide bond. The $F(ab)'_2$ may be reduced under mild conditions to break the disulfide linkage in the hinge region, thereby converting the $F(ab)'_2$ dimer into an Fab' monomer. The Fab' monomer is a Fab with part of the hinge region. (Paul, Fundamental Immunology 3d ed. (1993). While various antibody fragments are defined in terms of the digestion of an intact antibody, one of skill will appreciate that such fragments may be synthesized *de novo* either chemically or by using recombinant DNA methodology. Thus, the term "antibody," as used herein, also includes antibody fragments either produced by the modification of whole antibodies, or those synthesized *de novo* using recombinant DNA methodologies (e.g., single chain Fv) or those identified using phage display libraries (see, e.g., McCafferty et al., Nature 348:552-554 (1990)).

For preparation of monoclonal or polyclonal antibodies, any technique known in the art can be used (see, e.g., Kohler & Milstein, Nature 256:495-497 (1975); Kozbor et al., Immunology Today 4:72 (1983); Cole et al., Monoclonal Antibodies and Cancer Therapy, pp. 77-96. Alan R. Liss, Inc. 1985; Advances in the production of human monoclonal antibodies Shixia Wang, Antibody Technology Journal 2011:1 1-4; J Cell Biochem. 2005 Oct 1;96(2):305-13; Recombinant polyclonal antibodies for cancer therapy; Sharon J, Liebman MA, Williams BR; and Drug Discov Today. 2006 Jul, 11(13-14):655-60, Recombinant polyclonal antibodies: the next generation of antibody therapeutics?, Haurum JS). Techniques for the production of single chain antibodies (U.S. Pat. No. 4,946,778) can be adapted to produce antibodies to polypeptides of this invention. Also, transgenic mice, or other organisms such as other mammals, may be used to express fully human monoclonal antibodies. Alternatively, phage display technology can be used to identify high affinity binders to selected antigens (see, e.g., McCafferty et al., supra; Marks et al., Biotechnology, 10:779-783, (1992)).

Methods for humanizing or primatizing non-human antibodies are well known in the art. Generally, a humanized antibody has one or more amino acid residues introduced into it from a source which is non-human. These non-human amino acid residues are often referred to as import residues, which are typically taken from an import variable domain. In some embodiments, the terms "donor" and "acceptor" sequences can be employed. Humanization can be essentially

performed following the method of Winter and co-workers (see, e.g., Jones et al., Nature 321:522-525 (1986); Riechmann et al., Nature 332:323-327 (1988); Verhoeyen et al., Science 239:1534-1536 (1988) and Presta, Curr. Op. Struct. Biol. 2:593-596 (1992)), by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some complementarity determining region ("CDR") residues and possibly some framework ("FR") residues are substituted by residues from analogous sites in rodent antibodies.

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, and drug; or (b) the variable region, or a portion thereof, is altered, replaced or exchanged with a variable region having a different or altered antigen specificity.

Antibodies further include one or more immunoglobulin chains that are chemically conjugated to, or expressed as, fusion proteins with other proteins. In some embodiments, the antigen binding constructs can be monovalent scFv constructs. In some embodiments, the antigen binding constructs can be bispecific constructs. A bispecific or bifunctional antibody is an artificial hybrid antibody having two different heavy/light chain pairs and two different binding sites. Other antigen-binding fragments or antibody portions of the invention include bivalent scFv (diabody), bispecific scFv antibodies where the antibody molecule recognizes two different epitopes, single binding domains (sdAb or nanobodies), and minibodies.

The term "antibody fragment" includes, but is not limited to one or more antigen binding fragments of antibodies alone or in combination with other molecules, including, but not limited to Fab', $F(ab')_2$, Fab, Fv, rIgG (reduced IgG), scFv fragments (monovalent, tri-valent, etc.), single domain fragments (nanobodies), peptibodies, minibodies, diabodies, and cys-diabodies. The term "scFv" refers to a single chain Fv ("fragment variable") antibody in which the variable domains of the heavy chain and of the light chain of a traditional two chain antibody have been joined to form one chain.

A pharmaceutically acceptable carrier may be a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or some combination thereof. Each component of the carrier is "pharmaceutically acceptable" in that it is be compatible with the other ingredients of the formulation. It also must be suitable for contact with any tissue, organ, or portion of the body that it may encounter, meaning that it must not carry a risk of toxicity, irritation, allergic response, immunogenicity, or any other complication that excessively outweighs its therapeutic benefits. The pharmaceutical compositions described herein may be administered by any suitable route of administration. A route of administration may refer to any administration pathway known in the art, including but not limited to aerosol, enteral, nasal, ophthalmic, oral, parenteral, rectal, transdermal (e.g., topical cream or ointment, patch), or vaginal. "Transdermal" administration may be accomplished using a topical cream or ointment or by means of a transdermal patch. "Parenteral" refers to a route of administration that is generally associated with injection, including infraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal. In some embodiments, the antigen binding construct can be delivered intraoperatively as a local administration during an intervention or resection.

The term "CD8 dependent disorder" includes cancers for which there is an immunological component (including response to cancer immunotherapies), autoimmune disorders inflammation disorders, and cancers, including but not limited to lung, ovarian, colorectal melanoma etc.

A minibody is an antibody format that has a smaller molecular weight than the full-length antibody while maintaining the bivalent binding property against an antigen. Because of its smaller size, the minibody has a faster clearance from the system and enhanced penetration when targeting tumor tissue. With the ability for strong targeting combined with rapid clearance, the minibody is advantageous for diagnostic imaging and delivery of cytotoxic/radioactive payloads for which prolonged circulation times may result in adverse patient dosing or dosimetry.

The phrase "specifically (or selectively) bind," when used in the context of describing the interaction between an antigen, e.g., a protein, to an antibody or antibody-derived binding agent, refers to a binding reaction that is determinative of the presence of the antigen in a heterogeneous population of proteins and other biologics, e.g., in a biological sample, e.g., a blood, serum, plasma or tissue sample. Thus, under designated immunoassay conditions, in some embodiments, the antibodies or binding agents with a particular binding specificity bind to a particular antigen at least two times the background and do not substantially bind in a significant amount to other antigens present in the sample. Specific binding to an antibody or binding agent under such conditions may require the antibody or agent to have been selected for its specificity for a particular protein. A variety of immunoassay formats may be used to select antibodies specifically immunoreactive with a particular protein. For example, solid-phase ELISA immunoassays are routinely used

to select antibodies specifically immunoreactive with a protein (see, e.g., Harlow & Lane, Using Antibodies, A Laboratory Manual (1998), for a description of immunoassay formats and conditions that can be used to determine specific immunoreactivity). Typically a specific or selective binding reaction will produce a signal at least twice over the background signal and more typically at least than 10 to 100 times over the background.

The term "equilibrium dissociation constant ($K_D$, M)" refers to the dissociation rate constant ($k_d$, time$^{-1}$) divided by the association rate constant ($k_a$, time$^{-1}$, $M^{-1}$). Equilibrium dissociation constants can be measured using any known method in the art. The antibodies of the present invention generally will have an equilibrium dissociation constant of less than about $10^{-7}$ or $10^{-8}$ M, for example, less than about $10^{-9}$ M or $10^{-10}$ M, in some embodiments, less than about $10^{-11}$ M, $10^{-12}$ M, or $10^{-13}$ M.

The term "isolated," when applied to a nucleic acid or protein, denotes that the nucleic acid or protein is essentially free of other cellular components with which it is associated in the natural state. In some embodiments, it can be in either a dry or aqueous solution. Purity and homogeneity can be determined using analytical chemistry techniques such as polyacrylamide gel electrophoresis or high performance liquid chromatography. A protein that is the predominant species present in a preparation is substantially purified. In particular, an isolated gene is separated from open reading frames that flank the gene and encode a protein other than the gene of interest. The term "purified" denotes that a nucleic acid or protein gives rise to essentially one band in an electrophoretic gel. In some embodiments, this can denote that the nucleic acid or protein is at least 85% pure, more preferably at least 95% pure, and most preferably at least 99% pure of molecules that are present under in vivo conditions.

The term "nucleic acid" or "polynucleotide" refers to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The terms "polypeptide," "peptide," and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, gamma-carboxyglutamate, and O-phosphoserine. Amino acid analogs refer to compounds that have the same basic chemical structure as a naturally occurring amino acid, i.e., an .alpha.-carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing

functionally similar amino acids are well known in the art. Such conservatively modified variants are in addition to and do not exclude polymorphic variants, interspecies homologs, and alleles of the invention.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)).

"Percentage of sequence identity" can be determined by comparing two optimally aligned sequences over a comparison window, wherein the portion of the polynucleotide sequence in the comparison window may comprise additions or deletions (i.e., gaps) as compared to the reference sequence (e.g., a polypeptide of the invention), which does not comprise additions or deletions, for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The terms "identical" or percent "identity," in the context of two or more nucleic acids or polypeptide sequences, refer to two or more sequences or subsequences that are the same sequences. Two sequences are "substantially identical" if two sequences have a specified percentage of amino acid residues or nucleotides that are the same (for example, 70%, 75%, 80%, 85%, 90%, 95%, 96%, 97%, 98% or 99% sequence identity over a specified region, or, when not specified, over the entire sequence of a reference sequence), when compared and aligned for maximum correspondence over a comparison window, or designated region as measured using one of the following sequence comparison algorithms or by manual alignment and visual inspection. Some embodiments provided herein provide polypeptides or polynucleotides that are substantially identical to the polypeptides or polynucleotides, respectively, exemplified herein (e.g., the variable regions exemplified in any one FIGs. 2A, 2B, or 4-11, 12C-12I; the CDRs exemplified in any one of FIGs. 2A, 2B, or 12C to 12I; the FRs exemplified in any one of FIGs. 2A, 2B, or 12C-12I; and the nucleic acid sequences exemplified in any one of FIGs. 12A-12I or 4-11). Optionally, the identity exists over a region that is at least about 15, 25 or 50 nucleotides in length, or more preferably over a region that is 100 to 500 or 1000 or more nucleotides in length, or over the full length of the reference sequence. With respect to amino acid sequences, identity or substantial identity can exist over a region that is at least 5, 10, 15 or 20 amino acids in length, optionally at least about 25, 30, 35, 40, 50, 75 or 100 amino acids in length, optionally at least about 150, 200 or 250 amino acids in length, or over the full length of the reference sequence. With respect to shorter amino acid sequences, e.g., amino acid sequences of 20 or fewer amino acids, in some embodiments, substantial identity exists when one or two amino acid residues are conservatively substituted, according to the conservative substitutions defined herein.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, e.g., by the local homology algorithm of Smith and Waterman (1970) Adv. Appl. Math. 2:482c, by the homology alignment algorithm of Needleman and Wunsch (1970) J. Mol. Biol. 48:443, by the search for similarity method of Pearson and Lipman (1988) Proc. Nat'1. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection (see, e.g., Ausubel et al., Current Protocols in Molecular Biology (1995 supplement)).

Two examples of algorithms that are suitable for determining percent sequence identity and sequence similarity are the BLAST and BLAST 2.0 algorithms, which are described in Altschul et al. (1977) Nuc. Acids Res. 25:3389-3402, and Altschul et al. (1990) J. Mol. Biol. 215:403-410, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative

score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff (1989) Proc. Natl. Acad. Sci. USA 89:10915) alignments (B) of 50, expectation (E) of 10, M=5, N=-4, and a comparison of both strands.

The BLAST algorithm also performs a statistical analysis of the similarity between two sequences (see, e.g., Karlin and Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, in some embodiments, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence.

The terms "subject," "patient," and "individual" interchangeably refer to an entity that is being examined and/or treated. This can include, for example, a mammal, for example, a human or a non-human primate mammal. The mammal can also be a laboratory mammal, e.g., mouse, rat, rabbit, hamster. In some embodiments, the mammal can be an agricultural mammal (e.g., equine, ovine, bovine, porcine, camelid) or domestic mammal (e.g., canine, feline).

The term "therapeutically acceptable amount" or "therapeutically effective dose" interchangeably refer to an amount sufficient to effect the desired result. In some embodiments, a therapeutically acceptable amount does not induce or cause undesirable side effects. A therapeutically acceptable amount can be determined by first administering a low dose, and then incrementally increasing that dose until the desired effect is achieved.

The term "co-administer" refers to the administration of two active agents in the blood of an individual or in a sample to be tested. Active agents that are co-administered can be concurrently or sequentially delivered.

"Label" or "detectable marker" are used interchangeably herein and refer to a detectable compound or composition which is conjugated directly or indirectly associated with the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

The term "ImmunoPET" is a term used for positron emission tomography (PET) of radiolabelled antibodies.

The term "cytotoxic agent" as used herein refers to a substance that inhibits or prevents a cellular function and/or causes cell death or destruction. The term is intended to include radioactive isotopes (e.g., At.sup.211, I.sup.131, I.sup.125, Y.sup.90, Re.sup.186, Re.sup.188, Sm.sup.153, Bi.sup.212, P.sup.32, Pb.sup.212 and radioactive isotopes of Lu), chemotherapeutic agents (e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, toxins, growth inhibitory agents, drug moieties, and the various antitumor or anticancer agents disclosed below. Other cytotoxic agents are described below. A tumoricidal agent causes destruction of tumor cells.

A "toxin" is any substance capable of having a detrimental effect on the growth or proliferation of a cell.

A "chemotherapeutic agent" is a chemical compound useful in the treatment of cancer. Examples of chemotherapeutic agents include alkylating agents such as thiotepa and CYTOXAN™ cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylolomelamine; acetogenins (especially bullatacin and bullatacinone); delta-9-tetrahydrocannabinol (dronabinol, MARINOL™); beta-lapachone; lapachol; colchicines; betulinic acid; a camptothecin (including the synthetic analogue topotecan (HYCAMTIN™), CPT-11 (irinotecan, CAMPTOSAR™), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gammall and calicheamicin omegall (see, e.g., Agnew, Chem Intl. Ed. Engl., 33: 183-186 (1994)); dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, ADRIAMYCIN™ doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK.RTM. polysaccharide complex (JHS Natural Products, Eugene, Oreg.); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE™, FILDESIN™); dacarbazine; mannomustine; mitobronitol; mitolactol; pipobroman; gacytosine; arabinoside ("Ara-C"); thiotepa; taxoids, e.g., TAXOL.RTM. paclitaxel (Bristol-Myers Squibb Oncology, Princeton, N.J.), ABRAXANE™ Cremophor-free, albumin-engineered nanoparticle formulation of paclitaxel (American Pharmaceutical Partners, Schaumberg, Ill.), and TAXOTERE™ docetaxel (Rhone-Poulenc Rorer, Antony, France); chloranbucil; gemcitabine (GEMZAR™); 6-thioguanine; mercaptopurine; methotrexate; platinum analogs such as cisplatin and carboplatin; vinblastine (VELBAN™); platinum; etoposide (VP-16); ifosfamide; mitoxantrone; vincristine (ONCOVIN™); oxaliplatin; leucovovin; vinorelbine (NAVELBINE™); novantrone; edatrexate; daunomycin; aminopterin; ibandronate; topoisomerase inhibitor RFS 2000; difluoromethylornithine (DMFO); retinoids such as retinoic acid; capecitabine (XELODA™); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX™ tamoxifen), EVISTA™ raloxifene, droloxifene, 4-hydroxytamoxifen, trioxifene, keoxifene, LY117018, onapristone, and FARESTON™ toremifene; anti-progesterones; estrogen receptor down-regulators (ERDs); agents that function to suppress or shut down the ovaries, for example, leutinizing hormone-releasing hormone (LHRH) agonists such as LUPRON™ and ELIGARD™ leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE™ megestrol acetate, AROMASIN™ exemestane, formestanie, fadrozole, RIVISOR™ vorozole, FEMARA™ letrozole, and ARIMIDEX™ anastrozole. In addition, such definition of chemotherapeutic agents includes bisphosphonates such as clodronate (for example, BONEFOS™ or OSTAC™), DIDROCAL™ etidronate, NE-58095, ZOMETA™ zoledronic acid/zoledronate, FOSAMAX™ alendronate, AREDIA™ pamidronate, SKELID™ tiludronate, or ACTONEL™ risedronate; as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE™ vaccine and gene therapy vaccines, for example, ALLOVECTIN™ vaccine, LEUVECTIN™ vaccine, and VAXID™ vaccine; LURTOTECAN™ topoisomerase 1 inhibitor; ABARELIX™ rmRH; lapatinib ditosylate (an ErbB-2 and EGFR dual tyrosine kinase small-molecule inhibitor also known as GW572016); and pharmaceutically acceptable salts, acids or derivatives of any of the above.

A "growth inhibitory agent" when used herein refers to a compound or composition which inhibits growth of a cell either in vitro or in vivo. Thus, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating

agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE™, Rhone-Poulenc Rorer), derived from the European yew, is a semisynthetic analogue of paclitaxel (TAXOL™, Bristol-Myers Squibb). Paclitaxel and docetaxel promote the assembly of microtubules from tubulin dimers and stabilize microtubules by preventing depolymerization, which results in the inhibition of mitosis in cells.

"Immunotherapy" (also called "Immunostimulation" and "IOT") means the prevention or treatment of disease with a therapy (e.g. an agent or a course of treatment) that stimulates the host immune response to the disease. Many diseases are treatable with immunotherapy. Academic literature in recent years has often used immunotherapy to refer specifically immuno-oncology, which denotes cancer treatment which aims to reduce the immune avoidance characteristics of a tumor or neoplasia, thereby allowing natural or modified immune cells to identify and eliminate the cancerous tissue. As used in this specification, immunotherapy refers to immune-oncology unless the context specifically indicates otherwise. "Immunotherapy" also may refer to an immunotherapy agent, or to a method of using such an agent, depending on context. Various immunotherapeutic agents are now available, and many more are in clinical and pre-clinical development. Well known immunotherapeutic agents include but are not limited to, checkpoint inhibitor ("CPI") therapy (e.g. anti-PD-1 (Keytruda® pembrolizumab) or anti PD-L1 (Opdivo® nivolumab) binding agents), IL2 and fragments or prodrugs thereof (e.g. NKTR-214 , a prodrug of PEG-conjugated IL2 (aldesleukin)), other CD122 (IL2RB interleukin 2 receptor subunit beta) binding ligands, GAd-NOUS-20 neoantigen vaccine (D'Alise et al 2017; which may enhance NKTR-214 activity), T-cell bi-specific agent therapy, therapy for reversal of T-cell exhaustion, inhibition of indoleamine 2,3-dioxygenase (IDO) (such as with epacadostat (INCB024360)), and CAR-T therapy.

The terms "Immune check point inhibitor" (sometimes referred to as "ICI"), or "checkpoint inhibitor" (sometimes "CPI") or "immune checkpoint blockade inhibitor" and all similar terms, denote a subclass of immunotherapies. Examples include molecules that block certain proteins made by some types of immune system cells, such as T cells, and some cancer cells. These proteins help keep immune responses in check and can keep T cells from killing cancer cells. When these proteins are blocked, the immune system is tree to be active and T cells are able to kill cancer cells. Some embodiments include anti-PD1 and anti-PD-L1 binding agents, anti-CTLA4 agents, and multi-specific agents including, but not limited to, anti-CTLA-4/B7-1/B7-2. Additional immunotherapies include checkpoint inhibitors such as ipilimumab (Yervoy), pembrolizumab (Keytruda), nivolumab (Opdivo), atezolizumab (Tecentriq), avelumab (Bavencio), and durvalumab (Imfinzi). IOTs also include tremelimumab and pidilizumab, Small molecule ICIs are also in development including BMS-1001, BMS-1116, CA-170, CA-327, Imiquimod, Resiquimod, 852A, VTX-2337, ADU-S100, MK-1454, Ibrutinib, 3AC, Idelalisib, IPI-549, Epacadostat, AT-38, CPI-444, Vipadenant, Preladenant, PBF, AZD4635, Galuniseritib, OTX015/MK-8628, CPI-0610 (c.f. Kerr and Chisolm (2019) The Journal of Immunology, 2019, 202: 11-19.)

IOTs also include other modalities which are not CPIs but which also activate the host immune system against the cancer, or render the tumor vulnerable to CPI therapy. Such alternative IOTs include but are not limited to: T-cell immunomodulators such as the cytokines IL-2, IL-7, IL-15, IL-21, IL-12, GM- CSF and IFN$\alpha$ (including THOR-707 of Synthorx Therapeutics; and NKTR-214 bempegaldesleukin of Nektar Therapeutics); Various other interferons and interleukins; TGF$\beta$1 inhibitors (such as SRK-181 in development by Scholar Rock); Oncolytic therapy (including oncolytic virus therapy); Adoptive cell therapy such as T cell-therapy (including CAR-T cell therapy); Cancer vaccines (both preventative and treatment based). Immunotherapy also includes strategies that increase the burden of neoantigens in tumour cells, including targeted therapies which cause a tumor cell to express or reveal tumor associated antigens. (c.f. Galon and Bruni (2019) Nature Reviews Drug Discovery v. 18, pages197-218). Further IOTs include TLR9 ligands (Checkmate Pharmaceuticals), A2A/A2B dual antagonists (Arcus Biosciences) and vaccination peptides directed to endogenous enzymes such as IDO-1 and arginase (IO Biotech). IOTs include HS-110, HS-130 and PTX-35 (Heat Biologics),

Those skilled in the art recognize that immunotherapies may be used in combination with each other. Immunotherapies can also be used before, after, or in combination with other therapies for the disease, including in the case of cancer, radiation therapy, chemotherapy of all types (including the cytotoxic agents, chemotherapeutic agents, anti-hormonal agents, and growth inhibitory agents referred to above) and surgical resection.

In some embodiments, any method of therapy can be employed that acts on or through CD8. This is designated as a "CD8 therapy". This includes immunotherapies, cellular therapies, vaccines, therapeutics to boost/restore immune response/function, any treatment that affects CD8+ T cells presence, activation, number or trafficking in relation to tumor lesions, chemotherapies, oncolytic viruses, radiation therapy, cellular therapies etc.

"Tumor-infiltrating lymphocyte" or "TIL" refers to a lymphocyte, such as a CD8 bearing T-cell, which is found within the border of a solid tumor.

"Tumor-infiltrating lymphocyte status" or "TIL" status or other similar term denotes the degree to which lymphocytes, and in particular CD8 bearing T-cells can penetrate into a tumor or neoplasia or tumor stroma. A neoplasia is characterized as TIL positive, when the CD8 cells can penetrate into the tumor or tumor stroma itself and be found

within the tumor or tumor stroma itself. TIL positive can be further divided into TIL positive homogenous (even distribution throughout the interior of the tumor) or TIL positive heterogenous (significantly uneven distribution, or partial distribution, in the interior of the tumor). A TIL positive tumor may also be described as "inflamed". A neoplasia is characterized as TIL negative when practically no CD8+ cells infiltrate the tumor or neoplasia (e.g., apart from general background noise signal). Generally, these TIL negative neoplasias are immune excluded or immune deserts.

"PET" is a diagnostic technique that can be used to observe functions and metabolism of human organs and tissues at the molecular level. For PET, a positron radioactive drug (e.g., $^{18}$F-FDG) can be injected into a human body. If FDG is used, because the metabolism of fludeoxyglucose (FDG) is similar to glucose, the FDG will gather in cells that digest the glucose. The uptake of the radioactive drug by rapidly growing tumor tissues is different. A positron emitted by the decay of $^{18}$F and an electron in tissues will undergo an annihilation reaction to generate two gamma-photons with the same energy in opposite directions. A detector array surrounding the human body can detect the two photons using a coincidence measurement technique, and determine position information of the positron. A tomography image of positrons in the human body can then be constructed by processing the position information using an image reconstruction software. In some situations, Immuno-PET can be employed, where the label (e.g., 18F) is attached or associated with an antigen binding construct. In such embodiments, the distribution of the antigen binding construct can be monitored, which will depend upon the binding properties and distribution properties of the antigen binding construct. For example, if a CD8 directed minibody is used, then PET can be used to monitor the distribution of the CD8 molecules through the hosts' system. PET systems are known in the art and include, for example U.S. Pat. Pub. No. 20170357015, 20170153337, 20150196266, 20150087974, 20120318988, and 20090159804, the entireties of each of which are incorporated by reference herein for their description regarding PET and the use thereof.

Embodiments provided herein and corresponding detailed description may be presented in terms of software, or algorithms and symbolic representations of operation on data bits within a computer memory. These descriptions and representations are the ones by which those of ordinary skill in the art effectively convey the substance of their work to others of ordinary skill in the art. An algorithm, as the term is used here, and as it is used generally, is conceived to be a self-consistent sequence of steps leading to a desired result. The steps are those requiring physical manipulations of physical quantities. Usually, though not necessarily, these quantities take the form of optical, electrical, or magnetic signals capable of being stored, transferred, combined, compared, and otherwise manipulated. It has proven convenient at times, principally for reasons of common usage, to refer to these signals as bits, values, elements, symbols, characters, terms, numbers, or the like.

In the following description, illustrative embodiments may be described with reference to acts and symbolic representations of operations that may be implemented as program modules or functional processes include routines, programs, objects, components, data structures, etc., that perform particular tasks or implement particular abstract data types and may be implemented using existing hardware at existing network elements. Such existing hardware may include one or more Central Processing Units (CPUs), digital signal processors (DSPs), application-specific-integrated-circuits, field programmable gate arrays (FPGAs) computers or the like.

Note also that the software implemented aspects of the example embodiments may be typically encoded on some form of program storage medium or implemented over some type of transmission medium. The program storage medium (e.g., non-transitory storage medium) may be magnetic (e.g., a floppy disk or a hard drive) or optical (e.g., a compact disk read only memory, or "CD ROM"), and may be read only or random access. Similarly, the transmission medium may be twisted wire pairs, coaxial cable, optical fiber, or some other suitable transmission medium known to the art. The example embodiments not limited by these aspects of any given implementation.

It should be borne in mind, however, that all of these and similar terms are to be associated with the appropriate physical quantities and are merely convenient labels applied to these quantities. Unless specifically stated otherwise, or as is apparent from the discussion, terms such as "processing" or "computing" or "calculating" or "determining" of "displaying" or the like, refer to the action and processes of a computer system, or similar electronic computing device/hardware, that manipulates and transforms data represented as physical, electronic quantities within the computer system's registers and memories into other data similarly represented as physical quantities within the computer system memories or registers or other such information storage, transmission or display devices.

## IMAGING - MONITOR RESPONSE

In some embodiments, a method of determining if a subject is responding to an IOT comprises providing a baseline CD8 distribution for the subject within a ROI, via an in vivo technique, administering an IOT, and providing a treated CD8 distribution for the subject within the ROI. The treated CD8 distribution is obtained within 35 days of administering the IOT. The treated CD8 distribution is compared to the baseline CD8 distribution to determine if the subject is responding to the IOT.

In some embodiments, a method of determining if a subject is responding to an IOT comprises providing a baseline CD8 distribution for the subject within a ROI. In some embodiments, a method of determining if a subject is

responding to an IOT comprises providing a baseline CD8 distribution for the subject within a ROI via an in vivo technique. In some embodiments, a method of determining if a subject is responding to an IOT comprises providing a baseline CD8 distribution for the subject within a ROI via an in vivo technique, and administering an IOT.

In some embodiments, a method of determining if a subject is responding to an IOT comprises providing a baseline CD8 distribution for the subject within a ROI via an in vivo technique, administering an IOT, and providing a treated CD8 distribution for the subject within the ROI.

In some embodiments of a method of determining if a subject is responding to an IOT, treated CD8 distribution is obtained within 5 , 10, 15, 20, 25, or 30 days of administering the IOT.

In some embodiments, a method of predicting a subject's responsiveness to a checkpoint inhibitor comprises providing a baseline CD8 distribution for the subject within a ROI, via an in vivo technique, administering a checkpoint inhibitor, providing a checkpoint inhibitor CD8 distribution for the subject within the ROI. The checkpoint inhibitor CD8 distribution is obtained within 35 days of administering the IOT. The checkpoint inhibitor CD8 distribution is compared to the baseline CD8 distribution to determine if the subject is responsive to the checkpoint inhibitor. In some embodiments, a method of predicting a subject's responsiveness to a checkpoint inhibitor comprises providing a baseline CD8 distribution for the subject within a ROI.

In some embodiments, a method of predicting a subject's responsiveness to a checkpoint inhibitor comprises providing a baseline CD8 distribution for the subject within a ROI via an in vivo technique.

In some embodiments, a method of predicting a subject's responsiveness to a checkpoint inhibitor comprises providing a baseline CD8 distribution for the subject within a ROI via an in vivo technique, and administering a checkpoint inhibitor. In some embodiments, a method of predicting a subject's responsiveness to a checkpoint inhibitor comprises providing a baseline CD8 distribution for the subject within a ROI via an in vivo technique, administering a checkpoint inhibitor, and providing a checkpoint inhibitor CD8 distribution for the subject within the ROI.

In some embodiments of a method of predicting a subject's responsiveness to a checkpoint inhibitor, the treated CD8 distribution is obtained within 3-8 days of administering the IOT. In some embodiments of a method of predicting a subject's responsiveness to a checkpoint inhibitor, the checkpoint inhibitor CD8 distribution is obtained within 5, 10, 15, 20, 25, or 30 days of administering the IOT. In some embodiments of a method of predicting a subject's responsiveness to a checkpoint inhibitor, the treated CD8 distribution is obtained within 3-5 days, 4-6 days, 5-7 days, or 6-8 days of administering the IOT.

In some embodiments, a method of monitoring an effectiveness of a therapy comprises providing a first CD8 image for a subject, the subject being at a baseline for a therapy, administering a therapy to the subject, and providing a second CD8 image for the subject. At least one day has passed since the therapy was administered to the subject. If there is no significant increase in CD8 imaging agent, the subject is not responding to the therapy.

In some embodiments, a method of monitoring an effectiveness of a therapy comprises providing a first CD8 image. In some embodiments, a method of monitoring an effectiveness of a therapy comprises providing a first CD8 image for a subject, who is at a baseline for a therapy. In some embodiments, a method of monitoring an effectiveness of a therapy comprises providing a first CD8 image for a subject, who is at a baseline for a therapy, and administering a therapy to the subject. In some embodiments, a method of monitoring an effectiveness of a therapy comprises providing a first CD8 image for a subject, who is at a baseline for a therapy, administering a therapy to the subject, and providing a second CD8 image for the subject.

In some embodiments, a method of analysis of a tumor comprises characterizing a degree of relative CD8 infiltration in a tumor in a subject in response to a candidate therapeutic, said characterizing comprising observing if there is an increase or a decrease in degree of CD8 infiltration via a CD8 antigen binding construct comprising a radiolabel. An increase in CD8 infiltration indicates that the candidate therapeutic is functioning as a therapeutic. A decrease in CD8 infiltration indicates that the candidate therapeutic is not functioning as a therapeutic. The tumor has increased in size between before the candidate therapeutic is administered and after the candidate therapeutic is administered.

In some embodiments, a method of analysis of a tumor comprises characterizing a degree of relative CD8 infiltration in a tumor in a subject. In some embodiments, a method of analysis of a tumor comprises characterizing a degree of relative CD8 infiltration in a tumor in a subject in response to a candidate therapeutic. In some embodiments, a method of analysis of a tumor comprises characterizing a degree of relative CD8 infiltration in a tumor in a subject in response to a candidate therapeutic, said characterizing comprising observing if there is an increase or a decrease in degree of CD8 infiltration.

In some embodiments, a method of analysis of a tumor comprises characterizing a degree of relative CD8 infiltration in a tumor in a subject in response to a candidate therapeutic, said characterizing comprising observing if there is an increase or a decrease in degree of CD8 infiltration via a CD8 antigen binding construct comprising a radiolabel.

## USING CD8 ANTIGEN BINDING CONSTRUCT FOR NON-INVASIVE IMAGING

In some embodiments, a method of visualizing a distribution of CD8 bearing cells in a human subject by PET

comprises administering a CD8 antigen binding construct to the subject the CD8 antigen binding construct being labeled with a PET detectable label, and monitoring a distribution of the CD8 antigen binding construct in the subject within 6-36 hours of administering the CD8 antigen binding construct. The monitoring can detect a tissue infiltrated with 500 or less CD8 positive cells per $mm^2$ within the subject. The monitoring is achieved via PET and converted to an image for visualizing the distribution.

In some embodiments, a method of visualizing a distribution of CD8 bearing cells in a human subject by PET comprises administering a CD8 antigen binding construct to the subject. In some embodiments, a method of visualizing a distribution of CD8 bearing cells in a human subject by PET comprises administering a CD8 antigen binding construct to the subject, the CD8 antigen binding construct being labeled with a PET detectable label. In some embodiments, a method of visualizing a distribution of CD8 bearing cells in a human subject by PET comprises administering a CD8 antigen binding construct to the subject, the CD8 antigen binding construct being labeled with a PET detectable label, and monitoring a distribution of the CD8 antigen binding construct in the subject.

In some embodiments, a method of visualizing a distribution of CD8 bearing cells in a human subject by PET comprises administering a CD8 antigen binding construct to the subject, the CD8 antigen binding construct being labeled with a PET detectable label, and monitoring a distribution of the CD8 antigen binding construct in the subject within 6-36 hours of administering the CD8 antigen binding construct.

In some embodiments of a method of visualizing a distribution of CD8 bearing cells in a human subject by PET, the monitoring can detect a tissue infiltrated with 1, 5, 10, 25, 50, 100, 150, 200, 250, 300, 350, 400, 450, or less than 500 CD8 positive cells per $mm^2$ within the subject, or a value within a range defined by any two of the aforementioned values.

In some embodiments, a method of visualizing cells in a human comprises providing a means of binding human CD8 positive cells to a subject, the means comprising a detectable label, and monitoring and determining a first distribution of the means of binding human CD8 positive cells in the subject within 6-36 hours of administering the means of binding human CD8 positive cells to the subject.

In some embodiments, a method of visualizing cells in a human comprises providing a means of binding human CD8 positive cells to a subject, the means comprising a detectable label.

In some embodiments, a method of visualizing cells in a human comprises providing a means of binding human CD8 positive cells to a subject, the means comprising a detectable label, and monitoring and determining a first distribution of the means of binding human CD8 positive cells in the subject.

In some embodiments, a method of visualizing cells in a human comprises providing a means of binding human CD8 positive cells to a subject, the means comprising a detectable label, and monitoring and determining a first distribution of the means of binding human CD8 positive cells in the subject within 6-36 hours of administering the means of binding human CD8 positive cells to the subject.

In some embodiments, a method of determining the TIL status of a tumor in a human subject comprises administering a CD8 antigen binding construct to the subject, the CD8 antigen binding construct being labeled with a PET detectable label, and monitoring a distribution of the CD8 antigen binding construct having the PET detectable label in the subject, and evaluating the degree to which the PET detectable label has penetrated a known or suspected tumor in the subject. A tumor is TIL positive if the PET detectable label has penetrated the tumor and TIL negative if not.

In some embodiments, a method of determining the TIL status of a tumor in a human subject comprises administering a CD8 antigen binding construct to the subject.

In some embodiments, a method of determining the TIL status of a tumor in a human subject comprises administering a CD8 antigen binding construct to the subject, the CD8 antigen binding construct being labeled with a PET detectable label.

In some embodiments, a method of determining the TIL status of a tumor in a human subject comprises administering a CD8 antigen binding construct to the subject, the CD8 antigen binding construct being labeled with a PET detectable label, and monitoring a distribution of the CD8 antigen binding construct having the PET detectable label in the subject.

In some embodiments, a method of determining the TIL status of a tumor in a human subject comprises administering a CD8 antigen binding construct to the subject, the CD8 antigen binding construct being labeled with a PET detectable label, and monitoring a distribution of the CD8 antigen binding construct having the PET detectable label in the subject, and evaluating the degree to which the PET detectable label has penetrated a known or suspected tumor in the subject. In some embodiments, a method of imaging a change in distribution of CD8 cells in a human subject having cancer comprises administering to the subject a CD8 antigen binding construct, determining by PET a first distribution of CD8 antigen binding construct in the subject, administering an immunotherapy to the subject, and thereafter determining by PET a second distribution of CD8 antigen binding construct in the subject. A difference between the first distribution and the second distribution demonstrates the change. In some embodiments, a method of imaging a change in distribution of CD8 cells in a human subject having cancer comprises administering to the subject a CD8 antigen binding construct.

In some embodiments, a method of imaging a change in distribution of CD8 cells in a human subject having cancer comprises administering to the subject a CD8 antigen binding construct, and determining by PET a first distribution of CD8 antigen binding construct in the subject.

In some embodiments, a method of imaging a change in distribution of CD8 cells in a human subject having cancer comprises administering to the subject a CD8 antigen binding construct, and determining by PET a first distribution of CD8 antigen binding construct in the subject, and administering an immunotherapy to the subject.

In some embodiments, a method of imaging a change in distribution of CD8 cells in a human subject having cancer comprises administering to the subject a CD8 antigen binding construct, determining by PET a first distribution of CD8 antigen binding construct in the subject, administering an immunotherapy to the subject, and thereafter determining by PET a second distribution of CD8 antigen binding construct in the subject. In some embodiments of the a method of imaging a change in distribution of CD8 cells in a human subject having cancer, the second distribution is determined based on a second PET image of the subject obtained within 10 days of administering the CD8 antigen binding construct. In some embodiments of the a method of imaging a change in distribution of CD8 cells in a human subject having cancer, the second distribution is determined based on a second administration to the subject of CD8 antigen binding construct.

## Quantifying CD8 T-cell density of TIL Positive tumor

In some embodiments, a method of determining the TIL status of a tumor in a human subject comprises administering a CD8 antigen binding construct to the subject, thereafter monitoring a distribution of the CD8 antigen binding construct in the subject by PET. The monitoring identifies a TIL positive tumor. The monitoring provides at least one of: a tumor that is infiltrated with 500 or less CD8 bearing cells per $mm^2$ within the subject, a tumor that is infiltrated with greater than or less than 500 CD8 bearing cells per $mm^2$ within the subject, a tumor that is infiltrated with greater than or less than 200 CD8 bearing cells per $mm^2$ within the subject, a tumor that is infiltrated with greater than or less than 100-500 CD8 bearing cells/$mm^2$, a tumor that is infiltrated with less than 1% CD8 bearing cells, 1-50% CD8 bearing cells or greater than 50% CD8 bearing cells, a tumor that comprises CD8 bearing cells that corresponds to IHC measurement of 2.5 to 25% of cells in a 4 micron tissue section, a tumor that comprises between 10,000 to 100,000 CD8 bearing cells is per $mm^3$ of the corresponding tumor, a cell intensity of CD8 bearing cells, of the corresponding tumor that is low (cold) or high (hot). In some embodiments, this percentage can be calculated as a percentage of CD8 bearing cells per total tumor cells estimated in the corresponding tumor.

In some embodiments, a method of determining the TIL status of a tumor in a human subject comprises administering a CD8 antigen binding construct to the subject. In some embodiments, a method of determining the TIL status of a tumor in a human subject comprises administering a CD8 antigen binding construct to the subject, and thereafter monitoring a distribution of the CD8 antigen binding construct in the subject by PET. In some embodiments, a method of determining the TIL status of a tumor in a human subject comprises administering a CD8 antigen binding construct to the subject, and thereafter monitoring a distribution of the CD8 antigen binding construct in the subject by PET, the monitoring identifies a TIL positive tumor.

In some embodiments, a method of determining the TIL status of a tumor in a human subject comprises administering a CD8 antigen binding construct to the subject, thereafter monitoring a distribution of the CD8 antigen binding construct in the subject by PET, the monitoring identifies a TIL positive tumor, and the monitoring provides one or more of : a tumor that is infiltrated with 500 or less CD8 bearing cells per $mm^2$ within the subject, a tumor that is infiltrated with greater than or less than 500 CD8 bearing cells per $mm^2$ within the subject, a tumor that is infiltrated with greater than or less than 200 CD8 bearing cells per $mm^2$ within the subject, a tumor that is infiltrated with greater than or less than 100-500 CD8 bearing cells/$mm^2$, a tumor that is infiltrated with less than 1% CD8 bearing cells, 1-50% CD8 bearing cells or greater than 50% CD8 bearing cells, a tumor that comprises CD8 bearing cells that corresponds to IHC measurement of 2.5 to 25% of cells in a 4 micron tissue section, a tumor that comprises between 10,000 to 100,000 CD8 bearing cells is per $mm^3$ of the corresponding tumor, and a cell intensity of CD8 bearing cells, of the corresponding tumor that is low (cold) or high (hot).

In some embodiments of a method of determining the TIL status of a tumor in a human subject, tumor infiltration of CD8 bearing cells is alternatively confirmed by immunohistochemistry performed on biopsy of corresponding tumor.

In some embodiments of a method of determining the TIL status of a tumor in a human subject, tumor infiltration of CD8 bearing cell density is not confirmed by biopsy of corresponding tumor.

In some embodiments, a report on the status of tumor in a human subject having cancer is obtained. In some embodiments, the report comprises a section including results from the method of method of determining the TIL status of a tumor in a human subject. In some embodiments, the report comprises a section including results from the method of method of determining the TIL status of a tumor in a human subject, and optionally, a section on biopsy results of the corresponding tumor of the subject.

In some embodiments, the report comprises a section including results from the method of method of determining the TIL status of a tumor in a human subject, and optionally, a section on the status of the tumor according to RECIST

or PERCIST criteria.

In some embodiments, the report comprises a section including results from the method of method of determining the TIL status of a tumor in a human subject, optionally, a section on biopsy results of the corresponding tumor of the subject, and optionally, a section on the status of the tumor according to RECIST or PERCIST criteria.

**Brain**

In some embodiments, a method for identifying a solid tumor in the brain of a human subject comprises administering a CD8 antigen binding construct having a PET detectable label to the subject, scanning by PET at least the head of the subject, and providing a distribution of the PET detectable label in the brain. The identification of a CD8 concentrated region in the distribution indicates the presence of a solid tumor in the brain of the subject.

In some embodiments, a method for identifying a solid tumor in the brain of a human subject comprises administering a CD8 antigen binding construct having a PET detectable label to the subject, and scanning by PET at least the head of the subject.

In some embodiments, a method for identifying a solid tumor in the brain of a human subject comprises administering a CD8 antigen binding construct having a PET detectable label to the subject, and scanning by PET at least the head of the subject, and providing a distribution of the PET detectable label in the brain. In some embodiments, a method of determining an effectiveness of treatment for a tumor comprises identifying the tumor by a method for identifying a solid tumor in the brain of a human subject comprising administering a CD8 antigen binding construct having a PET detectable label to the subject, scanning by PET at least the head of the subject, and providing a distribution of the PET detectable label in the brain, the identification of a CD8 concentrated region in the distribution indicating the presence of a solid tumor in the brain of the subject, treating the subject using an immunotherapy, radiation and/or chemotherapy, repeating the method for identifying a solid tumor in the brain of a human subject comprising administering a CD8 antigen binding construct having a PET detectable label to the subject, scanning by PET at least the head of the subject, and providing a distribution of the PET detectable label in the brain. The identification of a CD8 concentrated region in the distribution indicates the presence of a solid tumor in the brain of the subject to determine if the tumor has changed in size or TIL status subsequent to the treatment in B, and determining if the treatment is effective.

In some embodiments, a method of determining an effectiveness of treatment for a tumor comprises identifying the tumor by a method for identifying a solid tumor in the brain of a human subject comprising administering a CD8 antigen binding construct having a PET detectable label to the subject.

In some embodiments, a method of determining an effectiveness of treatment for a tumor comprises identifying the tumor by a method for identifying a solid tumor in the brain of a human subject comprising administering a CD8 antigen binding construct having a PET detectable label to the subject, and scanning by PET at least the head of the subject.

In some embodiments, a method of determining an effectiveness of treatment for a tumor comprises identifying the tumor by a method for identifying a solid tumor in the brain of a human subject comprising administering a CD8 antigen binding construct having a PET detectable label to the subject, scanning by PET at least the head of the subject.

In some embodiments, a method of determining an effectiveness of treatment for a tumor comprises identifying the tumor by a method for identifying a solid tumor in the brain of a human subject comprising administering a CD8 antigen binding construct having a PET detectable label to the subject, scanning by PET at least the head of the subject, and providing a distribution of the PET detectable label in the brain, the identification of a CD8 concentrated region in the distribution indicating the presence of a solid tumor in the brain of the subject, treating the subject using an immunotherapy, radiation and/or chemotherapy.

In some embodiments, a method of determining an effectiveness of treatment for a tumor comprises identifying the tumor by a method for identifying a solid tumor in the brain of a human subject comprising administering a CD8 antigen binding construct having a PET detectable label to the subject, scanning by PET at least the head of the subject, and providing a distribution of the PET detectable label in the brain, the identification of a CD8 concentrated region in the distribution indicating the presence of a solid tumor in the brain of the subject, treating the subject using an immunotherapy, radiation and/or chemotherapy, repeating the method for identifying a solid tumor in the brain of a human subject comprising administering a CD8 antigen binding construct having a PET detectable label to the subject.

In some embodiments, a method of determining an effectiveness of treatment for a tumor comprises identifying the tumor by a method for identifying a solid tumor in the brain of a human subject comprising administering a CD8 antigen binding construct having a PET detectable label to the subject, scanning by PET at least the head of the subject, and providing a distribution of the PET detectable label in the brain, the identification of a CD8 concentrated region in the distribution indicating the presence of a solid tumor in the brain of the subject, treating the subject using an immunotherapy, radiation and/or chemotherapy, repeating the method for identifying a solid tumor in the brain of a human subject comprising administering a CD8 antigen binding construct having a PET detectable label to the subject, and scanning by PET at least the head of the subject.

In some embodiments, a method of determining an effectiveness of treatment for a tumor comprises identifying the tumor by a method for identifying a solid tumor in the brain of a human subject comprising administering a CD8 antigen binding construct having a PET detectable label to the subject, scanning by PET at least the head of the subject, and providing a distribution of the PET detectable label in the brain, the identification of a CD8 concentrated region in the distribution indicating the presence of a solid tumor in the brain of the subject, treating the subject using an immunotherapy, radiation and/or chemotherapy, repeating the method for identifying a solid tumor in the brain of a human subject comprising administering a CD8 antigen binding construct having a PET detectable label to the subject, and scanning by PET at least the head of the subject, and providing a distribution of the PET detectable label in the brain.

In some embodiments of a method of determining an effectiveness of treatment for a tumor, if the treatment is not effective, an alternative treatment is administered to the subject.

In some embodiments, a method of monitoring CD8 bearing cells in vivo comprises providing a CD8 minibody to a human subject, the CD8 minibody binding to a human CD8 as shown in FIG. 1C, and the minibody being labeled with a PET detectable marker, and monitoring distribution of the minibody in the subject. The monitoring distinguishes if a tissue volume in the subject is infiltrated with greater than or less than a selected amount of CD8 bearing cells. The monitoring is achieved via PET.

In some embodiments, a method of monitoring CD8 bearing cells in vivo comprises providing a CD8 minibody to a human subject.

In some embodiments, a method of monitoring CD8 bearing cells in vivo comprises providing a CD8 minibody to a human subject, the CD8 minibody binding to a human CD8 as shown in FIG. 1C.

In some embodiments, a method of monitoring CD8 bearing cells in vivo comprises providing a CD8 minibody to a human subject, the CD8 minibody binding to a human CD8 as shown in FIG. 1C, and the minibody being labeled with a PET detectable marker.

In some embodiments, a method of monitoring CD8 bearing cells in vivo comprises providing a CD8 minibody to a human subject, the CD8 minibody binding to a human CD8 as shown in FIG. 1C, and the minibody being labeled with a PET detectable marker, and monitoring distribution of the minibody in the subject.

In some embodiments, a method of visualizing CD8 bearing cells in a human subject comprises administering a CD8 minibody to a human subject, the minibody being labeled with a PET detectable marker, the minibody sequence being humanized and binds to human CD8, and monitoring a distribution of the minibody in the subject within 6-36 hours of administering the minibody to the subject. The method can distinguish whether a volume of the tissue is infiltrated with greater than or less than a selected amount of CD8 bearing cells within the subject. The monitoring is achieved via PET. In some embodiments, a method of visualizing CD8 bearing cells in a human subject comprises administering a CD8 minibody to a human subject.In some embodiments, a method of visualizing CD8 bearing cells in a human subject comprises administering a CD8 minibody to a human subject, the minibody being labeled with a PET detectable marker.

In some embodiments, a method of visualizing CD8 bearing cells in a human subject comprises administering a CD8 minibody to a human subject, the minibody being labeled with a PET detectable marker, and the minibody sequence being humanized and binds to human CD8.

In some embodiments, a method of visualizing CD8 bearing cells in a human subject comprises administering a CD8 minibody to a human subject, the minibody being labeled with a PET detectable marker, and the minibody sequence being humanized and binds to human CD8, and monitoring a distribution of the minibody in the subject within 6-36 hours of administering the minibody to the subject.

In some embodiments of a method of visualizing CD8 bearing cells in a human subject, the selected amount of CD8 bearing cells corresponds to IHC measurement of 100-500 cells/$mm^2$. In some embodiments of a method of visualizing CD8 bearing cells in a human subject, the selected amount of CD8 bearing cells corresponds to IHC measurement of 2.5 to 25% of cells in a 4 micron tissue section. In some embodiments of a method of visualizing CD8 bearing cells in a human subject, the selected amount of CD8 bearing cells is between 10,000 to 100,000 cells per $mm^3$.In some embodiments of a method of visualizing CD8 bearing cells in a human subject, the selected amount of CD8 bearing cells is between 1,000 to 1000,000 cells per mm3.

In some embodiments of a method of visualizing CD8 bearing cells in a human subject, the tissue is 4 microns thick. In some embodiments of a method of visualizing CD8 bearing cells in a human subject, an uptake time is consistent between all CD8 antigen binding constructs.

In any of the embodiments provided herein, the antigen binding construct can be an artificial construct that does not occur in nature.

## METHOD OF TREATMENT

In some embodiments, any of the methods of observation or diagnosis provided herein can be combined or supplemented with a method of treating the subject.

In some embodiments, a method of treating a patient is provided. The method comprises administering to a

human patient diagnosed with a cancer a dose of an antigen-binding construct that binds to human CD8. In some embodiments, the dose comprises a $^{89}Zr$-labeled antigen-binding construct providing a radiation activity of about 0.7-3 mCi, and about 10 mg or less of the antigen-binding construct. The method further comprises detecting the $^{89}Zr$-labeled antigen-binding construct in the patient at a first time point after administering the dose, to generate a first patient image corresponding to the first time point, wherein the first time point is about 6 hours to 36 hours after administering. The method further comprises determining a first abundance and/or distribution of CD8 cells in one or more tissues (and/or neoplasia) in the patient based on the first patient image and administering to the patient a first treatment for the cancer (designed to destroy the cancer or change the phenotype) based on the first abundance and/or distribution of CD8 cells in the one or more tissues.

In some embodiments, the method further comprises, after administering the first treatment, determining a second abundance and/or distribution of CD8 cells in the one or more tissues (and/or neoplasia) in the patient based on a second patient image corresponding to a second time point and comprising a second site-specific information for the target of the antigen-binding construct. The method can further comprise administering to the patient a second treatment for the cancer based on a comparison of the first and second patient images.

In some embodiments, the method further comprises optimizing a therapeutic dose of the second treatment based on the comparison of the first and second patient images.

In some embodiments, the method of treating a tumor is provide that comprises administering a minibody to a subject such that the minibody binds to a tumor within the subject to form a labeled tumor. The minibody binds to human CD8, and the minibody is linked to a detectable label. If the minibody binds in a biased manner to a surface of the tumor in forming the labeled tumor, then administering an immune checkpoint inhibitor and if the minibody binds throughout the tumor in forming the labeled tumor, then not administering an immune checkpoint inhibitor. If the minibody binds throughout all tumors in forming the labeled tumor, then not administering an immune checkpoint inhibitor.

In some embodiments, a method of treating a tumor is provided. The method comprises administering a minibody to a subject such that the minibody binds to a tumor within the subject to form a labeled tumor, wherein the minibody binds to human CD8, and wherein the minibody is linked to a detectable label. If the minibody binds in a biased manner to a surface of any tumor in forming the labeled tumor, then administering a treatment selected from the group consisting of an immune checkpoint inhibitor and if the minibody binds throughout all tumors in forming the labeled tumor, then not administering an immune checkpoint inhibitor.

In some embodiments, a method of treating a subject is provided. The method comprises administering to a patient that has a neoplasia an antigen-binding construct that binds to human CD8, monitoring a distribution of the antigen-binding construct to determine the tumor-infiltrating lymphocyte ("TIL") status within the neoplasia, and administering an immunotherapy ("IOT") to the patient if the TIL status of at least one neoplasia is negative. This allows one to then convert the neoplasia to TIL positive.

In some embodiments, a method of treating a subject is provided, the method comprises administering to a patient that has a neoplasia an antigen-binding construct that binds to human CD8, monitoring a distribution of the antigen-binding construct to determine the tumor-infiltrating lymphocyte ("TIL") status within the neoplasia, and administering an alternative immunotherapy ("IOT") to the patient if the TIL status of the neoplasia is not positive (e.g., it is TIL negative, such as a desert or immune excluded). Optionally continuing this variation until the status becomes TIL positive.

In some embodiments, a method of treating a human subject having cancer comprises, in response to providing a first image of a tumor in the subject using a CD8 antigen binding construct, administering a therapy including a candidate therapeutic to the subject, after administering the therapy including the candidate therapeutic, providing a second image of the tumor in the subject using the CD8 antigen binding construct, comparing the first and second images to determine if (a) the tumor demonstrates increased CD8 infiltration or (b) the tumor demonstrates the same or decreased CD8 infiltration, if a), then instructing the subject to continue the therapy.

In some embodiments, a method of treating a human subject having cancer comprises, in response to providing a first image of a tumor in the subject using a CD8 antigen binding construct, administering a therapy including a candidate therapeutic to the subject.

In some embodiments, a method of treating a human subject having cancer comprises, in response to providing a first image of a tumor in the subject using a CD8 antigen binding construct, administering a therapy including a candidate therapeutic to the subject, and after administering the therapy including the candidate therapeutic, providing a second image of the tumor in the subject using the CD8 antigen binding construct.

In some embodiments, a method of treating a human subject having cancer comprises, in response to providing a first image of a tumor in the subject using a CD8 antigen binding construct, administering a therapy including a candidate therapeutic to the subject, and after administering the therapy including the candidate therapeutic, and after providing a second image of the tumor in the subject using the CD8 antigen binding construct, comparing the first and second images.

In some embodiments, a method of treating a human subject having cancer comprises, in response to providing a first image of a tumor in the subject using a CD8 antigen binding construct, administering a therapy including a candidate

therapeutic to the subject, and after administering the therapy including the candidate therapeutic, and after providing a second image of the tumor in the subject using the CD8 antigen binding construct, comparing the first and second images to determine if the tumor demonstrates increased CD8 infiltration.

In some embodiments, a method of treating a human subject having cancer comprises, in response to providing a first image of a tumor in the subject using a CD8 antigen binding construct, administering a therapy including a candidate therapeutic to the subject, after administering the therapy including the candidate therapeutic, and after providing a second image of the tumor in the subject using the CD8 antigen binding construct, comparing the first and second images to determine if the tumor demonstrates the same or decreased CD8 infiltration.

In some embodiments, a method of treating a human subject having cancer comprises, in response to providing a first image of a tumor in the subject using a CD8 antigen binding construct, administering a therapy including a candidate therapeutic to the subject, after administering the therapy including the candidate therapeutic, after providing a second image of the tumor in the subject using the CD8 antigen binding construct, comparing the first and second images to determine if the tumor demonstrates increased CD8 infiltration, and if the tumor demonstrates increased CD8 infiltration, then instructing the subject to continue the therapy.

## METHOD OF TREATMENT USING AN IOT

In some embodiments, methods to treat and determine which candidate therapeuitic to give are provided. In some embodiments, a method of treating a human subject having cancer comprises providing a first image of a CD8 cells within a tumor in a subject using a CD8 antigen binding construct, administering a therapy including a candidate therapeutic to the subject, after administering the therapy including the candidate therapeutic, providing a second image of the CD8 cells within the tumor in the subject using the CD8 antigen binding construct, comparing the first and second images to determine if: (a) the tumor demonstrates increased CD8 infiltration or (b) the tumor demonstrates the same or decreased CD8 infiltration, wherein, if (a), then instructing the subject to continue the therapy.

In some embodiments, a method of treating a human subject having cancer comprises providing a first image of a CD8 cells within a tumor in a subject using a CD8 antigen binding construct, and administering a therapy including a candidate therapeutic to the subject.

In some embodiments, rather than monitoring a tumor to be treated (a first tumor in this context), one can monitor a different tumor (a second tumor in this context) and base the treatment for the first tumor upon the condition or responsiveness of the second tumor. This can be done of any of the embodiments provided herein.

In some embodiments, a method of treating a human subject having cancer comprises providing a first image of a CD8 cells within a tumor in a subject using a CD8 antigen binding construct, administering a therapy including a candidate therapeutic to the subject, and after administering the therapy including the candidate therapeutic, providing a second image of the CD8 cells within the tumor in the subject using the CD8 antigen binding construct.

In some embodiments, a method of treating a human subject having cancer comprises providing a first image of a CD8 cells within a tumor in a subject using a CD8 antigen binding construct, administering a therapy including a candidate therapeutic to the subject, and after administering the therapy including the candidate therapeutic, providing a second image of the CD8 cells within the tumor in the subject using the CD8 antigen binding construct, and comparing the first and second images.

In some embodiments, a method of treating a human subject having cancer comprises providing a first image of a CD8 cells within a tumor in a subject using a CD8 antigen binding construct, administering a therapy including a candidate therapeutic to the subject, after administering the therapy including the candidate therapeutic, providing a second image of the CD8 cells within the tumor in the subject using the CD8 antigen binding construct, and comparing the first and second images to determine if the tumor demonstrates increased CD8 infiltration .

In some embodiments, a method of treating a human subject having cancer comprises providing a first image of a CD8 cells within a tumor in a subject using a CD8 antigen binding construct, administering a therapy including a candidate therapeutic to the subject, after administering the therapy including the candidate therapeutic, providing a second image of the CD8 cells within the tumor in the subject using the CD8 antigen binding construct, and comparing the first and second imagesto determine if the tumor demonstrates the same or decreased CD8 infiltration.

In some embodiments, a method of treating a human subject having cancer comprises providing a first image of a CD8 cells within a tumor in a subject using a CD8 antigen binding construct, administering a therapy including a candidate therapeutic to the subject, and after administering the therapy including the candidate therapeutic, providing a second image of the CD8 cells within the tumor in the subject using the CD8 antigen binding construct, comparing the first and second imagesto determine if the tumor demonstrates the same or decreased CD8 infiltration, wherein, if if the tumor demonstrates increased CD8 infiltration , then instructing the subject to continue the therapy.

In some embodiments of a method of treating a human subject having cancer, if the tumor demonstrates the same or decreased CD8 infiltration, then administering another round of therapy, providing a third image of the CD8 cells within the tumor; comparing the first and third images to determine if the tumor demonstrates the same or decreased

CD8 infiltration; and discontinuing the therapy if the tumor demonstrates the same or decreased CD8 infiltration. In some embodiments of a method of treating a human subject having cancer, a therapy comprises radiotherapy. In some embodiments of a method of treating a human subject having cancer, a first image is an image of the CD8 cells within the tumor in the subject. In some embodiments of a method of treating a human subject having cancer, a first image is an image of CD8 cells within the tumor that is not from the subject. In some embodiments of a method of treating a human subject having cancer, a first image is from a database and from an individual or is a composite of data from a collection of individuals. In some embodiments of a method of treating a human subject having cancer, if the tumor demonstrates the same or decreased CD8 infiltration, then administering a therapy comprising an alternative candidate therapeutic.

In some embodiments of a method of treating a human subject having cancer, if the tumor demonstrates the same or decreased CD8 infiltration, then administering a therapy comprising an alternative candidate therapeutic, and after administering the therapy, providing a third image of the CD8 cells within the tumor in the subject using the CD8 antigen binding construct.

In some embodiments of a method of treating a human subject having cancer, if the tumor demonstrates the same or decreased CD8 infiltration, then administering a therapy comprising an alternative candidate therapeutic, after administering the therapy, providing a third image of the CD8 cells within the tumor in the subject using the CD8 antigen binding construct; and comparing the third and second or third and first images.

In some embodiments of a method of treating a human subject having cancer, if the tumor demonstrates the same or decreased CD8 infiltration, then administering a therapy comprising an alternative candidate therapeutic, after administering the therapy, providing a third image of the CD8 cells within the tumor in the subject using the CD8 antigen binding construct; and comparing the third and second or third and first images to determine if the tumor demonstrates increased CD8 infiltration.

In some embodiments of a method of treating a human subject having cancer, if the tumor demonstrates the same or decreased CD8 infiltration, then administering a therapy comprising an alternative candidate therapeutic, and after administering the therapy, providing a third image of the CD8 cells within the tumor in the subject using the CD8 antigen binding construct; and comparing the third and second or third and first images to determine if the tumor demonstrates the same or decreased CD8 infiltration. In some embodiments of a method of treating a human subject having cancer, if the tumor demonstrates the same or decreased CD8 infiltration, then administering a therapy comprising an alternative candidate therapeutic, after administering the therapy, providing a third image of the CD8 cells within the tumor in the subject using the CD8 antigen binding construct; comparing the third and second or third and first images to determine if the tumor demonstrates increased CD8 infiltration, and if the tumor demonstrates increased CD8 infiltration, then instructing the subject to continue the therapy comprising the alternative candidate therapeutic.

In some embodiments, a method of treating a human subject having cancer comprises administering a CD8 antigen binding construct to the subject, monitoring a distribution of the CD8 antigen binding construct within the subject; and discontinuing an immunotherapy treatment if at least one tumor within the subject is TIL negative per the distribution of the CD8 antigen binding construct.

In some embodiments, a method of treating a human subject having cancer comprises administering a CD8 antigen binding construct to the subject. In some embodiments, a method of treating a human subject having cancer comprises administering a CD8 antigen binding construct to the subject, and monitoring a distribution of the CD8 antigen binding construct within the subject. In some embodiments, a method of treating a human subject having cancer comprises administering a CD8 antigen binding construct to the subject, monitoring a distribution of the CD8 antigen binding construct within the subject, and discontinuing an immunotherapy treatment. In some embodiments, a method of treating a human subject having cancer comprises administering a CD8 antigen binding construct to the subject, monitoring a distribution of the CD8 antigen binding construct within the subject, and discontinuing an immunotherapy treatment if at least one tumor within the subject is TIL negative per the distribution of the CD8 antigen binding construct.

In some embodiments, a method of treating a human subject having cancer comprises administering a CD8 antigen binding construct to the subject, monitoring a distribution of the CD8 antigen binding construct within the subject, and continuing an immunotherapy treatment if at least one tumor within the subject is TIL positive per the distribution of the CD8 antigen binding construct.

In some embodiments, a method of treating a human subject having cancer comprises administering a CD8 antigen binding construct to the subject. In some embodiments, a method of treating a human subject having cancer comprises administering a CD8 antigen binding construct to the subject, and monitoring a distribution of the CD8 antigen binding construct within the subject. In some embodiments, a method of treating a human subject having cancer comprises administering a CD8 antigen binding construct to the subject, monitoring a distribution of the CD8 antigen binding construct within the subject, and continuing an immunotherapy treatment. In some embodiments, a method of treating a human subject having cancer comprises administering a CD8 antigen binding construct to the subject, monitoring a distribution of the CD8 antigen binding construct within the subject, and continuing an immunotherapy treatment if at least one tumor within the subject is TIL positive per the distribution of the CD8 antigen binding construct.

In some embodiments, the methods allow one to distinguish naive patients previously untreated with IOT from patients previously treated with IOT. In some embodiments, the methods are used on patients that have been previously treated with an IOT.

In some embodiments, a method of treating a human subject having cancer comprises administering an immunotherapy to the subject, then administering a CD8 antigen binding construct to the subject, monitoring a distribution of the CD8 antigen binding construct within the subject, discontinuing the immunotherapy if all of the tumors within the subject is TIL negative per the distribution of the CD8 antigen binding construct.

In some embodiments, a method of treating a human subject having cancer comprises administering an immunotherapy to the subject.

In some embodiments, a method of treating a human subject having cancer comprises administering an immunotherapy to the subject, and then administering a CD8 antigen binding construct to the subject.

In some embodiments, a method of treating a human subject having cancer comprises administering an immunotherapy to the subject, and then administering a CD8 antigen binding construct to the subject, and monitoring a distribution of the CD8 antigen binding construct within the subject.

In some embodiments, a method of treating a human subject having cancer comprises administering an immunotherapy to the subject, then administering a CD8 antigen binding construct to the subject, and monitoring a distribution of the CD8 antigen binding construct within the subject, and discontinuing the immunotherapy if all of the tumors within the subject is TIL negative per the distribution of the CD8 antigen binding construct.

In some embodiments, a method of treating a human subject having cancer comprises in a subject previously untreated with an immunotherapy, administering a CD8 antigen binding construct to the subject, monitoring a distribution of the CD8 antigen binding construct within the subject, administering an immunotherapy if at least one tumor within the subject is TIL negative per the distribution of the CD8 antigen binding construct.

In some embodiments, a method of treating a human subject having cancer comprises in a subject previously untreated with an immunotherapy, administering a CD8 antigen binding construct to the subject.

In some embodiments, a method of treating a human subject having cancer comprises in a subject previously untreated with an immunotherapy, administering a CD8 antigen binding construct to the subject, and monitoring a distribution of the CD8 antigen binding construct within the subject.

In some embodiments, a method of treating a human subject having cancer comprises in a subject previously untreated with an immunotherapy, administering a CD8 antigen binding construct to the subject, monitoring a distribution of the CD8 antigen binding construct within the subject, and administering an immunotherapy if at least one tumor within the subject is TIL negative per the distribution of the CD8 antigen binding construct.

In some embodiments, a method of treating a human subject having cancer comprises in a subject previously untreated with an immunotherapy, administering a CD8 antigen binding construct to the subject, monitoring a distribution of the CD8 antigen binding construct within the subject, advising against administering of an immunotherapy if all tumors within the subject are TIL positive per the distribution of the CD8 antigen binding construct.

In some embodiments, a method of treating a human subject having cancer comprises in a subject previously untreated with an immunotherapy, administering a CD8 antigen binding construct to the subject.

In some embodiments, a method of treating a human subject having cancer comprises in a subject previously untreated with an immunotherapy, administering a CD8 antigen binding construct to the subject, and monitoring a distribution of the CD8 antigen binding construct within the subject.

In some embodiments, a method of treating a human subject having cancer comprises in a subject previously untreated with an immunotherapy, administering a CD8 antigen binding construct to the subject, monitoring a distribution of the CD8 antigen binding construct within the subject, and advising against administering of an immunotherapy if all tumors within the subject are TIL positive per the distribution of the CD8 antigen binding construct.

In some embodiments, a method of treating a human subject having cancer comprises in a subject previously treated with an immunotherapy, administering a CD8 antigen binding construct to the subject, monitoring a distribution of the CD8 antigen binding construct within the subject, and administering an alternative immunotherapy if at least one tumor within the subject is TIL negative per the distribution of the CD8 antigen binding construct. In some embodiments, a method of treating a human subject having cancercomprises in a subject previously treated with an immunotherapy, administering a CD8 antigen binding construct to the subject.

In some embodiments, a method of treating a human subject having cancercomprises in a subject previously treated with an immunotherapy, administering a CD8 antigen binding construct to the subject, and monitoring a distribution of the CD8 antigen binding construct within the subject.

In some embodiments, a method of treating a human subject having cancer comprises in a subject previously treated with an immunotherapy, administering a CD8 antigen binding construct to the subject, monitoring a distribution of the CD8 antigen binding construct within the subject, and administering an alternative immunotherapy if at least one tumor within the subject is TIL negative per the distribution of the CD8 antigen binding construct.

In some embodiments, a method of treating a human subject having cancer comprises in a subject previously

treated with an immunotherapy, administering a CD8 antigen binding construct to the subject, monitoring a distribution of the CD8 antigen binding construct within the subject, administering the immunotherapy if all tumors within the subject are TIL positive per the distribution of the CD8 antigen binding construct.

In some embodiments, a method of treating a human subject having cancer comprises in a subject previously treated with an immunotherapy, administering a CD8 antigen binding construct to the subject.

In some embodiments, a method of treating a human subject having cancer comprises in a subject previously treated with an immunotherapy, administering a CD8 antigen binding construct to the subject, and monitoring a distribution of the CD8 antigen binding construct within the subject.

In some embodiments, a method of treating a human subject having cancer comprises in a subject previously treated with an immunotherapy, administering a CD8 antigen binding construct to the subject, monitoring a distribution of the CD8 antigen binding construct within the subject,administering the immunotherapy if all tumors within the subject are TIL positive per the distribution of the CD8 antigen binding construct.

In some embodiments, a method of treating a human subject having cancer comprises measurement of the level of an additional biomarker in a patient sample to inform the selection of immunotherapy. In some embodiments, a method of treating a human subject having cancer comprises measurement of the level of an additional biomarker in a patient sample to inform the selection of immunotherapy, the additional biomarker being selected from among PD-L1 status, T-cell receptor clonality, mutational burden, neoantigen burden, immune gene signatures, and multiplex immunohistochemistry.

## ID TUMOR STATE AND ADMINISTER COMPOUND

In some embodiments, a method of treating a tumor in a human subject comprises administering a CD8 antigen binding construct to the subject such that the CD8 antigen binding construct binds to CD8 cells within a tumor within the subject to form a labeled tumor, wherein the CD8 antigen binding construct binds to human CD8, and wherein the CD8 antigen binding construct is linked to a detectable label, detecting a distribution of the CD8 antigen binding construct in or around the tumor, if the CD8 antigen binding construct binds in a biased manner at a surface of any tumor, then administering a treatment selected from the group consisting of an Immunotherapy, and if the CD8 antigen binding construct binds in a non-biased manner and throughout all tumors, then not administering an immune checkpoint inhibitor, or if the CD8 antigen binding construct binds in a non-biased manner but is absent from the interior of the tumor, then administering an immunotherapy.

In some embodiments, a method of treating a tumor in a human subject comprises administering a CD8 antigen binding construct to the subject.

In some embodiments, a method of treating a tumor in a human subject comprises administering a CD8 antigen binding construct to the subject such that the CD8 antigen binding construct binds to CD8 cells within a tumor within the subject to form a labeled tumor.

In some embodiments, a method of treating a tumor in a human subject comprises administering a CD8 antigen binding construct to the subject such that the CD8 antigen binding construct binds to CD8 cells within a tumor within the subject to form a labeled tumor, wherein the CD8 antigen binding construct binds to human CD8.

In some embodiments, a method of treating a tumor in a human subject comprises administering a CD8 antigen binding construct to the subject such that the CD8 antigen binding construct binds to CD8 cells within a tumor within the subject to form a labeled tumor, wherein the CD8 antigen binding construct binds to human CD8, and wherein the CD8 antigen binding construct is linked to a detectable label.

In some embodiments, a method of treating a tumor in a human subject comprises administering a CD8 antigen binding construct to the subject such that the CD8 antigen binding construct binds to CD8 cells within a tumor within the subject to form a labeled tumor, wherein the CD8 antigen binding construct binds to human CD8, and wherein the CD8 antigen binding construct is linked to a detectable label, and detecting a distribution of the CD8 antigen binding construct in or around the tumor.

In some embodiments, a method of treating a tumor in a human subject comprises administering a CD8 antigen binding construct to the subject such that the CD8 antigen binding construct binds to CD8 cells within a tumor within the subject to form a labeled tumor, wherein the CD8 antigen binding construct binds to human CD8, and wherein the CD8 antigen binding construct is linked to a detectable label, and detecting a distribution of the CD8 antigen binding construct in or around the tumor, and if the CD8 antigen binding construct binds in a biased manner at a surface of any tumor, then administering a treatment selected from the group consisting of an immunotherapy.

In some embodiments, a method of treating a tumor in a human subject comprises administering a CD8 antigen binding construct to the subject such that the CD8 antigen binding construct binds to CD8 cells within a tumor within the subject to form a labeled tumor, wherein the CD8 antigen binding construct binds to human CD8, and wherein the CD8 antigen binding construct is linked to a detectable label, and detecting a distribution of the CD8 antigen binding construct in or around the tumor, and if the CD8 antigen binding construct binds in a non-biased manner and throughout all tumors,

then not administering an immune checkpoint inhibitor.

In some embodiments, a method of treating a tumor in a human subject comprises administering a CD8 antigen binding construct to the subject such that the CD8 antigen binding construct binds to CD8 cells within a tumor within the subject to form a labeled tumor, wherein the CD8 antigen binding construct binds to human CD8, and wherein the CD8 antigen binding construct is linked to a detectable label, and detecting a distribution of the CD8 antigen binding construct in or around the tumor, and if the CD8 antigen binding construct binds in a non-biased manner but is absent from the interior of the tumor, then administering an immunotherapy.

In some embodiments, a method of treating a tumor in a human subject comprises administering to a human subject a CD8 antigen binding construct, monitoring a distribution of the CD8 antigen binding construct to determine a first tumor-infiltrating lymphocyte ("TIL") status within the tumor, and treating the patient based upon at least the first TIL status of the neoplasia.

In some embodiments, a method of treating a tumor in a human subject comprises administering to a human subject a CD8 antigen binding construct. In some embodiments, a method of treating a tumor in a human subject comprises administering to a human subject a CD8 antigen binding construct, and monitoring a distribution of the CD8 antigen binding construct to determine a first tumor-infiltrating lymphocyte ("TIL") status within the tumor.

In some embodiments, a method of treating a tumor in a human subject comprises administering to a human subject a CD8 antigen binding construct, and monitoring a distribution of the CD8 antigen binding construct to determine a first tumor-infiltrating lymphocyte ("TIL") status within the tumor, and treating the patient based upon at least the first TIL status of the neoplasia.

In some embodiments of a method of treating a tumor in a human subject, a first TIL status is when the CD8 antigen binding construct binds throughout a volume of all identified tumors. In some embodiments of a method of treating a tumor in a human subject, a second TIL status is when the CD8 antigen binding construct binds in a biased manner to any tumor margin.

In some embodiments of a method of treating a tumor in a human subject, an immunotherapy is applied to the subject if the subject has the second TIL status. In some embodiments of a method of treating a tumor in a human subject, an immunotherapy is not administered to the subject if the subject has the first TIL status. In some embodiments of a method of treating a tumor in a human subject, at least the first TIL status is used for at least one of: classifying or selecting the human subject for a clinical trial; recommending or determining eligibility of the human subject for a therapeutic treatment; predicting response to therapy of the human subject; and/or predicting response to therapy selected from among the group consisting of radiotherapy, chemotherapy, biological therapy, and immunotherapy. In some embodiments of a method of treating a tumor in a human subject, monitoring CD8 antigen binding construct is achieved as a function of a distribution of the detectable marker at a tumor surface which demonstrates an increase in binding to CD8 positive cells at the tumor surface as compared to an internal area or volume of the tumor. In some embodiments of a method of treating a tumor in a human subject, monitoring CD8 antigen binding construct is achieved as a function of a distribution of the detectable marker at an internal area or volume of the tumor.

In some embodiments of a method of treating a tumor in a human subject, if monitoring CD8 antigen binding construct is achieved as a function of a distribution of the detectable marker at a tumor surface which demonstrates an increase in binding to CD8 positive cells at the tumor surface as compared to an internal area or volume of the tumorthen the subject is provided with an immunotherapy.

In some embodiments of a method of treating a tumor in a human subject, if monitoring CD8 antigen binding construct is achieved as a function of a distribution of the detectable marker at a tumor surface which demonstrates an increase in binding to CD8 positive cells at the tumor surface as compared to an internal area or volume of the tumor, then the subject is provided with an immunotherapy and if a distribution of the detectable marker at a tumor surface which demonstrates an increase in binding to CD8 positive cells at the tumor surface as compared to an internal area or volume of the tumor persists after treatment with such immunotherapy, then providing the subject with an alternate immunotherapy.

In some embodiments, a method of treating a human subject comprises administering to a patient that has a neoplasia an antigen-binding construct that binds to human CD8, monitoring a distribution of the antigen-binding construct to determine the tumor-infiltrating lymphocyte ("TIL") status within the neoplasia, and administering an immunotherapy ("IOT") to the patient if the TIL status of at least one neoplasia is negative so as to convert the TIL status from negative to positive.

In some embodiments, a method of treating a human subject comprises administering to a patient that has a neoplasia an antigen-binding construct. In some embodiments, a method of treating a human subject comprises administering to a patient that has a neoplasia an antigen-binding construct that binds to human CD8. In some embodiments, a method of treating a human subject comprises administering to a patient that has a neoplasia an antigen-binding construct that binds to human CD8, and monitoring a distribution of the antigen-binding construct to determine the tumor-infiltrating lymphocyte ("TIL") status within the neoplasia.

In some embodiments, a method of treating a human subject comprises administering to a patient that has a

neoplasia an antigen-binding construct that binds to human CD8, and monitoring a distribution of the antigen-binding construct to determine the tumor-infiltrating lymphocyte ("TIL") status within the neoplasia, and administering an immunotherapy ("IOT") to the patient if the TIL status of at least one neoplasia is negative.

In some embodiments, a method of treating a human subject comprises administering to a patient that has a neoplasia an antigen-binding construct that binds to human CD8, and monitoring a distribution of the antigen-binding construct to determine the tumor-infiltrating lymphocyte ("TIL") status within the neoplasia, and administering an immunotherapy ("IOT") to the patient if the TIL status of at least one neoplasia is negative so as to convert the TIL status from negative to positive.

In some embodiments, a method of treating a human subject comprises administering to a patient that has a neoplasia an antigen-binding construct that binds to human CD8, monitoring a distribution of the antigen-binding construct to determine the tumor-infiltrating lymphocyte ("TIL") status within the neoplasia, and administering an alternative immunotherapy ("IOT") to the patient if the TIL status of the neoplasia is not positive, until the TIL status of the neoplasia becomes positive. In some embodiments, a method of treating a human subject comprises administering to a patient that has a neoplasia an antigen-binding construct that binds to human CD8.

In some embodiments, a method of treating a human subject comprises administering to a patient that has a neoplasia an antigen-binding construct that binds to human CD8, and monitoring a distribution of the antigen-binding construct to determine the tumor-infiltrating lymphocyte ("TIL") status within the neoplasia.

In some embodiments, a method of treating a human subject comprises administering to a patient that has a neoplasia an antigen-binding construct that binds to human CD8, and monitoring a distribution of the antigen-binding construct to determine the tumor-infiltrating lymphocyte ("TIL") status within the neoplasia, and administering an alternative immunotherapy ("IOT") to the patient if the TIL status of the neoplasia is not positive.

In some embodiments, a method of treating a human subject comprises administering to a patient that has a neoplasia an antigen-binding construct that binds to human CD8, and monitoring a distribution of the antigen-binding construct to determine the tumor-infiltrating lymphocyte ("TIL") status within the neoplasia, and administering an alternative immunotherapy ("IOT") to the patient if the TIL status of the neoplasia is not positive, until the TIL status of the neoplasia becomes positive.

In some embodiments of a method of treating a human subject, monitoring is conducted within 8 hours of administering. In some embodiments of a method of treating a human subject, the monitoring or a second monitoring is done before 36 hours from administering.

In some embodiments, a method of treating a tumor in a human subjectcomprises, if a human subject is receiving a first treatment, determining via a CD8 binding molecule if a tumor in the subject has CD8 cells, and if so, then continuing the therapy, and if cold then changing the therapy to a second treatment, and if the human subject is not receiving a first treatment, determining via a CD8 binding molecule if a tumor in the subject has CD8 cells, and if so, then administering an IOT to the subject.

In some embodiments, a method of treating a tumor in a human subject comprises, if a human subject is receiving a first treatment, determining via a CD8 binding molecule if a tumor in the subject has CD8 cells.

In some embodiments, a method of treating a tumor in a human subject comprises, if a human subject is receiving a first treatment, determining via a CD8 binding molecule if a tumor in the subject has CD8 cells, and if so, then continuing the therapy.

In some embodiments, a method of treating a tumor in a human subject comprises, if a human subject is receiving a first treatment, determining via a CD8 binding molecule if a tumor in the subject has CD8 cells, and if cold then changing the therapy to a second treatment.

In some embodiments, a method of treating a tumor in a human subject comprises, if the human subject is not receiving a first treatment, determining via a CD8 binding molecule if a tumor in the subject has CD8 cells.

In some embodiments, a method of treating a tumor in a human subject comprises, if the human subject is not receiving a first treatment, determining via a CD8 binding molecule if a tumor in the subject has CD8 cells, and if so, then administering an IOT to the subject.

In some embodiments, a method of selecting a treatment for a patient comprises administering a CD8 binding construct to a human patient, determining a TIL status of a tumor in the patient, wherein if the tumor is cold, then administering an IOT that changes the TIL status of the tumor. In some embodiments, a method of selecting a treatment for a patient comprises administering a CD8 binding construct to a human patient.

In some embodiments, a method of selecting a treatment for a patient comprises administering a CD8 binding construct to a human patient, and determining a TIL status of a tumor in the patient.

In some embodiments, a method of selecting a treatment for a patient comprises administering a CD8 binding construct to a human patient, and determining a TIL status of a tumor in the patient, and if the tumor is cold, then administering an IOT.In some embodiments, a method of selecting a treatment for a patient comprises administering a CD8 binding construct to a human patient, and determining a TIL status of a tumor in the patient, and if the tumor is cold, then administering an IOT that changes the TIL status of the tumor.

In some embodiments, a method of changing a treatment for a subject comprises providing a human subject on a first therapy, administering a CD8 binding construct to the human subject, determining if the subject has a tumor that is TIL positive under the first therapy, and if the subject has a tumor that is TIL positive under the first therapy, continuing the therapy, and if the subject has a tumor that is TIL negative under the first therapy stopping the first therapy.

In some embodiments, a method of changing a treatment for a subject comprises identifying a human subject on a first therapy. In some embodiments, a method of changing a treatment for a subject comprises identifying a human subject on a first therapy, and administering a CD8 binding construct to the human subject. In some embodiments, a method of changing a treatment for a subject comprises identifying a human subject on a first therapy, and administering a CD8 binding construct to the human subject, and determining if the subject has a tumor that is TIL positive under the first therapy. In some embodiments, a method of changing a treatment for a subject comprises identifying a human subject on a first therapy, and administering a CD8 binding construct to the human subject, and determining if the subject has a tumor that is TIL positive under the first therapy, and if the subject has a tumor that is TIL positive under the first therapy, continuing the therapy.

In some embodiments, a method of changing a treatment for a subject comprises identifying a human subject on a first therapy, and administering a CD8 binding construct to the human subject, and determining if the subject has a tumor that is TIL positive under the first therapy, and if the subject has a tumor that is TIL negative under the first therapy stopping the first therapy.

As used herein, "selecting a treatment" and "changing a treatment" based on TIL status may mean one or more of selecting, stopping, adding, combining or modifying the administration of one or more therapeutic agent or one or more therapeutic course of treatment. The agent or treatment may be an immunotherapy, or it may be a chemotherapy, radiation or surgery. Typically, where the tumor has been identified as a member of a class of known tumor, the treatment is selected from among those known for the standard of care of such tumors. Alternatively it may be an experimental treatment used at the discretion of the doctor and patient. As an example of the foregoing, in the context of immunotherapy, first line of therapy is usually monotherapy with a PD1 or CTLA4 immune checkpoint inhibitor. Second line of therapy is often a combination of checkpoint inhibitors. Recommended therapeutic approaches change frequently in this developing field. A unifying feature of the instant invention is that such therapeutic approaches can now be selected (or by contrast ignored) based on rapid, non-invasive, determination of the CD8 TIL status of one or more tumors by PET-imaging of an administered CD8 antigen binding construct.

A summary of treatments that may be advised depending on CD8 TIL status, using the Hot/Altered-Excluded/Altered-immunosupressed/Cold scale is set out in Figure 3 of Galon and Bruni (2019) Nature Reviews Drug Discovery v. 18, pages197-218, specifically incorporated herein by reference. The invention now provides a major improvement on the method to determine CD8 TIL status, and thereby to make the appropriate selection of therapy.

An example of a therapeutic approach that is affected by the invention can be seen in the common cancers that respond to immunotherapy: melanoma and lung cancer. These and other cancers can metastasize to the brain. The current standard of care for such conditions include:

In the case of Malignant Melanoma: (Incidence rate of 2.8-3.1 per 100,000 persons) First-line treatement: surgery plus checkpoint single or combinantion inhibitors (PD-1 or PD-1 plus CTLA-4); Second-line treatment: depending on first treatment: repeat of first line therapy, chemotherapy, combination immune checkpoint inhibitors, or targeted therapy if tumor has acquired certain mutations.

In the case of Non-small cell lung cancer (NSCL): (Incidence rate of 52 to 75 per 100,000 persons) First-line treatement: surgery (or radiotherapy) plus chemotherapy; Second-line treatment: different chemotherapeutic agents (ocetaxel, gemcitabine, pemetrexed, and erlotinib), or targeted therapy if certain oncogenes show mutations (afatinib, osimertinib, crizotinib, alectinib, and ceritinib), or immunotherapy.

In the case of the aggressive form of brain cancer Glioblastoma Multiforme (GBM): (Incidence rate of 3.19 per 100,000 persons) First-line treatment is maximal surgical resection, radiotherapy, and concomitant and adjuvant chemotherapy with temozolomide; Second-line treatment: different chemotherapy (procarbazine/lomustine/vincristine (PCV)) or chemo therapy plus biologic (bevacizumab/irinotecan (BI)).

These exemplary treatments can now be improved with the insight provided by the rapid, non-invasive determination of CD8 TIL status as provided by the invention.

In some embodiments, the method comprises a non-invasive method to determine the TIL status of a tumour in a human subject. The method can comprise one or more of: a. administering a CD8 PET tracer to the subject, b. obtaining a PET image of the subject, and c. determining the TIL status of a tumour in the subject based on the SUV of the tumour in the PET image. In some embodiments, the SUV is calculated based on the ratios of tumour PET emission to L3 vertebrate to aorta.

## TREATMENT-COLD AND HOT COMBINATION

In some embodiments, a method of treating a human subject having a tumor comprises administering to the

subject a dose of a CD8 antigen binding construct, wherein the dose comprises a $^{89}$Zr-labeled antigen-binding construct providing a radiation activity of from about 0.5 mCI to about 3.6 mCi, and about 10 mg or less of the antigen binding construct, detecting the $^{89}$Zr-labeled antigen-binding construct in the patient at a first time point after administering the dose, to generate a first patient image corresponding to the first time point, wherein the first time point is about 6 hours to 36 hours after administering, determining a first abundance and/or distribution of CD8 positive cells in one or more tumor(s) in the patient based on the first patient image, and administering to the patient a first treatment for the first abundance and/or distribution of CD8 in the one or more tumor(s).

In some embodiments, a method of treating a human subject having a tumor comprises administering to the subject a dose of a CD8 antigen binding construct.

In some embodiments, a method of treating a human subject having a tumor comprises administering to the subject a dose of a CD8 antigen binding construct, wherein the dose comprises a $^{89}$Zr-labeled antigen-binding construct. In some embodiments, a method of treating a human subject having a tumor comprises administering to the subject a dose of a CD8 antigen binding construct, wherein the dose comprises a $^{89}$Zr-labeled antigen-binding construct providing a radiation activity. In some embodiments, a method of treating a human subject having a tumor comprises administering to the subject a dose of a CD8 antigen binding construct, wherein the dose comprises a $^{89}$Zr-labeled antigen-binding construct providing a radiation activity of from about 0.5 mCI to about 3.6 mCi. In some embodiments, a method of treating a human subject having a tumor comprises administering to the subject a dose of a CD8 antigen binding construct, wherein the dose comprises a $^{89}$Zr-labeled antigen-binding construct, wherein the dose comprises about 10 mg or less of the antigen binding construct.

In some embodiments, a method of treating a human subject having a tumor comprises administering to the subject a dose of a CD8 antigen binding construct, wherein the dose comprises a $^{89}$Zr-labeled antigen-binding construct providing a radiation activity of from about 0.5 mCI to about 3.6 mCi, and detecting the $^{89}$Zr-labeled antigen-binding construct in the patient.

In some embodiments, a method of treating a human subject having a tumor comprises administering to the subject a dose of a CD8 antigen binding construct, wherein the dose comprises a $^{89}$Zr-labeled antigen-binding construct, wherein the dose comprises about 10 mg or less of the antigen binding construct, and detecting the $^{89}$Zr-labeled antigen-binding construct in the patient.

In some embodiments, a method of treating a human subject having a tumor comprises administering to the subject a dose of a CD8 antigen binding construct, wherein the dose comprises a $^{89}$Zr-labeled antigen-binding construct providing a radiation activity of from about 0.5 mCI to about 3.6 mCi, and about 10 mg or less of the antigen binding construct.

In some embodiments, a method of treating a human subject having a tumor comprises administering to the subject a dose of a CD8 antigen binding construct, wherein the dose comprises a $^{89}$Zr-labeled antigen-binding construct providing a radiation activity of from about 0.5 mCI to about 3.6 mCi, and about 10 mg or less of the antigen binding construct, and detecting the $^{89}$Zr-labeled antigen-binding construct in the patient.

In some embodiments, a method of treating a human subject having a tumor comprises administering to the subject a dose of a CD8 antigen binding construct, wherein the dose comprises a $^{89}$Zr-labeled antigen-binding construct providing a radiation activity of from about 0.5 mCI to about 3.6 mCi, and about 10 mg or less of the antigen binding construct, and detecting the $^{89}$Zr-labeled antigen-binding construct in the patient at a first time point after administering the dose.

In some embodiments, a method of treating a human subject having a tumor comprises administering to the subject a dose of a CD8 antigen binding construct, wherein the dose comprises a $^{89}$Zr-labeled antigen-binding construct providing a radiation activity of from about 0.5 mCI to about 3.6 mCi, and about 10 mg or less of the antigen binding construct, and detecting the $^{89}$Zr-labeled antigen-binding construct in the patient at a first time point after administering the dose, to generate a first patient image corresponding to the first time point.

In some embodiments, a method of treating a human subject having a tumor comprises administering to the subject a dose of a CD8 antigen binding construct, wherein the dose comprises a $^{89}$Zr-labeled antigen-binding construct providing a radiation activity of from about 0.5 mCI to about 3.6 mCi, and about 10 mg or less of the antigen binding construct, detecting the $^{89}$Zr-labeled antigen-binding construct in the patient at a first time point after administering the dose, to generate a first patient image corresponding to the first time point, wherein the first time point is about 6 hours to 36 hours after administering the dose.

In some embodiments, a method of treating a human subject having a tumor comprises administering to the subject a dose of a CD8 antigen binding construct, wherein the dose comprises a $^{89}$Zr-labeled antigen-binding construct providing a radiation activity of from about 0.5 mCI to about 3.6 mCi, and about 10 mg or less of the antigen binding construct, detecting the $^{89}$Zr-labeled antigen-binding construct in the patient at a first time point after administering the dose, to generate a first patient image corresponding to the first time point, wherein the first time point is about 6 hours to 36 hours after administering, and determining a first abundance and/or distribution of CD8 positive cells in one or more tumor(s) in the patient based on the first patient image.

In some embodiments, a method of treating a human subject having a tumor comprises administering to the subject a dose of a CD8 antigen binding construct, wherein the dose comprises a $^{89}$Zr-labeled antigen-binding construct providing a radiation activity of from about 0.5 mCI to about 3.6 mCi, and about 10 mg or less of the antigen binding construct, detecting the $^{89}$Zr-labeled antigen-binding construct in the patient at a first time point after administering the dose, to generate a first patient image corresponding to the first time point, wherein the first time point is about 6 hours to 36 hours after administering, and determining a first abundance and/or distribution of CD8 positive cells in one or more tumor(s) in the patient based on the first patient image, and administering to the patient a first treatment for the first abundance and/or distribution of CD8 in the one or more tumor(s).

In some embodiments, the first time point is about 1, 4, 8, 12, 16, 20, 24, 28, 32, 36, 40, 44, or 48 hours, or a value within a range defined by ay two of the aforementioned values, after administering the dose.

In some embodiments, a method of treating a human subject having a tumor further comprises after administering the first treatment, determining a second abundance and/or distribution of CD8 cells in the one or more tissue and/or tumor in the patient.

In some embodiments, a method of treating a human subject having a tumor further comprises after administering the first treatment, determining a second abundance and/or distribution of CD8 cells in the one or more tissue and/or tumor in the patient based on a second patient image.

In some embodiments, a method of treating a human subject having a tumor further comprises after administering the first treatment, determining a second abundance and/or distribution of CD8 cells in the one or more tissue and/or tumor in the patient based on a second patient image corresponding to a second time point.

In some embodiments, a method of treating a human subject having a tumor further comprises after administering the first treatment, determining a second abundance and/or distribution of CD8 cells in the one or more tissue and/or tumor in the patient based on a second patient image comprising a second site-specific information for the target of the CD8 antigen binding construct.

In some embodiments, a method of treating a human subject having a tumor further comprises after administering the first treatment, determining a second abundance and/or distribution of CD8 cells in the one or more tissue and/or tumor in the patient based on a second patient image corresponding to a second time point and comprising a second site-specific information for the target of the CD8 antigen binding construct.

In some embodiments, a method of treating a human subject having a tumor further comprises after administering the first treatment, determining a second abundance and/or distribution of CD8 cells in the one or more tissue and/or tumor in the patient based on a second patient image corresponding to a second time point and comprising a second site-specific information for the target of the CD8 antigen binding construct, and administering to the patient a second treatment for the tumor based on a comparison of the first and second patient images.

In some embodiments, a method of treating a human subject having a tumor further comprisesoptimizing a therapeutic dose of the second treatment.

In some embodiments, a method of treating a human subject having a tumor further comprisesoptimizing a therapeutic dose of the second treatment based on the comparison of the first and second patient images.

## TREATMENT-GLOBAL APPROACH

In some embodiments, a method of treatment of a human subject having cancer comprises administering a CD8 antigen binding construct to a subject, wherein the CD8 antigen binding construct is labeled with a detectable label, scanning at least 30% of the subject's body to determine a distribution of the CD8 antigen binding construct within the body,identifying at least two separate tumors within the subject's body, via the distribution of the CD8 antigen binding construct, determining the tumor-infiltrating lymphocyte (TIL) status of each of the two separate tumors, and administering a first therapy to the subject if the TIL status of the at least two tumors are the same, or administering a second therapy to the subject if the TIL status of the at least two tumors are different. In some embodiments, a method of treatment of a human subject having cancer comprises administering a CD8 antigen binding construct to a subject. In some embodiments, a method of treatment of a human subject having cancer comprises administering a CD8 antigen binding construct to a subject, wherein the CD8 antigen binding construct is labeled.

In some embodiments, a method of treatment of a human subject having cancer comprises administering a CD8 antigen binding construct to a subject, wherein the CD8 antigen binding construct is labeled with a detectable label.

In some embodiments, a method of treatment of a human subject having cancer comprises administering a CD8 antigen binding construct to a subject, wherein the CD8 antigen binding construct is labeled with a detectable label, and scanning at least 30% of the subject's body.

In some embodiments, a method of treatment of a human subject having cancer comprises administering a CD8 antigen binding construct to a subject, wherein the CD8 antigen binding construct is labeled with a detectable label, and scanning at least 30% of the subject's body to determine a distribution of the CD8 antigen binding construct within the body.

In some embodiments, a method of treatment of a human subject having cancer comprises administering a CD8 antigen binding construct to a subject, wherein the CD8 antigen binding construct is labeled with a detectable label, and scanning at least 30% of the subject's body to determine a distribution of the CD8 antigen binding construct within the body, and identifying at least two separate tumors within the subject's body.

In some embodiments, a method of treatment of a human subject having cancer comprises administering a CD8 antigen binding construct to a subject, wherein the CD8 antigen binding construct is labeled with a detectable label, and scanning at least 30% of the subject's body to determine a distribution of the CD8 antigen binding construct within the body, and identifying at least two separate tumors within the subject's body, via the distribution of the CD8 antigen binding construct.

In some embodiments, a method of treatment of a human subject having cancer comprises administering a CD8 antigen binding construct to a subject, wherein the CD8 antigen binding construct is labeled with a detectable label, and scanning at least 30% of the subject's body to determine a distribution of the CD8 antigen binding construct within the body, and identifying at least two separate tumors within the subject's body, via the distribution of the CD8 antigen binding construct, and determining the tumor-infiltrating lymphocyte (TIL) status of each of the two separate tumors.

In some embodiments, a method of treatment of a human subject having cancer comprises administering a CD8 antigen binding construct to a subject, wherein the CD8 antigen binding construct is labeled with a detectable label, and scanning at least 30% of the subject's body to determine a distribution of the CD8 antigen binding construct within the body, and identifying at least two separate tumors within the subject's body, via the distribution of the CD8 antigen binding construct, and determining the tumor-infiltrating lymphocyte (TIL) status of each of the two separate tumors, and administering a first therapy to the subject if the TIL status of the at least two tumors are the same.

In some embodiments, a method of treatment of a human subject having cancer comprises administering a CD8 antigen binding construct to a subject, wherein the CD8 antigen binding construct is labeled with a detectable label, and scanning at least 30% of the subject's body to determine a distribution of the CD8 antigen binding construct within the body, and identifying at least two separate tumors within the subject's body, via the distribution of the CD8 antigen binding construct, and determining the tumor-infiltrating lymphocyte (TIL) status of each of the two separate tumors, and administering a second therapy to the subject if the TIL status of the at least two tumors are different.

In some embodiments, a method of treatment of a human subject having cancer comprises administering a CD8 antigen binding construct to a subject, wherein the CD8 antigen binding construct is labeled with a detectable label, and scanning at least 30, 35, 40, 45, 50, 55, 60, 65, 70, 75, 80, 85, 90, 95, or 99% of the subject's body, or a value defined by any two of the aforementioned values.

In some embodiments of a method of treatment of a human subject having cancer, determining if the at least two tumors are the same or different involves displaying the status and location of the at least two tumors. In some embodiments of a method of treatment of a human subject having cancer, displaying comprises a 2D or 3D representation of the location of the at least two tumors. In some embodiments of a method of treatment of a human subject having cancer, the CD8 antigen binding construct is associated with $^{89}Zr$.

In some embodiments, a method of administering an antigen binding construct to a subject is provided. The method comprises providing to a subject a labeled antigen binding construct that binds to human CD8. The label comprises $^{89}Zr$, the label provides 0.75 (or 0.5) - 3 mCi $\pm$20% of radiation at injection, and an amount between 0.2 and 10mg of total antigen binding construct is provided to the subject.

For any of the embodiments provided herein which reference an amount of radiation, it is noted that this amount can be the amount of radiation that is present upon injection or application to the subject (e.g., at that point in time).

In some embodiments, a method of treating a patient is provided. The method comprises: a) administering to a human patient diagnosed with a cancer a dose of an antigen-binding construct that binds to human CD8, wherein the dose comprises: a $^{89}Zr$-labeled antigen-binding construct providing a radiation activity of about 0.75 (or 0.5) to 3.6 mCi; and about 10 mg or less of the antigen-binding construct; b) detecting the $^{89}Zr$-labeled antigen-binding construct in the patient at a first time point after administering the dose, to generate a first patient image corresponding to the first time point, wherein the first time point is about 6 hours to 36 hours after administering; c) determining a first abundance and/or distribution of CD8 cells in one or more tissues and/or neoplasia in the patient based on the first patient image; d) administering to the patient a first treatment for the cancer; e) after administering the first treatment, administering to the human patient diagnosed with the cancer a second dose of the antigen-binding construct; f) determining a second abundance and/or distribution of CD8 cells in the one or more tissues and/or neoplasia in the patient based on a second patient image; and g) comparing the first patient image to the second patient image and administering to the patient a second treatment for the cancer based on a comparison of the first and second patient images.

In some embodiments, for the method above, comparing comprises determining if there is an increase in infiltration in the second image that indicates that the first treatment is working and therefore the first treatment is continued, and wherein no change or a decrease in infiltration in the second image indicates that the first treatment is not working and administering to the patient a second treatment for the cancer based on a comparison of the first and second patient images.

In some embodiments, a method of treating a patient is provided. The method comprises: a) administering to a human patient diagnosed with a cancer a dose of a CD8 PET tracer, wherein the dose comprises: a CD8 PET tracer that provides a radiation activity of about 0.75 (or 0.5) to 3.6 mCi; and between about 5 micrograms and 50 mg of the CD8 PET tracer (or between about 20 micrograms and 10 mg); b) detecting the CD8 PET tracer in the patient at a first time point after administering the dose, to generate a first patient image corresponding to the first time point, wherein the first time point is about 1 hour to 36 hours after administering; c) determining a first abundance and/or distribution of the CD8 PET tracer in one or more tissues and/or neoplasia in the patient based on the first patient image; d) administering to the patient a first treatment for the cancer; e) after administering the first treatment, administering to the human patient diagnosed with the cancer a second dose of the CD8 PET tracer; f) determining a second abundance and/or distribution of the CD8 PET tracer in the one or more tissues and/or neoplasia in the patient based on a second patient image; and g) comparing the first patient image to the second patient image and administering to the patient a second treatment for the cancer based on a comparison of the first and second patient images.

In some embodiments, a method of treating a subject comprises: administering to a patient that has a neoplasia and that is being treated with an immunotherapy ("IOT") an antigen-binding construct that binds to human CD8; wherein the antigen binding construct comprises a detectable marker, wherein the antigen binding construct does not provoke an immune response in the subject, wherein the detectable marker comprises $^{89}Zr$, wherein the detectable marker provides 0.5-3 mCi +20% of radiation at injection, and wherein an amount between 0.2 and 10mg of total antigen binding construct is provided to the patient; monitoring a distribution of the antigen-binding construct to determine the tumor-infiltrating lymphocyte ("TIL") status within the neoplasia; and administering an alternative IOT to the patient if the TIL status of the neoplasia is not positive, and repeating the foregoing method until the TIL status of the neoplasia becomes positive.

In some embodiments, for any one of the methods provide herein, the first abundance is a first intensity of the CD8 PET tracer or the $^{89}Zr$ signal on the first patient image, and wherein the second abundance is a second intensity of the CD8 PET tracer or the $^{89}Zr$ signal on the second patient image.

In some embodiments, for any one of the methods provide herein, a location of a tumor is indicated as an increase in radiation in the second patient image than in the first patient image.

In some embodiments, for any one of the methods provide herein, the first time point is between 20 and 34 hours after administering, and wherein determining the second abundance and/or distribution occurs 20 to 34 hours after the second dose is administered.

In some embodiments, for any one of the methods provide herein, the CD8 PET tracer comprises $^{18}F$. In some embodiments, the CD8 PET tracer comprises a PET isotope with a half-life of less than 3 hours.

In some embodiments, for any one of the methods provide herein, monitoring is conducted within 8 hours of administering. In some embodiments, for any one of the methods provide herein, monitoring or a further second monitoring is done before 36 hours from administering.

In some embodiments, a method of treating a human subject having cancer is provided. The method comprises: i) providing a first image of a CD8 cells within a tumor in a subject using a CD8 antigen binding construct; ii) administering a therapy including a candidate therapeutic to the subject; iii) after administering the therapy including the candidate therapeutic, providing a second image of the CD8 cells within the tumor in the subject using the CD8 antigen binding construct; and iv) comparing the first and second images to determine if: a) the tumor demonstrates increased CD8 infiltration or b) the tumor demonstrates the same or decreased CD8 infiltration, wherein, if a), then instructing the subject to continue the therapy. In some embodiments, if b), then administering another round of therapy, providing a third image of the CD8 cells within the tumor; comparing the first and third images to determine if the tumor demonstrates the same or decreased CD8 infiltration; and discontinuing the therapy if the tumor demonstrates the same or decreased CD8 infiltration.

In some embodiments, a method of treating a human subject having cancer is provided. The method comprises i) providing a first image of CD8 cells within a tumor in a subject using a CD8 PET tracer; ii) administering a therapy including a candidate therapeutic to the subject; iii) after administering the therapy, providing a second image of the CD8 cells within the tumor in the subject using the CD8 PET tracer; and iv) comparing the first and second images to determine if: a) the tumor demonstrates increased CD8 infiltration or b) the tumor demonstrates the same or decreased CD8 infiltration, wherein, if a), then instructing the subject to continue the therapy.

In some embodiments, a method of treating a human subject is provided. The method comprises: a) administering a candidate therapeutic to a human subject; b) determining if a size of a tumor in the human subject has increased or decreased in response to the candidate therapeutic; and c) if the size has increased following the candidate therapeutic, then using a CD8 antigen binding construct to determine if an amount of CD8 present within the tumor has increased or decreased in response to the candidate therapeutic. An increase in tumor size without an increase in the amount of CD8 indicates tumor progression, whereas an increase in tumor size with an increase in the amount of CD8 indicates tumor pseudoprogression, wherein a treatment is continued if the patient is experiencing tumor pseudoprogression, and wherein a treatment is changed if the patient is experiencing tumor progression.

In some embodiments, a method of administering a label to a human subject is provided. The method comprises: administering $^{18}F$ to a subject; within about 6 hours, conducting a PET scan of the subject for a $^{18}F$ distribution of the subject; administering a CD8 PET tracer to the subject, wherein the CD8 PET tracer comprises $^{89}Zr$; and within about 36 hours of administering the CD8 PET tracer, conducting a PET scan on the subject for a distribution of $^{89}Zr$. The time for a PET of the 18F can be, for example 0.5, 1, 2, 3, 4, 5or 6 hours, including any range therebetween.The time for PET for 89Zr can be6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or 36 hours, including any range therebetween.

In some embodiments, for any one of the methods provide herein (including, without limitation methods of treating or diagnosing or imaging), the subject or patient receives a therapeutic, and wherein the therapeutic is selected from at least one of: Ipilimumab, Pembrolizumab, Atezolizumab, Avelumab, and Durvalumab. In some embodiments, for any one of the methods provide herein, wherein the subject or patient receives a therapeutic, and wherein the therapeutic is selected from at least one of: BMS-1001, BMS-1116, CA-170, CA-327, Imiquimod, Resiquimod, 852A, VTX-2337, ADU-S100, MK-1454, Ibrutinib, 3AC, Idelalisib, IPI-549, Epacadostat, AT-38, CPI-444, Vipadenant, Preladenant, PBF, ZD4635, Galuniseritib, OTX015/MK-8628, CPI-0610. In some embodiments, for any one of the methods provide herein, wherein the subject or patient receives a therapeutic, and wherein the therapeutic is selected from at least one of: an antigen binding construct that is selective for one or more of LAG-3, TIM3, B7-H4 and/or TIGIT, or alternatively MK-4280, MK-7684, and/or any of the preceding in combination with Keytruda.

In some embodiments, for any one of the methods provide herein (including, without limitation methods of treating or diagnosing or imaging)the CD8 antigen binding construct is a minibody or a diabody and wherein the antigen binding construct comprises a heavy chain variable region and a light chain variable region, and wherein the heavy chain variable region consists essentially of a human amino acid sequence and the light chain variable region consists essentially of a human amino acid sequence.

In some embodiments, for any one of the methods provide herein (including, without limitation methods of treating or diagnosing or imaging), the antigen binding construct comprises the three heavy chain CDRs within SEQ ID NO:s 16 or 147 and the three light chain CDRs in SEQ ID Nos: 15 or 147. In some embodiments, for any one of the methods provide herein, the antigen binding construct comprises a heavy chain variable region that is at least 80% identical to the heavy chain variable region within SEQ ID NO:1, 3, 16, or 147, and a light chain variable region that is at least 80% identical to the light chain variable region within SEQ ID NO: 7, 9, 15, or 147.

In some embodiments, the antigen binding construct is at least 70, 75, 80, 85, 90, 91, 92, 93, 94, 95, 96, 97, 98, 99, or greater percent identical to the sequence in SEQ ID NO: 147. In some embodiments, the antigen binding construct is at least 70, 75, 80, 85, 90, 91, 92, 93, 94, 95, 96, 97, 98, 99, or greater percent similar to the sequence in SEQ ID NO: 147.

In some embodiments, for any one of the methods provide herein (including, without limitation methods of treating or diagnosing or imaging), the subject or patient has a cancer selected from one or more cancer of: carcinoma, lymphoma, blastoma, sarcoma, and leukemia, lymphoid malignancies, squamous cell cancer (e.g. epithelial squamous cell cancer), lung cancer, small-cell lung cancer, non-small cell lung cancer, adenocarcinoma of the lung and squamous carcinoma of the lung, cancer of the peritoneum, hepatocellular cancer, gastric or stomach cancer, gastrointestinal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain, head and neck cancer, adult and pediatric solid cancers, and solid tumors.

In some embodiments, for any one of the methods provide herein (including, without limitation methods of treating or diagnosing or imaging), the treatment comprises at least one of the therapies provided herein, including, but not limited to: an immune check point inhibitor, an IOT, or chemotherapeutic agent provided herein.

In some embodiments, for any one of the methods provide herein (including, without limitation methods of treating or diagnosing or imaging), the human CD8 is a human CD8 alpha chain. In some embodiments, for any one of the methods provide herein (including, without limitation methods of treating or diagnosing or imaging), the antigen binding construct is selective for human CD8 alpha chain over human CD8 beta chain. In some embodiments, for any one of the methods provide herein (including, without limitation methods of treating or diagnosing or imaging), the antigen binding construct is selective for human CD8 beta chain over human CD8 alpha chain. In some embodiments, for any one of the methods provide herein, the alpha chain as the sequence of SEQ ID NO: 134. In some embodiments, for any one of the methods provide herein (including, without limitation methods of treating or diagnosing or imaging), the human CD8 is a human CD8 beta chain. In some embodiments, for any one of the methods provided herein, the beta chain as the sequence of SEQ ID NO: 135. In some embodiments, for any one of the methods provide herein (including, without limitation methods of treating or diagnosing or imaging), an increase in detectable marker indicates that there is an increase in CD8 infiltration. In some embodiments, for any one of the methods provide herein (including, without limitation methods of treating or diagnosing or imaging), an increase in signal from a CD8 PET tracer, from a first image to a

second image, indicates that there is an increase in infiltration in the tumor.

In some embodiments, for any one of the methods provide herein (including, without limitation methods of treating or diagnosing or imaging), an increase in infiltration indicates that a first therapy is effective against the tumor, wherein an increase in infiltration is indicated by an increase in a PET detected signal between a first image and a second image, and wherein the PET detect signal is from a detectable marker of a CD8 PET tracer or a CD8 antigen binding construct.

In some embodiments, for any one of the methods provide herein (including, without limitation methods of treating or diagnosing or imaging), the increase in the PET detected signal is at least 10%. In some embodiments, for any one of the methods provide herein, the increase in the PET detected signal is at least 100%. In some embodiments, for any one of the methods provide herein, the increase in the PET detected signal is at least 1000%. In some embodiments, for any one of the methods provide herein, the increase in the PET detected signal is at least 5000%. In some embodiments, for any one of the methods provide herein, the increase in the PET detected signal is at least 10,000%.

In some embodiments, for any one of the methods provide herein (including, without limitation methods of treating or diagnosing or imaging), further comprising determining a standard uptake value ("SUV"), the method of determining the SUV comprising: a) determining r, where r is a radioactivity concentration (kBq/ml) measured by a PET scanner within a region of interest ("ROI") of radiation from the antigen binding construct; b) determining a', wherein a' is the decay-corrected amount of the injected detectable marker (kBq); c) determining w, the weight of the patient; and d) determining SUV as being the result of r(a'/W).

In some embodiments, for any one of the methods provide herein (including, without limitation methods of treating or diagnosing or imaging), the method further comprises repeating the process for a second ROI to determine a second summed signal level and comparing the first and the second summed signal levels, wherein when the first summed signal level is less than the second summed signal level, it indicates the presence of increased CD8 in the second ROI.

In some embodiments, for any one of the methods provide herein (including, without limitation methods of treating or diagnosing or imaging), wherein a sample that is analyzed 20 microns thick.

## METHOD OF MONITORING

In some embodiments, a method of monitoring CD8 *in vivo* is provided. The method can comprise providing a CD8 minibody to a subject. The CD8 minibody binds to a CD8 sequence as shown in FIG. 1C, and the minibody is labeled with a detectable marker. The method can further comprise monitoring a distribution of the CD8 minibody in the subject within 6-36 hours of administering the CD8 minibody to the subject. In some embodiments, any antigen binding construct can be employed. In some embodiments, the minibody employed is one of the ones designated herein, such as that in FIG. 39 (SEQ ID NO: 147), along with a label or detectable marker, such as $^{89}Zr$. The method can allow one to monitor a distribution of CD8 within a host, either in combination with other techniques, or alone. In some embodiments, monitoring and/or determining the CD8 distribution within a host provides one with a representation of the distribution of CD8+ cells, such as CD8+ T cells. Thus, using one or more of the constructs provided herein can allow one to monitor CD8+ T cells within a host.

In some embodiments, the duration of time between an administration of a CD8 minibody to a subject and the subsequent monitoring, is as needed or appropriate for the particular application. In some embodiments, it is within 6-36 hours, for example, 10-32, 12-30, 14-30, or 18-28 hours. In some embodiments, the duration between administration and monitoring (via, PET for example), is 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or 36 hours, including any range defined between any two of the preceding values. These ranges can apply to any of the methods provided herein.

In some embodiments, a method of monitoring CD8 in vivo is provided. The method comprises providing a CD8 minibody to a subject, wherein the CD8 minibody binds to a CD8 as shown in FIG. 1C, and the minibody is labeled with a detectable marker. The method further comprises monitoring a distribution of the CD8 minibody in the subject, wherein the monitoring can detect a tissue infiltrated with 500 or less CD8 bearing cells per $mm^2$ within the subject (e.g., in a 20 micron depth). Monitoring is achieved via PET.

In some embodiments, the minibody (or any antigen binding construct) is of the category that it binds to human CD8 (including that in SEQ ID NO: 24, FIG. 1C, FIG. 33, or FIG. 34). In some embodiments, the antigen binding construct can also bind to other CD8 sequences. In some embodiments, it does not bind to non-human CD8. In some embodiments, it can bind to naturally occurring human CD8.

In some embodiments, the sensitivity of detection can be of fewer than 500 cells/cubic mm, for example, 200, 250, 300, 350, 400, 410, 420, 430, 440, 450, 460, 470, 480, 490, 495, 499, or 500, including any range defined between any two of the preceding values. In some embodiments, more than 500 cells per $mm^2$ can be the level of sensitivity of detection (e.g., in a 20 micron depth). Any of the time durations provided herein can also be employed between the time of administration and detection in the subject.

In some embodiments, a method of visualizing CD8 positive cells is provided. The method comprises providing a CD8 minibody to a subject. The CD8 minibody is labeled with a detectable marker. The CD8 minibody is humanized and/or deimmunized in sequence and binds to human CD8. The method further comprises monitoring a distribution of the CD8 minibody in the subject within 6-36 hours of administering the CD8 minibody to the subject, wherein monitoring can detect a tissue infiltrated with 500 or less CD8 positive cells per $mm^2$ within the subject (e.g., in a 20 micron depth), and wherein monitoring is achieved via PET.

In some embodiments, a method of visualizing cells in a human is provided. The method comprises providing a means of binding human CD8 positive cells to a subject, wherein the means comprises a detectable label. The method further comprises monitoring and determining a first distribution of the means of binding human CD8 positive cells in the subject within 6-36 hours of administering the means of binding human CD8 positive cells to the subject.

In some embodiments, the means of binding human CD8 positive cells in the subject is any one or more of the CD8 antigen binding constructs provided herein. In some embodiments, the means is any one of the sequences provided in any one of the figures or tables herein. In some embodiments, the means is any antigen binding construct that includes all six CDRs from any of the antigen binding constructs provided herein.

In some embodiments, the method further comprises monitoring and determining a second distribution of the means of binding human CD8 positive cells in the subject after monitoring and determining the first distribution; and comparing the first and second distributions to determine a change in distribution.

In some embodiments, the method further comprises administering a first therapeutic agent to the subject with or prior to providing the means of binding human CD8 positive cells to a subject. The change in distribution is used to determine an effectiveness of the first therapeutic agent. If the change in distribution indicates an increase in an amount of CD8 positive cells within a tumor, at least a second dose of the first therapeutic agent is administered to the subject, and if the change in distribution indicates a decrease in an amount of CD8 positive cells within a tumor, a second therapeutic agent is administered to the subject.

In some embodiments, the method further comprises administering a first therapeutic agent to the subject with or prior to providing the means of binding human CD8 positive cells to a subject. The change in distribution is used to determine an effectiveness of the first therapeutic agent. If the change in distribution indicates an increase in an amount of CD8 positive cells within a tumor, at least a second dose of the first therapeutic agent is administered to the subject, and if the change in distribution indicates no change or a decrease in an amount of CD8 positive cells within a tumor, no second dose of the first therapeutic agent is administered to the subject.

In some embodiments, the method further comprises administering a first therapeutic agent to the subject with or prior to providing the means of binding human CD8 positive cells to a subject. The change in distribution is used to determine an effectiveness of the first therapeutic agent. If the change in distribution indicates no change or an increase in an amount of CD8 positive cells within a tumor, at least a second dose of the first therapeutic agent is administered to the subject, and if the change in distribution indicates a decrease in an amount of CD8 positive cells within a tumor, no second dose of the first therapeutic agent is administered to the subject.

Thus, in some embodiments (using any of the means of binding human CD8 positive cells provided herein, or using any of the antigen binding constructs to CD8 provided herein, one can determine a change in distribution of CD8 and/or CD8+ cells, and/or CD8+ Tcells in a subject. This change can be used for a variety of options, including, for example, determining the effectiveness of a potential or current therapeutic, and/or formulation thereof, and/or amount thereof, and/or administration scheme thereof. As described in more detail below, the method can also be used to monitor the TIL status of a neoplasia.

In some embodiments, a method of administering a minibody to a subject is provided. The method comprises providing to a subject a labeled minibody that binds to human CD8. An appropriate amount of radiation, administered via the minibody, can be provided. In some embodiments, 3 mCi +20% of radiation is provided to the subject via the labeled minibody and between 0.2 and 10mg of total protein is provided to the subject. In some embodiments, any amount of radiation provided herein can be administered to the subject, while having 1.5 to 10 mg of total protein being the delivery system for at least part of the radioactivity.

In some embodiments, a method of administering a minibody to a subject is provided. The method comprises providing to a subject a labeled minibody that binds to human CD8, wherein 0.75 (or 0.5) - 1.5 +/- 20% mCi of radiation is provided to the subject via the labeled minibody and wherein an amount between 0.2 and 10mg of total protein is provided to the subject. Where lower doses of radiation are employed, in some embodiments a second dose can be administered within 3-4 days after the first dose, which can allow for a sharper image.

In some embodiments, 0.5, 0.6, 0.7, 0.75, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, or more mCi of radiation is administered to the subject using 0.2, 0.5, 0.75, 1, 2, 3, 4, 5, 6, 7, 8, 9, or 10 or more mg of the minibody or antigen binding construct. In some embodiments, a dose of around 3 mCi allows for an initial image at 6-36 hours, plus a second image at 3-10 days, possibly 14 days, without additional dose administration.

## PET-BASED IMAGING

In some embodiments, a method of performing a PET scan in a human subject comprises administering any one or more of the compositions or formulations provided herein, waiting for a specified period of time for a distribution of the CD8 antigen binding construct to occur within the subject, and performing a PET scan over a specified period of time for scanning. In some embodiments, a method of performing a PET scan in a human subject comprises administering any one or more of the compositions or formulations provided herein.

In some embodiments, a method of performing a PET scan in a human subject comprises administering any one or more of the compositions or formulations provided herein, and waiting for a specified period of time for a distribution of the CD8 antigen binding construct to occur within the subject.

In some embodiments, a method of performing a PET scan in a human subject comprises administering any one or more of the compositions or formulations provided herein, and waiting for a specified period of time for a distribution of the CD8 antigen binding construct to occur within the subject, and performing a PET scan over a specified period of time for scanning. In some embodiments of the method of performing a PET scan in a human subject, the specified period of time for a distribution of the CD8 antigen binding construct is from 1 hour to 36 hours. In some embodiments of the method of performing a PET scan in a human subject, the specified period of time for a distribution of the CD8 antigen binding construct is from 5 hour to 24 hours. In some embodiments of the method of performing a PET scan in a human subject, the specified period of time for a distribution of the CD8 antigen binding construct is 0.25 h, 0.5 h, 1 h, 4 h, 8 h, 12 h, 16 h, 20 h, 24 h, 28 h, 32 h, 36 h, 40 h, 44 h, 48 h, 52 h, 56 h, 60 h, 64 h, 68 h, or 72 h, or a value within a range defined by any two of the aforementioned values.

In some embodiments of the method of performing a PET scan in a human subject, the specified period of time for scanning is between 10 minutes and one hour. In some embodiments of the method of performing a PET scan in a human subject, the specified period of time for scanning is between 12 minutes and 40 minutes. In some embodiments of the method of performing a PET scan in a human subject, the specified period of time for scanning is about 12 to about 20 minutes and the radio label provides more than 0.5 but less than 1.1 mCi of radiation. In some embodiments of the methodof performing a PET scan in a human subject, the radio label provides less than 1.0 mCi of radiation. In some embodiments of the method of performing a PET scan in a human subject, a PET scan is performed to obtain a location, distribution, ratio, and/or tumor status in the subject.

In some embodiments of the method of performing a PET scan in a human subject, a PET scan is performed to obtain a location, distribution, ratio, and/or tumor status in the subject, and wherein the PET scan device is configured for a human.

In some embodiments of the method of performing a PET scan in a human subject, the specified period of time for scanning is about 5, 10, 15, 20, 25, 30, 35, 40, 45, 50, 55, or 60 min, or a value within a range defined by any two of the aforementioned values.

In some embodiments of the method of performing a PET scan in a human subject, the radio label provides radiation of 0.25, 0.5, 0.75, 1.0, 1.25, 1.5, 1.75, or 2 mCi, or a value within a range defined by any two of the aforementioned values.

In some embodiments, a method of performing a PET scan in a human subject comprises administering a minibody that binds to a human CD8 antigen, wherein the minibody is conjugated to a radioactive label, and wherein administering the minibody provides more than 0.5 but less than 1.1 mCi of radiation, waiting for 1-36 hours, and performing a PET scan over 15-30 minutes for scanning. In some embodiments, any of the ranges regarding amounts of radiation, and duration or waiting, provided herein can be used in the context of this embodiment.

In some embodiments, a method of performing a PET scan in a human subject comprises administering a minibody. In some embodiments, a method of performing a PET scan in a human subject comprises administering a minibody that binds to a human CD8 antigen.

In some embodiments, a method of performing a PET scan in a human subject comprises administering a minibody that binds to a human CD8 antigen, wherein the minibody is conjugated to a radioactive label.

In some embodiments, a method of performing a PET scan in a human subject comprises administering a minibody that binds to a human CD8 antigen, wherein the minibody is conjugated to a radioactive label, and wherein administering the minibody provides more than 0.5 but less than 1.1 mCi of radiation.

In some embodiments, a method of performing a PET scan in a human subject comprises administering a minibody that binds to a human CD8 antigen, wherein the minibody is conjugated to a radioactive label, and waiting for 1-36 hours.

In some embodiments, a method of performing a PET scan in a human subject comprises administering a minibody that binds to a human CD8 antigen, wherein the minibody is conjugated to a radioactive label, wherein administering the minibody provides more than 0.5 but less than 1.1 mCi of radiation, and waiting for 1-36 hours.

In some embodiments, a method of performing a PET scan in a human subject comprises administering a minibody that binds to a human CD8 antigen, wherein the minibody is conjugated to a radioactive label, wherein administering the minibody provides more than 0.5 but less than 1.1 mCi of radiation, and waiting for 1-36 hours, and performing a PET scan over 15-30 minutes for scanning.

In some embodiments, a method of positron emission tomography ("PET") comprises administering a tracer that binds to CD8 to a human subject, providing a scintillator, using the scintillator to detect a pair of photons created by the tracer, using detection of the pair of photons to localize a source of the tracer via a list of coincidence events that are processed via a processor that is configured to take an output from the scintillator and convert it to the list of coincidence events, wherein between about 300 and about 500 CD8 positive cells can be detected per $mm^2$ of a tissue or neoplasia within the subject. In some embodiments, any of the ranges regarding the number of CD8 positive cells provided herein can be used in the context of this embodiment.

In some embodiments, a method of PET comprises administering a tracer that binds to CD8 to a human subject, providing a scintillator, using the scintillator to detect a pair of photons created by the tracer, using detection of the pair of photons to localize a source of the tracer via a list of coincidence events that are processed via a processor that is configured to take an output from the scintillator and convert it to the list of coincidence events, wherein between about 300 and about 500 CD8 positive cells can be detected per $mm^2$ of a tissue or neoplasia within the subject.

In some embodiments, a method of PET comprises administering a tracer. In some embodiments, a method of PET comprises administering a tracer that binds to CD8. In some mbodiments, a method of PET comprises administering a tracer that binds to CD8 to a human subject. In some embodiments, a method of PET comprises administering a tracer that binds to CD8 to a human subject, and providing a scintillator. In some embodiments, a method of PET comprises administering a tracer that binds to CD8 to a human subject, and providing a scintillator, and using the scintillator to detect a pair of photons created by the tracer. In some embodiments, a method of PET comprises administering a tracer that binds to CD8 to a human subject, and providing a scintillator, and using the scintillator to detect a pair of photons created by the tracer, and using detection of the pair of photons to localize a source of the tracer.

In some embodiments, a method of PET comprises administering a tracer that binds to CD8 to a human subject, and providing a scintillator, and using the scintillator to detect a pair of photons created by the tracer, and using detection of the pair of photons to localize a source of the tracer via a list of coincidence events that are processed via a processor that is configured to take an output from the scintillator and convert it to the list of coincidence events.

In some embodiments, a method of PET comprises administering a tracer that binds to CD8 to a human subject, providing a scintillator, using the scintillator to detect a pair of photons created by the tracer, using detection of the pair of photons to localize a source of the tracer via a list of coincidence events that are processed via a processor that is configured to take an output from the scintillator and convert it to the list of coincidence events, wherein between about 300 and about 500 CD8 positive cells can be detected per $mm^2$ of a tissue or neoplasia within the subject.In some embodiments, about 250 and about 550 CD8 positive cells can be detected per $mm^2$ of a tissue or neoplasia within the subject.

In some embodiments, about 200 and about 600 CD8 positive cells can be detected per $mm^2$ of a tissue or neoplasia within the subject. In some embodiments, about 150 and about 600 CD8 positive cells can be detected per $mm^2$ of a tissue or neoplasia within the subject.

In some embodiments, a method of analyzing CD8 distribution in a subject is provided. The method comprises providing an image of a distribution of a detectable marker via a first PET image. The detectable marker is linked to a CD8 minibody. The CD8 minibody binds to human CD8, and wherein the CD8 minibody does not provoke an immune response in the subject. The method further comprises providing an image of a distribution of a FDG marker (e.g., fludoxyglucose ($^{18}F$)) via a second PET image of the subject and creating a third PET image that comprises an overlay of the first PET image onto the second PET image and identifying a tumor as TIL, based upon the third PET image.

In some embodiments, a method of analyzing CD8 distribution in a subject is provided. The method comprises providing an image of a distribution of a detectable marker via a first PET image. The detectable marker is linked to a CD8 minibody. The CD8 minibody binds to human CD8, and wherein the CD8 minibody does not provoke an immune response in the subject. The method further comprises providing an image of a distribution of a FDG marker (e.g., fludoxyglucose ($^{18}F$)) via a second PET image of the subject. Identifying a tumor's status, based upon the fist and second PET image.

In some embodiments, a MRI and/or CT scan can be performed to identify the location of the tumor. This can be done for any of the methods provided herein. The other techniques provided herein can then be employed to determine the activity status of the tumor or neoplasia and its TIL status.

## IMAGING--DUAL ADMINISTRATION/DUAL IMAGING OPTIONS

In some embodiments, a method of administering a label to a human subject comprises administering $^{18}F$ to a subject, within about 6 hours, conducting a PET scan of the subject for a $^{18}F$ distribution of the subject, administering a CD8 antigen binding construct to the subject, wherein the CD8 antigen binding construct is linked to $^{89}Zr$, and within about 36 hours of administering the CD8 antigen binding construct, conducting a PET scan on the subject for a distribution

of $^{89}Zr$. In some embodiments, a method of administering a label to a human subject comprises administering $^{18}F$ to a subject.

In some embodiments, a method of administering a label to a human subject comprises administering $^{18}F$ to a subject, and within about 6 hours, conducting a PET scan of the subject. In some embodiments, a method of administering a label to a human subject comprises administering $^{18}F$ to a subject, and within about 6 hours, conducting a PET scan of the subject for a $^{18}F$ distribution of the subject. In some embodiments, a method of administering a label to a human subject comprises administering $^{18}F$ to a subject, and within about 6 hours, conducting a PET scan of the subject for a $^{18}F$ distribution of the subject, and administering a CD8 antigen binding construct to the subject. In some embodiments, a method of administering a label to a human subject comprises administering $^{18}F$ to a subject, and within about 6 hours, conducting a PET scan of the subject for a $^{18}F$ distribution of the subject, and administering a CD8 antigen binding construct to the subject, wherein the CD8 antigen binding construct is linked to $^{89}Zr$.

In some embodiments, a method of administering a label to a human subject comprises administering $^{18}F$ to a subject, and within about 6 hours, conducting a PET scan of the subject for a $^{18}F$ distribution of the subject, and administering a CD8 antigen binding construct to the subject, wherein the CD8 antigen binding construct is linked to $^{89}Zr$, and within about 36 hours of administering the CD8 antigen binding construct, conducting a PET scan on the subject.

In some embodiments, a method of administering a label to a human subject comprises administering $^{18}F$ to a subject, and within about 6 hours, conducting a PET scan of the subject for a $^{18}F$ distribution of the subject, and administering a CD8 antigen binding construct to the subject, wherein the CD8 antigen binding construct is linked to $^{89}Zr$, and within about 36 hours of administering the CD8 antigen binding construct, conducting a PET scan on the subject for a distribution of $^{89}Zr$.

In some embodiments of a method of administering a label to a human subject, conducting the PET scan of the subject for the $^{18}F$ distribution of the subject within about 2 hours.

In some embodiments, the method of administering a label to a human subject further comprises comparing a distribution of $^{18}F$ to a distribution of $^{89}Zr$ to determine if there are areas of elevated $^{18}F$ or $^{89}Zr$ distribution of the subject that overlap with one another, wherein areas where there is elevated $^{18}F$, that are indicative of a tumor that is TIL positive if there is also an overlapping elevated $^{89}Zr$ in the same area, and determined to be TIL negative if there is not an overlapping elevated $^{89}Zr$ in the same area.

In some embodiments, the method of administering a label to a human subject further comprises comparing a distribution of $^{18}F$ to a distribution of $^{89}Zr$. In some embodiments, the method of administering a label to a human subject further comprises comparing a distribution of $^{18}F$ to a distribution of $^{89}Zr$ to determine if there are areas of elevated $^{18}F$ or $^{89}Zr$ distribution of the subject that overlap with one another.

In some embodiments, the method of administering a label to a human subject further comprises comparing a distribution of $^{18}F$ to a distribution of $^{89}Zr$ to determine if there are areas of elevated $^{18}F$ or $^{89}Zr$ distribution of the subject that overlap with one another, and for areas where there is elevated $^{18}F$, if there is also an overlapping elevated $^{89}Zr$ in the same area, the areas are indicative of a tumor that is TIL positive.

In some embodiments, the method of administering a label to a human subject further comprises comparing a distribution of $^{18}F$ to a distribution of $^{89}Zr$ to determine if there are areas of elevated $^{18}F$ or $^{89}Zr$ distribution of the subject that overlap with one another, and for areas where there is elevated $^{18}F$, if there is also an overlapping elevated $^{89}Zr$ in the same area, the areas are indicative of a tumor that is TIL positive, and for areas where there is elevated $^{18}F$ if there is not an overlapping elevated $^{89}Zr$ in the same area, the areas are determined to be TIL negative.

In some embodiments of the method of administering a label to a human subject, the length of time between administering the $^{18}F$ and administering the CD8 antigen binding construct is not more than 10 days. In some embodiments of the method of administering a label to a human subject, the length of time between administering the $^{18}F$ and administering the CD8 antigen binding construct is 1, 2, 3, 4, 5, 6, 7, 8, 9, or 10 days, or a value within a range defined by any two of the aforementioned values

In some embodiments, a method of preparing an image of a human subject is provided. In some embodiments, the method comprises providing a first data set involving a representation of a MRI or FDG-PET scan, wherein the MRI or FDG-PET scan indicates at least one tumor; providing a second data set involving a representation of a PET scan of a 89Zr-CD8 antigen binding construct, wherein the second data set indicates at least one CD8 Region of Interest ("ROI"); and generating a combined image from the two data sets, wherein the combined image provides a comparison of the at least one tumor and the at least one CD8 ROI.

In some embodiments, a method of analyzing CD8 distribution in a subject comprises providing an image of a distribution of a detectable marker via a first PET image, wherein the detectable marker is linked to a CD8 antigen binding construct; providing an image of a distribution of a FDG marker via a second PET image of the subject, creating a third image that comprises an overlay of the first PET image onto the second PET image, and identifying the TIL status of a tumor based upon the third image.

In some embodiments of the method of analyzing CD8 distribution in a subject, the FDG marker is $^{18}F$.

In some embodiments, a method for identifying tumor characteristics comprises scanning for a distribution of

$^{18}F$ within a human subject to identify at least one tumor, scanning for a distribution of $^{89}Zr$ labeled CD8 antigen binding construct with the human subject to identify elevated concentrations of $^{89}Zr$ within the subject, and identifying the tumor as inflamed if the at least one tumor overlaps in location with any one or more elevated concentrations of $^{89}Zr$ within the subject. In some embodiments, a method for identifying tumor characteristics comprises scanning for a distribution of $^{18}F$.

In some embodiments, a method for identifying tumor characteristics comprises scanning for a distribution of $^{18}F$ within a human subject. In some embodiments, a method for identifying tumor characteristics comprises scanning for a distribution of $^{18}F$ within a human subject to identify at least one tumor.

In some embodiments, a method for identifying tumor characteristics comprises scanning for a distribution of $^{18}F$ within a human subject to identify at least one tumor, and scanning for a distribution of $^{89}Zr$ labeled CD8 antigen binding construct with the human subject.

In some embodiments, a method for identifying tumor characteristics comprises scanning for a distribution of $^{18}F$ within a human subject to identify at least one tumor, scanning for a distribution of $^{89}Zr$ labeled CD8 antigen binding construct with the human subject to identify elevated concentrations of $^{89}Zr$ within the subject.

In some embodiments, a method for identifying tumor characteristics comprises scanning for a distribution of $^{18}F$ within a human subject to identify at least one tumor, scanning for a distribution of $^{89}Zr$ labeled CD8 antigen binding construct with the human subject to identify elevated concentrations of $^{89}Zr$ within the subject, and identifying the tumor as inflamed if the at least one tumor overlaps in location with any one or more elevated concentrations of $^{89}Zr$ within the subject. In some embodiments of a method for identifying tumor characteristics, the one or more elevated concentrations of $^{89}Zr$ are elevated relative to a location that is not the at least one area of tumor growth. In some embodiments of a method for identifying tumor characteristics, the one or more elevated concentrations of $^{89}Zr$ are elevated relative to a location that is not the at least one area of tumor growth, but is within a same organ that contains the tumor.

In some embodiments, a method for identifying tumor characteristicsfurther comprises treating the tumor with an IOT if there is no overlap at the location for the elevated concentrations of $^{89}Zr$ and the tumor.

In some embodiments of a method for identifying tumor characteristics, if the at least one tumor does not overlap in location with any one or more elevated concentrations of $^{89}Zr$ within the subject, administering to the subject a candidate therapeutic to change the status of the tumor from immune excluded or immune desert to infiltrated. In some embodiments, the method for identifying tumor characteristicsfurther comprises repeating the scanning for a second distribution of $^{89}Zr$ labeled CD8 antigen binding construct with the human subject to identify if a location of the elevated concentrations of $^{89}Zr$ within the subject have changed due to the candidate therapeutic, and identifying the tumor as inflamed if the at least one tumor overlaps in location with any one or more elevated concentrations of $^{89}Zr$ within the subject from the second distribution of $^{89}Zr$ labeled CD8 antigen binding construct.

In some embodiments, a method for identifying tumor characteristics comprises providing $^{18}F$ to a subject, conducting a PET scan of the subject to identify $^{18}F$ distribution within the subject to render a representation of a tumor, providing a $^{89}Zr$ labeled CD8 antigen binding construct to the subject, conducting a PET scan of the subject to identify elevated concentrations of $^{89}Zr$ within the subject in a representation of CD8 distribution in the subject, and comparing the representation of the tumor with the representation of CD8 distribution to identify areas of overlap in the two representations. The areas of tumor location and elevated concentrations of $^{89}Zr$ indicate tumors that are inflamed and do not require an IOT and wherein areas where there are tumors, but no elevated concentrations of $^{89}Zr$ represent tumors that are not inflamed and require an IOT to adjust their TIL status.

In some embodiments, a method for identifying tumor characteristics comprises providing $^{18}F$ to a subject.

In some embodiments, a method for identifying tumor characteristics comprises providing $^{18}F$ to a subject, and conducting a PET scan of the subject.

In some embodiments, a method for identifying tumor characteristics comprises providing $^{18}F$ to a subject, and conducting a PET scan of the subject to identify $^{18}F$ distribution within the subject.

In some embodiments, a method for identifying tumor characteristics comprises providing $^{18}F$ to a subject, and conducting a PET scan of the subject to identify $^{18}F$ distribution within the subject to render a representation of a tumor.

In some embodiments, a method for identifying tumor characteristics comprises providing $^{18}F$ to a subject, and conducting a PET scan of the subject to identify $^{18}F$ distribution within the subject to render a representation of a tumor, and providing a $^{89}Zr$ labeled CD8 antigen binding construct to the subject. In some embodiments, a method for identifying tumor characteristics comprises providing $^{18}F$ to a subject, and conducting a PET scan of the subject to identify $^{18}F$ distribution within the subject to render a representation of a tumor, and providing a $^{89}Zr$ labeled CD8 antigen binding construct to the subject, and conducting a PET scan of the subject to identify elevated concentrations of $^{89}Zr$ within the subject in a representation of CD8 distribution in the subject.

In some embodiments, a method for identifying tumor characteristics comprises providing $^{18}F$ to a subject, and conducting a PET scan of the subject to identify $^{18}F$ distribution within the subject to render a representation of a tumor, and providing a $^{89}Zr$ labeled CD8 antigen binding construct to the subject, and conducting a PET scan of the subject to identify elevated concentrations of $^{89}Zr$ within the subject in a representation of CD8 distribution in the subject, and comparing the representation of the tumor with the representation of CD8 distribution to identify areas of overlap in the

two representations.

In some embodiments of the method for identifying tumor characteristics, the areas of tumor location and elevated concentrations of $^{89}Zr$ indicate tumors that are inflamed and do not require an IOT and wherein areas where there are tumors, but no elevated concentrations of $^{89}Zr$ represent tumors that are not inflamed and require an IOT to adjust their TIL status. In some embodiments of the method for identifying tumor characteristics, $^{18}F$ is administered to the subject, or the MRI scan is conducted, at a first time point and wherein $^{89}Zr$ is administered to the subject within 10 days after the first time point.

In some embodiments of the method for identifying tumor characteristics, $^{18}F$ is administered to the subject, or the MRI scan is conducted, at a first time point and wherein $^{89}Zr$ is administered to the subject within 1, 2, 3, 4, 5, 6, 7, 8, 9, or 10 days after the first time point, or a value within a range defined by any two of the aforementioned values. In some embodiments, each of the images is represented to a user via a display. In some embodiments, one or more of the images is stored in memory or recorded onto a computer readable medium. In some embodiments, the first and second images are each assigned different colors so that their overlap, or lack thereof, of signal and/or signal intensity, is readily visualized in the third PET image. In some embodiments, any imaging option can be used as an alternative to the second PET imaging vis FDG, as long as it indicates a local activity or presence of cancerous or neoplasia in the subject.

In some embodiments, the FDG image presents or defines the area of the tumor or neoplasia in the subject. The overlap of the first imaging (the CD8 based image) allows one to determine the degree or amount of TIL within the defined tumor or neoplasia. Thus, when the detectable marker for the first image (CD8) is absent from the area defined in the subject as the tumor or neoplasia (via the second image, such as an FDG PET), one can identify that neoplasia as being as CD8 desert or CD8 excluded (e.g., very few, if any CD8+ T cells are present within the tumor). Alternatively, if the first image indicates that CD8 is only present on a perimeter of the area defined as the neoplasia, then the tumor can be identified as an immune excluded tumor, with respect to CD8+ T cells. Finally, when the first image indicates that CD8 is present throughout the area defined as the neoplasia, then the tumor can be identified as being TIL infiltrated.

In some embodiments, a method of monitoring CD8 distribution in a human subject is provided. The method comprises providing a first PET scan, wherein the first PET scan comprises a CD8 minibody PET scan, providing a second PET scan, wherein the second PET scan comprises a fluorodeoxyglucose ("FDG") PET scan and when there is co-localization of the first and second PET scan, then treating the subject with a therapy that is appropriate for a TIL neoplasia, and when there is no significant co-localization of the first and second PET scan, then treating the subject with a therapy that is appropriate for an immune excluded tumor or an immune desert tumor.

In some embodiments, the treatment that is appropriate for a TIL positive neoplasia is at least one of: an immunotherapy, including strategies aimed to destroy the tumor by increasing T-cell activity (e.g., PD-L1, MEK, FAP-IL2v, VEGF, CEA-IL2v, CSf-1R, A2A, TIGIT, CD38 therapies) or redirecting and engaging T cells (e.g., CEA CD3 or CD20 CD3 therapies). Any of these therapies can be used for any of the methods provided herein, when the tumor is indicated as TIL positive (or similar term). In some embodiments, the therapies include external beam and targeted radiotherapy along with standard chemotherapy. In some embodiments, a therapy that has been shown to increase or modulate T-cells can be employed. In some embodiments, CART therapy can be used. In some embodiments, it can include any treatment that affects CD8+ T cells presence, activation, number or trafficking in relation to tumor lesions. This may include chemotherapies, oncolytic viruses, vaccines, radiation therapy, cellular therapies etc. In some embodiments, when one is converting a tumor from TIL negative to TIL positive, one can continue varying different therapeutics, amounts methods, combinations, etc., until one converts the tumor from TIL negative (e.g., immune excluded or immune desert) to TIL positive.

In some embodiments, the treatment that is appropriate for an immune excluded is one or more of an approach that will allow infiltration of the tumor by overcoming the stromal barrier (e.g., hyaluronan), recruiting T cells to the tumor (e..g., VEGF and CXCR4 therapies) or redirecting and engaging T cells (e.g., CEA CD3 or CD20 CD3 therapies). Any of these therapies can be used for any of the methods provided herein, when the tumor is indicated as immune excluded (or similar term). In some embodiments, the treatment that is appropriate for an immune desert neoplasia is one or more of: a strategy to increase T-cell immunity via increased T-Cell priming, recruitment and redirection by enhancing antigen generation and presentation, generatin/releasing/delivering antigens (including, but not limited to: NY-ESO-1, CD19, HDAC, EZH2, DNMT, HER2, BRAF, EGFR-TKI, ALK, PARP, MEK therapies), enhancing antigen presentation and T-cell priming (CD40 therapies), and redirecting and engaging T cells (e.g., CEA CD3 and CD20 CD3 therapies). Any of these therapies can be used for any of the methods provided herein, when the tumor is indicated as immune desert (or similar term). In some embodiments, immune excluded denotes that anti-tumor T cells accumulate at the tumor site by fail to efficiently infiltrate the tumor microenvironment, T cells are rendered ineffective by their inability to infiltrate the tumor stroma. In some embodiments immune desert denotes that there is a lack of pre-existing immunity and there is no effective generation of tumor-specific T cells. In some embodiments, TIL positive or "inflamed" denotes that T cells are infiltrating the tumor, and there is a pre-existing immunity at the tumor site. In some embodiments, vaccines can be used for either of these options and/or modulation of mesenchymal-epithelial transition.Antitumor T cells accumulate at

the tumor site but fail to efficiently infiltrate the tumor microenvironment. T cells are rendered ineffective by their inability to infiltrate the tumor stroma. Therefore, the rate-limiting step is T-cell penetration through the tumor stromaAntitumor T cells accumulate at the tumor site but fail to efficiently infiltrate the tumor microenvironment. T cells are rendered ineffective by their inability to infiltrate the tumor stroma. Therefore, the rate-limiting step is T-cell penetration through the tumor stroma Antitumor T cells accumulate at the tumor site but fail to efficiently infiltrate the tumor microenvironment. T cells are rendered ineffective by their inability to infiltrate the tumor stroma. Therefore, the rate-limiting step is T-cell penetration through the tumor stroma Antitumor T cells accumulate at the tumor site but fail to efficiently infiltrate the tumor microenvironment. T cells are rendered ineffective by their inability to infiltrate the tumor stroma. Therefore, the rate-limiting step is T-cell penetration through the tumor stroma

In some embodiments, a method of characterizing a tumor is provided. The method comprises applying an antigen-binding construct that binds to human CD8 to a human subject (such as a minibody provided herein), wherein the antigen-binding construct comprises a detectable marker. The method further comprises monitoring a distribution of the detectable marker, generating an image based on the distribution of the detectable marker, and displaying the image to a user in a manner to allow the user to characterize the tumor.

In some embodiments, one or more of the images provided or generated herein is stored to memory or displayed to a user and/or processed from PET data or combined with other PET data. In some embodiments, the image is stored in a non-transitory computer readable media.

In some embodiments, multiple PET scans can be performed, for example, at least 2 to 300 times. In some embodiments, the dose and/or method allows for a PET scan every 0.25, 0.5, 1, 2, 3, 4, 5, or 6 months until the subject is cancer free. In some embodiments, the lower radiation doses, such as less than 1 miC (e.g., 0.7 or o.75) can allow for continued monitoring of a subject for any of the methods provided herein.

In some embodiments, a method of generating an image is provided. The method comprises applying an antigen-binding construct that binds to CD8, wherein the antigen-binding construct comprises a detectable marker. The method further comprises detecting at least one detectable marker within a location within the tumor and assigning a positive pixel for each detectable marker detected. The method further comprises generating an image based on a distribution of each positive pixel assigned, wherein the image is stored in a non-transitory computer readable media.

In some embodiments, a method of positron emission tomography ("PET") is provided. The method comprises administering a tracer that binds to CD8 to a subject, providing a scintillator, using the scintillator to detect a pair of photons created by the tracer, using detection of the pair of photons to localize a source of the tracer via a list of coincidence events that are processed via a processor that is configured to take an output from the scintillator and convert it to the list of coincidence events. In some embodiments, the tracer comprises a CD8 minibody with a detectable marker or label. In some embodiments, between about 300 and about 500 CD8 positive cells can be detected per $mm^2$ of a tissue or neoplasia within the subject (e.g., in a 20 micron thick section). Any other range of cell count provided herein can also be applied to this method.

In some embodiments, a method of imaging a subject is provided. The method comprises administering a first dose of any one of the compositions provided herein, imaging the subject to obtain a first image, administering a second dose of any one of the compositions provided herein and imaging the subject to obtain a second image, and comparing the first image to the second image.

In some embodiments, the comparison assists in assessing a treatment response of the subject. This can show, for example, changes in tumor status (between TIL positive or immune excluded and/or immune desert) or a change in size or number of tumors, for example, when measuring the effectiveness of a particular drug or therapy in a subject.

In some embodiments, the comparison is used to determine a therapeutic dose to be provided to the subject. For example, a first potential dose is administered to the subject after the first image is obtained. The second image can then be compared to the fist to determine if the change is favorable or not at the given dose. The dose can be adjusted upwards or downwards after that and further images can be obtained. This can be used to test any variable of treatment, including, for example, formulations, concentrations, combinations, frequency of administration, alternative compounds, additional excipients, timing of administration, additional activities that the subject is undergoing during the therapy, etc. In some embodiments, the methods provided herein can be used to see if one can shift the phenotype of a tumor from or between immune desert, immune excluded, and/or TIL positive (or inflamed). In some embodiments, the methods provided herein can be used to see the effectiveness of a specific therapeutic compound on the treatment of the tumor to reduce or kill the tumor itself. In some embodiments, both goals can be employed.

In some embodiments, a pre-treatment baseline CD8 PET scan can inform one of the TIL status of tumor lesions. In addition, a post-treatment CD8 PET scan, acquired within 1-8 weeks after any CD8 T-cell affecting treatment, can detect the effect of the treatment on TIL status compared to baseline. Such a functional change in TIL status can correlate with eventual treatment response and clinical outcome for the patient. A CD8 PET scan, thus, can predict outcomes significantly earlier than currently detected with standard of care follow up imaging with CT, MRI or FDG PET scans.

In some embodiments, rather than simply scanning a single tumor/neoplasia or cluster thereof, the process is applied to the entire body or relevant section of the subject. With the lower doses of radiation and higher effectiveness of the CD8 construct, when combined with the relevant methods, visualization need not be limited to just one section of the subject, and instead, the antigen binding construct can be applied in sufficient levels and in an appropriate manner so as to distribute the antigen binding construct (such as the minibodies provided herein) across or throughout the subject. In some embodiments, when the majority of the tumors in the body are imaged, a more complete and/or effective prediction can be made and decision regarding the appropriate treatment. In some embodiments, at least 2 tumors are imaged at the same time, for example, 2, 5, 10, 20, 30, 40, 50, 60, 70, 80, 90 or 100 or more tumors are imaged in single PET or scanning session. In some embodiments, at least 10% of the subject's volume is scanned in a single session, for example, 10, 20, 30, 40, 50, 60, 70, 80, 90, 95, 96, 97, 98, 99, or 100% of the subject's volume is scanned in a single session. These sessions, and the number of tumors and/or present of subject's volume, can be scanned multiple times (e.g., weekly, biweekly, monthly, every two, or every three months, for example.

## TREATMENT-COMBINATION OF IMAGES PET/MRI

Some embodiments provided herein allow for one to monitor how effectively any one therapy is functioning in a subject by monitoring the distribution of CD8, either by itself, or in combination with other imaging techniques. Some embodiments provided herein allow for one to combine various CD8 imaging techniques.

In some embodiments, a method of treatment of a human subject having a tumor comprises providing a PET and a) MRI or b) CT combined image of the subject,wherein the combined image emphasizes an overlap in an area of at least one tumor and a CD8 region of interest (ROI), wherein the area of at least one tumor is defined by a) MRI data or b) CT data and the CD8 ROI is defined by PET data, reviewing the PET and a) MRI or b) CT combined image to determine a TIL status of a tumor in a patient, and providing an IOT treatment to the patient if the tumor is TIL negative.

In some embodiments, a method of treatment of a human subject having a tumor comprises providing a PET and MRI combined image of the subject. In some embodiments, a method of treatment of a human subject having a tumor comprises providing a PET and CT combined image of the subject. In some embodiments, a method of treatment of a human subject having a tumor comprises providing a PET and MRI and/or CT combined image of the subject. In some embodiments, a method of treatment of a human subject having a tumor comprises providing a PET and MRI combined image of the subject,wherein the combined image emphasizes an overlap in an area of at least one tumor and a CD8 ROI. In some embodiments, a method of treatment of a human subject having a tumor comprises providing a PET and CT combined image of the subject,wherein the combined image emphasizes an overlap in an area of at least one tumor and a CD8 ROI. In some embodiments, a method of treatment of a human subject having a tumor comprises providing a PET and MRI and/or CT combined image of the subject,wherein the combined image emphasizes an overlap in an area of at least one tumor and a CD8 ROI.

In some embodiments, a method of treatment of a human subject having a tumor comprises providing a PET and MRI and/or CT combined image of the subject,wherein the combined image emphasizes an overlap in an area of at least one tumor and a CD8 ROI, wherein the area of at least one tumor is defined by MRI data and the CD8 ROI is defined by PET data.

In some embodiments, a method of treatment of a human subject having a tumor comprises providing a PET and MRI and/or CT combined image of the subject,wherein the combined image emphasizes an overlap in an area of at least one tumor and a CD8 ROI, wherein the area of at least one tumor is defined by CT data and the CD8 ROI is defined by PET data.

In some embodiments, a method of treatment of a human subject having a tumor comprises providing a PET and MRI and/or CT combined image of the subject,wherein the combined image emphasizes an overlap in an area of at least one tumor and a CD8 ROI, wherein the area of at least one tumor is defined by MRI data or CT data and the CD8 ROI is defined by PET data, reviewing the PET and MRI combined image to determine a TIL status of a tumor in a patient.

In some embodiments, a method of treatment of a human subject having a tumor comprises providing a PET and MRI and/or CT combined image of the subject,wherein the combined image emphasizes an overlap in an area of at least one tumor and a CD8 ROI, wherein the area of at least one tumor is defined by MRI data or CT data and the CD8 ROI is defined by PET data, reviewing the PET and CT combined image to determine a TIL status of a tumor in a patient.

In some embodiments, a method of treatment of a human subject having a tumor comprises providing a PET and MRI and/or CT combined image of the subject,wherein the combined image emphasizes an overlap in an area of at least one tumor and a CD8 ROI, wherein the area of at least one tumor is defined by MRI data or CT data and the CD8 ROI is defined by PET data, reviewing the PET and MRI or CT combined image to determine a TIL status of a tumor in a patient, and providing an IOT treatment to the patient if the tumor is TIL negative.

**TREATMENT-SPECIFIC TUMOR**

In some embodiments, a method of treatment of a human subject having cancer comprises administering a CD8 antigen binding construct to a subject, wherein the CD8 antigen binding construct is labeled with a detectable label, identifying at least a primary tumor and a secondary tumor status within the subject's body, identifying, via a distribution of the CD8 antigen binding construct the TIL status of the primary tumor, administering a first therapy to the subject based on the TIL status of the primary tumor,administering a CD8 antigen binding construct to the subject to determine a subsequent TIL status of the primary tumor, and using the TIL status of the primary tumor to determine a subsequent appropriate treatment for the subject. In some embodiments, a method of treatment of a human subject having cancer comprises administering a CD8 antigen binding construct to a subject.

In some embodiments, a method of treatment of a human subject having cancer comprises administering a CD8 antigen binding construct to a subject, wherein the CD8 antigen binding construct is labeled.

In some embodiments, a method of treatment of a human subject having cancer comprises administering a CD8 antigen binding construct to a subject, wherein the CD8 antigen binding construct is labeled with a detectable label.

In some embodiments, a method of treatment of a human subject having cancer comprises administering a CD8 antigen binding construct to a subject, wherein the CD8 antigen binding construct is labeled with a detectable label, and identifying at least a primary tumor status within the subject's body.

In some embodiments, a method of treatment of a human subject having cancer comprises administering a CD8 antigen binding construct to a subject, wherein the CD8 antigen binding construct is labeled with a detectable label, and identifying at least a primary tumor and a secondary tumor status within the subject's body.

In some embodiments, a method of treatment of a human subject having cancer comprises administering a CD8 antigen binding construct to a subject, wherein the CD8 antigen binding construct is labeled with a detectable label, and identifying at least a primary tumor status within the subject's body, and identifying, via a distribution of the CD8 antigen binding construct the TIL status of the primary tumor.

In some embodiments, a method of treatment of a human subject having cancer comprises administering a CD8 antigen binding construct to a subject, wherein the CD8 antigen binding construct is labeled with a detectable label, and identifying at least a primary tumor status within the subject's body, and identifying, via a distribution of the CD8 antigen binding construct the TIL status of the primary tumor, and administering a first therapy to the subject based on the TIL status of the primary tumor.

In some embodiments, a method of treatment of a human subject having cancer comprises administering a CD8 antigen binding construct to a subject, wherein the CD8 antigen binding construct is labeled with a detectable label, and identifying at least a primary tumor status within the subject's body, and identifying, via a distribution of the CD8 antigen binding construct the TIL status of the primary tumor, and administering a first therapy to the subject based on the TIL status of the primary tumor, and administering a CD8 antigen binding construct to the subject to determine a subsequent TIL status of the primary tumor.

In some embodiments, a method of treatment of a human subject having cancer comprises administering a CD8 antigen binding construct to a subject, wherein the CD8 antigen binding construct is labeled with a detectable label, and identifying at least a primary tumor status within the subject's body, and identifying, via a distribution of the CD8 antigen binding construct the TIL status of the primary tumor, and administering a first therapy to the subject based on the TIL status of the primary tumor, and administering a CD8 antigen binding construct to the subject to determine a subsequent TIL status of the primary tumor, and using the TIL status of the primary tumor to determine a subsequent appropriate treatment for the subject.

In some embodiments, a method of treatment of a human subject having cancer comprises administering a CD8 antigen binding construct to a subject, wherein the CD8 antigen binding construct is labeled with a detectable label, and identifying at least a primary tumor and a secondary tumor status within the subject's body, and identifying, via a distribution of the CD8 antigen binding construct the TIL status of the primary tumor, and administering a first therapy to the subject based on the TIL status of the primary tumor, and administering a CD8 antigen binding construct to the subject to determine a subsequent TIL status of the primary tumor, and using the TIL status of the primary tumor to determine a subsequent appropriate treatment for the subject.

In some embodiments of a method of treatment of a human subject having cancer, the TIL status of the secondary tumor is also determined subsequent to the first therapy. In some embodiments of a method of treatment of a human subject having cancer the TIL status of the secondary tumor is also determined subsequent to the first therapy, but the TIL status is not used for the treatment of the primary tumor. In some embodiments of a method of treatment of a human subject having cancer,the TIL status of the primary tumor indicates that the first therapy is effective for the first tumor. In some embodiments of a method of treatment of a human subject having cancer, the TIL status of the primary tumor indicates that the first therapy is effective for the first tumor, and thus, the first therapy is continued.

In some embodiments of a method of treatment of a human subject having cancer, the first therapy is not effective for the secondary tumor. In some embodiments of a method of treatment of a human subject having cancer,the

first therapy is not effective for the secondary tumor, and the first therapy is continued.

In some embodiments of a method of treatment of a human subject having cancer, the first therapy is not effective for the primary tumor.

In some embodiments of a method of treatment of a human subject having cancer, the first therapy is not effective for the primary tumor, but is effective for the secondary tumor.

In some embodiments of a method of treatment of a human subject having cancer, the first therapy is not effective for the primary tumor, but is effective for the secondary tumor, and the method further comprises administering a second therapy to the subject.

## ADDITIONAL EMBODIMENTS OF MONITORING/TREATING

Any of the embodiments provided herein can include one or more, or replace one or more component noted herein with one or more of the following embodiments and/or alternatives.

In any of the embodiments provided herein, in order to determine the importance of the SUV value (peak, mean, change, etc.), it is compared to a standard or reference value. In some embodiments, this standard or reference value can be a set or predefined value. In some embodiments, the standard or reference value is from the PET image itself, but another location. In some embodiments, the standard or reference is from the PET image and is one or more of the level at the: liver, spleen, bone marrow (L3 vertebrae), skeletal muscle (right paraspinal muscle at L5) and/or aorta. In some embodiments, this is a visual comparison in terms of the degree of change in one of the reference areas, compared to the degree of change at the tumor. Thus, any of the embodiments provided herein, where one is looking at a change in the detetctable marker (signal, CD8 antigen binding, etc.), one can look at the change in the tumor location, in comparison to a change (or the absence of any change) at one or more of: liver, spleen, bone marrow (L3 vertebrae), skeletal muscle (right paraspinal muscle at L5) and/or aorta.

As provided herein, using these CD8 antigen binding constructs, methods, and formulations can be useful to determine the CD8 status of a neoplasia. This information can then be employed for various methods, for example, to determine if, generally, an immunotherapy is a good match for a subject (e.g., is the neoplasia TIL positive). Alternatively, this information can be used for screening various therapies in a subject, to determine if a particular immunotherapy is effective in a subject (e.g., the neoplasia is initially immune excluded or an immune desert, but changes to TIL positive (or inflamed) when the subject receives the immunotherapy. Generally speaking, any of the methods provided (for monitoring or treating, etc.) can be used for either arrangement. Thus, the disclosure of one in a particular situation or embodiment is also intended to disclose the other (in the corresponding particular situation or embodiment).

In some embodiments, monitoring CD8 is achieved as a function of determining: a) distribution of detectable marker at an external margin of the tumor which demonstrates an increase in binding at the external margin as compared to an internal area or volume of the tumor, b) a distribution of detectable marker at an internal area or volume of the tumor (e.g., throughout the tumor); or c) a distribution of detectable marker that is not significantly biased or linked to the location of the tumor (e.g., an immune desert). In some embodiments, if a) then one provides the subject with a non-immunotherapy based therapy. If b) or c) then one provides the subject with an immunotherapy based therapy. This type of approach does not require the use of immunotherapy in all situations, but allows one to determine if immunotherapy is appropriate for the subject. In other embodiments, one can monitor CD8 for determining how well a particular immunotherapy is working in converting options b) or c) (situations where immunotherapy are not functioning) to option a) (where the immunotherapy is functioning for the subject).

In some embodiments, a) is defined as having at least 1% greater presence of marker at an outermost perimeter of the tumor, wherein the outermost perimeter of the tumor is no more than (for example) 1, 2, 4, 5, 6, 7, 8, 9, or 10% of an area of an image of the tumor. In some embodiments, this is determined as a count density per area on an image.

In some embodiments, any relative difference between the various distribution patterns can be considered in characterizing the type of tumor. In some embodiments, a) is at least 10, 15, 20, 25, 30, 35, 40, 45, 50, 60, 70, 80, 90, or 100% of an increase is present in one scenario vs the other (e.g., perimeter bias vs consistent throughout, vs absent). In some embodiments, the increase in a) is at least 2, 3, 4, 5, 6, 7, 8, 9, 10, 15, 20, 30, 40, 50 fold or greater. In some embodiments, b) is at least 10, 15, 20, 25, 30, 35, 40, 45, 50, 60, 70, 80, 90, or 100% of an increase. In some embodiments, the increase in b) is at least 2, 3, 4, 5, 6, 7, 8, 9, 10, 15, 20, 30, 40, 50 fold or greater. The increase in b) can be an increase in marker or signal from the marker on the inside of the neoplasia compared to the outside or non-neoplasia tissue surrounding the neoplasia. In some embodiments, the comparison can be of the tumor area to a known control, that is, for example, known to be tumor free. In some embodiments, the comparison can also be made to a positive control, or to a tissue that is known to meet the conditions of a), b), or c). In some embodiments, a) is representative of an immune exclusive tumor. In some embodiments, b) is representative of a TIL tumor (or inflamed). In some embodiments, c) is representative of an immune desert tumor.

In some embodiments, the CD8 signal is indicative of the number of CD8+ T cells, which may, or may not, localize to a neoplasia or tumor. The co-localization can be determined via any technique or assay that allows one to

identify a tumor or neoplasia mass. In some embodiments, the distribution of the CD8 molecules (or signal, or pixels within in an image or data representing such information within an electronic file) is used to determine CD8 T cell distribution and/or whether or not a tumor or neoplasia is CD8+ and/or infiltrated, or degree of infiltration of the tumor or change in infiltration of the tumor via CD8+ T cells.

In some embodiments, b) is defined as having no more difference in image intensity between an inner area and an outer area than 100%, wherein the outer area is defined as an outermost perimeter of the tumor that is no more than 10% of an area of an image of the tumor. That is, b) indicates that there is relatively minor or insignificant distribution of the CD8 within the tumor compared to the external surface of the tumor. In other words, cells with CD8 are capable of infiltrating the neoplasia. In some embodiments, as long as infiltration is detectable, then b) can apply. In some embodiments, one monitors the change in degree of b) (e.g., is the distribution between the perimeter and the inner section improving (becoming more even) or decreasing (become more biased such that less CD8 is present within the tumor.

Any of the embodiments provided herein can employ any or all of the parameters for the PET scan process as shown in Table 0.1A below, or any other set of parameters, as appropriate.

Table 0.1A

| | |
|---|---|
| **Radiopharmaceutical** | $^{89}$Zr-Df-IAB22M2C |
| **Dose** | 3(±20%) mCi of $^{89}$Zr-Df-IAB22M2C administered over 5-10 minutes I.V. |
| **Mode** | 2D, 3D list-mode, or TOF<br>When possible, acquisition should be acquired in List Mode. |
| **Patient Position** | Supine with arms down by side. Keep position consistent between CT and PET acquisitions. |
| **Scan Location/Coverage** | Vertex of Skull through feet including all areas of known or suspected disease |
| **Transmission Scan** | • 140 kVp<br>• mAs:<br>◦ 24(±3) hrs. p.i. scans: low dose CT (40-80 mAs)<br>◦ Optional visit 5 post infusion scan: low dose CT (10 mAs)<br>• 0.8 sec per CT rotation<br>• 3-5 Millimeter (mm) slice thickness<br>• Table speed of 15 mm/rotation<br>• Pitch: 1:5:1 |
| **Emission Scan** | 2-7 min/bed position<br>Depending on the height of the subject, a single bed position may provide adequate anatomic coverage. |
| **Image Reconstruction** | Reconstruction algorithms that are consistent and tailored to provide equal quality images by NMCTG were used. |
| **Matrix Size** | Matrix size defined in the NMCTG repot (256x256) |
| **Views** | Non-attenuation corrected and Attenuation corrected |

In some embodiments provided herein, a relative "intensity" is described of a signal. Another option for describing this is as signal distribution. For TIL positive situations, the detectable marker can be found throughout the tumor. That is, there is relatively little bias in the signal distribution throughout the tumor and the detectable marker is within the tumor. For an immune exclude situation, the detectable marker for the antigen binding construct is biased in its distribution to a perimeter of the tumor or neoplasia. In an immune desert, the detectable marker for the antigen binding construct is biased such that there is less inside any part of the tumor, than outside the tumor. Any of the embodiments provided herein can be described in terms of relative intensities or signal intensities or distributions or signal distributions. In

addition, it is to be understood that embodiments that are described in terms of "signals" or "pixel" also disclose the actual location of the antigen binding constructs and/or the detectable markers. Thus, the terms can be exchanged for one another throughout the application, as appropriate.

In some embodiments, distinction between the outer perimeter and the inner area (distinguishing a from b) can be defined by known TIL positive tumors vs known CD8 excluded tumors. In some embodiments, the distinction can be numerical, for example, the outer most 1, 2, 3, 4, 5, 6, 7, 8, 9, or 10% of a tumor can be defined as the perimeter, and the remaining area, surrounded by this outer area, can be the internal area. Other values can be used as appropriate. In some embodiments, ratios or percents between the perimeter and internal area can be used. For example, if 50, 60, 70, 80, 90, 95, 96, 97, 98, 99, or 100% of the signal or distribution of the CD8 (or CD8 label or marker) is present at the perimeter, then, depending on the situation, the tumor may be more a) (excluded) than b) (TIL). Of course, when there is no significant signal at all within the tumor, then the tumor is still option c) (immune desert).

In some embodiments, the image or analysis can be both a PET scan and a CT image. That is, for any of the embodiments provided herein, any reference to "PET" as far as imaging is concerned, is also contemplated to include the further addition of a CT image in combination with a PET image (e.g., a a fused PET/CT image or scan process or result). Thus, in some embodiments, the methods provided herein that reference a scan or scanning process or the like, can include a PET scan or a PET and CT scan. For various embodiments, the CT scan will allow for better information regarding a particular organ.

In some embodiments, when monitoring the effectiveness of a therapy, one can monitor the status of tumor and change in status of the tumor (e.g., TIL positive, immune excluded (just a perimeter) or immune desert (no association of T cells at all with the tumor). In some embodiments, one looks at the change within each category, for example, is the tumor becoming more or less TIL or immune excluded. In some embodiments, any positive change (towards TIL) will indicate that the therapy is working for such a transition. In some embodiments, the change is at least 5, 10, 20, 30, 40, 50, 60, 70, 80, 90, 100%. In some embodiments, the change is at least 2, 3, 4, 5, 10, 20, 30, 40, 50, 60, 70, 80, 90 or 100 fold. In some embodiments, any negative change (away from TIL, or towards immune excluded) will indicate that the therapy is not working. In some embodiments, the change is at least 5, 10, 20, 30, 40, 50, 60, 70, 80, 90, 100%. In some embodiments, the change is at least 2, 3, 4, 5, 10, 20, 30, 40, 50, 60, 70, 80, 90 or 100 fold.

In some embodiments, monitoring is conducted within 6 hours of administering. In some embodiments, monitoring is conducted within 8 hours of administering. In some embodiments, monitoring is done between 1 and 48 hours of administering. For example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48, including any range defined between any two of the preceding values. As will be appreciated, earlier monitoring, (e.g., 5 hours or earlier, including with administration) can be done to obtain a negative control, or determine if there is any label or marker from a previous administration remaining. Typically, 6-36 hours will provide the right amount of time for distribution of the marker throughout the tissue of interest.

In some embodiments, more than one monitoring step can be performed, e.g., 2, 3, 4, 5, 6, 7, or more times. In some embodiments, the monitoring or a second monitoring is done before 36 hours from administering. The same can be applied to other repetitions of monitoring steps. In some embodiments, subsequent monitoring steps involve the readministration of the antigen binding construct.

In some embodiments, multiple antigen binding constructs can be applied at the same time. In some embodiments, other diagnostics, such as FDG and/or 18F, can also be administered to the subject, so that the tumor can be imaged and/or co-localized with the CD8 data. In some embodiments, any option for imaging or visualizing a tumor or neoplasia can be combined with the present approach for CD8 imaging, so that one can co-localize CD8 to a tumor.

All of the methods of monitoring and/or treatment provided herein are also contemplated and can be recast in terms of the subject's perspective. Thus, any of the embodiments provided herein can be recharacterized from the subject's perspective, as, for example receiving, rather than providing or administering etc. Thus, in some embodiments, the subject first receives and goes through a monitoring process, and then receives an appropriate therapy based upon the particular tumor type. Similarly, in some embodiments, the subject first receives a first test therapeutic, and has the effectiveness of the first therapeutic monitored to determine if they should receive (and do receive) more of the first therapeutic or an alternative therapeutic.

In some embodiments, monitoring is achieved via providing a first PET scan. In some embodiments, the first or second or subsequent monitoring is achieved via an alternative scan, such as a CT scan. In some embodiments, any scanning system or method that allows one to monitor the CD8 antigen binding constructs can be used. In some embodiments, this can be combined with any scanning system or method that allows one to monitor or determine the location and shape of the tumor or neoplasia, as long as the results from the two systems can be combined to allow one to determine whether or not there is co-localization.

In some embodiments, monitoring further comprises providing a second PET fluorodeoxyglucose ("FDG") PET scan. Any scan that allows tumor imaging can be employed, in some embodiments.

In some embodiments, the first scan and the second scan are compared to determine if there is co-localization

of the CD8 in a neoplasia. In some embodiments, the first and second scan are compared to determine if a therapy administered to a subject is improving the health of a subject, or changing the immune status of the tumor (from, for example, immune excluded to TIL positive).

In some embodiments, the neoplasia is a known tumor. In some embodiments, the known tumor is selected from any one of more of the cancer types provided herien.

In some embodiments, the treatment comprises: a) directly treating the tumor according to the status of the tumor identified (e.g., TIL positive appropriate therapy for a TIL positive tumor or a immune desert appropriate therapy for a immune desert tumor, or b) ongoing therapies (that can be varied over time while monitoring) to convert an immune desert tumor to a immune excluded or TIL positive therapy (at which point the appropriate therapy is given) or to convert an immune excluded tumor to a TIL positive tumor (at which point a TIL therapy can be administered to the subject). Any of the therapies provided herein can be administered as appropriate, for the particular tumor status.

In some embodiments, a CD8 distribution determination comprises determining CD8 positive cell density observed by PET. In some embodiments, a CD8 determination is done by imaging CD8 positive T cells within the subject, via PET.

In some embodiments, a method of treating a subject is provided. The method comprises administering to a patient that has a neoplasia a human minibody that binds to human CD8, monitoring a distribution of the human minibody to determine a first tumor-infiltrating lymphocyte ("TIL") status within the neoplasia, and treating the patient based upon at least the first TIL status (e.g., is it an immune desert, immune excluded, or TIL positive) of the neoplasia.

In some embodiments, the subject is receiving a therapy between a first TIL status determination and a second TIL status determination. The effectiveness of the therapy on TIL status can then be determined. The therapy can also be altered, either in the first round, or after the second TIL status determination, to see how to improve and/or customize a therapy for the subject. Any variable can be changed and monitored via this process, including, for example, the timing of doses, the amount of the dose, combinations of different therapies, a shift in therapy based upon the status of the tumor (to, for example, TIL status when it had been an immune desert tumor). In some embodiments, when the therapy does not function, or function as desired, the first therapy is stopped and a second, different therapy, is then administered. In some embodiments, the first therapy is not stopped, but a second and/or additional number of therapies are added. Thus, the method can allow one to monitor the effectiveness of a therapy, and then select a better therapy if the first therapy is not working (either as a direct treatment or as an option to transform an immune excluded and/or immune desert tumor or neoplasia to a TIL positive tumor or neoplasia.

## PSEUDOPROGRESSION

In some embodiments, another advantage of some of the methods provided herein is that they permit clinicians to distinguish tumor progression from pseudo-progression. The nature of the antitumor immune response can create the appearance of disease progression, either as tumor growth or appearance of new lesions. (1) This is known as pseudo-progression. Pseudo-progression does not reflect tumor cell growth, but may be misclassified as disease progression. (1) Psuedo-progression in fact reflects the acquisition of anti-tumour immunity, which may be induced by a therapeutic agent such as an immunotherapy. Tumors may appear to grow or new lesions may appear when immune cells infiltrate the tumor site. (1) Due to the time required to mount an adaptive immune response, pseudo-progression may also reflect continued tumor growth until a sufficient response develops. (1,3) It can be difficult to differentiate pseudo-progression from disease progression. (1) Histologic confirmation is not always possible. Some embodiments provided herein provide a convenient and rapid method to identify and distinguish pseudo-progression. Pseudo-progression is identified by observation of neoplasia conversion from TIL negative status to TIL positive status, while at the same time the neoplasia demonstrates continued growth in total volume. Patients will ideally continue with the existing treatment regime rather than changing, if the progression can be safely categorized as pseudo-progression. (1. Wolchok JD, Hoos A, O'Day S, et al. Guidelines for the Evaluation of Immune Therapy Activity in Solid Tumors: Immune-Related Response Criteria. Clin Cancer Res. 2009;15:7412-7420. 2. Chiou VL, Burotto M. Pseudoprogression and Immune-Related Response in Solid Tumors. J Clin Oncol. 2015;33:3541-3543. 3. Hales RK, Banchereau J, Ribas A, et al. Assessing oncologic benefit in clinical trials of immunotherapy agents. Ann Oncol. 2010;21:1944-1951.)

In some embodiments, a method of treating a human subject comprises administering a candidate therapeutic to a human subject,determining if a size of a tumor in the human subject has increased or decreased in response to the candidate therapeutic,if the size has increased following the candidate therapeutic, then using a CD8 antigen binding construct to determine if an amount of CD8 present within the tumor has increased or decreased in response to the candidate therapeutic, wherein an increase in tumor size without an increase in the amount of CD8 indicates tumor progression, whereas an increase in tumor size with an increase in the amount of CD8 indicates tumor pseudoprogression, wherein a treatment is continued if the patient is experiencing tumor pseudoprogression and wherein a treatment is changed if the patient is experiencing tumor progression. In some embodiments, a method of treating a human subject comprises administering a candidate therapeutic to a human subject.

In some embodiments, a method of treating a human subject comprises administering a candidate therapeutic to a human subject, and determining if a size of a tumor in the human subject has increased or decreased in response to the candidate therapeutic.

In some embodiments, a method of treating a human subject comprises administering a candidate therapeutic to a human subject, and determining if a size of a tumor in the human subject has increased or decreased in response to the candidate therapeutic, and if the size has increased following the candidate therapeutic, then using a CD8 antigen binding construct to determine if an amount of CD8 present within the tumor has increased or decreased in response to the candidate therapeutic.

In some embodiments, a method of treating a human subject comprises administering a candidate therapeutic to a human subject, and determining if a size of a tumor in the human subject has increased or decreased in response to the candidate therapeutic, and if the size has increased following the candidate therapeutic, then using a CD8 antigen binding construct to determine if an amount of CD8 present within the tumor has increased or decreased in response to the candidate therapeutic, wherein if there is an increase in tumor size without an increase in the amount of CD8, it indicates tumor progression.

In some embodiments, a method of treating a human subject comprises administering a candidate therapeutic to a human subject, and determining if a size of a tumor in the human subject has increased or decreased in response to the candidate therapeutic, and if the size has increased following the candidate therapeutic, then using a CD8 antigen binding construct to determine if an amount of CD8 present within the tumor has increased or decreased in response to the candidate therapeutic, wherein if there is an increase in tumor size with an increase in the amount of CD8, it indicates tumor pseudoprogression.

In some embodiments, a method of treating a human subject comprises administering a candidate therapeutic to a human subject, and determining if a size of a tumor in the human subject has increased or decreased in response to the candidate therapeutic, and if the size has increased following the candidate therapeutic, then using a CD8 antigen binding construct to determine if an amount of CD8 present within the tumor has increased or decreased in response to the candidate therapeutic, wherein if there is an increase in tumor size without an increase in the amount of CD8 it indicates tumor progression, and a treatment is changed if the patient is experiencing tumor progression.

In some embodiments, a method of treating a human subject comprises administering a candidate therapeutic to a human subject, and determining if a size of a tumor in the human subject has increased or decreased in response to the candidate therapeutic, and if the size has increased following the candidate therapeutic, then using a CD8 antigen binding construct to determine if an amount of CD8 present within the tumor has increased or decreased in response to the candidate therapeutic, wherein if there is an increase in tumor size with an increase in the amount of CD8, it indicates tumor pseudoprogression, and a treatment is continued if the patient is experiencing tumor pseudoprogression.

In some embodiments, a method of treating a human subject comprises administering a candidate therapeutic to a human subject, and determining if a size of a tumor in the human subject has increased or decreased in response to the candidate therapeutic, and if the size has increased following the candidate therapeutic, then using a CD8 antigen binding construct to determine if an amount of CD8 present within the tumor has increased or decreased in response to the candidate therapeutic, wherein an increase in tumor size without an increase in the amount of CD8 indicates tumor progression, whereas an increase in tumor size with an increase in the amount of CD8 indicates tumor pseudo-progression, wherein a treatment is continued if the patient is experiencing tumor pseudoprogression, and wherein a treatment is changed if the patient is experiencing tumor progression. In some embodiments of a method of treating a human subject, the patient is first identified as one suspected of having pseudo progression.

In some embodiments, a method of distinguishing tumor progression from tumor pseudoprogression after immunotherapy in a human patient comprises administering a CD8 antigen binding construct to a human subject, detecting a first distribution of the CD8 antigen binding construct within the subject and a first ROI, then administering a candidate immunotherapy to the subject,then detecting a second distribution of the CD8 antigen binding construct within the subject and a second ROI, wherein an increase in tumor size without an increase in CD8 binding indicates tumor progression, whereas an increase in tumor size with an increase in CD8 binding indicates tumor pseudoprogression, wherein a treatment is continued if the patient is experiencing tumor pseudoprogression and wherein a treatment is changed if the patient is experiencing tumor progression.

In some embodiments, a method of distinguishing tumor progression from tumor pseudoprogression after immunotherapy in a human patient comprises administering a CD8 antigen binding construct.

In some embodiments, a method of distinguishing tumor progression from tumor pseudoprogression after immunotherapy in a human patient comprises administering a CD8 antigen binding construct to a human subject.

In some embodiments, a method of distinguishing tumor progression from tumor pseudoprogression after immunotherapy in a human patient comprises administering a CD8 antigen binding construct to a human subject, and detecting a first distribution of the CD8 antigen binding construct within the subject.

In some embodiments, a method of distinguishing tumor progression from tumor pseudoprogression after immunotherapy in a human patient comprises administering a CD8 antigen binding construct to a human subject, and

detecting a first ROI.

In some embodiments, a method of distinguishing tumor progression from tumor pseudoprogression after immunotherapy in a human patient comprises administering a CD8 antigen binding construct to a human subject, and detecting a first distribution of the CD8 antigen binding construct within the subject and a first ROI, then administering a candidate immunotherapy to the subject.

In some embodiments, a method of distinguishing tumor progression from tumor pseudoprogression after immunotherapy in a human patient comprises administering a CD8 antigen binding construct to a human subject, and detecting a first distribution of the CD8 antigen binding construct within the subject and a first ROI, then administering a candidate immunotherapy to the subject, then detecting a second distribution of the CD8 antigen binding construct within the subject and a second ROI, wherein an increase in tumor size without an increase in CD8 binding indicates tumor progression, whereas an increase in tumor size with an increase in CD8 binding indicates tumor pseudoprogression, wherein a treatment is continued if the patient is experiencing tumor pseudoprogression, and wherein a treatment is changed if the patient is experiencing tumor progression.

In some embodiments, a method of distinguishing tumor progression from tumor pseudoprogression after immunotherapy in a human patient comprises administering a CD8 antigen binding construct to a human subject, and detecting a first distribution of the CD8 antigen binding construct within the subject and a first ROI, then administering a candidate immunotherapy to the subject, and then detecting a second distribution of the CD8 antigen binding construct within the subject and a second ROI, wherein an increase in tumor size without an increase in CD8 binding indicates tumor progression, whereas an increase in tumor size with an increase in CD8 binding indicates tumor pseudoprogression, wherein a treatment is continued if the patient is experiencing tumor pseudoprogression, and wherein a treatment is changed if the patient is experiencing tumor progression.

In some embodiments, a method of treating a human subject having a tumor is provided, which comprises administering a CD8 antigen binding construct to the subject and detecting a first distribution of the CD8 antigen binding construct within the subject and a first ROI; administering a candidate immunotherapy to the subject detecting a second distribution of the CD8 antigen binding construct within the subject and a second ROI, wherein, if the second ROI relative to the first ROI is the same or larger in area (relative to the subject) and demonstrates increased CD8 infiltration, then continuing with administration of the candidate immunotherapy; or if the second ROI relative to the first ROI is the same or larger in area (relative to the subject) and demonstrates no increase in CD8 infiltration, then discontinuing administration of the candidate immunotherapy.

In some embodiments, a first TIL status is one where the human minibody binds throughout a volume of the tumor, and a second TIL status one where the human minibody binds selectively to a tumor margin, a particular therapy or immunotherapy is applied to the subject if the subject has the second TIL status (e.g., immune excluded, or in other embodiments, immune desert). In some embodiments, the immunotherapy is not applied to the subject if the subject has the first TIL status, as their body is already targeting the tumor. In some embodiments, a therapy appropriate for targeting TIL positive tumors is used.

In some embodiments, a first TIL status is when the human minibody binds throughout a volume of all identified tumors (e.g., inflamed or TIL positive), and a second TIL status is when the human minibody binds selectively to any tumor margin without homogenously perfusing the volume of the tumor (e.g., immune excluded). "Binding throughout a volume" denotes that the tumor is not immune excluded or an immune desert. Instead, the tumor allows, into it, T cells at a level where they can be functional within a host. Often, this can be referred to as TIL positive or "inflamed". In contrast to a situation where binding occurs throughout a volume of the tumor, there are also situations where binding occurs "at the tumor margin" or "primarily at the margin". These are distinct scenarios. While it is technically correct that some amount of binding at a tumor margin is occurring for the TIL positive (or inflamed) scenario, it is approximately even with the presence of the T-cells within the tumor. In contrast, when something is characterized as binding "at the margin" it is designating that there is significantly more binding occurring at the margin than elsewhere. That is, it is designating that there is a bias in localization to the margin of the tumor. In some embodiments, a tumor is immune excluded when there is at least some binding the tumor margin, without the cells homogeneously perfusing the volume of the tumor.

In some embodiments, the status can be used for at least one of: a) classifying or select the human subject for a clinical trial, b) recommending or determining eligibility of the human subject for a therapeutic treatment, c) predicting response to therapy of the human subject; and/or d) predicting response to therapy selected from at least one of the group consisting of: radiotherapy, chemotherapy, biological therapy, and immunotherapy. In some embodiments, the method includes one or more of: immunostimulation, checkpoint inhibitor therapy, viral vector therapy (e.g. Nektar), vaccine therapy, bispecific agents therapy, chemotherapy, T-cell exhaustion therapy (e.g. tryptophans), IDO therapy, CAR-T therapy, and/or PDL1 inhibition therapy

In some embodiments, with TIL status of a tumor a clinician can then predict and/or plan for a response to therapy. Clinicians can use the TIL status to select or recommend treatments selected from among radiotherapy, chemotherapy, biological therapies (tumour targeted therapeutic biologic agents), and immunotherapy.

Preclinical imaging data showed that CD8+ T cell densities ranging from approximately 400 to 12,000 cells per $mm^2$ can be imaged. Tumor samples from patients have been shown to contain CD8+ T cell densities from 58 to 7230 cell per $mm^2$ [Tumeh et al., 2014], which indicates that $^{89}Zr$-Df-IAB22M2C is able to image tumors at various cell densities above 400 cells per $mm^2$ in patients.

In some embodiments, any one or more of the methods or compositions can include one or more of the following aspects (or be configured to so include in the case of a composition).

With respect to radiopharmaceutical aspects, the following can apply. The compound can be 89Zr-Df-IAB22M2C. 89Zr has a positron Emean of 398 KeV and Emax of 897 KeV, values that are substantially different from 18F. Some scanners may have an option for selecting the 89Zr isotope for image acquisition. Others may benefit by having 89Zr settings manually entered. Relevant input parameters for 89Zr imaging include (for example only) T 1/2 = 3.3 days (78.41 hr or 4705 min or 282,276 secs) and a positron abundance fraction of 0.227(22.7%). Note that failing to use the correct settings will result in substantial degradation of image quality that cannot be recovered by post-imaging processing algorithms.

With respect to dosing aspects, a dose of 3 (±20%) mCi of 89Zr-Df-IAB22M2C (0.2 to 1.5 mg of protein) can be used (by way of example only). Dose can be consistent across timepoints. For follow-up scans the radiotracer dose should be ± 10% from the net baseline dose, unless there is a drastic patient weight change.

With respect to mode, many options can be employed, such as 2D, 3D list-mode, or TOF. When possible, acquisition can be conducted in List Mode.

With respect to patient position, many options can be employed, including supine with arms down by side. Position should be consistent between CT and PET acquisitions.

With respect to scan location /coverage, a variety of options can be considered. For melanoma subjects: Skull vertex through feet and including upper extremities, can be used. For non-melanoma subjects: skull vertex to knees or area of interest ensuring consistent scan coverage for a subject can be used.

With respect to transmission scan parameters, a variety of options are available, including, by way of example only:

140 kVp,
mAs:

- ◦ 1-2 hour post infusion scan: Low dose CT (80 mAs)
- ◦ 6-8 hour (optional), 24 hour, 48 hour, and 92-144 hour post infusion scans: Low dose CT (10 mAs)

- 0.8 sec per CT rotation
- 3 - 5 mm slice thickness
- Table speed of 15 mm/rotation
- Pitch: 1.5:1

With respect to emission scans, a variety of options are available. By way of example only, the following can be relevant: 2-7 min/bed position and depending on the height of the subject, a single bed position may provide adequate anatomic coverage.

With respect to image reconstruction, a variety of options are available. By way of example only, the following can be relevant:

Reconstruction algorithm should be consistent across timepoints.
Quantitative iterative reconstruction preferred.
Corrections for normalization, dead time, random events, scatter, attenuation and sensitivity

In some embodiments, a matrix can be 128 x 128 or better

In some embodiments, the following views can be employed: Non-attenuation corrected and/or attenuation corrected

In some embodiments, the patient should have an empty bladder prior to imaging.

In some embodiments, the following can also apply: DICOM requirements. The location of administration can prepare a small sample vial (container) of 89Zr-Df-IAB22M2C which can be placed at the patient's ankles, or at the bottom of the planned scanning field of view (e.g. thighs or knees), during scanning to serve as a reference for bio dosimetry analysis. The purpose of the sample vial is to have an external source of known amount of 89Zr-activity for scanner count calibration needed for biodosimetry estimates. One exemplary practice for preparation and storage of this sample vial is as follows:

1) Remove 0.15 cc of 89Zr-Df-IAB22M2C from the provided vial after infusion, using a tuberculin syringe.
2) Place in dose calibrator and record measurement and the time of measurement. Report these values to IE within the comments section of the Data Transmittal Form (see Appendix VI)
3) Dilute to 5 cc normal saline in a 10 cc syringe
4) Double bag the syringe and seal to prevent any leakage
5) Place the sample vial near ankles of the patient at each scan or at the bottom of the planned scanning field of view (e.g. mid thighs or knees if below the thigh imaging is not performed).
6) Store in refrigerator with proper shielding in between scannings and dispose after final scan for patient.

## Methods of detecting the presence or absence of the target molecule

Antigen binding constructs can be used to detect the presence or absence of the target molecule *in vivo* and/or *in vitro.* Any of the antigen binding constructs, including any of the minibodies, provided herein, can be used for any of the methods and/or formulations provided herein. Accordingly, some embodiments include methods of detecting the presence or absence of the target. The method can include applying an antigen binding construct to a sample. The method can include detecting a binding or an absence of binding of the antigen binding construct to the target molecule, CD8.

**FIG. 14** illustrates some embodiments of methods of detecting the presence or absence of CD8 in vitro. It will be appreciated that the steps shown in **FIG. 14** can be performed in any sequence, and/or can be optionally repeated and/or eliminated, and that additional steps can optionally be added to the method. An antigen binding construct as described herein can be applied to a sample 100. An optional wash 110 can be performed. Optionally, a secondary antigen binding construct can be applied to the sample 120. An optional wash can be performed 130. A binding or absence of binding of the antigen binding construct to the target molecule can be detected 140.

In some embodiments, an antigen binding construct as described herein is applied to a sample *in vivo.* The antigen binding construct can be administered to a subject. In some embodiments, the subject is a human. In some embodiments, the antigen binding construct is infused into the subject. In some embodiments, the infusion is intravenous. In some embodiments, the infusion is intraperitoneal. In some embodiments, the antigen binding construct is applied topically or locally (as in the case of an interventional or intraoperative application) to the subject. In some embodiments, a capsule containing the antigen binding construct is applied to the subject, for example orally or intraperitoneally. In some embodiments, the antigen binding construct is selected to reduce the risk of an immunogenic response by subject. For example, for a human subject, the antigen binding construct can be humanized as described herein. In some embodiments, following *in vivo* application of the antigen binding construct, the sample, or a portion of the sample is removed from the host. In some embodiments, the antigen binding construct is applied *in vivo,* is incubated in *vivo* for a period of time as described herein, and a sample is removed for analysis *in vitro,* for example *in vitro* detection of antigen binding construct bound to the target molecule or the absence thereof as described herein.

In some embodiments, the antigen binding construct is applied to a sample *in vitro.* In some embodiments, the sample is freshly harvested from a subject, for example a biopsy. In some embodiments, the sample is incubated following harvesting from a subject. In some embodiments, the sample is fixed. In some embodiments the sample includes a whole organ and/or tissue. In some embodiments, the sample includes one or more whole cells. In some embodiments the sample is from cell extracts, for example lysates. In some embodiments, antigen binding construct in solution is added to a solution in the sample. In some embodiments, antigen binding construct in solution is added to a sample that does not contain a solution, for example a lyophilized sample, thus reconstituting the sample. In some embodiments, lyophilized antigen binding construct is added to a sample that contains solution, thus reconstituting the antigen binding construct.

In some embodiments, the antigen binding construct is optionally incubated with the sample for in vitro analysis. The antigen binding construct can be incubated for a period of no more than about 10 days, for example no more than about 10 days, 9, 8, 7, 6, 5, 4, 3, 2, or 1 day, or no more than about 23 hours, for example no more than about 23 hours, 22, 21, 20, 19, 18, 17, 16, 15, 14, 13, 12, 11, 10, 9, 8, 7, 6, 5, 4, 3, 2, 1, 0.75, 0.5, 0.25, or 0.1 hour, including ranges between any two of the listed values. In some embodiments, the incubation is within a subject to which the antigen binding construct was administered. In some embodiments, the incubation is within an incubator. In some embodiments, the incubator is maintained at a fixed temperature, for example about 21°C, room temperature, 25°C, 29°C, 34°C, 37°C, or 40°C.

In some embodiments, the antigen binding construct that is not bound to the target is optionally removed from the sample. In some embodiments, the sample is washed. Washing a sample can include removing solution that contains unbound antigen binding construct, and adding solution that does not contain antigen binding construct, for example buffer solution. In some embodiments, an *in vitro* sample is washed, for example by aspirating, pipetting, pumping, or draining solution that contains unbound antigen binding construct, and adding solution that does not contain antigen binding construct. In some embodiments, an *in vivo* sample is washed, for example by administering to the subject

solution that does not contain antigen binding construct, or by washing a site of topical antigen binding construct administration. In some embodiments, the wash is performed at least two times, for example at least 2, 3, 4, 5, 6, 7, 8, 9, 10, 15, or 20 times. In some embodiments, following the wash or washes, at least about 50% of unbound antibody is removed from the sample, for example at least about 50%, 60%, 70%, 75%, 80%, 85%, 90%, 95%, 97%, 98%, 99% or greater.

In some embodiments, unbound antigen binding construct is eliminated from the sample. Following application of the antigen binding construct to the sample, antigen binding construct bound to the target reaches an equilibrium with antigen binding construct unbound to the target, so that at some time after application of the antigen binding construct, the amount of antigen binding construct bound to the target does not substantially increase. After this time, at least part of the quantity of the antigen binding construct that is unbound to the target can be eliminated. In some embodiments, unbound antigen binding construct is eliminated by metabolic or other bodily processes of the subject to whom the antibody or fragment was delivered. In some embodiments, unbound antigen binding construct is eliminated by the addition of an agent that destroys or destabilized the unbound antigen binding construct, for example a protease or a neutralizing antibody. In some embodiments, 1 day after application of the antigen binding construct, at least about 30% of the antigen binding construct that was applied has been eliminated, for example at least about 30%, 40%, 60%, 70%, 75%, 80%, 85%, 90%, 95%, 97%, 98%, 99%, or 99.9%. In some embodiments, 2 days after application of the antigen binding construct, at least about 40% of the antigen binding construct that was applied has been eliminated, for example at least about 40%, 50%, 60%, 70%, 75%, 80%, 85%, 90%, 95%, 97%, 98%, 99%, or 99.9%.

In some embodiments, the presence or absence of the target, CD8, is detected. The presence or absence of the target can be detected based on the presence or absence of the antigen binding construct in the sample. After removal and/or elimination of the antigen binding construct from the sample, for example by washing and/or metabolic elimination, remaining antigen binding construct in the sample can indicate the presence of the target, while an absence of the antigen binding construct in the sample can indicate the absence of the target. In some embodiments, the antigen binding construct is configured such that it is biased for elimination via the kidneys. In some embodiments, the antigen binding construct is configured such that it is biased for elimination via the liver. In some embodiments, an antigen binding construct comprising at least a portion of a hIgG2 or IgG2 hinge and/or $C_H3$ region as described herein can be configured such that it is cleared substantially via the liver. In some embodiments, an antigen binding construct comprising at least a portion of an IgG2 hinge and/or $C_H3$ region as described herein can be configured such that it is cleared substantially via the liver, but not substantially cleared via the kidney. In some embodiments, the ratio of antigen binding construct eliminated in the liver to antigen binding construct eliminated in the kidney is at least about 2:1, for example about 2:1, 3:1, 3.5:1, 4:1, 5:1, 6:1, 7:1, 8:1, 9:1, 10:1, 20:1, 30:1, 40:1, 50:1, 100:1, or 200:1, including ranges between any of the listed values. In some embodiments, the antigen binding construct remains as a dimer during elimination so as to have a molecular weight that increases the likelihood of elimination via the liver rather than the kidney. In some embodiments, the antigen binding construct maintains an atomic mass greater than the renal threshold of about 60 kDa, for example about 65 kDa, 70 kDa, 80 kDa, 90 kDa, 100 kDa, 120 kDa, 150 kDa, or 200 kDa, including ranges between any two of the listed values.

In some embodiments, the antigen binding construct includes a detectable marker as described herein. Thus, the presence of the antigen binding construct can be inferred by detecting the detectable marker.

In some embodiments, a secondary antigen binding construct is used to detect the antigen binding construct. The secondary antigen binding construct can bind specifically to the antigen binding construct. For example, the secondary antigen binding construct can include a polyclonal or monoclonal antibody, diabody, minibody, etc. against the host type of the antibody, or against the antigen binding construct itself. The secondary antigen binding construct can be conjugated to a detectable marker as described herein. The secondary antigen binding construct can be applied to the sample. In some embodiments, the secondary antigen binding construct is applied to the sample in substantially the same manner as the antigen binding construct. For example, if the antigen binding construct was infused into a subject, the secondary antigen binding construct can also be infused into the subject.

In some embodiments, binding or the absence of binding of the antigen binding construct is detected via at least one of: positron emission tomography (PET), single-photon emission computed tomography (SPECT), magnetic resonance imaging (NMR), or detection of fluorescence emissions. PET can include, but is not limited to microPET imaging. In some embodiments, binding of the absence of binding of the antigen binding construct is detected via two or more forms of imaging. In some embodiments, detection can be via near-infrared (NIR) and/or Cerenkov.

Some embodiments include detection of human CD8 which is a specific biomarker found on the surface of a subset of T-cells for diagnostic imaging of the immune system. Imaging of the target molecule can allow for the in vivo detection of T-cell localization. Changes in T-cell localization can reflect the progression of an immune response and can occur over time as a result various therapeutic treatments or even disease states. For example, imaging T-cell localization can be useful in immunotherapy. Adoptive immunotherapy is a form of therapy where a patient's own T-cells are manipulated in vitro and re-introduced into the patient. For this form of treatment, imaging of T-cells can be useful for monitoring and/or determining the status of the treatment. Thus, in some embodiments, monitoring the localization of the target molecule can be a useful for analyzing a mechanism of action, efficacy, and/or safety in the development

of drugs and/or can aid in the clinical management of disease.

In some embodiments, the CDRs of an antigen binding construct that binds specifically to a target, for example for the antibody OKT8, have been adjusted to minibody and cys-diabody arrangements. The CDRs of a murine antibody have been grafted onto a human minibody and cys-diabody framework, thus producing a chimeric minibody. Antibody V domains typically contain two cysteines that form intra-disulfide bonds. The OKT8 $V_H$ has an extra cysteine in framework 3 (FR3) which could interfere with the expression of the protein as it may lead to aggregation and consequently retention in the endoplasmic reticulum. Thus, some embodiments include minibodies made with a serine replacing the extra cysteine in the framework.

In some embodiments, a method of targeting a CD8+ cell to a first antigen is provided. The method can include applying a bispecific antigen binding construct to a sample. The bispecific antigen binding construct can include a CD8 antigen binding construct as described herein. The bispecific antibody can include an antigen binding construct that binds to the first antigen, for example 1, 2, 3, 4, 5, or 6 CDR's, an scFv, or a monomer of a minibody or cys-diabody. In some embodiments, the bispecific antibody includes 1, 2, or 3 HCDR's of an antigen binding construct as described herein, and/or 1, 2, or 3 LCDR's of an antigen binding construct as described herein. In some embodiments, the bispecific antigen binding construct includes an scFv of an antigen binding construct as described herein. In some embodiment, the bispecific antigen binding construct includes a $V_H$ or $V_L$ sequence as described herein. In some embodiments, the bispecific an antigen binding construct includes a minibody or cys-diabody monomer as described herein. In some embodiments, the bispecific an antigen binding construct is applied to a sample *in vivo,* for example an organ or tissue of a subject. In some embodiments, the bispecific an antigen binding construct is applied to an *in vitro* sample. Without being limited to any one theory, in some embodiments, the bispecific an antigen binding construct binds to the target on the target positive cell, and binds to the first antigen (which can be different from CD8) on the first cell, and thus brings the target positive cell in proximity to the first cell. For example, a CD8+ T cell can be brought into proximity of a cancer cell, and can facilitate an immune response against that cancer cell.

In some embodiments, the anti-CD8 antigen binding constructs can be imaging agents that specifically target human CD8+ T-cells. In some embodiments, the anti-CD8 fragments can directly bind and detect the localization of the specific subclass of T-cells that express CD8. In some embodiments, engineered fragments able to cross link CD8 can potentiate signaling through the T cell receptor and enhance the ability of a subject to clear viral pathogens and respond to tumor antigens and vaccines.

In some embodiments, the minibody and cys-diabody antibody formats have desired pharmacokinetic characteristics for diagnostic imaging while maintaining the high binding affinity and specificity of the parental antibody. Compared to imaging with the full-length parental antibody, these fragments clear much faster; yet they are able to target the antigen for rapid high-contrast imaging. The same favorable pharmacokinetic properties are advantageous for targeting immune responses allowing for more controlled T cell stimulation and preventing undesirable effects of overstimulation (for example, cytokine storms). In preclinical models, the shorter serum half lives for the minibody and the cys-diabody allow for optimal imaging at approximately 16-20 hours post injection for the minibody and 2-6 hours post-injection for the cys-diabody. Same day imaging can provide a significant advantage in the clinic with respect to patient care management.

In addition, the cys-diabody antibody format features the C-terminus cysteine tail. These two sulfhydryl groups (following mild reduction) provide a strategy for site-specific conjugation of functional moieties such as radiolabels that will not interfere with the cys-diabody's binding activity.

In some embodiments, these antigen binding constructs can be diagnostic imaging agents (following labeling with an appropriate radioisotope such as Iodine-124, Cu-64 or Zr-89 (for PET imaging) or fluorophore (for fluorescent imaging)). As clinical imaging agents, these CD8 antigen binding constructs can help to monitor treatment and be used as a patient selection tool.

In some embodiments, the antigen binding constructs can be used for applications where highly specific and high-affinity binding to CD8 is required. Outside of diagnostic imaging, these fragments could serve different purposes depending on the attachment of different functional groups.

With the attachment of the appropriate infrared or fluorescent dye, these constructs can be used as the targeting agent for image-guided intraoperative surgery.

In some embodiments, in addition to the modifications to the functional groups attached to the fragments, through the use of bispecific fragments (where the fragment is able to bind 2 different antigens) it is possible to bring together CD8+ cells to a second antigen. Bispecific full-length antibodies have been used in cancer immunotherapy to bring cytotoxic cells of the immune system to tumor cells. Thus, such embodiments are also contemplated for the appropriate antigen binding constructs.

In some embodiments, provided herein are engineered scFv, minibody, and cys-diabody antibody fragments that are able to bind and specifically target human CD8 alpha both *in vitro* and *in vivo.*

COMPOSITIONS/FORMULATIONS/KITS

In some embodiments, any composition suitable for the methods provided herein can be employed in the noted methods. In some embodiments, the composition used includes at least: 3.0±20% mCi of a 89Zr-Df-labeled minibody, 20 mM Histidine, 5% sucrose, 51-62 mM Sodium Chloride, 141-194 Arginine, and 2-20 mM Glutamic acid. In some embodiments, the amount of radiation is between 0.5 and 3.6 mCi, for example 0.5, 0.6, 0.7, 0.8, 0.9, 1, 1.1, 1.2, 1.3, 1.4, 1.5, 1.6, 1.7, 1.8. 1.9, 2, 2.1, 2.2, 2.3, 2.4, 2.5, 2.6, 2.7, 2.8, 2.9, 3.0, 3.1, 3.2, 3.3, 3.4, 3.5 and 3.6, including any amount defined by any two of the preceding values. In some embodiments, 10, 15, 20, 25, or 30 mM of histidine can be present, including any amount defined by any two of the preceding values, can be employed. In some embodiments, 2, 3, 4, 5, 6, 7, 8, 9 or 10% sucrose or an alternative to sucrose, including any amount defined by any two of the preceding values, can be employed. In some embodiments, the amount of sodium chloride can be 40, 45, 50, 55, 60, 65, or 70 mM, including any amount defined by any two of the preceding values, can be employed. In some embodiments, the amount of arginine can be 120, 125, 130, 135, 140, 145, 150, 155, or 160 mM, including any amount defined by any two of the preceding values, can be employed. In some embodiments, the amount of glutamic acid can be 1, 2, 5, 10, 20, 25, or 30 mM, including any amount defined by any two of the preceding values, can be employed. In some embodiments, the amount of radiation is between 0.5 and 1 and can be, for example 0.7 mCi or 0.75 mCi. In some embodiments, this lower amount of radiation allows for shorter periods of time between subsequent repeat administration of the labeled minibody.

In some embodiments, 1 to 250 micrograms of the composition is used per kg of subject weight of minibody or antigen binding construct, for example, 1, 2, 3, 4, 5, 10, 20, 30, 40, 50, 60, 70, 80, 90, 100, 110, 120, 130, 140, 150, 160, 170, 180, 190, 200, 210, 220, 230, 240, or 250 can be employed, including any amount defined between any two of the preceding values.

In some embodiments, the composition comprises 1.2-1.5mg of a labeled minibody (or antigen binding construct, which can be higher).

In some embodiments, the diagnostic composition comprises a labeled minibody having the structure of:

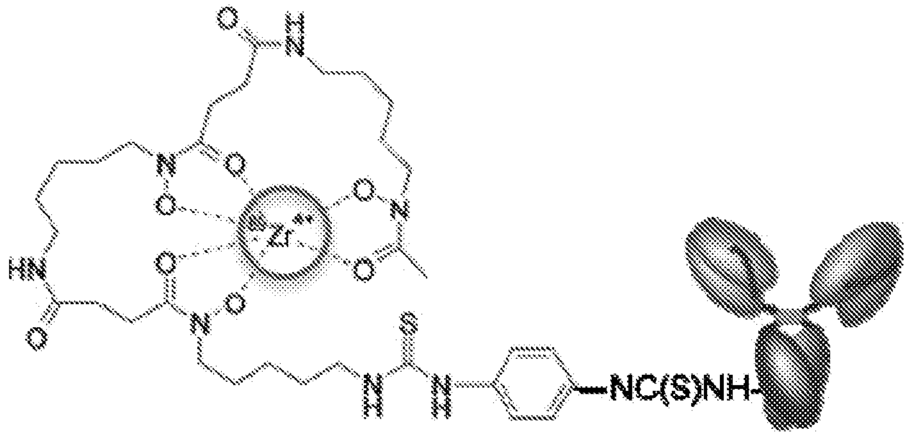

In some embodiments, a diagnostic composition comprises a labeled antiben binding construct comprising the structure of:

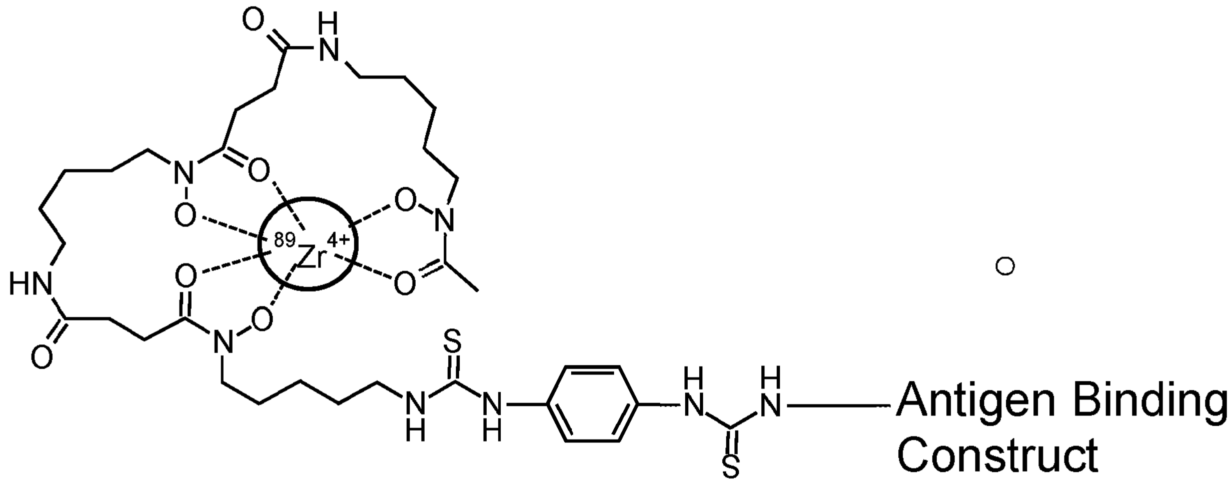

,

wherein the minbody comprises a heavy chain variable region of SEQ ID NO:1, 3 or 16 (or the heavy variable region within 147) and a light chain variable region of SEQ ID NO: 7, 9, or 15 (or the light variable region within 147) and wherein the composition provides at least 3 mCi of radiation.

In some embodiments, the metal chelator is deferoxamine ("DF"). In some embodiments, the metal chelator is DOTA. In some embodiments, the metal chelator is PCTA. In some embodiments, the metal chelator is DTPA. In some embodiments, the metal chelator is NODAGA. In some embodiments, any of these (or others) can be used to carry modifications as isothiocyanate, NHS-esters, CHX-A"-DTPA, HBED, NOTA, DO2P, cyclam, TETA, TE2P, SBAD, NOTAM, DOTAM, PCTA, NO2A, or maleimide to allow conjugation to the protein. When the detectable marker is a radioactive metal or paramagnetic ion, in some embodiments, the marker can be reacted with a reagent having a long tail with one or more chelating groups attached to the long tail for binding these ions. The long tail can be a polymer such as a polylysine, polysaccharide, or other derivatized or derivatizable chain having pendant groups to which may be bound to a chelating group for binding the ions. Examples of chelating groups that may be used according to the embodiments herein include, but are not limited to, ethylenediaminetetraacetic acid (EDTA), diethylenetriaminepentaacetic acid (DTPA), DOTA, NOTA, NOGADA, NETA, deferoxamine (DfO), porphyrins, polyamines, crown ethers, bis-thiosemicarbazones, polyoximes, and like groups. The chelate can be linked to the antigen binding construct by a group which allows formation of a bond to the molecule with minimal loss of immunoreactivity and minimal aggregation and/or internal cross-linking. The same chelates, when complexed with non-radioactive metals, such as manganese, iron and gadolinium are useful for MRI, when used along with the antigen binding constructs and carriers described herein. Macrocyclic chelates such as NOTA, NOGADA, DOTA, and TETA are of use with a variety of metals and radiometals including, but not limited to, radionuclides of gallium, yttrium and copper, respectively. Other ring-type chelates such as macrocyclic polyethers, which are of interest for stably binding nuclides, such as $^{223}Ra$ for RAIT may be used. In certain embodiments, chelating moieties may be used to attach a PET imaging agent, such as an $Al\text{-}^{18}F$ complex, to a targeting molecule for use in PET analysis.

In some embodiments, the minbody (e.g., SEQ ID NO: 147) further comprises a heavy chain variable region shown in FIG. 39 and a light chain variable region shown in FIG. 39, and wherein the composition provides at least 0.7 to 1.5 to 3 mCi of radiation. In some embodiments, just the CDRs shown in FIG. 39 are employed in the minibody or antigen binding construct.

In some embodiments, a composition is provided that comprises a first minibody that binds to CD8 that comprises an active marker (e.g., a detectable marker); and a second minibody that binds to CD8, wherein the second minbody comprises an inactive marker or lacks an active marker (e.g., does not indicate a presence or signal that the detectable marker does).

In some embodiments, the first minibody to second minibody are present in the composition at a ratio of about 1:7.

In some embodiments, a diagnostic composition comprises a first CD8 minibody that is conjugated to a radiolabel, and a second CD8 minibody that is not conjugated to the radiolabel. The first and second CD8 minibodies can have a same sequence, the composition provides about 0.7 to 1.5 to 3 mCi of radiation, and wherein the composition provides 1.5-10 mg of total mass protein.

In some embodiments, any of the compositions provided herein can be used in any of the methods provided herein. In some embodiments, any CD8 antigen binding construct can be used in the present methods, as long as it is

configured to provide the aspects described herein.

In some embodiments, the CD8 minibody comprises a heavy chain variable region and a light chain variable region. In some embodiments the heavy chain variable region consists essentially of a human amino acid sequence and the light chain variable region consists essentially of a human amino acid sequence. In some embodiments, any one or more of the CD8 minibody (or antigen binding constructs) provided herein can be used, wherein the CD8 minibody (or antigen binding construct) binds to a CD8 sequence consisting of the sequence of SEQ ID NO: 24, FIG. 1C, or FIG. 33, or FIG. 34.

In some embodiments, the minibody (or antigen binding construct; SEQ ID NO: 147) comprises the heavy chain CDRs in FIG. 39 (e.g. the heavy chain CDRs within SEQ ID NO 147), and the light chain CDRs in FIG. 39 (e.g. the light chain CDRs within SEQ ID NO 147).

In some embodiments, the minibody (or antigen binding construct) comprises a heavy chain variable region that is at least 80% identical to the heavy chain variable region amino acid sequence in FIG. 39 (within SEQ ID NO: 147), and a light chain variable region that is at least 80% identical to the light chain amino acid sequence FIG. 39 (withing SEQ ID NO:147).

In some embodiments, a diagnostic composition can include one or more of 3.0±20% mCi of a 89Zr-Df-labeled antigen binding construct; 20 mM Histidine; 5% sucrose; 51-62 mM Sodium Chloride; 141-194 Arginine; and 2-20 mM Glutamic acid. In further embodiments, there is 1-250 micrograms per kg of subject weight of antigen binding construct, and wherein the antigen binding construct is a minibody or a diabody. In some embodiments, a CD8 PET tracer is substituted for the antigen binding construct.

In some embodiments, a formulation for a CD8 composition (for the detection of CD8) can include one or more of: a CD8 antigen binding construct, wherein the CD8 antigen binding construct is less than 105 kDa in size; and a $^{89}Zr$ radiolabel associated with the CD8 antigen binding construct, wherein the radiolabel provides more than 0.5 but less than 3 (or 3.6) mCi of radiation for the formulation, wherein the formulation is configured for administration to a human. In further embodiments, the radiolabel provides more than 0.5 but less than 1.0 mCi of radiation. In further embodiments, the radiolabel provides less than 0.75 mCi of radiation. In some embodiments, the antigen binding construct is a minibody or a diabody. In some embodiments, a CD8 PET tracer is substituted for the antigen binding construct.

## LOW RADIATION DOSE

In some embodiments, a formulation for a CD8 composition comprises a CD8 antigen binding construct, the CD8 antigen binding construct being less than 105 kDa in size, and a $^{89}Zr$ radiolabel associated with the CD8 antigen binding construct. The radiolabel provides more than 0.5 but less than 3 mCi of radiation for the formulation. The formulation is configured for administration to a human. In some embodiments, a formulation for a CD8 composition comprises a CD8 antigen binding construct. In some embodiments, a formulation for a CD8 composition comprises a CD8 antigen binding construct, the CD8 antigen binding construct being less than 105 kDa in size.

In some embodiments, a formulation for a CD8 composition comprises a CD8 antigen binding construct, the CD8 antigen binding construct being less than 105 kDa in size, and a $^{89}Zr$ radiolabel associated with the CD8 antigen binding construct.

In some embodiments of a formulation for a CD8 composition, the radiolabel provides more than 0.5 but less than 1.0 mCi of radiation. In some embodiments of a formulation for a CD8 composition, the radiolabel provides less than 0.75 mCi of radiation.

In some embodiments, a CD8 minibody formulation comprises at least 1.5mg of a CD8 minibody, and 1.5 mCi or less of $^{89}Zr$. The formulation is configured for administration to a human.

In some embodiments, a CD8 minibody formulation comprises at least 1.5mg of a CD8 minibody, and 1.5 mCi or less of $^{89}Zr$, wherein the formulation is configured for administration to a human.

In some embodiments, a CD8 minibody formulation comprises at least 1.5mg of a CD8 minibody, and 1.5 mCi or less of $^{89}Zr$.

## HOT/COLD

In some embodiments, a composition comprises a first portion that comprises a CD8 antigen binding construct having an active marker, and a second portion that comprises a CD8 antigen binding construct having an inactive marker or lacks an active marker. In some embodiments, a composition comprises a first portion, and a second portion.

In some embodiments, a composition comprises a first portion that comprises a CD8 antigen binding construct having an active marker.

In some embodiments, a composition comprises a second portion that comprises a CD8 antigen binding construct having an inactive marker or lacks an active marker.In some embodimens of a composition, the first portion to second portion are present in the composition at a molar ratio of from about 1:1 to about 1:50 or about 1:7.

In some embodiments, a diagnostic composition comprises a first CD8 antigen binding construct that is bound to a radiolabel, and a second CD8 antigen binding construct that is not bound to a radiolabel. The first and second CD8 antigen binding constructs have the same amino acid sequence and are present in a molar ratio of from 1:1 to 1:50. The composition provides about 0.5 mCi to about 3.6 mCi of radiation, and wherein the composition provides 0.1-10 mg of total mass protein. In some embodiments, a diagnostic composition comprises a first CD8 antigen binding construct, and a second CD8 antigen binding construct. In some embodiments, a diagnostic composition comprises a first CD8 antigen binding construct that is bound to a radiolabel.

In some embodiments, a diagnostic composition comprises a second CD8 antigen binding construct that is not bound to a radiolabel.

In some embodiments of a disgnostic composition, total mass protein is between 0.5 and 10 mg.

In some embodiments, a method of manufacturing a diagnostic composition comprises conjugating a CD8 antigen binding construct to desferrioxamine to form a Df-labelled CD8 antigen binding construct, radiolabeling the Df-labelled CD8 antigen binding construct with $^{89}$Zr to form a radiolabeled CD8 antigen binding construct, and mixing the radiolabeled CD8 antigen binding construct with non-radiolabelled (cold) Df-labelled CD8 antigen binding construct to form a diagnostic composition. The formulation is configured for human administration.

In some embodiments, a method of manufacturing a diagnostic composition comprises conjugating a CD8 antigen binding construct to desferrioxamine to form a Df-labelled CD8 antigen binding construct.

In some embodiments, a method of manufacturing a diagnostic composition comprises conjugating a CD8 antigen binding construct to desferrioxamine to form a Df-labelled CD8 antigen binding construct, and radiolabeling the Df-labelled CD8 antigen binding construct with $^{89}$Zr to form a radiolabeled CD8 antigen binding construct.

In some embodiments, a method of manufacturing a diagnostic composition comprises conjugating a CD8 antigen binding construct to desferrioxamine to form a Df-labelled CD8 antigen binding construct, and radiolabeling the Df-labelled CD8 antigen binding construct with $^{89}$Zr to form a radiolabeled CD8 antigen binding construct, and mixing the radiolabeled CD8 antigen binding construct with non-radiolabelled (cold) Df-labelled CD8 antigen binding construct to form a diagnostic composition.

## FORMULATION/ARGININE

In some embodiments, a composition comprises $^{89}$Zr-labeled CD8 antigen binding construct, 20 mM Histidine, 5% sucrose, 51-62 mM Sodium Chloride, Arginine, and 2-20 M Glutamic acid. The composition is configured for administration to a human subject as a diagnostic. In some embodiments, a composition comprises $^{89}$Zr-labeled CD8 antigen binding construct.

In some embodiments, a composition comprises $^{89}$Zr-labeled CD8 antigen binding construct, and 20 mM Histidine. In some embodiments, a composition comprises $^{89}$Zr-labeled CD8 antigen binding construct, 20 mM Histidine, and 5% sucrose. In some embodiments, a composition comprises $^{89}$Zr-labeled CD8 antigen binding construct, 20 mM Histidine, 5% sucrose, and 51-62 mM Sodium Chloride. In some embodiments, a composition comprises $^{89}$Zr-labeled CD8 antigen binding construct, 20 mM Histidine, 5% sucrose, 51-62 mM Sodium Chloride, and Arginine. In some embodiments, a composition comprises $^{89}$Zr-labeled CD8 antigen binding construct, 20 mM Histidine, 5% sucrose, 51-62 mM Sodium Chloride, Arginine, and 2-20 M Glutamic acid. In some embodiments, a composition comprises $^{89}$Zr-labeled CD8 antigen binding construct, and one or more of 20 mM Histidine, 5% sucrose, 51-62 mM Sodium Chloride, Arginine, and 2-20 M Glutamic acid. In some embodiments of the composition, the 89Zr-labelled CD8 antigen binding construct is a minibody and composition comprises 1-250 micrograms per kg of weight of the subject.

In some embodiments of the composition, the 89Zr-labelled CD8 antigen binding construct is a minibody and composition comprises 0.5-500, 0.25-750, or 0.125-1000 micrograms per kg of weight of the subject. In some embodiments of the composition, the 89Zr-labelled CD8 antigen binding construct is a minibody and composition comprises 1.2-1.5 mg of CD8 antigen binding construct. In some embodiments of the composition, the 89Zr-labelled CD8 antigen binding construct is a minibody and composition comprises 1.1-1.6, 1.0-1.7, 0.9-1.8, 0.8-1.9, or 0.7-2.0 mg of CD8 antigen binding construct.

In some embodiments, a CD8 antigen binding construct formulation comprises a CD8 antigen binding construct, and free arginine. The formulation is configured for administration to a human. In some embodiments, a CD8 antigen binding construct formulation comprises a CD8 antigen binding construct.

In some embodiments, a CD8 antigen binding construct formulation comprises a CD8 antigen binding construct, and free arginine.

In some embodiments of a CD8 antigen binding construct formulation the free arginine is present at 80 to 200 mM. In some embodiments of a CD8 antigen binding construct formulation the free arginine is present 60 to 250 mM. In some embodiments of a CD8 antigen binding construct formulation the free arginine is present 40 to 300 mM. In some embodiments of a CD8 antigen binding construct formulation the free arginine is present at about 10, 20, 40, 80, 120, 160, 200, 240, 280, or 320 mM, or a value within a range defined by any two of the aforementioned values.

***Antigen binding constructs (including antibodies and binding fragments)***

An antigen binding construct is a molecule that includes one or more portions of an immunoglobulin or immunoglobulin-related molecule that specifically binds to, or is immunologically reactive with the target molecule. Any of the antigen binding constructs that bind to CD8 can be used for any of the compositions (such as formulations) or methods provided herein.

In some embodiments, the antigen binding constructs allow for the detection of human CD8 which is a specific biomarker found on the surface of a subset of T-cells for diagnostic imaging of the immune system. Imaging of CD8 allows for the in vivo detection of T-cell localization. Changes in T-cell localization can reflect the progression of an immune response and can occur over time as a result various therapeutic treatments or even disease states.

In some embodiments, this is useful for imaging T-cell localization for immunotherapy. Adoptive immunotherapy is a form of therapy where a patient's own T-cells are manipulated in vitro and re-introduced into the patient. For this form of treatment, imaging of T-cells is useful for determining the status of the treatment.

In addition, CD8 plays a role in activating downstream signaling pathways that are important for the activation of cytolytic T cells that function to clear viral pathogens and provide immunity to tumors. CD8 positive T cells can recognize short peptides presented within the MHCI protein of antigen presenting cells. In some embodiments, engineered fragments directed to CD8 can potentiate signaling through the T cell receptor and enhance the ability of a subject to clear viral pathogens and respond to tumor antigens. Thus, in some embodiments, the antigen binding constructs provided herein can be agonists and can activate the CD8 target. In some embodiments, an agonist scFv, minibody, cys-diabody, and/or antibody is provided. In some embodiments, the agonist antigen binding construct includes one or more of the CDRs, heavy chain variable regions, or light chain variable regions provided herein. In some embodiments, the agonist can activate downstream signaling pathways through CD8 for the activation of cytolytic T cells that function to clear viral pathogens and provide immunity to tumors.

In some situations, using full-length antibodies for imaging is not optimal since they typically require imaging times to be scheduled more than 1 week after administration due to the long serum half-lives of full-length antibodies.

Another target-based approach for imaging subtypes of immune cells involves small molecules. For example, one approach for diagnostic imaging of the endogenous immune system has involved the use of small molecule tracers which detect changes in the cell's metabolic pathway such as $^{18}$F-fluoroacetate ([$^{18}$F]FAC). Since such tracers detect changes in the metabolic pathway, they target cell populations with elevated metabolic activities which primarily include activated T-cells. The limitation of this approach is that it will only detect the activated subset of T-cells, whereas imaging with anti-CD8 antibody fragments will detect the entire population of CD8 expressing T-cells as the target is expressed on both activated and resting CD8 cells. In some embodiments, both approaches can be employed.

In some embodiments, the minibody format is a homodimer with each monomer having a single-chain variable fragment (scFv) linked to the human IgG1 $C_H3$ domain (see FIGs. 1A and 1B). In some embodiments, the scFv is composed of the variable heavy ($V_H$) and light ($V_L$) domains and is connected by an 18 amino acid GlySer-rich linker. In some embodiments, the scFv is tethered to the human IgG1 $C_H3$ domain by the human IgG1 upper and core hinge regions (15 residues) followed by a 10 amino acid GlySer linker. The minibody ($V_H$-$V_L$-$C_H3$) exists as a stable dimer due to the association between the $C_H3$ domains as well as the formation of disulfide bonds within the hinge regions. To allow for secretion of the minibody, a signal sequence is fused at the N-terminus of the variable heavy domain. In some embodiments, the GlySer residues allow for flexibility. In some embodiments, glutamine and/or lysine residues can be added to enhance solubility.

Two variants of the chimeric IAB-huCD8 minibody have also been engineered that differed in the orientation of the variable regions ($V_H$ to $V_L$ and $V_L$ to $V_H$). Every antibody V domain contains two cysteines that form intra-disulfide bonds. The OKT8 $V_H$ has an extra cysteine in framework 3 (FR3) which may interfere with the expression of the protein as it may lead to aggregation and consequently retention in the endoplasmic reticulum (ER). The chimeric minibodies were made with a serine replacing the extra cysteine in the framework (C84S of the murine $V_H$). In some embodiments, any of the embodiments provided herein can be adjusted to include the C84S adjustment. Tables 0.1, 0.2, and 0.3 provide a summary of come embodiments of the arrangements of various antigen binding constructs provided herein.

Table 0.1 Minibodies (SEQ ID reference those in FIGs. 12A-12I)

| 1 | 2 | 3 | 4 | 5 | 6 |
|---|---|---|---|---|---|
| Name | Leader | Region 1 | Linker | Region 2 | Remainder |
| Chimeric IAb_ Mb1_CD8 | Leader SEQ ID NO: 34 | murine $V_L$ SEQ ID NO: 40 | 18 aa linker SEQ ID NO: 36 | Murine $V_H$ SEQ ID NO: 44 | IgG1 hinge/linker-$C_H3$ domain SEQ ID NO: 38 |

(continued)

| 1 | 2 | 3 | 4 | 5 | 6 |
|---|---|---|---|---|---|
| Name | Leader | Region 1 | Linker | Region 2 | Remainder |
| Chimeric IAb_ Mb2_CD8 | Leader SEQ ID NO: 34 | murine $V_H$ SEQ ID NO: 44 | 18 aa linker SEQ ID NO: 36 | Murine $V_L$ SEQ ID NO: 40 | IgG1 hinge/linker-$C_H3$ domain SEQ ID NO: 38 |
| Humanized IAb_ Mb1_CD8 | Leader SEQ ID NO: 34 | hu $V_L$ SEQ ID NO: 9 | 18 aa linker SEQ ID NO: 36 | hu$V_H$ (2nd) SEQ ID NO: 6 | IgG1 hinge/linker-$C_H3$ domain SEQ ID NO: 38 |
| Humanized IAb_ Mb2_CD8 | Leader SEQ ID NO: 34 | Hu $V_H$ (2nd) SEQ ID NO: 6 | 18 aa linker SEQ ID NO: 36 | hu$V_L$ SEQ ID NO: 9 | IgG1 hinge/linker-$C_H3$ domain SEQ ID NO: 38 |

Table 0.2 Affinity Matured Minibodies (SEQ ID reference those in FIGs. 12A-12I)

| 1 | 2 | 3 | 4 | 5 | 6 |
|---|---|---|---|---|---|
| Name | Leader | Region 1 | Linker | Region 2 | Remainder |
| IAb_Mb1a_ CD8 | Leader SEQ ID NO: 34 | hu$V_L$ SEQ ID NO: 42 | 18aa Linker SEQ ID NO: 36 | hu$V_H$ (version a) SEQ ID NO: 46 | IgG1 hinge/linker-$C_H3$ domain SEQ ID NO: 38 |
| IAb_Mb2a_ CD8 | Leader SEQ ID NO: 34 | hu$V_H$ (version a) SEQ ID NO: 46 | 18aa Linker SEQ ID NO: 36 | hu$V_L$ SEQ ID NO: 42 | IgG1 hinge/linker-$C_H3$ domain SEQ ID NO: 38 |
| IAb_Mb1b_ CD8 | Leader SEQ ID NO: 34 | hu$V_L$ SEQ ID NO: 42 | 18aa Linker SEQ ID NO: 36 | hu$V_H$ (version b) SEQ ID NO: 48 | IgG1 hinge/linker-$C_H3$ domain SEQ ID NO: 38 |
| IAb_M1b_CD8 IgG2 NH | Leader SEQ ID NO: 34 | hu$V_L$ SEQ ID NO: 42 | 18aa Linker SEQ ID NO: 36 | hu$V_H$ (version b) SEQ ID NO: 48 | IgG2 native hinge SEQ ID NO: 55, IgG2 $C_H3$ domain, SEQ ID NO: 80 |

(continued)

| 1 | 2 | 3 | 4 | 5 | 6 |
|---|---|---|---|---|---|
| Name | Leader | Region 1 | Linker | Region 2 | Remainder |
| IAb_M1b_CD8 IgG2 EH | Leader SEQ ID NO: 34 | $huV_L$ SEQ ID NO: 42 | 18aa Linker SEQ ID NO: 36 18aa Linker SEQ ID NO: 36 18aa Linker SEQ ID NO: 36 18aa Linker SEQ ID NO: 36 18aa Linker SEQ ID NO: 36 18aa Linker SEQ ID NO: 36 18aa Linker SEQ ID NO: 36 18aa Linker SEQ ID NO: 36 18aa Linker SEQ ID NO: 36 18aa Linker SEQ ID NO: 36 | $huV_H$ (version b) SEQ ID NO: 48 | IgG2 hinge-extension SEQ ID NO: 79, IgG2 $C_H3$ domain, SEQ ID NO: 80 |
| IAb_Mb2b_ CD8 | Leader SEQ ID NO: 34 | $huV_H$ (version b) SEQ ID NO: 48 | 18aa Linker SEQ ID NO: 36 | $huV_L$ SEQ ID NO: 42 | IgG1 hinge/linker-$C_H3$ domain SEQ ID NO: 38 |

(continued)

| 1 | 2 | 3 | 4 | 5 | 6 |
|---|---|---|---|---|---|
| Name | Leader | Region 1 | Linker | Region 2 | Remainder |
| IAb_Mb1c_ CD8 | Leader SEQ ID NO: 34 | $huV_L$ SEQ ID NO: 42 | 18aa Linker SEQ ID NO: 36 | $huV_H$ (version c) SEQ ID NO: 50 | IgG1 hinge/linker-$C_H3$ domain SEQ ID NO: 38 |
| IAb_Mb2c_ CD8 | Leader SEQ ID NO: 34 | $huV_H$ (version c) SEQ ID NO: 50 | 18aa Linker SEQ ID NO: 36 | $huV_L$ SEQ ID NO: 42 | IgG1 hinge/linker-$C_H3$ domain SEQ ID NO: 38 |
| IAb_Mb1d_ CD8 | Leader SEQ ID NO: 34 | $huV_L$ SEQ ID NO: 42 | 18aa Linker SEQ ID NO: 36 | $huV_H$ (version d) SEQ ID NO: 52 | IgG1 hinge/linker-$C_H3$ domain SEQ ID NO: 38 |
| IAb_Mb2d_ CD8 | Leader SEQ ID NO: 34 | $huV_H$ (version d) SEQ ID NO: 52 | 18aa Linker SEQ ID NO: 36 | $huV_L$ SEQ ID NO: 42 | IgG1 hinge/linker-$C_H3$ domain SEQ ID NO: 38 |

Table 0.3 Cys-Diabodies (SEQ ID reference those in FIGs. 12A-12I)

| 1 | 2 | 3 | 4 | 5 | 6 |
|---|---|---|---|---|---|
| Name | Leader | Region 1 | Linker | Region 2 | Remainder |
| IAb_Cys-Db1b_ CD8 | Leader SEQ ID NO: 26 | $huV_L$ SEQ ID NO: 42 | 5aa Linker SEQ ID NO: 28 | $huV_H$ (version b) SEQ ID NO: 48 | Cys Tail SEQ ID NO: 32 |
| IAb_Cys-Db2b_ CD8 | Leader SEQ ID NO: 26 | $huV_H$ (version b) SEQ ID NO: 48 | 5aa Linker SEQ ID NO: 28 | $huV_L$ SEQ ID NO: 42 | Cys Tail SEQ ID NO: 32 |
| IAb_Cys-Db3b_ CD8 | Leader SEQ ID NO: 26 | $huV_L$ SEQ ID NO: 42 | 8aa Linker SEQ ID NO: 30 | $huV_H$ (version b) SEQ ID NO: 48 | Cys Tail SEQ ID NO: 32 |
| IAb_Cys-Db4b_ CD8 | Leader SEQ ID NO: 26 | $huV_H$ (version b) SEQ ID NO: 48 | 8aa Linker SEQ ID NO: 30 | $huV_L$ SEQ ID NO: 42 | Cys Tail SEQ ID NO: 32 |

Depicted in Tables 0.1, 0.2, and 03 (SEQ ID reference those in FIGs. 12A-12I) are arrangements of sequences for monomers that can be used in minibodies (Table 0.1 and 0.2) and cys-diabodies (Table 0.3). Each row of the table represents the sequence of a monomer construct, with left-to-right representing N-terminus to C-terminus. In some embodiments, the shown sequences of each monomer construct are directly linked to each other. Thus, in some embodiments, the construct can include any of the constructs on a single row in Table 0.1, Table 0.2, or Table 0.3 (SEQ ID reference those in FIGs. 12A-12I). In some embodiments, the constructs can include any combination in Table 0.1, Table 0.2, or Table 0.3 (SEQ ID reference those in FIGs. 12A-12I). In some embodiments, for example, the first item in the first row, column 2 can be combined with the first row, column 3 to the first row column 4, to the first row column 5, to the first row, column 6. In some embodiments, column 3 and column 6 can be swapped with one another. In some embodiments, the first item in the first row, column 2 can be combined with the first row, column 3 to the second row column 4, to the second row column 5, to the second row, column 6. Thus, the tables represent all possible combinations,

both within a single row and across various rows (and with columns swapped).

In some embodiments, an antigen binding construct includes a heavy chain CDR1 (HCDR1) of the HCDR1 in SEQ ID NOs: 3, 6, 16, 44, 46, 48, 50, 52, or 147; a heavy chain CDR2 (HCDR2) of the HCDR2 in SEQ ID NOs: 3, 6, 16, 44, 46, 48, 50, 52 or 147; a heavy chain CDR3 (HCDR3) of the HCDR3 in SEQ ID NOs: 3, 6, 16, 44, 46, 48, 50, 52, or 147; a light chain CDR1 (LCDR1) of the LCDR1 in SEQ ID NOs: 9, 15, 42 or 147; a light chain CDR2 (LCDR2) of the LCDR2 in SEQ ID NOs: 9, 15, 42, or 147; and/or a light chain CDR3 (LCDR3) of the LCDR3 in SEQ ID NOs: 9, 15, 42, or 147. In some embodiments, an antigen binding construct includes the HCDR1 of the HCDR1 in SEQ ID NO: 48 or 147, the HCDR2 of the HCDR2 in SEQ ID NO: 48 or 147, the HCDR3 of the HCDR3 in SEQ ID NO: 48 or 147, the LCDR1 of the LCDR1 in SEQ ID NO: 42 or 147, the LCDR2 of the LCDR2 in SEQ ID NO: 42 or 147, and the LCDR3 of the LCDR3 in SEQ ID NO: 42 or 147. (SEQ ID reference those in FIGs. 12A-12I)

In some embodiments, the antigen binding construct includes 6, 5, 4, 3, 2, or 1, the above CDRs (some embodiments of the CDRs are indicated in FIGs. 2A, 2B, 12C-12I or FIG. 39). In some embodiments, the antigen binding construct includes HCDR3. In some embodiments, the antigen binding construct binds specifically to the target molecule. In some embodiments, the antigen binding construct competes for binding with one or more of the antibodies having the herein provided CDRs. In some embodiments, the antigen binding construct includes at least the 3 heavy chain CDRs noted herein. In some embodiments, the antigen binding construct includes heavy chain CDR3. In some embodiments, the antigen binding construct further includes any one of the heavy chain CDR2 sequences provided herein.

In some embodiments, the antigen binding construct is human or humanized. In some embodiments, the antigen binding construct includes at least one human framework region, or a framework region with at least about 80% sequence identity, for example at least about 80%, 85%, 90%, 93%, 95%, 97%, or 99% identity to a human framework region. In some embodiments the antigen binding construct includes a heavy chain FR1 (HFR1) of the HFR1 in SEQ ID NO: 3, 6, 16, 44, 46, 48, 50, 52, or 147; a heavy chain FR2 (HFR2) of the HFR2 in SEQ ID NO: 3, 6, 16, 44, 46, 48, 50, 52, or 147; a heavy chain FR3 (HFR3) of the HFR3 in SEQ ID NO: 3, 6, 16, 44, 46, 48, 50, 52, or 147; a heavy chain FR4 (HFR4) of the HFR4 in SEQ ID NO: 3, 6, 16, 44, 46, 48, 50, 52, or 147; a light chain FR1 (LFR1) of the LFR1 in SEQ ID NO: 9, 15, 42, or 147; a light chain FR2 (LFR2) of the LFR2 in SEQ ID NO: 9, 15, 42, or 147; a light chain FR3 (LFR3) of the LFR3 in SEQ ID NO: 9, 15, 42, or 147; and a light chain FR4 (LFR4) of the LFR4 in SEQ ID NO: 9, 15, 42, or 147. In some embodiments the antigen binding construct includes a heavy chain FR1 (HFR1) of the HFR1 in SEQ ID NO: 48 or 147; a heavy chain FR2 (HFR2) of the HFR2 in SEQ ID NO: 48 or 147; a heavy chain FR3 (HFR3) of the HFR3 in SEQ ID NO: 48 or 147; a heavy chain FR4 (HFR4) of the HFR4 in SEQ ID NO: 48 or 147; a light chain FR1 (LFR1) of the LFR1 in SEQ ID NO: 42 or 147; a light chain FR2 (LFR2) of the LFR2 in SEQ ID NO: 42 or 147; a light chain FR3 (LFR3) of the LFR3 in SEQ ID NO: 42 or 147; and a light chain FR4 (LFR4) of the LFR4 in SEQ ID NO: 42 or 147. In some embodiments, the antigen binding construct includes 8, 7, 6, 5, 4, 3, 2, or 1 of the listed FRs. (SEQ ID reference those in FIGs. 12A-12I)

In some embodiments, the antigen binding construct includes a detectable marker. In some embodiments, the antigen binding construct includes a therapeutic agent.

In some embodiments, the antigen binding construct is bivalent. Bivalent antigen binding construct can include at least a first antigen binding domain, for example a first scFv, and at least a second antigen binding domain, for example a second scFv. In some embodiments, a bivalent antigen binding construct is a multimer that includes at least two monomers, for example at least 2, 3, 4, 5, 6, 7, or 8 monomers, each of which has an antigen binding domain. In some embodiments, the antigen binding construct is a minibody. In some embodiments, the antigen binding construct is a diabody, including, for example, a cys-diabody. The scFv, and/or minibody and/or the cys-diabody can include any of the CDR and heavy chain variable region and/or light chain variable region embodiments provided herein (for example, the CDR sequences provided in FIGs. 2A, 2B, and 12C-12I). In some embodiments, the antigen binding construct is a monovalent scFv. In some embodiments, a monovalent scFv is provided that includes the HCDR1 in the HCDR1 of FIG. 2A, FIG. 12E-12I, or SEQ ID NO: 3, 6, 16, 44, 46, 48, 50, 52, or 147; the HCDR2 in the HCDR2 of FIG. 2A, FIG. 12E-12I, or SEQ ID NO: 3, 6, 16, 44, 46, 48, 50, 52, or 147; the HCDR3 in the HCDR3 of FIG. 2A, FIG. 12E-12I, or SEQ ID NO: 3, 6, 16, 44, 46, 48, 50, 52, or 147; the LCDR1 in the LCDR1 of FIG. 2B, FIG. 12C, FIG. 12D, SEQ ID NO: 9, 15, 42, or 147; the LCDR2 in the LCDR2 of FIG. 2B, FIG. 12C, FIG. 12D, SEQ ID NO: 9, 15, 42, or 147, and the LCDR3 in the LCDR3 of FIG. 2B, FIG. 12C, FIG. 12D, SEQ ID NO: 9, 15, 42, or 147. In some embodiments, a monovalent scFv is provided that includes the HCDR1 in the HCDR1 of SEQ ID NO: 48 or 147, the HCDR2 in the HCDR2 of SEQ ID NO: 48 or 147, the HCDR3 in the HCDR3 of SEQ ID NO: 48 or 147, the LCDR1 in the LCDR1 of SEQ ID NO: 42 or 147, the LCDR2 in the LCDR2 of SEQ ID NO: 42 or 147, and the LCDR3 in the LCDR3 of SEQ ID NO: 42 or 147. In some embodiments, the CDRs are defined in accordance with Chothia, as shown in FIG. 12D. (SEQ ID reference those in FIGs. 12A-12I)

In some embodiments, the monovalent scFv includes the heavy chain variable region of the heavy chain variable region in FIG. 2A, FIGs. 4-11, FIGs. 12C-12I, or SEQ ID NO: 3, 6, 16, 44, 46, 48, 50, 52, or 147. In some embodiments, the monovalent scFv includes the light chain variable region of the light chain variable region in FIG. 2B, 4-11, 12C, 12D, SEQ ID NO: 9, 15, 40, 42, or 147. In some embodiments, the monovalent scFv includes the heavy chain variable

region of the heavy chain variable region in FIG. 2A, FIGs. 4-11, FIGs. 12C-12I, or SEQ ID NO: 3, 6, 16, 44, 46, 48, 50, 52, or 147and the light chain variable region of the light chain variable region in in FIG. 2B, 4-11, 12C, 12D, SEQ ID NO: 9, 15, 40, 42, or 147. In some embodiments, the monovalent scFv includes the heavy chain variable region of the heavy chain variable region in SEQ ID NO: 48 or 147, and the light chain variable region of the light chain variable region in in SEQ ID NO: 42 or 147. (SEQ ID reference those in FIGs. 12A-12I)

In some embodiments, the antigen binding construct is bispecific. Bispecific antibodies can include at least a first binding domain, for example an scFv that binds specifically to a first epitope, and at least a second binding domain, for example an scFv that binds specifically to a second epitope. Thus, bispecific antigen binding constructs can bind to two or more epitopes. In some embodiments, the first epitope and the second epitope are part of the same antigen, and the bispecific antigen binding construct can thus bind to two epitopes of the same antigen. In some embodiments, the first epitope is part of a first antigen, and the second epitope is part of a second antigen, and the bispecific antigen binding construct can thus bind to two different antigens. In some embodiments, the antigen binding construct binds to two epitopes simultaneously.

In some embodiments, the antigen binding construct has a heavy chain variable region of the heavy chain variable region in SEQ ID NO: 3, 6, 16, 18, 20, 22, 44, 46, 48, 50, 52, or 147. In some embodiments, the antigen binding construct has a heavy chain variable region that includes a sequence with at least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 3. In some embodiments, the antigen binding construct has a heavy chain variable region that includes a sequence with at least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 6. In some embodiments, the antigen binding construct has a heavy chain variable region that includes a sequence with at least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 44. In some embodiments, the antigen binding construct has a heavy chain variable region that includes a sequence with at least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 46. In some embodiments, the antigen binding construct has a heavy chain variable region that includes a sequence with at least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 48. In some embodiments, the antigen binding construct has a heavy chain variable region that includes a sequence with at least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 50. In some embodiments, the antigen binding construct has a heavy chain variable region that includes a sequence with at least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 52. (SEQ ID reference those in FIGs. 12A-12I). In some embodiments, the antigen binding construct has a heavy and a light chain variable region that includes a sequence with at least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 52.

In some embodiments, the antigen binding construct has a light chain variable region that includes SEQ ID NO: 9, 16, 18, 20, 22, 40, or 42. In some embodiments, the antigen binding construct has a light chain variable region that includes a sequence with least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 9. In some embodiments, the antigen binding construct has a light chain variable region that includes a sequence with least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 40. In some embodiments, the antigen binding construct has a light chain variable region that includes a sequence with least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 42. In some embodiments, the antigen binding construct is a human antigen binding construct and has a heavy chain variable region, a light chain variable region, or a heavy and light chain that is at least as identical as at least the heavy and/or light chain variable sequences noted above. In some embodiments, the antigen binding construct includes a heavy chain variable region that includes a sequence with least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 48, and a light chain variable region that includes a sequence with least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 42. (SEQ ID reference those in FIGs. 12A-12I)

Some embodiments provided herein include an antigen binding construct that competes for binding to the target molecule with one or more antigen binding constructs provided herein. In some embodiments, the competing antigen binding construct binds to the same epitope on the target molecule as the reference antigen binding construct. In some embodiments, the reference antigen binding construct binds to a first epitope of the target molecule, and the competing antigen binding construct binds to a second epitope of the target molecule, but interferes with binding of the reference antigen binding construct to the target molecule, for example by sterically blocking binding of the reference antigen

binding construct, or by inducing a conformational change in the target molecule. In some embodiments, the first epitope overlaps with the second epitope. In some embodiments, columns 3 and 5 of Tables 0.1 and/or 0.2 can be swapped. In some embodiments, any of the heavy chains variable regions provided herein can be combined with any of the light chain variable regions herein for a scFv, minibody, and/or diabody. In some embodiments, any of the heavy and/or light chain variable regions (columns 3 and 5) in tables 0.1, 0.2, and 0.3 can be exchanged with one another or another light or heavy chain variable region, to produce an antigen binding construct (such as a scFv, a cys-diabody, a minibody , or an antibody).

In some embodiments, the minibody and cys-diabody formats have advantageous pharmacokinetic characteristics for diagnostic imaging and certain therapeutic applications while maintaining the high binding affinity and specificity of a parental antibody. Compared to imaging with the full-length parental antibody, the pharmacokinetics are more desirable for these fragments in that they are able to target the antigen and then rapidly clear the system for rapid high-contrast imaging. In some embodiments, the shorter serum half lives for the minibody and the cys-diabody allow for imaging to occur over a range of times, approximately 8-48 hours post injection for the minibody and 2-24 hours post-injection for the cys-diabody. The rapid serum clearance together with better tissue penetration can allow for same day imaging, providing a significant advantage in the clinic with respect to patient care management.

In addition, the cys-diabody antibody format features the C-terminus cysteine tail. These two sulfhydryl groups (following mild reduction) provide a strategy for site-specific conjugation of functional moieties such as radiolabels that need not interfere with the cys-diabody's binding activity.

***Diabodies that bind to the Target Molecule***

In some embodiments, the antigen binding construct can be a diabody. The diabody can include a first polypeptide chain which includes a heavy ($V_H$) chain variable domain connected to a light chain variable domain ($V_L$) on the first polypeptide chain. In some embodiments, the light and heavy variable chain domains can be connected by a linker. The linker can be of the appropriate length to reduce the likelihood of pairing between the two domains on the first polypeptide chain and a second polypeptide chain comprising a light chain variable domain ($V_L$) linked to a heavy chain variable domain $V_H$ on the second polypeptide chain connected by a linker that is too short to allow significant pairing between the two domains on the second polypeptide chain.

In some embodiments, the appropriate length of the linker encourages chain pairing between the complementary domains of the first and the second polypeptide chains and can promote the assembly of a dimeric molecule with two functional antigen binding sites. Thus, in some embodiments, the diabody is bivalent. In some embodiments, the diabody can be a cysteine linked diabody (a Cys-Db). A schematic of a Cys-Db binding to two antigen sites is illustrated in FIG. 3A and 3B.

In some embodiments, the linker can be a peptide. In some embodiments, the linker can be any suitable length that promotes such assembly, for example, between 1 and 20 amino acids, such as 5 and 10 amino acids in length. As described further herein, some cys-diabodies can include a peptide linker that is 5 to 8 amino acids in length. In some embodiments, the linker need not be made from, or exclusively from amino acids, and can include, for example, modified amino acids (see, for example, Increased Resistance of Peptides to Serum Proteases by Modification of their Amino Groups, Rossella Galati, Alessandra Verdina, Giuliana Falasca, and Alberto Chersi, (2003) Z. Naturforsch, 58c, 558-561). In some embodiments, the linker can be 1, 2, 3, 4, 5, 6, 7, 8, 9, 10, 11, 12, 13, 14, 15, 16, 17, 18, 19, or 20 amino acids in length. In some embodiments, the linker can be from 2 to 30 angstroms in length, for example 2.5 to 27 angstroms.

In some embodiments, the antigen binding construct includes a humanized cys-diabody. The humanized cys-diabody can include a single-chain variable fragment (scFv) that includes a variable heavy ($V_H$) domain linked to a variable light ($V_L$) domain, and a C-terminal cysteine. In some embodiments, the humanized cys-diabody is a homodimer. In some embodiments, the humanized diabody is a heterodimer. In some embodiments, individual monomers are provided that each have a cysteine terminal residue.

In some embodiments, the scFv of the humanized cys-diabody has a $V_H$-$V_L$ orientation or a $V_L$-$V_H$ orientation. As used herein, a $V_H$-$V_L$ (which may also be referred to herein as "$V_HV_L$") orientation means that the variable heavy domain ($V_H$) of the scFv is upstream from the variable light domain ($V_L$) and a $V_LV_H$ orientation means that the $V_L$ domain of the scFv is upstream from the $V_H$ domain. As used herein, "upstream" means toward the N-terminus of an amino acid or toward the 5' end of a nucleotide sequence.

The antibody variable regions can be linked together by a linker as described herein. In some embodiments, the linker is a GlySer linker as described herein.

In some embodiments, the cys-diabody includes a detectable marker.

In some embodiments, the cys-diabody includes a pair of monomers. Each monomer can include a polypeptide. In some embodiments, the polypeptides of the monomers are identical (for example, cys-diabody can be a homodimer). In some embodiments, the polypeptides of the monomers are different (for example, the cys-diabody can be a heterodimer).

In some embodiments, the polypeptide of the monomer includes SEQ ID NO: 12 (See FIG. 8). In some embodiments, the polypeptide of the monomer includes a sequence with least about 80% identity, for example at least about 80%, 85%, 86%, 87%, 88%, 89%, 90%, 91%, 92%, 93%, 94%, 95%, 96%, 97%, 98%, or 99% identity to SEQ ID NO: 12 (cys-diabody ($V_L$-5-$V_H$)). (SEQ ID reference those in FIG. 8)

In some embodiments, the polypeptide of the monomer includes SEQ ID NO: 13 (See FIG. 9). In some embodiments, the polypeptide of the monomer includes a sequence with least about 80% identity, for example at least about 80%, 85%, 86%, 87%, 88%, 89%, 90%, 91%, 92%, 93, 94, 95%, 96%, 97%, 98%, or 99% identity to SEQ ID NO: 13 (cys-diabody ($V_H$-5-$V_L$)). (SEQ ID reference those in FIG. 9).

In some embodiments, the polypeptide of the monomer includes SEQ ID NO: 14 ($V_L$-8-$V_H$)] (See FIG. 10). In some embodiments, the polypeptide of the monomer includes a sequence with least about 80% identity, for example at least about 80%, 85%, 86%, 87%, 88%, 89%, 90%, 91%, 92%, 93, 94, 95%, 96%, 97%, 98%, 99%, or 100% identity to SEQ ID NO: 14.

In some embodiments, the polypeptide of the monomer includes SEQ ID NO: 15 (humanized IAB-huCD8 cys-diabody ($V_H$-8-$V_L$)) (See **FIG. 11).** In some embodiments, the polypeptide of the monomer includes a sequence with least about 80% identity, for example at least about 80%, 85%, 86%, 87%, 88%, 89%, 90%, 91%, 92%, 93, 94, 95%, 96%, 97%, 98%, 99%, or 100% identity to SEQ ID NO: 15.

In some embodiments, the polypeptide of the monomer includes SEQ ID NO: 147. In some embodiments, the polypeptide of the monomer includes a sequence with least about 80% identity, for example at least about 80%, 85%, 86%, 87%, 88%, 89%, 90%, 91%, 92%, 93, 94, 95%, 96%, 97%, 98%, 99%, or 100% identity to SEQ ID NO: 147.

In some embodiments, the polypeptide of the monomer includes any of the combined sections as indicated in Table 0.3, including polypeptides of the monomer with a sequences of at least about 80% identity, for example at least about 80%, 85%, 86%, 87%, 88%, 89%, 90%, 91%, 92%, 93, 94, 95%, 96%, 97%, 98%, 99%, or 100% identity to the monomers as set forth in Table 0.3.

In some embodiments, the cysteines are cross-linked with one another. In some embodiments, the cysteines are reduced, and thus, these tail forming cysteines do not form a disulfide bond with one another. In some embodiments, one or more of the "tail forming" cysteines form a covalent bond with one or more detectable marker, such as a fluorescent probe.

As will be appreciated by those of skill in the art, while the present disclosure generally references "cys-diabodies" alternative arrangements can be employed to achieve the same or similar ends. In some embodiments, any covalently modifiable moiety can be employed in place of one or more of the cysteines. For example, this can include a GlySer linker, a GlyLeu linker, and/or an insert cysteine after a short tag. In some embodiments, the connection can be established via a coiled coil or a leucine zipper. In some embodiments, the "tail" itself can include functional groups on its end so that it can selectively bind to a desired residue and/or location at the ends of each of the polypetides, in place of the disulfide bond itself. In some embodiments, rather than the tail providing space between the two polypeptide chains, the covalently modifiable moieties can be attached directly to the end of the heavy or light chain polypeptide, but the two covalently modifiable moieties can be connected by a linker.

In some embodiments, a chimeric cys-diabody that binds to the target molecule is provided. In some embodiments, the chimeric cys-diabody includes a monomer in the $V_L$-$V_H$ format, and includes the sequence of SEQ ID NO: 12 or 14, (FIGs. 8 or 10)or a sequence having at least about 80% identity thereto, for example at least about 80%, 85%, 90%, 95%, 96%, 97%, 98%, or 99%% identity thereto. In some embodiments, the chimeric cys-diabody includes a monomer in the $V_H$-$V_L$ format, and includes the sequence of SEQ ID NO: 13 or 15 (FIGs. 9 or 11) or a sequence having at least about 80% identity thereto, for example at least about 80%, 85%, 90%, 95%, 96%, 97%, 98%, or 99%% identity thereto.

In some embodiments, any of the constructs provided herein (including those arrangements noted as cys-diabody embodiments, can be provided as a scFv embodiment. In such embodiments, the construct can still include the cysteine on the tail, but simply not be cross-linked. In other embodiments, the construct need not have the cysteine in a tail or the tail at all.

***Linker and/or Tail Options***

In some embodiments, for individual antibodies, the heavy and light chain variable domains can associate in different ways. For this reason, the use of different linker lengths allows for conformational flexibility and range-of-motion to ensure formation of the disulfide bonds.

In some embodiments, the two linker lengths can be somewhere between (and including) about 1 to 50 amino acids, for example, 2 to 15, 2 to 14, 3 to 13, 4 to 10, or 5 amino acids to 8 amino acids. In some embodiments, each linker within a pair for a diabody can be the same length. In some embodiments, each linker within the pair can be a different length. In some embodiments, any combination of linker length pairs can be used, as long as they allow and/or promote the desired combinations. In some embodiments, a modified amino acid can be used.

FIGs. 8-11 provide four Cys-Db variants, $V_H$-5-$V_L$, $V_H$-8-$V_L$, $V_L$-5-$V_H$, and VL8VH (see FIGs. 8 - 11, and Table 0.3). Producing and testing the expression and binding of all four variants allows for identification of a desired format for protein production for each new Cys-Db. Evaluating the set of variants can help to make certain that a high-quality, stable protein is produced where the disulfide bridge is available. Therefore, engineering a Cys-Db can involve using two distinct linker lengths, not one - as in the minibody, as well as both orientations of the variable regions, $V_H$-$V_L$ and $V_L$-$V_H$.

In some embodiments, the linker is a GlySer linker. The GlySer linker can be a polypeptide that is rich in Gly and/or Ser residues. In some embodiments, at least about 40% of the amino acid residues of the GlySer linker are Gly, Ser, or a combination of Gly and Ser, for example at least about 40%, 50%, 60%, 70%, 80%, or 90%. In some embodiments, the GlySer linker is at least about 2 amino acids long, for example at least about 3, 4, 5, 6, 7, 8, 9, 10, 11, 12, 13, 14, 15, 16, 17, 18, 19, 20, 25, 30, 35, or 40 amino acids long. In some embodiments, the linker includes at least one of SEQ ID NO: 28, 30, and/or 36. (FIG. 12A)

In some embodiments, a cysteine is added at the C-terminus of the diabody. This cysteine can allow the diabody complex to form covalent cysteine bonds and provides the option for available sulfur residues for site-specific conjugation of functional moieties such as radiolabels. In some embodiments, a terminal end of the antibody itself is altered so as to contain a cysteine. In some embodiments, a tail sequence, for example (Gly-Gly-Cys) is added at the C-terminus. In some embodiments, the cysteine tail sequence allows two monomers of a cys-diabody to form disulfide bonds with each other. In some embodiments, the cysteine tail sequence allows a cys-diabody to form disulfide linkages with a detectable moiety such as a detectable marker and/or therapeutic agent. The sulfhydryl groups of the cysteine tail can undergo mild reduction prior to site-specific conjugation of a desired functional moiety, for example a detectable marker and/or therapeutic agent. In some embodiments, the tail is at least about 1 amino acid long, for example at least about 1, 2, 3, 4, 5, 6, 7, 8, 9, 10, 11, 12, 13, 14, 15, 16, 17, 18, 19, 20, 25, 30, 35, or 40 amino acids long. In some embodiments, the tail includes at least one of SEQ ID NO: 32 (FIG. 12A). In some embodiments, the tail is 3 to 8 amino acids in length. In some embodiments, the tail can and/or include a coiled coil and/or a leucine zipper. As noted above, in some embodiments, the cysteine is located at the c-terminus; however, this does not require that the cysteine be located as the last c-terminal amino acid. Instead, this denotes that the cysteine can be part of any of the residues that are located in the C-terminus of the protein.

In some embodiments, the linking option between the two C-terminuses can be achieved by a cysteine, for direct and/or indirect, cross-linking.

## *Minibodies that bind to the Target Molecule*

In some embodiments, the antigen binding construct is or comprises a minibody. A "minibody" as described herein includes a homodimer, wherein each monomer is a single-chain variable fragment (scFv) linked to a human IgG1 $C_H3$ domain by a linker, such as a hinge sequence. In some embodiments, the hinge sequence is a human IgG1 or IgG2 hinge sequence as shown in **FIG. 12B,** SEQ ID NOs: 53-60. In some embodiments, the CH3 sequence comprises an IgG1 $C_H3$, orIgG2 $C_H3$ sequence as shown in FIG. 12A and 12B, SEQ ID NOs: 37-38 and 80-81.

In some embodiments, the hinge sequence is an artificial hinge sequence. In some embodiments, the hinge sequence can be an IgG hinge from any one or more of the four classes. The artificial hinge sequence may include a portion of a human IgG1 or IgG2 hinge and a GlySer linker (also known as an "extension" when distinguishing this section from the generic linker sequence that links the Vh and Vl regions) sequence.

In some embodiments, the artificial hinge sequence includes approximately the first 14 or 15 residues of the human IgG1 hinge followed by an extension sequence. In some embodiments, the extension can be any of those provided herein. In some embodiments, the extension can be a GlySer extension sequence that is 6, 7, 8, 9 or 10 amino acids in length. In some embodiments, the artificial hinge sequence includes approximately the first 15 residues of the IgG1 hinge followed by a GlySer extension sequence that is about 10 amino acids in length. In some embodiments, association between the $C_H3$ domains causes the minibody to exist as a stable dimer.

In some embodiments, the hinge sequence comprises a human IgG2 hinge sequence. In some embodiments, the hinge sequence comprises an IgG2 hinge sequence. In some embodiments, the hinge sequence comprises a native hIgG2IgG2 sequence ("NH"). An exemplary native hinge sequence that can be used in conjunction with embodiments herein is shown in FIG. 12B, SEQ ID NO: 55. In some embodiments, any and all of the constructs provided herein can be used with an hIgG2 hinge region.

In some embodiments, the hinge sequence comprises a native human IgG2 sequence of SEQ ID NO: 55, FIG. 12B. In some embodiments, the hinge sequence comprises an artificial IgG2 hinge sequence. In some embodiments the native IgG2 sequence comprises an artificial hinge-extension (EH) sequence. In some embodiments, the artificial hinge-extensions sequence comprises SEQ ID NO: 79, FIG. 12B. In some embodiments, the artificial hinge sequence includes approximately the first 12 to 15 residues, for example 12 residues of the human IgG2 hinge sequence followed by a GlySer extension sequence that is 6, 7, 8, 9, 10, 11, or 12 amino acids in length. An exemplary artificial hinge sequences that can be used in conjunction with embodiments herein is shown in FIG. 12B, SEQ ID NO: 79. In some

embodiments, any of the above noted hinge region options (e.g., Tables 0.1 or 0.2) can be employed in a minibody. In some embodiments, any of the hinge regions noted in Tables 0.1 or 0.2 can be replaced with an IgG2 hinge region. In some embodiments, any of the constructs in tables 0.1 or 0.2, and/or any of the constructs depicted in the figures can have the depicted hinge region replaced with part or all of a hinge region from hIgG2 or IgG2.

In some embodiments, the minibody scFv sequence can include CDR and/or FR, and or variable region sequences that are similar and/or the same to a diabody sequence described herein (for Example, as found **FIGs. 2A, 2B, 4, 5, 6, 7, 8, 9, 10, 11**, and **12C-12I** and tables 0.1 and 0.2). In some embodiments, the minibody scFv has a sequence (CDR, CDRs, full set of 6 CDRS, heavy chain variable region, light chain variable region, heavy and light chain variable regions, etc.) that is at identical to a scFv of a cys-diabody described herein.

In some embodiments, the minibody has a sequence that is at least about 80% identical to a sequence in SEQ ID NO: 3, 6, 9, 16, 18, 20, 22, 34, 36, 38, 53-60, 40, 42, 44, 46, 48, 50, 52, 147 and/or the sequence for the arrangements in Tables 0.1 and/or 0.2, for example at least about 80%, 85%, 86%, 87%, 88%, 89%, 90%, 91%, 92%, 93, 94, 95%, 96%, 97%, 98%, or 99% identity. (SEQ ID NO: reference those in FIGs. 2A-12I)

In some embodiments, the minibody has a variable chain region that is at least about 80% identical to a sequence in SEQ ID NO: 3, 6, 9, 16, 18, 20, 22, 40, 42, 44, 46, 48, 50, 52, or 147, for example at least about 80%, 85%, 86%, 87%, 88%, 89%, 90%, 91%, 92%, 93, 94, 95%, 96%, 97%, 98%, or 99% identity. (SEQ ID NO: reference those in FIGs. 2A-12I)

The scFv can have a $V_H$-$V_L$ or a $V_L$-$V_H$ orientation. In some embodiments, the $V_H$ and $V_L$ are linked to each other by an amino acid linker sequence. The amino acid linker can be a linker as described herein. In some embodiments, the linker is GlySer-rich and approximately 15-20 amino acids in length. In another embodiment, the linker is GlySer rich and is 18 amino acids in length. In some embodiments, the linker length varies between (and including) about 1 to 50 amino acids, for example, 2 to 30, 3 to 20, 4 to 15, or 5 amino acids to 8 amino acids. In some embodiments, the minibody scFv has a sequence that is at least about 80% identical to a scFv of a cys-diabody described herein, for example at least about 80%, 85%, 86%, 87%, 88%, 89%, 90%, 91%, 92%, 93, 94, 95%, 96%, 97%, 98%, or 99% identity. The scFv can have a $V_HV_L$ or a $V_LV_H$ orientation.

In some embodiments, each monomer of the minibody includes the following elements, from N-terminus to C-terminus: (a) an scFv sequence that includes a $V_H$ domain linked to a $V_L$ domain and that binds to the target molecule, (b) a hinge-extension domain comprising a human IgG1 hinge region, and (c) a human IgG $C_H3$ sequence. In some embodiments, each monomer of the minibody includes the following elements, from N-terminus to C-terminus: (a) an scFv sequence that includes a $V_H$ domain linked to a $V_L$ domain and that binds to the target molecule, (b) a hinge-extension domain comprising an IgG2 hinge region as described herein, and (c) a human IgG $C_H3$ sequence. In some embodiments, each monomer of the minibody includes an IgG2, an IgG3, or an IgG4 $C_H3$. In some embodiments, each monomer of the minibody can include a CH3 domain of an IgA or IgD and/or a CH4 domain of an IgM and/or an IgE. In some embodiments, the minibody is encoded by a nucleic acid can be expressed by a cell, a cell line or other suitable expression system as described herein. Thus, a signal sequence can be fused to the N-terminus of the scFv to enable secretion of the minibody when expressed in the cell or cell line.

In some embodiments, the scFv, minibody, cys-diabody and/or antibody includes one or more of the residues in the humanized sequence shown in FIG. 2A and/or 2B and denoted with an asterisk. In some embodiments, while one or more of the residues marked with an asterisk in FIG. 2A or 2B is present; the remaining sequence can be varied. For example, the sequence can be 80, 81, 82, 83, 84, 85, 86, 87, 88, 89, 90, 91, 92, 93, 94, 95, 96, 97, 98, 99 percent or greater identity to the remaining sections of the sequence. In some embodiments, the human and/or humanized antigen binding construct will include one or more of the asterisked residues in FIG. 2A, for example 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, or 47. In some embodiments, the antigen binding construct includes one or more of the underlined residues in FIG. 2A. In some embodiments, the antigen binding construct includes one or more of the non-underlined residues in FIG. 2A. In some embodiments, the antigen binding construct includes one or more of the non-underlined residues in FIG. 2A as well as the boxed CDR sections, whereas other residues are allowed to vary. In some embodiments, the antigen binding construct

Alternatively, and/or in addition to, the antigen binding construct can include one or more of the asterisked residues in FIG. 2A or 2B, for example 1, 2, 3, 4, 5, 6, 7, 8, 9, 10, 11, 12, 13, 14, 15, 16, 17, 18, 19, or 20. In some embodiments, the antigen binding construct includes one or more of the non-underlined residues in FIG. 2A or 2B. In some embodiments, the antigen binding construct includes one or more of the non-underlined residues in FIG. 2A or 2B as well as the boxed CDR sections, whereas other residues are allowed to vary. In some embodiments, the CDR residues are maintained and the residues with the asterisk are maintained, but one or more of the other residues are allowed to vary.

In some embodiments, a chimeric minibody that binds to the target molecule is provided. In some embodiments, the chimeric minibody includes a monomer in the $V_L$-$V_H$ format, and includes the sequence of SEQ ID NO: 16 or 20, or a sequence having at least about 80% identity thereto, for example at least about 80%, 85%, 90%, 95%, 96%, 97%,

98%, or 99%% identity thereto. In some embodiments, the chimeric minibody includes a monomer in the $V_H$-$V_L$ format, and includes the sequence of SEQ ID NO: 18 or 22, or a sequence having at least about 80% identity thereto, for example at least about 80%, 85%, 90%, 95%, 96%, 97%, 98%, or 99%% identity thereto. (SEQ ID NO: reference those in FIGs. 2A-12I)

In some embodiments, the polypeptide of the monomer includes any of the combined sections as indicated in Tables 0.1 and 0.2, including polypeptides of the monomer with a sequences of at least about 80% identity, for example at least about 80%, 85%, 86%, 87%, 88%, 89%, 90%, 91%, 92%, 93, 94, 95%, 96%, 97%, 98%, 99%, or 100% identity to the monomers as set forth in Tables 0.1 and 0.2.

In some embodiments, the minibody can be IAB22M Mbs, made with huIgG2 hinge variants, either extended hinge (γ2 EH2) (FIG. 16) or natural hinge (γ2 NH1) (FIG. 17B).

Additional structural aspects of some embodiments of minibodies that can be used are depicted in figures 17A, 17B, and 16, and include: $^{89}$Zr-Df-IAB22M-γ1-EH1 (FIG. 17A), -γ2 NH1 (FIG. 17B) and -γ2 EH2 (FIG. 16) variants of CD8 binding constructs.

In some embodiments, the minibody can be as shown in FIG. 17C, which depicts protein sequence information for some embodiments of IAB22M γ2 NH2.

In some embodiments, the minibody can include hinge variants, as shown in FIG. 18.

In some embodiments, the minibody include engineered hinges EH2, EH3 derived from IgG1 sequence (γ1 EH2 (FIG. 20) and γ1 EH3 (FIG. 19A)).

In some embodiments, the monovalent scFv includes the light chain variable region of the light chain variable region in FIG. 21.

In some embodiments, the minibody can include one or more of the linker sequences as depicted in FIG. 22.

In some embodiments, any of the hinge regions provided herein (e.g., table 0.4) can be applied to any one or more of the minibodies provided herein.

TABLE 0.4

| ***Hinge variants (numbering with reference to FIG. 23)*** | | | |
|---|---|---|---|
| **Full hinge** | **Upper hinge** | **Core hinge** | **Lower hinge** |
| Human IgG1 NH1 (SEQ ID NO: 116) | EPKSCDKTHT (SEQ ID NO: 93) | CPPCP (SEQ ID NO: 122) | APELLGGP (SEQ ID NO: 123) |
| Human IgG1 EH1 (SEQ ID NO: 92) | EPKSCDKTHT (SEQ ID NO: 93) | CPPC (SEQ ID NO: 94) | GGGSSGGGSG (SEQ ID NO: 88) |
| Human IgG1 NH2 (SEQ ID NO: 117) | EPKSSDKTHT (SEQ ID NO: 101) | CPPCP (SEQ ID NO: 122) | APELLGGP (SEQ ID NO: 123) |
| Human IgG1 EH2 (SEQ ID NO: 111) | EPKSSDKTHT (SEQ ID NO: 101) | CPPC (SEQ ID NO: 94) | GGGSSGGGSG (SEQ ID NO: 88) |
| Human IgG1 NH3 (SEQ ID NO: 118) | EPKSSDKTHT (SEQ ID NO: 101) | CPPCPPC (SEQ ID NO: 102) | APELLGGP (SEQ ID NO: 123) |
| Human IgG1 EH3 (SEQ ID NO: 100) | EPKSSDKTHT (SEQ ID NO: 101) | CPPCPPC (SEQ ID NO: 102) | GGGSSGGGSG (SEQ ID NO: 88) |
| Human IgG1 NH4 (SEQ ID NO: 119) | EPKSSDKTHT (SEQ ID NO: 101) | CPPCVECPPC (SEQ ID NO: 105) | APELLGGP (SEQ ID NO: 123) |
| Human IgG1 EH4 (SEQ ID NO: 120) | EPKSSDKTHT (SEQ ID NO: 101) | CPPCVECPPC (SEQ ID NO: 105) | GGGSSGGGSG (SEQ ID NO: 88) |
| Human IgG1 NH5 (SEQ ID NO: 121) | EPKSSDKTHT (SEQ ID NO: 101) | CPPCPPCPPC (SEQ ID NO: 104) | APELLGGP (SEQ ID NO: 123) |
| Human IgG1 EH5 (SEQ ID NO: 103) | EPKSSDKTHT (SEQ ID NO: 101) | CPPCPPCPPC (SEQ ID NO: 104) | GGGSSGGGSG (SEQ ID NO: 88) |
| Human IgG2 NH1 (SEQ ID NO: 97) | ERK (SEQ ID NO: 86) | CCVECPPCP (SEQ ID NO: 87) | APPVAGP (SEQ ID NO: 98) |
| Human IgG2 EH1 (SEQ ID NO: 85) | ERK (SEQ ID NO: 86) | CCVECPPCP (SEQ ID NO: 87) | GGGSSGGGSG (SEQ ID NO: 88) |

(continued)

| Hinge variants (numbering with reference to FIG. 23) | | | |
|---|---|---|---|
| **Full hinge** | **Upper hinge** | **Core hinge** | **Lower hinge** |
| Human IgG2 NH2 (SEQ ID NO: 99) | ERK (SEQ ID NO: 86) | SCVECPPCP (SEQ ID NO: 91) | APPVAGP (SEQ ID NO: 98) |
| Human IgG2 EH2 (SEQ ID NO: 90) | ERK (SEQ ID NO: 86) | SCVECPPCP (SEQ ID NO: 91) | GGGSSGGGSG (SEQ ID NO: 88) |
| IgG3/IgG1 EH6 (SEQ ID NO: 106) | ELKTPLGDTTHT (SEQ ID NO: 107) | CVECPPC (SEQ ID NO: 108) | GGGSSGGGSG (SEQ ID NO: 88) |
| IgG3/IgG1 EH7 (SEQ ID NO: 109) | ELKTPLGDTTHT (SEQ ID NO: 107) | CPPCPPC (SEQ ID NO: 102) | GGGSSGGGSG (SEQ ID NO: 88) |
| IgG3/IgG1 EH8 (SEQ ID NO: 110) | ELKTPLGDTTHT (SEQ ID NO: 107) | CPPCPPCPPC (SEQ ID NO: 104) | GGGSSGGGSG (SEQ ID NO: 88) |
| IgG4 NH (SEQ ID NO: 124) | ESKYGPP (SEQ ID NO: 126) | CPPCP (SEQ ID NO: 122) | APEFLGGP (SEQ ID NO: 127) |
| **Full hinge** | **Upper hinge** | **Core hinge** | **Lower hinge** |
| IgG4 EH (SEQ ID NO: 146) | ESKYGPP (SEQ ID NO: 126) | CPPCP (SEQ ID NO: 122) | GGGSSGGGSG (SEQ ID NO: 88) |

Some embodiments of components of an antigen binding construct are shown in FIG. 24, which shows protein sequence information of various embodiments of $C_H3$ domains.

FIG. 25 shows an alignment of protein sequences of an embodiment each of IAB22M γ1 EH1(M1) and IAB22M γ 1 EH3(M1). Sequence differences are shown in boxes. In some embodiments, one or more of these constructs can be used, or an alternative construct that includes the conserved sequences between the various constructs.

FIG. 26 shows the DNA and translated protein sequence of an embodiment of IAB22M γ1 EH3(M1). In boxes are shown the signal, CDR, linker and hinge sequences. In some embodiments, one or more of these components can be employed, either together or separately, in any one or more of the methods provided herein or formulations provided herein.

FIG. 27 shows the DNA and translated protein sequence of an embodiment of IAB22M γ1 EH5(M1). In some embodiments, one or more of these components can be employed, either together or separately, in any one or more of the methods provided herein or formulations provided herein.

FIG. 28 shows the DNA and translated protein sequence of an embodiment of IAB22M γ1 EH7(M1). In some embodiments, one or more of these components can be employed, either together or separately, in any one or more of the methods provided herein or formulations provided herein.

FIG. 29 shows the DNA and translated protein sequence of an embodiment of IAB22M γ1 EH8(M1). In some embodiments, one or more of these components can be employed, either together or separately, in any one or more of the methods provided herein or formulations provided herein.

FIG. 30 shows the DNA and translated protein sequence of an embodiment of IAB22M γ2 EH2(M1). In some embodiments, one or more of these components can be employed, either together or separately, in any one or more of the methods provided herein or formulations provided herein.

FIG. 31 shows the DNA and translated protein sequence of an embodiment of IAB22M γ2 EH2(M1) with VH-K67R polymorphism. In some embodiments, one or more of these components can be employed, either together or separately, in any one or more of the methods provided herein or formulations provided herein.

FIG. 32 shows the protein sequence of an embodiment of IAB22M VH domain. In some embodiments, one or more of the components of this construct can be employed, either together or separately, in any one or more of the methods provided herein or formulations provided herein.

As described herein, the antigen binding construct can bind to CD8. These CD8 proteins are known in the art. Examples of such proteins include, for example CD8 (such as the α-chain) (FIG. 33; SEQ ID NO: 134) and CD8 (such as the β-chain) (FIG. 34; SEQ ID NO: 135).

FIG. 35 shows an illustration of an engineered minibody (Mb). In some embodiments, this construct can be employed in one or more of the arrangements (methods, formulations, etc.) provided herein.

FIG. 36 shows an illustration of various embodiments of Mb hinges based on human IgG1 (γ 1 EH1 top and γ

1 EH2 bottom). In some embodiments, these components can be employed in one or more of the arrangements (methods, formulations, etc.) of a CD8 antigen binding construct provided herein.

FIG. 37 shows an illustration of various embodiments of Mb hinges based on human IgG2 (γ2 EH1 top and γ2 EH2 bottom). In some embodiments, these components can be employed in one or more of the arrangements (methods, formulations, etc.) of a CD8 antigen binding construct provided herein.

FIG. 38 shows an illustration of various embodiments of additional Mb hinges based on human IgG2 (γ2 NH1 top and γ2 NH2 bottom). In some embodiments, these components can be employed in one or more of the arrangements (methods, formulations, etc.) of a CD8 antigen binding construct provided herein.

FIG. 39 depicts some embodiments of a CD8 minibody. In some embodiments, this construct (or a variant thereof) can be employed in any of the other arrangements (e.g., methods, formulations, etc.) provided herein. In some embodiments, this molecule is an improvement over other CD8 binding constructs, when used in one or more of the methods provided herein or as part of one or more of the formulations provided herein. In some embodiments, this molecule is linked, via a cysteine to a detectable marker, and typically two detectable markers, of:

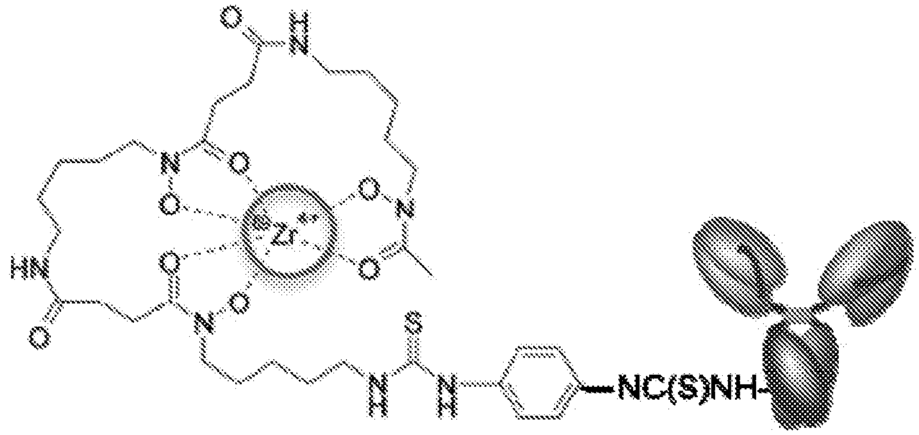

or

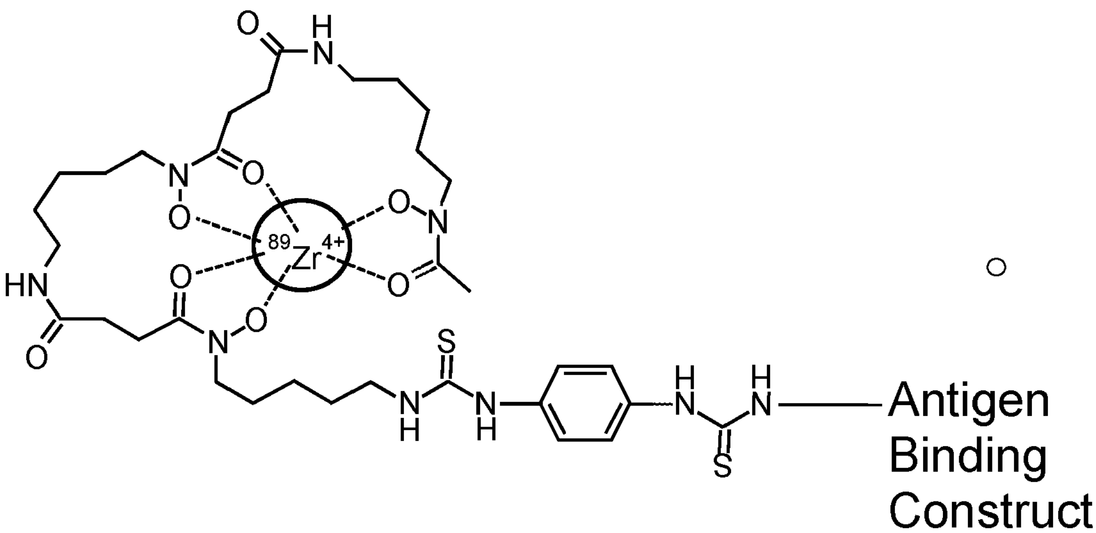

.

***Antigen Binding Construct Modifications***

In some embodiments, the antigen binding construct includes at least one modification. Exemplary modifications include, but are not limited to, antigen binding constructs that have been modified by glycosylation, acetylation, pegylation, phosphorylation, amidation, derivatization by known protecting/blocking groups, proteolytic cleavage, and linkage to a cellular ligand or other protein. Any of numerous chemical modifications may be carried out by known techniques, including, but not limited to, specific chemical cleavage, acetylation, formylation and metabolic synthesis of tunicamycin.

In some embodiments, the derivative can contain one or more non-natural amino acids.

In some embodiments, the antigen binding construct is conjugated to another substance to form an anti-target conjugate. The conjugates described herein can be prepared by known methods of linking antigen binding constructs with lipids, carbohydrates, protein or other atoms and molecules. In some embodiments, the conjugate is formed by site-specific conjugation using a suitable linkage or bond. Site-specific conjugation is more likely to preserve the binding activity of an antigen binding construct. The substance may be conjugated or attached at the hinge region of a reduced antigen binding construct via disulfide bond formation. For example, introduction of cysteine residues at the C-terminus of a scFv fragment, such as those that can be introduced in the cys-diabodies described herein, allows site-specific thiol-reactive coupling at a site away from the antigen binding site to a wide variety of agents. Other linkages or bonds used to form the conjugate can include, but are not limited to, a covalent bond, a noncovalent bond, a sulfide linkage, a hydrazone linkage, a hydrazine linkage, an ester linkage, an amido linkage, and amino linkage, an imino linkage, a thiosemicabazone linkage, a emicarbazone linkage, an oxime linkage and a carbon-carbon linkage.

***Detectable markers***

In some embodiments, a modified antigen binding construct is conjugated to a detectable marker. As used herein, a "detectable marker" includes an atom, molecule, or compound that is useful in diagnosing, detecting or visualizing a location and/or quantity of a target molecule, cell, tissue, organ and the like. Detectable markers that can be used in accordance with the embodiments herein include, but are not limited to, radioactive substances (e.g., radioisotopes, radionuclides, radiolabels or radiotracers), dyes, contrast agents, fluorescent compounds or molecules, bioluminescent compounds or molecules, enzymes and enhancing agents (e.g., paramagnetic ions). In addition, some nanoparticles, for example quantum dots and metal nanoparticles (described below) can be suitable for use as a detection agent. In some embodiments, the detectable marker is IndoCyanine Green (ICG). When used in a particular context (for example, PET), then a detectable marker that is visible via PET is employed.

Exemplary radioactive substances that can be used as detectable markers in accordance with the embodiments herein include, but are not limited to, $^{18}F$, $^{18}F$-FAC, $^{32}P$, $^{33}P$, $^{45}Ti$, $^{47}Sc$, $^{52}Fe$, $^{59}Fe$, $^{62}Cu$, $^{64}Cu$, $^{67}Cu$, $^{67}Ga$, $^{68}Ga$, $^{75}Sc$, $^{77}As$, $^{86}Y$, $^{90}Y$, $^{89}Sr$, $^{89}Zr$, $^{94}Tc$, $^{94}Tc$, $^{99}mTc$, $^{99}Mo$, $^{105}Pd$, $^{105}Rh$, $^{111}Ag$, $^{111}In$, $^{123}I$, $^{124}I$, $^{125}I$, $^{131}I$, $^{142}Pr$, $^{143}Pr$, $^{149}Pm$, $^{153}Sm$, $^{154-158}Gd$, $^{161}Tb$, $^{166}Dy$, $^{166}Ho$, $^{169}Er$, $^{175}Lu$, $^{177}Lu$, $^{186}Re$, $^{188}Re$, $^{189}Re$, $^{194}Ir$, $^{198}Au$, $^{199}Au$, $^{211}At$, $^{211}Pb$, $^{212}Bi$, $^{212}Pb$, $^{213}Bi$, $^{223}Ra$ and $^{225}Ac$. Exemplary Paramagnetic ions substances that can be used as detectable markers include, but are not limited to ions of transition and lanthanide metals (e.g. metals having atomic numbers of 6 to 9, 21-29, 42, 43, 44, or 57-71). These metals include ions of Cr, V, Mn, Fe, Co, Ni, Cu, La, Ce, Pr, Nd, Pm, Sm, Eu, Gd, Tb, Dy, Ho, Er, Tm, Yb and Lu.

When the detectable marker is a radioactive metal or paramagnetic ion, in some embodiments, the marker can be reacted with a reagent having a long tail with one or more chelating groups attached to the long tail for binding these ions. The long tail can be a polymer such as a polylysine, polysaccharide, or other derivatized or derivatizable chain having pendant groups to which may be bound to a chelating group for binding the ions. Examples of chelating groups that may be used according to the embodiments herein include, but are not limited to, ethylenediaminetetraacetic acid (EDTA), diethylenetriaminepentaacetic acid (DTPA), DOTA, NOTA, NOGADA, NETA, deferoxamine (DfO), porphyrins, polyamines, crown ethers, bis-thiosemicarbazones, polyoximes, and like groups. The chelate can be linked to the antigen binding construct by a group which allows formation of a bond to the molecule with minimal loss of immunoreactivity and minimal aggregation and/or internal cross-linking. The same chelates, when complexed with non-radioactive metals, such as manganese, iron and gadolinium are useful for MRI, when used along with the antigen binding constructs and carriers described herein. Macrocyclic chelates such as NOTA, NOGADA, DOTA, and TETA are of use with a variety of metals and radiometals including, but not limited to, radionuclides of gallium, yttrium and copper, respectively. Other ring-type chelates such as macrocyclic polyethers, which are of interest for stably binding radionuclides, such as Radium-223 for RAIT may be used. In certain embodiments, chelating moieties may be used to attach a PET imaging agent, such as an Aluminum-$^{18}F$ complex, to a targeting molecule for use in PET analysis.

Exemplary contrast agents that can be used as detectable markers in accordance with the embodiments of the disclosure include, but are not limited to, barium, diatrizoate, ethiodized oil, gallium citrate, iocarmic acid, iocetamic acid, iodamide, iodipamide, iodoxamic acid, iogulamide, iohexyl, iopamidol, iopanoic acid, ioprocemic acid, iosefamic acid, ioseric acid, iosulamide meglumine, iosemetic acid, iotasul, iotetric acid, iothalamic acid, iotroxic acid, ioxaglic acid, ioxotrizoic acid, ipodate, meglumine, metrizamide, metrizoate, propyliodone, thallous chloride, or combinations thereof.

Bioluminescent and fluorescent compounds or molecules and dyes that can be used as detectable markers in accordance with the embodiments of the disclosure include, but are not limited to, fluorescein, fluorescein isothiocyanate (FITC), OREGON GREEN™, rhodamine, Texas red, tetrarhodimine isothiocynate (TRITC), Cy3, Cy5, and the like), fluorescent markers (e.g., green fluorescent protein (GFP), phycoerythrin, and the like), autoquenched fluorescent compounds that are activated by tumor-associated proteases, enzymes (e.g., luciferase, horseradish peroxidase, alkaline phosphatase, and the like), nanoparticles, biotin, digoxigenin or combination thereof.

Enzymes that can be used as detectable markers in accordance with the embodiments of the disclosure include, but are not limited to, horseradish peroxidase, alkaline phosphatase, acid phosphatase, glucose oxidase, β-galactosidase, β-glucoronidase or β-lactamase. Such enzymes may be used in combination with a chromogen, a fluorogenic compound or a luminogenic compound to generate a detectable signal.

In some embodiments, the antigen binding construct is conjugated to a nanoparticle. The term "nanoparticle" refers to a microscopic particle whose size is measured in nanometers, e.g., a particle with at least one dimension less than about 100 nm. Nanoparticles can be used as detectable substances because they are small enough to scatter visible light rather than absorb it. For example, gold nanoparticles possess significant visible light extinction properties and appear deep red to black in solution. As a result, compositions comprising antigen binding constructs conjugated to nanoparticles can be used for the in vivo imaging of T-cells in a subject. At the small end of the size range, nanoparticles are often referred to as clusters. Metal, dielectric, and semiconductor nanoparticles have been formed, as well as hybrid structures (e.g. core-shell nanoparticles). Nanospheres, nanorods, and nanocups are just a few of the shapes that have been grown. Semiconductor quantum dots and nanocrystals are examples of additional types of nanoparticles. Such nanoscale particles, when conjugated to an antigen binding construct, can be used as imaging agents for the in vivo detection of T-cells as described herein.

*Kits*

In some embodiments, kits are provided. In some embodiments, the kit includes an antigen binding construct as described herein. In some embodiments, the kit includes a nucleic acid that encodes an antigen binding construct as described herein. In some embodiments, the kit includes a cell line that produces an antigen binding construct as described herein. In some embodiments, the kit includes a detectable marker as described herein. In some embodiments, the kit includes a therapeutic agent as described herein. In some embodiments, the kit includes buffers. In some embodiments, the kit includes positive controls, for example CD8, CD8+ cells, or fragments thereof. In some embodiments, the kit includes negative controls, for example a surface or solution that is substantially free of CD8. In some embodiments, the kit includes packaging. In some embodiments, the kit includes instructions. In some embodiments, the kit includes documentation or a piece of paper or other medium for one or more of the following regarding the compositions: date received, date dispensed, quantity dispensed, and the patient identification number to whom the drug was dispensed.

***Nucleic Acids***

In some embodiments, the polypeptides of the antigen binding constructs can be encoded by nucleic acids and expressed *in vivo* or *in vitro,* or these peptide can be synthesized chemically. Thus, in some embodiments, a nucleic acid encoding an antigen binding construct is provided. In some embodiments, the nucleic acid encodes one part or monomer of a cys-diabody or minibody. In some embodiments, the nucleic acid encodes two or more monomers, for example, at least 2 monomers. Nucleic acids encoding multiple monomers can include nucleic acid cleavage sites between at least two monomers, can encode transcription or translation start site between two or more monomers, and/or can encode proteolytic target sites between two or more monomers.

In some embodiments, an expression vector contains a nucleic acid encoding an antigen binding construct as disclosed herein. In some embodiments, the expression vector includes pcDNA3.1™/myc-His (-) Version A vector for mammalian expression (Invitrogen, Inc.), or a variant thereof (*see* FIG. 13). The pcDNA3.1 expression vector features a CMV promoter for mammalian expression and both mammalian (Neomycin) and bacterial (Ampicillin) selection markers (see FIG. 10). In some embodiments, the expression vector includes a plasmid. In some embodiments, the vector includes a viral vector, for example a retroviral or adenoviral vector. In embodiments, the vector includes a cosmid, YAC, or BAC.

In some embodiments, the nucleotide sequence encoding at least one of the minibody monomers comprises at least one of SEQ ID NOs: 17, 19, 21, 23, 39, 41, 43, 45, 47, 49, 51, or a sequence having at least about 80% identity, for example about 80%, 85%, 86%, 87%, 88%, 89%, 90%, 91%, 92%, 93, 94, 95%, 96%, 97%, 98%, 99%, or greater identity thereto.

In some embodiments, the nucleotide sequence encoding at least one of the cys-diabody monomers includes SEQ ID NOs: 77, 78, 10, 11, 39, 41, 43, 45, 47, 49, 51, or a sequence having at least about 80% identity, for example about 80%, 85%, 86%, 87%, 88%, 89%, 90%, 91%, 92%, 93, 94, 95%, 96%, 97%, 98%, 99% or greater identity thereto. In some embodiments a nucleotide encoding the sequence of SEQ ID NO: 147 is provided.

**METHOD OF MAKING A COMPOSITION**

In some embodiments, a method of manufacturing a diagnostic composition is provided. The method comprises conjugating a minibody to desferrioxamine to form a Df-minibody, radiolabeling the Df-minibody with $^{89}Zr$ to form radi-

olabeled minibody, purifying the radiolabeled minibody, and mixing the radiolabeled minibody with a cold minibody to form a diagnostic composition, wherein the minibody and the cold minibody bind to a same epitope on CD8.

FIG. 40 depicts a reaction mechanism for making a labeled minibody, which is also discussed in greater detail in the examples below (e.g., Example 10).

## DATA AND ANALYSIS

In some embodiments, a non-transitory computer readable medium is provided. It comprises data stored regarding an image of a PET scan, wherein the data has been obtained by the method provided herein.

There are several methods for measuring the rate and/or total amount of FDG accumulation in tumors. PET scanners are designed to measure the in vivo radioactivity concentration [kBq/ml], which is directly linked to the radiotracer concentration. In some embodiments, however, it is the relative tissue uptake of radiotracer that is of interest. The two most significant sources of variation that occur in practice are the amount of injected radiotracer and the patient size. To compensate for these variations, at least to first order, the standardized uptake value (SUV) is commonly used as a relative measure of radiotracer uptake. The basic expression for SUV is

$$SUV=r(a'/w)$$

where *r* is the radioactivity concentration [kBq/ml] measured by the PET scanner within a region of interest (ROI), *a'* is the decay-corrected amount of injected radiolabeled tracer [kBq], and w is the weight of the patient [g], which is used a surrogate for a distribution volume of tracer. If all the injected radiotracer is retained and uniformly distributed throughout the body, the SUV everywhere will be 1 g/ml regardless of the amount of radiotracer injected or patient size. SUVs are dimensionless under the assumption that 1 ml of tissue weights 1 gm.

In some embodiments, a user draws a 2D region-of-interest (ROI) on a single cross section of image, or a 3D volume of interest (VOI) on a lesion/organ etc. across multiple sections. The image analysis software looks at the signal intensities of all the individual voxels (3D volume equivalent of pixels) within the ROI/VOI, selects the maximum value and calculates the mean value of all signal intensities. The SUVs for each voxel can be automatically calculated based on signal intensity in the particular voxel, which has been previously calibrated to represent activity concentrations of the radiotracer in question. The software then provides SUVmean or SUVmax as output values from the ROI or VOI specified.

In some embodiments, a method of determining a standard uptake value is provided. The method comprises applying an antigen binding construct to a subject, wherein the antigen binding construct comprises a radioactive probe, determining r, where r is the radioactivity concentration (kBq/ml) measured by a PET scanner within a ROI of radiation from the antigen binding construct, determining a', wherein a' is the decay-corrected amount of the injected radiolableeld tracer (kBq), determining w, the weight of the patient, and determining SUV as being the result of r(a'/W).

In some embodiments, a method of analyzing an image is provided. The method comprises providing an image, defining on the image a first region of interest (ROI) by marking the image, determining a signal intensity for a data point within the first ROI, determining a maximum signal intensity within the first ROI, determining a mean value of the signal intensities within the first ROI, and summing together each signal intensity within the first ROI to obtain a first summed signal level for the first ROI. The first ROI represents data for an amount of a detectable marker associated with an antigen binding construct that has been administered to a subject.

In some embodiments, the method further comprises repeating the process for a second ROI to determine a second summed signal level and comparing the first and the second summed signal levels. When the first summed signal level is greater than the second summed signal level, it indicates the presence of less CD8 in the second ROI.

In some embodiments, the first ROI is a same location as the second ROI, but differs in that the first ROI is provided at a first time point and the second ROI is provided at a second time point and a candidate therapeutic has been administered after the first time point but before the second time point. In some embodiments, any of the variables or options for the methods provided herein can be applied to the method of analyzing the data provided herein.

In some embodiments, a method of determining a standard uptake value comprises applying an antigen binding construct to a subject, the antigen binding construct comprising a radioactive probe, determining r, where r is the radioactivity concentration (kBq/ml) measured by a PET scanner within a ROI of radiation from the antigen binding construct, determining a', wherein a' is the decay-corrected amount of the injected radiolableeld tracer (kBq), determining w, the weight of the patient, and determining SUV as being the result of r(a'/W).

In some embodiments, a method of determining a standard uptake value comprises applying an antigen binding construct to a subject.

In some embodiments, a method of determining a standard uptake value comprises applying an antigen binding construct to a subject, the antigen binding construct compising a radioactive probe.

In some embodiments, a method of determining a standard uptake value comprises applying an antigen binding construct to a subject, the antigen binding construct compising a radioactive probe, and determining r, where r is the radioactivity concentration (kBq/ml) measured by a PET scanner within a ROI of radiation from the antigen binding construct, and determining a', wherein a' is the decay-corrected amount of the injected radiolableeld tracer (kBq), and determining w, the weight of the patient, and determining SUV as being the result of r(a'/W).In some embodiments, a method of analyzing an image comprises providing an image, defining on the image a first region of interest (ROI) by marking the image, determining a signal intensity for a data point within the first ROI, determining a maximum signal intensity within the first ROI, determining a mean value of the signal intensities within the first ROI, and summing together each signal intensity within the first ROI to obtain a first summed signal level for the first ROI. The first ROI represents data for an amount of a detectable marker associated with an antigen binding construct that has been administered to a subject. In some embodiments, a method of analyzing an image comprises providing an image, defining on the image a first region of interest (ROI) by marking the image, determining a signal intensity for a data point within the first ROI, determining a maximum signal intensity within the first ROI, determining a mean value of the signal intensities within the first ROI, and summing together each signal intensity within the first ROI to obtain a first summed signal level for the first ROI.

In some embodiments, a method of analyzing an image further comrpises repeating the method of analyzing an image for a second ROI to determine a second summed signal level and comparing the first and the second summed signal levels. Whent the first summed signal level is greater than the second summed signal level, it indicates the presence of less CD8 in the second ROI. In some embodimentsof a method of analyzing an image, the first ROI is a same location as the second ROI, but differ in that the first ROI is provided at a first time point and the second ROI is provided at a second time point and a candidate therapeutic has been administered after the first time point but before the second time point.

In some embodiments, a method of generating an image comprises applying an antigen-binding construct that binds to CD8, the antigen-binding construct comprising a detectable marker, and detecting at least one detectable marker within a location within the tumor and assigning a positive pixel for each detectable marker detected, and generating an image based on a distribution of each positive pixel assigned. The image is stored in a non-transitory computer readable media.

In some embodiments, a method of generating an image comprises applying an antigen-binding construct that binds to CD8.

In some embodiments, a method of generating an image comprises applying an antigen-binding construct that binds to CD8, the antigen-binding construct comprising a detectable marker.

In some embodiments, a method of generating an image comprises applying an antigen-binding construct that binds to CD8, the antigen-binding construct comprising a detectable marker, and detecting at least one detectable marker within a location within the tumor and assigning a positive pixel for each detectable marker detected.

In some embodiments, a method of generating an image comprises applying an antigen-binding construct that binds to CD8, the antigen-binding construct comprising a detectable marker, and detecting at least one detectable marker within a location within the tumor and assigning a positive pixel for each detectable marker detected, and generating an image based on a distribution of each positive pixel assigned.

In some embodiments, a non-transitory computer readable medium comprises data regarding an image of a PET scan, wherein the data has been obtained by the any of the imaging methods provided herein.

## IMMUNO-PET

PET imaging systems create images based on the distribution of positron-emitting isotopes in the tissue of a patient. The isotopes are typically administered to a patient by injection of probe molecules that comprise a positron-emitting isotope, such as F-18, C-11, N-13, or 0-15, covalently attached to a molecule that is readily metabolized or localized in the body (e.g., glucose) or that chemically binds to receptor sites within the body. In some cases, the isotope is administered to the patient as an ionic solution or by inhalation. Small immuno-PET imaging agents, such as Fab antibody fragments (50 kDa) or diabodies, paired dimers of the covalently associated VH-VL region of Mab, 55 kDa (Shively et al (2007) J Nucl Med 48:170-2), may be particularly useful since they exhibit a short circulation half-life, high tissue permeability, and reach an optimal tumor to background ratio between two to four hours after injection facilitating the use of short half-life isotopes such as the widely available 18F (109.8 min).

Antigen binding constructs may be conjugated with any label moiety which can be covalently attached to the antibody through a reactive moiety, an activated moiety, or a reactive cysteine thiol group (Singh et al (2002) Anal. Biochem. 304:147-15; Harlow E. and Lane, D. (1999) Using Antibodies: A Laboratory Manual, Cold Springs Harbor Laboratory Press, Cold Spring Harbor, N.Y.; Lundblad R. L. (1991) Chemical Reagents for Protein Modification, 2nd ed. CRC Press, Boca Raton, Fla.). The attached label may function to: (i) provide a detectable signal; (ii) interact with a second label to modify the detectable signal provided by the first or second label, e.g. to give FRET (fluorescence

resonance energy transfer); (iii) stabilize interactions or increase affinity of binding, with antigen or ligand; (iv) affect mobility, e.g. electrophoretic mobility or cell-permeability, by charge, hydrophobicity, shape, or other physical parameters, or (v) provide a capture moiety, to modulate ligand affinity, antibody/antigen binding, or ionic complexation.

Labelled cysteine engineered antigen binding constructs may be useful in diagnostic assays, e.g., for detecting expression of an antigen of interest in specific cells, tissues, or serum. For diagnostic applications, the antibody will typically be labeled with a detectable moiety. Numerous labels are available which can be generally grouped into the following categories:

The antibody can be labeled with ligand reagents that bind, chelate or otherwise complex a radioisotope metal where the reagent is reactive with the engineered cysteine thiol of the antibody, using the techniques described in Current Protocols in Immunology, Volumes 1 and 2, Coligen et al, Ed. Wiley-Interscience, New York, N.Y., Pubs. (1991). Chelating ligands which may complex a metal ion include DOTA, DOTP, DOTMA, DTPA and TETA (Macrocyclics, Dallas, Tex.). Radionuclides can be targeted via complexation with the antibody-drug conjugates of the invention (Wu et al (2005) Nature Biotechnology 23(9):1137-1146).

Linker reagents such as DOTA-maleimide (4-maleimidobutyramidobenzyl-DOTA) can be prepared by the reaction of aminobenzyl-DOTA with 4-maleimidobutyric acid (Fluka) activated with isopropylchloroformate (Aldrich), following the procedure of Axworthy et al (2000) Proc. Natl. Acad. Sci. USA 97(4):1802-1807). DOTA-maleimide reagents react with the free cysteine amino acids of the cysteine engineered antibodies and provide a metal complexing ligand on the antibody (Lewis et al (1998) Bioconj. Chem. 9:72-86). Chelating linker labelling reagents such as DOTA-NHS (1,4,7,10-tetraazacyclododecane-1,4,7,10-tetraacetic acid mono (N-hydroxysuccinimide ester) are commercially available (Macrocyclics, Dallas, Tex.). Receptor target imaging with radionuclide labelled antibodies can provide a marker of pathway activation by detection and quantitation of progressive accumulation of antibodies in tumor tissue (Albert et al (1998) Bioorg. Med. Chem. Lett. 8:1207-1210). The conjugated radio-metals may remain intracellular following lysosomal degradation.

Metal-chelate complexes suitable as antibody labels for imaging experiments are disclosed: U.S. Pat. No. 5,342,606; U.S. Pat. No. 5,428,155; U.S. Pat. No. 5,316,757; U.S. Pat. No. 5,480,990; U.S. Pat. No. 5,462,725; U.S. Pat. No. 5,428,139; U.S. Pat. No. 5,385,893; U.S. Pat. No. 5,739,294; U.S. Pat. No. 5,750,660; U.S. Pat. No. 5,834,456; Hnatowich et al (1983) J. Immunol. Methods 65:147-157; Meares et al (1984) Anal. Biochem. 142:68-78; Mirzadeh et al (1990) Bioconjugate Chem. 1:59-65; Meares et al (1990) J. Cancer 1990, Suppl. 10:21-26; Izard et al (1992) Bioconjugate Chem. 3:346-350; Nikula et al (1995) Nucl. Med. Biol. 22:387-90; Camera et al (1993) Nucl. Med. Biol. 20:955-62; Kukis et al (1998) J. Nucl. Med. 39:2105-2110; Verel et al (2003) J. Nucl. Med. 44:1663-1670; Camera et al (1994) J. Nucl. Med. 21:640-646; Ruegg et al (1990) Cancer Res. 50:4221-4226; Verel et al (2003) J. Nucl. Med. 44:1663-1670; Lee et al (2001) Cancer Res. 61:4474-4482; Mitchell, et al (2003) J. Nucl. Med. 44:1105-1112; Kobayashi et al (1999) Bioconjugate Chem. 10:103-111; Miederer et al (2004) J. Nucl. Med. 45:129-137; DeNardo et al (1998) Clinical Cancer Research 4:2483-90; Blend et al (2003) Cancer Biotherapy & Radiopharmaceuticals 18:355-363; Nikula et al (1999) J. Nucl. Med. 40:166-76; Kobayashi et al (1998) J. Nucl. Med. 39:829-36; Mardirossian et al (1993) Nucl. Med. Biol. 20:65-74; Roselli et al (1999) Cancer Biotherapy & Radiopharmaceuticals, 14:209-20.

(b) Fluorescent labels such as rare earth chelates (europium chelates), fluorescein types including FITC, 5-carboxyfluorescein, 6-carboxy fluorescein; rhodamine types including TAMRA; dansyl; Lissamine; cyanines; phycoerythrins; Texas Red; and analogs thereof. The fluorescent labels can be conjugated to antibodies using the techniques disclosed in Current Protocols in Immunology, supra, for example. Fluorescent dyes and fluorescent label reagents include those which are commercially available from Invitrogen/Molecular Probes (Eugene, Oreg.) and Pierce Biotechnology, Inc. (Rockford, Ill.).

(c) Various enzyme-substrate labels are available or disclosed (U.S. Pat. No. 4,275,149). The enzyme generally catalyzes a chemical alteration of a chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (e.g., firefly luciferase and bacterial luciferase; U.S. Pat. No. 4,737,456), luciferin, 2,3-dihydrophthalazinediones, malate dehydrogenase, urease, peroxidase such as horseradish peroxidase (HRP), alkaline phosphatase (AP), .beta.-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al (1981) "Methods for the Preparation of Enzyme-Antibody Conjugates for use in Enzyme Immunoassay", in Methods in Enzym. (ed J. Langone & H. Van Vunakis), Academic Press, New York, 73:147-166.

Labelled antigen binding constructs are useful as imaging biomarkers and probes by the various methods and techniques of biomedical and molecular imaging such as: (i) MRI (magnetic resonance imaging); (ii) MicroCT (computerized tomography); (iii) SPECT (single photon emission computed tomography); (iv) positron emission topography (PET) or Immuno-positron emission tomography (Immuno-PET) (see van Dongen G A, et al "Immuno-PET: a navigator in monoclonal antibody development and applications" Oncologist 2007; 12:1379-89. (v) bioluminescence; (vi) fluorescence; and (vii) ultrasound. Immunoscintigraphy is an imaging procedure in which antibodies labeled with radioactive substances are administered to an animal or human patient and a picture is taken of sites in the body where the antibody localizes (U.S. Pat. No. 6,528,624). Imaging biomarkers may be objectively measured and evaluated as an indicator of normal biological processes, pathogenic processes, or pharmacological responses to a therapeutic intervention. Biomarkers may be of several types: Type 0 are natural history markers of a disease and correlate longitudinally with known clinical indices, e.g. MRI assessment of synovial inflammation in rheumatoid arthritis; Type I markers capture the effect of an intervention in accordance with a mechanism-of-action, even though the mechanism may not be associated with clinical outcome; Type II markers function as surrogate endpoints where the change in, or signal from, the biomarker predicts a clinical benefit to "validate" the targeted response, such as measured bone erosion in rheumatoid arthritis by CT. Imaging biomarkers thus can provide pharmacodynamic (PD) therapeutic information about: (i) expression of a target protein, (ii) binding of a therapeutic to the target protein, i.e. selectivity, and (iii) clearance and half-life pharmacokinetic data. Advantages of in vivo imaging biomarkers relative to lab-based biomarkers include: non-invasive treatment, quantifiable, whole body assessment, repetitive dosing and assessment, i.e. multiple time points, and potentially transferable effects from preclinical (small animal) to clinical (human) results. For some applications, bioimaging supplants or minimizes the number of animal experiments in preclinical studies.

Peptide labelling methods are well known. See Haugland, 2003, Molecular Probes Handbook of Fluorescent Probes and Research Chemicals, Molecular Probes, Inc.; Brinkley, 1992, Bioconjugate Chem. 3:2; Garman, (1997) Non-Radioactive Labelling: A Practical Approach, Academic Press, London; Means (1990) Bioconjugate Chem. 1:2; Glazer et al (1975) Chemical Modification of Proteins. Laboratory Techniques in Biochemistry and Molecular Biology (T. S. Work and E. Work, Eds.) American Elsevier Publishing Co., New York; Lundblad, R. L. and Noyes, C. M. (1984) Chemical Reagents for Protein Modification, Vols. I and II, CRC Press, New York; Pfleiderer, G. (1985) "Chemical Modification of Proteins", Modern Methods in Protein Chemistry, H. Tschesche, Ed., Walter DeGryter, Berlin and New York; and Wong (1991) Chemistry of Protein Conjugation and Cross-linking, CRC Press, Boca Raton, Fla.); De Leon-Rodriguez et al (2004) Chem. Eur. J. 10:1149-1155; Lewis et al (2001) Bioconjugate Chem. 12:320-324; Li et al (2002) Bioconjugate Chem. 13:110-115; Mier et al (2005) Bioconjugate Chem. 16:237-240.

Peptides and proteins labelled with two moieties, a fluorescent reporter and quencher in sufficient proximity undergo fluorescence resonance energy transfer (FRET). Reporter groups are typically fluorescent dyes that are excited by light at a certain wavelength and transfer energy to an acceptor, or quencher, group, with the appropriate Stokes shift for emission at maximal brightness. Fluorescent dyes include molecules with extended aromaticity, such as fluorescein and rhodamine, and their derivatives. The fluorescent reporter may be partially or significantly quenched by the quencher moiety in an intact peptide. Upon cleavage of the peptide by a peptidase or protease, a detectable increase in fluorescence may be measured (Knight, C. (1995) "Fluorimetric Assays of Proteolytic Enzymes", Methods in Enzymology, Academic Press, 248:18-34).

## Biopsy:

In some embodiments, a method of performing a biopsy in a subject comprises providing an antigen binding construct that binds to CD8 to the subject, selecting, based upon an elevated level of the antigen binding construct within a particular tumor, the particular tumor for a biopsy, and collecting a sample from the particular tumor. In any of the CD8 detection embodiments provided herein, the method can be performed without a biopsy in some embodiments. Similarly, in some embodiments, any of the methods employing CD8 detection (e.g., via CD8 antigen binding constructs or CD8 PET tracers, etc), the method can be performed without an invasive technique or method (e.g., no IHC involved or required).

In some embodiments, a method of performing a biopsy in a subject comprises providing an antigen binding construct that binds to CD8 to the subject.

In some embodiments, a method of performing a biopsy in a subject comprises providing an antigen binding construct that binds to CD8 to the subject, and selecting, based upon an elevated level of the antigen binding construct within a particular tumor, the particular tumor for a biopsy.

In some embodiments, a method of performing a biopsy in a subject comprises providing an antigen binding construct that binds to CD8 to the subject, and selecting, based upon an elevated level of the antigen binding construct within a particular tumor, the particular tumor for a biopsy, and collecting a sample from the particular tumor.

In some embodiments, a method of performing a biopsy in a subject comprises providing an antigen binding construct that binds to CD8 to the subject, selecting, based upon a distribution the antigen binding construct within a particular tumor, the particular location within the tumor to biopsy, and collecting a sample from the particular location within the tumor. The particular location is either internal to the tumor or on a surface of the tumor.

In some embodiments, a method of performing a biopsy in a subject comprises providing an antigen binding construct that binds to CD8 to the subject.

In some embodiments, a method of performing a biopsy in a subject comprises providing an antigen binding construct that binds to CD8 to the subject, and selecting, based upon a distribution the antigen binding construct within a particular tumor, the particular location within the tumor to biopsy.

In some embodiments, a method of performing a biopsy in a subject comprises providing an antigen binding construct that binds to CD8 to the subject, and selecting, based upon a distribution the antigen binding construct within a particular tumor, the particular location within the tumor to biopsy, and collecting a sample from the particular location within the tumor.

## irAE

In some embodiments, a method of detecting an immune-related adverse event (irAE) comprises identifying a human subject on a checkpoint inhibitor therapy, administer a CD8 antigen binding construct to the human subject, the CD8 antigen binding construct comprising a detectable label, monitoring the detectable label within the subject to determine if there is immune related toxicity. irAE may result from the fact that many IOTs targeting the PD-1 - PD-L1 axis (or other targets) can also promote an immune attack on normal tissues, including the gastrointestinal tract, lung, and thyroid. Such immune-related adverse events occur at a significant frequency and, in some patients, with severe and sometimes fatal consequences. The extended in vivo t1/2 of humanized CPIs has been implicated in contributing to these toxicities. The immune related toxicity is indicated by an increase in CD8 binding localization. The invention therefore provides a rapid and non-invasive method to identify irAEs in non-tumor tissues caused by CPIs. The method employs immuno-PET with a CD8 antigen binding construct to identify aberrant accumulation of CD8 bearing cells in non-tumour tissue in a patient receiving CPI therapy. If the immune related toxicity is severe or irreversible, stopping the checkpoint inhibitor therapy, if the immune related toxicity is not severe or irreversible, suspending the checkpoint inhibitor therapy while continuing to monitor CD8 levels until they return to a lower levelIn some embodiments, a method of detecting an irAE comprises identifying a human subject on a checkpoint inhibitor therapy.In some embodiments of a method of detecting an irAE, a location of a tumor and indicates which organ or system is experiencing an irAE.

## ADDITIONAL EMBODIMENTS THAT CAN BE COMBINED/SUBSTITUTED INTO ANY OF THE ABOVE

In some embodiments, for any of the methods provided herein, a radiation dose is administered in an amount of 0.5-3.0 mCi +/- 20%. In some embodiments, for any of the methods provided herein, a radiation dose is administered in an amount of 0.5-3.0 mCi +/- 10%. In some embodiments, for any of the methods provided herein, a radiation dose is administered in an amount of 0.5-3.0 mCi +/- 5%.

In some embodiments, for any of the methods provided herein, a radiation dose is administered in an amount of 0.4-3.6 mCi. In some embodiments, for any of the methods provided herein, a radiation dose is administered in an amount of 0.45-3.3 mCi. In some embodiments, for any of the methods provided herein, a radiation dose is administered in an amount of 0.475-3.15 mCi.

In some embodiments, for any of the methods provided herein, an amount of the antigen binding construct is 0.2 - 10 mg. In some embodiments, for any of the methods provided herein, an amount of the antigen binding construct is about 0.1, 0.2. 0.5, 1, 2.5, 5, 7.5, 10, 12.5, 15, 17.5, or 20 mg, or a value within a range defined by any two of the aforementioned values.

In some embodiments, for any of the methods provided herein, the CD8 antigen binding construct is less than 105 kDa in size. In some embodiments, for any of the methods provided herein, the CD8 antigen binding construct is about 75, 80, 85, 90, 95, 100, or less than 105 kDa in size, or a value within a range defined by any two of the aforementioned values.

In some embodiments, for any of the methods provided herein, the CD8 antigen binding construct is linked to $^{89}Zr$.

In some embodiments, for any of the methods provided herein, 6 to 36 hours passes after administration of an antigen binding construct prior to a subsequent step in the process. In some embodiments, for any of the methods provided herein, about 6, 9, 12, 15, 18, 21, 24, 27, 30, 33, or 36 hours passes after administration of an antigen binding construct prior to a subsequent step in the process, or a value within a range defined by any two of the aforementioned values.

In some embodiments, for any of the methods provided herein, a PET scan time is less than 40 minutes. In some embodiments, for any of the methods provided herein, a PET scan time is about 30, 25, 20, 15, 10, or 5 minutes, or a value within a range defined by any two of the aforementioned values.

In some embodiments, for any of the methods provided herein, a PET scan time is less than 25 minutes and more than 2 minutes. In some embodiments, for any of the methods provided herein, a PET scan time is less than 25 minutes and more than 5 minutes. In some embodiments, for any of the methods provided herein, a PET scan time is

less than 25 minutes and more than 10 minutes. In some embodiments, for any of the methods provided herein, a PET scan time is less than 25 minutes and more than 15 minutes. In some embodiments, for any of the methods provided herein, a PET scan time is less than 25 minutes and more than 20 minutes.

In some embodiments, for any of the methods provided herein, the subject receives a therapeutic, and wherein the therapeutic is selected from at least one of: eftilagimod alpha, relatlimab, OMP-313M32, EOS8844488, TJT6, AB154, and dual TIGIT/PD-L1-targeted small-molecule drugs.

In some embodiments, for any of the methods provided herein, the subject receives a therapeutic, and wherein the therapeutic is selected from at least one of: an antibody to LAG-3, and antibody to TIGIT, MK-4280, MK-7684, and/or any of the preceding in combination with Keytruda.

In some embodiments, for any of the methods provided herein, the subject receives a therapeutic, and wherein the therapeutic is selected from at least one of: EOS8844488, TJT6, AB154, Aurigene, and/or a dual TIGIT/PD-L1 inhibitor.

In some embodiments, for any of the methods provided herein, the status is selected from one or more of: (A) i) immune inflamed or ii) immune excluded or immune desert, (B) homogeneous infiltration, heterogeneous infiltration, and/or (C) for inflamed tumors: high, medium, low degree of inflammation.

In some embodiments, for any of the methods provided herein, at least 30% of a subject's body is scanned via PET for CD8+ TIL load. In some embodiments, for any of the methods provided herein, at least 40% of a subject's body is scanned via PET for CD8+ TIL load. In some embodiments, for any of the methods provided herein, at least 50% of a subject's body is scanned via PET for CD8+ TIL load. In some embodiments, for any of the methods provided herein, at least 60% of a subject's body is scanned via PET for CD8+ TIL load. In some embodiments, for any of the methods provided herein, at least 70% of a subject's body is scanned via PET for CD8+ TIL load. In some embodiments, for any of the methods provided herein, at least 80% of a subject's body is scanned via PET for CD8+ TIL load. In some embodiments, for any of the methods provided herein, at least 90% of a subject's body is scanned via PET for CD8+ TIL load. In some embodiments, for any of the methods provided herein, 100% of a subject's body is scanned via PET for CD8+ TIL load.

In some embodiments, for any of the methods provided herein, further comprising a treatment that alters CD8+ T cell presence, CD8+ T cell activation, CD8+ T cell number or CD8+ T cell trafficking in relation to tumor lesions.

In some embodiments, for any of the methods provided herein, the treatment comprises at least one of chemotherapies, oncolytic viruses, vaccines, radiation therapy, cellular therapies etc.

In some embodiments, for any of the methods provided herein, or for any of the compositions provided herein, wherein the CD8 minibody comprises a heavy chain variable region and a light chain variable region, and wherein the heavy chain variable region consists essentially of a human amino acid sequence and the light chain variable region consists essentially of a human amino acid sequence.

In some embodiments, for any of the methods provided herein, the CD8 minibody binds to a CD8 sequence consisting of the sequence of at least one of: SEQ ID NOs: 24, 134, and/or 135 (FIGs. 34 and 33).

In some embodiments, for any of the methods provided herein, the minibody comprises the heavy chain CDRs in any of the CDR SEQ ID Nos: provided herein, and the light chain CDRs in any of the CDR SEQ ID Nos: provided herein.

In some embodiments, for any of the methods provided herein, the minibody comprises a heavy chain variable region that is at least 80% identical to the heavy chain amino acid sequence in any of the heavy chain SEQ ID Nos: provided herein, and a light chain variable region that is at least 80% identical to the light chain amino acid sequence in any of the heavy chain SEQ ID NOs: provided herein.

In any of the embodiments of the methods herein, the known tumor is selected from one or more cancer of: carcinoma, lymphoma, blastoma, sarcoma, and leukemia, lymphoid malignancies, squamous cell cancer (e.g. epithelial squamous cell cancer), lung cancer, small-cell lung cancer, non-small cell lung cancer, adenocarcinoma of the lung and squamous carcinoma of the lung, cancer of the peritoneum, hepatocellular cancer, gastric or stomach cancer, gastrointestinal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain, head and neck cancer, adult and pediatric solid cancers, head and neck squamous cell carcinoma, classical Hodgkin lymphoma, primary mediastinal large B-cell lymphoma, urothelial carcinoma, microsatellite instability-high cancer, cervical cancer, Merkel cell carcinoma, esophageal cancer, Cutaneous Squamous Cell Carcinoma, prostate cancer, nasopharyngeal cancer, small cell lung mesothelioma, Primary Mediastinal B-Cell Lymphoma and solid tumors.

In some embodiments, an antigen-binding construct for use, or for use as a medicament, in the method described herein is provided.

In some embodiments, a CD8 PET tracer for use, or for use as a medicament, in the method described herein is provided.

In some embodiments, the antigen-binding construct or the CD8 PET tracer has a radiolabel that provides more than 0.5 but less than 1.0 mCi of radiation. In some embodiments, the antigen-binding construct or the CD8 PET tracer

has a radiolabel that provides less than 0.75 mCi of radiation.

In some embodiments, the formulation, the antigen-binding construct, or the CD8 PET tracer, is for use in at least one of: imaging, detection, diagnosis, surgery, staging, treatment, monitoring of treatment, monitoring of disease progression, and monitoring therapy.

In some embodiments, the composition, the formulation, the antigen-binding construct of, or the CD8 PET tracer, is for use in at least one of: imaging, detection, diagnosis, surgery, staging, treatment, monitoring of treatment, monitoring of disease progression, and monitoring therapy of a CD8 dependent disorder, optionally cancer.

## Additional Embodiments

In some embodiments, a dynamic workflow involving patient care cycle and diagnostic tests for screening and patient selection is provided (FIG 56).

In some embodiments, a patient with cancer is identified (5608).

In some embodiments, a patient identified with cancer (5608) is diagnosed and staged (5609) based on cancer type, imaging data (e.g., CT, PET, MRI, US), tumor bipsy an pathology, lab tests, molecular diagnostic tests (e.g., PCR, sequencing).

In some embodiments, a tumor biopsy (5610) from a patient identified with cancer (5608) is diagnosed and staged (5609) and used for screening and patient selection based on testing the tumor biopsy for standard of care markers and additional biomakrers (5611).

In some embodiments, a tumor tissue biopsy (5610) from a patient identified with cancer (5608) is diagnosed and staged (5609) and screened for PD-L1 IHC (5601), which is a prognostic marker for CPI therapythat target PD-1 or PD-L1. In some embodiments, Pembrolizumab or an alternative is indicated for PD-L1 positive NSCLC, gastric, and cervical cancers.

In some embodiments, a tumor tissue biopsy (5610) from a patient identified with cancer (5608) is diagnosed and staged (5609) and screened for mcrosatellite Instability (MSI) and Mismatch Repair Deficiency (dMMR) (5602), which is used as prognostic markers for CPI therapy with several CPIs indicated for MSI-H or dMMR patients. In some embodiments, Pembrolizumab or an alternative is indicated for use in all MSI-H tumors. In some embodiments, Nivolumab or an alternative is indicated for MSI-H or dMMR metastatic CRC.

In some embodiments, tumor biopsy (5610) from a patient identified with cancer (5608) is diagnosed and staged (5609) and screened for TMB (5603), which is an emerging marker for Tumor Mutation Burden (FICDx). In some embodiments, high TMB may be prognostic for response to CPI therapy. In some embodiments, there may be challenges to implement TMB as a clinical test due to variability by tumor type and test, and definition of cutoff metrics.

In some embodiments, tumor biopsy (5610) from a patient identified with cancer (5608) is diagnosed and staged (5609) and screened for Tumor Immune Markers by IHC (TIM) (5604). In some embodiments, TIM is used to assess whether tumor was immunologically active, e.g., presence and type of Tumor Infiltrating Lymphocytes (TILs).

In some embodiments, based of the information obtained from testing the tumor biopsy (5610), it is determined whether the tumor type and stage are CPI eligible (5612).

In some embodiments, based of the information obtained from testing the tumor biopsy (5610), it is determined if the tumor contains actionable driver mutations (5613).

In some embodiments, based of the information obtained from testing the tumor biopsy (5610), it is determined whether the tumor type and stage are CPI eligible (5612) and if the tumor contains actionable driver mutations (5613).

In some embodiments, if the tumor contained actionable driver mutations (5613), mutation-specific therapy (5615) is resorted to, which entails consideration of immunotherapy in combination with, or as second-line after, targeted therapy (5616).

In some embodiemnts, if the tumor type and stage are CPI eligible (5612), a CPI eligibility screening is performed (5614), which entails screening for PD-L1 by IHC (5601), Microsatellite Instability (MSI) and Mismatch Repair Deficiency (dMMR) (5602), and TMB (5603), and assessment of additional biomarkers such as Circulating Tumor Cells (CTC) (5605), PDL-1 PET imaging (5606), markers of T-ell activity (Granzyme-B, VisAct, or IL2) (5607), and clinical assessment.

In some embodiemnts, if the tumor did not contain actionable driver mutations (5613), a CPI eligibility screening is performed (5614), which entails screening for PD-L1 by IHC (5601), Microsatellite Instability (MSI) and Mismatch Repair Deficiency (dMMR) (5602), and TMB (5603), and assessment of additional biomarkers such as Circulating Tumor Cells (CTC) (5605), PDL-1 PET imaging (5606), markers of T-ell activity (Granzyme-B, VisAct, or IL2) (5607), and clinical assessment.

In some embodiments, assessment of additional biomarkers involves screening a patient blood sample for CTC (Circulating Tumor Cells) (5605).

In some embodiments, assessment of additional biomarkers involves imaging a tumor tissue biopsy for PD-L1 by PET (5606) as a potential alternative to PDL-1 IHC test.

In some embodiments, assessment of additional biomarkers involves imaging a tumor tissue biopsy for T-Cell

Activity (5607) by determining Granzyme B, VisAct® (Cell Slight), and IL2 by intracellular cytokine statining.

In some embodiments, based on a CPI eligibility screening (5614) for standard and additional biomarkers (5617), a determination is made on whether or not a patient should be indicated for CPI therapy (5621).

In some embodiments, based on a CPI eligibility screening (5614) for PD-L1 IHC (5601) and PD-L1 by PET (5606), it is determined if the cancer is found to be PD-L1+ (5618). If the cancer is found to be PD-L1+ (5618), a determination is made on whether or not a patient should be indicated for CPI therapy (5621), i.e., whether the patient should be indicated for CPI Therapy (5623).

In some embodiments, based on a CPI eligibility screening (5614) for PD-L1 IHC (5601) and PD-L1 by PET (5606), it is determined if the cancer is found to be PD-L1+ (5618). If the cancer is found to be PD-L1+ (5618), a determination is made on whether or not a patient should be indicated for CPI therapy (5621), i.e., whether the patient should be indicated for Alternate/Other therapies (5622).

In some embodiments, based on a CPI eligibility screening (5614) for PD-L1 IHC (5601) and PD-L1 by PET (5606), it is determined if the cancer is found to be PD-L1+ (5618). If the cancer is found to be PD-L1+ (5618), a determination is made on whether or not a patient should be indicated for CPI therapy (5621), i.e., whether the patient should be indicated for CPI Therapy (5622) and Alternate/Other therapies (5623).

In some embodiments, based on a CPI eligibility screening (5614) for mcrosatellite Instability (MSI) and Mismatch Repair Deficiency (dMMR) (5602), and TMB (5603), it is determined if the cancer is found to have a high mutation load (5619). If the cancer is found to have a high mutation load (5619), a determination is made on whether or not a patient should be indicated for CPI therapy (5621), i.e., whether the patient should be indicated for CPI Therapy (5622).

In some embodiments, based on a CPI eligibility screening (5614) for mcrosatellite Instability (MSI) and Mismatch Repair Deficiency (dMMR) (5602), and TMB (5603), it is determined if the cancer is found to have a high mutation load (5619). If the cancer is found to have a high mutation load (5619), a determination is made on whether or not a patient should be indicated for CPI therapy (5621), i.e., whether the patient should be indicated or Alternate/Other therapies (5623).

In some embodiments, based on a CPI eligibility screening (5614) for mcrosatellite Instability (MSI) and Mismatch Repair Deficiency (dMMR) (5602), and TMB (5603), it is determined if the cancer is found to have a high mutation load (5619). If the cancer is found to have a high mutation load (5619), a determination is made on whether or not a patient should be indicated for CPI therapy (5621), i.e., whether the patient should be indicated for CPI Therapy (5623) and Alternate/Other therapies (5622).

In some embodiments, based on a CPI eligibility screening (5614) for Tumor Immune Markers (5604), and T-Cell Activity (5607), it is determined if the cancer immune microenvironment has a hot or cold tumor immune response (5620). If the the cancer shows a "hot" tumor immune response (5620), a determination is made (5621) on whether or not a patient should be indicated for CPI Therapy (5622).

In some embodiments, based on a CPI eligibility screening (5614) for Tumor Immune Markers (5604), and T-Cell Activity (5607), it is determined if the cancer immune microenvironment has a hot or cold tumor immune response (5620). If the the cancer shows a "hot" tumor immune response (5620), a determination is made (5621) on whether or not a patient should be indicated or Alternate/Other therapies (5623).

In some embodiments, based on a CPI eligibility screening (5614) for Tumor Immune Markers (5604), and T-Cell Activity (5607), it is determined if the cancer immune microenvironment has a hot versus cold tumor immune response (5620). If the the cancer shows a "hot" tumor immune response (5620), a determination is made (5621) on whether or not a patient should be indicated for CPI Therapy (5622) or Alternate/Other therapies (5623).

In some embodiments, a dynamic workflow involving patient care cycle and diagnostic tests for treatment initiation and monitoring effectiveness is provided (FIG. 57).

In some embodiments, a patient indicated for CPI Therapy is identified (5711).

In some embodiments, a treatment regimen is selected and therapy initiated (5712) for a patient indicated for CPI Therapy (5711).

In some embodiments, a treatment regimen selected and therapy initiated (5712) for a patient indicated for CPI Therapy (5711) involves Mono- or Combination CPI, Select CPI Rx (PD-1, PD-L1, CTLA-4), and/or non-CPI therapy. In some embodiments, approved CPIs include PD-1 checkpoint inhibitors (Pembrolizumab, Nivolumab, Cemiplimab), PD-L1 checkpoint inhibitors (Atezolizumab, Avelumab, Durvalumab), and CTLA-4 checkpoint inhibitors (Ipilimumab), or an alternative. Thereafter, in some embodiments, therapy effectiveness is monitored based on imaging and/or clinical assessment.

In some embodiments, a therapy effectiveness is monitored (5713).

In some embodiments, a therapy effectiveness is monitored (5713) based on imaging (5720) and/or clinical assessment.

In some embodiments, a therapy effectiveness is monitored (5713) based on standard of care imaging (5720) and involved a determination of Tumor burden by CT (5708), which entails overall clinical assessment. In some embodiments, for an assessment of Tumor burden by CT (5708) in clinical trials, tumor burden by CT is determined by RECIST

1.1 criteria on contrast-enhanced CT (5708). In some embodiments, assessment of tumor burden by CT involves imaging at baseline and at (t1) -6-12 weeks, (t2) -4-12 weeks after t1, then every -6-12 weeks (5708).

In some embodiments, a therapy effectiveness is monitored (5713) based on standard of care imaging (5720) and involves an assessment of tumor metabolism by PET (5709). In some embodiments an assessment of tumor metabolism by PET (5709) entails imaging with FDG-PET to monitor tumor metabolism as an indicator of tumor growth and activity (5709).

In some embodiments, based on a determination of Tumor burden by CT (5708), a determination is made regarding whether there is a response to therapy (5714).

In some embodiments, based on an assessment of tumor metabolism by PET (5709), a determination is made regarding whether there is a response to therapy (5714).

In some embodiments, based on a determination of Tumor burden by CT (5708) and an assessment of tumor metabolism by PET (5709), a determination is made regarding whether there is a response to therapy (5714).

In some embodiements, based on an assessment of response to therapy (5714), if it is determined that there is response to therapy, it is determined whether the therapy is completed (5716).

In some embodiements, based on an assessment of response to therapy (5714), if it is determined that there is response to therapy, it is determined whether the therapy is completed (5716). If the therapy is not completed, CPI treatment is continued (5717).

In some embodiements, based on an assessment of response to therapy (5714), if it is determined that there is response to therapy, it is determined whether the therapy is completed (5716).

In some embodiements, based on an assessment of response to therapy (5714), if it is determined that there is response to therapy, it is determined whether the therapy is completed (5716). If the therapy is completed, CPI treatment is terminated (5718).

In some embodiments, based on an assessment of response to therapy (5714), if it is determined that there is no response to therapy, additional imaging and/or clinical assessment is performed.

In some embodiments, based on an assessment of response to therapy (5714), if it is determined that there is no response to therapy, additional imaging and/or clinical assessment involved ruling out pseudoprogression (5710). If imaging Tumor burden by CT (5708) indicates progression, the patient is followed up at least 4 weeks later to confirm following iRC or iRECIST guidance to rule out pseudoprogression (5710). In some embodiments, FDG-PET is also used (5709).

In some embodiments, based on an assessment of response to therapy (5714), if it is determined that there is no response to therapy, additional imaging and/or clinical assessment is performed ruling out pseudoprogression (5710), and optionally, a therapy effectiveness is monitored (5713).

In some embodiments, based on an assessment of response to therapy (5714), if it is determined that there is no response to therapy, the therapry is changed (5715).

In some embodiments, based on an assessment of response to therapy (5714), if it is determined that there is no response to therapy, the therapry is chaged (5715) involving a change in CPI dose or regimen.

In some embodiments, based on an assessment of response to therapy (5714), if it is determined that there is no response to therapy, the therapry is chaged (5715) involving non-CPI therapy.

In some embodiments, based on an assessment of response to therapy (5714), if it is determined that there is no response to therapy, the therapry is chaged (5715) involving a change in CPI dose or regimen and non-CPI therapy.

In some embodiments, a dynamic workflow involving patient care cycle and diagnostic tests for treatment monotoring for toxicity is provided (FIG. 58).

In some embodiments, a patient on CPI Therapy is identified (5813).

In some embodiments, a patient on CPI Therapy is identified (5813), and a treatment regimen is selected and therapy initiated (5814).

In some embodiments, a patient on CPI Therapy is identified (5813), and a treatment regimen is selected and therapy initiated (5814) involving Mono- or Combination CPI, Select CPI Rx (PD-1, PD-L1, CTLA-4), and/or non-CPI therapy. In some embodiments, approved CPIs include PD-1 checkpoint inhibitors (Pembrolizumab, Nivolumab, Cemiplimab), PD-L1 checkpoint inhibitors (Atezolizumab, Avelumab, Durvalumab), and CTLA-4 checkpoint inhibitors (Ipilimumab), or an alternative.

In some embodiments, a patient on CPI Therapy (5813), for whom a treatment regimen is selected and therapy initiated (5814), is monitored for toxicity (Immune-Related Adverse Events, irAEs) (5815).

In some embodiments, a patient on CPI Therapy (5813), for whom a treatment regimen is selected and therapy initiated (5814), is monitored for toxicity (irAEs) (5815) based on patient history, imaging, and/or lab tests.

In some embodiments, a patient on CPI Therapy (5813), for whom a treatment regimen is selected and therapy initiated (5814), is monitored for toxicity (irAEs) (5815). In some embodiments, the standard of care monitoring tests (5823) involve immune-Related Toxicity Detection (5811) and Differential Diagnosis irAEs (5812).

In some embodiments, a patient on CPI Therapy (5813), for whom a treatment regimen is selected and therapy

initiated (5814), is monitored for toxicity (irAEs) (5815). In some embodiments, the standard of care monitoring tests involved immune-Related Toxicity Detection (5811) involving detecting irAE by patient history, lab tests, and/or imaging based on CT, and/or MRI.

In some embodiments, after monitoring for toxicity based on immune-Related Toxicity Detection (5811), a determination is made regarding toxicity (5816).

In some embodiments, after monitoring for toxicity based on immune-Related Toxicity Detection (5811), a determination is made regarding toxicity (5816). If there is no toxicity, CPI treatment is continued/resumed (5817).

In some embodiments, after monitoring for toxicity based on immune-Related Toxicity Detection (5811), a determination is made regarding toxicity (5816). If there is no toxicity, CPI treatment is continued/resumed (5817) involving mono- or combination CPI therapy.

In some embodiments, after monitoring for toxicity based on immune-Related Toxicity Detection (5811), a determination is made regarding toxicity (5816). If there is toxicity, an assessment based on Differential Diagnosis irAEs (5812) is performed to confirm diagnosis of immune-related toxicity, rule out other causes, and direct management of patient toxicity. This assessment involved diagnosis by patient history, lab tests, imaging.

In some embodiments, following an assessment based on Differential Diagnosis irAEs (5812), optionally, the toxicity is treated while continuing immunotherapy (5818).

In some embodiments, after an assessment based on Differential Diagnosis irAEs (5812), it is determined if the toxicity is severe or irreversible (5819).

In some embodiments, after an assessment based on Differential Diagnosis irAEs (5812), if it is determined if the toxicity is severe or irreversible (5819), CPI treatment is stopped and immunsuppressants are initiated (5822).

In some embodiments, after an assessment based on Differential Diagnosis irAEs (5812), if it is determined if the toxicity is severe or irreversible (5819), CPI treatment is stopped and immunsuppressants are initiated (5822) involving steroids and/or imunosuppressants.

In some embodiments, after an assessment based on Differential Diagnosis irAEs (5812), if it is determined if the toxicity is not severe or irreversible (5819), CPI treatment is suspended and immunsuppressants are initiated (5820).

In some embodiments, after an assessment based on Differential Diagnosis irAEs (5812), if it is determined if the toxicity is not severe or irreversible (5819), CPI treatment is suspended and immunsuppressants are initiated (5820) involving steroids and/or imunosuppressants.

In some embodiments, after it is determined that the toxicity is not severe or irreversible (5819), and after CPI treatment is suspended and immunsuppressants are initiated (5820) involving steroids and/or imunosuppressants, it is assessed whether the toxicity is resolved (5821).

In some embodiments, if an assessment of whether the toxicity is resolved (5821) indicated that the toxicity is not resolved, CPI treatment is stopped and immunsuppressants are initiated (5822).

In some embodiments, if an assessment of whether the toxicity is resolved (5821) indicated that the toxicity is not resolved, CPI treatment is stopped and immunsuppressants are initiated (5822) involving steroids and/or imunosuppressants.

In some embodiments, if an assessment of whether the toxicity is resolved (5821) indicated that the toxicity is resolved, CPI treatment is continued/resumed (5817).

In some embodiments, if an assessment of whether the toxicity is resolved (5821) indicated that the toxicity is resolved, CPI treatment is continued/resumed (5817) involving mono- or combination CPI therapy.

In some embodiments, a dynamic workflow involving patient care cycle and diagnostic tests for screening and patient selection for product concept use cases is provided (FIG. 59).

In some embodiments, a patient with cancer is identified (5904).

In some embodiments, a patient identified with cancer (5904) is diagnosed and staged (5905) based on cancer type, imaging data (e.g., CT, PET, MRI, US), tumor biopsy and pathology, lab tests, molecular diagnostic tests (e.g., PCR, sequencing).

In some embodiments, a patient with cancer is identified (5904) and diagnosed and staged (5905) and a tumor biopsy is taken (5906) and T-Cell PET Imaging is performed (5907).

In some embodiments, a patient with cancer is identified (5904) and diagnosed and staged (5905) and a tumor biopsy is taken (5906) and T-Cell PET Imaging is performed (5907) involving Detection of T cell trafficking in vivo (5901).

In some embodiments, a patient with cancer is identified (5904) and diagnosed and staged (5905) and a tumor biopsy is taken (5906) and T-Cell PET Imaging is performed (5907) involving Detection of T cell trafficking in vivo (5901), to guide therapy decisions. In some embodiments, Detection of T cell trafficking in vivo (5901) to provides measures of Patient's total body immune status (5921) (e.g., T-cell trafficking).

In some embodiments, a patient with cancer is identified (5904) and diagnosed and staged (5905) and a tumor biopsy is taken (5906) and for T-Cell PET Imaging is performed (5907) involving Detection of T cell trafficking in vivo (5901), to guide therapy decisions. In some embodiments, Detection of T cell trafficking in vivo (5901) provides measures of Patient's cancer immune status (e.g., T-cell trafficking) (5922) including: (i) entire cancer immune load; and/or (ii)

immune status (e.g., T-cell trafficking) of each tumor lesion.

In some embodiments, a patient with cancer is identified (5904) and diagnosed and staged (5905) and a tumor biopsy is taken (5906) and T-Cell PET Imaging is performed (5907) involving Detection of T cell trafficking in vivo (5901), to guide therapy decisions. In some embodiments, Detection of T cell trafficking in vivo (5901) provides measures of Patient's total body immune status (5921) (e.g., T-cell trafficking) and Patient's cancer immune status (e.g., T-cell trafficking) (5922) including: (i) entire cancer immune load; and/or (ii) immune status (e.g., T-cell trafficking) of each tumor lesion.

In some embodiments, a patient with cancer is identified (5904) and diagnosed and staged (5905) and a tumor biopsy is taken (5906) and T-Cell PET Imaging is performed (5907) as test for disease prognosis used to guide therapy selection (5902).

In some embodiments, a patient with cancer is identified (5904) and diagnosed and staged (5905) and a tumor biopsy is taken (5906) and T-Cell PET Imaging is performed (5907) as test to predict response to CPI therapy (5903).

In some embodiments, a patient with cancer is identified (5904) and diagnosed and staged (5905) and a tumor biopsy is taken (5906) and T-Cell PET Imaging is performed (5907) as test to predict response to CPI therapy (5903) to identify patients where CPI therapy should be used because it will be of benefit (5923).

In some embodiments, a patient with cancer is identified (5904) and diagnosed and staged (5905) and a tumor biopsy is taken (5906) and T-Cell PET Imaging is performed (5907) as test to predict response to CPI therapy (5903) to identify patients where CPI therapy should not be used because it will not be of benefit (5924).

In some embodiments, a patient with cancer is identified (5904) and diagnosed and staged (5905) and a tumor biopsy is taken (5906) and T-Cell PET Imaging is performed (5907) as test to predict response to CPI therapy (5903) to: A. Identify patients where CPI therapy should be used because it will be of benefit (5923) and identify patients where CPI therapy should not be used because it will not be of benefit (5924).

In some embodiments, based of the information obtained from testing the tumor biopsy (5906), or based on T-Cell PET Imaging (5907), it is determined whether the tumor type and stage are CPI eligible (5908).

In some embodiments, based of the information obtained from testing the tumor biopsy (5906),, or based on T-Cell PET Imaging (5907), it is determined if the tumor contained actionable driver mutations (5909).

In some embodiments, based of the information obtained from testing the tumor biopsy (5906),, or based on T-Cell PET Imaging (5907), it is determined whether the tumor type and stage are CPI eligible (5908) and if the tumor contains actionable driver mutations (5909).

In some embodiments, if the tumor contained actionable driver mutations (5908), mutation-specific therapy (5911) is resorted to, which entails consideration of immunotherapy in combination with, or as second-line after, targeted therapy (5912).

In some embodiemnts, if the tumor type and stage are CPI eligible (5908), a CPI eligibility screening is performed (5910), which entails screening for PD-L1 by IHC (5601; FIG. 56), Microsatellite Instability (MSI) and Mismatch Repair Deficiency (dMMR) (5602; FIG. 56), and TMB (5603; FIG. 56), and assessment of additional biomarkers (5605-5607; FIG. 56), and clinical assessment.

In some embodiemnts, if the tumor did not contain actionable driver mutations (5909), a CPI eligibility screening is performed (5910), which entails screening for PD-L1 IHC (5601; FIG. 56), Microsatellite Instability (MSI) and Mismatch Repair Deficiency (dMMR) (5602; FIG. 56), and TMB (5603; FIG. 56), and assessment of additional biomarkers (5605-5607; FIG. 56), and clinical assessment.

In some embodiments, based on a CPI eligibility screening (5910) for standard and additional biomarkers (5913), a determination is made on whether or not a patient should be indicated for CPI therapy (5917).

In some embodiments, based on a CPI eligibility screening (5910) for standard and additional biomarkers (5913), it is determined if the cancer is found to be PD-L1+ (5914). If the cancer is found to be PD-L1+ (5914), a determination is made on whether or not a patient should be indicated for CPI therapy (5917).

In some embodiments, based on a CPI eligibility screening (5910) for standard and additional biomarkers (5913), it is determined if the cancer is found to be PD-L1+ (5914). If the cancer is found to be PD-L1+ (5914), a determination is made on whether or not a patient should be indicated for CPI therapy (5917), i.e., whether the patient should be indicated for Alternate/Other therapies (5918).

In some embodiments, based on a CPI eligibility screening (5910) for standard and additional biomarkers (5913), it is determined if the cancer is found to be PD-L1+ (5914). If the cancer is found to be PD-L1+ (5914), a determination is made on whether or not a patient should be indicated for CPI therapy (5917), i.e., whether the patient should be indicated for CPI Therapy (5919).

In some embodiments, based on a CPI eligibility screening (5910) for standard and additional biomarkers (5913), it is determined if the cancer is found to be PD-L1+ (5914). If the cancer is found to be PD-L1+ (5914), a determination is made on whether or not a patient should be indicated for CPI therapy (5917), i.e., whether the patient should be indicated or Alternate/Other therapies (5918) or whether the patient should be indicated for CPI Therapy (5919).

In some embodiments, based on a CPI eligibility screening (5910) for standard and additional biomarkers

(5913), it is determined if the cancer is found to have a high mutation load (5915). If the cancer is found to have a high mutation load (5915), a determination is made on whether or not a patient should be indicated for CPI therapy (5917).

In some embodiments, based on a CPI eligibility screening (5910) for standard and additional biomarkers (5913), it is determined if the cancer is found to have a high mutation load (5915). If the cancer is found to have a high mutation load (5915), a determination is made on whether or not a patient should be indicated for CPI therapy (5917), i.e., whether the patient should be indicated or Alternate/Other therapies (5918).

In some embodiments, based on a CPI eligibility screening (5910) for standard and additional biomarkers (5913), it is determined if the cancer is found to have a high mutation load (5915). If the cancer is found to have a high mutation load (5915), a determination is made on whether or not a patient should be indicated for CPI therapy (5917), i.e., whether the patient should be indicated for CPI Therapy (5919).

In some embodiments, based on a CPI eligibility screening (5910) for standard and additional biomarkers (5913), it is determined if the cancer is found to have a high mutation load (5915). If the cancer is found to have a high mutation load (5915), a determination is made on whether or not a patient should be indicated for CPI therapy (5917), i.e., whether the patient should be indicated or Alternate/Other therapies (5918) and whether the patient should be indicated for CPI Therapy (5919).

In some embodiments, based on a CPI eligibility screening (5910) for standard and additional biomarkers (5913) involving, in the alternative, detection of T cell trafficking in vivo (5901), test for disease prognosis used to guide therapy selection (5902), and/or test to predict response to CPI therapy (5903), it is determined if the cancer had a hot versus cold tumor immune response (5916).

In some embodiments, based on a determination of whether the cancer had a hot versus cold tumor immune response (5916), if it is determined that the the cancer showed a "hot" tumor immune response, a determination is made on whether or not a patient should be indicated for CPI therapy (5917).

In some embodiments, based on a determination of whether the cancer had a hot versus cold tumor immune response (5916), if it is determined that the cancer showed a "hot" tumor immune response, a determination is made on whether or not a patient should be indicated for CPI therapy (5917), i.e., whether the patient should be indicated or Alternate/Other therapies (5918).

In some embodiments, based on a determination of whether the cancer had a hot versus cold tumor immune response (5916), if it is determined that the cancer showed a "hot" tumor immune response, a determination is made on whether or not a patient should be indicated for CPI therapy (5917), i.e., whether the patient should be indicated for CPI Therapy (5919).

In some embodiments, based on a determination of whether the cancer had a hot versus cold tumor immune response (5916), if it is determined that the the cancer showed a "hot" tumor immune response, a determination is made on whether or not a patient should be indicated for CPI therapy (5917), i.e., whether the patient should be indicated for CPI Therapy (5919) or Alternate/Other therapies (5918).

In some embodiments, a dynamic workflow involving patient care cycle and diagnostic tests for treatment initiation and monitoring effectiveness for product use cases is shown in FIG. 60.

In some embodiments, a patient on CPI Therapy is identified (6010).

In some embodiments, a treatment regimen is selected and therapy initiated (6011) for a patient indicated for CPI Therapy (6010).

In some embodiments, a therapy is selected, the effectiveness of a therapy monitored, and therapy adjusted by imaging based on T-cell PET imaging (6014).

In some embodiments, a treatment regimen selected and therapy initiated (6011) for a patient indicated for CPI Therapy (6010), a therapy is selected that is most likely to be effective for the patient (mono- or combination) (6005).

In some embodiments, a treatment regimen selected and therapy initiated (6011) for a patient indicated for CPI Therapy (6010) involved Mono- or Combination CPI, Select CPI Rx (PD-1, PD-L1, CTLA-4), and/or non-CPI therapy. In some embodiments, approved CPIs include PD-1 (Pembrolizumab, Nivolumab, Cemiplimab), PD-L1 (Atezolizumab, Avelumab, Durvalumab), and CTLA-4 (Ipilimumab), or an alternative. Thereafter, in some embodiments, therapy effectiveness is monitored based on imaging and/or clinical assessment.

In some embodiments, therapy effectiveness is monitored (6012) by T-cell PET imaging (6014) based on measurement of tumor inter-lesion heterogeneity in immune activation (e.g., T-cell trafficking) to guide therapy decisions (6004).

In some embodiments, therapy effectiveness is monitored (6012) by T-cell PET imaging (6014) for early detection of immune activation (e.g., T-cell trafficking) to monitor for pharmacodynamic response to immunotherapy to make a determination regarding whether there is a response to therapy (6006).

In some embodiements, based on early detection of immune activation (e.g., T-cell trafficking) to monitor for pharmacodynamic response to immunotherapy to make a determination regarding whether there is a response to therapy (6006), it is determined that there is response to therapy (6013).

In some embodiements, based on early detection of immune activation (e.g., T-cell trafficking) to monitor for

pharmacodynamic response to immunotherapy to make a determination regarding whether there is a response to therapy (6006), if it is determined that there is response to therapy (6013), it is determined whether the therapy is completed (6016).

In some embodiements, based on early detection of immune activation (e.g., T-cell trafficking) to monitor for pharmacodynamic response to immunotherapy to make a determination regarding whether there is a response to therapy (6006), if it is assessed for response to therapy (6013), and if it is determined whether the therapy is not completed (6016), CPI treatment is continued based on mono- or combination CPI therapy (6017).

In some embodiements, based on early detection of immune activation (e.g., T-cell trafficking) to monitor for pharmacodynamic response to immunotherapy to make a determination regarding whether there is a response to therapy (6006), if it is assessed for response to therapy (6013), and if it is determined whether the therapy is completed (6016), the tehrapy duration is assessed and it is determined when therapy is finished [PP8] (6009), and CPI treatment terminated (6018).

In some embodiments, based on early detection of immune activation (e.g., T-cell trafficking) to monitor for pharmacodynamic response to immunotherapy to make a determination regarding whether there is a response to therapy (6006), if it is assessed for response to therapy (6013), if it is determined that there is no response to therapy, T-cell PET imaging is performed (6014) for differential diagnosis of pseudoprogression vs true progression (6007).

In some embodiments, based on early detection of immune activation (e.g., T-cell trafficking) to monitor for pharmacodynamic response to immunotherapy to make a determination regarding whether there is a response to therapy (6006), if it is assessed for response to therapy (6013), if it is determined that there is no response to therapy, T-cell PET imaging is performed (6014) for differential diagnosis of pseudoprogression vs true progression (6007), and optionally, a therapy effectiveness is monitored (6012).

In some embodiments, based on early detection of immune activation (e.g., T-cell trafficking) to monitor for pharmacodynamic response to immunotherapy to make a determination regarding whether there is a response to therapy (6006), if it is assessed for response to therapy (6013), if it is determined that there is no response to therapy, T-cell PET imaging is performed (6014) for differential diagnosis of pseudoprogression vs true progression (6007), and determined whether to change therapy (6015) by switching from mono- to combination-therapy (prediction of efficacy of combo CPI after failure of mono-therapy) (6008), and/or involving changing CPI dose or regimen and/or using non-CPI therapy.

In some embodiments, based on early detection of immune activation (e.g., T-cell trafficking) to monitor for pharmacodynamic response to immunotherapy to make a determination regarding whether there is a response to therapy (6006), if it is assessed for response to therapy (6013), if it is determined that there is no response to therapy, T-cell PET imaging is peformed (6014) for differential diagnosis of pseudoprogression versus true progression (6007), and determined whether to change therapy (6015) by switching from mono- to combination-therapy (prediction of efficacy of combo CPI after failure of mono-therapy) (6008), and/or involving changing CPI dose or regimen and/or using non-CPI therapy, and CPI treatment terminated (6018).

In some embodiments, a dynamic workflow involving patient care cycle and diagnostic tests for treatment minotoring for toxicity is provided (FIG. 61).

In some embodiments, a patient on CPI Therapy is identified (6112).

In some embodiments, a patient on CPI Therapy is identified (6112), and a treatment regimen is selected, and therapy initiated (6113).

In some embodiments, a patient on CPI Therapy is identified (6112), and a treatment regimen is selected, and therapy initiated (6113) involving Mono- or Combination CPI, Select CPI Rx (PD-1, PD-L1, CTLA-4), and/or non-CPI therapy. In some embodiments, approved CPIs include PD-1 (Pembrolizumab, Nivolumab, Cemiplimab), PD-L1 (Atezolizumab, Avelumab, Durvalumab), and CTLA-4 (Ipilimumab), or an alternative.

In some embodiments, a patient on CPI Therapy (6112), for whom a treatment regimen is selected, and therapy initiated (6113), is monitored for toxicity (irAEs) (6114).

In some embodiments, a patient on CPI Therapy (6112), for whom a treatment regimen is selected, and therapy initiated (6113), is monitored for toxicity (irAEs) (6114) based on patient history, imaging, and/or lab tests.

In some embodiments, a patient on CPI Therapy (6112), for whom a treatment regimen is selected, and therapy initiated (6113), is monitored for toxicity (irAEs) (6114). In some embodiments, the monitoring tests are based on T-cell PET imaging (6122).

In some embodiments, a patient on CPI Therapy (6112), for whom a treatment regimen is selected, and therapy initiated (6113), is monitored for toxicity (irAEs) (6114) based on T-cell PET imaging (6122), as prediction or early detection method to mintor for early signs or risk of immune-related toxicity before the patient is symptomatic (6110).

In some embodiments, after monitoring for toxicity (6114), as prediction or early detection method to monitor for early signs or risk of immune-related toxicity (irAEs) before the patient is symptomatic (6110), a determination is made regarding toxicity (6115).

In some embodiments, after monitoring for toxicity (6114), as prediction or early detection to monitor for early signs or risk of immune-related toxicity (irAEs) before patient is symptomatic (6110), a determination is made regarding

toxicity (6115). If there is no toxicity, CPI treatment is continued (6116) based on mono- or combination CPI therapy.

In some embodiments, after monitoring for toxicity (6114), as prediction or early detection method to monitor for early signs or risk of immune-related toxicity (irAEs) before patient is symptomatic (6110), a determination is made regarding toxicity (6115). If there is toxicity, an assessment regarding irAE differential diagnosis is made to distinguish between immune-related toxicity and non-immune-related signs and symptoms (6111).

In some embodiments, after monitoring for toxicity (6114), as prediction or early detection method to monitor for early signs or risk of immune-related toxicity (irAEs) before patient is symptomatic (6110), a determination is made regarding toxicity (6115). If there is toxicity, an assessment regarding irAE differential diagnosis is made to distinguish between immune-related toxicity and non-immune-related signs and symptoms (6111), and optionally, toxicity is treated while continuing immunotherapy (6117).

In some embodiments, after monitoring for toxicity (6114), as prediction or early detection method to monitor for early signs or risk of immune-related toxicity (irAEs) before patient is symptomatic (6110), a determination is made regarding toxicity (6115). If there is toxicity, an assessment regarding irAE differential diagnosis is made to distinguish between immune-related toxicity and non-immune-related signs and symptoms (6111) to distinguish immune-realted toxicity versus non-immuen conditions (6123).

In some embodiments, after monitoring for toxicity (6114), as prediction or early detection method to monitor for early signs or risk of immune-related toxicity (irAEs) before patient is symptomatic (6110), a determination is made regarding toxicity (6115). If there is toxicity, an assessment regarding irAE differential diagnosis is made to distinguish between immune-related toxicity and non-immune-related signs and symptoms (6111) to distinguish tumor-realted versus drug-related toxicity (6124).

In some embodiments, after irAE differential diagnosis is made to distinguish between immune-related toxicity and non-immune-related signs and symptoms (6111), it is determined if the toxicity is severe or irreversible (6118).

In some embodiments, after irAE differential diagnosis is made to distinguish between immune-related toxicity and non-immune-related signs and symptoms (6111), if it is determined that toxicity is severe or irreversible (6118), CPI treatment is stopped and immunsuppressants are initiated (6121).

In some embodiments, after irAE differential diagnosis is made to distinguish between immune-related toxicity and non-immune-related signs and symptoms (6111), if it is determined that toxicity is severe or irreversible (6118), CPI treatment is stopped and immunsuppressants are initiated (6121) involving steroids and/or imunosuppressants.

In some embodiments, after irAE differential diagnosis is made to distinguish between immune-related toxicity and non-immune-related signs and symptoms (6111), if it is determined that toxicity is not severe or irreversible, CPI treatment is suspended and immunsuppressants are initiated (6119).

In some embodiments, after irAE differential diagnosis is made to distinguish between immune-related toxicity and non-immune-related signs and symptoms (6111), if it is determined that toxicity is not severe or irreversible, CPI treatment is suspended and immunsuppressants are initiated (6119) involving steroids and/or imunosuppressants.

In some embodiments, after it is determined that the toxicity is not severe or irreversible, and after CPI treatment is suspended and immunsuppressants are initiated (6119) involving steroids and/or imunosuppressants, it is assessed whether the toxicity is resolved (6120).

In some embodiments, if an assessment of whether the toxicity is resolved (6120) indicated that the toxicity is resolved, CPI treatment is continued (6116).

In some embodiments, if an assessment of whether the toxicity is resolved (6120) indicated that the toxicity is resolved, CPI treatment is continued (6116) involving mono- or combination CPI therapy.

In some embodiments, if an assessment of whether the toxicity is resolved (6120) indicated that the toxicity is not resolved, CPI treatment is stopped and immunsuppressants are initiated (6121).

In some embodiments, if an assessment of whether the toxicity is resolved (6120) indicated that the toxicity is not resolved, CPI treatment is stopped and immunsuppressants are initiated (6121) involving involving steroids and/or imunosuppressants.

## Additional Numbered Embodiments

The following embodiments are set forth as optional combinations and are not limiting. The numbered arrangements can be combined with any of the other numbered arrangements or combined with any of the embodiments or disclosures provided herein.

Arrangement number:

1. A method of monitoring CD8 in vivo, the method comprising:

providing a CD8 minibody to a subject, wherein the CD8 minibody binds to a CD8 as shown in FIG. 1C, and wherein the minibody is labeled with a detectable marker; and
monitoring a distribution of the CD8 minibody in the subject within 6-36 hours of administering the CD8 minibody to the subject.

2. A method of monitoring CD8 in vivo, the method comprising:

providing a CD8 minibody to a subject, wherein the CD8 minibody binds to aCD8 as shown in FIG. 1C, and wherein the minibody is labeled with a detectable marker; and
monitoring a distribution of the CD8 minibody in the subject, wherein the monitoring can detect a tissue infiltrated with 500 or less CD8 bearing cells per $mm^2$ within the subject, and wherein monitoring is achieved via PET.

3. A method of visualizing CD8 positive cells, the method comprising:

providing a CD8 minibody to a subject, wherein the CD8 minibody is labeled with a detectable marker, wherein the CD8 minibody is humanized in sequence and binds to human CD8; and
monitoring a distribution of the CD8 minibody in the subject within 6-36 hours of administering the CD8 minibody to the subject, wherein monitoring can detect a tissue infiltrated with 500 or less CD8 positive cells per $mm^2$ within the subject, and wherein monitoring is achieved via PET.

4. A method of visualizing cells in a human, the method comprising:

providing a means of binding human CD8 positive cells to a subject, wherein the means comprises a detectable label; and
monitoring and determining a first distribution of the means of binding human CD8 positive cells in the subject within 6-36 hours of administering the means of binding human CD8 positive cells to the subject.

5. The method of arrangement 4, further comprising:

monitoring and determining a second distribution of the means of binding human CD8 positive cells in the subject after monitoring and determining the first distribution; and
comparing the first and second distributions to determine a change in distribution.

6. The method of arrangement 5, wherein the method further comprises:

administering a first therapeutic agent to the subject with or prior to providing the means of binding human CD8 positive cells to a subject,
wherein the change in distribution is used to determine an effectiveness of the first therapeutic agent,
wherein if the change in distribution indicates an increase in an amount of CD8 positive cells within a tumor, at least a second dose of the first therapeutic agent is administered to the subject, and
wherein if the change in distribution indicates a decrease in an amount of CD8 positive cells within a tumor, a second therapeutic agent is administered to the subject.

7. The method of arrangement 5, wherein the method further comprises:

administering a first therapeutic agent to the subject with or prior to providing the means of binding human CD8 positive cells to a subject,
wherein the change in distribution is used to determine an effectiveness of the first therapeutic agent,
wherein if the change in distribution indicates an increase in an amount of CD8 positive cells within a tumor, at least a second dose of the first therapeutic agent is administered to the subject, and
wherein if the change in distribution indicates no change or a decrease in an amount of CD8 positive cells within a tumor, no second dose of the first therapeutic agent is administered to the subject.

8. The method of arrangement 5, wherein the method further comprises:

administering a first therapeutic agent to the subject with or prior to providing the means of binding human CD8 positive cells to a subject,
wherein the change in distribution is used to determine an effectiveness of the first therapeutic agent,

wherein if the change in distribution indicates no change or an increase in an amount of CD8 positive cells within a tumor, at least a second dose of the first therapeutic agent is administered to the subject, and wherein if the change in distribution indicates a decrease in an amount of CD8 positive cells within a tumor, no second dose of the first therapeutic agent is administered to the subject.

9. A method of administering a minibody to a subject, the method comprising:
providing to a subject a labeled minibody that binds to human CD8, wherein 3 mCi ±20% of radiation is provided to the subject via the labeled minibody and wherein an amount between 0.2 and 10mg of total protein is provided to the subject.
10. A method of administering a minibody to a subject, the method comprising:
providing to a subject a labeled minibody that binds to human CD8, wherein 0.75-1.5 +/- 20% mCi of radiation is provided to the subject via the labeled minibody and wherein an amount between 0.2 and 10mg of total protein is provided to the subject.
11. A method of treating a patient, comprising:
administering to a human patient diagnosed with a cancer a dose of an antigen-binding construct that binds to human CD8, wherein the dose comprises:

a $^{89}$Zr-labeled antigen-binding construct providing a radiation activity of about 3 mCi; and
about 10 mg or less of the antigen-binding construct;
detecting the $^{89}$Zr-labeled antigen-binding construct in the patient at a first time point after administering the dose, to generate a first patient image corresponding to the first time point, wherein the first time point is about 6 hours to 36 hours after administering;
determining a first abundance and/or distribution of CD8 cells in one or more tissues and/or neoplasia in the patient based on the first patient image; and
administering to the patient a first treatment for the cancer based on the first abundance and/or distribution of CD8 cells in the one or more tissues.

12. The method of arrangement 8, further comprising:

after administering the first treatment, determining a second abundance and/or distribution of CD8 cells in the one or more tissues and/or neoplasia in the patient based on a second patient image corresponding to a second time point and comprising a second site-specific information for the target of the antigen-binding construct; and administering to the patient a second treatment for the cancer based on a comparison of the first and second patient images.

13. The method of arrangement 9, further comprising optimizing a therapeutic dose of the second treatment based on the comparison of the first and second patient images.
14. A method of treating a tumor, the method comprising:

administering a minibody to a subject such that the minibody binds to a tumor within the subject to form a labeled tumor, wherein the minibody binds to human CD8, and wherein the minibody is linked to a detectable label;
if the minibody binds in a biased manner to a surface of any tumor in forming the labeled tumor, then administering a treatment selected from the group consisting of an immune checkpoint inhibitor; and
if the minibody binds throughout all tumors in forming the labeled tumor, then not administering an immune checkpoint inhibitor.

15. A method of treating a subject, the method comprising:

administering to a patient that has a neoplasia a human minibody that binds to human CD8;
monitoring a distribution of the human minibody to determine a first tumor-infiltrating lymphocyte ("TIL") status within the neoplasia; and
treating the patient based upon at least the first TIL status of the neoplasia.

16. The method of arrangement 12, wherein when a first TIL status is when the human minibody binds throughout a volume of all identified tumors, and wherein a second TIL status is when the human minibody binds selectively to any tumor margin without homogenously perfusing the volume of the tumor.
17. The method of arrangement 13, wherein an appropriate immunotherapy is applied to the subject if the subject has the second TIL status.

18. The method of arrangement 12, wherein an immunotherapy is not applied to the subject if the subject has the first TIL status, as the body is adequately fighting the tumor.

19. The method of any one of arrangements 12-15, wherein at least the first TIL status is used for at least one of:

a. Classifying or selecting the human subject for a clinical trial
b. Recommending or determining eligibility of the human subject for a therapeutic treatment
c. Predicting response to therapy of the human subject; and/or
d. Predicting response to therapy selected from among the group consisting of

i. Radiotherapy,
ii. Chemotherapy,
iii. Biological therapy, and
iv. Immunotherapy

20. The method of any one of arrangements 1-5 and 12, wherein monitoring CD8 is achieved as a function of:

a) a distribution of detectable marker at an external margin of the tumor which demonstrates an increase in binding at the external margin as compared to an internal area or volume of the tumor; or
b) a distribution of detectable marker at an internal area or volume of the tumor.

21. The method of arrangement 17, wherein
if a) then providing the subject with an immunotherapy based therapy to convert the tumor to a TIL positive tumor and if a) persists after treatment with such immunotherapy then providing the subject with an alternate immunotherapy based therapy.

22. The method of arrangement 17, wherein a) is defined as having at least 10% greater presence of marker at an outermost perimeter of the tumor, wherein the outermost perimeter of the tumor is no more than 10% of an area of an image of the tumor.

23. The method of arrangement 17, wherein b) is defined as having no more difference in image intensity between an inner area and an outer area than 100%, wherein the outer area is defined as an outermost perimeter of the tumor that is no more than 10% of an area of an image of the tumor.

24. A method of analyzing CD8 distribution in a subject, the method comprising:

providing an image of a distribution of a detectable marker via a first PET image, wherein the detectable marker is linked to a CD8 minibody, wherein the CD8 minibody binds to human CD8, and wherein the CD8 minibody does not provoke an immune response in the subject;
providing an image of a distribution of a FDG marker via a second PET image of the subject;
creating a third PET image that comprises an overlay of the first PET image onto the second PET image;
identifying a tumor as TIL, based upon the third PET image.

25. A method of treating a subject, the method comprising:

administering to a patient that has a neoplasia an antigen-binding construct that binds to human CD8;
monitoring a distribution of the antigen-binding construct to determine the tumor-infiltrating lymphocyte ("TIL") status within the neoplasia; and
administering an immunotherapy ("IOT") to the patient if the TIL status of at least one neoplasia is negative so as to convert the TIL status from negative to positive.

26. A method of treating a subject, the method comprising:

administering to a patient that has a neoplasia an antigen-binding construct that binds to human CD8;
monitoring a distribution of the antigen-binding construct to determine the tumor-infiltrating lymphocyte ("TIL") status within the neoplasia; and
administering an alternative immunotherapy ("IOT") to the patient if the TIL status of the neoplasia is not positive, until the TIL status of the neoplasia becomes positive.

27. The method of arrangement 24, wherein monitoring is conducted within 8 hours of administering.

28. The method of arrangement 24 or 25, wherein the monitoring or a second monitoring is done before 36 hours from administering.

29. A method of characterizing a tumor, the method comprising:

applying an antigen-binding construct that binds to human CD8 to a human subject, wherein the antigen-binding construct comprises a detectable marker;
monitoring a distribution of the detectable marker;
generating an image based on the distribution of the detectable marker; and
displaying the image to a user in a manner to allow the user to characterize the tumor.

30. A method of generating an image, the method comprising:

applying an antigen-binding construct that binds to CD8, wherein the antigen-binding construct comprises a detectable marker; and
detecting at least one detectable marker within a location within the tumor and assigning a positive pixel for each detectable marker detected; and
generating an image based on a distribution of each positive pixel assigned, wherein the image is stored in a non-transitory computer readable media.

31. A method of positron emission tomography ("PET"), the method comprising:

administering a tracer that binds to CD8 to a subject;
providing a scintillator;
using the scintillator to detect a pair of photons created by the tracer;
using detection of the pair of photons to localize a source of the tracer via a list of coincidence events that are processed via a processor that is configured to take an output from the scintillator and convert it to the list of coincidence events, wherein between about 300 and about 500 CD8 positive cells can be detected per $mm^2$ of a tissue or neoplasia within the subject.

32. A method of manufacturing a diagnostic composition, the method comprising:

conjugating a minibody to desferrioxamine to form a Df-minibody;
radiolabeling the Df-minibody with $^{89}Zr$ to form radiolabeled minibody;
purifying the radiolabeled minibody;
mixing the radiolabeled minibody with a cold minibody to form a diagnostic composition, wherein the minibody and the cold minibody bind to a same epitope on CD8.

33. A diagnostic composition comprising:

3.0+20% mCi of a 89Zr-Df-labeled minibody;
20 mM Histidine;
5% sucrose;
51-62 mM Sodium Chloride;
141-194 Arginine; and
2-20 mM Glutamic acid.

34. The composition of arrangement 31, wherein there is 1-250 micrograms per kg of subject weight of minibody.
35. The composition of arrangement 31, wherein there is 1.2-1.5mg of a labeled minibody.
36. A diagnostic composition comprising:

a labeled minibody comprising the structure of:

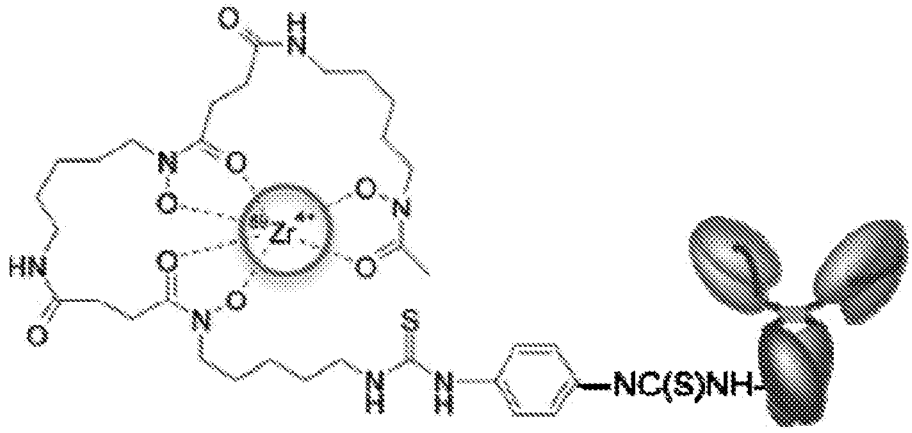

,

or

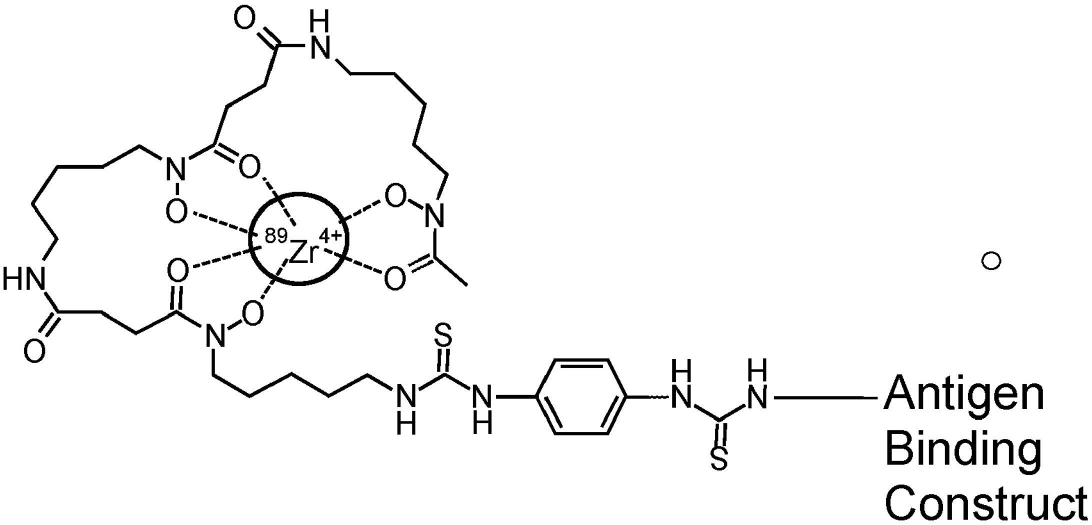

wherein the minbody comprises a heavy chain variable region of SEQ ID NO:1, 3, 16, or 147 and a light chain variable region of SEQ ID NO: 7, 9, 15, or 147, and wherein the composition provides at least 3 mCi of radiation.

37. A composition comprising:

a first minibody that binds to CD8 that comprises an active marker; and

a second minibody that binds to CD8, wherein the second minbody comprises an inactive marker or lacks an active marker.

38. The composition of arrangement 1, wherein the first minibody to second minibody are present in the composition at a ratio of about 1:7.

39. A diagnostic composition comprising:

a first CD8 minibody that is conjugated to a radiolabel;

a second CD8 minibody that is not conjugated to the radiolabel, wherein the first and second CD8 minibodies have a same sequence, wherein the composition provides about 3 mCi of radiation, and wherein the composition provides 1.5-10 mg of total mass protein.

40. A method of imaging a subject, the method comprising:

administering a first dose of any one of the compositions provided above;

imaging the subject to obtain a first image;
administering a second dose of any one of the compositions provided above; and
imaging the subject to obtain a second image; and
comparing the first image to the second image.

41. The method of arrangement 38, wherein the comparison assists in assessing a treatment response of the subject.
42. The method of arrangement 38, wherein the comparison is used to determine a therapeutic dose to be provided to the subject, wherein a first potential dose is administered to the subject after the first image is obtained.
43. The method of any one of arrangements 1-28 and 37-39 or the composition of any one of arrangements 30-36, wherein the CD8 minibody comprises a heavy chain variable region and a light chain variable region, and wherein the heavy chain variable region consists essentially of a human amino acid sequence and the light chain variable region consists essentially of a human amino acid sequence.
44. The method of any one of arrangements 1-28 and 37-39 or the composition of any one of arrangements 30-36, wherein the CD8 minibody binds to a CD8 sequence consisting of the sequence of SEQ ID NO: 134 and/or 135.
45. The method of any one of arrangements 1-28 and 37-39, wherein the minibody comprises the heavy chain CDRs in any of the CDR SEQ ID Nos: provided herein, and the light chain CDRs in any of the CDR SEQ ID Nos: provided herein.
46. The method of any one of arrangements 1-28 and 37-39, wherein the minibody comprises a heavy chain variable region that is at least 80% identical to the heavy chain amino acid sequence in any of the heavy chain SEQ ID Nos: provided herein, and a light chain variable region that is at least 80% identical to the light chain amino acid sequence in any of the heavy chain SEQ ID NOs: provided herein
47. The method of any one of the arrangements involving monitoring, wherein the monitoring is achieved via providing a first PET scan.
48. The method of Arrangement 44, wherein monitoring further comprises providing a second PET fluorodeoxyglucose ("FDG") PET scan.
49. The method of any one of the arrangements involving a first and second scan, wherein the first scan and the second scan are compared to determine if there is co-localization of the CD8 in a neoplasia.
50. The method of any one of the arrangements involving a neoplasia, wherein the neoplasia is a known tumor.
51. The method of any one of arrangements 1-28 and 37-39, wherein the known tumor is selected from one or more cancer of: carcinoma, lymphoma, blastoma, sarcoma, and leukemia, lymphoid malignancies, squamous cell cancer (e.g. epithelial squamous cell cancer), lung cancer, small-cell lung cancer, non-small cell lung cancer, adenocarcinoma of the lung and squamous carcinoma of the lung, cancer of the peritoneum, hepatocellular cancer, gastric or stomach cancer, gastrointestinal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain, head and neck cancer, adult and pediatric solid cancers, and solid tumors.
52. The method of treatment of any one of arrangements 1-28 and 37-39, wherein the treatment comprises at least one of the therapies provided herein, including, but not limited to: an immune check point inhibitor, an IOT, or chemotherapeutic agent provided herein..
53. The method of any one of arrangements 1-28 and 37-39, wherein CD8 distribution comprises determining CD8 positive cell density observed by PET.
54. A non-transitory computer readable medium comprising:
data regarding an image of a PET scan, wherein the data has been obtained by the method of any one of arrangements 1-28 and 37-39 above.
55. A method of determining a standard uptake value, the method comprising:

applying an antigen binding construct to a subject, wherein the antigen binding construct comprises a radioactive probe;
determining r, where r is the radioactivity concentration (kBq/ml) measured by a PET scanner within a ROI of radiation from the antigen binding construct;
determining a’, wherein a’ is the decay-corrected amount of the injected radiolableeld tracer (kBq);
determining w, the weight of the patient; and
determining SUV as being the result of r(a’/W).

56. A method of analyzing an image, the method comprising:

providing an image;

defining on the image a first region of interest (ROI) by marking the image;
determining a signal intensity for a data point within the first ROI;
determining a maximum signal intensity within the first ROI;
determining a mean value of the signal intensities within the first ROI;
summing together each signal intensity within the first ROI to obtain a first summed signal level for the first ROI, wherein the first ROI represents data for an amount of a detectable marker associated with an antigen binding construct that has been administered to a subject.

57. The method of arrangement 55, further comprising repeating the process of arrangement 2 for a second ROI to determine a second summed signal level and comparing the first and the second summed signal levels, wherein when the first summed signal level is less than the second summed signal level, it indicates the presence of increased CD8 in the second ROI.
58. The method of arrangement 56, wherein the first ROI is a same location as the second ROI, but differ in that the first ROI is provided at a first time point and the second ROI is provided at a second time point and a candidate therapeutic has been administered after the first time point but before the second time point.
59. The method of one of arrangements 2, 3, or 28, wherein the tissue is 20 microns thick.
60. A method of treating a human subject having cancer, the method comprising:
in response to:

providing a first image of a tumor in the subject using a CD8 antigen binding construct;
administering a therapy including a candidate therapeutic to the subject;
after administering the therapy including the candidate therapeutic, providing a second image of the tumor in the subject using the CD8 antigen binding construct;
comparing the first and second images to determine if:

a) the tumor demonstrates increased CD8 infiltration or
b) the tumor demonstrates the same or decreased CD8 infiltration,

if a), then instructing the subject to continue the therapy.

61. A method of treating a human subject having cancer, the method comprising:

providing a first image of a CD8 cells within a tumor in a subject using a CD8 antigen binding construct;
administering a therapy including a candidate therapeutic to the subject;
after administering the therapy including the candidate therapeutic, providing a second image of the CD8 cells within the tumor in the subject using the CD8 antigen binding construct;
comparing the first and second images to determine if:

a) the tumor demonstrates increased CD8 infiltration or
b) the tumor demonstrates the same or decreased CD8 infiltration,

wherein, if a), then instructing the subject to continue the therapy.

62. The method of arrangement 61, wherein if b), then administering another round of therapy, providing a third image of the CD8 cells within the tumor; comparing the first and third images to determine if the tumor demonstrates the same or decreased CD8 infiltration; and discontinuing the therapy if the tumor demonstrates the same or decreased CD8 infiltration.
63. The method of arrangement 61, wherein the therapy comprises radiotherapy.
64. The method of arrangement 61, wherein the first image is an image of the CD8 cells within the tumor in the subject.
65. The method of arrangement 61, wherein the first image is an image of CD8 cells within the tumor that is not from the subject.
66. The method of arrangement 62, wherein the first image is from a database and from an individual or is a composite of data from a collection of individuals.
67. The method of arrangement 61,

wherein if b), then administering a therapy comprising an alternative candidate therapeutic and after administering the therapy, providing a third image of the CD8 cells within the tumor in the subject using the CD8 antigen binding construct;

comparing the third and second or third and first images to determine if:

c) the tumor demonstrates increased CD8 infiltration or
d) the tumor demonstrates the same or decreased CD8 infiltration,

wherein, if c) then instructing the subject to continue the therapy comprising the alternative candidate therapeutic.

68. A method of treating a human subject having cancer, the method comprising:

administering a CD8 antigen binding construct to the subject;
monitoring a distribution of the CD8 antigen binding construct within the subject; and
discontinuing an immunotherapy treatment if at least one tumor within the subject is TIL negative per the distribution of the CD8 antigen binding construct.

69. A method of treating a human subject having cancer, the method comprising:

administering a CD8 antigen binding construct to the subject;
monitoring a distribution of the CD8 antigen binding construct within the subject; and
continuing an immunotherapy treatment if at least one tumor within the subject is TIL positive per the distribution of the CD8 antigen binding construct.

70. A method of treating a human subject having cancer, the method comprising

administering an immunotherapy to the subject;
then administering a CD8 antigen binding construct to the subject;
monitoring a distribution of the CD8 antigen binding construct within the subject;
discontinuing the immunotherapy if all of the tumors within the subject is TIL negative per the distribution of the CD8 antigen binding construct.

71. A method of treating a human subject having cancer, the method comprising:

in a subject previously untreated with an immunotherapy,
administering a CD8 antigen binding construct to the subject;
monitoring a distribution of the CD8 antigen binding construct within the subject;
administering an immunotherapy if at least one tumor within the subject is TIL negative per the distribution of the CD8 antigen binding construct.

72. A method of treating a human subject having cancer, the method comprising:

in a subject previously untreated with an immunotherapy,
administering a CD8 antigen binding construct to the subject;
monitoring a distribution of the CD8 antigen binding construct within the subject;
advising against administering of an immunotherapy if all tumors within the subject are TIL positive per the distribution of the CD8 antigen binding construct.

73. A method of treating a human subject having cancer, the method comprising:

in a subject previously treated with an immunotherapy, administering a CD8 antigen binding construct to the subject;
monitoring a distribution of the CD8 antigen binding construct within the subject; and
administering an alternative immunotherapy if at least one tumor within the subject is TIL negative per the distribution of the CD8 antigen binding construct.

74. A method of treating a human subject having cancer, the method comprising:

in a subject previously treated with an immunotherapy, administering a CD8 antigen binding construct to the subject;
monitoring a distribution of the CD8 antigen binding construct within the subject;

administering the immunotherapy if all tumors within the subject are TIL positive per the distribution of the CD8 antigen binding construct.

75. The method of arrangements 61-74, further comprising measurement of the level of an additional biomarker in a patient sample to inform the selection of immunotherapy.
76. The method of arrangement 75, wherein the additional biomarker is selected from among PD-L1 status, T-cell receptor clonality, mutational burden, neoantigen burden, immune gene signatures, and multiplex immunohistochemistry.
77. A method of treating a tumor in a human subject, the method comprising:

administering a CD8 antigen binding construct to the subject such that the CD8 antigen binding construct binds to CD8 cells within a tumor within the subject to form a labeled tumor, wherein the CD8 antigen binding construct binds to human CD8, and wherein the CD8 antigen binding construct is linked to a detectable label;
detecting a distribution of the CD8 antigen binding construct in or around the tumor;
if the CD8 antigen binding construct binds in a biased manner at a surface of any tumor, then administering a treatment selected from the group consisting of an Immunotherapy; and
if the CD8 antigen binding construct binds in a non-biased manner and throughout all tumors, then not administering an immune checkpoint inhibitor
if the CD8 antigen binding construct binds in a non-biased manner but is absent from the interior of the tumor, then administering an Immunotherapy.

78. A method of treating a tumor in a human subject, the method comprising:

administering to a human subject a CD8 antigen binding construct;
monitoring a distribution of the CD8 antigen binding construct to determine a first tumor-infiltrating lymphocyte ("TIL") status within the tumor; and
treating the patient based upon at least the first TIL status of the neoplasia.

79. The method of arrangement 78, wherein when a first TIL status is when the CD8 antigen binding construct binds throughout a volume of all identified tumors, and wherein a second TIL status is when the CD8 antigen binding construct binds in a biased manner to any tumor margin.
80. The method of arrangement 79, wherein an immunotherapy is applied to the subject if the subject has the second TIL status.
81. The method of arrangement 79, wherein an immunotherapy is not administered to the subject if the subject has the first TIL status.
82. The method of any one of arrangements 78-81, wherein at least the first TIL status is used for at least one of:

a. classifying or selecting the human subject for a clinical trial;
b. recommending or determining eligibility of the human subject for a therapeutic treatment;
c. predicting response to therapy of the human subject; and/or
d. predicting response to therapy selected from among the group consisting of

i. radiotherapy,
ii. chemotherapy,
iii. biological therapy, and
iv. immunotherapy

83. The method of any one of arrangements 78-82, wherein monitoring CD8 antigen binding construct is achieved as a function of:

a) a distribution of the detectable marker at a tumor surface which demonstrates an increase in binding to CD8 positive cells at the tumor surface as compared to an internal area or volume of the tumor; or
b) a distribution of the detectable marker at an internal area or volume of the tumor.

84. The method of arrangement 83, wherein
if a) then providing the subject with an immunotherapy and if a) persists after treatment with such immunotherapy then providing the subject with an alternate immunotherapy.
85. A method of treating a human subject, the method comprising:

administering to a patient that has a neoplasia an antigen-binding construct that binds to human CD8;
monitoring a distribution of the antigen-binding construct to determine the tumor-infiltrating lymphocyte ("TIL") status within the neoplasia; and
administering an immunotherapy ("IOT") to the patient if the TIL status of at least one neoplasia is negative so as to convert the TIL status from negative to positive.

86. A method of treating a human subject, the method comprising:

administering to a patient that has a neoplasia an antigen-binding construct that binds to human CD8;
monitoring a distribution of the antigen-binding construct to determine the tumor-infiltrating lymphocyte ("TIL") status within the neoplasia; and
administering an alternative immunotherapy ("IOT") to the patient if the TIL status of the neoplasia is not positive, until the TIL status of the neoplasia becomes positive.

87. The method of arrangement 86, wherein monitoring is conducted within 8 hours of administering.
88. The method of arrangement 86 or 87, wherein the monitoring or a second monitoring is done before 36 hours from administering.
89. A method of treating a tumor in a human subject, the method comprising:

if a human subject is receiving a first treatment, determining via a CD8 binding molecule if a tumor in the subject has CD8 cells, and if so, then continuing the therapy, and if cold then changing the therapy to a second treatment; and
if the human subject is not receiving a first treatment, determining via a CD8 binding molecule if a tumor in the subject has CD8 cells, and if so, then administering an IOT to the subject.

90. A method of selecting a treatment for a patient, the method comprising:

administering a CD8 binding construct to a human patient;
determining a TIL status of a tumor in the patient, wherein if the tumor is cold, then administering an IOT that changes the TIL status of the tumor.

91. A method of changing a treatment for a subject, the method comprising:

providing a human subject on a first therapy;
administering a CD8 binding construct to the human subject;
determining if the subject has a tumor that is TIL positive under the first therapy; and
if the subject has a tumor that is TIL positive under the first therapy, continuing the therapy, and if the subject has a tumor that is TIL negative under the first therapy stopping the first therapy.

92. A method of treating a human subject having a tumor, comprising:
administering to the subject a dose of a CD8 antigen binding construct, wherein the dose comprises:

a $^{89}$Zr-labeled antigen-binding construct providing a radiation activity of from about 0.5 mCI to about 3.6 mCi; and about 10 mg or less of the antigen binding construct;
detecting the $^{89}$Zr-labeled antigen-binding construct in the patient at a first time point after administering the dose, to generate a first patient image corresponding to the first time point, wherein the first time point is about 6 hours to 36 hours after administering;
determining a first abundance and/or distribution of CD8 positive cells in one or more tumor(s) in the patient based on the first patient image; and
administering to the patient a first treatment for the first abundance and/or distribution of CD8 in the one or more tumor(s).

93. The method of arrangement 92, further comprising:

after administering the first treatment, determining a second abundance and/or distribution of CD8 cells in the one or more tissue and/or tumor in the patient based on a second patient image corresponding to a second time point and comprising a second site-specific information for the target of the CD8 antigen binding construct; and

administering to the patient a second treatment for the tumor based on a comparison of the first and second patient images.

94. The method of arrangement 93, further comprising optimizing a therapeutic dose of the second treatment based on the comparison of the first and second patient images.
95. A method of treatment of a human subject having cancer, the method comprising:

administering a CD8 antigen binding construct to a subject, wherein the CD8 antigen binding construct is labeled with a detectable label;
scanning at least 30% of the subject's body to determine a distribution of the CD8 antigen binding construct within the body;
identifying at least two separate tumors within the subject's body, via the distribution of the CD8 antigen binding construct;
determining the tumor-infiltrating lymphocyte (TIL) status of each of the two separate tumors; and
administering a first therapy to the subject if the TIL status of the at least two tumors are the same, or administering a second therapy to the subject if the TIL status of the at least two tumors are different.

96. The method of Arrangement 95, wherein determining if the at least two tumors are the same or different involves displaying the status and location of the at least two tumors.
97. The method of Arrangement 95, wherein displaying comprises a 2D or 3D representation of the location of the at least two tumors.
98. The method of arrangement 95, wherein the CD8 antigen binding construct is associated with $^{89}$Zr.
99. A method of treatment of a human subject having cancer, the method comprising:

administering a CD8 antigen binding construct to a subject, wherein the CD8 antigen binding construct is labeled with a detectable label;
identifying at least a primary tumor and a secondary tumor status within the subject's body,
identifying, via a distribution of the CD8 antigen binding construct the TIL status of the primary tumor;
administering a first therapy to the subject based on the TIL status of the primary tumor;

administering a CD8 antigen binding construct to the subject to determine a subsequent TIL status of the primary tumor; and
using the TIL status of the primary tumor to determine a subsequent appropriate treatment for the subject.

100. The method of arrangement 99, wherein the TIL status of the secondary tumor is also determined subsequent to the first therapy, but the TIL status is not used for the treatment of the primary tumor.
101. The method of arrangement 100, wherein the TIL status of the primary tumor indicates that the first therapy is effective for the first tumor, and thus, the first therapy is continued.
102. The method of arrangement 101, wherein the first therapy is not effective for the secondary tumor, and the first therapy is continued.
103. The method of arrangement 100, wherein the first therapy is not effective for the primary tumor, but is effective for the secondary tumor, and the method further comprises administering a second therapy to the subject.
104. A formulation for a CD8 composition, the composition comprising:

a CD8 antigen binding construct, wherein the CD8 antigen binding construct is less than 105 kDa in size; and
a $^{89}$Zr radiolabel associated with the CD8 antigen binding construct, wherein the radiolabel provides more than 0.5 but less than 3 (or 3.6) mCi of radiation for the formulation,
wherein the formulation is configured for administration to a human.

105. The formulation of arrangement 104, wherein the radiolabel provides more than 0.5 but less than 1.0 mCi of radiation.
106. The formulation of arrangement 104 or 105, wherein the radiolabel provides less than 0.75 mCi of radiation.
107. A CD8 minibody formulation comprising:

at least 1.5mg of a CD8 minibody; and

1.5 mCi or less of $^{89}$Zr, wherein the formulation is configured for administration to a human.

108. A composition comprising:

a first portion that comprises a CD8 antigen binding construct having an active marker; and

a second portion that comprises a CD8 antigen binding construct having an inactive marker or lacks an active marker.

109. The composition of arrangement 108, wherein the first portion to second portion are present in the composition at a molar ratio of from about 1:1 to about 1:50 or about 1:7.

110. A diagnostic composition comprising:

a first CD8 antigen binding construct that is bound to a radiolabel; and
a second CD8 antigen binding construct that is not bound to a radiolabel,
wherein the first and second CD8 antigen binding constructs have the same amino acid sequence and are present in a molar ratio of from 1:1 to 1:50,
wherein the composition provides about 0.5 mCi to about 3.6 mCi of radiation,
and wherein the composition provides 0.1-10 mg of total mass protein.

111. The diagnostic composition of arrangement 110, wherein total mass protein is between 0.5 and 10 mg.

112. A method of manufacturing a diagnostic composition, the method comprising:

conjugating a CD8 antigen binding construct to desferrioxamine to form a Df-labelled CD8 antigen binding construct;
radiolabeling the Df-labelled CD8 antigen binding construct with $^{89}Zr$ to form a radiolabeled CD8 antigen binding construct; and
mixing the radiolabeled CD8 antigen binding construct with non-radiolabelled (cold) Df-labelled CD8 antigen binding construct to form a diagnostic composition, and wherein the formulation is configured for human administration.

113. A composition comprising:

$^{89}Zr$-labeled CD8 antigen binding construct;
20 mM Histidine;
5% sucrose;
51-62 mM Sodium Chloride;
141-194 Arginine; and
2-20 M Glutamic acid, wherein the composition is configured for administration to a human subject as a diagnostic.

114. The composition of arrangement 113, wherein the 89Zr-labelled CD8 antigen binding construct is a minibody and composition comprises 1-250 micrograms per kg of weight of the subject.

115. The composition of arrangement 114, comprising 1.2-1.5mg of CD8 antigen binding construct.

116. A CD8 antigen binding construct formulation, the formulation comprising:

a CD8 antigen binding construct; and
free arginine, and wherein the formulation is configured for administration to a human.

117. The CD8 antigen binding construct formulation of arrangement 116, wherein the arginine is present at 80 to 200 mM.

118. A method of performing a PET scan in a human subject, the method comprising:

administering any one or more of the compositions or formulations of arrangements involving compositions or formulations;
waiting for a specified period of time for a distribution of the CD8 antigen binding construct to occur within the subject; and
performing a PET scan over a specified period of time for scanning.

119. The method of arrangement 118, wherein the specified period of time for a distribution of the CD8 antigen

binding construct is from 1 hour to 36 hours.
120. The method of arrangement 118 or 119, wherein the specified period of time for a distribution of the CD8 antigen binding construct is from 5 hour to 24 hours.
121. The method of any one of arrangements 118-120, wherein the specified period of time for scanning is between 10 minutes and one hour.
122. The method of any one of arrangements 118-121, wherein the specified period of time for scanning is between 12 minutes and 40 minutes.
123. The method of any one of arrangements 118-122, wherein the specified period of time for scanning is about 12 to about 20 minutes and the radio label provides more than 0.5 but less than 1.1 mCi of radiation.
124. The method of any one of arrangements 118-123, wherein the radio label is less than 1.0 mCi of radiation.
125. The method of any one of arrangements 118-124, wherein a PET scan is performed to obtain a location, distribution, ratio, and/or tumor status in the subject, and wherein the PET scan device is configured for a human.
126. A method of performing a PET scan in a human subject, the method comprising:

administering a minibody that binds to a human CD8 antigen, wherein the minibody is conjugated to a radioactive label, and wherein administering the minibody provides more than 0.5 but less than 1.1 mCi of radiation;
waiting for 1-36 hours; and
performing a PET scan over 15-30 minutes for scanning.

127. A method of positron emission tomography ("PET"), the method comprising:

administering a tracer that binds to CD8 to a human subject;
providing a scintillator;
using the scintillator to detect a pair of photons created by the tracer;
using detection of the pair of photons to localize a source of the tracer via a list of coincidence events that are processed via a processor that is configured to take an output from the scintillator and convert it to the list of coincidence events, wherein between about 300 and about 500 CD8 positive cells can be detected per $mm^2$ of a tissue or neoplasia within the subject.

128. A method of treatment of a human subject having a tumor, comprising:

providing a PET and a) MRI or b) CT combined image of the subject, wherein the combined image emphasizes an overlap in an area of at least one tumor and a CD8 ROI, wherein the area of at least one tumor is defined by a) MRI data or b) CT data and the CD8 ROI is defined by PET data;
reviewing the PET and a) MRI or b) CT combined image to determine a TIL status of a tumor in a patient; and providing an IOT treatment to the patient if the tumor is TIL negative.

129. A method of treating a human subject, the method comprising:

administering a candidate therapeutic to a human subject;
determining if a size of a tumor in the human subject has increased or decreased in response to the candidate therapeutic;
if the size has increased following the candidate therapeutic, then using a CD8 antigen binding construct to determine if an amount of CD8 present within the tumor has increased or decreased in response to the candidate therapeutic,
wherein an increase in tumor size without an increase in the amount of CD8 indicates tumor progression, whereas an increase in tumor size with an increase in the amount of CD8 indicates tumor pseudoprogression, wherein a treatment is continued if the patient is experiencing tumor pseudoprogression and wherein a treatment is changed if the patient is experiencing tumor progression.

130. The method of arrangement 129, wherein the patient is first identified as one suspected of having pseudo progression.
131. A method of distinguishing tumor progression from tumor pseudoprogression after immunotherapy in a human patient, the method comprising:

administering a CD8 antigen binding construct to a human subject,
detecting a first distribution of the CD8 antigen binding construct within the subject and a first ROI;
then administering a candidate immunotherapy to the subject

then detecting a second distribution of the CD8 antigen binding construct within the subject and a second ROI, wherein an increase in tumor size without an increase in CD8 binding indicates tumor progression, whereas an increase in tumor size with an increase in CD8 binding indicates tumor pseudoprogression, wherein a treatment is continued if the patient is experiencing tumor pseudoprogression and wherein a treatment is changed if the patient is experiencing tumor progression.

132. A method of treating a human subject having a tumor, the method comprising:

administering a CD8 antigen binding construct to the subject and detecting a first distribution of the CD8 antigen binding construct within the subject and a first ROI;
administering a candidate immunotherapy to the subject
detecting a second distribution of the CD8 antigen binding construct within the subject and a second ROI, wherein,

a) if the second ROI relative to the first ROI is the same or larger in area (relative to the subject) and demonstrates increased CD8 infiltration, then continuing with administration of the candidate immunotherapy; or
b) if the second ROI relative to the first ROI is the same or larger in area (relative to the subject) and demonstrates no increase in CD8 infiltration, then discontinuing administration of the candidate immunotherapy.

133. A method of administering a label to a human subject, the method comprising:

administering $^{18}F$ to a subject;
within about 6 hours, conducting a PET scan of the subject for a $^{18}F$ distribution of the subject;
administering a CD8 antigen binding construct to the subject, wherein the CD8 antigen binding construct is linked to $^{89}Zr$; and
within about 36 hours of administering the CD8 antigen binding construct, conducting a PET scan on the subject for a distribution of $^{89}Zr$.

134. The method of arrangement 133, wherein conducting the PET scan of the subject for the $^{18}F$ distribution of the subject is done within about 2 hours.
135. The method of arrangement 133, further comprising comparing a distribution of $^{18}F$ to a distribution of $^{89}Zr$ to determine if there are areas of elevated $^{18}F$ or $^{89}Zr$ distribution of the subject that overlap with one another, wherein areas where there is elevated 18F, that are indicative of a tumor that is TIL positive if there is also an overlapping elevated $^{89}Zr$ in the same area, and determined to be TIL negative if there is not an overlapping elevated $^{89}Zr$ in the same area.
136. The method of arrangement 133, wherein the length of time between administering the 18F and administering the CD8 antigen binding construct is not more than 10 days.
137. A method of preparing an image of a human subject, the method comprising:

providing a first data set involving a representation of a MRI or FDG-PET scan, wherein the MRI or FDG-PET scan indicates at least one tumor;
providing a second data set involving a representation of a PET scan of a 89Zr-CD8 antigen binding construct, wherein the second data set indicates at least one CD8 Region of Interest ("ROI"); and
generating a combined image from the two data sets, wherein the combined image provides a comparison of the at least one tumor and the at least one CD8 ROI.

138. A method of analyzing CD8 distribution in a subject, the method comprising:

providing an image of a distribution of a detectable marker via a first PET image, wherein the detectable marker is linked to a CD8 antigen binding construct;
providing an image of a distribution of a FDG marker via a second PET image of the subject;
creating a third image that comprises an overlay of the first PET image onto the second PET image; and
identifying the TIL status of a tumor based upon the third image.

139. The method of arrangement 138, wherein the FDG marker is $^{18}F$.
140. A method for identifying tumor characteristics, the method comprising:

scanning for a distribution of $^{18}F$ within a human subject to identify at least one tumor;
scanning for a distribution of $^{89}Zr$ labeled CD8 antigen binding construct with the human subject to identify elevated concentrations of $^{89}Zr$ within the subject; and
identifying the tumor as inflamed if the at least one tumor overlaps in location with any one or more elevated concentrations of $^{89}Zr$ within the subject.

141. The method of arrangement 140, wherein the one or more elevated concentrations of $^{89}Zr$ are elevated relative to a location that is not the at least one area of tumor growth, but is within a same organ that contains the tumor.
142. The method of arrangement 140, further comprising treating the tumor with an IOT if there is no overlap at the location for the elevated concentrations of $^{89}Zr$ and the tumor.
143. The method of arrangement 140, wherein if the at least one tumor does not overlap in location with any one or more elevated concentrations of $^{89}Zr$ within the subject, administering to the subject a candidate therapeutic to change the status of the tumor from immune excluded or immune desert to infiltrated.
144. The method of arrangement 143, further comprising:
repeating the scanning for a second distribution of $^{89}Zr$ labeled CD8 antigen binding construct with the human subject to identify if a location of the elevated concentrations of $^{89}Zr$ within the subject have changed due to the candidate therapeutic; and
identifying the tumor as inflamed if the at least one tumor overlaps in location with any one or more elevated concentrations of $^{89}Zr$ within the subject from the second distribution of $^{89}Zr$ labeled CD8 antigen binding construct.
145. A method for identifying tumor characteristics, the method comprising:

providing $^{18}F$ to a subject;
conducting a PET scan of the subject to identify $^{18}F$ distribution within the subject to render a representation of a tumor;
providing a $^{89}Zr$ labeled CD8 antigen binding construct to the subject;
conducting a PET scan of the subject to identify elevated concentrations of $^{89}Zr$ within the subject in a representation of CD8 distribution in the subject; and
comparing the representation of the tumor with the representation of CD8 distribution to identify areas of overlap in the two representations, wherein areas of tumor location and elevated concentrations of $^{89}Zr$ indicate tumors that are inflamed and do not require an IOT and wherein areas where there are tumors, but no elevated concentrations of $^{89}Zr$ represent tumors that are not inflamed and require an IOT to adjust their TIL status.

146. The method of any one of arrangements 138-145, wherein $^{18}F$ is administered to the subject, or the MRI scan is conducted, at a first time point and wherein $^{89}Zr$ is administered to the subject within 10 days after the first time point.
147. A method for identifying a solid tumor in the brain of a human subject comprising:

administering a CD8 antigen binding construct having a PET detectable label to the subject;
scanning by PET at least the head of the subject; and
providing a distribution of the PET detectable label in the brain,
wherein identification of a CD8 concentrated region in the distribution indicates the presence of a solid tumor in the brain of the subject.

148. A method of determining an effectiveness of treatment for a tumor comprising:

A. identifying the tumor by the method of arrangement 147;
B. treating the subject using an immunotherapy, radiation and/or chemotherapy,
C. repeating the method of arrangement 147 to determine if the tumor has changed in size or TIL status subsequent to the treatment in B, thereby determining if the treatment is effective.

149. The method of arrangement 148, wherein if the treatment is not effective, an alternative treatment is administered to the subject.
150. A method of monitoring CD8 bearing cells in vivo, the method comprising:

providing a CD8 minibody to a human subject, wherein the CD8 minibody binds to a human CD8 as shown in FIG. 1C, and wherein the minibody is labeled with a PET detectable marker; and
monitoring distribution of the minibody in the subject, wherein the monitoring distinguishes if a tissue volume in the subject is infiltrated with greater than or less than a selected amount of CD8 bearing cells, and wherein monitoring is achieved via PET.

151. A method of visualizing CD8 bearing cells in a human subject, the method comprising:
administering a CD8 minibody to a human subject, wherein the minibody is labeled with a PET detectable marker, wherein the minibody sequence is humanized and binds to human CD8; and monitoring a distribution of the minibody in the subject within 6-36 hours of administering the minibody to the subject, wherein the method can distinguish whether a volume of the tissue is infiltrated with greater than or less than a selected amount of CD8 bearing cells within the subject, and wherein monitoring is achieved via PET.
152. The method of arrangement 151, wherein the selected amount of CD8 bearing cells corresponds to IHC measurement of 100-500 cells/$mm^2$.
153. The method of any one of arrangements 151-153, wherein the selected amount of CD8 bearing cells corresponds to IHC measurement of 2.5 to 25% of cells in a 4 micron tissue section.
154. The method of any one of arrangements 151-153, wherein the selected amount of CD8 bearing cells is between 10,000 to 100,000 cells per $mm^3$.
155. The method of one of arrangements 151-154, wherein the tissue is 4 microns thick.
156. The method of any one of arrangements 151-155, wherein an uptake time is consistent between all CD8 antigen binding constructs.
157. A method of determining the TIL status of a tumor in a human subject comprising:

a) administering a CD8 antigen binding construct to the subject;
b) thereafter monitoring a distribution of the CD8 antigen binding construct in the subject by PET;

wherein the monitoring identifies a TIL positive tumor,
and wherein monitoring provides at least one of:

a) a tumor that is infiltrated with 500 or less CD8 bearing cells per $mm^2$ within the subject,
b) a tumor that is infiltrated with greater than or less than 500 CD8 bearing cells per $mm^2$ within the subject,
c) a tumor that is infiltrated with greater than or less than 200 CD8 bearing cells per $mm^2$ within the subject,
d) a tumor that is infiltrated with greater than or less than 100-500 CD8 bearing cells/$mm^2$,
e) a tumor that is infiltrated with less than 1% CD8 bearing cells, 1-50% CD8 bearing cells or greater than 50% CD8 bearing cells,
f) a tumor that comprises CD8 bearing cells that corresponds to IHC measurement of 2.5 to 25% of cells in a 4 micron tissue section,
g) a tumor that comprises between 10,000 to 100,000 CD8 bearing cells is per $mm^3$ of the corresponding tumor,
h) a cell intensity of CD8 bearing cells, of the corresponding tumor that is low (cold) or high (hot).

158. The method of arrangement 157, wherein tumor infiltration of CD8 bearing cells is alternatively confirmed by immunohistochemistry performed on biopsy of corresponding tumor.
159. The method of arrangement 158, wherein tumor infiltration of CD8 bearing cell density is not confirmed by biopsy of corresponding tumor.
160. A report on the status of tumor in a human subject having cancer comprising

a. a section including results from the method of arrangement 157.
b. optionally, a section on biopsy results of the corresponding tumor of the subject;
c. optionally, a section on the status of the tumor according to RECIST or PERCIST criteria.

161. A method of visualizing a distribution of CD8 bearing cells in a human subject by PET, the method comprising:

administering a CD8 antigen binding construct to the subject, wherein the CD8 antigen binding construct is labeled with a PET detectable label; and
monitoring a distribution of the CD8 antigen binding construct in the subject within 6-36 hours of administering the CD8 antigen binding construct, wherein monitoring can detect a tissue infiltrated with 500 or less CD8 positive cells per $mm^2$ within the subject, and wherein monitoring is achieved via PET and converted to an image for visualizing the distribution.

162. A method of visualizing cells in a human, the method comprising:

providing a means of binding human CD8 positive cells to a subject, wherein the means comprises a detectable label; and
monitoring and determining a first distribution of the means of binding human CD8 positive cells in the subject within 6-36 hours of administering the means of binding human CD8 positive cells to the subject.

163. A method of determining the TIL status of a tumor in a human subject comprising:

administering a CD8 antigen binding construct to the subject, wherein the CD8 antigen binding construct is labeled with a PET detectable label, and monitoring a distribution of the CD8 antigen binding construct having the PET detectable label in the subject; and
evaluating the degree to which the PET detectable label has penetrated a known or suspected tumor in the subject, wherein a tumor is TIL positive if the PET detectable label has penetrated the tumor and TIL negative if not.

164. A method of imaging a change in distribution of CD8 cells in a human subject having cancer comprising:

administering to the subject a CD8 antigen binding construct;
determining by PET a first distribution of CD8 antigen binding construct in the subject;
administering an immunotherapy to the subject; and
thereafter determining by PET a second distribution of CD8 antigen binding construct in the subject, wherein a difference between the first distribution and the second distribution demonstrates the change.

165. The method of arrangement 164, wherein the second distribution is determined based on a second PET image of the subject obtained within 10 days of administering the CD8 antigen binding construct.
166. The method of arrangement 164, wherein the second distribution is determined based on a second administration to the subject of CD8 antigen binding construct.
167. A method of determining if a subject is responding to an IOT, the method comprising:
providing a baseline CD8 distribution for the subject within a ROI, via an in vivo technique;

administering an IOT;
providing a treated CD8 distribution for the subject within the ROI, wherein the treated CD8 distribution is obtained within 35 days of administering the IOT; and
comparing the treated CD8 distribution to the baseline CD8 distribution to determine if the subject is responding to the IOT.

168. A method of predicting a subject's responsiveness to a checkpoint inhibitor, the method comprising:

providing a baseline CD8 distribution for the subject within a ROI, via an in vivo technique; administering a checkpoint inhibitor;
providing a checkpoint inhibitor CD8 distribution for the subject within the ROI, wherein the checkpoint inhibitor CD8 distribution is obtained within 35 days of administering the IOT; and
comparing the checkpoint inhibitor CD8 distribution to the baseline CD8 distribution to determine if the subject is responsive to the checkpoint inhibitor.

169. The method of arrangement 168, wherein the treated CD8 distribution is obtained within 3-8 days of administering the IOT.
170. A method of monitoring an effectiveness of a therapy, the method comprising:

providing a first CD8 image for a subject, wherein the subject is at a baseline for a therapy; administering a therapy to the subject;
providing a second CD8 image for the subject, wherein at least one day has passed since the therapy was administered to the subject, wherein, if there is no significant increase in CD8 imaging agent, the subject is not responding to the therapy.

171. A method of analysis of a tumor, the method comprising:
characterizing a degree of relative CD8 infiltration in a tumor in a subject in response to a candidate therapeutic, said characterizing comprising observing if there is an increase or a decrease in degree of CD8 infiltration via a CD8 antigen binding construct comprising a radiolabel, wherein an increase in CD8 infiltration indicates that the

candidate therapeutic is functioning as a therapeutic, wherein a decrease in CD8 infiltration indicates that the candidate therapeutic is not functioning as a therapeutic, and wherein the tumor has increased in size between before the candidate therapeutic is administered and after the candidate therapeutic is administered.

172. A method of determining a standard uptake value, the method comprising:

applying an antigen binding construct to a subject, wherein the antigen binding construct comprises a radioactive probe;
determining r, where r is the radioactivity concentration (kBq/ml) measured by a PET scanner within a ROI of radiation from the antigen binding construct;
determining a', wherein a' is the decay-corrected amount of the injected radiolableeld tracer (kBq);
determining w, the weight of the patient; and
determining SUV as being the result of r(a'/W).

173. A method of analyzing an image, the method comprising:

providing an image;
defining on the image a first region of interest (ROI) by marking the image;
determining a signal intensity for a data point within the first ROI;
determining a maximum signal intensity within the first ROI;
determining a mean value of the signal intensities within the first ROI; and
summing together each signal intensity within the first ROI to obtain a first summed signal level for the first ROI, wherein the first ROI represents data for an amount of a detectable marker associated with an antigen binding construct that has been administered to a subject.

174. The method of arrangement 173, further comprising repeating the process of arrangement 97 for a second ROI to determine a second summed signal level and comparing the first and the second summed signal levels, wherein when the first summed signal level is less than the second summed signal level, it indicates the presence of increased CD8 in the second ROI.

175. The method of arrangement 173, wherein the first ROI is a same location as the second ROI, but differ in that the first ROI is provided at a first time point and the second ROI is provided at a second time point and a candidate therapeutic has been administered after the first time point but before the second time point.

176. A method of generating an image, the method comprising:

applying an antigen-binding construct that binds to CD8, wherein the antigen-binding construct comprises a detectable marker; and
detecting at least one detectable marker within a location within the tumor and assigning a positive pixel for each detectable marker detected; and
generating an image based on a distribution of each positive pixel assigned, wherein the image is stored in a non-transitory computer readable media.

177. A non-transitory computer readable medium comprising:
data regarding an image of a PET scan, wherein the data has been obtained by the method of any one of arrangements above involving a PET scan.

178. A method of performing a biopsy in a subject, the method comprising:

providing an antigen binding construct that binds to CD8 to the subject;
selecting, based upon an elevated level of the antigen binding construct within a particular tumor, the particular tumor for a biopsy; and
collecting a sample from the particular tumor.

179. A method of performing a biopsy in a subject, the method comprising:

providing an antigen binding construct that binds to CD8 to the subject;
selecting, based upon a distribution the antigen binding construct within a particular tumor, the particular location within the tumor to biopsy; and
collecting a sample from the particular location within the tumor, wherein the particular location is either a) internal to the tumor or b) on a surface of the tumor.

180. A method of detecting an immune-related adverse event (irAE), the method comprising:

identifying a human subject on a checkpoint inhibitor therapy;
administer a CD8 antigen binding construct to the human subject, wherein the CD8 antigen binding construct comprises a detectable label;
monitoring the detectable label within the subject to determine if there is immune related toxicity, wherein the immune related toxicity is indicated by an increase in CD8 binding;
if the immune related toxicity is severe or irreversible, stopping the checkpoint inhibitor therapy,

if the immune related toxicity is not severe or irreversible, suspending the checkpoint inhibitor therapy while continuing to monitor CD8 levels until they return to a lower level;
181. The method of arrangement 180, wherein a location of a tumor indicates which organ or system is experiencing an immune-related adverse event (irAE).
182. The method of arrangement 180, wherein the comparison provides an indication of the severity of the irAE.
183. The method of any one of arrangements involving radiation, wherein a radiation dose is administered in an amount of 0.5-3.0 mCi +/- 20%.
184. The method of any one of arrangements involving radiation, wherein a radiation dose is administered in an amount of 0.4-3.6 mCi.
185. The method of any one of arrangements involving an antigen binding construct, wherein an amount of the antigen binding construct is 0.2 - 10 mg.
186. The method of any one of arrangements involving a CD8 antigen binding construct, wherein the CD8 antigen binding construct is less than 105 kDa in size.
187. The method of any one of arrangements involving a CD8 antigen binding construct, wherein the CD8 antigen binding construct is linked to $^{89}Zr$.
188. The method of any one of arrangements involving administration of an antigen binding construct, wherein 6 to 36 hours passes after administration of an antigen binding construct prior to a subsequent step in the process.
189. The method of any one of arrangements involving a PET scan, wherein a PET scan time is less than 40 minutes.
190. The method of any one or arrangements involving a PET scan, wherein a PET scan time is less than 25 minutes and more than 2 minutes.
191. The method of any one of arrangements 1-32, 40-53, 55-103, 112, 118-159, and 161-190, wherein the subject receives a therapeutic, and wherein the therapeutic is selected from at least one of: eftilagimod alpha, relatlimab, OMP-313M32, EOS8844488, TJT6, AB154, and dual TIGIT/PD-L1-targeted small-molecule drugs.
192. The method of any one of arrangements 1-32, 40-53, 55-103, 112, 118-159, and 161-191, wherein the subject receives a therapeutic, and wherein the therapeutic is selected from at least one of: an antibody to LAG-3, and antibody to TIGIT, MK-4280, MK-7684, and/or any of the preceding in combination with Keytruda.
193. The method of any one of arrangements arrangements 1-32, 40-53, 55-103, 112, 118-159, and 161-192, wherein the subject receives a therapeutic, and wherein the therapeutic is selected from at least one of: EOS8844488, TJT6, AB154, Aurigene, and/or a dual TIGIT/PD-L1 inhibitor.
194. The method of any one of arrangements 1-32, 40-53, 55-103, 112, 118-159, and 161-193, wherein the status is selected from one or more of:

A) i) immune inflamed or ii) immune excluded or immune desert,
B) homogeneous infiltration, heterogeneous infiltration, and/or
C) for inflamed tumors: high, medium, low degree of inflammation

195. The method of any one of arrangements 1-32, 40-53, 55-103, 112, 118-159, and 161-194, wherein at least 30% of a subject's body is scanned via PET for CD8+ TIL load.
196. The method of any one of arrangements 1-32, 40-53, 55-103, 112, 118-159, and 161-195, further comprising a treatment that alters CD8+ T cell presence, CD8+ T cell activation, CD8+ T cell number or CD8+ T cell trafficking in relation to tumor lesions.
197. The method of any one of the arrangements involving a treatment, wherein the treatment comprises at least one of chemotherapies, oncolytic viruses, vaccines, radiation therapy, cellular therapies etc.
198. The method of any one of arrangements involving a composition or the composition of any one of arrangements involving a composition, wherein the CD8 minibody comprises a heavy chain variable region and a light chain variable region, and wherein the heavy chain variable region consists essentially of a human amino acid sequence and the light chain variable region consists essentially of a human amino acid sequence.
199. The method of any one of arrangements involving a CD8 minibody, wherein the CD8 minibody binds to a CD8 sequence consisting of the sequence of SEQ ID NO: 134 and/or 135.

200. The method of any one of arrangements involving a minibody, wherein the minibody comprises the heavy chain CDRs in any of the CDR SEQ ID Nos: provided herein, and the light chain CDRs in any of the CDR SEQ ID Nos: provided herein.
201. The method of any one of arrangements involving a minibody, wherein the minibody comprises a heavy chain variable region that is at least 80% identical to the heavy chain amino acid sequence in any of the heavy chain SEQ ID Nos: provided herein, and a light chain variable region that is at least 80% identical to the light chain amino acid sequence in any of the heavy chain SEQ ID NOs: provided herein.
202. The method of any one of arrangements involving a known tumor, wherein the known tumor is selected from one or more cancer of: carcinoma, lymphoma, blastoma, sarcoma, and leukemia, lymphoid malignancies, squamous cell cancer (e.g. epithelial squamous cell cancer), lung cancer, small-cell lung cancer, non-small cell lung cancer, adenocarcinoma of the lung and squamous carcinoma of the lung, cancer of the peritoneum, hepatocellular cancer, gastric or stomach cancer, gastrointestinal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain, head and neck cancer, adult and pediatric solid cancers, head and neck squamous cell carcinoma, classical Hodgkin lymphoma, primary mediastinal large B-cell lymphoma, urothelial carcinoma, microsatellite instability-high cancer, cervical cancer, Merkel cell carcinoma, esophageal cancer, Cutaneous Squamous Cell Carcinoma, prostate cancer, nasopharyngeal cancer, small cell lung mesothelioma, Primary Mediastinal B-Cell Lymphoma and solid tumors.
203. An antigen-binding construct for use, or for use as a medicament, in the method described in any one of the preceeding arrangements.
204. A CD8 PET tracer for use, or for use as a medicament, in the method described in any one of the preceding arrangements.
205. The antigen-binding construct, or the CD8 PET tracer of any one of the preceding arrangments, wherein the radiolabel provides more than 0.5 but less than 1.0 mCi of radiation.
206. The antigen-binding construct, or the CD8 PET tracer of any one of the preceding arrangments, wherein the radiolabel provides less than 0.75 mCi of radiation.
207. The composition, the formulation, the antigen-binding construct, or the CD8 PET tracer of any one of the preceding arrangements, for use in at least one of: imaging, detection, diagnosis, surgery, staging, treatment, monitoring of treatment, monitoring of disease progression, and monitoring therapy.
208. The composition, the formulation, the antigen-binding construct, or the CD8 PET tracer of any one of the preceding arrangements, for use in at least one of: imaging, detection, diagnosis, surgery, staging, treatment, monitoring of treatment, monitoring of disease progression, and monitoring therapy of a CD8 dependent disorder, optionally cancer.
209. A non-invasive method to visualize CD8 bearing cells in a human subject, the method comprising:

(a) administering to a human subject a tracer that selectively binds to CD8 and is labeled with a PET detectable marker,
(b) monitoring a distribution of the tracer in the subject within 1-36 hours of (a) by PET, in the absence of IHC (immunohistochemistry), wherein a PET scan indicates whether a volume of tissue within the subject is infiltrated with greater than or less than a selected amount of CD8 bearing cells.

## EXAMPLES

### *Example 1*

Immunohistochemical (IHC) staining of formalin fixed, paraffin embedded normal human and cancer tissues was performed using a validated anti-CD8 reagent (Clone C8/144B; Dako) to determine the distribution of CD8+ T cells. The results demonstrated specific membrane and cytoplasmic staining of CD8+ T cells in normal spleen, tonsil, lymph nodes and thymus as well as in a variety of other tissues including the gut. Staining of melanoma and lung cancers revealed that the CD8+ T cells were mostly distributed in a sporadic and non-aggregated fashion and did not show a strong association with a particular anatomic structure in the disease setting.

### *Example 2*

$^{89}$Zr-Df-IAB22M2C, an ~80 kDa minibody (Mb) with high affinity to CD8 glycoprotein (binding EC50 = 0.4 nM) that is conjugated with desferrioxamine (Df) and radiolabeled with the positron emitting radionuclide Zirconium-89 ($^{89}$Zr;

$T_{1/2}$ 78.4 hours) was developed for imaging CD8+ T cells in humans. The Mb fragment is a bivalent homodimer with each monomer having of a single-chain variable fragment (scFv) linked to the human IgG1 CH3 domain via modified IgG1 hinge sequence. The Mb lacks Fc receptor interaction domains, and has a smaller size compared to intact mAbs. IAB22M2C was engineered from (i.e. was developed using information about) the humanized variable heavy and light chain sequences of OKT8 antibody targeting human CD8.

In vitro and in vivo tests were conducted to evaluate the safety and potential efficacy of $^{89}$Zr-Df-IAB22M2C. IAB22M2C and Df-IAB22M2C bound with high affinity to CD-8 expressing purified human T cells and HPB-ALL leukemia cells (DSMZ, Braunschweig, Germany), with an EC50 of 0.4 nM and 0.3 nM, respectively. Binding affinity and specificity were not impacted by conjugation and chelation with Zirconium. Studies using peripheral blood mononuclear cells (PBMCs) obtained from 10 healthy human donors showed no measurable or reproducible impact on proliferation or depletion of CD8+ T cells and no consistent activation of cytokines when donor T cells were exposed to soluble or immobilized Mb protein. Studies that evaluated the effect of saturating concentrations of $^{89}$Zr-Df-IAB22M2C on proliferation and viability of CD8+ T cells also showed no impact.

Preclinical studies conducted in vivo in humanized NSG™ mice engrafted with CD34+ stem cells showed no acute effects of Df-IAB22M2C on CD8+ T cell populations or cytokine release after intravenous administration. In contrast, IAB22FL, the full length human IgG1 molecule having the same variable sequences that binds to the same epitope as IAB22M2C, caused depletion of CD8+ T cells in hu-CD34 NSG™ mice. The inert pharmacology of Df-IAB22M2C compared with IAB22FL and OKT3, another antibody known to induce T cell depletion and cytokine activation, is likely due to the removal of the CH2 domain and corresponding effector functions that are associated with intact IgGs. The Phase 1 clinical trial proposed human starting dose of 1.5 mg is 16.6 fold lower than the dose administered to humanized mice in these toxicity studies where no adverse effects on CD8+ T cell populations and cytokine release were observed.

The non-human primate (NHP) GLP toxicology study that was conducted showed that intravenous administration of Zr-Df-IAB22M2C once weekly for two doses at 0, 2, 10, or 25 mg/kg/dose resulted in no early deaths, no gross observations, no alterations in organ weights, and no microscopic findings at both terminal and recovery time points. The No Observable Adverse Effect Level (NOAEL) from this toxicology study was determined to be 25 mg/kg and supports the planned human doses of 1.5 mg, 5 and 10 mg total protein. The maximum feasible dose administered in the NHP study, 25 mg/kg, was equivalent to administering 1.5 gm of drug to a 60 kg person or 1.75 gm to a 70 kg person. These doses provide a greater than 1000-fold safety margin for the proposed 1.5 mg starting dose and a 150 and 175-fold safety margins, respectively for a 60 or 70 kg person at the maximum 10 mg dose.

Preclinical imaging and biodistribution studies have shown favorable pharmacokinetics and demonstrated the ability of $^{89}$Zr-Df-IAB22M2C to detect infiltrating CD8+ T cells in a Graft versus Host Disease (GvHD) model established by engrafting NSG™ mice with human PBMCs and in MAtrigel plug assays.

***Example 3***

The manufacturing process for $^{89}$Zr-Df-IAB22M2C incorporated both conjugation and radiolabeling of IAB22M2C to generate unit doses for each patient. Deferoxamine (Df) was conjugated to lysine residues on IAB22M2C to produce the intermediate Df-IAB22M2C, and subsequently radiolabeled with the positron emitting radionuclide $^{89}$Zr (as oxalate salt) as shown in FIG. 40. Following radiolabeling and purification, the product is formulated to yield cohort-specific protein doses and sterile filtered prior to administration.

**FIG. 40:** Schematic structure of IAB22M2C Mb (having the sequence provided in FIG. 39; SEQ ID NO: 147) reacted with chemical structure of desferrioxamine (Df) and radiolabeled to yield the drug product $^{89}$Zr-Df-IAB22M2C. DS = drug substance; DP = drug product.

The IAB22M2C Mb used as a starting reference the public domain murine anti-huCD8 monoclonal antibody (mAb) OKT8. The DNA sequence information taken from variable regions of OKT8 was humanized by CDR grafting wherein amino acids in the murine framework were replaced with amino acids suitable for use in humans. The resulting humanized variable domains were predicted to have a decreased likelihood of eliciting anti-drug antibody (ADA) responses in humans. The humanized variable VL and VH sequences were linked by an 18 amino acid Gly-Ser-rich linker sequence to form a scFv that was joined to the human IgG1 CH3 domain via a modified human IgG1 hinge sequence to yield IAB22M2C.

The drug substance IAB22M2C was manufactured, tested and released in accordance with current good manufacturing practices (cGMP) and general biologics products standards by Lonza Biologics plc (Slough, UK).

The isothiocyanate-activated form of the desferrioxamine (Df) chelating agent (1-(4-Isothiocyanatophenyl)-3-[6,17-dihyroxy-7,10,18,21-tetraoxo-27-[Nacetylhydroxylamino)- 6,11,17,22-tetra-azaheptaeicosane]thiourea); B-705) (GMP grade) was conjugated to IAB22M2C to produce the Df-IAB22M2C critical intermediate with an chelator:minibody ratio (CMR) of approximately 2. The final drug product 89Zr-Df- IAB22M2C was manufactured by reacting Df-IAB22M2C with $^{89}$Zr-oxalate. Following the reaction, the $^{89}$Zr-Df-IAB22M2C was challenged with an excess of the chelator DTPA to remove any free or loosely bound $^{89}$Zr-oxalate. $^{89}$Zr-Df-IAB22M2C is separated from $^{89}$Zr-DTPA by gel filtration and

buffer exchanged into the formulation buffer. The product is then sterile filtered through a 0.2 μm PVDF membrane and samples removed for Quality Control. A flow diagram depicting the conjugation and radiolabeling process is presented in FIG. 41. $^{89}$Zr-Df-IAB22M2C was manufactured according to cGMP for PET drugs, as well as USP requirements for PET radiopharmaceuticals.

The manufacturing process incorporated both conjugation and radiolabeling of IAB22M2C to generate unit doses for each patient. Analytical testing was performed on the conjugated intermediate, Df-IAB22M2C, prior to proceeding to radiolabeling. The in-process testing specifications for the Df-IAB22M2C intermediate is shown in Table 3.1. The testing and acceptance criteria for release of 89Zr-Df-IAB22M2C are presented in Table 3.2.

**Table 3.1:** In-process testing specifications for Df-IAB22M2C

| In-Process testing: Df-IAB22M2C | Specification |
|---|---|
| Antibody concentration | 1.3 - 4.5 mg/mL |
| Purity by UV SE-HPLC (CYCRAD. QC.009) | LMW: ≤ 10%<br>Main: ≥ 80% monomer<br>HMW: ≤ 10% |
| Chelator to Minibody Ratio (CMR) | 0.8 - 2.5 |

**Table 3.2:** Release testing and acceptance criteria for $^{89}$Zr-Df-IAB22M2C

| Pre-release criteria 89Zr-Df-IAB22M2C | Specification |
|---|---|
| Radiochemical Purity by iTLC | ≥90% |
| Purity by Radio-SE-HPLC | LMW: ≤ 10%<br>Main: ≥ 80% monomer<br>HMW: ≤ 10% |
| Bacterial Endotoxins | ≤ 20 EU/mL |
| Visual Inspection | Clear and particle free |
| pH | 6.5 ± 1.0 |
| Membrane Filter Integrity Test | Bubble point pressure ≥ 50 psi |
| Radionuclidic identity | Peaks at 511 and 909 KeV |
| Immunoreactivity | ≥70% |
| **Post-release criteria** | **Specification** |
| Sterility Test | No Growth |

The final drug product was 3.0 (±20%) mCi of 89Zr-Df-IAB22M2C formulated in 20 mM Histidine, 5% sucrose, 51 - 62 mM Sodium Chloride, 141 - 194 mM Arginine, and 2-20 mM Glutamic acid depending on the total protein dose administered. The route of administration was intravenous infusion. The dose was stored at room temperature and was used within 24 hours of the end of synthesis.

In some embodiments, the final drug product can be placed within a container. On the container or associated with the container can be a label. In some embodiments, for a bulk substance, the label will instruct storage at -65 C or below and the protein concentration is 3.0±0.3 mg/ml. In some embodiments, for the drug substance, the label will state: 20mM histidine, 100mM sodium chloride, 0.2 M L-arginine hydrochloride, pH 6.5, and storage at 2-8 C. The appropriate compositions are in each of the respective vials. In some embodiments, there is an in-process vial label. The label will include space for records regarding, one or more of: Lot#, calibration date, expiration date, IRB, calibration time, expiration time, activity, mass and volume.

***Example 4***

Non clinical studies to determine the in vitro properties of IAB22M2C, Df-IAB22M2C, Zr-Df-IAB22M2C and 89Zr-Df-IAB22M2C were conducted to establish binding and functional properties in CD8 over-expressing cell lines, purified human T cells and peripheral blood mononuclear cells (PBMCs) obtained from different donors. Functional

assays included positive controls, OKT3 and TGN1412, two reference IgGs that are known to activate T cells in humans. IAB22M2C lacks the CH1 and CH2 domains present in intact antibodies and therefore the pharmacologic effects of both unconjugated and the respective Df-conjugated forms are mediated primarily by engagement of the CD8 antigen on the cell surface. To date neither IAB22M2C, Df-IAB22M2C, Zr-Df- IAB22M2C nor 89Zr-Df-IAB22M2C have mediated any CD8-specific adverse pharmacological effects indicating that $^{89}$Zr-Df-IAB22M2C can be used as an agent for immunoPET imaging of CD8+ T cells in response to immuno- and other therapies.

*Binding affinity and specificity*

The binding affinity and specificity of IAB22M2C and Zr-Df-IAB22M2C to human CD8 expressed on cell surfaces were evaluated by flow cytometry for a several cell types, including HPB-ALL leukemia cells (DSMZ, Braunschweig, Germany), purified human T cells, and PC3 prostate cancer cells engineered to express huCD8 (ImaginAb, Inc.). The results from competitive cell based binding assays are presented in FIGs. 42A and 42B.

IAB22M2C bound to CD8 expressed on human T cells and the HPB-ALL cell line with an EC50 of 0.4 nM and 0.3 nM respectively. Df-conjugation and Zr-chelation did not significantly impact the binding of Zr-Df-IAB22M2C to human CD8 expressed on HPBALL or human T cells. The IC50 values for both IAB22M2C and Zr-Df-IAB22M2C were similar and ranged from 0.9 to 1.5 nM in this binding assay.

*In Vitro Proliferation Assays with Cultured Human Peripheral Blood Mononuclear Cells (PBMCs)*

T cells can be stimulated by appropriate stimuli (e.g. antigen or TCR crosslinking) to proliferate and secrete cytokines when cultured in vitro. The ability of IAB22M2C and Df-IAB22M2C to stimulate T cell proliferation was evaluated using human PBMCs isolated from donors. OKT3 as well as TGN1412, were used as positive controls. The assay included pre-incubation using the RESTORE protocol which has been optimized to detect T cell responses to potentially activating mAbs such as TGN1412 [Römer et al., 2011]. Immobilization of proteins to tissue culture plates promotes T cell receptor complex cross-linking to deliver a stronger mitogenic response [Stebbings et al., 2007]. The ability of Zr-Df-IAB22M2C, IAB22M2C and the control antibodies OKT3 and TGN1412 to stimulate proliferation in the presence of proteins immobilized to tissue culture plates was evaluated and the results presented in FIG. 43.

**FIG. 43:** Effect of OKT3, Zr-Df-IAB22M2C (22-160209-03), IAB22M2C (CHT020916), IAB22FL, TGN1412, Df-Mb control and human IgG isotype controls on T cell proliferation after incubating human PBMCs from 4 of the same human donors with the indicated concentrations of immobilized investigational compounds for 4 days. (**** $P<0.0001$)

No measurable proliferation was detected when PBMCs from 4 different human donors were incubated in the presence of immobilized Zr-Df-IAB22M2C, IAB22M2C, Dfcontrol Mb or isotype control antibody. In contrast, plate bound OKT3 induced a statistically significant proliferative response when compared to human IgG1 control ($p < 0.0001$ analysis using one-way ANOVA with Dunnetts multiple comparison).

*Cytokine Release from Cultured Human PBMCs*

Activation of T cells in vivo and in vitro is accompanied by secretion of proinflammatory cytokines, including IL-2, IFN-$\gamma$ and TNF-$\alpha$. Df-IAB22MC and IAB22MC were evaluated for their ability to elicit cytokine release when cultured with human PBMCs. Human PBMCs from 5 donors were pre-cultured using the RESTORE protocol to evaluate the activity of the test articles Zr-Df-IAB22M2C and IAB22M2C as well as TGN1412 and OKT3 as positive controls. In this study the test articles were evaluated by adding them to the aqueous culture medium (solution) or after immobilizing them on a solid support to mimic delivery of a localized signal. The results are presented in FIG. 44.

**FIG. 44:** Cytokine release from human PBMCs incubated with OKT3, Zr-Df-IAB22M2C (22-160209-03), IAB22M2C (CHT020916), IAB22FL, Df-Mb control and IgG1 isotype in solution (Left panel) or following immobilization (Right panel) for 18 hours. Cytokine levels were determined using LegendPlex™ multiplex bead assay.

The results demonstrated no significant release of IFN-$\gamma$, TNF-$\alpha$. or IL-2 when human PBMCs from 5 different donors were incubated with soluble or immobilized Zr-Df- IAB22M2C, IAB22M2C, IAB22FL, Df-Control Mb and isotype control antibody for 18 hours, a time period sufficient for primary and secondary cytokine release. An increase in IL-6 was detected in PBMCs from one donor following incubation with immobilized Zr- Df-IAB22M2C or aqueous Df-IAB22M2C. OKT3 stimulated robust cytokine release in PBMCs from five donors when test articles were presented in solution or immobilized to plates. In contrast, TGN1412 mediated cytokine release that was lower than OKT3 but consistently increased when PBMCs were incubated with immobilized test articles.

*Effect of 89Zr-Df-IAB22M2C on T Cell Proliferation, Viability*

The potential effect of radiolabeled 89Zr-Df-IAB22M2C on T cell proliferation and survival was evaluated in

vitro. All preparations contained an approximated 20-fold excess of $^{89}$Zr-Df-IAB22M2C or Zr-Df-IAB22M2C over the number of estimated CD8 receptors present on the cells. RESTORED human PBMCs were incubated at 37°C for 2 hours with $^{89}$Zr-Df-IAB22M2C or with an unlabeled Zr-Df-IAB22M2C Mb preparation. After incubation and washing, the cells were counted and plated into tissue culture plates in RPMI medium containing 50 IU/mL of IL-2 and 10% heat inactivated human serum with or without 1 μg/mL OKT3 to stimulate proliferation. The cells were counted every other day for 6 days to determine viability or proliferation in response to OKT3. Cells treated with no Mb or with nonradioactive Zr-Df-IAB22M2C served as controls. The results are presented in FIG. 45.

**FIG. 45:** Effect of 89Zr-Df-IAB22M2C (10 μCi/μg), $^{89}$Zr-Df-IAB22M2C (0.1 μCi/μg) and Zr-Df-IAB22M2C compared to no treatment on: (A) T cell survival and (B) T cell proliferation after incubating human PBMCs with the indicated radiolabeled and control compounds for 2, 4 and 6 days.

Incubation with radiolabeled $^{89}$Zr-Df-IAB22M2C had no statistically significant impact on cell viability after 2, 4 and 6 days of incubation in media that supports the survival of CD8+ T cells (2-Way ANOVA analysis with Turkey's multiple comparison test). Cell viability was not altered in cells treated with $^{89}$Zr-Df-IAB22M2C when compared to cells treated with nonradioactive Zr-Df-IAB22M2C or no treatment controls. Additionally, incubation with radiolabeled $^{89}$Zr-Df-IAB22M2C had no statistically significant impact on CD8+ T cell proliferation stimulated by OKT3 and IL-2 after 2, 4 and 6 days of incubation using a 2-Way ANOVA analysis with Turkey's multiple comparison test. A statistically significant increase in cell numbers on Day 6 was measurable under all conditions evaluated when compared to the number of viable cells present on Day 2 or Day 4 ($P < 0.0001$ using 2-Way ANOVA analysis with Turkey's multiple comparison test). These results indicate that exposure of human PBMCs to high specific activity $^{89}$Zr- Df-IAB22M2C had no measurable effect on CD8+ T cell viability or proliferation.

## *Example 7*

A Phase I, open label, single-center, non-randomized, single-dose safety study in patients with metastatic melanoma who were being treated with one or more checkpoint inhibitors (CIs) as a part of their standard medical care was carried out. This was a dose escalation study using Positron Emission Tomography (PET) imaging with 3 mCi $^{89}$Zr-Df-IAB22M2C administered as 1.5, 5, and 10 mg total mass protein doses. Safety tests and assessments was performed across study visits. The data gathered in this study was used to evaluate and recommend the superior time point and protein dose for detecting CD8+ activity with PET/CT imaging and $^{89}$Zr-Df-IAB22M2C. Secondary endpoints included: determining the ability of $^{89}$Zr-Df-IAB22M2C PET/CT to localize to lymphoid organs and tumor sites where CD8 T cells may reside, correlating $^{89}$Zr-Df- IAB22M2C PET/CT signals with CD8 expressing T cells and other immune biomarkers in tissue samples (e.g. PD1, PD-L1, IFN-γ and CD107a) obtained by optional biopsy, and correlating the $^{89}$Zr-Df-IAB22M2C PET/CT uptake in tumor sites with tumor response as evaluated on follow-up conventional imaging.

Once initial safety and feasibility of $^{89}$Zr-Df-IAB22M2C was established including dose optimization and image acquisition timing, the safety of repeat doses (two) of $^{89}$Zr-Df-IAB22M2C was evaluated. Product development included testing the feasibility of repeat dosing and repeat imaging to better assess treatment responses and therapeutic dose optimization upon administration of immunotherapy.

## *Example 8*

The use of $^{89}$Zr-Df-IAB22M2C did not pose any significant risk to human subjects based upon the extensive and well controlled non clinical in vitro and in vivo assessments demonstrating no functional activity of the CD8 targeting Mb.

Because $^{89}$Zr-Df-IAB22M2C is a humanized antibody fragment, the risk of an infusion reaction exists. In prior trials using a radiolabeled Mb fragment directed to PSMA, no incidents of infusion reaction occurred in the 57 patients studied. The risk of developing an infusion reaction may be decreased by pre-medicating with intravenous diphenhydramine (25-50 mg) and oral acetaminophen (650 mg). See Example 9.

Human anti-Drug Antibody (ADA) testing was evaluated in subjects receiving a different investigational radiolabeled Mb ($^{89}$Zr-Df-IAB2M). Of the 47 evaluable serum samples, 5 were immunoreactive. Transience or permanence of the immunoreactivity was not established. The titers in these positive samples were low and did not correlate with total protein dose, nor were they associated with any safety findings that differentiated them from the patients who did not generate antibodies to the product. It is unknown whether $^{89}$Zr-Df-IAB22M2C will result in any immunogenicity.

## *Example 9*

The dose of $^{89}$Zr-Df-IAB22M2C is infused using a syringe pump, over a 5 to 10-minute time period. Following the infusion, the IV tubing is cleared with 10 - 20 cc of Normal Saline for Injection slowly. Because of the potential for infusion or allergic reaction, the first patient enrolled in each cohort is monitored for any possible adverse effects (AEs)

for two hours after the infusion for any clinically significant change in vital signs from pre-infusion to post-infusion that results in a change in subject management. This monitoring includes vital signs pre-infusion and every 5 minutes out to 60 minutes, then every 15 minutes for another hour. If the first subject in a cohort does not experience any AEs, then subsequent subjects' vital signs is monitored for 1 hour. This monitoring will include vital signs pre-infusion and every 5 minutes out to 15 minutes and then every 15 minutes out to 1 hour. If an AE is detected within a cohort revert to the 2-hour monitoring period. Longer monitoring times may be performed at the discretion of the attending physician.

For severe infusion reactions immediately discontinue the infusion. Signs and symptoms of a severe infusion reaction could include urticaria, hypotension, angioedema, hypoxia, bronchospasm, pulmonary infiltrates, acute respiratory distress syndrome, myocardial infarction, ventricular fibrillation, and/or cardiogenic shock. For less severe or minor infusion reactions (signs or symptoms without cardiopulmonary compromise), temporarily reduce the rate of infusion or halt the infusion until symptoms improve, then continue the infusion at a slower rate

Example 10

Radiolabeling

A generic radiolabeling procedure was used for test-labeling of the Df-conjugates with $^{89}Zr$. The drug product $^{89}Zr$-Df-IAB22M2C is manufactured by conjugation of deferoxamine isothiocyanate B-705 (Df) to the drug substance, IAB22M2C, followed by radiolabeling with Zirconium-89 ($^{89}Zr$). In brief, a pH-neutralized solution of $^{89}Zr$ Oxalate is mixed with Df-IAB22M2C at specific activity that varies between 3 and 30 mCi/mg. The solution is allowed to incubate for time that ranges from 30 min to 1 hour at the ambient temperature. At the end, the reaction is quenched using 5 mM DTPA solution (pH 7-8) and the radiolabeled product is recovered by desalting using a G-25 Sephadex column equilibrated with an appropriate formulation buffer. Prior to purification, the reaction labeling efficiency is assessed by iTLC. The final $^{89}Zr$-Df-IAB22M2C drug product is prepared with cold, unconjugated IAB22M2C and formulation buffer, such that the dose administered at the time of infusion is 3.0 mCi.

Table 10.1: Representative radiolabeling summary using generic procedure

| | |
|---|---|
| Product | 89Zr-Df-IAB22M Lot: 42-040A |
| Activity used | 972 μCi |
| Amount Df-IAB22M2C Protein | 70 μg |
| Labeling efficiency | 97.89% |
| Purity | 99.45% |
| Specific activity | 13.59 μCi/μg |
| Immunoreactivity (HPB-All cells) | 83.3% |
| Purity by SE HPLC: less than 2%. | |

Example 11

Various patients were given $^{89}Zr$-Df-IAB22M2C, as outlined in Example 7-9. The results of the imaging of the patients are provided in FIG. 50-FIG. 54.

The results of the use of the noted minibody in the subjects confirmed that the construct has a number of additional superior properties. There were no drug-related adverse events or side effects reported. In addition, the results again confirmed that one can observe CD8 T cells using this construct. In addition, the signal achieved by the compositions and techniques allowed for striking visualization of the CD8 immune system in cancer patients by PET. In addition, the results demonstrated that same day imaging of tumor co-localization was possible.

The results also demonstrated a low background activity. This should provide for accurate interpretation in areas of known tumor (colocalization). For example, Patient 1 (Melanoma) demonstrated focal intramuscular uptake co-localizing with tumor. Patient 2 (Liver) demonstrated multifocal $^{89}Zr$ IAB22M2C up take colocalizing with known areas of tumor and potential radio-occult lesions. Patient 3 (Lung) showed no co-localization of $^{89}Zr$-IAB22M2C which could be related to lack of sufficient CD8 T-cell density within the tumor itself (as 30% of subjects treated with IOT do not respond). The results are consistent with the initial goals based upon pre-clinical work. The results highlight the effectiveness of $^{89}Zr$-IAB22M2C to address and monitor CD8 distribution in vivo for the methods provided herein, including for immunotherapy selection against cancer.

The results in Example 11 also indicate that the method can allow for stable imaging over multiple days. Thus,

in some embodiments, imaging is performed over 2, 3, 4 or more days.

Example 12

The safety, tolerability, pharmacokinetic (PK) properties and radiation dosimetry of 89Zr-Df-IAB22M2C have been investigated in 15 adult patients with selected solid malignancies in a 2 stage Phase I study. The study included a dose escalation stage with one subject each of increasing minibody protein dose levels (active pharmaceutical ingredient; API): 0.2 mg, 0.5 mg, 1.0 mg, 1.5 mg, 5.0 mg, and 10.0 mg, followed by a dose cohort expansion stage in which pharmacokinetics and dosimetry were further investigated in 9 subjects at 0.5 mg and 1.5 mg API. All patients received 3 mCi of $^{89}$Zr-Df-IAB22M2C.

All dose levels were found to be safe to use. No drug-related adverse events were reported in any of the 15 subjects. Blood samples collected from all subjects didn't show any evidence of immune reaction as measured by cytokine levels or anti-drug antibody measurements.

Dose-proportional trends in biodistribution and tracer elimination were observed, with slowest blood pool clearance in the 5 mg and 10 mg API doses, while rapid clearance from the blood pool within hours was seen at 1.5 mg or less of API dose. CD8 cell populated tissues such as spleen, bone marrow and lymph nodes were visualized at all dose ranges and demonstrated API dose-dependent trends in uptake. Liver and kidneys also showed significant but API dose-independent uptake, with primarily hepatic excretion of the tracer. Variable tracer uptake was seen in tumor lesions and metastatic (target) nodes, while consistently low background uptake across all API doses was seen in non-CD8 rich tissues such as muscle, lung and brain. All tissue activities were relatively similar between the expanded cohorts of API doses of 0.5 mg to 1.5 mg.

Dosimetry was performed on all 15 subjects with an effective radiation dose (ICRP 103) ranging from 1.7 to 2.7 rem/mCi, with a mean of 2.4 rem/mCi. No correlation was seen between the API dose administered and effective radiation dose.

Example 13

This example will further establish the degree of safety of two doses of 89Zr-Df-IAB22M2C, generate confirmation of efficacy and provide further evidence of clinical utility.

This is an Open Label, Multi-Dose Study of Positron Emission Tomography (PET/CT) with 89Zr-Df-IAB22M2C (CD8 Tracer) in Patients with Metastatic Solid Tumors. This study will evaluate the safety of repeat doses of 89Zr-Df-IAB22M2C; establish the relationship of 89Zr-Df-IAB22M2C PET/CT lesion positivity with CD8+ T cells infiltrating lymphocytes (TIL); assess 89Zr-Df-IAB22M2C uptake at Baseline and post-Treatment in tumor lesions and reference tissues, including T-cell rich tissues; measure potential change in 89Zr-Df-IAB22M2C uptake in tumor lesions between Baseline and post-Treatment and reference tissues, including T-cell rich tissues; measure potential change in CD8+ TIL in tumor lesions between Baseline and post-Treatment in biopsied samples; and assess biodistribution for 89Zr-Df-IAB22M2C on PETbaseline and PETTx.

The objectives that are confirmed with the study include: ☐ Evaluate safety of repeat doses of 89Zr-Df-IAB22M2C (CD8 PET Tracer)

☐ Establish the relationship of 89Zr-Df-IAB22M2C PET/CT lesion positivity with CD8+ T cells infiltrating lymphocytes (TIL)

☐ Assess 89Zr-Df-IAB22M2C uptake at Baseline and post-Treatment in tumor lesions and reference tissues, including T-cell rich tissues

☐ Measure potential change in 89Zr-Df-IAB22M2C uptake in tumor lesions between Baseline and post-Treatment and reference tissues, including T-cell rich tissues

☐ Measure potential change in CD8+ TIL in tumor lesions between Baseline and post-Treatment in biopsied samples

☐ Assess biodistribution for 89Zr-Df-IAB22M2C on PETbaseline and PETTx

☐ Explore visual and semi-quantitative 89Zr-Df-IAB22M2C PET measurements that best correlate with standard of truth.

☐ Estimate positive predictive value, negative predictive value, sensitivity and specificity of 89Zr-IAB22M2C PET for detecting CD8+ T cells as confirmed by IHC.

☐ Assess and measure potential change in 89Zr-Df-IAB22M2C distribution in tumor lesions and reference tissues between Baseline and 5-7 day post-Treatment.

☐ Evaluate the gene expression in subjects pre- and post-Treatment as measured in whole blood sample.

☐ Explore the visual and semi-quantitative 89Zr-Df-IAB22M2C PET measurements with clinical outcomes.

☐ Evaluate the visual and semi-quantitative 89Zr-Df-IAB22M2C PET measurements with RECIST 1.1 radiology responses on a subject basis and waterfall plot analysis of lesion diameter on a per lesion basis; and

☐ Evaluate the correlation of 89Zr-Df-IAB22M2C uptake with immune infiltrates and other molecular biomarkers (CD4, CD8, PD-1 and PD-L1) expression by IHC

**STUDY DESIGN of Example 13**

Eligible subjects who meet Inclusion/Exclusion criteria will receive 89Zr-Df-IAB22M2C as an IV infusion within 1 week prior to the onset of cancer treatment, and 4 to 5 weeks after start of cancer treatment. They will receive convention CT Chest, Abdomen and Pelvis (including Neck for SCCHN) or MRI and/or whole body 18FDG-PET scan will all be performed within 45 days prior to 1st infusion of 89Zr-Df-IAB22M2C. Fresh tumor biopsy (1 to 3 core biopsies) is performed at Baseline (-28 to -7 days prior to 1st infusion of 89Zr-Df-IAB22M2C, or after 89Zr-Df-IAB22M2C PET/CT scan and before start of cancer treatment), and within 0 to 2 weeks after 2nd infusion of 89Zr-Df-IAB22M2C and its associated PET/CT scan. Archival tumor biopsy tissues obtained within 3 months prior to 1st infusion of 89Zr-Df-IAB22M2C may be submitted in place of Baseline fresh biopsy. PET/CT scans (PETbaseline and PETTx) are obtained at 24±3 hours after each infusion of 89Zr-Df-IAB22M2C. Optional 89Zr-Df-IAB22M2C PET/CT is obtained within 5-7 days after start of cancer treatment. Anti-drug antibody (ADA) blood samples are collected at the following time points: at Baseline, prior to receiving the 2nd infusion of 89Zr-Df-IAB22M2C, and at End-of-Study safety follow-up visit.

The detailed study schedule and assessments in each visit are provided in the Table below. The general application schedule is shown in FIG. 55.

**Study Schedule of Events**

| | Baseline | | | On Treatment | | | | | End of Study | Follow Up |
|---|---|---|---|---|---|---|---|---|---|---|
| | Visit 1 | Visit 2 | Visit 3 | Visit 4 | Visit 5 | Visit 6 | Visit 7 | Visit 8 | Visit 9 | |
| Protocol Assessments | Screening (Day -28 to Day -1) | Day 1 | 24±3 hours after V2D1 | Start of Treatment (0-7 Days after V3) | Optional PET/CT (5-7 Days after V4) | 4-5 Weeks after V4 | 24±3 hours after V6 | 0-2 Weeks after V6 | 4-5 weeks after V8 | Up to 1 Year after Start of Treatment |
| Informed consent | X | | | | | | | | | |
| Medical history, demographics | X | | | | | | | | | |
| Eligibility Assessment | X | X | | | | | | | | |
| Physical examination including ECOG PS | X | | | | | X | | | X | |
| Vital signs | X | X | | | | X | | | X | |
| 12-lead ECG | | X | | | | X | | | | |
| CT/MRI and/or FDG-PET | X | | | | | | | | | |
| Safety Labs per SOC | X | | | | | X | | | X | |
| Urine Pregnancy Test (in women of child bearing potential) | X | | | | | X | | | X | Collect SOC Imaging Only<br>No additional study procedures |
| Blood sample for Anti-drug antibody (ADA) analysis | X | | | | | X | | | X | |
| Blood sample for Nanostring analysis | X | | | | | X | | | | |
| Fresh tumor biopsy | X | | | | | | | X | | |
| 89Zr-Df-IAB22M2C infusion | | X | | | | X | | | | |
| 89Zr-Df-IAB22M2C PET/CT Scan | | | X | | X | | X | | | |
| Administration of Cancer Treatment | | | | SOC | → | → | → | → | → | → |
| Adverse events | | X | X | | X | X | X | X | X | |
| Concomitant medications | X | X | | | X | X | | X | X | |

Notes:

1. To be assessed prior to and 15 (+/- 5) minutes post infusion of 89Zr-Df-IAB22M2C.
2. CT/MRI/FDG-PET performed up to 45 days prior to Visit 2 Day 1 is acceptable.
3. Can be performed on Visit 2 Day 1 prior to first infusion of 89Zr-Df-IAB22M2C instead of Visit 1 Screening, provided that the lab results will be available to investigator for safety assessment before infusion.
4. Blood sample collection for ADA and Nanostring can be collected on Visit 2 Day 1 as prior to infusion.
5. Baseline fresh tumor biopsy to occur between -28 to -7 days prior to first infusion of 89Zr-Df-IAB22M2C, or after the 1st 89Zr-Df-IAB22M2C PET/CT scan and before start of cancer treatment. If the patient has had a diagnostic/tumor staging biopsy (excluding skin biopsy for melanoma patients) done within 3 months prior to first infusion of 89Zr-Df-IAB22M2C, archival tissues/slides are acceptable, and the patient is exempt from Baseline fresh tumor biopsy.
6. To be performed prior to infusion of 89Zr-Df-IAB22M2C. Safety lab results to be available to investigator for safety assessment before infusion.
7. Second tumor biopsy may be performed on the same day of 2nd PET imaging post 89Zr-Df-IAB22M2C infusion.
8. For early discontinued patients, End-of-Study Visit to occur 4 to 5 weeks after the last infusion of 89Zr-Df-IAB22M2C.
9. To be performed if the subjects come in for optional Visit 5.

Approximately 40 subjects are enrolled in this study from up to 20 Centers within the USA, Canada and Europe. Subjects are eligible for enrollment if they meet the following criteria:

1. Patients with metastatic solid tumors.

2. At least 1 measurable lesion documented on CT/MRI (RECIST criteria 1.1) within 45 days prior to first 89Zr-Df-IAB22M2C infusion.

3. At least 1 lesion that is accessible per investigator's assessment and eligible for biopsy. If only a single RECIST measurable lesion is present, investigator to determine if the tumor biopsy could interfere with RECIST assessments of response.

4. Eastern Cooperative Oncology Group (ECOG) performance status ≤ 2.

5. Meeting all clinical safety lab values per institution's standard of care, or Investigator's discretion, for patients receiving cancer treatment.

6. Age ≥ 18 years.

Subjects are eligible for enrollment in the study only if they meet none of the following criteria:

1. Serious nonmalignant disease or conditions that in the opinion of the investigator and/or ImaginAb could compromise protocol objectives.

2. Patients with a single RECIST measurable lesion, biopsy of which, per investigator's assessment, is likely to interfere with RECIST assessments of response.

3. Patients who have any splenic disorders, or had splenectomy, that in the opinion of the investigator and/or ImaginAb could compromise protocol objectives.

4. Pregnant women or nursing mothers.

**DOSING INFORMATION**

89Zr-Df-IAB22M2C is an ~80 kDa minibody (Mb) molecular weight with high affinity to CD8 glycoprotein (binding EC50 = 0.4nM) that is conjugated with desferrioxamine (Df) and radiolabeled with 89Zr for imaging CD8+ T cells in humans. 3.0 (±20%) mCi of 89Zr-Df-IAB22M2C between 0.5mg to 1.5mg of API is administered intravenously over 5-10 minutes, within one week prior to the onset of cancer treatment, and 4 to 5 weeks after start of IOT. The patient will undergo at least two PET/CT scans (imaging from top of skull through the feet).

The dispensed dose is infused as an IV infusion with an infusion pump over no less than 5 minutes followed by saline flush. The total infusion time should be between 5 to 10 minutes. Vital signs are assessed prior to and 15 (± 5 minutes) minutes post infusion of 89Zr-Df-IAB22M2C.

**TUMOR BIOPSY**

Fresh tumor biopsy from a RECIST measurable lesion (Excluding cutaneous lesions) within -28 to -7 days prior to 1st infusion or after the 1st 89Zr-Df-IAB22M2C PET/CT scan and before start of cancer treatment, and 0-2 weeks after 2nd infusion of 89Zr-Df-IAB22M2C is performed in accordance with the instructions contained within the central laboratory biopsy manual.

**PET/CT SCANNER VALIDATION**

In an effort to generate most accurate and consistent imaging data across all participating investigative sites to support the data collection and analysis for this study, a nuclear medicine organization (Nuclear Medicine Society of Clinical Research, or NMSCR) will conduct the validation of PET/CT scanners at each participating site that is used in this trial.

The NMSCR will provide a phantom (dimensions: 16"W x 14"L x 7" H) and a dose of 89Zr to sites to perform validation on the PET scanner(s) intended for this trial with detailed instructions how to carry out the phantom imaging.

Once the validation is completed, site will submit the data to NMSCR for analysis.

Upon completion of the PET/CT scanner validation, the NMSCR will provide a validation certificate and the setting parameters specific to the validated scanner before sites can start imaging their first enrolled subject onto this trial. It is required that sites to use the validated PET/CT scanner with the setting parameters generated from the phantom-validated procedure to scan subjects enrolled in this trial as per protocol.

## PET/CT IMAGING

Whole body scans (WBS) from the vertex of the skull to through mid-thighs including additional areas of known or suspected disease are obtained using acquisition settings specific for 89Zr imaging.

The results of Example 13 will provide one or more of the following:

The safety and tolerability of repeat doses of 89Zr-Df-IAB22M2C are assessed by local and systemic signs and symptoms of infusion reactions, incidence of adverse events per CTCAE criteria, changes in laboratory test results, vital signs and 12-lead electrocardiogram (ECG) findings.

Analyze 89Zr-Df-IAB22M2C uptake in biopsied tumors as determined by SUV-based quantitative measures (e.g. SUVmax, SUVpeak, SUVmean, CD8 tumor volume defined as volume of tumor tissues with increased CD8 uptake, and Tumor: Reference ratio) with CD8+ TIL density determined by IHC from biopsy samples.

Assessment of Baseline and post-Treatment 89Zr-Df-IAB22M2C uptake and distribution in tumors, and measurement of any change between the paired observations, as determined by:

o SUV-based quantitative analysis (e.g. SUVmax, SUVpeak, SUVmean, 89Zr-Df-IAB22M2C volume, total CD8 index as defined by SUVmean x Tumor Volume)

o Intratumoral uptake pattern (heterogeneity) analysis - visual and software-based

Assessment of Baseline and post-Treatment 89Zr-Df-IAB22M2C uptake and distribution in lymphoid organs including involved and systemic lymph nodes, and measurement of any change between the paired observations, as determined by:

◦ Visual and SUV-based quantitative analysis (e.g. SUVmax, SUVpeak, SUVmean, 89Zr-Df-IAB22M2C nodal uptake volume, total CD8 index as defined by SUVmean x nodal Volume).

◦ Lymph node chain visibility defined as location and number of nodal chains identified with elevated 89Zr-Df-IAB22M2C activity

◦ Pattern(s) of changes in uptake within lymphoid tissue including spleen and lymph nodes, other T-cell rich tissues such as bone marrow and other reference organs.

Measurement of change in 89Zr-Df-IAB22M2C uptake in biopsied tumors as determined by SUV-based quantitative analysis (e.g. SUVmax, SUVpeak, SUVmean, 89Zr-Df-IAB22M2C volume, tissue modeling/compartmental analysis and Tumor: Reference ratio) from Baseline to post-Treatment PET scans and correlation with standard of truth as measured by change in CD8+ TIL density determined by IHC from biopsy samples obtained prior to and 4 to 7 weeks after the start of cancer treatment.

Description of biodistribution patterns of 89Zr-Df-IAB22M2C on PETbaseline and PETIOT and any changes in biodistribution between baseline and post-Treatment.

Evaluation of the gene expression profiles in subjects prior to and post-Treatment as measured by whole blood Nanostring analysis.

Correlation of 89Zr-Df-IAB22M2C uptake with radiologic responses based upon:

o Correlation of subject response rate compared by RECIST 1.1 compared to visual and semi- quantitative SUV-based analysis

o Correlation of target lesion response rate as determined by change in lesion diameter following cancer therapy using waterfall plot analysis with to semi quantitative SUV-based analysis at baseline and post cancer therapy

Correlation of 89Zr-Df-IAB22M2C uptake with clinical outcomes (response rates, duration of response, disease stability rate at 12 weeks and PFS at defined intervals as determined.

Correlation of 89Zr-Df-IAB22M2C tumor and lymph node uptake with immune infiltrates and other molecular

biomarker (CD8) expression by IHC

Assessment of changes in 89Zr-Df-IAB22M2C uptake and distribution from baseline to 5-7 day post start of cancer therapy.

Additional details regarding Example 13 are provided in the following paragraphs: The dose is delivered by a central manufacturing facility to the investigational site's radiopharmacy in the Nuclear Medicine Department. Upon receipt, qualified personnel will ensure that the study drug is delivered in good condition, inventoried, stored properly, labeled and dispensed in compliance with the pharmacy manual, all regulatory agencies and per the investigator physician's prescription order.

The 89Zr dose is measured by the qualified personnel in a dose calibrator prior to dispensing. Then the syringe is placed in a shielded carrier along with a designated IV infusion pump for radioactive infusion and administration. After the dose administration, the qualified personnel will return the syringe for residual measurement by the nuclear pharmacist at the site. Measured radioactivity values and times of measurement are documented, as well as the total injected volume.

Acquisition and reconstruction parameters should be obtained according to the recommendation provided to each site based upon phantom validation of the scanner and should be obtained in List Mode, whenever possible, to allow for virtual radioactive dose reconstruction at a later date. Whole body scans (WBS) from the vertex of the skull to through mid-thighs including additional areas of known or suspected disease should be obtained at every scan visit using acquisition settings optimized for 89Zr imaging.

## Example 14

The present example provides additional guidance regarding the details of dosing and administration of some embodiments provided herein, including Examples 7-9 and 11.

### DOSING ADMINISTRATION

89Zr-Df-IAB22M2C is an ~80 kDa minibody (Mb) molecular weight with high affinity to CD8 glycoprotein (binding EC50 = 0.4nM) that is conjugated with desferrioxamine (Df) and radiolabeled with 89Zr for imaging CD8+ T cells in humans. 89Zr-Df-IAB22M2C is manufactured by conjugating IAB22M2C with desferrioxamine (Df) and subsequently radiolabeling with 89Zr to produce the PET imaging agent.

A single dose of 3.0 (±20%) mCi of 89Zr-Df-IAB22M2C was injected for imaging at Visit 2 (Day 1). The patient underwent five PET/CT scans (imaging from top of skull through the feet). A patient-specific calibration phantom was placed alongside the lower legs for each PET/CT scan.

☐ Fasting: There was no fasting required prior to 89Zr-Df-IAB22M2C infusion or PET/CT imaging.

☐ IV catheter insertion for the 89Zr-Df-IAB22M2C

☐ PET Scan: If feasible, 2 intravenous (IV) catheters (heplock, 20 to 24 gauge) was placed for radiopharmaceutical administration and blood sampling, or alternatively a central line was used for infusion or sampling. The IV catheter was removed at the conclusion of the imaging session for the day. If 2 lines are not feasible, 1 line is used for both infusion and PK sampling with an intervening 10 cc saline flush between infusion and PK sampling.

### 89Zr-Df-IAB22M2C Preparation for PET/CT Scan

89Zr-Df-IAB22M2C drug product was prepared at the central manufacturing facility using a process in which approximately 20 - 25 mg of IAB22M2C was conjugated with desferrioxamine, and a fraction of the conjugate (approx. 1 mg) was immediately radiolabeled with 89Zr to achieve the specific activity of 6 mCi/mg. The 3 mCi patient unit dose was prepared and dispensed under the supervision of a licensed nuclear pharmacist. The dose was dispensed after the aseptic addition of IAB22M2C, yielded an overall total protein dose of 0.2mg, 0.5mg, 1.0mg, 1.5mg, 5.0mg and 10.0mg for dose escalation.

Each single dose was radiolabeled at the central manufacturing facility for single IV administration to the specific subject for whom the dose was prepared. For all drug products, a system of medication numbering in accordance with all requirements of cGMP was used. This ensured that for each subject, any dose of 89Zr-Df-IAB22M2C can be identified and traced back to the production run. The prepared 89Zr-Df-IAB22M2C tracer was be kept in a secure location at the central manufacturing facility at ambient temperature, shielded and shipped at 2-8°C until delivery to the radiopharmacy for dispensing for infusion.

### Radiopharmacy Dispensation of 89Zr-Df-IAB22M2C

The dose was delivered by the central manufacturing facility to the investigational site's radiopharmacy in the Nuclear Medicine Department. Upon receipt, the radiopharmacist ensured that the study drug was delivered in good condition, inventoried, stored properly, labeled and dispensed in compliance with all regulatory agencies and per the investigator physician's prescription order. An additional syringe-vial containing a small amount of 89Zr-Df-IAB22M2C (50 ± 5 microcuries in 10 cc saline) was provided by the central manufacturing facility to the radiopharmacy to serve as a patient specific calibration phantom.

The 89Zr dose was measured by the central manufacturing facility nuclear pharmacist in a dose calibrator prior to dispensing. Then the syringe was placed in a shielded carrier along with a designated IV infusion pump for radioactive infusion and administration. After the dose administration, the treating physician returned the syringe for residual measurement by the central manufacturing facility nuclear pharmacist.

The additional syringe-vial with a small amount of 89Zr-Df-IAB22M2C (50 microcuries in 10 cc saline) served as a patient specific calibration phantom that was retrieved from the site's radiopharmacy at each imaging session. This vial was positioned in the scanner during patient image acquisition at each imaging time window. The vial was stored between imaging visits in an appropriate shielded container when not in use according to the policy and procedures of the institution.

### Infusion of 89Zr-Df-IAB22M2C & Intervention

The dispensed dose was infused under the direct supervision of a nuclear medicine physician as an IV infusion with an infusion pump over no less than 5 minutes followed by saline flush. The first patient enrolled in each cohort was monitored for any possible AEs for 2 hours after the infusion for any clinically significant change in vital signs from pre-infusion to post-infusion that results in a change in subject management. This monitoring included:

☐ Vital signs pre-infusion and every 10 minutes out to 60 minutes.

☐ Vital sign every 15 minutes (±5 minutes) for another hour.

If the first subject in a cohort does not experience any infusion related AEs, then subsequent subjects' vital signs was monitored for 1 hour as follows:

☐ Vital signs pre-infusion and every 10 minutes out to 60 minutes

If an AE was detected within a cohort revert to the 2-hour monitoring period. Longer monitoring times were performed at the discretion of the attending physician. Signs and symptoms of a severe infusion reaction included urticaria, hypotension, angioedema, hypoxia, bronchospasm, pulmonary infiltrates, acute respiratory distress syndrome, myocardial infarction, ventricular fibrillation, and/or cardiogenic shock. For less severe or minor infusion reactions (signs or symptoms without cardiopulmonary compromise), temporarily reduced rate of infusion or halting of the infusion until symptoms improved was done, then continued infusion at a slower rate.

### Example 15

The present example provides details on an imaging schedule and imaging parameter. This approach was used in Examples 7-9 and 11.

### IMAGING SCHEDULE

Imaging for the 2015-22M2C-01 protocol should be acquired according to the protocol imaging

schedule, which is summarized in the Table below, and using the parameters for image acquisition provided below.

| Visit | Schedule | Imaging |
|---|---|---|
| **Historical Scan:** Conventional Imaging[1] | Within the 28 days prior to C1D1 of checkpoint inhibitor therapy | CT scan of chest, abdomen, pelvis, and any areas of relevant anatomy of known or suspected disease |
| **Visit 2:** Infusion + PET/CT scans | 1-2 hours following $^{89}Zr$-Df-IAB22M2C infusion | Whole body PET/CT |
| | 6-8 hours following $^{89}Zr$-Df-IAB22M2C infusion (optional) | |
| **Visit 3:** PET/CT scan | 24 hours following $^{89}Zr$-Df-IAB22M2C infusion (±4 hours) | |
| **Visit 4:** PET/CT scan | 48 hours following $^{89}Zr$-Df-IAB22M2C infusion (±4 hours) | |
| **Visit 5:** PET/CT scan | 92-144 hours following $^{89}Zr$-Df-IAB22M2C infusion | |

The parameters for image acquisition for the ImaginAb 2015-22M2C-01 are provided within the listing that follows.

**89Zr-Df-IAB22M2C PET (Whole Body)**

**Radiopharmaceutical**

89Zr-Df-IAB22M2C
89Zr has a positron Emean of 398 KeV and Emax of 897 KeV, values that are substantially different from 18F. Some scanners may have an option for selecting the 89Zr isotope for image acquisition. Others may need to have 89Zr settings manually entered. Relevant input parameters for 89Zr imaging include T 1/2 = 3.3 days (78.41 hr or 4705 min or 282,276 secs) and a positron abundance fraction of 0.227(22.7%).

**Dose**

3 (±20%) mCi of 89Zr-Df-IAB22M2C (0.2 to 1.5 mg of protein)

Dose should be consistent across timepoints. For follow-up scans the radiotracer dose should be ± 10% from the net baseline dose, unless there is a drastic patient weight change.

**Mode**

2D, 3D list-mode, or TOF
When possible, acquisition is conducted in List Mode.

**Patient Position**

Supine with arms down by side
Position should be consistent between CT and PET acquisitions.

**Scan Location /Coverage**

Melanoma Subjects: Skull vertex through feet and including upper extremities
Non-Melanoma Subjects: Skull vertex to knees or area of interest ensuring consistent scan coverage for a subject throughout the trial.

**Transmission Scan Guidelines**

- 140 kVp
- mAs
    - 1-2 hour post infusion scan: Low dose CT (80 mAs)
    - 6-8 hour (optional), 24 hour, 48 hour, and 92-144 hour post infusion scans: Low dose CT (10 mAs)
- 0.8 sec per CT rotation
- 3 - 5 mm slice thickness
- Table speed of 15 mm/rotation
- Pitch: 1.5:1

**Emission Scan**

2-7 min/bed position

Depending on the height of the subject, a single bed position may provide adequate anatomic coverage.

**Image Reconstruction**

Reconstruction algorithm should be consistent across timepoints.
Quantitative iterative reconstruction preferred.
Corrections for normalization, dead time, random events, scatter, attenuation and sensitivity

**Matrix**

128 x 128 or better

**Views**

Non-attenuation corrected
Attenuation corrected

**Patient prep instructions**

Have subject empty bladder prior to imaging

**Additional Instructions**

DICOM requirements can be observed or followed.
Each location should prepare a small sample vial (container) of 89Zr-Df-IAB22M2C which should be placed at the patient's ankles, or at the bottom of the planned scanning field of view (e.g. thighs or knees), during scanning to serve as a reference for bio dosimetry analysis. The purpose of the sample vial is to have an external source of known amount of 89Zr-activity for scanner count calibration needed for biodosimetry estimates. One practice for preparation

and storage of this sample vial is as follows:

1) Remove 0.15 cc of 89Zr-Df-IAB22M2C from the provided vial after infusion, using a tuberculin syringe.
2) Place in dose calibrator and record measurement and the time of measurement. Report these values to IE within the comments section of the Data Transmittal Form (see Appendix VI)
3) Dilute to 5 cc normal saline in a 10 cc syringe
4) Double bag the syringe and seal to prevent any leakage
5) Place the sample vial near ankles of the patient at each scan or at the bottom of the planned scanning field of view (e.g. mid thighs or knees if below the thigh imaging is not performed).
6) Store in refrigerator with proper shielding in between scannings and dispose after final scan for patient.

**Example 16 - Compositions for diagnosis and/or treatment**

This Example outlines the various compositions that can be used in any of the following examples.

A diagnostic composition is manufactured by a method comprising conjugating a minibody to desferrioxamine to form a Df-minibody. The Df-minibody is radiolabeled with $^{89}Zr$ to form radiolabeled minibody. The radiolabeled minibody is purified. The radiolabeled minibody is mixed with a cold minibody to form a diagnostic composition. The minibody and the cold minibody bind to a same epitope on CD8.

A diagnostic composition comprises 3.0±20% mCi of a 89Zr-Df-labeled minibody, 20 mM Histidine, 5% sucrose, 51-62 mM Sodium Chloride, 141-194 Arginine, and 2-20 mM Glutamic acid.

A diagnostic composition comprises a labeled minibody or antigen binding construct comprising the structure of:

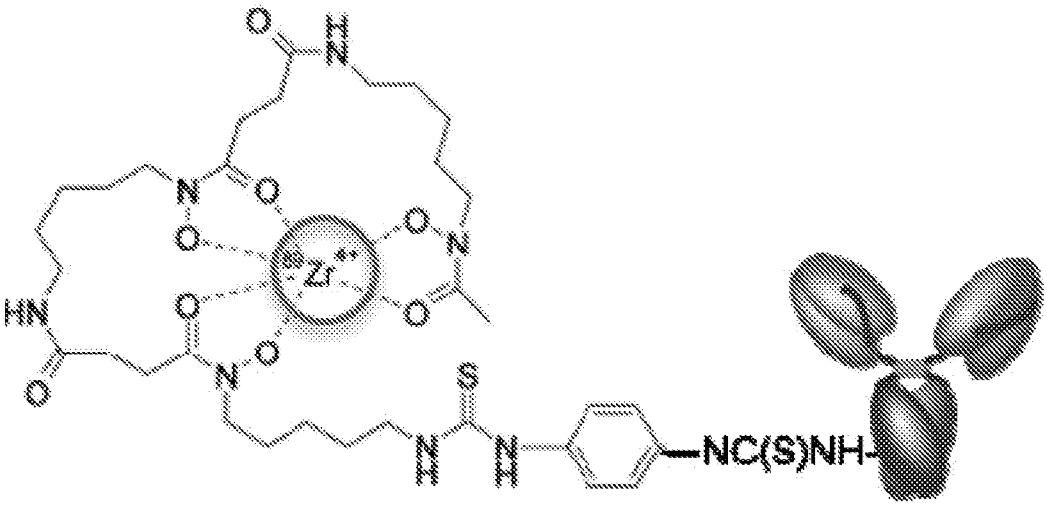

or

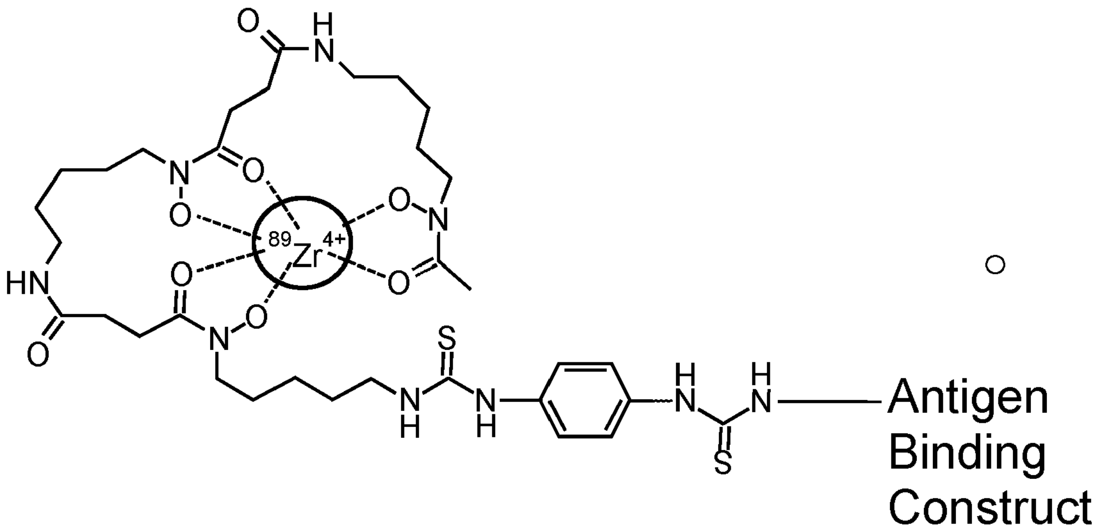

. The minbody comprises a heavy chain variable region of SEQ ID NO:1, 3, 16, or within 147 and a light chain variable region of SEQ ID NO: 7, 9, 16, or within 147. The composition provides at least 3 mCi of radiation.

A composition comprises a first minibody that binds to CD8 that comprises an active marker, and a second minibody that binds to CD8. The second minbody comprises an inactive marker or lacks an active marker.

A diagnostic composition comprises a first CD8 minibody that is conjugated to a radiolabel, and a second CD8 minibody that is not conjugated to the radiolabel. The first and second CD8 minibodies have a same sequence. The composition provides about 3 mCi of radiation. The composition provides 1.5-10 mg of total mass protein.

A composition comprises $^{89}$Zr-labeled CD8 antigen binding construct, 20 mM Histidine, 5% sucrose, 51-62 mM Sodium Chloride, Arginine, and 2-20 M Glutamic acid. The composition is configured for administration to a human subject as a diagnostic.

A composition comprises a first portion that comprises a CD8 antigen binding construct having an active marker, and a second portion that comprises a CD8 antigen binding construct having an inactive marker or lacks an active marker.

A diagnostic composition comprises a first CD8 antigen binding construct that is bound to a radiolabel, and a second CD8 antigen binding construct that is not bound to a radiolabel. The first and second CD8 antigen binding constructs have the same amino acid sequence and are present in a molar ratio of 1:1 or in the alternative of 1:50 . The composition provides about 0.5 mCi to about 3.6 mCi of radiation. The composition provides 0.1 mg or in the alternative 10 mg of total mass protein.

**Example 17 - In vivo CD8 monitoring**

One of the compositions from Example 16 is selected for use in the present example

CD8 is monitored in vivo by a method by providing a CD8 minibody to a subject, wherein the CD8 minibody binds to a CD8 as shown in FIG. 1C, the minibody being labeled with a detectable marker. A distribution of the CD8 minibody is monitored in the subject within 6 or in the alternative within 36 hours of administering the CD8 minibody to the subject. This can be use for the treatment and/or diagnosis of the subject.

**Example 18- In vivo CD8 monitoring**

One of the compositions from Example 16 is selected for use in the present example.

CD8 is monitored in vivo by a method by providing a CD8 minibody to a subject, wherein the CD8 minibody binds to aCD8 as shown in FIG. 1C, the minibody being labeled with a detectable marker. A distribution of the CD8 minibody is monitored in the subject. The monitoring can detect a tissue infiltrated with 500 or less CD8 bearing cells per $mm^2$ within the subject. The monitoring is achieved via PET. This can be use for the treatment and/or diagnosis of the subject.

**Example 19 - Vsulaization of CD8 positive cells**

One of the compositions from Example 16 is selected for use in the present example.

CD8 positive cells are visualized by a method by providing a CD8 minibody to a subject, the CD8 minibody being labeled with a detectable marker, and the CD8 minibody is humanized in sequence and binds to human CD8. A

distribution of the CD8 minibody is monitored in the subject within 6-36 hours of administering the CD8 minibody to the subject. The monitoring can detect a tissue infiltrated with 500 or less CD8 positive cells per $mm^2$ within the subject. The monitoring is achieved via PET. This can be use for the treatment and/or diagnosis of the subject.

## Example 20 - Visualization of cells in a human

One of the compositions from Example 16 is selected for use in the present example.

Cells in a human are visualized by a method by providing a means of binding human CD8 positive cells to a subject, the means comprising a detectable label. A first distribution of the means of binding human CD8 positive cells is monitored and determined in the subject within 6-36 hours of administering the means of binding human CD8 positive cells to the subject. This can be use for the treatment and/or diagnosis of the subject.

## Example 21 - Minibody administration method

One of the compositions from Example 16 is selected for use in the present example.

A minibody is administered to a subject by a method by providing to a subject a labeled minibody that binds to human CD8. 3 mCi ±20% of radiation is provided to the subject via the labeled minibody. An amount between 0.2 and 10mg of total protein is provided to the subject. This can be use for the treatment and/or diagnosis of the subject.

## Example 22 - Minibody administration method

One of the compositions from Example 16 is selected for use in the present example.

A minibody is administered to a subject by a method by providing to a subject a labeled minibody that binds to human CD8. 0.75-1.5 +/- 20% mCi of radiation is provided to the subject via the labeled minibody. An amount between 0.2 and 10mg of total protein is provided to the subject. This can be use for the treatment and/or diagnosis of the subject.

## Example 23 - Method of treatment

One of the compositions from Example 16 is selected for use in the present example.

A patient is treated by a method by administering to a human patient diagnosed with a cancer a dose of an antigen-binding construct that binds to human CD8. The dose comprises a $^{89}Zr$-labeled antigen-binding construct providing a radiation activity of about 3 mCi, and about 10 mg or less of the antigen-binding construct. The $^{89}Zr$-labeled antigen-binding construct is detected in the patient at a first time point after administering the dose, to generate a first patient image corresponding to the first time point, wherein the first time point is about 6 hours to 36 hours after administering, a first abundance and/or distribution of CD8 cells is determined in one or more tissues and/or neoplasia in the patient based on the first patient image, and the patient is administered a first treatment for the cancer based on the first abundance and/or distribution of CD8 cells in the one or more tissues. This can be use for the treatment and/or diagnosis of the subject.

## Example 24 - Tumor treatment method

One of the compositions from Example 16 is selected for use in the present example.

A tumor is treated by a method by administering a minibody to a subject such that the minibody binds to a tumor within the subject to form a labeled tumor. The minibody binds to human CD8, and the minibody is linked to a detectable label. If the minibody binds in a biased manner to a surface of any tumor in forming the labeled tumor, a treatment is administered selected from the group consisting of an immune checkpoint inhibitor. If the minibody binds throughout all tumors in forming the labeled tumor, then an immune checkpoint inhibitor is not administered. This can be use for the treatment and/or diagnosis of the subject.

## Example 25- Neoplasia treatment method

One of the compositions from Example 16 is selected for use in the present example.

A subject is treated by a method by administering to a patient that has a neoplasia a human minibody that binds to human CD8. A distribution of the human minibody is monitored to determine a first tumor-infiltrating lymphocyte ("TIL") status within the neoplasia, and the patient is treated based upon at least the first TIL status of the neoplasia. This can be use for the treatment and/or diagnosis of the subject.

**Example 26 - CD8 distribution analysis**

One of the compositions from Example 16 is selected for use in the present example.

CD8 distribution in a subject is analyzed by a method by providing an image of a distribution of a detectable marker via a first PET image. The detectable marker is linked to a CD8 minibody, the CD8 minibody binds to human CD8, and the CD8 minibody does not provoke an immune response in the subject. An image is provided of a distribution of a FDG marker via a second PET image of the subject. A third PET image is created that comprises an overlay of the first PET image onto the second PET image. A tumor is identified as TIL, based upon the third PET image. This can be use for the treatment and/or diagnosis of the subject.

**Example 27 - Neoplasia treatment method**

One of the compositions from Example 16 is selected for use in the present example.

A subject is treated by a method by administering to a patient that has a neoplasia an antigen-binding construct that binds to human CD8. A distribution of the antigen-binding construct is monitored to determine the tumor-infiltrating lymphocyte ("TIL") status within the neoplasia. An immunotherapy ("IOT") is adminsiteed to the patient if the TIL status of at least one neoplasia is negative so as to convert the TIL status from negative to positive. This can be use for the treatment and/or diagnosis of the subject.

**Example 28 - Neoplasia treatment method**

One of the compositions from Example 16 is selected for use in the present example.

A subject is treated by a method by administering to a patient that has a neoplasia an antigen-binding construct that binds to human CD8. A distribution is monitored of the antigen-binding construct to determine the tumor-infiltrating lymphocyte ("TIL") status within the neoplasia. An alternative immunotherapy ("IOT") is administered to the patient if the TIL status of the neoplasia is not positive, until the TIL status of the neoplasia becomes positive. This can be use for the treatment and/or diagnosis of the subject.

**Example 29 - Characterization of tumor**

One of the compositions from Example 16 is selected for use in the present example.

A tumor is characterized by a method comprising applying an antigen-binding construct that binds to human CD8 to a human subject. The antigen-binding construct comprises a detectable marker. A distribution is monitored of the detectable marker. An image is generated based on the distribution of the detectable marker. The image is displayed to a user in a manner to allow the user to characterize the tumor. This can be use for the treatment and/or diagnosis of the subject.

**Example 30 - Image generation method**

One of the compositions from Example 16 is selected for use in the present example.

An image is generated by a method comprising applying an antigen-binding construct that binds to CD8. The antigen-binding construct comprises a detectable marker. At least one detectable marker is detected within a location within the tumor and assigning a positive pixel for each detectable marker detected. An image is generated based on a distribution of each positive pixel assigned, wherein the image is stored in a non-transitory computer readable media. This can be use for the treatment and/or diagnosis of the subject.

**Example 31 - PET method**

One of the compositions from Example 16 is selected for use in the present example.

Positron emission tomography ("PET") is performed by a method comprising administering a tracer that binds to CD8 to a subject. A scintillator is provided. The scintillator is used to detect a pair of photons created by the tracer. The detection of the pair of photons is used to localize a source of the tracer via a list of coincidence events that are processed via a processor that is configured to take an output from the scintillator and convert it to the list of coincidence events. Between about 300 and about 500 CD8 positive cells are detected per $mm^2$ of a tissue or neoplasia within the subject. This can be use for the treatment and/or diagnosis of the subject.

**Example 32 - Determinign standard uptake value (SUV)**

One of the compositions from Example 16 is selected for use in the present example.

A standard uptake value (SUV) is determined by a method by applying an antigen binding construct to a subject. The antigen binding construct comprises a radioactive probe. An r is determined, where r is the radioactivity concentration (kBq/ml) measured by a PET scanner within a ROI of radiation from the antigen binding construct. An a' is determined, where a' is the decay-corrected amount of the injected radiolableeld tracer (kBq). A w is determined, wherei w is the weight of the patient. An SUV is detemiend, where SUV is the result of r(a'/W). This can be use for the treatment and/or diagnosis of the subject.

**Example 33 - Image analysis method**

One of the compositions from Example 16 is selected for use in the present example.

An image is analyzed by a method. An image is provided. On the image is defined a first region of interest (ROI) by marking the image. A signal intensity is determined for a data point within the first ROI. A maximum signal intensity is determined within the first ROI. A mean value is determined of the signal intensities within the first ROI. Each signal intensity is summed within the first ROI to obtain a first summed signal level for the first ROI. The first ROI represents data for an amount of a detectable marker associated with an antigen binding construct that has been administered to a subject. This can be use for the treatment and/or diagnosis of the subject.

**Example 34 - Cancer treatment method**

One of the compositions from Example 16 is selected for use in the present example.

A human subject having cancer is treated by a method. In response to providing a first image of a tumor in the subject using a CD8 antigen binding construct, a therapy is administered including a candidate therapeutic to the subject. After administering the therapy including the candidate therapeutic, a second image is provided of the tumor in the subject using the CD8 antigen binding construct. The first and second images are compared to determine if the tumor demonstrates increased CD8 infiltration or the tumor demonstrates the same or decreased CD8 infiltration. If the tumor demonstrates increased CD8 infiltration, the subject is instructed to continue the therapy. This can be use for the treatment and/or diagnosis of the subject.

**Example 35 - Cancer treatment method**

One of the compositions from Example 16 is selected for use in the present example.

A human subject having cancer is treated by method providing a first image of a CD8 cells within a tumor in a subject using a CD8 antigen binding construct. A therapy is administered including a candidate therapeutic to the subject. After administering the therapy including the candidate therapeutic, a second image is provided of the CD8 cells within the tumor in the subject using the CD8 antigen binding construct. The first and second images are compared to determine if the tumor demonstrates increased CD8 infiltration or the tumor demonstrates the same or decreased CD8 infiltration. If the tumor demonstrates increased CD8 infiltration, then the subject is instructed to continue the therapy. This can be use for the treatment and/or diagnosis of the subject.

**Example 36 - Cancer treatment method**

One of the compositions from Example 16 is selected for use in the present example.

A human subject having cancer is treated by a the method by administering a CD8 antigen binding construct to the subject. A distribution is monitored of the CD8 antigen binding construct within the subject. An immunotherapy treatment is discontinued if at least one tumor within the subject is TIL negative per the distribution of the CD8 antigen binding construct. This can be use for the treatment and/or diagnosis of the subject.

**Example 37 - Cancer treatment method**

One of the compositions from Example 16 is selected for use in the present example.

A human subject having cancer is treated by a method by administering a CD8 antigen binding construct to the subject monitoring a distribution of the CD8 antigen binding construct within the subject. An immunotherapy treatment is continued if at least one tumor within the subject is TIL positive per the distribution of the CD8 antigen binding construct. This can be use for the treatment and/or diagnosis of the subject.

**Example 38 - Cancer treatment method**

One of the compositions from Example 16 is selected for use in the present example.

A human subject having cancer is treated by a method by administering an immunotherapy to the subject. Then a CD8 antigen binding construct is adminsitered to the subject. A distribution is monitored of the CD8 antigen binding construct within the subject. The immunotherapy is discontinued if all of the tumors within the subject is TIL negative per the distribution of the CD8 antigen binding construct. This can be use for the treatment and/or diagnosis of the subject.

**Example 39 - Cancer treatment method**

One of the compositions from Example 16 is selected for use in the present example.

A human subject having cancer is treated by a method. In a subject previously untreated with an immunotherapy, a CD8 antigen binding construct is administed to the subject. A distribution is monitored of the CD8 antigen binding construct within the subject. An immunotherapy is administered if at least one tumor within the subject is TIL negative per the distribution of the CD8 antigen binding construct. This can be use for the treatment and/or diagnosis of the subject.

**Example 40 - Cancer treatment method**

One of the compositions from Example 16 is selected for use in the present example.

A human subject having cancer is treated. In a subject previously untreated with an immunotherapy, a CD8 antigen binding construct is administered to the subject. A distribution is monitored of the CD8 antigen binding construct within the subject. The subject is advised against administering of an immunotherapy if all tumors within the subject are TIL positive per the distribution of the CD8 antigen binding construct. This can be use for the treatment and/or diagnosis of the subject.

**Example 41 - Cancer treatment method**

One of the compositions from Example 16 is selected for use in the present example.

A human subject having cancer is treated. In a subject previously treated with an immunotherapy, a CD8 antigen binding construct is administered to the subject. A distribution of the CD8 antigen binding construct is monitored within the subject. An alternative immunotherapy is administered if at least one tumor within the subject is TIL negative per the distribution of the CD8 antigen binding construct. This can be use for the treatment and/or diagnosis of the subject.

**Example 42 - Cancer treatment method**

One of the compositions from Example 16 is selected for use in the present example.

A human subject having cancer is treated. In a subject previously treated with an immunotherapy, a CD8 antigen binding construct is administered to the subject. A distribution of the CD8 antigen binding construct is monitored within the subject. The immunotherapy is administered if all tumors within the subject are TIL positive per the distribution of the CD8 antigen binding construct. This can be use for the treatment and/or diagnosis of the subject.

**Example 43 - Tumor treatment method**

One of the compositions from Example 16 is selected for use in the present example.

A tumor in a human subject is treated by a method by administering a CD8 antigen binding construct to the subject such that the CD8 antigen binding construct binds to CD8 cells within a tumor within the subject to form a labeled tumor. The CD8 antigen binding construct binds to human CD8. The CD8 antigen binding construct is linked to a detectable label. A distribution of the CD8 antigen binding construct is detected in or around the tumor. If the CD8 antigen binding construct binds in a biased manner at a surface of any tumor, then a treatment selected from the group consisting of an Immunotherapy is administered. If the CD8 antigen binding construct binds in a non-biased manner and throughout all tumors, then an immune checkpoint inhibitor is not administered. If the CD8 antigen binding construct binds in a non-biased manner but is absent from the interior of the tumor, then an Immunotherapy is administered. This can be use for the treatment and/or diagnosis of the subject.

**Example 44 - Treatment method**

One of the compositions from Example 16 is selected for use in the present example.

A tumor in a human subject is treated by a method by administering to a human subject a CD8 antigen binding

construct. A distribution of the CD8 antigen binding construct is monitored to determine a first tumor-infiltrating lymphocyte ("TIL") status within the tumor. The patient is treated based upon at least the first TIL status of the neoplasia. This can be use for the treatment and/or diagnosis of the subject.

**Example 45 - Neoplasia treatment method**

One of the compositions from Example 16 is selected for use in the present example.

A human subject is treated by a method by administering to a patient that has a neoplasia an antigen-binding construct that binds to human CD8. A distribution of the antigen-binding construct is monitored to determine the tumor-infiltrating lymphocyte ("TIL") status within the neoplasia. An immunotherapy ("IOT") is administered to the patient if the TIL status of at least one neoplasia is negative so as to convert the TIL status from negative to positive. This can be use for the treatment and/or diagnosis of the subject.

**Example 46 - Neoplasia treatment method**

One of the compositions from Example 16 is selected for use in the present example.

A human subject is treated by a method by administering to a patient that has a neoplasia an antigen-binding construct that binds to human CD8. A distribution of the antigen-binding construct is monitored to determine the tumor-infiltrating lymphocyte ("TIL") status within the neoplasia. An alternative immunotherapy ("IOT") is administered to the patient if the TIL status of the neoplasia is not positive, until the TIL status of the neoplasia becomes positive. This can be use for the treatment and/or diagnosis of the subject.

**Example 47 - Treating tumor in a subject**

One of the compositions from Example 16 is selected for use in the present example.

A tumor in a human subject is treated by a method. If a human subject is receiving a first treatment, it is determined via a CD8 binding molecule if a tumor in the subject has CD8 cells, and if so, the therapy is continued, and if cold then the therapy is changed to a second treatment. If the human subject is not receiving a first treatment, it is determined via a CD8 binding molecule if a tumor in the subject has CD8 cells, and if so, then an IOT is administered to the subject. This can be use for the treatment and/or diagnosis of the subject.

**Example 48 - Treatment method for a patient**

One of the compositions from Example 16 is selected for use in the present example.

A treatment for a patient is selected by a method by administering a CD8 binding construct to a human patient. A TIL status of a tumor is determined in the patient. If the tumor is cold, then an IOT is administered that changes the TIL status of the tumor. This can be use for the treatment and/or diagnosis of the subject.

**Example 49 - Treatment method**

One of the compositions from Example 16 is selected for use in the present example.

A treatment for a subject is changed by a method by identifying a human subject on a first therapy. A CD8 binding construct is administered to the human subject. It is determined if the subject has a tumor that is TIL positive under the first therapy. If the subject has a tumor that is TIL positive under the first therapy, the therapy is continued. If the subject has a tumor that is TIL negative under the first therapy, the first therapy is stopped. This can be use for the treatment and/or diagnosis of the subject.

**Example 50 - Method of treating a human having tumor**

One of the compositions from Example 16 is selected for use in the present example.

A human subject having a tumor is treated by a method by administering to the subject a dose of a CD8 antigen binding construct. The dose comprises a $^{89}$Zr-labeled antigen-binding construct providing a radiation activity of from about 0.5 mCI or in the alternative about 3.6 mCi. The dose comprises about 10 mg or less of the antigen binding construct;. The $^{89}$Zr-labeled antigen-binding construct is detected in the patient at a first time point after administering the dose, to generate a first patient image corresponding to the first time point. The first time point is about 6 hours or in the alternative about 36 hours after administering. A first abundance and/or distribution of CD8 positive cells is determined in one or more tumor(s) in the patient based on the first patient image. A first treatment is administered to the patient for the first abundance and/or distribution of CD8 in the one or more tumor(s). This can be use for the treatment

and/or diagnosis of the subject.

**Example 51 - Method of treating a human having cancer**

One of the compositions from Example 16 is selected for use in the present example.

A human subject having cancer is treated by a method by administering a CD8 antigen binding construct to a subject, wherein the CD8 antigen binding construct is labeled with a detectable label. At least a primary tumor and a secondary tumor status is identified within the subject's body. Via a distribution of the CD8 antigen binding construct the TIL status of the primary tumor is identified. A first therapy is administred to the subject based on the TIL status of the primary tumor. A CD8 antigen binding construct is administred to the subject to determine a subsequent TIL status of the primary tumor. The TIL status of the primary tumor is used to determine a subsequent appropriate treatment for the subject. This can be use for the treatment and/or diagnosis of the subject.

**Example 52 - Formulation for a CD8 composition**

A formulation for a CD8 composition comprises a CD8 antigen binding construct. The CD8 antigen binding construct is less than 105 kDa in size, and a $^{89}$Zr radiolabel is associated with the CD8 antigen binding construct. The radiolabel provides more than 0.5 but less than 3 mCi of radiation for the formulation. The formulation is configured for administration to a human.

**Example 53 - CD8 minibody formulation**

A CD8 minibody formulation comprises at least 1.5mg of a CD8 minibody, and 1.5 mCi or less of $^{89}$Zr. The formulation is configured for administration to a human.

**Example 54 - Diagnostic composition amanufacture method**

One of the compositions from Example 16 is selected for use in the present example.

A diagnostic composition is manufactured by a method by conjugating a CD8 antigen binding construct to desferrioxamine to form a Df-labelled CD8 antigen binding construct. The Df-labelled CD8 antigen binding construct is radiolabeled with $^{89}$Zr to form a radiolabeled CD8 antigen binding construct. The radiolabeled CD8 antigen binding construct is mixed with non-radiolabelled (cold) Df-labelled CD8 antigen binding construct to form a diagnostic composition. The formulation is configured for human administration. This can be use for the treatment and/or diagnosis of the subject.

**Example 55 - CD8 antigen binding construct formulation**

A CD8 antigen binding construct formulation comprises a CD8 antigen binding construct, and free arginine. The formulation is configured for administration to a human.

**Example 56 - PET scan method**

One of the compositions from Example 16 is selected for use in the present example.

A PET scan is performed in a human subject by a method comprising administering a minibody that binds to a human CD8 antigen. The minibody is conjugated to a radioactive label. The minibody is administered and provides more than 0.5 but less than 1.1 mCi of radiation. After a wait of 1-36 hours, a PET scan is performed over 15-30 minutes for scanning. This can be use for the treatment and/or diagnosis of the subject.

**Example 57 - PET method**

One of the compositions from Example 16 is selected for use in the present example.

A positron emission tomography ("PET") method comprises administering a tracer that binds to CD8 to a human subject. A scintillator is provided. The scintillator is used to detect a pair of photons created by the tracer. Detection of the pair of photons is used to localize a source of the tracer via a list of coincidence events that are processed via a processor that is configured to take an output from the scintillator and convert it to the list of coincidence events. Between about 300 and about 500 CD8 positive cells are detected per mm$^2$ of a tissue or neoplasia within the subject. This can be use for the treatment and/or diagnosis of the subject.

**Example 58 - Method of treating a humn having tumor**

One of the compositions from Example 16 is selected for use in the present example.

A human subject having a tumor is treated by a method. A PET and a) MRI or b) CT combined image of the subject is provided. The combined image emphasizes an overlap in an area of at least one tumor and a CD8 ROI. The area of at least one tumor is defined by a) MRI data or b) CT data and the CD8 ROI is defined by PET data. The PET and a) MRI or b) CT combined image is reviewed to determine a TIL status of a tumor in a patient. An IOT treatment is provided to the patient if the tumor is TIL negative. This can be use for the treatment and/or diagnosis of the subject.

**Example 59 - Method of treating a human with a candidate therapeutic**

One of the compositions from Example 16 is selected for use in the present example.

A human subject s treated by a method. A candidate therapeutic is administered to a human subject. It is determined if a size of a tumor in the human subject has increased or decreased in response to the candidate therapeutic. If the size has increased following the candidate therapeutic, then a CD8 antigen binding construct is used to determine if an amount of CD8 present within the tumor has increased or decreased in response to the candidate therapeutic. An increase in tumor size without an increase in the amount of CD8 indicates tumor progression. An increase in tumor size with an increase in the amount of CD8 indicates tumor pseudoprogression. A treatment is continued if the patient is experiencing tumor pseudoprogression. A treatment is changed if the patient is experiencing tumor progression. This can be use for the treatment and/or diagnosis of the subject.

**Example 60 - Distinguishing tumor progression from tumor pseudoprogression**

One of the compositions from Example 16 is selected for use in the present example.

Tumor progression is distinguished from tumor pseudoprogression after immunotherapy in a human patient by a method by administering a CD8 antigen binding construct to a human subject. A first distribution of the CD8 antigen binding construct within the subject and a first ROI are detected. A candidate immunotherapy is administered to the subject. A second distribution of the CD8 antigen binding construct within the subject and a second ROI are detected. An increase in tumor size without an increase in CD8 binding indicates tumor progression. An increase in tumor size with an increase in CD8 binding indicates tumor pseudoprogression. A treatment is continued if the patient is experiencing tumor pseudoprogression. A treatment is changed if the patient is experiencing tumor progression. This can be use for the treatment and/or diagnosis of the subject.

**Example 61 - Method of treating a human having tumor**

One of the compositions from Example 16 is selected for use in the present example.

A human subject having a tumor is treated by a method by administering a CD8 antigen binding construct to the subject and detecting a first distribution of the CD8 antigen binding construct within the subject and a first ROI. A candidate immunotherapy is administered to the subject. A second distribution of the CD8 antigen binding construct within the subject and a second ROI are detected. If the second ROI relative to the first ROI is the same or larger in area (relative to the subject) and demonstrates increased CD8 infiltration, then administration of the candidate immunotherapy is continued. If the second ROI relative to the first ROI is the same or larger in area (relative to the subject) and demonstrates no increase in CD8 infiltration, then administration of the candidate immunotherapy is discontinued. This can be use for the treatment and/or diagnosis of the subject.

**Example 62 - Label administration method**

One of the compositions from Example 16 is selected for use in the present example.

A label is administered to a human subject by a method by administering $^{18}F$ to a subject. Within about 6 hours, a PET scan is conducted of the subject for a $^{18}F$ distribution of the subject. A CD8 antigen binding construct is administered to the subject. The CD8 antigen binding construct is linked to $^{89}Zr$. Within about 36 hours of administering the CD8 antigen binding construct, a PET scan is conducted on the subject for a distribution of $^{89}Zr$. This can be use for the treatment and/or diagnosis of the subject.

**Example 63 - Human image preparation method**

One of the compositions from Example 16 is selected for use in the present example.

An image of a human subject is prepared by a method by providing a first data set involving a representation

of a MRI or FDG-PET scan. The MRI or FDG-PET scan indicates at least one tumor. A second data set is provided involving a representation of a PET scan of a 89Zr-CD8 antigen binding construct. The second data set indicates at least one CD8 Region of Interest ("ROI"). A combined image from the two data sets is generated, wherein the combined image provides a comparison of the at least one tumor and the at least one CD8 ROI. This can be use for the treatment and/or diagnosis of the subject.

**Example 64 - Analysis of CD8 distribution**

One of the compositions from Example 16 is selected for use in the present example.

CD8 distribution in a subject is analyzed by a method by providing an image of a distribution of a detectable marker via a first PET image.The detectable marker is linked to a CD8 antigen binding construct. An image is provided of a distribution of a FDG marker via a second PET image of the subject. A third image is created that comprises an overlay of the first PET image onto the second PET image. The TIL status is identified of a tumor based upon the third image. This can be use for the treatment and/or diagnosis of the subject.

**Example 65 - Identification of tumor characteristics**

One of the compositions from Example 16 is selected for use in the present example.

Tumor characteristics are identified by a method by scanning for a distribution of $^{18}F$ within a human subject to identify at least one tumor. A scanning is performed for a distribution of $^{89}Zr$ labeled CD8 antigen binding construct with the human subject to identify elevated concentrations of $^{89}Zr$ within the subject. The tumor is identified as inflamed if the at least one tumor overlaps in location with any one or more elevated concentrations of $^{89}Zr$ within the subject. This can be use for the treatment and/or diagnosis of the subject.

**Example 66 - Identification of tumor characteristics**

One of the compositions from Example 16 is selected for use in the present example.

Tumor characteristics are identified by a method by providing $^{18}F$ to a subject. A PET scan is conducted of the subject to identify $^{18}F$ distribution within the subject to render a representation of a tumor. A $^{89}Zr$ labeled CD8 antigen binding construct is provided to the subject. A PET scan is conducted of the subject to identify elevated concentrations of $^{89}Zr$ within the subject in a representation of CD8 distribution in the subject. The representation of the tumor with the representation of CD8 distribution are compared to identify areas of overlap in the two representations. Areas of tumor location and elevated concentrations of $^{89}Zr$ indicate tumors that are inflamed and do not require an IOT. Areas where there are tumors, but no elevated concentrations of $^{89}Zr$ represent tumors that are not inflamed and require an IOT to adjust their TIL status. This can be use for the treatment and/or diagnosis of the subject.

**Example 67 - Identification of solid tumor in brain**

One of the compositions from Example 16 is selected for use in the present example.

A solid tumor in the brain of a human subject is identified by a method by administering a CD8 antigen binding construct having a PET detectable label to the subject. PET scanning is performed of at least the head of the subject. A distribution of the PET detectable label in the brain is obtained.Identification of a CD8 concentrated region in the distribution indicates the presence of a solid tumor in the brain of the subject. This can be use for the treatment and/or diagnosis of the subject.

**Example 68 - Monitoring CD8 bearing cells**

One of the compositions from Example 16 is selected for use in the present example.

CD8 bearing cells are monitored in vivo by a method by providing a CD8 minibody to a human subject. The CD8 minibody binds to a human CD8 as shown in FIG. 1C. The minibody is labeled with a PET detectable marker. Distribution of the minibody is monitored in the subject. The monitoring distinguishes if a tissue volume in the subject is infiltrated with greater than or less than a selected amount of CD8 bearing cells. The monitoring is achieved via PET. This can be use for the treatment and/or diagnosis of the subject.

**Example 69 - Visualizing CD8 bearing cells**

One of the compositions from Example 16 is selected for use in the present example.

CD8 bearing cells are visualized in a human subject by a method by administering a CD8 minibody to a human

subject. The minibody is labeled with a PET detectable marker. The minibody sequence is humanized and binds to human CD8. A distribution of the minibody is monitored in the subject within 6-36 hours of administering the minibody to the subject. The method can distinguish whether a volume of the tissue is infiltrated with greater than or less than a selected amount of CD8 bearing cells within the subject. The monitoring is achieved via PET. This can be use for the treatment and/or diagnosis of the subject.

**Example 70 - Determining TIL status of tumor**

One of the compositions from Example 16 is selected for use in the present example.

The TIL status of a tumor is determined in a human subject by a method by administering a CD8 antigen binding construct to the subject. A distribution of the CD8 antigen binding construct is monitored in the subject by PET. The monitoring identifies a TIL positive tumor. The monitoring provides at least one of:

a tumor that is infiltrated with 500 or less CD8 bearing cells per $mm^2$ within the subject,
a tumor that is infiltrated with greater than or less than 500 CD8 bearing cells per $mm^2$ within the subject,
a tumor that is infiltrated with greater than or less than 200 CD8 bearing cells per $mm^2$ within the subject,
a tumor that is infiltrated with greater than or less than 100-500 CD8 bearing cells/$mm^2$,
a tumor that is infiltrated with less than 1% CD8 bearing cells, 1-50% CD8 bearing cells or greater than 50% CD8 bearing cells,
a tumor that comprises CD8 bearing cells that corresponds to IHC measurement of 2.5 to 25% of cells in a 4 micron tissue section,
a tumor that comprises between 10,000 to 100,000 CD8 bearing cells is per $mm^3$ of the corresponding tumor,
a cell intensity of CD8 bearing cells, of the corresponding tumor that is low (cold) or high (hot).

This can be use for the treatment and/or diagnosis of the subject.

**Example 71 - Visualization of distribution of CD8 bearing cells**

One of the compositions from Example 16 is selected for use in the present example.

A distribution of CD8 bearing cells is visualized in a human subject by PET. A CD8 antigen binding construct is administered to the subject. The CD8 antigen binding construct is labeled with a PET detectable label. A distribution of the CD8 antigen binding construct is monitored in the subject within 6-36 hours of administering the CD8 antigen binding construct. The monitoring can detect a tissue infiltrated with 500 or less CD8 positive cells per $mm^2$ within the subject. The monitoring is achieved via PET and converted to an image for visualizing the distribution. This can be use for the treatment and/or diagnosis of the subject.

**Example 72 - Visualization of cells in a human**

One of the compositions from Example 16 is selected for use in the present example.

Cells in a human are visulied by a method by providing a means of binding human CD8 positive cells to a subject. The means comprises a detectable label. A first distribution of the means of binding human CD8 positive cells is monitored and determined in the subject within 6-36 hours of administering the means of binding human CD8 positive cells to the subject. This can be use for the treatment and/or diagnosis of the subject.

**Example 73 - Determining TIL status of tumor**

One of the compositions from Example 16 is selected for use in the present example.

The TIL status of a tumor in a human subject is determined by a method by administering a CD8 antigen binding construct to the subject. The CD8 antigen binding construct is labeled with a PET detectable label. A distribution of the CD8 antigen binding construct having the PET detectable label is monitored in the subject. The degree to which the PET detectable label has penetrated a known or suspected tumor is evaluated in the subject. The tumor is TIL positive if the PET detectable label has penetrated the tumor and TIL negative if not. This can be use for the treatment and/or diagnosis of the subject.

**Example 74 - Determining change in CD8 distribution**

One of the compositions from Example 16 is selected for use in the present example.

A change in distribution of CD8 cells in a human subject having cancer is imaged by a method by administering

to the subject a CD8 antigen binding construct. PET is used to determine a first distribution of CD8 antigen binding construct in the subject. An immunotherapy is administered to the subject. PET is used to determine a second distribution of CD8 antigen binding construct in the subject. A difference between the first distribution and the second distribution demonstrates the change. This can be use for the treatment and/or diagnosis of the subject.

## Example 75 - Assessing responsiveness to IOT

One of the compositions from Example 16 is selected for use in the present example.

If a subject is responding to an IOT is determined by a method by providing a baseline CD8 distribution for the subject within a ROI, via an in vivo technique. An IOT is administered. A treated CD8 distribution is provided for the subject within the ROI. The treated CD8 distribution is obtained within 35 days of administering the IOT. The treated CD8 distribution is compared to the baseline CD8 distribution to determine if the subject is responding to the IOT. This can be use for the treatment and/or diagnosis of the subject.

## Example 76 - Predciting checkpoint inhibitor responsiveness

One of the compositions from Example 16 is selected for use in the present example.

A subject's responsiveness to a checkpoint inhibitor is predicted by a method by providing a baseline CD8 distribution for the subject within a ROI, via an in vivo technique. A checkpoint inhibitor is administered. A checkpoint inhibitor CD8 distribution is provided for the subject within the ROI. The checkpoint inhibitor CD8 distribution is obtained within 35 days of administering the IOT. The checkpoint inhibitor CD8 distribution is compared to the baseline CD8 distribution to determine if the subject is responsive to the checkpoint inhibitor. This can be use for the treatment and/or diagnosis of the subject.

## Example 77 - Monitoring therapy effectiveness

One of the compositions from Example 16 is selected for use in the present example.

An effectiveness of a therapy is monitored by a method by providing a first CD8 image for a subject. The subject is at a baseline for a therapy. A therapy is administered to the subject. A second CD8 image is provided for the subject. At least one day has passed since the therapy is administered to the subject. If there is no significant increase in CD8 imaging agent, the subject is not responding to the therapy. This can be use for the treatment and/or diagnosis of the subject.

## Example 78 - Tumor analysis mehtod

One of the compositions from Example 16 is selected for use in the present example.

A tumor is analyzed by a method by characterizing a degree of relative CD8 infiltration in a tumor in a subject in response to a candidate therapeutic. It is observed if there is an increase or a decrease in degree of CD8 infiltration via a CD8 antigen binding construct comprising a radiolabel. An increase in CD8 infiltration indicates that the candidate therapeutic is functioning as a therapeutic. A decrease in CD8 infiltration indicates that the candidate therapeutic is not functioning as a therapeutic, and the tumor is increased in size between before the candidate therapeutic is administered and after the candidate therapeutic is administered. This can be use for the treatment and/or diagnosis of the subject.

## Example 79 - Determination of standard uptake value (SUV)

One of the compositions from Example 16 is selected for use in the present example.

A standard uptake value (SUV) is determined by a method by applying an antigen binding construct to a subject. The antigen binding construct comprises a radioactive probe. An r is determined, where r is the radioactivity concentration (kBq/ml) measured by a PET scanner within a ROI of radiation from the antigen binding construct. An a' is determined, wherein a' is the decay-corrected amount of the injected radiolableeld tracer (kBq). A w is determined, where w is the weight of the patient. An SUV is determined as the result of r(a'/W). This can be use for the treatment and/or diagnosis of the subject.

## Example 80 - Image analysis method

One of the compositions from Example 16 is selected for use in the present example.

An image is analyzed by providing an image. A first region of interest (ROI) is defined on the image by marking the image. A signal intensity is determined for a data point within the first ROI. A maximum signal intensity is determined

within the first ROI. A mean value of the signal intensities is determined within the first ROI. Each signal intensity within the first ROI is summed together to obtain a first summed signal level for the first ROI. The first ROI represents data for an amount of a detectable marker associated with an antigen binding construct that has been administered to a subject. This can be use for the treatment and/or diagnosis of the subject.

**Example 81 - Image generation method**

One of the compositions from Example 16 is selected for use in the present example.

An image is generated by applying an antigen-binding construct that binds to CD8. The the antigen-binding construct comprises a detectable marker; and detecting at least one detectable marker within a location within the tumor and assigning a positive pixel for each detectable marker detected; and generating an image based on a distribution of each positive pixel assigned, wherein the image is stored in a non-transitory computer readable media. This can be use for the treatment and/or diagnosis of the subject.

**Example 82 - Biopsy method**

One of the compositions from Example 16 is selected for use in the present example.

A biopsy is performed in a subject by providing an antigen binding construct that binds to CD8 to the subject. Based upon an elevated level of the antigen binding construct within a particular tumor, the particular tumor for a biopsy is selected, and sample from the particular tumor is collected. This can be use for the treatment and/or diagnosis of the subject.

**Example 83 - Biopsy method**

One of the compositions from Example 16 is selected for use in the present example.

A biopsy is performed in a subject by providing an antigen binding construct that binds to CD8 to the subject, selecting, based upon a distribution the antigen binding construct within a particular tumor, the particular location within the tumor to biopsy, and collecting a sample from the particular location within the tumor. The particular location is either a) internal to the tumor or b) on a surface of the tumor. This can be use for the treatment and/or diagnosis of the subject.

**Example 84 - Immune-related adverse event (irAE) is detection method**

One of the compositions from Example 16 is selected for use in the present example.

An immune-related adverse event (irAE) is detected by a method by identifying a human subject on a checkpoint inhibitor therapy. A CD8 antigen binding construct is administered to the human subject. The antigen binding construct comprises a detectable label. The detectable label is monitored within the subject to determine if there is immune related toxicity. The immune related toxicity is indicated by a change in CD8 distribution. If the immune related toxicity is severe or irreversible, the checkpoint inhibitor therapy is stopped. If the immune related toxicity is not severe or irreversible, the checkpoint inhibitor therapy is suspended while continuing to monitor CD8 levels until they return to a lower level. This can be use for the treatment and/or diagnosis of the subject.

**Example 85-Phase I**

The central imaging review for the 2015-22M2C-01 clinical trial was conducted according to a single radiology review paradigm, meaning a single radiologist reviewed imaging for a given subject. Prior to the start of imaging reads, IE selected and trained a single reader for this study. The $^{89}$Zr-Df-IAB22M2C PET/CT time points acquired for a subject were presented to the reader simultaneously for assessment along with the pre-study conventional imaging scan(s). The reader made assessments for each $^{89}$Zr-Df-IAB22M2C PET/CT time point and the conventional imaging scan with the ability to go back and forth amongst the time points and modalities before signing off on the reads. Once the assessments were signed, they were locked and could only be edited through a data change or re-review. The central read design is outlined in Fig. 62A and each of the numbered steps within the diagram is further described following the figure.

The following steps were applied to the central imaging read for each subject:

**1. Select and measure target lesions identified on conventional imaging:**

- Identified lesions and/or abnormal lymph nodes using the pre-study conventional imaging scan. These lesions were measured on the pre-study scan using a bi-dimensional ruler.

  - ◦ Lymph nodes were ≥10 millimeters (mm) in short axis to be considered abnormal, however, this was not an absolute requirement; if the node was unequivocally abnormal by morphology but <10 mm in short axis, then it could be selected as a target lesion.
  - ◦ If the pre-study conventional imaging scan was an $^{18}$F-PDG-PET, then the SUV of the lesions in addition to bi-dimensional diameters was measured.
  - ◦ Up to three target lesions per organ that were identified by conventional imaging were selected.

    - ▪ If there were more than three lesions, then the three largest nodes and/or solid tumors were selected. If both abnormal nodes and solid tumors were present, at least one of each was selected.

- The SUV of each target lesion on each of the 89Zr-Df-IAB22M2C PET/CT scans was measured. If a lesion was not visible on the 89Zr-Df-IAB22M2C PET/CT, then the lesion was indicated as PET-negative and the SUV of the area was still measured where the lesion was visualized on the other 89Zr-Df-IAB22M2C PET/CT scan(s) or at the equivalent anatomic site equivalent to its location on the conventional scans. If any target lesion could not be measured on a 89Zr-Df-IAB22M2C PET/CT, a comment on the analysis form was entered.

  - ◦ If the lesions were PET-positive, the type of uptake was indicated as homogeneous, peripheral, mixed, or central for each target lesion at each $^{89}$Zr-Df-IAB22M2C PET/CT time point.

**2. Select and measure target lesions identified on $^{89}$Zr-Df-IAB22M2C PET/CT**

- Up to three lesions/abnormal nodes presented on the series of $^{89}$Zr-Df-IAB22M2C PET/CTs could be selected if not be seen on the pre-study conventional imaging scan.

  - ◦ If there were more than three lesions/abnormal nodes showing uptake across the series of $^{89}$Zr-Df-IAB22M2C PET/CTs, then the three nodes showing the most uptake were selected.
  - ◦ The SUV of these lesions were measured on each $^{89}$Zr-Df-IAB22M2C PET/CT. If any target lesion could not be measured on a 89Zr-Df-IAB22M2C PET/CT, this was indicated by entering a comment on the analysis form.
  - ◦ Each of these lesions were measured or marked on the pre-study conventional imaging scan. A bi-dimensional ruler was used to measure each lesion unless it was not visible on the conventional imaging, in which case the location was marked with an arrow and assessed as 'absent' or if not able to annotate at all, indicated this by recording a comment on the analysis form.

**3. Evaluate each lymph node chain for uptake on $^{89}$Zr-Df-IAB22M2C PET/CT:**

- Whether there is 89Zr-Df-IAB22M2C PET/CT uptake in each lymph node chain was indicated.

  - ◦ The lymph node chains include: cervical, supraclavicular, axillary, mediastinal, hilar, mesenteric, retrocrural, retroperitoneal, para-aortic, pelvic, and inguinal.

**4. Measure the SUVs of lymphoid and non-lymphoid reference tissues:**

- The SUV of the following reference tissues were measured: liver, spleen, aorta, muscle, bone marrow, lung, and left kidney. Reference tissues must have been uninvolved and assessed as PET negative. If any reference tissue could not be measured, an explanatory comment was entered. The reference tissues were measured as follows:

  - ◦ Liver - volume of interest (VOI) ~3 centimeters (cm) in diameter within the right lobe
  - ◦ Spleen - VOI ~ 1.5 cm in diameter within the spleen
  - ◦ Aorta - VOI ~ 1.5 cm in diameter at a mid-thoracic location
  - ◦ Muscle - VOI ~ 1.5 cm in diameter with the para spinal muscle at L5
  - ◦ Bone Marrow - VOI ~ 1.5 cm within the L5 or similar vertebral body
  - ◦ Lung - VOI ~3 cm in diameter within the right lower lobe
  - ◦ Left Kidney - VOI ~3 cm within the left kidney. The right kidney can be used if left cannot be used (e.g., if subject has no left kidney).

**5. Select the optimal 89Zr-Df-IAB22M2C PET/CT scan(s):**

- Once all $^{89}$Zr-Df-IAB22M2C PET/CT scans were reviewed, the scan(s) which demonstrated the optimal distribution of $^{89}$Zr-Df-IAB22M2C on PET/CT was indicated. The optimal time point(s) was/were considered to be the time point(s) showing the most focal $^{89}$Zr-Df-IAB22M2C PET/CT uptake consistent with the presence of CD8+ T cells.
- For the assessment of optimal scan, multiple scans could be indicated as optimal or only a single scan could be assessed as optimal. Alternatively, it was acceptable not to select any scan as optimal. This was expected for cases that did not demonstrate uptake on any $^{89}$Zr-Df-IAB22M2C PET/CT. If no optimal scan was selected and any $^{89}$Zr-Df-IAB22M2C PET/CT shows uptake, then a comment was entered to provide a rationale.

The imaging schedule is outline in Table 85.1A below.

**Table 85.1A**

| Visit | Schedule | Imaging |
|---|---|---|
| **Historical Scan:** Conventional Imaging[1] | N/A | CT scan of chest, abdomen, pelvis, and any areas of relevant anatomy of known or suspected disease<br><br>Additional historical scans used for tumor staging (such as $^{18}$F-FDG-PET or Bone Scans) can also be submitted |
| **Visit 2:** Infusion + PET/CT scans | 1-2 hours following $^{89}$Zr-Df-IAB22M2C infusion<br>6-8 hours following $^{89}$Zr-Df-IAB22M2C infusion (optional) | Whole body $^{89}$Zr-Df-IAB22M2C PET/CT |
| **Visit 3:** PET/CT scan | 24 hours following $^{89}$Zr-Df-IAB22M2C infusion (± 4 hours) | |
| **Visit 4:** PET/CT scan | 48 hours following $^{89}$Zr-Df-IAB22M2C infusion (± 4 hours) | |
| **Visit 5:** PET/CT scan | 92-144 hours following $^{89}$Zr-Df-IAB22M2C infusion | |

[1]Historical CT scans were accepted if performed on a non-approved scanner

**$^{89}$Zr-Df-IAB22M2C Uptake in Reference Tissues:**

The uptake of 89Zr-Df-IAB22M2C in reference tissues from both cohorts were measured at each time point on each available scan. The reference tissues included Spleen, Bone Marrow (L5 vertebrae) and Lymph Nodes as the expected sites of CD8+ TIL rich populations. Lymph Node uptake was included as part of CD8+ TIL-rich reference tissue if it was of normal size (<15 mm in short axis diameter on conventional imaging), normal morphology and/or demonstrated lack of FDG uptake if a PET scan was available for review. The Liver, Lung, Kidney (left) and Muscle (paraspinal muscle) was selected as reference tissues because of their anticipated low CD8+ TIL densities. The Aorta was selected as measure of 89Zr-Df-IAB22M2C activity for the blood pool (circulating reservoir- arterial input function) activity.

Table 85.1B lists 89Zr-Df-IAB22M2C activity in reference tissue for all subjects. Please note that the times indicated in the table and figures represent the average time that scans were acquired unless otherwise stated in the document. For example, the 1-2hr, 6-8hr, 24hr, 48hr and 5-7 day scans acquisition time points as written in the protocol were obtained on average at 1.6hr, 6.2hr, 24.3hr, 48.4hr and 135.0hr, respectively. Based on the patient data provided, the user can apply any of the analytical techniques also disclosed herein to draw the diagnostic conclusions and make treatment decisions as described herein."

## TABLE 85.1B

## INDIVIDUAL SUBJECT SUMMARY MEASUREMENTS

| | Hour of Imaging post injections | | | | |
|---|---|---|---|---|---|
| **Subject 1 (0.2 mg)** | **2.1** | **6.1** | **25.2** | **49.8** | **121.2** |
| Spleen (mean) | 74.7 | 72.1 | 49.6 | 46.7 | 40.3 |
| BM (L5-mean) | 9.0 | 10.1 | 8.3 | 7.5 | 5.9 |
| Aorta (mean) | 0.9 | 0.6 | 0.8 | 0.7 | 0.6 |
| Liver (mean) | 3.4 | 3.0 | 3.1 | 3.2 | 3.1 |
| Muscle (mean) | 0.2 | 0.2 | 0.3 | 0.3 | 0.8 |
| Left Kidney (mean) | 2.6 | 2.7 | 3.8 | 3.9 | 3.4 |
| Lung (mean) | 0.5 | 0.6 | 0.5 | 0.3 | 0.4 |
| T01 Muscle (max) | 1.5 | 2.8 | 6.4 | 5.6 | 3.1 |
| T02 Muscle (max) | 0.4 | 0.4 | 0.6 | 0.8 | 0.9 |
| T03 Muscle (max) | 0.5 | 0.3 | 0.3 | 0.5 | 1.2 |
| T01 Muscle (peak) | 0.7 | 1.2 | 2.3 | 2.1 | 1.4 |

| | Hour of Imaging post injections | | | | |
|---|---|---|---|---|---|
| **Subject 1 (0.2 mg)** | **2.1** | **6.1** | **25.2** | **49.8** | **121.2** |
| T02 Muscle (peak) | 0.2 | 0.4 | 0.3 | 0.4 | 0.4 |
| T03 Muscle (peak) | 0.3 | 0.3 | 0.2 | 0.3 | 0.4 |

| | Hour of Imaging post injections | | | | |
|---|---|---|---|---|---|
| **Subject 2 (0.5 mg)** | **1.4** | **No Scan** | **25.0** | **45.5** | **141.0** |
| Spleen (mean) | 54.5 | No Scan | 44.9 | 40.5 | 38.1 |
| BM (L5-mean) | 7.9 | No Scan | 6.3 | 4.7 | 3.2 |
| Aorta (mean) | 1.9 | No Scan | 0.5 | 0.4 | 0.3 |
| Liver (mean) | 4.6 | No Scan | 5.1 | 4.7 | 4.0 |
| Muscle (mean) | 0.3 | No Scan | 0.3 | 0.3 | 0.2 |
| Left Kidney (mean) | 2.7 | No Scan | 3.8 | 3.9 | 3.1 |
| Lung (mean) | 0.5 | No Scan | 0.2 | 0.2 | 0.1 |
| T01 LN Other, Portocaval (max) | 1.5 | No Scan | 6.3 | 3.7 | 5.6 |
| T02 LN Retroperitoneal (max) | 0.6 | No Scan | 1.5 | 1.5 | 2.3 |
| T03 Liver (max) | 1.6 | No Scan | 3.5 | 2.8 | 4.2 |
| T01 LN Other, Portocaval (peak) | 1.2 | No Scan | 4.4 | 2.9 | 3.4 |
| T02 LN Retroperitoneal (peak) | 0.5 | No Scan | 1.2 | 0.7 | 1.5 |
| T03 Liver (peak) | 1.5 | No Scan | 3.5 | 2.6 | 3.9 |

| | Hour of Imaging post injections | | | | |
|---|---|---|---|---|---|
| **Subject 10 (0.5 mg)** | **1.6** | **6.5** | **23.8** | **48.9** | **121.4** |
| Spleen (mean) | 56.5 | 55.8 | 53.5 | 50.4 | 47.3 |
| BM (L5-mean) | 5.4 | 6.2 | 6.0 | 5.2 | 3.7 |
| Aorta (mean) | 1.1 | 0.9 | 0.7 | 0.5 | 0.7 |
| Liver (mean) | 10.2 | 9.3 | 8.1 | 7.9 | 8.4 |
| Muscle (mean) | 0.3 | 0.3 | 0.5 | 0.3 | 0.3 |
| Left Kidney (mean) | 1.4 | 2.0 | 7.0 | 4.5 | 2.4 |
| Lung (mean) | 1.0 | 1.0 | 0.6 | 0.5 | 0.4 |
| T01 LN inguinal (max) | 0.7 | 0.8 | 1.6 | 1.7 | 2.0 |
| T01 LN inguinal (peak) | 0.3 | 0.6 | 1.1 | 1.3 | 1.4 |

| | Hour of Imaging post injections | | | | |
|---|---|---|---|---|---|
| **Subject 11 (0.5 mg)** | **1.3** | **No Scan** | **24.6** | **46.7** | **121.5** |
| Spleen (mean) | 57.7 | No Scan | 47.2 | 48.6 | 38.3 |
| BM (L5-mean) | 6.2 | No Scan | 5.8 | 4.1 | 8.2 |
| Aorta (mean) | 1.7 | No Scan | 0.7 | 0.6 | 0.6 |
| Liver (mean) | 4.7 | No Scan | 4.2 | 4.6 | 4.9 |
| Muscle (mean) | 0.3 | No Scan | 0.5 | 0.4 | 0.4 |
| Left Kidney (mean) | 3.3 | No Scan | 4.2 | 6.0 | 4.1 |
| Lung (mean) | 0.7 | No Scan | 0.5 | 0.9 | 0.6 |
| T01 Lung (max) | 1.6 | No Scan | 0.5 | 1.0 | 1.8 |
| T02 Lung (max) | 0.8 | No Scan | 0.4 | 0.8 | 1.8 |

| | Hour of Imaging post injections | | | | |
|---|---|---|---|---|---|
| **Subject 11 (0.5 mg)** | **1.3** | **No Scan** | **24.6** | **46.7** | **121.5** |
| T03 Liver (max) | 4.7 | No Scan | 4.1 | 8.4 | 8.3 |
| T01 Lung (peak) | 0.9 | No Scan | 0.3 | 0.5 | 0.9 |
| T02 Lung (peak) | 0.7 | No Scan | 0.4 | 0.4 | 0.0 |
| T03 Liver (peak) | 4.2 | No Scan | 3.2 | 4.5 | 4.4 |

| | Hour of Imaging post injections | | | | |
|---|---|---|---|---|---|
| **Subject 14 (0.5 mg)** | **1.2** | **No Scan** | **26.2** | **48.0** | **123.0** |
| Spleen (mean) | 106.4 | No Scan | 82.1 | 71.2 | 72.5 |
| BM (L5-mean) | 10.9 | No Scan | 10.1 | 8.4 | 6.6 |
| Aorta (mean) | 2.3 | No Scan | 1.0 | 0.8 | 0.8 |
| Liver (mean) | 8.3 | No Scan | 5.9 | 7.0 | 5.8 |
| Muscle (mean) | 0.4 | No Scan | 0.4 | 0.5 | 0.4 |
| Left Kidney (mean) | 2.9 | No Scan | 4.1 | 4.3 | 4.9 |
| Lung (mean) | 0.8 | No Scan | 0.6 | 0.5 | 0.4 |
| T01 LN Cervical (max) | 0.4 | No Scan | 0.4 | 0.5 | 1.3 |
| T02 LN Cervical (max) | 0.3 | No Scan | 0.9 | 0.6 | ND |
| T03 LN Axillary (max) | 1.9 | No Scan | 3.7 | 12.8 | 10.0 |
| T01 LN Cervical (peak) | 0.3 | No Scan | 0.4 | 0.4 | 0.5 |
| T02 LN Cervical (peak) | 0.2 | No Scan | 0.5 | 0.3 | ND |
| T03 LN Axillary (peak) | 0.0 | No Scan | 1.9 | 6.9 | 5.4 |

| | Hour of Imaging post injections | | | | |
|---|---|---|---|---|---|
| **Subject 15 (0.5 mg)** | **1.1** | **No Scan** | **21.7** | **45.5** | **123.9** |
| Spleen (mean) | 74.9 | No Scan | 60.8 | 51.0 | 45.6 |
| BM (L5-mean) | 4.7 | No Scan | 4.3 | 3.3 | 3.3 |
| Aorta (mean) | 2.1 | No Scan | 0.8 | 0.5 | 0.4 |
| Liver (mean) | 3.9 | No Scan | 3.9 | 3.7 | 4.0 |
| Muscle (mean) | 0.3 | No Scan | 0.3 | 0.4 | 0.3 |
| Left Kidney (mean) | 1.8 | No Scan | 4.0 | 4.1 | 3.4 |
| Lung (mean) | 0.3 | No Scan | 0.3 | 0.3 | 0.4 |
| T01 LN Mediastinal (max) | 1.8 | No Scan | 1.0 | 1.1 | 1.7 |
| T02 LN Mediastinal (max) | 2.0 | No Scan | 1.2 | 1.3 | 1.0 |
| T03 LN Mediastinal (max) | 2.3 | No Scan | 2.2 | 1.8 | 2.3 |
| T01 LN Mediastinal (peak) | 1.6 | No Scan | 0.8 | 0.7 | 0.9 |
| T02 LN Mediastinal (peak) | 1.9 | No Scan | 1.0 | 1.0 | 0.7 |
| T03 LN Mediastinal (peak) | 1.9 | No Scan | 1.5 | 1.4 | 1.6 |

| | Hour of Imaging post injections | | | | |
|---|---|---|---|---|---|
| **Subject 3 (1.0mg)** | **1.2** | **6.2** | **24.2** | **48.1** | **144.0** |
| Spleen (mean) | 43.3 | 30.7 | 22.5 | 21.6 | 20.7 |
| BM (L5-mean) | 3.7 | 3.6 | 3.5 | 4.0 | 4.2 |
| Aorta (mean) | 1.3 | 0.6 | 0.4 | 0.3 | 0.1 |

| | Hour of Imaging post injections | | | | |
|---|---|---|---|---|---|
| **Subject 3 (1.0mg)** | **1.2** | **6.2** | **24.2** | **48.1** | **144.0** |
| Liver (mean) | 6.5 | 6.0 | 5.1 | 4.9 | 4.9 |
| Muscle (mean) | 0.3 | 0.6 | 0.2 | 0.3 | 0.2 |
| Left Kidney (mean) | 1.8 | 2.0 | 1.8 | 2.0 | 1.6 |
| Lung (mean) | 0.5 | 0.4 | 0.3 | 0.3 | 0.3 |
| T01 LN Cervical (max) | 0.9 | 0.8 | 0.7 | 0.6 | 0.7 |
| T02 Liver (max) | 6.1 | 8.2 | 3.6 | 5.1 | 4.2 |
| T03 Lung (max) | 1.6 | 0.9 | 0.6 | 0.7 | 1.0 |
| T01 LN Cervical (peak) | 0.7 | 0.5 | 0.4 | 0.4 | 0.4 |
| T02 Liver (peak) | 6.1 | 7.2 | 3.8 | 4.6 | 3.7 |
| T03 Lung (peak) | 1.1 | 0.7 | 0.4 | 0.4 | 0.6 |

| | Hour of Imaging post injections | | | | |
|---|---|---|---|---|---|
| **Subject 4 (1.5 mg)** | **2.1** | **No Scan** | **23.0** | **47.2** | **119.3** |
| Spleen (mean) | 41.8 | No Scan | 35.2 | 34.1 | 29.1 |
| BM (L5-mean) | 6.4 | No Scan | 5.6 | 5.1 | 4.9 |
| Aorta (mean) | 3.0 | No Scan | 0.9 | 0.6 | 0.6 |
| Liver (mean) | 5.5 | No Scan | 6.2 | 5.9 | 5.3 |
| Muscle (mean) | 0.4 | No Scan | 0.3 | 0.2 | 0.4 |
| Left Kidney (mean) | 3.8 | No Scan | 4.9 | 5.1 | 4.7 |
| Lung (mean) | 0.7 | No Scan | 0.4 | 0.4 | 0.4 |
| T01 LN inguinal (max) | 0.3 | No Scan | 0.4 | 0.5 | 0.2 |
| T02 LN inguinal (max) | 0.5 | No Scan | 0.2 | 0.4 | 0.4 |
| T03 LN inguinal (max) | 0.3 | No Scan | 0.3 | 0.2 | 0.3 |
| T01 LN inguinal (peak) | 0.2 | No Scan | 0.2 | 0.2 | 0.2 |
| T02 LN inguinal (peak) | 0.3 | No Scan | 0.1 | 0.2 | 0.2 |
| T03 LN inguinal (peak) | 0.0 | No Scan | 0.2 | 0.2 | 0.1 |

| | Hour of Imaging post injections | | | | |
|---|---|---|---|---|---|
| **Subject 9 (1.5 mg)** | **1.1** | **6.0** | **27.7** | **50.5** | **289.5** |
| Spleen (mean) | 36.5 | 35.9 | 29.5 | 22.6 | 29.3 |
| BM (L5-mean) | 4.0 | 3.1 | 2.7 | 2.5 | 3.5 |
| Aorta (mean) | 2.9 | 2.1 | 0.7 | 0.9 | 0.4 |
| Liver (mean) | 4.1 | 4.4 | 4.0 | 4.0 | 4.0 |
| Muscle (mean) | 0.5 | 0.4 | 0.3 | 0.7 | 0.3 |
| Left Kidney (mean) | 1.8 | 2.1 | 3.8 | 3.1 | 2.9 |
| Lung (mean) | 0.7 | 0.6 | 0.4 | 0.4 | 0.4 |
| T01 LN Pelvic (max) | 1.2 | 0.8 | 1.2 | 1.5 | 2.5 |
| T02 LN Pelvic (max) | 1.7 | 2.3 | 3.9 | 2.9 | 2.3 |
| T01 LN Pelvic (peak) | 0.9 | 0.6 | 0.9 | 1.2 | 1.1 |
| T02 LN Pelvic (peak) | 1.5 | 1.7 | 2.7 | 2.0 | 1.5 |

| | Hour of Imaging post injections | | | | |
|---|---|---|---|---|---|
| **Subject 7 (1.5 mg)** | **2.1** | **6.4** | **24.6** | **48.2** | **121.0** |

| | | | | | |
|---|---|---|---|---|---|
| Spleen (mean) | 56.3 | 56.7 | 69.3 | 47.3 | 45.8 |
| BM (L5-mean) | 3.9 | 3.4 | 4.7 | 3.2 | 2.2 |
| Aorta (mean) | 4.2 | 3.3 | 1.2 | 0.8 | 0.9 |
| Liver (mean) | 8.0 | 7.0 | 8.4 | 7.1 | 7.3 |
| Muscle (mean) | 0.4 | 0.4 | 0.2 | 0.4 | 0.6 |
| Left Kidney (mean) | 4.9 | 6.1 | 7.2 | 6.4 | 5.6 |
| Lung (mean) | 1.4 | 1.3 | 1.1 | 0.7 | 0.8 |
| T01 LN Other, parapharyngeal (max) | 1.3 | 2.0 | 5.9 | 6.5 | 5.6 |
| T02 LN Cervical (max) | 0.8 | 1.4 | 5.1 | 4.7 | 4.2 |
| T03 LN Cervical (max) | 3.3 | 8.5 | 13.8 | 12.5 | 11.1 |
| T01 LN Other, parapharyngeal (peak) | 1.0 | 1.4 | 4.3 | 4.5 | 3.4 |
| T02 LN Cervical (peak) | 0.6 | 0.9 | 2.4 | 1.7 | 2.1 |
| T03 LN Cervical (peak) | 1.9 | 5.1 | 9.4 | 9.6 | 7.2 |

| | Hour of Imaging post injections | | | | |
|---|---|---|---|---|---|
| **Subject 8 (1.5 mg)** | **1.6** | **No Scan** | **24.4** | **50.1** | **121.3** |
| Spleen (mean) | 52.6 | No Scan | 52.6 | 47.6 | 42.5 |
| BM (L5-mean) | 9.0 | No Scan | 7.1 | 5.5 | 5.0 |
| Aorta (mean) | 2.4 | No Scan | 0.7 | 0.7 | 0.4 |
| Liver (mean) | 6.3 | No Scan | 7.0 | 5.3 | 5.2 |
| Muscle (mean) | 0.4 | No Scan | 0.3 | 0.5 | 0.5 |
| Left Kidney (mean) | 2.9 | No Scan | 4.4 | 3.4 | 3.9 |
| Lung (mean) | 0.7 | No Scan | 0.5 | 0.6 | 0.4 |
| T01 Spleen (max) | 57.2 | No Scan | 59.3 | 53.8 | 47.9 |
| T02 Spleen (max) | 56.4 | No Scan | 57.8 | 49.1 | 50.3 |
| T01 Spleen (peak) | 54.6 | No Scan | 56.0 | 49.1 | 45.3 |
| T02 Spleen (peak) | 54.9 | No Scan | 53.4 | 46.7 | 46.4 |

| | Hour of Imaging post injections | | | | |
|---|---|---|---|---|---|
| **Subject 13 (1.5 mg)** | **1.0** | **6.0** | **25.0** | **50.5** | **120.8** |
| Spleen (mean) | 22.3 | 14.4 | 10.8 | 11.3 | 10.3 |
| BM (L5-mean) | 6.3 | 3.9 | 2.0 | 2.2 | 2.5 |
| Aorta (mean) | 1.2 | 0.7 | 0.5 | 0.2 | 0.3 |
| Liver (mean) | 5.0 | 3.4 | 3.0 | 2.7 | 2.5 |
| Muscle (mean) | 0.5 | 0.4 | 0.5 | 0.4 | 0.6 |
| Left Kidney (mean) | 1.8 | 2.4 | 3.3 | 3.0 | 2.3 |
| Lung (mean) | 1.1 | 0.6 | 0.4 | 0.3 | 0.3 |
| T01 Other-spine (max) | 5.5 | 2.4 | 1.3 | 1.5 | 2.5 |
| T02 LN Pelvic (max) | 2.6 | 1.1 | 0.6 | 0.7 | 0.7 |
| T03 Other-subcutaneous nodule (max) | 0.2 | 0.1 | 0.1 | 0.1 | 0.1 |
| T01 Other-spine (peak) | 4.6 | 2.1 | 0.7 | 0.4 | 2.1 |
| T02 LN Pelvic (peak) | 2.4 | 0.8 | 0.5 | 0.4 | 0.3 |
| T03 Other-subcutaneous nodule (peak) | 0.2 | 0.1 | 0.1 | 0.1 | 0.0 |

| | Hour of Imaging post injections | | | | |
|---|---|---|---|---|---|
| **Subject 12 (1.5 mg)** | **1.6** | **6.3** | **24.1** | **48.3** | **119.1** |
| Spleen (mean) | 37.3 | 38.3 | 50.7 | 38.2 | 28.6 |
| BM (L5-mean) | 2.7 | 2.1 | 2.4 | 2.3 | 2.2 |
| Aorta (mean) | 3.7 | 2.5 | 1.0 | 0.7 | 0.9 |
| Liver (mean) | 4.5 | 4.6 | 4.9 | 4.1 | 4.0 |
| Muscle (mean) | 0.8 | 0.5 | 0.5 | 0.9 | 0.5 |
| Left Kidney (mean) | 4.2 | 7.5 | 9.8 | 8.9 | 6.7 |
| Lung (mean) | 1.3 | 1.0 | 0.8 | 0.8 | 0.6 |
| T01 Muscle (max) | 6.2 | 4.8 | ND | 3.4 | 3.9 |
| T02 Muscle (max) | 1.6 | 6.1 | 5.0 | 3.1 | 3.9 |
| T01 Muscle (peak) | 3.6 | 3.5 | ND | 2.6 | 2.7 |
| T02 Muscle (peak) | 1.2 | 3.5 | 3.2 | 2.1 | 2.6 |

| | Hour of Imaging post injections | | | | |
|---|---|---|---|---|---|
| **Subject 5 (5.0 mg)** | **2.2** | **6.1** | **21.2** | **48.7** | **117.4** |
| Spleen (mean) | 16.2 | 16.1 | 18.7 | 18.8 | 19.6 |
| BM (L5-mean) | 3.1 | 3.2 | 3.2 | 3.4 | 3.6 |
| Aorta (mean) | 7.5 | 6.4 | 2.5 | 0.9 | 0.5 |
| Liver (mean) | 4.6 | 4.7 | 4.9 | 5.1 | 4.9 |
| Muscle (mean) | 0.4 | 0.3 | 0.3 | 0.2 | 0.3 |
| Left Kidney (mean) | 5.1 | 6.9 | 10.1 | 10.0 | 9.0 |
| Lung (mean) | 0.6 | 1.0 | 0.5 | 0.5 | 0.4 |
| T01 Lung (max) | 3.6 | 1.6 | 1.1 | 0.6 | 0.4 |
| T01 Lung (peak) | 3.6 | 1.6 | 1.1 | 0.6 | 0.4 |

| | Hour of Imaging post injections | | | | |
|---|---|---|---|---|---|
| **Subject 6 (10.0 mg)** | **2.1** | **6.5** | **24.6** | **49.4** | **120.5** |
| Spleen (mean) | 11.0 | 10.7 | 10.9 | 12.2 | 15.3 |
| BM (L5-mean) | 1.6 | 1.8 | 1.4 | 1.4 | 1.5 |
| Aorta (mean) | 8.2 | 6.1 | 3.6 | 2.1 | 0.7 |
| Liver (mean) | 3.5 | 3.6 | 4.1 | 4.7 | 5.0 |
| Muscle (mean) | 0.5 | 0.7 | 0.2 | 0.2 | 0.2 |
| Left Kidney (mean) | 2.9 | 3.6 | 3.9 | 3.4 | 3.9 |
| Lung (mean) | 1.0 | 0.9 | 0.6 | 0.5 | 0.4 |
| T01 Lung (max) | 1.1 | 2.6 | 1.3 | 1.3 | 1.3 |
| T02 Lung (max) | 2.9 | 2.9 | 3.6 | 3.9 | 5.8 |
| T01 Lung (peak) | 1.1 | 2.1 | 1.1 | 1.1 | 1.1 |
| T02 Lung (peak) | 2.9 | 2.8 | 3.6 | 4.1 | 5.8 |

FIGs. 62B-62H show the time activity curves (TACs) for reference tissues as determined from the SUVmean of each subject enrolled in Stage I.

The evaluation of reference tissue uptake of $^{89}$Zr-Df-IAB22M2C including subjects enrolled in Stage I and II at 0.5 mg and 1.5 mg in particular is shown in Fig. 62I and Fig. 62J. The graphs are displayed as CD8+ TIL rich and non-CD8+ TIL rich reference organ tissues.

In most subjects, visualization of normal size nodes was seen throughout the neck, chest, abdomen, pelvis and inguinal canals in both cohorts of subjects. The frequency of nodal uptake is shown below in Fig. 62K. Nodal uptake in the neck and inguinal canals were the most frequently visualized sites of $^{89}$Zr-Df-IAB22M2C uptake while pelvic nodes

were the lowest frequency sites of visualized uptake.

**$^{89}$Zr-Df-IAB22M2C in Tumor Tissue**

As described herein, the measurements of co-localization of $^{89}$Zr-Df-IAB22M2C tracer uptake in tumor lesions involved the identification of up to 3 sites and/or regions of tumor observed on conventional imaging (CT and/or FDG PET) obtained prior to $^{89}$Zr-Df-IAB22M2C. These lesions are labeled as Target Lesions (T01, T02 or T03) in the following tables and figures.

The independent reader measured $^{89}$Zr-Df-IAB22M2C activity (SUVmax and SUVpeak) in tumor lesions noted on conventional imaging all 15 subjects.

Table 85.2 and Table 85.3 show the uptake values of each of the T0X lesions selected by the reader.

**TABLE 85.2: $^{89}$Zr-Df-IAB22M2C SUVmax In Tumor Lesions Identified On Conventional Imaging**

| Subject | Cancer Type | $^{89}$Zr-Df-IAB22M2C Dose | Lesion Location | 1.6 Hour | 6.2 Hour | 24.3 Hou r | 48.4 Hou r | 135.0 Hour |
|---|---|---|---|---|---|---|---|---|
| 1 | Melanoma | 0.2mg | T01 Muscle | 1.5 | 2.8 | 6.4 | 5.6 | 3.1 |
| 1 | Melanoma | 0.2mg | T02Muscle | 0.4 | 0.4 | 0.6 | 0.8 | 0.9 |
| 1 | Melanoma | 0.2mg | T03 Muscle | 0.5 | 0.3 | 0.3 | 0.5 | 1.2 |
| 2 | Hepatocellular | 0.5mg | T01 LN Other Portocaval | 1.5 | No Scan | 6.3 | 3.7 | 5.6 |
| 2 | Hepatocellular | 0.5mg | T02 LN Retroperitoneal | 0.6 | No Scan | 1.5 | 1.5 | 2.3 |
| 2 | Hepatocellular | 0.5mg | T03 Liver | 1.6 | No Scan | 3.5 | 2.8 | 4.2 |
| 4 | Non Small Cell Lung Cancer (NSCLC) | 1.5mg | T01 LN inguinal | 0.3 | No Scan | 0.4 | 0.5 | 0.2 |
| 4 | Non Small Cell Lung Cancer (NSCLC) | 1.5mg | T02 LN iniguinal | 0.5 | No Scan | 0.2 | 0.4 | 0.4 |
| 4 | Non Small Cell Lung Cancer (NSCLC) | 1.5mg | T03 LN inguinal | 0.3 | No Scan | 0.3 | 0.2 | 0.3 |
| 5 | Non Small Cell Lung Cancer (NSCLC) | 5.0mg | T01 Lung | 3.6 | 1.6 | 1.1 | 0.6 | 0.4 |
| 6 | Non Small Cell Lung Cancer (NSCLC) | 10.0m | T01 Lung | 1.1 | 2.6 | 1.3 | 1.3 | 1.3 |
| 6 | Non Small Cell Lung Cancer (NSCLC) | 10.0mg | T02 Lung | 2.9 | 2.9 | 3.6 | 3.9 | 5.8 |
| 7 | Melanoma | 1.5mg | T01 LN Other parapharyngeal | 1.3 | 2.0 | 5.9 | 6.5 | 5.6 |
| 7 | Melanoma | 1.5mg | T02 LN Cervical | 0.8 | 1 .4 | 5.1 | 4.7 | 4.2 |
| 7 | Melanoma | 1.5mg | T03 LN Cervical | 3.3 | 8.5 | 13.8 | 12.5 | 11.1 |
| 8 | Non Small Cell Lung Cancer (NSCLC) | 1.5mg | T01 Spleen | 57.2 | No Scan | 59.3 | 53.8 | 47.9 |
| 8 | Non Small Cell Lung Cancer (NSCLC) | 1.5mg | T02 Spleen | 56.4 | No Scan | 57.8 | 49.1 | 50.3 |
| 12 | Melanoma | 1.5mg | TOI Muscle | 6.2 | 4.8 | ND | 3.4 | 3.9 |
| 12 | Melanoma | 1.5mg | T02 Muscle | 1.6 | 6.1 | 5.0 | 3.1 | 3.9 |

(continued)

| Subject | Cancer Type | $^{89}$Zr-Df-IAB22M2C Dose | Lesion Location | 1.6 Hour | 6.2 Hour | 24.3 Hour | 48.4 Hour | 135.0 Hour |
|---|---|---|---|---|---|---|---|---|
| 3 | Non Small Cell Lung Cancer (NSCLC) | 1.0mg | T01 LN Cervical | 0.9 | 0.8 | 0.7 | 0.6 | 0.7 |
| 3 | Non Small Cell Lung Cancer (NSCLC) | 1.0mg | T02 Liver | 6.1 | 8.2 | 3.6 | 5.1 | 4.2 |
| 3 | Non Small Cell Lung Cancer (NSCLC) | 1.0mg | T03 Lung | 1.6 | 0.9 | 0.6 | 0.7 | 1.0 |
| 9 | Melanoma | 1.5mg | T01 LN Pelvic | 1.2 | 0.8 | 1.2 | 1.5 | 2.5 |
| 9 | Melanoma | 1.5mg | T02 LN Pelvic | 1.7 | 2.3 | 3.9 | 2.9 | 2.3 |
| 10 | Melanoma | 0.5mg | T01 LN inguinal | 0.7 | 0.8 | 1.6 | 1.7 | 2.0 |
| 13 | Melanoma | 1.5mg | T01 Other spine | 5.5 | 2.4 | 1.3 | 1.5 | 2.5 |
| 13 | Melanoma | 1.5mg | T02 LN Pelvic | 2.6 | 1.1 | 0.6 | 0.7 | 0.7 |
| 13 | Melanoma | 1.5mg | T03 Other subcutaneous nodule | 0.2 | 0.1 | 0.1 | 0.1 | 0.1 |
| 15 | Non Small Cell Lung Cancer (NSCLC) | 0.5mg | T01 LN Mediastinal | 1.8 | No Scan | 1.0 | 1.1 | 1.7 |
| 15 | Non Small Cell Lung Cancer (NSCLC) | 0.5mg | T02 LN Mediastinal | 2.0 | No Scan | 1.2 | 1.3 | 1.0 |
| 15 | Non Small Cell Lung Cancer (NSCLC) | 0.5mg | T02 LN Mediastinal | 2.3 | No Scan | 2.2 | 1.8 | 2.3 |
| 11 | Melanoma | 0.5mg | T01 Lung | 1.6 | No Scan | 0.5 | 1.0 | 1.8 |
| 11 | Melanoma | 0.5mg | T02 Lung | 0.8 | No Scan | 0.4 | 0.8 | 1.8 |
| 11 | Melanoma | 0.5mg | T03 Liver | 4.7 | No Scan | 4.1 | 8.4 | 8.3 |
| 14 | Melanoma | 0.5mg | T01 LN Cervical | 0.4 | No Scan | 0.4 | 0.5 | 1.3 |
| 14 | Melanoma | 0.5mg | T02 LN Cervical | 0.3 | No Scan | 0.9 | 0.6 | ND |
| 14 | Melanoma | 0.5mg | T03 LN Axillary | 1.9 | No Scan | 3.7 | 12.8 | 10.0 |

**TABLE 85.3: $^{89}$Zr-Df-IAB22M2C SUVpeak In Tumor Lesions Identified On Conventional Imaging**

| Subject | Cancer Type | $^{89}$Zr-Df-IAB22M2C Dose | Lesion Location | 1.6 Hour | 6.2 Hour | 24.3 Hour | 48.4 Hour | 135.0 Hour |
|---|---|---|---|---|---|---|---|---|
| 1 | Melanoma | 0.2mg | T01 Muscle | 0.7 | 1.2 | 2.3 | 2.1 | 1.4 |
| 1 | Melanoma | 0.2mg | T02Muscle | 0.2 | 0.4 | 0.3 | 0.4 | 0.4 |
| 1 | Melanoma | 0.2mg | T03 Muscle | 0.3 | 0.3 | 0.2 | 0.3 | 0.4 |

(continued)

| Subject | Cancer Type | $^{89}$Zr-Df-IAB22M2C Dose | Lesion Location | 1.6 Hour | 6.2 Hour | 24.3 Hour | 48.4 Hour | 135.0 Hour |
|---|---|---|---|---|---|---|---|---|
| 2 | Hepatocellular | 0.5mg | T01 LN Other Portocaval | 1.2 | No Scan | 4.4 | 2.9 | 3.4 |
| 2 | Hepatocellular | 0.5mg | T02 LN Retroperitoneal | 0.5 | No Scan | 1.2 | 0.7 | 1.5 |
| 2 | Hepatocellular | 0.5mg | T03 Liver | 1.5 | No Scan | 3.5 | 2.6 | 3.9 |
| 4 | Non Small Cell Lung Cancer (NSCLC) | 1.5mg | T01 LN inguinal | 0.2 | No Scan | 0.2 | 0.2 | 0.2 |
| 4 | Non Small Cell Lung Cancer (NSCLC) | 1.5mg | T02 LN iniguinal | 0.3 | No Scan | 0.1 | 0.2 | 0.2 |
| 4 | Non Small Cell Lung Cancer (NSCLC) | 1.5mg | T03 LN inguinal | 0.0 | No Scan | 0.2 | 0.2 | 0.1 |
| 5 | Non Small Cell Lung Cancer (NSCLC) | 5.0mg | T01 Lung | 3.1 | 1.4 | 1.1 | 0.5 | 0.3 |
| 6 | Non Small Cell Lung Cancer (NSCLC) | 10.0mg | T01 Lung | 1.1 | 2.1 | 1.1 | 1.1 | 1.1 |
| 6 | Non Small Cell Lung Cancer (NSCLC) | 10.0mg | T02 Lung | 2.9 | 2.8 | 3.6 | 4.1 | 5.8 |
| 7 | Melanoma | 1.5mg | T01 LN Other parapharyngeal | 1.0 | 1.4 | 4.3 | 4.5 | 3.4 |
| 7 | Melanoma | 1.5mg | T02 LN Cervical | 0.6 | 0.9 | 2.4 | 1.7 | 2.1 |
| 7 | Melanoma | 1.5mg | T03 LN Cervical | 1.9 | 5.1 | 9.4 | 9.6 | 7.2 |
| 8 | Non Small Cell Lung Cancer (NSCLC) | 1.5mg | T01 Spleen | 54.6 | No Scan | 56.0 | 49.1 | 45.3 |
| 8 | Non Small Cell Lung Cancer (NSCLC) | 1.5mg | T02 Spleen | 54.9 | No Scan | 53.4 | 46.7 | 46.4 |
| 12 | Melanoma | 1.5mg | TOI Muscle | 3.6 | 3.5 | ND | 2.6 | 2.7 |
| 12 | Melanoma | 1.5mg | T02 Muscle | 1.2 | 3.5 | 3.2 | 2.1 | 2.6 |
| 3 | Non Small Cell Lung Cancer (NSCLC) | 1.0mg | T01 LN Cervical | 0.7 | 0.5 | 0.4 | 0.4 | 0.4 |
| 3 | Non Small Cell Lung Cancer (NSCLC) | 1.0mg | T02 Liver | 6.1 | 7.2 | 3.8 | 4.6 | 3.7 |
| 3 | Non Small Cell Lung Cancer (NSCLC) | 1.0mg | T03 Lung | 1.1 | 0.7 | 0.4 | 0.4 | 0.6 |
| 9 | Melanoma | 1.5mg | T01 LN Pelvic | 0.9 | 0.6 | 0.9 | 1.2 | 1.1 |
| 9 | Melanoma | 1.5mg | T02 LN Pelvic | 1.5 | 1.7 | 2.7 | 2.0 | 1.5 |
| 10 | Melanoma | 0.5mg | T01 LN inguinal | 0.3 | 0.6 | 1.1 | 1.3 | 1.4 |
| 13 | Melanoma | 1.5mg | T01 Other spine | 4.6 | 2.1 | 0.7 | 0.4 | 2.1 |
| 13 | Melanoma | 1.5mg | T02 LN Pelvic | 2.4 | 0.8 | 0.5 | 0.4 | 0.3 |

(continued)

| Subject | Cancer Type | $^{89}$Zr-Df-IAB22M2C Dose | Lesion Location | 1.6 Hour | 6.2 Hour | 24.3 Hour | 48.4 Hour | 135.0 Hour |
|---|---|---|---|---|---|---|---|---|
| 13 | Melanoma | 1.5mg | T03 Other subcutaneous nodule | 0.2 | 0.1 | 0.1 | 0.1 | 0.0 |
| 15 | Non Small Cell Lung Cancer (NSCLC) | 0.5mg | T01 LN Mediastinal | 1.6 | No Scan | 0.8 | 0.7 | 0.9 |
| 15 | Non Small Cell Lung Cancer (NSCLC) | 0.5mg | T02 LN Mediastinal | 1.9 | No Scan | 1.0 | 1.0 | 0.7 |
| 15 | Non Small Cell Lung Cancer (NSCLC) | 0.5mg | T02 LN Mediastinal | 1.9 | No Scan | 1.5 | 1.4 | 1.6 |
| 11 | Melanoma | 0.5mg | T01 Lung | 0.9 | No Scan | 0.3 | 0.5 | 0.9 |
| 11 | Melanoma | 0.5mg | T02 Lung | 0.7 | No Scan | 0.4 | 0.4 | 0.0 |
| 11 | Melanoma | 0.5mg | T03 Liver | 4.2 | No Scan | 3.2 | 4.5 | 4.4 |
| 14 | Melanoma | 0.5mg | T01 LN Cervical | 0.3 | No Scan | 0.4 | 0.4 | 0.5 |
| 14 | Melanoma | 0.5mg | T02 LN Cervical | 0.2 | No Scan | 0.5 | 0.3 | ND |
| 14 | Melanoma | 0.5mg | T03 LN Axillary | 0.0 | No Scan | 1.9 | 6.9 | 5.4 |

A plot of the distribution of SUVmax and SUVpeak uptake values at each of the average time points of the target tumor lesions is shown in FIG. 62L and FIG. 62M, respectively. Table 85.4 provides a summary of $^{89}$Zr-Df-IAB22M2C nodal lesion to aorta uptake over time

## TABLE 85.4: Summary Of $^{89}$Zr-Df-IAB22M2C Nodal Lesion To Aorta Uptake Over Time

| Summary of $^{89}$Zr-Df-IAB22M2C Tumor to Aorta Uptake in Non Nodal Lesion | | | | | |
|---|---|---|---|---|---|
| Parameter | 1.6 hr | 6.2 hr | 24.3 hr | 48.4 hr | 135.0 hr |
| Mean | 3.8 | 2.5 | 12.4 | 12.3 | 20.5 |
| Median | 0.9 | 1.1 | 1.5 | 3.4 | 4.8 |
| Minimum | 0.1 | 0.1 | 0.2 | 0.5 | 0.3 |
| Quartile 25% | 0.5 | 0.5 | 0.6 | 1.2 | 2.3 |
| Quartile 75% | 2.5 | 2.7 | 7.0 | 7.9 | 12.9 |
| Maximum | 23.8 | 13.7 | 84.7 | 76.9 | 125.8 |
| Standard Deviation | 7.3 | 3.8 | 27.0 | 22.8 | 38.4 |
| 95% CI | 3.4 | 2.1 | 12.8 | 10.5 | 17.8 |
| Count | 18 | 12 | 17 | 18 | 18 |

Descriptive statistics of $^{89}$Zr-Df-IAB22M2C Tumor: Aorta in Non-Nodal tumor lesions.

IE has conducted a central blinded independent review of subjects enrolled in the Phase I study of 89Zr-Df-IAB22M2C PET/CT scans in selected subjects with metastatic solid malignancies. The study was performed as a single reader paradigm. The imaging objectives of this study was to determine the feasibility of using 89Zr-Df-IAB22M2C as a PET agent for the detection of CD8+ TILs populations within reference organ tissues and tumors. The analysis involved both qualitative and quantitative evaluation of the distribution of 89Zr-Df-IAB22M2C radiotracer in lymphoid organs (spleen, bone marrow and lymph nodes) and tumor.

The specific quantitative evaluation of PET/CT scans focused mainly upon SUV analysis as a measure of CD8+ TIL activity. In addition, the API of $^{89}$Zr-Df-IAB22M2C along with the best timing for PET/CT scan acquisition following $^{89}$Zr-Df-IAB22M2C injection was derived from this dataset.

## Study Protocol Design

Stage I was a rapid dose escalation phase. A single subject was enrolled at one of 6 unlabeled API protein doses: 0.2 mg, 0.5 mg, 1.0 mg, 1.5 mg, 5.0 mg and 10.0 mg. The purpose of this phase was to test a range of protein doses for scan optimization based upon saturation of CD8+ TIL sink in CD8+ rich organ tissues while preserving low background activity in non-CD8+ rich organ tissues and to evaluate the feasibility of detecting tumor colocalization on the $^{89}$Zr-Df-IAB22M2C PET/CT scans.

Stage II was a dose expansion phase including an additional 9 subjects enrolled at either 0.5 mg (n=4) and 1.5 mg (n =5) $^{89}$Zr-Df-IAB22M2C API protein doses. The purpose of this phase was to further define the behavior of $^{89}$Zr-Df-IAB22M2C uptake in lymphoid and non-lymphoid reference organ tissues (both CD8+ rich and non CD8+ rich organs) as well as to investigate tumor colocalization.

## Stage I Results and Rationale

The results from the Stage I subjects demonstrated robust uptake of $^{89}$Zr-Df-IAB22M2C activity in CD8+ TIL rich organ tissues at all protein dose levels tested and relatively low uptake in the non-CD8+ TIL rich organ tissues. Based on the evaluation of the SUVmean[1] in reference organ tissues (Figs. 62B-62I) the following observations were made:

1. There was strong visualization of $^{89}$Zr-Df-IAB22M2C activity in the spleen and bone marrow at all protein dose levels with variable uptake in normal size lymph nodes. While the 0.2 mg $^{89}$Zr-Df-IAB22M2C API dose showed the highest number of normal size lymph nodes stations other dose levels showed lymph node uptake.
2. There was relatively low uptake of $^{89}$Zr-Df-IAB22M2C in non-CD8+ rich organ tissues compared to CD8+ rich organ tissues at all API doses tested suggesting a favorable target to background environment for the potential detection of tumor co-localization of $^{89}$Zr-Df-IAB22M2C.
3. The highest uptake of $^{89}$Zr-Df-IAB22M2C was noted in those organ tissues known to be rich in CD8+ TILs with spleen > bone marrow > lymph node uptake at all protein dose levels.[2]
4. The signal of $^{89}$Zr-Df-IAB22M2C in non-CD8+ TIL rich organ tissue had comparatively lower SUVmean values with the liver containing the highest activity in this class of reference organ tissues while the lowest signal was noted in lung and spinal muscles.
5. Increasing doses of $^{89}$Zr-Df-IAB22M2C API led to lower SUVmean values in CD8+ TIL rich reference organ tissues while having only modest effects in uptake in non-CD8+ TIL rich organ tissues.
6. TACs showed a change in $^{89}$Zr-Df-IAB22M2C uptake over time in almost all organ tissues which may allow the examination of T Cell Trafficking in the future

   a. The TACs for CD8+ TIL rich organ tissues decreased over time in subjects from 0.2 mg to 1.5 mg API doses but increased over time at 5.0 mg and 10.0 mg.

1 The use of SUVmean is an approach to measure reference tissue activity. The benefits of SUVmean over SUVmax or peak measurements in reference tissues is related to a more statistically robust signal within an organ system that is expected to have homogenous activity compared to SUVpeak and SUVmax.

2 The count recovery coefficients for each of the PET/CT scanners were unknown to allow for correction of partial volume and count recovery effects. In other words, small anatomic structures (e.g. lymph nodes) may have a lower SUVmean than their larger organ counterparts despite having similar CD8+ TIL density.

   b. The TACs for non-CD8+ TIL rich organ tissues were more variable but there was a trend towards increasing SUVmean values over time at almost all protein dose levels.

7. Aorta (blood pool) clearance was generally rapid but delayed with increasing API doses of tracer.

These results suggest that $^{89}$Zr-Df-IAB22M2C is following typical receptor saturation behavior characteristics with higher doses of unlabeled $^{89}$Zr-Df-IAB22M2C API leading to lower $^{89}$Zr-Df-IAB22M2C uptake due to more binding, occupancy and saturation of the CD8+ TIL receptor (so-called saturation of the CD8+ sinks) along with a shift in the distribution of $^{89}$Zr-Df-IAB22M2C agent towards non-CD8+ rich organ tissue (i.e., higher uptake in organs of clearance such as liver) and slower clearance from the blood pool. The findings are also in keeping with the reported results from pre-clinical models.

In addition, a change in the TAC pattern of $^{89}$Zr-Df-IAB22M2C uptake was noted at doses > 5.0 mg. For example, the TACs for API doses less than 5.0 mg showed a reduction in $^{89}$Zr-Df-IAB22M2C activity (clearance) over time from CD8+ rich organ reference tissues while API doses 5.0 mg or higher showed increasing accumulation of $^{89}$Zr-Df-IAB22M2C over time. This suggested that there was no more benefit derived from CD8+ visualization in lymphoid organ tissues at these higher dose levels. Finally, since 0.5 mg and 1.5 mg dose levels had much less global uptake of $^{89}$Zr-Df-IAB22M2C in lymphoid rich organ tissues (especially lymph nodes) than at 0.2 mg dose levels and since there was no real targeting of tumor lesions in the 1.0 mg dose levels the sponsor elected to focus upon the 0.5 mg and 1.5 mg $^{89}$Zr-Df-IAB22M2C API dose levels for further investigation in stage II of the protocol.

**Results of Localization of 89Zr-Df-IAB22M2C Reference Tissues and Tumor Lesions in Dose Expansion**

The $^{89}$Zr-Df-IAB22M2C uptake in organ tissues was very similar regardless of enrollment in stage I and II subjects at 0.5 mg and 1.5 mg (Fig. 62I and Fig. 62.J). This suggests that $^{89}$Zr-Df- IAB22M2C uptake produces a stable signal for potential CD8+ TIL detection in these reference organ tissues. In addition, visualization of $^{89}$Zr-Df-IAB22M2C uptake in normal nodes was noted in many subjects. The frequency of identifying normal nodal stations varied by anatomic location (FIG. 62K). Normal size nodes were noted more frequently in the head/neck and inguinal canals than in the chest, abdomen and pelvis. Neck and inguinal canal nodes are common sites for exposure to environmental, infectious and inflammatory processes compared to other sites leading to a greater opportunity to expand their CD8+ TIL populations when exposed to foreign antigens. In addition, their relative closer proximity to the PET camera detectors and the thinner surrounding soft tissues (i.e., attenuation effects) may make them more readily detectable than deeper normal lymph nodes.

The visualization of normal size lymph nodes using $^{89}$Zr-Df-IAB22M2C and lack of morphology findings on conventional imaging or 18F-FDG-PET uptake to suggest metastatic disease does not necessarily rule out entirely that there were no metastatic tumor deposits at these nodal stations. There were no planned biopsies from subjects enrolled in this trial in either cohort to confirm the benign nature of these nodes. However, a trial using $^{89}$Zr-Df-IAB22M2C in normal subjects would address this issue more directly as it would be anticipated that both the detection and frequency of nodal station visualization would be similar in a healthy normal population compared to subjects enrolled in our study.

**Evaluation of $^{89}$Zr-Df-IAB22M2C Tumor Co-localization**

The results of tumor co-localization analysis of $^{89}$Zr-Df-IAB22M2C PET/CT signal demonstrated the following:

1. Signal was detected in tumor lesions in 9 out of 15 subjects. Tumor co-localization was seen in 1 out of 1 subjects enrolled at 0.2 mg API dose, 4 out of 5 subjects enrolled at 0.5 mg API dose, 0 subjects enrolled at 1.0 mg API dose, 3 out of 6 subjects enrolled at 1.5 mg API dose, 0 subjects enrolled at 5.0 mg API dose, and 1 subject enrolled at 10.0 mg API dose cohort. All uptake in lesions was considered homogeneous in nature.
2. SUV (max and peak) in tumors visualized on PET ranged from ~ 1.0 to 59. There was greater $^{89}$Zr-Df-IAB22M2C signal in 1.5 mg API dose cohort lesions than in 0.5 mg API dose cohort lesions even when excluding outlier lesions (splenic lesions from one subject in the 1.5 mg API cohort).
3. The TACs showed increasing $^{89}$Zr-Df-IAB22M2C uptake over time when evaluating all tumors from all subjects at all $^{89}$Zr-Df-IAB22M2C API dose levels with general flattening of the TAC between 24-48hrs.
4. When $^{89}$Zr-Df-IAB22M2C signal was stratified by tumor types, melanoma and NSCLC tumor lesions had similar $^{89}$Zr-Df-IAB22M2C uptake values and TACs. One subject with HCC had higher SUV values compared to the other histologies.
5. The mean $^{89}$Zr-Df-IAB22M2C signal was generally less in non-nodal than nodal disease.
7. There was a variable range of signal in tumor lesions across the different API doses. This may be related to the variation in lesion type, treatment history, tumor metastatic burden and expected variability in CD8+ TIL density across subjects and lesions.
8. The size and type of lesion may influence the $^{89}$Zr-IAB22M2C signal. For example, uptake of $^{89}$Zr-Df-IAB22M2C in nodal disease tends to show higher signal in smaller lesions (<10 mm) than larger lesions (>30 mm) and reaches

a plateau faster in smaller lesions based upon the TACs. However, in non-nodal disease there is higher signal in larger lesions but with a similar plateau of signal to nodal disease. It is interesting to note that if splenic lesions are excluded from the size analysis a similar pattern of higher uptake in larger lesions is still seen at 0.5 mg API doses but not at 1.5 mg $^{89}$Zr-Df-IAB22M2C doses. As such, these results should be interpreted with caution. Further investigation into these observations in next phase trials may be warranted.

9. $^{89}$Zr-Df-IAB22M2C signal in lesions located in various anatomic show the greatest uptake in liver lesions compared to other anatomic sites (excluding the two splenic lesions). How much contribution of the signal within liver tumors are due to a "spill-in" effect of counts from partial volume effects of normal higher $^{89}$Zr-Df-IAB22M2C liver activity compared to lower normal uptake in lung is unknown

Taken together, these results demonstrate that co-localization of $^{89}$Zr-Df-IAB22M2C in tumor lesions is feasible in most subjects. Furthermore, tumor co-localization is noted at both 0.5 mg and 1.5 mg API dose levels without a clear advantage of one dose over the other and similar plateau of signal between 24-48hrs in most cases.

**Tumor: Reference (Aorta-blood pool) Activity**

Measured $^{89}$Zr-Df-IAB22M2C PET signal is composed of both non-specific uptake (dependent on the tissue blood volume fraction, as well as other tissue characteristics such as enhanced permeability and retention) and targeted CD8+ receptor-mediated specific uptake. Distinguishing between these two contributions to the PET signal can be involved. However, evaluating the ratio of tumor uptake to aorta activity can help to reduce the influence of non-specific uptake by normalizing to the arterial input function. Lymph node disease and non-nodal disease demonstrated similar TACs but with greater $^{89}$Zr-Df-IAB22M2C activity over time, especially in nodal lesions. These results included all tumor lesions regardless of size, API dose levels, or tumor type. These results suggest that specific CD8+ TIL binding is occurring at tumor sites since non-specific uptake should parallel Aortic (arterial input function) activity as the agent clears from the body.

**Summary Results of 89Zr-Df-IAB22M2C Uptake in Tumor Lesions and Reference Tissues**

The above findings show that of all $^{89}$Zr-Df-IAB22M2C API doses tested from 0.2 mg to 10.0 mg in both reference organ tissues with CD8+ TIL rich populations and tumor lesions can be seen. Results from testing 5.0 mg and 10.0 mg dose in stage I showed unfavorable conditions for maintaining high target to background levels given the increasing uptake in non-CD8+ TIL rich tissues at these levels, delayed aortic clearance and potential blocking of the CD8+ TIL receptors in low T cell density population in tumors. In addition, 0.2 mg API doses was felt to be too little unlabeled API due to both higher lymphoid tissue uptake and nodal visualization. The 1.0 mg dose showed expected uptake in spleen and bone marrow but no tumor visualization nor significant nodal uptake. Dose expansion (Stage II) using 0.5 mg and 1.5 mg API doses was done. Within this stage, the 0.5 mg and 1.5 mg protein dose showed 1) favorable signal within lymphoid and non-CD8+ rich reference organ tissues, 2) favorable TACS by 24hrs both by SUV analysis and Tumor:Aorta ratios and 3) tumor colocalization in both nodal and non-nodal lesions. The 1.5 mg API dose level did show higher signal compared to 0.5 mg API dose. Based upon these results, 1.5 mg of $^{89}$Zr-Df- IAB22M2C API and acquisition of images at 24hrs was a suitable option for RP2P dose. The 0.5 mg dose can also be an acceptable alternative for imaging.

A $^{89}$Zr-Df-IAB22M2C API dose of 1.5 mg has shown favorable characteristics in terms of visualization of the lymphoid organs and tumor lesions while maintaining low non-CD8+ rich organ tissue background activity. By 24hrs post-injection, both nodal and non-nodal tumor lesions were visualized in many subjects with suitable background activity providing evidence for the recommendation of using this time point for Phase II studies. The 0.5 mg dose may also be an acceptable alternative for $^{89}$Zr-Df-IAB22M2C PET/CT imaging. However, additional information from radiodosimetry, biodistribution studies, and safety signal should be considered when making the final selection of the RP2D. The results from the Phase I study of $^{89}$Zr-Df-IAB22M2C PET/CT scans in selected subjects with metastatic solid malignancies contained within this report has shown the tremendous value of $^{89}$Zr-Df-IAB22M2C in visualizing the CD8+ TIL immune system.

Further results from the above phase I study are shown in FIG. 62N, which generally depicts a PET scan showing the ability of an antigen binding construct, labeled with $^{89}$Zr, to bind to a target (CD8), within 6 hours or 6 days of administration.

A summary of the results of the phase I data, in terms of the ability to image various tumors on various treatments and obtain SUV values and from that and a relative review of the background signal, determine the positive or negative infiltration status is provided in table 85.5.

TABLE 85.5

| Subject # | Tumour | Treatment | Protein dose of CD8 tracer (~3 mCi) in mg | Lesion number and lesion Location | SUV max lesion (CD8 tracer at 18-30h) | Relevant tissue background for assessment | Assessment of Tumor (or Lymph Node) CD8 + ve infiltration status* |
|---|---|---|---|---|---|---|---|
| 1 | Melanoma | Long term CPI (Ipilimumab/ Nivolumab treatment (2 Years): Pembrolizumab: Ongoing) | 0.2 | T01 Muscle | 6.4 | Aorta or L3 | Positive |
| 2 | Metastatic hepatocellul ar carcinoma | Short term CPI (Nivolumab: Ongoing CPI treatment for 2 weeks prior to scan) | 0.5 | T03 Liver | 3.5 | Aorta or L3 | Positive |
| 3 | NSCLC | Not on Treatment | 1.0 | T02 Liver | 3.6 | Normal liver | Negative |
| 4 | NSCLC | Nivolumab/Ipilimumab | 1.5 | T01 LN Inguinal | 0.4 | Aorta or L3 | Negative |
| 5 | NSCLC | Ipilimumab/Nivolumab (Prior CPI treatment not continued) | 5.0 | T01 Lung | 1.1 | Aorta or L3 | Negative |
| 6 | NSCLC | Nivolumab (Ongoing CPI treatment from 2 years prior to scan) | 10.0 | T02 Lung | 3.6 | Aorta or L3 | Positive |
| 7 | Melanoma | Pembrolizumab (Ongoing treatment from 1 year prior to scan) | 1.5 | T01 LN, parapharynge al | 5.9 | Aorta or L3 | Positive |
| 8 | NSCLC | Carboplatin/Pemetrexed (not ongoing); Pembrolizumab (ongoing from 8 approximately 4 months prior to scan) | 1.5 | T01 Spleen | 59.3 | Normal spleen | Negative |
| 9 | Melanoma | Interferonx 10m; Ipilimumab/ Nivolumab; Dabrafenib/Trametinib ongoing for approximately 7 months | 1.5 | T01 LN Pelvic | 1.2 | Aorta or L3 | Positive |
| 10 | Melanoma | Not on Treatment | 0.5 | T01 LN Inguinal | 1.6 | Aorta or L3 | Positive |
| 11 | Melanoma | Pembrolizumab (Ongoing from 5 Weeks prior to scan) | 0.5 | T03 Liver | 4.1 | Normal Liver | Positive |

(continued)

| Subject # | Tumour | Treatment | Protein dose of CD8 tracer (~3 mCi) in mg | Lesion number and lesion Location | SUV max lesion (CD8 tracer at 18-30h) | Relevant tissue background for assessment | Assessment of Tumor (or Lymph Node) CD8 + ve infiltration status* |
|---|---|---|---|---|---|---|---|
| 12 | Melanoma | Ipilimumab/Nivolumab x1m (For 2 yrs, and stopped 2 yrs prior to scan) | 1.5 | T01 Muscle | 5.0 | Aorta or L3 | Positive |
| 13 | Melanoma | Nivolumab (Ongoing from last 4 months); Dabrafenib/Trametinib (stopped 4 months prior to scan) | 1.5 | T01 Other-Spine | 1.3 | Aorta or L3 | Negative |
| 14 | Melanoma | Pembrolizumab (ongoing, initiated approximately 1 month prior to scan) | 0.5 | T01 LN Cervical | 0.4 | Aorta or L3 | Positive |
| 15 | NSCLC | Pembrolizumab/Carbopl atin (Ongoing treatment from last one month prior to scan) | 0.5 | T01 LN Mediastinal | 1.0 | Aorta or L3 | Negative |

**Exanple 86-Phase II**

$^{89}Zr$-Df-IAB22M2C is a Zirconium labeled CD8 minibody which has retained its specificity and high affinity for the CD8+ T cell receptor, accelerated blood clearance, inert immunogenicity, and effector function. The results from IAB's First-in-Human Phase I trial (Trial 2015-22M2C- 01-2) has demonstrated a favorable safety profile and biodistribution while retaining imaging characteristics for the visualization of tumor lesions and lymphoid organs and maintaining low non-CD8+ T Cell organ tissue background activity. Although the phase I (1) trial detected the co-localization of $^{89}Zr$-Df-IAB22M2C in tumor lesions as early as 1-2 hrs. P.I., final analysis of the data concluded that whole body PET imaging acquisition at 24hrs P.I. using 3.0 mCi $^{89}Zr$-Df-IAB22M2C with 0.5-1.5 mg of API will provide the valuable imaging conditions for the pursuing the objectives for the Phase II trial.

**Phase II Study Objectives and Outcome Measures:**

The Phase II study IAB-CD8-201 was an open label, multi-dose study of $^{89}Zr$-Df-IAB22M2C for positron emission tomography/computed tomography (PET/CT) in patients with metastatic solid tumors. In brief, the specific image-based primary and secondary objectives of this trial included:

1. Primary Outcome measures
Analyze $^{89}Zr$-Df-IAB22M2C uptake in biopsied tumors as determined by SUV-based quantitative measures (e.g., SUVmax, SUVpeak, SUVmean, and Tumor: Reference ratio) with CD8+ TIL measurements determined by immunohistochemistry (IHC) from a biopsy sample.
2. Secondary outcome measures

- Assessment of $^{89}Zr$-Df-IAB22M2C uptake at Visit 3(baseline) and at Visit 7 (on- treatment) in tumor lesions, lymphoid organs, and reference tissues, including T-cell rich tissues
- Measurement of potential change in $^{89}Zr$-Df-IAB22M2C uptake in tumor lesions between baseline and on-treatment and reference tissues, including T-cell rich tissues.

The $^{89}Zr$-Df-IAB22M2C PET/CT scan analysis was performed via independent central imaging review. The quantitative IHC analysis and the qualitative pathology reads, and statistical analysis using the measurements performed were done independently as well.

**Imaging Schedule**

**PET/CT and Image Guide Biopsy Scan Data Acquisition for Image Analysis**

Whole Body $^{89}Zr$-Df-IAB22M2C PET/CT imaging data was acquired in up to three different time points P.I. as outlined in imaging visit schedule provided in the Table 86.1. The mandatory $^{89}Zr$-Df-IAB22M2C PET/CT images to be acquired included Visit 3 (referred to as $PET_{Baseline}$) and Visit 7 (referred to as $PET_{Tx}$) at 24 $\pm$ 3 hours P.I. of 3.0 $\pm$ 20% mCi of $^{89}Zr$- Df-IAB22M2C with 1.5mg of API IAB22M2C. The image acquisition at Visit 5 ($PET_{day5}$, an optional scan) can be performed 5-7 days after the commencement of IOT (Visit 4). All the images in this example were obtained as per the scanner specific acquisition parameters provided by NMCTG Scanner Report (2), catered for every scanner at every site and in accordance with the IM. Acquired images were transferred for further analysis as provided in the IRC (3). To be included in the clinical trial analysis, a subject must have had at least one acceptable $^{89}Zr$-Df-IAB22M2C PET scan.

**TABLE 86.1:**

| Imaging Visit Schedule As Published In The Phase-II Protocol # IAB-CD8-201 | | | |
|---|---|---|---|
| **Visit** | **Type** | **Schedule** | **Imaging** |
| Visit 1-screening image guided biopsy scan | Biopsy: Fresh[1] Tumor Biopsy from a RECIST measurable[2] lesion (Excluding cutaneous lesion) | -28 to -7 days prior to $1^{st}$ infusion of $^{89}$Zr- Df-IAB22M2C (Visit 2 days 1) | Guide Biopsy Imaging: CT<br>• The pre-planning biopsy CT which contains slices through the target lesion to be biopsied<br>• The CT imaging which contains the biopsy needle within the target lesion<br>• CT biopsy guided imaging is to be performed per site standard of care.<br>• In-case of image guided biopsy scan with needle placement is not available, identify the lesion on CT imaging with an annotation to show the location of the lesion. |
| **Visit** | **Type** | **Schedule** | **Imaging** |
| | | | ◦ An ultrasound image can be submitted demonstrating needle placement and identifying the location of the lesion on CT with an arrow mark<br>◦ An MRI image can be submitted identifying the location of lesion with an arrow mark |
| Visit 3- PET/CT scan ($PET_{baseline}$) | $^{89}$Zr-Df-IAB22M2C PET/CT scan | 24±3 hrs. after injection of $^{89}$Zr- Df-IAB22M2C at Visit 2 | Whole Body $^{89}$Zr-Df-IAB22M2C PET/CT scan |
| Visit 5- PET/CT scan ($PET_{day5}$) | $^{89}$Zr-Df-IAB22M2C PET/CT optional scan | 5-7 days after the commencement of IOT (Visit 4) | Whole Body $^{89}$Zr-Df-IAB22M2C PET/CT scan |
| Visit 7-PET/CT scan ($PET_{TX}$) | $^{89}$Zr-Df-IAB22M2C PET/CT optional scan | 24±3 hrs. after injection of $^{89}$Zr- Df-IAB22M2C at Visit 6 | Whole Body $^{89}$Zr-Df-IAB22M2C PET/CT scan |

**Image Acquisition Parameters**

**Image Acquisition Parameters and Analysis Methodology**

The parameter for image acquisition for the ImaginAb Phase-II protocol #IAB-CD8-201 and used with the 5 patients, are provided within table 86.2 as follows:

**TABLE 86.2: General Image Acquisition Parameters.**

| IMPORTANT: Please use the scanner parameters outlined in the site-specific report generated by NMCTG | |
|---|---|
| **Radiopharmaceutical** | $^{89}$Zr-Df-IAB22M2C |
| **Dose[1]** | 3.0 (±20%) mCi of $^{89}$Zr-Df-IAB22M2C administered over 5-10 minutes I.V. |

(continued)

| IMPORTANT: Please use the scanner parameters outlined in the site-specific report generated by NMCTG | |
|---|---|
| **Mode** | 2D, 3D list-mode, or TOF When possible, acquisition should be acquired in List Mode. |
| **Patient Position** | Supine with arms down by side. Keep position consistent between CT and PET acquisitions. |
| **Scan Location/Coverage** | Vertex of Skull through feet including all areas of known or suspected disease |
| **Transmission Scan** | • 140 kVp<br>• mAs:<br>◦ 24(±3) hrs. p.i. scans: low dose CT (40-80 mAs)<br>◦ Optional visit 5 post infusion scan: low dose CT (10 mAs)<br>• 0.8 sec per CT rotation<br>• 3-5 Millimeter (mm) slice thickness<br>• Table speed of 15 mm/rotation<br>• Pitch: 1:5:1 |
| **Emission Scan** | 2-7 min/bed position<br>Depending on the height of the subject, a single bed position may provide adequate anatomic coverage. |
| **Image Reconstruction** | Reconstruction algorithms that are consistent and tailored to provide equal quality images by NMCTG were used. |
| **Matrix Size** | Matrix size defined in the NMCTG repot (256x256) |
| **Views** | Non-attenuation corrected and Attenuation corrected |
| [1] Subject BA received an estimated injected $^{89}$Zr-IAB22M2C dose of 1.4 mCi at visit 2, baseline; otherwise all additional injected doses for this subject at Visit 6 (on-Treatment) and the other 4 subjects was 3.0 (±20%) mCi of $^{89}$Zr-Df-IAB22M2C administered over 5-10 minutes I.V. | |

**Image Analysis Methodology:**

**IHC Analysis Methodology for Measuring CD8 T-cells**

Image Guided Core Biopsy samples were collected at baseline (Visit 1) and 0-14 days after the on- Treatment $^{89}$Zr-Df-IAB22M2C PET/CT scan (Visit 8) as outlined above (Table 86.1) and as found in the Phase II protocol IAB-CD8-201. For any biopsy sample to be included in the analysis, a subject must have had at least one acceptable (diagnostic quality) biopsy sample. The collected samples were sent as described in the Biopsy Protocol (5). Histopathologic analysis of core biopsy samples was performed according to methods outlined in the BIOCARE protocol manual. The CD8+ T cell counts was determined using an automated quantitative detection algorithm on core biopsy samples stained with (CD8 antibody [SP57] (6). The results of CD8+ T cell counts were reported as the average number of CD8+ cell per sq. mm of total tissue area collected during image guided biopsies (7). Qualitative confirmation of CD8+ T cell staining along with tumor content were reviewed centrally by a board-certified pathologist with expertise in CD8+ T cell IHC interpretation. Additional information provided upon pathologic review but not included in this report include (1) CD8+ T cell based H-Score, (2) % Tumor and Necrosis in tissue sample, (3) estimation of % CD8 T cells in the total tissue sample, (4) location of CD8+ T cell distribution and (5) qualitative results regarding the intra tumoral vs peri tumoral distribution of CD8+ T cells.

**Central Imaging Review Design and Methodology**

The central imaging review for the Phase-II IAB-CD8-201 clinical trial was conducted using a single reader paradigm as outlined in the IRC (3). The reader selected for this analysis is a board- certified radiologist and Nuclear Medicine Physician with expertise in PET interpretation and $^{89}$Zr- Imaging with appropriate education and qualifications

including a working knowledge of published RECIST 1.1 criteria (8). A dedicated reader training and testing session in the diagnostic interpretation of $^{89}$Zr-Df-IAB22M2C PET/CT interpretation was conducted using the remote online training method using 5 test case studies from the Phase I $^{89}$Zr-Df-IAB22M2C clinical trial.

For interpretation reads contained within this report, the local investigational sites submitted de- identified and HIPPA compliant images data sets from the CT guided biopsy imaging sessions (Visit 1 and Visit 8) and the corresponding $^{89}$Zr-Df-IAB22M2C PET/CTs from Visit 3 and Visit 7 using a secure transfer services (AGMedNet). The images were quality checked for compliance to the parameters provided in the Imaging Manual (9). Query-free scans were transferred to the PET analysis workstation configured and validated for use to interpret $^{89}$Zr-Df-IAB22M2C PET scans and conventional imaging studies (mint Lesion, v3.2.3).

For image interpretation, CT guided biopsy scans including the image series containing the placement of the core biopsy needle tip identified the target lesion(s) on CT guided biopsy images.

Once identified, the reader selected the same area of the lesion biopsy on the appropriate $^{89}$Zr- Df-IAB22M2C PET scan for image interpretation and analysis. The reader was instructed to evaluate the baseline biopsy guided images and baseline $^{89}$Zr-Df-IAB22M2C PET/CT together as one read session and the on-treatment image guided biopsy scan and on treatment $^{89}$Zr-Df- IAB22M2C PET/CT scans as the second read session.

## Quantitative Image Analysis

Image interpretation of the $^{89}$Zr-Df-IAB22M2C PET/CT images from Visit 3 and Visit 7 included the following steps to provide quantitative results for correlation of radiotracer uptake with CD8+ T cell counts as determined by IHC analysis (Fig. 63A).

1. Identify the biopsy tumor lesion(s) on biopsy CT. The central reader should review the biopsy CT to locate the targeted lesion and the biopsy needle position within that lesion.
2. Identify the biopsied lesion on $^{89}$Zr-Df-IAB22M2C PET scan
3. Draw a VOI on the biopsy tumor lesion on the $^{89}$Zr-Df-IAB22M2C PET scan (assign a location to the VOI) where the biopsy needle placement occurred in order to obtain SUV based measurements

    ◦ SUVmax
    ◦ SUVpeak
    ◦ SUVmean

4. Complete a questionnaire for each lesion that includes an approximation of lesion size on CTAC and SUV measurements of reference tissue. In order to obtain a SUVmean for reference tissues, the Reader should draw an ROI on reference tissue by placing an appropriately sized VOI in the thoracic Ao (VOI should be contained within the lumen of the aorta and may not be approximately 1.5 $cm^3$ in diameter, liver, spleen, bone marrow (L3 vertebral body), right paraspinal muscle (at L3 vertebral body)

## Statistical analysis for Primary Outcome Measures

Biopsied tissue samples was acquired at two time points- Visit 1, which is prior to Baseline $^{89}$Zr-Df-IAB22M2C PET/CT and IOT treatment and Visit 8 which is 0-2 weeks after the acquisition of on-treatment $^{89}$Zr-Df-IAB22M2C PET/CT;

Descriptive statistical analysis was performed on all the quantitative PET imaging data ($PET_{Baseline}$, $PET_{Tx}$) measurements acquired from the biopsied lesions and the reference tissues. This descriptive statistical analysis includes measurement of arithmetic mean, standard deviation, median, maximum, minimum and 1$^{st}$ and 3$^{rd}$ quartile for the SUV values from the biopsied lesion (SUVmax and SUVpeak), and the reference tissues (SUVmean).

In addition, Target: Reference (T:R) ratios of $^{89}$Zr-Df-IAB22M2C uptake in the biopsied lesion compared to the reference tissue have been computed for SUVmax and SUVpeak values from the biopsied lesions to SUVmean from reference tissues at each $^{89}$Zr-Df- IAB22M2C time point (i.e. $PET_{Baseline}$ and $PET_{Tx}$ scans). The reference tissue selected for inclusion in the T:R ratio estimates include aorta, normal liver, spleen, normal bone marrow (calculated at the L3 vertebral body) and right paraspinal (calculated at L5 vertebral body). A regression analysis (using Pearson's linear correlation) was performed to evaluate relationship of the SUVmax and SUVpeak measurements normalized by activity from the reference tissues with respect to the IHC measurements.

A regression analysis (using Pearson's linear correlation) was performed to provide evidence of the diagnostic performance of $^{89}$Zr-Df-IAB22M2C PET signal as a reliable indicator of CD8+ T cells in the tumor lesions using IHC-determined CD8+ T-cells counts as the gold standard. For primary outcomes analysis that includes correlation of PET imaging measurement with IHC from the biopsied lesion, SUVmax and SUVpeak from $PET_{Baseline}$ scan was compared to IHC measurements from baseline (Visit 1) biopsy, whereas SUVmax and SUVpeak from $PET_{Tx}$ scan was compared

to on-Treatment (Visit 8) biopsy. The CD8+ T cell counts used for this analysis included the average CD8+ cell counts per sq. mm of tissue area reported out during IHC analysis. Statistical significance was defined as p values $\leq 0.05$. Correction for overfitting was not performed.

To evaluate the potential change in $^{89}$Zr-Df-IAB22M2C uptake in reference tissues from baseline to the on-treatment $^{89}$Zr-Df-IAB22M2C scans, SUVmean measurements of reference tissue was calculated on the basis of both the absolute and the percentage change in the $^{89}$Zr-Df-IAB22M2C uptake. For the biopsied lesions SUVmax and SUVpeak from $PET_{Baseline}$ and $PET_{Tx}$ scans were used to calculate absolute change and percentage change from baseline provided that the same lesion was selected for tissue sampling during both biopsy sessions. In the current analysis, all biopsies were from the same lesion with the exception of a baseline only left parietal brain lesion in a subject.

## Results And Analysis

All the five subjects who successfully completed both baseline and on-treatment $^{89}$Zr-Df-IAB22M2C PET scans and biopsy is show in Table 86.3.

**TABLE 86.3: Subject Profiles Undergoing $^{89}$Zr-Df-IAB22M2C PET/CT Imaging**

| Subject | Age[1] & Gender | Tumor Type | Tissue Samples Visit 1 | Tissue Samples Visit 8 |
|---|---|---|---|---|
| BA | 63 M | NSCLC | Fresh Bone Biopsy | Fresh Bone 2 |
| BB | 75 M | NSCLC | Archival Brain[3] Fresh Lung | Fresh Lung[2] |
| BC | 67 M | Renal Cell | Fresh Peritoneal | Fresh Peritoneal[2] |
| BD | 65 M | Renal Cell | Fresh Bone | Fresh Bone[2] |
| BE | 60 F | NSCLC | Fresh Lung | Fresh Lung[2] |

1 Age in years

2 Tissue sample was obtained from the same lesion on baseline and on-treatment Visits

3 Archival tissue from left parietal brain lesion was sent for central pathology review

The following should be noted:

1. Subject BA: at baseline had two separate biopsies obtained from the same bone lesion including a segment of cortical bone (biopsy sample 1) and a marrow aspirate (biopsy sample 2) per personal communication with site PI. The IHC CD8+ T cell results from sample biopsy 2 was chosen to be included in this report because of more tumor tissue the sample (90% for sample 2 vs 40% for sample 1) and, the decalcification procedures required to dissolve cortical bone in sample biopsy 1 could have potentially influenced the CD8+ T cell staining by SP57 antibody.
2. Subject BB: had two separate tissue samples submitted for central pathology review from different anatomic locations at baseline. Sample biopsy 1 included archival tissues from a left parietal brain metastasis lesion while sample biopsy 2 included a left upper lobe lung lesion. The results of both samples and their corresponding $^{89}$Zr-Df-IAB22M2C PET lesion analysis were included in this report.
3. Except for subject BA baseline (visit2) study, all subjects received 3$\pm$20% mCi of $^{89}$Zr-Df- IAB22M2C at both baseline and on-treatment scan injection visits. However, Subject BA, received approximately 1.4 mCi of the expected 3.0mCi dose. Despite the low dose injection on subject BA, the image quality was rated as acceptable by the central reader. Nevertheless, the results of the PETbaseline scan were included in the report.

All the biopsy samples were successfully received and evaluated at BIOCARE Medical and the results from the automatic IHC analysis were sent to AMAREX for further statistical calculations.

## $^{89}$Zr-Df-IAB22M2C Uptake in Reference Tissues at Baseline and On-Treatment PET scans

$^{89}$Zr-Df-IAB22M2C uptake in reference tissues at $PET_{Baseline}$ and $PET_{Tx}$ scans were evaluated for each subject. The reference tissues include the liver, spleen, bone marrow (L3 vertebrae), skeletal muscle (right paraspinal muscle at L5) and aorta. Table 86.4A lists the SUVmean measured on the $PET_{Baseline}$ and $PET_{Tx}$ for each reference region in each subject. The arithmetic mean, standard deviation, median, and quartile (1$^{st}$ and 3$^{rd}$), min and max are provided as well for each scan.

**TABLE 86.4A: SUVmean Of Reference Tissues From $PET_{Baseline}$ And $PET_{Tx}$ Scans FOR All 5 Subjects.**

| Parameter | Tissue | Subject BA | Subject BB | Subject BC | Subject BD | Subject BE | Mean (SD) | Mean | Quartile (1,3) | Min, Max |
|---|---|---|---|---|---|---|---|---|---|---|
| SUVmean Baseline | Bone Marrow | 13.0 | 5.7 | 7.0 | 7.0 | 5.3 | 7.6 (3.1) | 7.0 | 5.7, 7.0 | 5.3, 13.0 |
| | Paraspinal Muscle | 3.0 | 0.6 | 1.6 | 1.5 | 1.1 | 1.6 (0.9) | 1.5 | 1.1,1.6 | 0.6, 3.0 |
| | Liver | 14.7 | 9.4 | 7.5 | 5.0 | 6.0 | 8.5 (3.8) | 7.5 | 6.0, 9.4 | 5.0, 14.7 |
| | Spleen | 113.9 | 46.2 | 73.7 | 50.8 | 60.1 | 68.9 (27.2) | 60.1 | 50.8, 73.7 | 46.2, 113.9 |
| | Aorta | 3.1 | 1.8 | 2.3 | 1.7 | 1.5 | 2.1 (0.6) | 1.8 | 1.7, 2.3 | 1.5, 3.1 |
| [1] Subject BA received approximately 1.4 mCi at baseline | | | | | | | | | | |
| | | | | | | | | | | |
| SUVmean On-Treatment | Bone Marrow | 7.8 | 11.1 | 5.7 | 14.3 | 5.4 | 8.9 (3.8) | 7.8 | 5.7,11.1 | 5.4, 14.3 |
| | Paraspinal Muscle | 2.9 | 2.3 | 0.5 | 2.7 | 1.0 | 1.9 (1.0) | 2.3 | 1.0, 2.7 | 0.5, 2.9 |
| | Liver | 7.8 | 16.5 | 6.9 | 7.8 | 5.8 | 10.0 (4.6) | 7.8 | 6.9, 13.2 | 5.8, 16.5 |
| | Spleen | 95.8 | 88.4 | 56.9 | 59.2 | 45.8 | 69.2 (21.6) | 59.2 | 56.9, 88.4 | 45.8, 95.8 |
| | Aorta | 2.1 | 4.4 | 2.1 | 2.5 | 1.4 | 2.5 (1.1) | 2.1 | 2.1,2.5 | 1.4, 4.4 |

## $^{89}$Zr-Df-IAB22M2C Uptake in Biopsied Lesion at Baseline and On-Treatment PET Scans

$^{89}$Zr-Df-IAB22M2C uptake in biopsied lesions from $PET_{baseline}$ and $PET_{Tx}$ scans were evaluated for each subject. SUVmax and SUVpeak values for each biopsied lesion were measured and recorded in the Table 86.4B. The arithmetic mean, standard deviation, median, and quartile (1st and 3rd) were calculated on the all the values at a visit for each reference region at included in the table.

**TABLE 86.4B: SUVmax And SUVpeak Values Of Biopsied Lesions From $PET_{baseline}$ AND $PET_{tx}$ Scans For 5 Subjects.**

| Subject | Lesion | SUVmax Baseline | SUVmax On-Treatment | SUVpeak Baseline | SUVpeak On-Treatment |
|---|---|---|---|---|---|
| BA[1] | Bone | 4.5 | 3.1 | 4.3 | 1.8 |
| BB | Brain | 1.3 | N/A[2] | 0.8 | N/A[2] |
| BB | Lung | 3.7 | 5.5 | 2.2 | 4.2 |
| BC | Peritoneum | 3.6 | 1.9 | 0.0[3] | 1.3 |
| BD | Bone | 4.2 | 3.4 | 3.4 | 2.7 |
| BE | Lung | 4.1 | 3.8 | 3.1 | 2.6 |
| **Summary Statistics** | | | | | |
| Mean (SD) | | 3.6 (1.16) | 3.5 (1.3) | 2.3 (1.6) | 2.5 (1.1) |
| Median | | 3.9 | 3.4 | 2.7 | 2.6 |
| Quartile (1,3) | | 3.6, 4.2 | 3.1, 3.8 | 1.1, 3.4 | 1.8, 2.7 |
| Min, Max | | 1.3,4.5 | 1.9,5.5 | 0.0,4.3 | 1.3, 4.2 |

[1] Subject BA received approximately 1.4 mCi at baseline. Therefore the measurements reported in this table may be outlier values
[2] Brain biopsy was not acquired at on-treatment for the patient BB
[3] Small lesions with VOI reduced ability to obtain a reliable SUVpeak measurement

## IHC CD8 T-Cell Analysis Measurements Performed at Baseline and On-Treatment Biopsy Samples Collected from Five Subjects

IHC analysis was performed on the biopsy samples received from the five subjects reported above at baseline Visit 1 and on-treatment Visit 8. From the IHC analysis, the total number of CD8 positive cells across the total tissue area (sq. mm) was assessed for each biopsy sample at each visit as shown Table 86.5 below.

**TABLE 86.5: IHC Analysis Measure Performed For Each Biopsy Sample For All Five Subjects**

| Subject | Biopsy Site | Average Number of CD8 + T cells per sq. mm of Total Tissue Area at Baseline | Average Number of CD8+ T cells per sq. mm of Total Tissue Area On-Treatment |
|---|---|---|---|
| BA | Bone | 40 | 125 |
| BB | Brain | 78 | N/A[1] |
| BB | Lung | 191 | 767 |
| BC | Peritoneum | 81 | 61 |
| BD | Bone | 371 | 409 |
| BE | Lung | 279 | 869 |

[1]Brain biopsy was not acquired at on-treatment for patient BB

## Correlation of $^{89}$Zr-Df-IAB22M2C Uptake Using PET Measurements SUVmax, SUVpeak and with IHC Measurements of CD8 T-cells at Baseline and On-Treatment PET Scans for the Biopsied Lesions

In order to determine the relationship between $^{89}$Zr-Df-IAB22M2C uptake and CD8+ T cell counts, a regression analysis was performed between the SUV values (SUVmax and SUVpeak) from the biopsied lesions on $PET_{baseline}$ and $PET_{Tx}$ scans and the IHC measurements collected at Visit 1 and Visit 8 using Pearson's "r" correlation. Table 86.6 demonstrates the correlation analysis between PET measurements and their respective IHC measurements at baseline and on-treatment (Table 86.6 A and B, respectively) as well as combined results (Table 86.6 C). The analysis includes the correlation based on SUVmax and SUVpeak.

**TABLE 86.6A: Analysis Of Pet Measurements (SUVmax) And IHC Analysis For 5 Subjects With Correlation Performed Separately For Baseline And On-Treatment Data.**

| Subject | Average Number of CD8 Positive cells per sq. mm of Total Tissue Area Baseline | SUVmax $PET_{Basline}$ | Correlation Pearson's r (p-value)5 | Average Number of CD8 Positive cells per sq. mm of Total Tissue Area on Tx | SUVmax $PET_{Tx}$ | Correlation Pearson's r (p-value)[5] |
|---|---|---|---|---|---|---|
| BA | 40 | 4.5[1] | 0.4 (0.48) | 125 | 3.1 | 0.8 (0.10) |
| BB[2] | 78 | 1.3 | | N/A[3] | NA[3] | |
| BB[4] | 191 | 3.7 | | 767 | 5.5 | |
| BC | 81 | 3.6 | | 61 | 1.9 | |
| BD | 371 | 4.2 | | 409 | 3.4 | |
| BE | 279 | 4.1 | | 869 | 3.8 | |

[1] SUVmax values may be an outlier due to low $^{89}$Zr-Df-IAB22M2C injected dose at baseline
[2] Samples and PET measurements obtained from left parietal brain metastasis
[3] Subject 01-002 Brain was not included in the analysis since a biopsy sample was not available for this time point
[4] Sample and PET measurements obtained from left upper lobe lesion
[5] p-value <0.05 statistically significant

**TABLE 86.6 B: Analysis Of Pet Measurements (SUVpeak) And IHC Analysis For 5 Subjects With Correlation Performed Separately For Baseline And On-Treatment Data**

| Subject | Average Number of CD8 Positive cells per sq. mm of Total Tissue Area Baseline | SUVmax $PET_{Basline}$ | Correlation Pearson's r (p-value)56 | Average Number of CD8 Positive cells per sq. mm of Total Tissue Area on Tx | SUVpeak $PET_{Tx}$ | Correlation Pearson's r (p-value)[5] |
|---|---|---|---|---|---|---|
| BA | 40 | 4.3 | 0.4 (0.48) | 125 | 1.8 | 0.8 (0.11) |
| BB[2] | 78 | 0.8 | | N/A[3] | NA[3] | |
| BB[4] | 191 | 2.2 | | 767 | 4.2 | |
| BC | 81 | 0.0[5] | | 61 | 1.3 | |
| BD | 371 | 3.4 | | 409 | 2.7 | |
| BE | 279 | 3.1 | | 869 | 2.6 | |

[1] SUVmax values may be an outlier due to low $^{89}$Zr-Df-IAB22M2C injected dose at baseline
[2] Samples and PET measurements obtained from left parietal brain metastasis
[3] Subject 01-002 Brain was not included in the analysis since a biopsy sample was not available for this time point
[4] Sample and PET measurements obtained from left upper lobe lung lesion
[5] Actual SUVpeak = 0.02
[6] p-value <0.05 statistically significant

**TABLE 86.6C: Correlation Analysis Between Pet Measurements (SUVmax And SUVpeak) And IHC Analysis For Five Subjects With Baseline And On-Treatment Data Combined**

| Subject | Average Number of CD8 Positive cells per sq. mm of Total Tissue Area Baseline | SUVmax $PET_{Basline}$ | Correlation Pearson's r (p-value)[5 6] | Average Number of CD8 Positive cells per sq. mm of Total Tissue Area on Tx | SUVpeak $PET_{Tx}$ | Correlation Pearson's r (p-value)[5] |
|---|---|---|---|---|---|---|
| BA $PET_{Baseline}$ | 40 | 4.5[1] | | 4.3 | | |
| BB $PET^2_{Baseline}$ | 78[2] | 1.3[2] | | 0.8[2] | | |
| BB $PET^3_{Baseline}$ | 191 | 3.7 | | 2.2 | | |
| BC $PET_{Baseline}$ | 81 | 3.6 | | 0.0- | | |
| BD $PET_{Baseline}$ | 371 | 4.2 | | 3.4 | | |
| BE $PET_{Baseline}$ | 279 | 4.1 | 0.5 (0.09) | 3.1 | 0.5 (0.14) | |
| BA $PET_{Tx}$ | 125 | 3.1 | | 1.8 | | |
| BB[3,5] $PET_{Tx}$ | 767 | 5.5 | | 4.2 | | |
| BC $PET_{Tx}$ | 61 | 1.9 | | 1.3 | | |
| BD $PET_{Tx}$ | 409 | 3.4 | | 2.7 | | |
| BE $PET_{Tx}$ | 869 | 3.8 | | 2.6 | | |

[1] SUV values at baseline may be an outlier due to low $^{89}$Zr-Df-IAB22M2C injected dose
[2] Samples and PET measurements obtained from left parietal brain metastasis
[3] Sample and PET measurements obtained from left upper lobe lung lesion
[4] Actual SUV peak = 0.02
[5] Subject 01-002 did not have a brain biopsy sample at $PET^{tx}$
[6] p-value <0.05 statistically significant

Fig. 63B and 63C demonstrates the linear relationship between SUV based measurements (SUVmax and SUVpeak) and Average CD8+ T cell count of biopsy samples at baseline (a) and on-treatment (b) including the regression coefficients ($r^2$). Fig. 63D demonstrates the linear relationship including regression coefficients ($r^2$) when both baseline and on-treatment results are plotted together for SUVmax (a) and SUVpeak(b). Note that the SUV values for Subject BA at baseline are included in the figures.

**Target (Biopsied Lesion): Reference Tissue Ratios**

The mean and standard deviation of SUVratios between SUVmax of the biopsied lesion at baseline and SUVmean of the reference tissues are 1.83±0.74 (aorta), 0.47±0.26 (liver), 0.06±0.02 (spleen) 0.52±0.20 (bone marrow) and 3.10±1.68 (muscle), respectively. Similarly, the mean and standard deviation of SUVratios between SUVmax of the biopsied lesion at on-treatment and SUVmean of the reference tissues using aorta, liver, spleen, bone marrow (13 vertebrae), and right paraspinal muscle L5 are 1.10±0.83 (aorta), 0.30±0.26 (liver), 0.04±0.02 (spleen), 0.32±0.22 (bone marrow) and 1.92±1.28 (muscle), respectively.

Similar calculation as above was performed using lesion SUVpeak: Reference SUVmean. The mean and standard are 1.54±0.69 (aorta), 0.39±0.17(liver), 0.05±0.02(spleen), 0.43±0.18(bone marrow) and 2.46±1.32(muscle), respectively. Similarly, on-treatment Tumor: Reference ratios of the mean and standard deviation are 1.07±0.47 (aorta), 0.27±0.12 (liver), 0.04±0.02(spleen), 0.30±0.12(bone marrow) and 1.73±0.91(muscle), respectively.

Table 86.7 (A and B) demonstrates the correlation analysis between PET measurements represented by SUV

ratios (SUVmax/SUVmean or SUVpeak/SUVmean) and IHC measurements for $PET_{Baseline/Tx}$ combined -to- all biopsy results using each data point as an independent variable in the analysis.

**TABLE 86.7A: Combined Pearson's Correlation Of IHC Measurement With SUVr (SUVmax/SUVmean Reference Tissues) From Biopsy Samples Obtained At Baseline And On-Treatment**

| Subject | Average # CD8 + T cells per sq. mm of Total Tissue Area | SUV/max/SUV mean Bone Marrow | SUVmax/SUV mean Paraspinal Muscle | SUVmax/SUVmean Liver | SUVmax/SUV mean Spleen | SUVmax/SUVmean Aorta |
|---|---|---|---|---|---|---|
| BA $PET_{Baseline}$ | 40 | 0.3 | 1.5 | 0.3 | 0.0 | 1.4 |
| BB[1] $PET_{Baseline}$ | 78 | 0.2 | 2.1 | 0.1 | 0.0 | 0.7 |
| BB PET[3] $_{Baseline}$ | 191 | 0.6 | 6.1 | 0.3 | 0.0 | 2.0 |
| BC $PET_{Baseline}$ | 81 | 0.5 | 2.2 | 0.4 | 0.0 | 1.5 |
| BD $PET_{Baseline}$ | 371 | 0.6 | 2.8 | 0.8 | 0.0 | 2.47 |
| BE $PET_{Baseline}$ | 279 | 0.7 | 3.7 | 0.6 | 0.0 | 2.7 |
| BA $PET_{Tx}$ | 124 | 0.4 | 1.1 | 0.2 | 0.0 | 1.4 |
| BB[2] $PET_{Tx}$ | 767 | 0.5 | 2.3 | 0.3 | 0.0 | 1.2 |
| BC $PET_{Tx}$ | 61 | 0.3 | 3.8 | 0.2 | 0.0 | 0.9 |
| BD $PET_{Tx}$ | 409 | 0.2 | 1.2 | 0.4 | 0.0 | 1.3 |
| BE $PET_{Tx}$ | 869 | 0.7 | 3.8 | 0.6 | 0.0 | 2.7 |
| Correlation Pearson's r (p-value)[4] | | 0.4 (0.19)[5] | 0.1 (0.73)[5] | 0.4(0.18)[5] | 0.6(0.04)[5] | 0.4(0.18)[5] |

[1] Samples and PET measurements obtained from left parietal brain metastasis

[2] Subject 01-002 Brain was not included in the analysis since a biopsy sample was not available for this time point

[3] Sample and PET measurements obtained from left upper lobe lung lesion

[4] p-value <0.05 statistically significant

[5] Summary statistics as calculated by sponsor

**TABLE 86.7B: Combined Pearson's Correlation Of IHC Measurement With SUVr (SUVpeak/SUVmean Reference Tissues) From Biopsy Samples Obtained At Baseline And On-Treatment**

| Subject | Average # CD8 + T cells per sq. mm of Total Tissue Area | SUV/max/SUV mean Bone Marrow | SUVmax/SUV mean Paraspinal Muscle | SUVmax/SUV mean Liver | SUVmax/SUV mean Spleen | SUVmax/SUV mean Aorta |
|---|---|---|---|---|---|---|
| BA $PET_{Baseline}$ | 40 | 0.3 | 1.4 | 0.2 | 0.0 | 1.4 |
| BB[1] $PET_{Baseline}$ | 78 | 0.1 | 1.3 | 0.1 | 0.0 | 0.4 |
| BB[3] $PET_{Baseline}$ | 191 | 0.4 | 3.7 | 0.2 | 0.0 | 1.2 |
| BC $PET_{Baseline}$ | 81 | 0.0[4] | 0.0[4] | 0.0[4] | 0.0[4] | 0.0[4] |
| BD $PET_{Baseline}$ | 371 | 0.4 | 2.2 | 0.6 | 0.0 | 2 |
| BE $PET_{Baseline}$ | 279 | 0.6 | 2.8 | 0.5 | 0.0 | 2.1 |
| BA $PET_{Tx}$ | 125 | 0.2 | 0.6 | 0.1 | 0.0 | 0.9 |
| BB[2] $PET_{Tx}$ | 767 | 0.4 | 1.8 | 0.2 | 0.0 | 0.9 |
| BC $PET_{Tx}$ | 61 | 0.2 | 2.6 | 0.2 | 0.0 | 0.6 |
| BD $PET_{Tx}$ | 409 | 0.2 | 1 | 0.3 | 0.0 | 1.1 |
| BE $PET_{Tx}$ | 869 | 0.5 | 2.6 | 0.4 | 0.7 | 1.9 |
| Correlation Pearson's r (p-value)[4] | | 0.5 (0.11)[6] | 0.3 (0.44)[6] | 0.5(0.14)[6] | 0.6(0.03)[5] | 0.4(0.16)[6] |

[1] Samples and PET measurements obtained from left parietal brain metastasis
[2] Subject 01-002 Brain was not included in the analysis since a biopsy sample was not available for this time point
[3] Sample and PET measurements obtained from left upper lobe lung lesion
[4] Actual SUVpeak = 0.02
[5] p-value <0.05 statistically significant
[6] Summary statistics as calculated by sponsor

## Changes In $^{89}$Zr-Df-IAB22M2C Uptake in Reference Tissues and Biopsied Lesions

Tables 86.8 and 86.9 demonstrate the absolute and percent change in $^{89}$Zr-Df-IAB22M2C uptake for biopsied lesions and reference tissues, respectively. For biopsied lesions, both the SUVmax and SUVpeak value changes were calculated; whereas the SUVmean values were used for reference tissue. Please note that the same lesion needed to be biopsied at baseline and on-treatment to be included in the lesion analysis table. For this reason, the left parietal brain metastasis in a was not included. In addition, the SUVpeak at baseline for a subject's Peritoneum was not included because of the very low SUV (0.02) leading to an unreliable estimate of the $^{89}$Zr-Df-IAB22M2C uptake.

**TABLE 86.8: Absolute And Percentage Change In $^{89}$Zr-Df-IAB22M2C Uptake From $PET_{tx}$ To $PET_{Baseline}$ For Biopsy Lesions**

| Subject | Lesion | Absolute Change SUVmax | % Change SUVmax | Absolute Change SUVpeak | % Change SUVpeak |
|---|---|---|---|---|---|
| BA[1] | Bone | -1.4 | -31.1 | -2.5 | -58.1 |
| BB[2] | Lung | 1.8 | 48.6 | 2.0 | 90.9 |
| BC3 | Peritoneum | -1.7 | -47.2 | N/A | N/A |
| BD | Bone | -0.8 | -19.0 | -0.7 | -20.5 |
| BE | Lung | -0.3 | -7.31 | -0.5 | -16.1 |
| Mean4 | | -0.5 | -11.2 | -0.4 | -1.0 |
| STD. Deviation[4] | | 1.4 | 36.6 | 1.9 | 64.1 |
| Median[4] | | -0.8 | -19.0 | -0.6 | -18.4 |
| Quartile (1,3)[4] | | (-1.4, -0.3) | (-31.1, 7.3) | (-0.6, 1.5) | (-30.0, 10.6) |

[1]SUV values at baseline may be an outlier due to low $^{89}$Zr-Df-IAB22M2C injected dose and therefore affect the absolute and % change estimates

[2]Subject BB Brain was not included in the analysis since a biopsy sample was not available for this time point

[3]Actual SUVpeak = 0.02 at baseline and thus the change cannot be calculated in this sample

[4]Summary statistics as calculated by sponsor

**TABLE 86.9A: Absolute Change In SUVmean $^{89}$Zr-Df-IAB22M2C Uptake From $PET_{Tx}$ To $PET_{Baseline}$ In Reference Tissue**

| Absolute Change in SUVmean $^{89}$Zr-Df-IAB22M2C Uptake from $PET_{Tx}$ to PETBaseline[2] | | | | | | | | | |
|---|---|---|---|---|---|---|---|---|---|
| Reference Tissue | Subject BA | Subject BB | Subject BC | Subject BD | Subject BE | Mean | SD | Median | Quartile (1,3) |
| Bone Marrow | -5.2 | 5.4 | -1.3 | 7.3 | 0.1 | 1.3 | 5.1 | 0.1 | -1.35.4 |
| Liver | -1.5 | 7.1 | -0.6 | 2.8 | -0.2 | 1.5 | 3.5 | -0.2 | -0.6,2.8 |
| Spleen | -18.1 | 42.2 | -16.8 | 8.4 | -14.3 | 0.3 | 25.8 | -14.3 | -16.8,8.4 |
| Aorta | -1.0 | 2.6 | -0.2 | 0.8 | -0.1 | 0.4 | 1.4 | -0.1 | -0.2,0.8 |
| Paraspinal Muscle | -0.1 | 1.7 | -1.1 | 1.2 | -0.1 | 0.3 | 1.1 | -0.1 | -0.1,1.2 |

[1] SUV values at baseline may be an outlier due to low 89Zr-Df-IAB22M2C injected dose and therefore affect the absolute and % change estimates

[2] Summary statistics as calculated by sponsor

**TABLE 86.9B: Percent Change In Suvmean $^{89}$zr-Df-Iab22m2c Uptake From $Pet_{tx}$ To $Pet_{baseline}$ In Reference Tissue**

| % Change in SUVmean $^{89}$Zr-Df-IAB22M2C Uptake from PETTx to PETBaseline2 | | | | | | | | | |
|---|---|---|---|---|---|---|---|---|---|
| **Reference Tissue** | **Subject BA** | **Subject BB** | **Subject BC** | **Subject BD** | **Subject BE** | **Mean** | **SD** | **Median** | **Quartile (1,3)** |
| Bone Marrow | -40 | 94.7 | -18.5 | 104.2 | 1.8 | 28.4 | 42.1 | 31.6 | -18.5,94.7 |
| Liver | -10.2 | 75.5 | -8 | 56 | -3.3 | 22.0 | 28.4 | 19.0 | -8.056.0 |
| Spleen | -15.8 | 91.3 | -22.7 | 16.5 | -23.7 | 9.1 | 14.1 | -1.3 | -22.7,16.5 |
| Aorta | -32.2 | 144.4 | -8.6 | 47.0 | -6.6 | 28.8 | 41.0 | 20.3 | -8.6,47.0 |
| Paraspinal Muscle | -3.3 | 283.3 | -68.75 | 80 | -9.0 | 56.5 | 68.4 | 25.4 | -9.0,80.0 |
| 1 SUV values at baseline may be an outlier due to low 89Zr-Df-IAB22M2C injected dose and therefore affect the absolute and % change estimates<br>2 Summary statistics as calculated by sponsor | | | | | | | | | |

This example provides the first analyses of the diagnostic performance of $^{89}$Zr-Df-IAB22M2C PET for detecting CD8+ T cells in biopsied tumor samples from 5 subjects who have completed baseline and on-treatment PET/CT scans and concomitant biopsies of tumor lesions. Additional information contained within this example include measurements of $^{89}$Zr-Df-IABM22C uptake in selected reference tissues and changes in the $^{89}$Zr-Df-IAB22M2C uptake between baseline and on-treatment scans.

Table 86.3 provides a summary of the tumor type and location of the biopsy lesions for PET and IHC CD8+ T cell evaluation in the first 5 subjects. Three of the 5 subject had metastatic Non-Small Cell Lung Cancer and 2 subjects had metastatic Renal Cell Carcinoma. All subjects in this report were enrolled at CARTI and imaged on the same PET/CT system that was qualified by NMCTG using an anthropometric phantom specifically tailored to $^{89}$Zr PET imaging. In this regard, the variability of $^{89}$Zr- Df-IAB22M2C uptake due to PET scanner issues between patients and time points was potentially minimized. A subject received approximately 1.4 mCi of $^{89}$Zr-Df-IAB22M2C at baseline. As a result, less than the 1.5 mg API IAB22MC was delivered intravenously. Nevertheless, the image quality was considered acceptable the central reviewer who was blinded to this information.

The location of biopsy samples from the 5 subjects included lung tumors (n=2; subjects BB and BE), bone lesions (n=2; subjects BA and BD), brain metastasis (n=1; subject BB at baseline) and peritoneal metastasis (n=1; subject BC). Of note, Subject BA had a right iliac bone metastasis that underwent both bone biopsy and marrow aspiration at baseline. The CD8+ T cell IHC results from the later sample was used for correlation with $^{89}$Zr-Df-IAB22M2C uptake in this report. This was based upon pathologists who indicated that decalcification of bone trabeculae in the first sample could potentially interfere with CD8+ T cell binding and that the second sample contained more tumor cells over a larger area of sampled tissue compared to the first sample (i.e. 90% tumor cells compared to 40% tumor cells, respectively).

**Correlation of PET measurements vs. IHC Measurements of CD8 T-cells Counts**

Table 86.6 and Figures 63B-63D demonstrate the correlation of $^{89}$Zr-Df-IAB22M2C uptake and CD8+ T cell counts. As can be seen in Table 86.6C and Figure 63D, there is a positive association between SUVmax uptake and CD8+ T cell counts (Pearson's r = 0.5, p-value = 0.09; $r^2$ = 0.2) when all biopsy lesions regardless if they were from the baseline or on-treatment PETs are included in the analysis. Similarly, SUVpeak uptake and CD8+ T cell counts tend to show a positive correlation though not as robust as SUVmax (Pearson's r = 0.5, p-value = 0.14; $r^2$ = 0.2186).

Furthermore, there is a positive correlation between $PET_{tx}$ SUVmax and SUVpeak uptake and on- treatment CD8+ T cell counts for SUVmax measurements (Pearson's r = 0.8, p-value = 0.10; $r^2$=0.6 for SUVmax vs Pearson's r = 0.8, p-value = 0.11; $r^2$=0.6 for SUVpeak). A less robust correlation of $PET_{Baseline}$ SUVmax measurements and CD8+ T cell counts (Pearson's r = 0.4, p-value = 0.48; $r^2$=0.1) and SUVpeak (Pearson's r = 0.4, p-value = 0.48; $r^2$=0.1) is noted. An improvement in the correlation statistics was noted when subject BA baseline SUV lesion measurements were removed (data not shown here but will be included in follow reports).

The threshold for detectability of CD8 T cells by $^{89}$Zr-Df-IAB22M2C PET/CT is one of the goals of the Phase II trial. An inspection of the linear regression graphs in FIGs. 86B-86D show that when the CD8 T cell count is at approximately 150/mm$^2$ or more the data points tend to lie closer to the line of regression then those data points less

than 150/$mm^2$ despite the recognized issue with the subject injected with a lower $^{89}$Zr-Df-IAB22M2C dose at baseline. The final threshold for detecting CD8+ T cells by $^{89}$Zr-Df-IAB22M2C PET may benefit from more subjects and biopsy samples.

## Summary Results of $^{89}$Zr-Df-IAB22M2C Uptake in Biopsied Lesion and Reference Tissues

In this Example is described the measured uptake of $^{89}$Zr-Df-IAB22M2C in reference tissues and changes in reference tissues between the $PET_{tx}$ and $PET_{Baseline}$ scans. In keeping with the results in Example 85, the uptake of radiotracer in tumor lesions and reference tissues was similar both from a quantitative perspective and within the range of values previously reported. (1). However, this trial was designed to evaluate the changes in uptake in tumor and reference tissues prior to and following IOT. In this regards, Tables 86.4 and 86.8 and 86.9 highlights the changes noted between $PET_{Baseline}$ and $PET_{Tx}$ scans. In general, uptake in reference tissue values tended to be higher on the on-treatment scans than at baseline- ranging from small changes (9.1% in splenic tissue) to 22.0 and 28.4% changes in bone marrow and liver, respectively. Thoracic aorta and muscle uptake were also higher by 28.8% and 56.5% respectively. But, the absolute change between scans was less apparent with the mean absolute difference especially in these later two reference tissues being less than 0.5 SUV units.

The average SUVr whether using SUVmax and SUVpeak from baseline and on-treatment data were similar. SUVr values are often used to normalize PET uptake measurements in target regions. When using these values in regression (Table 86.7 A and B) it was observed that both the SUVmax and SUVpeak divided by SUVmean from Spleen shows a correlation with highest r value 0.6 for both as compared to the correlation results performed using other reference tissues. The correlation using spleen as a reference, also showed significant relationship with the low p-values ($p<0.05$). On using other reference regions such as bone marrow or Aorta as reference tissue, a positive correlation was observed for either SUVmax (Bone marrow: Pearson's r = 0.5, p=0.19; Aorta: Pearson's r = 0.4, p = 0.18) and SUVpeak (Bone Marrow: Pearson's r = 0.5, p = 0.11; Aorta: Pearson's r = 0.4, p = 0.16). The ratios calculated using the SUVmax with respect to bone marrow as a reference tissues gave the correlation coefficient of 0.5 (second best than Spleen) and the p- values were trending toward significance ($0.05<p<0.10$).

There are few considerations that should be kept in mind while interpreting the results. This data set is relatively small. The present example employed core biopsy to provide tumor tissue samples for IHC data correlation. Core biopsy samples represent a small fraction of the total tumor mass, and this methodology could lead to sampling errors including mis-representation of the cellular mass and cellular distribution in the total tumor. The current imaging analysis methodology tries to compensate for this limitation, by requesting the central PET reader to restrict the ROI analysis to the region around where the biopsy needle is seen in the tumor. This approach may lead to increased signal variability in both SUV in an isolated section of the tumor as well as uncertainty in placement of the ROI to the exact location on PET/CT where the core biopsy sample was obtained. In some cases, such as subject BA and BC the entire lesions were evaluated on $^{89}$Zr-Df-IAB22M2C PET scans due to their small size. In the other cases, only part of the lesion was included in the ROI. Another methodologywould be changing the analysis of the biopsy lesion to include the whole tumor volume is being considered.

Additional observations noted from the first 5 subjects imaged to date include:

1. No drug related SAEs have been reported with repeat $^{89}$Zr-Df-IAB22M2C injections. Safety monitoring continues.
2. Image quality and conformance to protocol required image acquisition and processing was well executed via central core lab instructions in the IM.
3. The distribution of $^{89}$Zr-Df-IAB22M2C activity seems to follow the same pattern of uptake as noted in Phase I without apparent alterations in the pattern of $^{89}$Zr-Df-IAB22M2C uptake in reference tissue uptake between baseline and on-treatment scans.
4. There continues to be a dynamic range of $^{89}$Zr-Df-IAB22M2C uptake in tumor lesions and lymph nodes between baseline and on-treatment scans.
5. $^{89}$Zr-Df-IAB22M2C uptake in bone lesions and the peritoneal metastasis in the subjects with RCC was interpreted by the central reader as negative for both baseline and on-treatment scan while uptake in the lung lesions was interpreted positive in both subjects with NSCLC.
6. In a subject, there was new and increasing uptake in regional lymph nodes (mediastinum) on the on-treatment $^{89}$Zr-Df-IAB22M2C PET scan compared to baseline and the amount of $^{89}$Zr-Df-IAB22M2C in the primary tumor lesion appeared greater following therapy.
7. Discussions with local PIs at CARTI have noted that some subjects (both with negative positive $PET_{Baseline}$ and $PET_{Tx}$ scans) did not respond to IOT while subjects BB (with positive $PET_{Baseline}$ and PETTx scans) had responded to treatment. Furthermore, subject BE had developed clinical pseudoprogression (positive $PET_{Baseline}$ and $PET_{Tx}$ scans) but elected to seek comfort care. No clinical information is available for BD at this time.

The results presented in this example demonstrate the ability to obtain high quality whole body $^{89}$Zr-Df-IAB22M2C PET/CT scans with repeat dosing of $^{89}$Zr-Df-IAB22M2C. A positive correlation between $^{89}$Zr-Df-IAB22M2C uptake using simple SUV methods and CD8+ T Cell count by IHC is trending towards statistical significance for all biopsied lesions with stronger correlation seen for on-treatment data points. In addition, the $^{89}$Zr-Df-IAB22M2C uptake and findings in reference of tissues are similar to Phase I results. The current data is very encouraging in demonstrating the ability of the $^{89}$Zr-Df-IAB22M2C to detect CD8+ T cells in people with cancer.

**References**

1. Pandit-Taskar N., et.al. First-in-human imaging with $^{89}$Zr-Df-IAB22M2C anti-CD8 minibody in patients with solid malignancies: preliminary pharmacokinetics, biodistribution, and lesion targeting. JNM 2019: (accepted for publication).
2. NMCTG A scanner specific report generated by the NMCTG as a part of the scanner validation process, AMAREX TMF Site Specific 2.10.3 SIISA
3. Image Review Charter "IA010_IRC" Version 1.0 dated 29Jul2019 developed by Imaging Endpoint.
4. Image Guided Biopsy Manual "IA0101-IGBM" Version 2.0 dated 15Aug2019 developed by Imaging Endpoint.
5. LABORATORY MANUAL Sample Collection, Processing and Shipping Instructions Version 3.0 Date 15FEB2019 developed by ImaginAb.
6. Validation Report "CD8 [SP57] Immunohistochemistry IHC Assay Staining-ImaginAb" Version 1.0 dated 3MAR2019 developed by BIOCARE Medical.
7. Immunohistochemistry for CD8 and H-score Calculation Technical Report BCM IAB-CD8-201/02 Version 1.0 dated 8MAR2019 developed by BIOCARE Medical.
8. Eisenhauer, E., Therasse, P., Bogaerts, J., et.al. New response evaluation criteria in solid tumors: Revised RECIST guideline (version 1.1). 2009 Eur J Cancer. 2008; 45: (97-110).
9. Imaging Manual "IA010-IM" Version 1.0 dated 14SEP2018 developed by Imaging Endpoint.


**INDIVIDUAL SUBJECTS SUMMARY MEASUREMENTS FROM EXAMPLE 86**

Table 86.10 lists the values of SUVmax and SUVpeak for all the biopsied lesion for each subject. And the values of SUVmean for the all the reference tissues for each subject for the 89Zr-DF-IAB22M2C PET scans at baseline and on-treatment. Also listed is the IHC analysis measure at baseline and on-treatment for each subject.

**Table 86.10**

| **Patient id BA. baseline** | | |
|---|---|---|
| **Patient id** | **Description** | **Values** |
| PETBaseline | Biopsied lesion SUVmax (Bone) | 4.5 |
| PETBaseline | Biopsied lesion SUVpeak (Bone) | 4.3 |
| PETBaseline | Bone marrow (L3) SUVmean | 13.0 |
| PETBaseline | Paraspinal Muscle (SUVmean) | 3.0 |
| PETBaseline | Liver SUVmean | 15.0 |
| PETBaseline | Spleen SUVmean | 114.0 |
| PETBaseline | AORTA SUVmean | 3.1 |
| IHC baseline | (Average Number of CD8 Positive cells per sq. mm of Total Tissue Area) | 40 |
| **Patient id BA. on-treatment** | | |
| **Patient id** | **Description** | **Values** |
| $PET_{Tx}$ | Biopsied lesion SUVmax (Bone) | 3.1 |
| $PET_{Tx}$ | Biopsied lesion SUVpeak (Bone) | 1.8 |
| $PET_{Tx}$ | Bone marrow (L3) SUVmean | 7.8 |

(continued)

| Patient id BA. on-treatment | | |
|---|---|---|
| **Patient id** | **Description** | **Values** |
| $PET_{Tx}$ | Paraspinal Muscle (SUVmean) | 2.9 |
| $PET_{Tx}$ | Liver SUVmean | 13.2 |
| $PET_{Tx}$ | Spleen SUVmean | 96.0 |
| $PET_{Tx}$ | AORTA SUVmean | 2.1 |
| IHC on-treatment | Average Number of CD8 Positive cells per sq. mm of Total Tissue Area | 125 |
| **Patient id BB. baseline** | | |
| **Patient id** | **Description** | **Values** |
| PETBaseline | Biopsied lesion SUVmax (Brain) | 1.3 |
| PETBaseline | Biopsied lesion SUVpeak (Brain) | 1.0 |
| PETBaseline | Biopsied lesion SUVmax (Lung) | 4.0 |
| PETBaseline | Biopsied lesion SUVpeak (Lung) | 2.2 |
| PETBaseline | Bone marrow (L3) SUVmean | 6.0 |
| PETBaseline | Paraspinal Muscle (SUVmean) | 0.6 |
| PETBaseline | Liver SUVmean | 9.4 |
| PETBaseline | Spleen SUVmean | 46.2 |
| PETBaseline | AORTA SUVmean | 2.0 |
| IHC baseline | Average Number of CD8 Positive cells per sq. mm of Total Tissue Area | 78 |
| **Patient id BB. On-treatment** | | |
| **Patient id** | **Description** | **Values** |
| $PET_{Tx}$ | Biopsied lesion SUVmax (Lung) | 5.5 |
| $PET_{Tx}$ | Biopsied lesion SUVpeak (Lung) | 4.2 |
| $PET_{Tx}$ | Bone marrow (L3) SUVmean | 11.1 |
| $PET_{Tx}$ | Paraspinal Muscle (SUVmean) | 2.3 |
| $PET_{Tx}$ | Liver SUVmean | 16.5 |
| $PET_{Tx}$ | Spleen SUVmean | 88.4 |
| $PET_{Tx}$ | AORTA SUVmean | 4.4 |
| IHC on-treatment | Average Number of CD8 Positive cells per sq. mm of Total Tissue Area | 767 |
| **Patient id BC. baseline** | | |
| **Patient id** | **Description** | **Values** |
| PETBaseline | Biopsied lesion SUVmax (Peritoneum) | 3.6 |
| PETBaseline | Biopsied lesion SUVpeak (Peritoneum) | 0[1] |
| PETBaseline | Bone marrow (L3) SUVmean | 7.0 |
| PETBaseline | Paraspinal Muscle (SUVmean) | 1.6 |
| PETBaseline | Liver SUVmean | 7.5 |
| PETBaseline | Spleen SUVmean | 74.0 |
| PETBaseline | AORTA SUVmean | 2.3 |
| IHC baseline | Average Number of CD8 Positive cells per sq. mm of Total Tissue Area | 81 |

(continued)

| Patient id BC. baseline | | |
|---|---|---|
| Patient id | Description | Values |
| [1]Actual SUVpeak = 0.02 | | |
| **Patient id BC. On-treatment** | | |
| **Patient id** | **Description** | **Values** |
| PETTx | Biopsied lesion SUVmax | 1.9 |
| PETTx | Biopsied lesion SUVpeak | 1.3 |
| PETTx | Bone marrow (L3) SUVmean | 6.0 |
| PETTx | Paraspinal Muscle (SUVmean) | 0.5 |
| PETTx | Liver SUVmean | 7.0 |
| PETTx | Spleen SUVmean | 57.0 |
| PETTx | AORTA SUVmean | 2.1 |
| IHC on-treatment | Average Number of CD8 Positive cells per sq.mm of Total Tissue Area | 61 |
| **Patient id BD. baseline** | | |
| **Patient id** | **Description** | **Values** |
| PETBaseline | Biopsied lesion SUVmax | 4.2 |
| PETBaseline | Biopsied lesion SUVpeak | 3.4 |
| PETBaseline | Bone marrow (L3) SUVmean | 7.0 |
| PETBaseline | Paraspinal Muscle (SUVmean) | 1.5 |
| PETBaseline | Liver SUVmean | 5.0 |
| PETBaseline | Spleen SUVmean | 50.8 |
| PETBaseline | AORTA SUVmean | 1.7 |
| IHC baseline | Average Number of CD8 Positive cells per sq. mm of Total Tissue Area | 371 |
| **Patient id BD. On-treatment** | | |
| **Patient id** | **Description** | **Values** |
| PETTx | Biopsied lesion SUVmax | 3.4 |
| PETTx | Biopsied lesion SUVpeak | 3.0 |
| PETTx | Bone marrow (L3) SUVmean | 14.3 |
| PETTx | Paraspinal Muscle (SUVmean) | 3.0 |
| PETTx | Liver SUVmean | 8.0 |
| PETTx | Spleen SUVmean | 59.2 |
| PETTx | AORTA SUVmean | 2.5 |
| IHC on-treatment | Average Number of CD8 Positive cells per sq. mm of Total Tissue Area | 409 |
| **Patient id BE. baseline** | | |
| **Patient id** | **Description** | **Values** |
| PETBaseline | Biopsied lesion SUVmax | 4.1 |
| PETBaseline | Biopsied lesion SUVpeak | 3.1 |
| PETBaseline | Bone marrow (L3) SUVmean | 5.3 |
| PETBaseline | Paraspinal Muscle (SUVmean) | 1.1 |

(continued)

| **Patient id BE. baseline** | | |
|---|---|---|
| **Patient id** | **Description** | **Values** |
| PETBaseline | Liver SUVmean | 6 |
| PETBaseline | Spleen SUVmean | 60.1 |
| PETBaseline | AORTA SUVmean | 1.5 |
| IHC baseline | Average Number of CD8 Positive cells per sq. mm of Total Tissue Area | 279 |
| **Patient id BE. On-treatment** | | |
| **Patient id** | **Description** | **Values** |
| PETTx | Biopsied lesion SUVmax | 4.0 |
| PETTx | Biopsied lesion SUVpeak | 3.0 |
| PETTx | Bone marrow (L3) SUVmean | 5.4 |
| PETTx | Paraspinal Muscle (SUVmean) | 1 |
| PETTx | Liver SUVmean | 6 |
| PETTx | Spleen SUVmean | 46 |
| PETTx | AORTA SUVmean | 1.4 |
| IHC on-treatment | Average Number of CD8 Positive cells per sq. mm of Total Tissue Area | 869 |

Further PET images from the present example are provided in FIGs. 64D-64F.

A responder image is depicted in FIG. 64D (Phase-II, **IAB-CD8-201** Responder: Patient BE, Tumor Type: NSCLC, Treatment Type: OPDIVOs). A 60-year-old subject diagnosed with non-small cell lung carcinoma. Images were acquired at 24±3 hrs. after injection of 111±20% MBq of 89Zr-Df-IAB22M2C prior (before treatment) and within one month (On-Treatment) after the start of OPDIVO therapy. The 89Zr-Df-IAB22M2C PET/CT fused images (demonstrates uptake in the biopsied left upper lobe lung tumor (white circle; lesion >=3cm lesion) with 89Zr-Df-IAB22M2C uptake at before treatment that increased on treatment. The uptake at baseline and on-treatment was visually assessed as positive with a visual assessment score of 3 (equal to normal vertebrae calculated at L3). The grey color scale used for these images' ranges from lower SUV limit of 0 to upper SUV limit of 5.

FIG. 64E is a PET scan and a fused PET/CT scan showing the results of using the minibody construct for testing the effectiveness of various treatments. The image is of a 75-year-old Male diagnosed with metastatic Non Small Cell Lung Carcinoma Axial fused PET/CT images from baseline and on-treatment (Keytruda, Carboplatin, Paclitaxel). The images were acquired at 24 ±3 hrs. after injection of 111±20% MBq of $^{89}$Zr-Df-IAB22M2C. The arrows demonstrates a >3cm metastatic lesion in upper left lung. This lesion was biopsied at baseline and on-treatment. The uptake at baseline and on-treatment was assessed as positive with a visual assessment score of 2 (uptake between Aorta and Bone Marrow (calculated at L3 Vertebrae)). Thus, this demonstrates that imaging using the present minibody can determine the effectiveness of an IOT therapy.

A non-responder image is shown in FIG. 64F (Patient BC, Tumor Type: RCC, Treatment Type: OPDIVO. Phase-II, IAB-CD8-201). The non-responder was a 67-year-old subject diagnosed with metastatic Renal Cell Carcinoma. Images were acquired at 24±3 hrs. after injection of 111±20% MBq of $^{89}$Zr-Df-IAB22M2C prior (before treatment) and within one month (On-Treatment) after the start of OPDIVO therapy.

Tables 86.11 and 86.12 provide a summary of the subject's image assessments (SUV values and number of CD8+ cells) and treatments they were on, for ease of review. As shown in Table 86.12, the method allows for one to identify responders and non-responders.

**Table 86.11:** The SUVmax, SUVpeak, andCD8 SUV load values for the identified lesions from the two subjects BC and BE.

| **Patient ID** | **Tumor type** | **Lesion/Tumor type** | **Visit** | **Score** | **Iamge Assessment** | **SUVmax** | **SUVpeak** | **Average number of CD8+ cells per mm$^2$ of total tissue** | **Treatment** |
|---|---|---|---|---|---|---|---|---|---|
| BC | RCC | Peritoneum | **Baseline** | 1 | Negative | 3.6 | 0.0 | 81 | OPDIVO |
| | | | **On-Treatment** | 1 | Negative | 1.9 | 1.3 | 61 | |
| BE | NSCLC | Left upper lobe lung | **Baseline** | 1 | Positive | 4.1 | 3.1 | 279 | OPDIVO |
| | | | **On-Treatment** | 1 | Positive | 3.8 | 2.6 | 869 | |
| BB | NSCLC | Left upper lobe ling lesion | **Baseline** | 2 | Negative | 3.7 | 2.2 | 191 | KEYTRUDA, CARBOPLATIN, PACILATEXAL |
| | | | **On-Treatment** | 2 | Positive | 5.5 | 4.2 | 767 | |

**Table 86.12:** Phase 2 data-SUV values

| Patient | Tumour | Baseline SUVmax** | Baseline IHC* | Treatment | Tx SUVmax** | Tx IHC* | Image Assessment |
|---|---|---|---|---|---|---|---|
| BA | NSCLC | 4.5 | 40 | Key truda, carboplatin, Alimta | 3.1 | 125 | Non-responder |
| BB | NSCLC | 3.7 | 191 | Key truda, carboplatin, paclitaxel | 5.5 | 767 | Response to therapy |
| BC | Renal Cell Carcinoma | 3.6 | 81 | OPDIVO | 1.9 | 61 | Non-responder |
| BD | Renal Cell Carcinoma | 4.2 | 371 | Yervoy, Opdivo, Xgeva | 3.4 | 409 | Non-responder |
| BE | NSCLC | 4.1 | 279 | OPDIVO | 3.8 | 869 | Response to therapy |

**Example 87--Image analysis**

The present example outlines options for image analysis.

For image interpretation, CT guided biopsy scans including image series containing the placement of the core biopsy needle tip identify the target lesion(s) on CT guided biopsy images. Once identified, the reader selected the same lesion on the appropriate $^{89}$Zr-Df-IAB22M2C PET scan for image interpretation and analysis. The reader was instructed to evaluate the baseline biopsy guided images and baseline $^{89}$Zr-Df-IAB22M2C PET/CT together as one read session and the on-treatment image guided biopsy scan and on treatment $^{89}$Zr-Df-IAB22M2C PET/CT scans as the second read session.

**Qualitative Image Analysis**

The following outlines the process for qualitative generalized lesion uptake of $^{89}$Zr-Df-IAB22M2C without taking into consideration reference tissue uptake. In this approach, the reader was trained to rate a lesion as follows:

- Positive (any uptake noted in biopsied lesion)
- Negative (no uptake noted in biopsied lesion)
- Equivocal (uncertain uptake in biopsied lesion)

This approach was like the method(s) used by radiologist and nuclear medicine physicians to interpret FDG-PET scans in oncology (5) subjects.

The following outlines the process for for generalized lesion uptake of $^{89}$Zr-Df-IAB22M2C without taking into consideration Visual Score scale based upon $^{89}$Zr-Df-IAB22M2C lesion uptake compared to selected reference tissues. This scale was constructed upon similar approached used in FDG PET scans in the analysis of lymphoma according to the Deauville Criteria (6) found in the Lugano Criteria. In this $^{89}$Zr-Df-IAB22M2C uptake rating system, a score between 1 to 5 was assigned to the biopsied lesion according to the following definition:

- VS1= uptake <blood pool (aorta);
- VS2= AORTA< uptake < normal L3 vertebral body;
- VS3= uptake equals bone marrow (normal L3 vertebral body);
- VS4= uptake>normal L3 vertebral body
- VS5= uptake >> normal L3 vertebral body

These reference tissues were to provide the range of low (Aorta) to high (bone marrow) CD8+ T cell concentration found in each of these anatomic sites.

**Quantitative Image Analysis**

Image interpretation of the $^{89}$Zr-Df-IAB22M2C PET/CT images from before treatment and On-treatment included the following steps to provide quantitative results for correlation of radiotracer uptake with CD8+ T cell counts as determined by IHC analysis.

1.1.1. Identify the before treatment or On-treatment tumor lesion on $^{89}$Zr-Df-IAB22M2C PET/CT that underwent CT guided biopsy
1.1.2. Estimate lesion size of biopsy lesion on the CTAC (Lesions will also be stratified by size (<3 cm or > 3cm) for future analysis to further understand how the size of a lesion may influence $^{89}$Zr-Df-IAB22M2C uptake on PET scans. Since tumor growth rates can vary between subjects, lesion size on CTAC from 89Zr-Df-IAB22M2C PET/CT scans should represent the closest size estimates of tumor lesions. However, the CTAC scans may not be of sufficient diagnostic quality for accurate lesion size measurements compared to diagnostic CT or MRI.)
1.1.3. Draw a VOI around the biopsy tumor lesion to obtain SUV based measurements

1. SUVmax
2. SUVpeak-
3. SUVmean-
4. CD8 SUV load (SUVmea X Isocontour volume)

2.2.4 Once complete, place approximately 3cm diameter VOI on reference tissues to obtain SUVmean values. Reference tissues include thoracic Ao (VOI should be contained within the lumen of the aorta and may not be approximately 3cm in diameter), liver, spleen, bone marrow (L3 vertebral body), right paraspinal muscle (at L5 vertebral body)

Table 87.1 outlines the Image Acquisition Parameters.

Table 87.1

| **Use the scanner parameters outlined in the site-specific report generated by NMCTG** | |
|---|---|
| **Radiopharmaceutical** | $^{89}$Zr-Df-IAB22M2C |
| **Dose** | 3($\pm$20%) mCi of $^{89}$Zr-Df-IAB22M2C administered over 5-10 minutes I.V. |
| Mode | 2D, 3D list-mode, or TOF<br>When possible, acquisition should be acquired in List Mode. |
| **Patient Position** | Supine with arms down by side. Keep position consistent between CT and PET acquisitions. |
| **Scan Location/Coverage** | Vertex of Skull through feet including all areas of known or suspected disease |
| **Transmission Scan** | • 140 kVp<br>• mAs:<br>◦ 24($\pm$3) hrs. p.i. scans: low dose CT (40-80 mAs)<br>◦ Optional visit 5 post infusion scan: low dose CT (10 mAs)<br>• 0.8 sec per CT rotation<br>• 3-5 Millimeter (mm) slice thickness<br>• Table speed of 15 mm/rotation<br>• Pitch: 1:5:1 |
| **Emission Scan** | 2-7 min/bed position<br>Depending on the height of the subject, a single bed position may provide adequate anatomic coverage. |

(continued)

| Use the scanner parameters outlined in the site-specific report generated by NMCTG | |
|---|---|
| **Image Reconstruction** | Reconstruction algorithms that are consistent and tailored to provide equal quality images by NMCTG were used. |
| **Matrix Size** | Matrix size defined in the NMCTG repot (256x256) |
| **Views** | Non-attenuation corrected and Attenuation corrected |

**References for present Example.**

1.2. Pandit-Taskar N., et.al. First-in-human imaging with 89Zr-Df-IAb22m@c anti-CD8 minibody in patients with solid malignancies: preliminary pharmacokinetics, biodistribution, and lesion targeting. JNM 2019: (in press).

**1.3.** Image Review Charter "IA010_IRC" Version 1.0 dated 29Jul2019 developed by Imaging Endpoint.

**1.4.** Eisenhauer, E., Therasse, P., Bogaerts, J., et.al. New response evaluation criteria in solid tumors: Revised RECIST guideline (version 1.1). 2009 Eur J Cancer. 2008; 45: (97-110).

**1.5.** Imaging Manual "IA010-IM" Version 1.0 dated 14SEP2018 developed by Imaging Endpoint.

**1.6.** Hoffman MS., and Hicks RJ. How we read Oncologic FDG PET/CT. Cancer Img. 2016; 16 :35

**1.7.** Barrington SF., and Kluge R. FDG PET for therapy monitoring in Hodgkin and non-Hodgkin lymphomas Eur J Nucl. Med Mol Imaging. 2017; 44(Suppl 1): 97-110

**Example 88**

Further PET images from the present example are provided in FIGs. 64D-64F (data generated in the contect of Example 85).

FIG. 64A depicts PET images from two subjects over several different points in time. One can observe the increase in signal at the circled location from the added detectable marker that is associated with the CD8 antigen binding molecule.

FIGs. 64B and 64C depict a combination of FDG PET with CD8 PET, to characterize the tumor micro environment more completely. As shown, FDG can be used to identify the tumor, while CD8 antigen binding construct can be used to determine infiltration.

**Example 89--Dosing at 1.0 mCi and below**

The method of Example 10 is employed with adjustments to produce a $^{89}$Zr-Df-IAB22M2C drug product such that the dose administered to the patient at the time of infusion is about 0.5 mCi, about 0.75 mCi or about 1.0 mCi, in each case +20% of radiation. All other components of the dose administered are the same, including the use of cold, unconjugated IAB22M2C and formulation buffer. It is observed that due to shipping requirements from a typical centralized production facility, which may be geographically removed from the site of administration to the patient, the radiation dose at time of manufacturing is substantially higher than the administered dose.

Various patients receive the dose of $^{89}$Zr-Df-IAB22M2C, as outlined in Example 7-9. Imaging of the patients preferably occurs between 2 and 24 hours after dosing, most preferably 2-6 hours after dosing.

The benefits of the reduced dose of about 0.5 mCi, about 0.75 mCi or about 1.0 mCi, in each case +20%, are significant in terms of 1) improved patient safety due to the lower overall radiation dose provided; 2) accelerated imaging which allows administration and imaging to take place during a single day visit to the imaging center, 3) improved flexibility on manufacturing, whereby unanticipated delays in delivery can be accommodated due to the lower final dose required, 4) imaging results which are equal to, or at least not inadequate to provide the diagnostic and treatment insights described elsewhere herein, and other reasons which will be apparent to those skilled in the art.

Other aspects of the invention include the following:

Aspect 1. A method of administering an antigen binding construct to a subject, the method comprising:

providing to a subject a labeled antigen binding construct that binds to human CD8,
wherein the label comprises $^{89}Zr$,
wherein the label provides 0.5-3 mCi ±20% of radiation at injection,
wherein an amount between 0.2 and 10mg of total antigen binding construct is provided to the subject.

Aspect 2. A method of treating a patient, comprising:
administering to a human patient diagnosed with a cancer a dose of an antigen-binding construct that binds to human CD8, wherein the dose comprises:

a $^{89}Zr$-labeled antigen-binding construct providing a radiation activity of about 0.5 to 3.6 mCi; and
about 10 mg or less of the antigen-binding construct;
detecting the $^{89}Zr$-labeled antigen-binding construct in the patient at a first time point after administering the dose, to generate a first patient image corresponding to the first time point, wherein the first time point is about 6 hours to 36 hours after administering;
determining a first abundance and/or distribution of CD8 cells in one or more tissues and/or neoplasia in the patient based on the first patient image;
administering to the patient a first treatment for the cancer;
after administering the first treatment, administering to the human patient diagnosed with the cancer a second dose of the antigen-binding construct;
determining a second abundance and/or distribution of CD8 cells in the one or more tissues and/or neoplasia in the patient based on a second patient image;
comparing the first patient image to the second patient image and administering to the patient a second treatment for the cancer based on a comparison of the first and second patient images.

Aspect 3. The method ofAspect2, wherein comparing comprises determining if there is an increase in infiltration in the second image that indicates that the first treatment is working and therefore the first treatment is continued, and wherein no change or a decrease in infiltration in the second image indicates that the first treatment is not working and administering to the patient a second treatment for the cancer based on a comparison of the first and second patient images.

Aspect 4. A method of treating a patient, comprising:
administering to a human patient diagnosed with a cancer a dose of a CD8 PET tracer, wherein the dose comprises:

a CD8 PET tracer that provides a radiation activity of about 0.5 to 3.6 mCi at injection; and
between about 5 micrograms and 50 mg of the CD8 PET tracer;
detecting the CD8 PET tracer in the patient at a first time point after administering the dose, to generate a first patient image corresponding to the first time point, wherein the first time point is about 1 hour to 36 hours after administering;
determining a first abundance and/or distribution of the CD8 PET tracer in one or more tissues and/or neoplasia in the patient based on the first patient image;
administering to the patient a first treatment for the cancer;
after administering the first treatment, administering to the human patient diagnosed with the cancer a second dose of the CD8 PET tracer;
determining a second abundance and/or distribution of the CD8 PET tracer in the one or more tissues and/or neoplasia in the patient based on a second patient image;
comparing the first patient image to the second patient image and administering to the patient a second treatment for the cancer based on a comparison of the first and second patient images.

Aspect 5. The method of any one of Aspects 1-4, wherein the first abundance is a first intensity of the CD8 PET tracer or the $^{89}Zr$ signal on the first patient image, and wherein the second abundance is a second intensity of the CD8 PET tracer or the $^{89}Zr$ signal on the second patient image.

Aspect 6. The method of any one of Aspectsl-4, wherein a location of a tumor is indicated as an increase in radiation in the second patient image than in the first patient image.

Aspect 7. The method of any one ofAspects2-6, wherein the first time point is between 20 and 34 hours after

administering, and wherein determining the second abundance and/or distribution occurs 20 to 34 hours after the second dose is administered.

Aspect 8. The method ofAspect4, wherein CD8 PET tracer comprises a PET isotope with a half-life of less than 3 hours.

Aspect 9. A method of treating a subject, the method comprising:

administering to a patient that has a neoplasia and that is being treated with an immunotherapy ("IOT") an antigen-binding construct that binds to human CD8;
wherein the antigen binding construct comprises a detectable marker,
wherein the antigen binding construct does not provoke an immune response in the subject,
wherein the detectable marker comprises $^{89}Zr$,
wherein the detectable marker provides 0.5-3 mCi $\pm$20% of radiation at injection, and
wherein an amount between 0.2 and 10mg of total antigen binding construct is provided to the patient;
monitoring a distribution of the antigen-binding construct to determine the tumor-infiltrating lymphocyte ("TIL") status within the neoplasia; and
administering an alternative IOT to the patient if the TIL status of the neoplasia is not positive, and repeating the foregoing method until the TIL status of the neoplasia becomes positive.

Aspect 10. The method of Aspect 9, wherein monitoring is conducted within 8 hours of administering.

Aspect 11. The method ofAspect9, wherein monitoring or a further second monitoring is done before 36 hours from administering.

Aspect 12. A method of treating a human subject having cancer, the method comprising:

providing a first image of a CD8 cells within a tumor in a subject using a CD8 antigen binding construct;
administering a therapy including a candidate therapeutic to the subject;
after administering the therapy including the candidate therapeutic, providing a second image of the CD8 cells within the tumor in the subject using the CD8 antigen binding construct;
comparing the first and second images to determine if:

a) the tumor demonstrates increased CD8 infiltration or
b) the tumor demonstrates the same or decreased CD8 infiltration,

wherein, if a), then instructing the subject to continue the therapy.

Aspect 13. The method ofAspect12, wherein if b), then administering another round of therapy, providing a third image of the CD8 cells within the tumor; comparing the first and third images to determine if the tumor demonstrates the same or decreased CD8 infiltration; and discontinuing the therapy if the tumor demonstrates the same or decreased CD8 infiltration.

Aspect 14. A method of treating a human subject having cancer, the method comprising:

providing a first image of CD8 cells within a tumor in a subject using a CD8 PET tracer;
administering a therapy including a candidate therapeutic to the subject;
after administering the therapy, providing a second image of the CD8 cells within the tumor in the subject using the CD8 PET tracer;
comparing the first and second images to determine if:

a) the tumor demonstrates increased CD8 infiltration or
b) the tumor demonstrates the same or decreased CD8 infiltration,

wherein, if a), then instructing the subject to continue the therapy.

Aspect 15. A method of treating a human subject, the method comprising:

administering a candidate therapeutic to a human subject;
determining if a size of a tumor in the human subject has increased or decreased in response to the candidate therapeutic;
if the size has increased following the candidate therapeutic, then using a CD8 antigen binding construct to determine if an amount of CD8 present within the tumor has increased or decreased in response to the candidate therapeutic,
wherein an increase in tumor size without an increase in the amount of CD8 indicates tumor progression, whereas an increase in tumor size with an increase in the amount of CD8 indicates tumor pseudoprogression, wherein a treatment is continued if the patient is experiencing tumor pseudoprogression and wherein a treatment is changed if the patient is experiencing tumor progression.

Aspect 16. A method of administering a label to a human subject, the method comprising:

administering $^{18}F$ to a subject;
within about 6 hours, conducting a PET scan of the subject for a $^{18}F$ distribution of the subject;
administering a CD8 PET tracer to the subject, wherein the CD8 PET tracer comprises $^{89}Zr$; and
within about 36 hours of administering the CD8 PET tracer, conducting a PET scan on the subject for a distribution of $^{89}Zr$.

Aspect 17. The method of any one of Aspects 1-16, wherein the subject or patient receives a therapeutic, and wherein the therapeutic is selected from at least one of: ipilimumab, pembrolizumab, nivolumab, atezolizumab, avelumab, and durvalumab.

Aspect 18. The method of any one of Aspects 1-16, wherein the subject or patient receives a therapeutic, and wherein the therapeutic is selected from at least one of: BMS-1001, BMS-1116, CA-170, CA-327, Imiquimod, Resiquimod, 852A, VTX-2337, ADU-S100, MK-1454, Ibrutinib, 3AC, Idelalisib, IPI-549, Epacadostat, AT-38, CPI-444, Vipadenant, Preladenant, PBF, ZD4635, Galuniseritib, OTX015/MK-8628, CPI-0610.

Aspect 19. The method of any one of Aspects 1-16, wherein the subject or patient receives a therapeutic, and wherein the therapeutic is selected from at least one of: an antibody to LAG-3, an antibody to TIGIT, MK-4280, MK-7684, and/or any of the preceding in combination with Keytruda.

Aspect 20. The method of any one of Aspects 1-3, 5-7, 9-11, 12, 13, 15, 17, 18, or 19, wherein the CD8 antigen binding construct is a minibody or a diabody and wherein the antigen binding construct comprises a heavy chain variable region and a light chain variable region, and wherein the heavy chain variable region consists essentially of a human amino acid sequence and the light chain variable region consists essentially of a human amino acid sequence.

Aspect 21. The method of any one of Aspectsl-3, 5-7, 9-11, 12, 13, 15, 17, 18, or 19, wherein the antigen binding construct comprises the three heavy chain CDRs within SEQ ID NO: 147 and the three light chain CDRs within SEQ ID No: 147.

Aspect 22. The method of any one of Aspectsl-3, 5-7, 9-11, 12, 13, 15, 17, 18, or 19, wherein the antigen binding construct comprises a heavy chain variable region that is at least 80% identical to the heavy chain amino acid sequence in SEQ ID NO: 147, and a light chain variable region that is at least 80% identical to the light chain amino acid sequence in SEQ ID NO: 147.

Aspect 23. The method of any one of Aspectsl-3, 5-7, 9-11, 12, 13, 15, 17, 18, or 19, wherein the subject or patient has a cancer selected from one or more cancer of: carcinoma, lymphoma, blastoma, sarcoma, and leukemia, lymphoid malignancies, squamous cell cancer (e.g. epithelial squamous cell cancer), lung cancer, small-cell lung cancer, non-small cell lung cancer, adenocarcinoma of the lung and squamous carcinoma of the lung, cancer of the peritoneum, hepatocellular cancer, gastric or stomach cancer, gastrointestinal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain, head and neck cancer, adult and pediatric solid cancers, and solid tumors.

Aspect 24. The method of treatment of any one of Aspects 2-15 and 17, wherein the treatment comprises at least one of the therapies provided herein, including, but not limited to: an immune check point inhibitor, an IOT, or a chemotherapeutic agent provided herein.

Aspect 25. The method of any one of the preceding Aspects,wherein the human CD8 is a human CD8 alpha chain.

Aspect 26. The method of any one of the preceding Aspects, wherein the antigen binding construct is selective for human CD8 alpha chain over human CD8 beta chain.

Aspect 27. The method of any one of the preceding Aspects, wherein the antigen binding construct is selective for human CD8 beta chain over human CD8 alpha chain.

Aspect 28. The method of any one of the preceding Aspects, wherein the alpha chain has the sequence of SEQ ID NO: 134.

Aspect 29. The method of any one of the preceding Aspects, wherein the human CD8 is a human CD8 beta chain.

Aspect 30. The method of any one of the preceding Aspects,wherein an increase in detectable marker indicates that there is an increase in CD8 infiltration.

Aspect 31. The method of any one of the preceding Aspects wherein an increase in signal from a CD8 PET tracer, from a first image to a second image, indicates that there is an increase in infiltration in the tumor.

Aspect 32. The method of any one of the preceding Aspects,

wherein an increase in infiltration indicates that a first therapy is effective against the tumor,
wherein an increase in infiltration is indicated by an increase in a PET detected signal between a first image and a second image, and
wherein the PET detect signal is from a detectable marker of a CD8 PET tracer or a CD8 antigen binding construct.

Aspect 33. The method ofAspect32, wherein the increase in the PET detected signal is at least 10%.

Aspect 34. The method ofAspect32, wherein the increase in the PET detected signal is at least 100%.

Aspect 35. The method ofAspect32, wherein the increase in the PET detected signal is at least 1000%.

Aspect 36. The method of any one of Aspects 1-15 and 17, further comprising determining a standard uptake value ("SUV"), the method of determining the SUV comprising:

determining r, where r is a radioactivity concentration (kBq/ml) measured by a PET scanner within a region of interest ("ROI") of radiation from the antigen binding construct;
determining a’, wherein a’ is the decay-corrected amount of the injected detectable marker (kBq);
determining w, the weight of the patient; and
determining SUV as being the result of r(a’/W).

Aspect 37. The method ofAspect36, further comprising repeating the process for a second ROI to determine a second summed signal level and comparing the first and the second summed signal levels, wherein when the first summed signal level is less than the second summed signal level, it indicates the presence of increased CD8 in the second ROI.

Aspect 38. A non-invasive method to visualize CD8 bearing cells in a human subject, the method comprising:

(a) administering to a human subject a tracer that selectively binds to CD8 and is labeled with a PET detectable marker,
(b) monitoring a distribution of the tracer in the subject within 1-36 hours of (a) by PET, in the absence of IHC (immunohistochemistry), wherein a PET scan indicates whether a volume of tissue within the subject is infiltrated with greater than or less than a selected amount of CD8 bearing cells.

Aspect 39. A diagnostic composition comprising:

3.0±20% mCi of a 89Zr-Df-labeled antigen binding construct;
20 mM Histidine;
5% sucrose;
51-62 mM Sodium Chloride;
141-194 Arginine; and
2-20 mM Glutamic acid.

Aspect 40. The composition ofAspect39, wherein there is 1-250 micrograms per kg of subject weight of antigen binding construct, and wherein the antigen binding construct is a minibody.

Aspect 41. A formulation for a CD8 composition, the composition comprising:

a CD8 antigen binding construct, wherein the CD8 antigen binding construct is less than 105 kDa in size; and
a $^{89}$Zr radiolabel associated with the CD8 antigen binding construct, wherein the radiolabel provides more than 0.5 but less than 3.6 mCi of radiation for the formulation,
wherein the formulation is configured for administration to a human.

Aspect 42. The formulation ofAspect41, wherein the radiolabel provides more than 0.5 but less than 1.0 mCi of radiation.

Aspect 43. The formulation ofAspect42, wherein the radiolabel provides less than 0.75 mCi of radiation.

Aspect 44. An antigen-binding construct for use, or for use as a medicament, in the method described in any one of Aspectsl-3, 5-7, 9-13, 15, or 17-38.

Aspect 45. A CD8 PET tracer for use, or for use as a medicament, in the method described in any one ofAspects4-8, 14, 16, 17-19, or 24-38.

Aspect 46. The antigen-binding construct of Aspect 44, or the CD8 PET tracer of Aspect 45, wherein the radiolabel provides more than 0.5 but less than 1.0 mCi of radiation.

Aspect 47. The antigen-binding construct of Aspect 44, or the CD8 PET tracer of Aspect 45, wherein the radiolabel provides less than 0.75 mCi of radiation.

Aspect 44. The composition of Aspect 39-40, the formulation of Aspect 41-43, the antigen-binding construct ofAspect44, 46 or 47, or the CD8 PET tracer of Aspect 45, 46 or 47, for use in at least one of: imaging, detection, diagnosis, surgery, staging, treatment, monitoring of treatment, monitoring of disease progression, and monitoring therapy.

Aspect 49. The composition of Aspect 39-40, the formulation of Aspect 41-43, the antigen-binding construct ofAspect44, 46 or 47, or the CD8 PET tracer ofAspect45, 46 or 47, for use in at least one of: imaging, detection, diagnosis, surgery, staging, treatment, monitoring of treatment, monitoring of disease progression, and monitoring therapy of a CD8 dependent disorder, optionally cancer.

## Claims

**1.** An antigen-binding construct that binds to human CD8 for use in the treatment of a neoplasia in a human subject, the method of treatment comprising:

administering to a human subject that has a neoplasia and that is being treated with an

immunotherapy ("IOT") an antigen-binding construct that binds to human CD8,
wherein the antigen binding construct comprises a detectable marker,
wherein the antigen binding construct does not provoke an immune response in the subject,
wherein the detectable marker comprises at least one of $^{64}$Cu, $^{124}$I, or$^{89}$Zr,

wherein the detectable marker provides 0.5-3 mCi ±20% of radiation at injection, and
wherein an amount between 0.2 and 10mg of total antigen binding construct is provided to the human subject;

monitoring a distribution of the antigen-binding construct to determine the tumor-infiltrating lymphocyte ("TIL") status within the neoplasia; and
administering an alternative IOT to the patient if the TIL status of the neoplasia is not positive, and repeating the foregoing method until the TIL status of the neoplasia becomes positive.

**2.** The antigen-binding construct for the use of claim 1, wherein monitoring is conducted within 8 hours of administering.

**3.** The antigen-binding construct for the use of claim 1, wherein monitoring or a further second monitoring is done before 36 hours from administering.

**4.** The antigen-binding construct for the use of any preceding claim, wherein the subject or patient receives a therapeutic, and wherein the therapeutic is selected from at least one of: ipilimumab, pembrolizumab, nivolumab, atezolizumab, avelumab, and durvalumab.

**5.** The antigen-binding construct for the use of any one of claims 1-3, wherein the subject or patient receives a therapeutic, and wherein the therapeutic is selected from at least one of: BMS-1001, BMS-1116, CA-170, CA-327, Imiquimod, Resiquimod, 852A, VTX-2337, ADU-S100, MK-1454, Ibrutinib, 3AC, Idelalisib, IPI-549, Epacadostat, AT-38, CPI-444, Vipadenant, Preladenant, PBF, ZD4635, Galuniseritib, OTX015/MK-8628, CPI-0610.

**6.** The antigen-binding construct for the use of any one of claims 1-3, wherein the subject or patient receives a therapeutic, and wherein the therapeutic is selected from at least one of: an antibody to LAG-3, an antibody to TIGIT, MK-4280, MK-7684, and/or any of the preceding in combination with Keytruda.

**7.** The antigen-binding construct for the use of any preceding claim, wherein the CD8 antigen binding construct is a minibody or a diabody and wherein the antigen binding construct comprises a heavy chain variable region and a light chain variable region, and wherein the heavy chain variable region consists essentially of a human amino acid sequence and the light chain variable region consists essentially of a human amino acid sequence.

**8.** The antigen-binding construct for the use of any one of claims 1-6, wherein the antigen binding construct comprises a heavy chain variable region that is at least 80% identical to the heavy chain amino acid sequence in SEQ ID NO: 147, and a light chain variable region that is at least 80% identical to the light chain amino acid sequence in SEQ ID NO: 147.

**9.** The antigen-binding construct for the use of any one of claims 1-6, wherein the subject or patient has a cancer selected from one or more cancer of: carcinoma, lymphoma, blastoma, sarcoma, and leukemia, lymphoid malignancies, squamous cell cancer (e.g. epithelial squamous cell cancer), lung cancer, small-cell lung cancer, non-small cell lung cancer, adenocarcinoma of the lung and squamous carcinoma of the lung, cancer of the peritoneum, hepatocellular cancer, gastric or stomach cancer, gastrointestinal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain, head and neck cancer, adult and pediatric solid cancers, and solid tumors.

**10.** The antigen-binding construct for the use of any one of the preceding claims, wherein the human CD8 is a human CD8 alpha chain.

**11.** The antigen-binding construct for the use of any one of the preceding claims, wherein the antigen binding construct is selective for human CD8 alpha chain over human CD8 beta chain.

**12.** The antigen-binding construct for the use of any one of the preceding claims, wherein the antigen binding construct is selective for human CD8 beta chain over human CD8 alpha chain.

**13.** The antigen-binding construct for the use of any preceding claim, wherein the radiolabel provides more than 0.5 but less than 1.0 mCi of radiation.

**14.** The antigen-binding construct for the use of any preceding claim, wherein the radiolabel provides less than 0.75 mCi of radiation.

**15.** The antigen-binding construct for the use of any preceding claim, wherein an increase in tumor size without an increase in the amount of CD8 indicates tumor progression, whereas an increase in tumor size with an increase in the amount of CD8 indicates tumor pseudo-progression, wherein a treatment is continued if the patient is experiencing tumor pseudo-progression and wherein a treatment is changed if the patient is experiencing tumor progression.

# FIGURE 1A

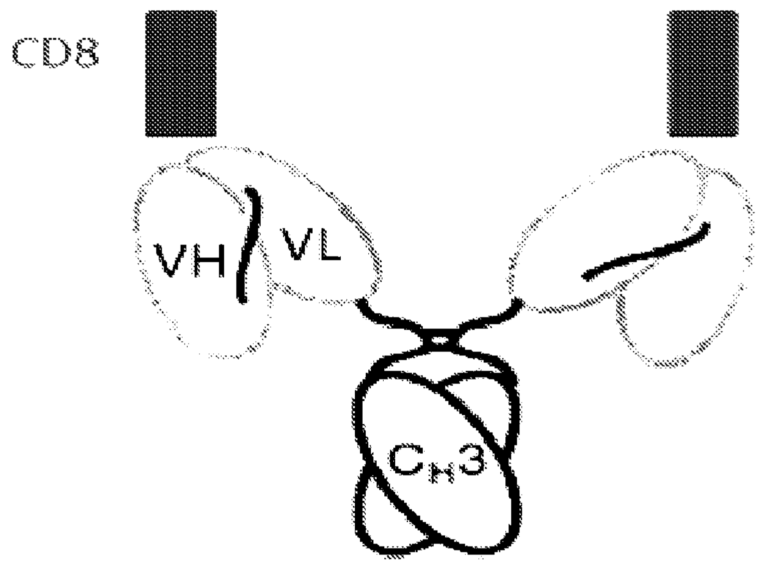


# FIGURE 1B

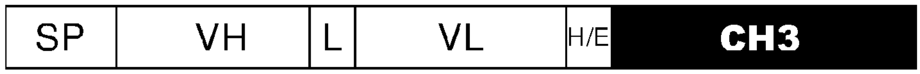

# FIGURE 1C

```
        10         20         30         40         50         60
MALPVTALLL PLALLLHAAR PSQFRVSPLD RTWNLGETVE LKCQVLLSNP TSGCSWLFQP

        70         80         90        100        110        120
RGAAASPTFL LYLSQNKPKA AEGLDTQRFS GKRLGDTFVL TLSDFRRENE GYYFCSALSN

       130        140        150        160        170        180
SIMYFSHFVP VFLPAKPTTT PAPRPPTPAP TIASQPLSLR PEACRPAAGG AVHTRGLDFA

       190        200        210        220        230
CDIYIWAPLA GTCGVLLLSL VITLYCNHRN RRRVCKCPRP VVKSGDKPSL SARYV
```

**SEQ ID NO: 24**

# FIGURE 2A

```
Humanized VH (1st version):            HCDR1                              HCDR2

muOKT8   EVQLQQSGAELVKPGASVKLSCTAS GFNIKD TYIHFVRQRPEQGLEWIG RIDPANDNT LY
              **  **  *  * **    *          *   * **    **   * * **   *
4D5v8    EVQLVESGGGLVQPGGSLRLSCAAS GFNIKD TYIHWVRQAPGKGLEWVA RIYPTNGYT RY
huOKT8   EVQLVESGGGLVQPGGSLRLSCAAS GFNIKD TYIHWVRQAPGKGLEWVA RIDPANDNT LY

                                                   HCDR3
muOKT8   ASKFQGKATITADTSSNTAYMHLCSLTSGDTAVYYCGR GYG--YYVFDH WGQGTTLTVSS   SEQ ID NO: 1
          **** **  *    *    ****  ***        *  ** *** ** *     **
4D5v8    ADSVKGRFTISADTSKNTAYLQMNSLRAEDTAVYYCSR WGGDGFYAMDY WGQGTLVTVSS   SEQ ID NO: 2
huOKT8   ASKFQGRATISADTSKNTAYLQMNSLRAEDTAVYYCGR GYG--YYVFDH WGQGTLVTVSS   SEQ ID NO: 3


Humanized VH (2nd version):             HCDR1                             HCDR2

muOKT8   QVQLQQSGAELVKPGASVKLSCTAS GFNIKD TYIHFVRQRPEQGLEWIG RIDPANDNT LY
         *    *     **    *  *  **   ****  * * * * * **    *  **  ****  *
Human    EVQLVQSGAEVKKPGATVKISCKVS GYTFTD YYMHWVQQAPGKGLEWMG LVDPEDGET IY
huOKT8   QVQLVQSGAEVKKPGATVKISCKVS GFNIKD TYIHWVQQAPGKGLEWMG RIDPANDNT LY
                                                    HCDR3
muOKT8   ASKFQGKATITADTSSNTAYMHLCSLTSGDTAVYYCGR GYGYYVFDH WGQGTTLTVSS   SEQ ID NO: 4
           *    **       **    * *  * *        ** ****** **      **
Human    AEKFQGRVTITADTSTDTAYMELSSLRSEDTAVYYCAT A---EYFQH WGQGTLVTVSS   SEQ ID NO: 5
huOKT8   ASKFQGRVTITADTSTDTAYMELSSLRSEDTAVYYCAR GYGYYVFDH WGQGTLVTVSS   SEQ ID NO: 6
```

# FIGURE 2B

```
Humanized VL:                       LCDR1                        LCDR2

muOKT8    DVQINQSPSFLAASPGETITINC RTSRSISQYLA WYQEKPGKTNKLLIY SGSTLQS GIPS
           * **    * *  * ***  *   * **  *     *    **     **       *
Human     DIQMTQSPSSLSASVGDRVTITC RASQGISNYLA WYQQKPGKVPKLLIY AASTLQS GVPS
huOKT8    DVQITQSPSSLSASVGDRVTITC RTSRSISQYLA WYQQKPGKVPKLLIY SGSTLQS GVPS

                                           LCDR3
muOKT8    RFSGSGSGTDFTLTISGLEPEDFAMYYC QQHNENPLT FGAGTKLELK SEQ ID NO: 7
                                  * *   * *     ** **     *   * *
Human     RFSGSGSGTDFTLTISSLQPEDVATYYC QKYNSAPLT FGGGTKVEIK SEQ ID NO: 8
huOKT8    RFSGSGSGTDFTLTISSLQPEDVATYYC QQHNENPLT FGGGTKVEIK SEQ ID NO: 9
```

In huOKT8, the residues V and I in "DVQITQ" are underlined.

# FIGURE 3A

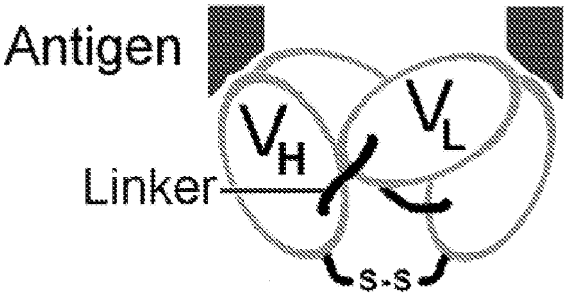


# FIGURE 3B

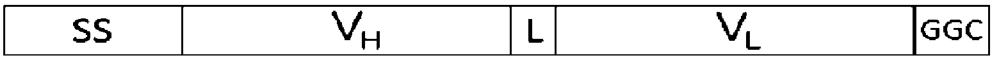

# FIGURE 4

```
Tctagagccgccacc  SEQ ID NO: 61
 XbaI Kozak
    1   M  E  T  D  T  L  L  L  W  V  L  L  L  W  V  P  G  S  T  G
    1   ATGGAGACAGACACCCTGCTCCTGTGGGTGCTGCTCCTCTGGGTCCCTGGATCCACCGGC
  Signal Peptide
   21   D  V  Q  I  N  Q  S  P  S  F  L  A  A  S  P  G  E  T  I  T
   61   GATGTCCAGATCAACCAAAGCCCCAGCTTTCTGGCTGCCTCCCCTGGAGAGACAATCACC
         VL
   41   I  N  C  R  T  S  R  S  I  S  Q  Y  L  A  W  Y  Q  E  K  P
  121   ATCAATTGCCGGACCAGCCGGAGCATTTCCCAGTACCTCGCCTGGTACCAGGAAAAGCCT

   61   G  K  T  N  K  L  L  I  Y  S  G  S  T  L  Q  S  G  I  P  S
  181   GGCAAGACCAACAAGCTGCTGATCTACTCCGGCTCCACACTCCAGAGCGGCATTCCCTCC

   81   R  F  S  G  S  G  S  G  T  D  F  T  L  T  I  S  G  L  E  P
  241   AGGTTTAGCGGATCCGGATCCGGAACCGACTTCACACTCACCATCTCCGGCCTGGAGCCC

  101   E  D  F  A  M  Y  Y  C  Q  Q  H  N  E  N  P  L  T  F  G  A
  301   GAGGACTTCGCCATGTATTATTGCCAGCAGCACAATGAGAACCCCCTGACCTTCGGCGCT

  121   G  T  K  L  E  L  K  G  S  T  S  G  G  G  S  G  G  G  S  G
  361   GGCACCAAGCTGGAGCTGAAAGGCTCCACCAGCGGAGGCGGATCCGGAGGAGGAAGCGGC
                                Linker
  141   G  G  G  S  S  E  V  Q  L  Q  Q  S  G  A  E  L  V  K  P  G
  421   GGCGGAGGCTCCTCCGAAGTGCAGCTGCAACAGAGCGGCGCCGAACTGGTGAAGCCTGGA
                          VH
  161   A  S  V  K  L  S  C  T  A  S  G  F  N  I  K  D  T  Y  I  H
  481   GCTTCCGTGAAACTCAGCTGTACCGCCAGCGGCTTCAACATCAAGGATACCTACATCCAC

  181   F  V  R  Q  R  P  E  Q  G  L  E  W  I  G  R  I  D  P  A  N
  541   TTCGTGCGGCAAAGGCCTGAGCAGGGCCTGGAATGGATCGGCAGGATCGACCCCGCCAAC

  201   D  N  T  L  Y  A  S  K  F  Q  G  K  A  T  I  T  A  D  T  S
  601   GACAACACCCTCTACGCCTCCAAGTTCCAAGGCAAGGCCACAATCACCGCTGATACAAGC

  221   S  N  T  A  Y  M  H  L  S  S  L  T  S  G  D  T  A  V  Y  Y
  661   TCCAACACCGCCTACATGCACCTCAGCTCCCTGACCAGCGGAGACACCGCCGTGTACTAC

  241   C  G  R  G  Y  G  Y  Y  V  F  D  H  W  G  Q  G  T  T  L  T
  721   TGCGGACGGGGATACGGCTACTATGTGTTCGACCACTGGGGCCAAGGCACCACACTCACC

  261   V  S  S  E  P  K  S  C  D  K  T  H  T  C  P  P  C  G  G  G
  781   GTGTCCTCCGAGCCCAAGTCCTGCGACAAGACACACACCTGTCCCCCTTGTGGAGGAGGA
                Hinge/Extension
  281   S  S  G  G  G  S  G  G  Q  P  R  E  P  Q  V  Y  T  L  P  P
  841   TCCTCCGGAGGCGGCTCCGGCGGACAGCCTAGGGAGCCCCAGGTGTACACACTGCCCCCT
                                  CH3
  301   S  R  D  E  L  T  K  N  Q  V  S  L  T  C  L  V  K  G  F  Y
  901   TCCAGGGACGAACTCACCAAGAACCAGGTGTCCCTGACCTGCCTGGTGAAGGGATTCTAC

  321   P  S  D  I  A  V  E  W  E  S  N  G  Q  P  E  N  N  Y  K  T
  961   CCCAGCGACATCGCCGTGGAGTGGGAGTCCAACGGCCAACCCGAGAACAATTACAAGACC

  341   T  P  P  V  L  D  S  D  G  S  F  F  L  Y  S  K  L  T  V  D
 1021   ACCCCCCCTGTGCTCGATTCCGACGGCTCCTTCTTCCTGTACTCCAAGCTCACCGTGGAC

  361   K  S  R  W  Q  Q  G  N  V  F  S  C  S  V  M  H  E  A  L  H
 1081   AAGTCCCGGTGGCAACAGGGCAATGTGTTCTCCTGCAGCGTCATGCACGAGGCCCTGCAT

  381   N  H  Y  T  Q  K  S  L  S  L  S  P  G  K  -   SEQ ID NO: 16

 1141   AACCACTACACCCAGAAATCCCTCAGCCTCTCCCCTGGAAAATGA  SEQ ID NO: 17
 aagctt   SEQ ID NO: 64  _HinDIII
```

# FIGURE 5

```
TCTAGAGCCGCCACC   SEQ ID NO:62

XbaI   Kozak

   1   M  E  T  D  T  L  L  L  W  V  L  L  L  W  V  P  G  S  T  G
   1   ATGGAGACCGACACACTCCTGCTCTGGGTGCTCCTGCTGTGGGTGCCTGGCAGCACAGGA
  Signal Peptide
  21   E  V  Q  L  Q  Q  S  G  A  E  L  V  K  P  G  A  S  V  K  L
  61   GAAGTGCAGCTGCAGCAGTCCGGCGCCGAACTCGTCAAACCCGGAGCCTCCGTCAAACTG
       VH
  41   S  C  T  A  S  G  F  N  I  K  D  T  Y  I  H  F  V  R  Q  R
 121   TCCTGCACAGCCAGCGGCTTCAACATCAAGGACACCTACATCCATTTCGTGCGGCAAAGG

  61   P  E  Q  G  L  E  W  I  G  R  I  D  P  A  N  D  N  T  L  Y
 181   CCTGAACAGGGACTCGAGTGGATCGGCAGGATCGACCCCGCCAACGACAATACCCTCTAC

  81   A  S  K  F  Q  G  K  A  T  I  T  A  D  T  S  S  N  T  A  Y
 241   GCCTCCAAGTTCCAGGGAAAGGCCACCATTACCGCCGACACATCCAGCAACACCGCCTAC

 101   M  H  L  S  S  L  T  S  G  D  T  A  V  Y  Y  C  G  R  G  Y
 301   ATGCACCTCAGCTCCCTGACATCCGGCGACACCGCCGTGTACTACTGCGGCAGGGGCTAC

 121   G  Y  Y  V  F  D  H  W  G  Q  G  T  T  L  T  V  S  S  G  S
 361   GGCTACTACGTGTTTGACCACTGGGGCCAGGGAACAACCCTGACCGTGTCCAGCGGCTCC
                                                               Linker
 141   T  S  G  G  G  S  G  G  G  S  G  G  G  G  S  S  D  V  Q  I
 421   ACCTCCGGAGGCGGAAGCGGCGGAGGATCCGGAGGAGGAGGCTCCTCCGACGTGCAAATC
                                                         VL
 161   N  Q  S  P  S  F  L  A  A  S  P  G  E  T  I  T  I  N  C  R
 481   AACCAGTCCCCTAGCTTCCTGGCCGCTAGCCCTGGCGAGACAATCACAATCAATTGTCGG

 181   T  S  R  S  I  S  Q  Y  L  A  W  Y  Q  E  K  P  G  K  T  N
 541   ACCAGCCGGTCCATCTCCCAGTATCTGGCCTGGTACCAGGAGAAGCCCGGCAAGACAAAC

 201   K  L  L  I  Y  S  G  S  T  L  Q  S  G  I  P  S  R  F  S  G
 601   AAGCTGCTCATCTACAGCGGCAGCACCCTCCAATCCGGCATCCCTTCCCGGTTTAGCGGC

 221   S  G  S  G  T  D  F  T  L  T  I  S  G  L  E  P  E  D  F  A
 661   TCCGGATCCGGAACCGACTTTACCCTGACCATCAGCGGCCTGGAACCCGAGGATTTCGCC

 241   M  Y  Y  C  Q  Q  H  N  E  N  P  L  T  F  G  A  G  T  K  L
 721   ATGTACTACTGCCAGCAGCACAACGAGAATCCCCTGACCTTTGGAGCCGGCACAAAGCTC

 261   E  L  K  E  P  K  S  C  D  K  T  H  T  C  P  P  C  G  G  G
 781   GAGCTGAAGGAGCCCAAGAGCTGCGACAAAACCCACACCTGTCCCCCTTGCGGAGGAGGA
           Hinge/Extension
 281   S  S  G  G  G  S  G  G  Q  P  R  E  P  Q  V  Y  T  L  P  P
 841   TCCTCCGGCGGCGGAAGCGGAGGACAACCCAGGGAGCCCCAGGTCTACACCCTGCCTCCT
                CH3
 301   S  R  D  E  L  T  K  N  Q  V  S  L  T  C  L  V  K  G  F  Y
 901   AGCCGGGACGAACTGACAAAGAACCAGGTGTCCCTGACCTGTCTCGTCAAGGGCTTCTAC

 321   P  S  D  I  A  V  E  W  E  S  N  G  Q  P  E  N  N  Y  K  T
 961   CCTTCCGACATCGCCGTCGAGTGGGAAAGCAACGGCCAGCCCGAGAACAATTACAAGACC

 341   T  P  P  V  L  D  S  D  G  S  F  F  L  Y  S  K  L  T  V  D
1021   ACACCCCCCGTCCTGGACAGCGATGGCAGCTTCTTCCTCTACTCCAAGCTGACCGTGGAC

 361   K  S  R  W  Q  Q  G  N  V  F  S  C  S  V  M  H  E  A  L  H
1081   AAGAGCCGGTGGCAACAAGGCAACGTGTTCTCCTGCAGCGTCATGCATGAGGCCCTGCAC

 381   N  H  Y  T  Q  K  S  L  S  L  S  P  G  K  -  SEQ ID NO: 18
1141   AATCACTACACCCAGAAGAGCCTGAGCCTCTCCCCCGGCAAGTGA SEQ ID NO: 19
                                                    Stop
AAGCTT SEQ ID NO: 63   HinDIII
```

# FIGURE 6

```
Tctagagccgccacc   SEQ ID NO: 65
 XbaI Kozak

   1  M  E  T  D  T  L  L  L  W  V  L  L  L  W  V  P  G  S  T  G
   1  ATGGAGACAGACACCCTCCTGCTGTGGGTCCTGCTGCTGTGGGTGCCTGGCAGCACAGGA
 Signal Peptide
  21  D  I  Q  M  T  Q  S  P  S  S  L  S  A  S  V  G  D  R  V  T
  61  GACATCCAAATGACCCAGTCCCCTAGCAGCCTCAGCGCTTCCGTCGGAGACAGGGTCACC
       VL
  41  I  T  C  R  T  S  R  S  I  S  Q  Y  L  A  W  Y  Q  Q  K  P
 121  ATCACATGCAGGACCTCCAGGTCCATCAGCCAGTATCTGGCCTGGTATCAGCAGAAACCC

  61  G  K  V  P  K  L  L  I  Y  S  G  S  T  L  Q  S  G  V  P  S
 181  GGCAAGGTGCCTAAGCTGCTGATCTACAGCGGCAGCACACTCCAGAGCGGAGTGCCCAGC

  81  R  F  S  G  S  G  S  G  T  D  F  T  L  T  I  S  S  L  Q  P
 241  CGGTTTTCCGGAAGCGGATCCGGAACCGACTTCACCCTGACCATTTCCAGCCTGCAACCT

 101  E  D  V  A  T  Y  Y  C  Q  Q  H  N  E  N  P  L  T  F  G  G
 301  GAAGACGTGGCCACCTACTACTGTCAGCAGCACAACGAGAACCCCCTCACCTTCGGCGGA

 121  G  T  K  V  E  I  K  G  S  T  S  G  G  G  S  G  G  G  S  G
 361  GGCACCAAAGTCGAGATCAAGGGCAGCACCAGCGGAGGAGGAAGCGGCGGAGGCTCCGGA
                             Linker
 141  G  G  G  S  S  Q  V  Q  L  V  Q  S  G  A  E  V  K  K  P  G
 421  GGAGGAGGCTCCTCCCAAGTGCAGCTCGTCCAAAGCGGCGCTGAGGTGAAAAAGCCCGGC
                      VH
 161  A  T  V  K  I  S  C  K  V  S  G  F  N  I  K  D  T  Y  I  H
 481  GCCACAGTCAAAATCTCCTGCAAGGTCAGCGGCTTCAACATCAAGGATACCTACATCCAC

 181  W  V  Q  Q  A  P  G  K  G  L  E  W  M  G  R  I  D  P  A  N
 541  TGGGTGCAACAGGCCCCCGGCAAAGGACTCGAATGGATGGGCCGGATCGACCCTGCTAAC

 201  D  N  T  L  Y  A  S  K  F  Q  G  R  V  T  I  T  A  D  T  S
 601  GACAACACACTCTACGCCTCCAAGTTCCAGGGCAGGGTGACCATCACCGCCGATACCTCC

 221  T  D  T  A  Y  M  E  L  S  S  L  R  S  E  D  T  A  V  Y  Y
 661  ACCGACACAGCCTACATGGAGCTGAGCAGCCTGAGGTCCGAGGACACCGCCGTCTATTAC

 241  C  A  R  G  Y  G  Y  Y  V  F  D  H  W  G  Q  G  T  L  V  T
 721  TGCGCCCGGGGATACGGCTACTACGTGTTTGACCATTGGGGACAGGGAACACTCGTGACC

 261  V  S  S  E  P  K  S  C  D  K  T  H  T  C  P  P  C  G  G  G
 781  GTGAGCTCCGAGCCCAAGAGCTGCGACAAGACCCACACATGTCCTCCTTGCGGAGGAGGC
         Hinge/Extension
 281  S  S  G  G  G  S  G  G  Q  P  R  E  P  Q  V  Y  T  L  P  P
 841  AGCTCCGGAGGCGGATCCGGCGGACAACCTAGGGAGCCCCAGGTCTATACCCTGCCCCCC
                              CH3
 301  S  R  D  E  L  T  K  N  Q  V  S  L  T  C  L  V  K  G  F  Y
 901  AGCAGGGACGAGCTGACAAAGAACCAGGTCTCCCTGACCTGCCTGGTGAAAGGATTCTAC

 321  P  S  D  I  A  V  E  W  E  S  N  G  Q  P  E  N  N  Y  K  T
 961  CCCAGCGACATCGCTGTCGAATGGGAGTCCAACGGCCAGCCCGAGAACAACTACAAGACA

 341  T  P  P  V  L  D  S  D  G  S  F  F  L  Y  S  K  L  T  V  D
1021  ACCCCCCCCGTGCTGGATTCCGACGGCAGCTTCTTCCTCTACTCCAAGCTGACCGTCGAC

 361  K  S  R  W  Q  Q  G  N  V  F  S  C  S  V  M  H  E  A  L  H
1081  AAGTCCAGGTGGCAGCAGGGCAACGTGTTTTCCTGCTCCGTGATGCATGAGGCCCTGCAC

 381  N  H  Y  T  Q  K  S  L  S  L  S  P  G  K  -   SEQ ID NO: 20
1141  AACCACTACACCCAGAAGTCCCTGAGCCTCAGCCCTGGCAAGTGA  SEQ ID NO: 21
                                                    Stop
aagctt      SEQ ID NO: 66  HinDIII
```

# FIGURE 7

```
Tctagagccgccacc    SEQ ID NO: 67

 XbaI Kozak

   1   M  E  T  D  T  L  L  L  W  V  L  L  L  W  V  P  G  S  T  G
   1  ATGGAGACCGATACACTGCTGCTCTGGGTGCTGCTGCTGTGGGTGCCTGGAAGCACCGGA
  Signal Peptide
  21   Q  V  Q  L  V  Q  S  G  A  E  V  K  K  P  G  A  T  V  K  I
  61  CAGGTGCAACTGGTCCAGTCCGGCGCCGAGGTGAAAAAGCCTGGCGCCACCGTCAAGATC
   VH
  41   S  C  K  V  S  G  F  N  I  K  D  T  Y  I  H  W  V  Q  Q  A
 121  TCCTGTAAGGTGAGCGGCTTCAACATCAAGGACACCTACATCCACTGGGTGCAGCAGGCT

  61   P  G  K  G  L  E  W  M  G  R  I  D  P  A  N  D  N  T  L  Y
 181  CCCGGAAAGGGACTGGAGTGGATGGGCAGGATCGACCCTGCCAATGACAACACCCTCTAC

  81   A  S  K  F  Q  G  R  V  T  I  T  A  D  T  S  T  D  T  A  Y
 241  GCCAGCAAGTTCCAAGGACGGGTGACCATCACAGCCGACACATCCACCGACACAGCCTAT

 101   M  E  L  S  S  L  R  S  E  D  T  A  V  Y  Y  C  A  R  G  Y
 301  ATGGAGCTCTCCAGCCTGAGGTCCGAGGACACCGCCGTGTACTACTGTGCCAGGGGATAC

 121   G  Y  Y  V  F  D  H  W  G  Q  G  T  L  V  T  V  S  S  G  S
 361  GGCTATTACGTGTTCGACCACTGGGGACAGGGCACCCTGGTGACCGTGAGCAGCGGAAGC
                                                               Linker
 141   T  S  G  G  G  S  G  G  G  S  G  G  G  G  S  S  D  I  Q  M
 421  ACCAGCGGCGGAGGCAGCGGAGGCGGAAGCGGCGGCGGCGGATCCTCCGACATTCAGATG
                                                            VL
 161   T  Q  S  P  S  S  L  S  A  S  V  G  D  R  V  T  I  T  C  R
 481  ACCCAATCCCCCTCCAGCCTGTCCGCTAGCGTGGGAGACAGGGTGACAATCACATGTCGG

 181   T  S  R  S  I  S  Q  Y  L  A  W  Y  Q  Q  K  P  G  K  V  P
 541  ACCTCCAGGTCCATCAGCCAATATCTCGCCTGGTATCAGCAGAAGCCCGGCAAGGTGCCC

 201   K  L  L  I  Y  S  G  S  T  L  Q  S  G  V  P  S  R  F  S  G
 601  AAGCTCCTGATCTACAGCGGCTCCACCCTCCAAAGCGGAGTGCCTTCCCGGTTTAGCGGA

 221   S  G  S  G  T  D  F  T  L  T  I  S  S  L  Q  P  E  D  V  A
 661  AGCGGCAGCGGCACAGACTTTACCCTGACAATCAGCTCCCTGCAACCTGAGGACGTCGCC

 241   T  Y  Y  C  Q  Q  H  N  E  N  P  L  T  F  G  G  G  T  K  V
 721  ACATACTACTGCCAGCAGCACAACGAGAACCCTCTCACCTTTGGCGGCGGCACCAAAGTG

 261   E  I  K  E  P  K  S  C  D  K  T  H  T  C  P  P  C  G  G  G
 781  GAGATCAAGGAGCCCAAATCCTGCGACAAGACACACACCTGCCCCCCTTGTGGAGGAGGC
      Hinge/Extension
 281   S  S  G  G  G  S  G  G  Q  P  R  E  P  Q  V  Y  T  L  P  P
 841  AGCTCCGGCGGCGGCAGCGGCGGACAACCCCGGGAACCTCAGGTGTATACACTCCCCCCT
                            CH3
 301   S  R  D  E  L  T  K  N  Q  V  S  L  T  C  L  V  K  G  F  Y
 901  TCCAGGGATGAGCTGACCAAGAACCAAGTCTCCCTGACCTGTCTGGTGAAAGGCTTCTAC

 321   P  S  D  I  A  V  E  W  E  S  N  G  Q  P  E  N  N  Y  K  T
 961  CCCTCCGACATCGCTGTCGAGTGGGAGAGCAACGGCCAGCCCGAAAACAACTATAAGACC

 341   T  P  P  V  L  D  S  D  G  S  F  F  L  Y  S  K  L  T  V  D
1021  ACCCCCCCCGTGCTCGATTCCGATGGCAGCTTCTTCCTGTACTCCAAGCTCACAGTCGAC

 361   K  S  R  W  Q  Q  G  N  V  F  S  C  S  V  M  H  E  A  L  H
1081  AAGAGCCGGTGGCAACAGGGCAACGTCTTCTCCTGTAGCGTCATGCACGAGGCCCTCCAC

 381   N  H  Y  T  Q  K  S  L  S  L  S  P  G  K  -          SEQ ID NO: 22
1141  AACCACTACACCCAGAAGTCCCTCTCCCTGAGCCCCGGAAAATGA          SEQ ID NO: 23
                                                        Stop
aagctt    SEQ ID NO: 68   _HinDIII
```

# FIGURE 8

```
Tctagagccgccacc  SEQ ID NO: 69

 XbaI Kozak


  1   M  E  T  D  T  L  L  L  W  V  L  L  L  W  V  P  G  S  T  G
  1   ATGGAGACCGATACACTGCTGCTGTGGGTGCTGCTGCTCTGGGTCCCTGGCAGCACAGGA
    Signal Peptide
 21   D  I  Q  M  T  Q  S  P  S  S  L  S  A  S  V  G  D  R  V  T
 61   GACATCCAGATGACACAGAGCCCTAGCTCCCTGAGCGCTTCCGTGGGAGATAGGGTGACC
      VL
 41   I  T  C  R  T  S  R  S  I  S  Q  Y  L  A  W  Y  Q  Q  K  P
121   ATCACATGCCGGACCTCCAGGTCCATCTCCCAGTACCTGGCCTGGTACCAGCAGAAGCCC

 61   G  K  V  P  K  L  L  I  Y  S  G  S  T  L  Q  S  G  V  P  S
181   GGCAAGGTGCCCAAGCTGCTCATCTATAGCGGCAGCACCCTGCAGAGCGGAGTGCCTTCC

 81   R  F  S  G  S  G  S  G  T  D  F  T  L  T  I  S  S  L  Q  P
241   CGGTTTTCCGGATCCGGCTCCGGCACAGACTTTACCCTGACCATCTCCAGCCTGCAGCCT

101   E  D  V  A  T  Y  Y  C  Q  Q  H  N  E  N  P  L  T  F  G  G
301   GAGGATGTCGCCACCTACTACTGCCAACAGCACAACGAGAACCCCCTGACCTTCGGCGGC

121   G  T  K  V  E  I  K  S  G  G  G  G  Q  V  Q  L  V  Q  S  G
361   GGAACCAAGGTCGAGATCAAGTCCGGAGGAGGAGGCCAAGTGCAGCTGGTCCAATCCGGC
                           Linker           VH
141   A  E  V  K  K  P  G  A  T  V  K  I  S  C  K  V  S  G  F  N
421   GCCGAAGTGAAAAAGCCCGGCGCCACCGTGAAGATCAGCTGCAAGGTGTCCGGCTTCAAC

161   I  K  D  T  Y  I  H  W  V  Q  Q  A  P  G  K  G  L  E  W  M
481   ATCAAGGACACCTATATCCACTGGGTCCAGCAAGCCCCCGGAAAAGGCCTGGAGTGGATG

181   G  R  I  D  P  A  N  D  N  T  L  Y  A  S  K  F  Q  G  R  V
541   GGACGGATTGACCCCGCCAACGACAACACACTCTATGCCTCCAAGTTCCAGGGCAGGGTG

201   T  I  T  A  D  T  S  T  D  T  A  Y  M  E  L  S  S  L  R  S
601   ACAATCACCGCCGACACCAGCACCGACACAGCTTATATGGAGCTGTCCTCCCTCCGGTCC

221   E  D  T  A  V  Y  Y  C  A  R  G  Y  G  Y  Y  V  F  D  H  W
661   GAGGATACCGCCGTCTACTACTGCGCCAGGGGCTACGGCTACTACGTGTTTGACCACTGG

241   G  Q  G  T  L  V  T  V  S  S  G  G  C  SEQ ID NO: 12
721   GGCCAGGGCACCCTGGTGACAGTGTCCAGCGGAGGCTGC  SEQ ID NO: 77
                                    Cys
aagctt   SEQ ID NO: 70

HinDIII
```

# FIGURE 9

```
Tctagagccgccacc  SEQ ID NO: 71

 XbaI Kozak

  1   M  E  T  D  T  L  L  L  W  V  L  L  L  W  V  P  G  S  T  G
  1   ATGGAGACCGACACCCTGCTGCTCTGGGTCCTCCTGCTGTGGGTGCCTGGCAGCACAGGA
     Signal Peptide
 21   Q  V  Q  L  V  Q  S  G  A  E  V  K  K  P  G  A  T  V  K  I
 61   CAGGTGCAACTGGTGCAGAGCGGCGCCGAGGTCAAGAAACCTGGCGCCACCGTGAAGATC
      VH
 41   S  C  K  V  S  G  F  N  I  K  D  T  Y  I  H  W  V  Q  Q  A
121   AGCTGCAAGGTGTCCGGCTTCAACATCAAGGACACCTACATCCACTGGGTCCAACAAGCC

 61   P  G  K  G  L  E  W  M  G  R  I  D  P  A  N  D  N  T  L  Y
181   CCCGGAAAGGGCCTGGAATGGATGGGCCGGATTGACCCCGCCAACGACAACACCCTCTAT

 81   A  S  K  F  Q  G  R  V  T  I  T  A  D  T  S  T  D  T  A  Y
241   GCCAGCAAGTTCCAGGGCAGGGTCACCATCACCGCCGACACCAGCACCGACACCGCCTAC

101   M  E  L  S  S  L  R  S  E  D  T  A  V  Y  Y  C  A  R  G  Y
301   ATGGAGCTGAGCAGCCTGCGGAGCGAAGACACCGCCGTGTACTACTGCGCCAGGGGCTAC

121   G  Y  Y  V  F  D  H  W  G  Q  G  T  L  V  T  V  S  S  S  G
361   GGCTACTACGTCTTCGACCATTGGGGACAGGGCACCCTCGTGACAGTGTCCAGCTCCGGC
                                                           Linker
141   G  G  G  D  I  Q  M  T  Q  S  P  S  S  L  S  A  S  V  G  D
421   GGAGGAGGAGATATCCAGATGACCCAGAGCCCTTCCAGCCTGTCCGCTTCCGTGGGAGAT
               VL
161   R  V  T  I  T  C  R  T  S  R  S  I  S  Q  Y  L  A  W  Y  Q
481   CGGGTGACCATCACATGCAGGACCTCCAGGTCCATCTCCCAGTACCTGGCCTGGTACCAA

181   Q  K  P  G  K  V  P  K  L  L  I  Y  S  G  S  T  L  Q  S  G
541   CAGAAGCCCGGCAAGGTGCCCAAGCTGCTGATCTACAGCGGCAGCACACTGCAATCCGGC

201   V  P  S  R  F  S  G  S  G  S  G  T  D  F  T  L  T  I  S  S
601   GTCCCTTCCCGGTTTTCCGGATCCGGATCCGGCACCGACTTCACCCTGACCATCAGCTCC

221   L  Q  P  E  D  V  A  T  Y  Y  C  Q  Q  H  N  E  N  P  L  T
661   CTGCAACCCGAGGACGTGGCCACCTACTACTGTCAGCAGCACAACGAGAACCCCCTCACC

241   F  G  G  G  T  K  V  E  I  K  G  G  C  SEQ ID NO: 13
721   TTTGGCGGCGGAACCAAGGTCGAGATCAAGGGCGGCTGC SEQ ID NO: 78
                                    Cys

aagctt  SEQ ID NO: 72

HinDIII
```

# FIGURE 10

Tctagagccgccacc SEQ ID NO: 73

**XbaI** ***Kozak***

```
  1   M  E  T  D  T  L  L  L  W  V  L  L  L  W  V  P  G  S  T  G
  1   ATGGAGACCGATACACTGCTGCTGTGGGTGCTGCTGCTCTGGGTCCCTGGCAGCACAGGA
     Signal Peptide
 21   D  I  Q  M  T  Q  S  P  S  S  L  S  A  S  V  G  D  R  V  T
 61   GACATCCAGATGACACAGAGCCCTAGCTCCCTGAGCGCTTCCGTGGGAGATAGGGTGACC
      VL
 41   I  T  C  R  T  S  R  S  I  S  Q  Y  L  A  W  Y  Q  Q  K  P
121   ATCACATGCCGGACCTCCAGGTCCATCTCCCAGTACCTGGCCTGGTACCAGCAGAAGCCC

 61   G  K  V  P  K  L  L  I  Y  S  G  S  T  L  Q  S  G  V  P  S
181   GGCAAGGTGCCCAAGCTGCTCATCTATAGCGGCAGCACCCTGCAGAGCGGAGTGCCTTCC

 81   R  F  S  G  S  G  S  G  T  D  F  T  L  T  I  S  S  L  Q  P
241   CGGTTTTCCGGATCCGGCTCCGGCACAGACTTTACCCTGACCATCTCCAGCCTGCAGCCT

101   E  D  V  A  T  Y  Y  C  Q  Q  H  N  E  N  P  L  T  F  G  G
301   GAGGATGTCGCCACCTACTACTGCCAACAGCACAACGAGAACCCCCTGACCTTCGGCGGC

121   G  T  K  V  E  I  K  G  G  G  S  G  G  G  G  Q  V  Q  L  V
361   GGAACCAAGGTCGAGATCAAGGGAGGAGGCTCCGGAGGAGGAGGCCAAGTGCAGCTGGTC
                              Linker                VH
141   Q  S  G  A  E  V  K  K  P  G  A  T  V  K  I  S  C  K  V  S
421   CAATCCGGCGCCGAAGTGAAAAAGCCCGGCGCCACCGTGAAGATCAGCTGCAAGGTGTCC

161   G  F  N  I  K  D  T  Y  I  H  W  V  Q  Q  A  P  G  K  G  L
481   GGCTTCAACATCAAGGACACCTATATCCACTGGGTCCAGCAAGCCCCCGGAAAAGGCCTG

181   E  W  M  G  R  I  D  P  A  N  D  N  T  L  Y  A  S  K  F  Q
541   GAGTGGATGGGACGGATTGACCCCGCCAACGACAACACACTCTATGCCTCCAAGTTCCAG

201   G  R  V  T  I  T  A  D  T  S  T  D  T  A  Y  M  E  L  S  S
601   GGCAGGGTGACAATCACCGCCGACACCAGCACCGACACAGCTTATATGGAGCTGTCCTCC

221   L  R  S  E  D  T  A  V  Y  Y  C  A  R  G  Y  G  Y  Y  V  F
661   CTCCGGTCCGAGGATACCGCCGTCTACTACTGCGCCAGGGGCTACGGCTACTACGTGTTT

241   D  H  W  G  Q  G  T  L  V  T  V  S  S  G  G  C          SEQ ID NO: 14
721   GACCACTGGGGCCAGGGCACCCTGGTGACAGTGTCCAGCGGAGGCTGC        SEQ ID NO: 10
                                              CYS
```

***aagctt*** SEQ ID NO:74

***HinDIII***

# FIGURE 11

Tctagagccgccacc SEQ ID NO:75

**XbaI** ***Kozak***

```
  1   M  E  T  D  T  L  L  L  W  V  L  L  L  W  V  P  G  S  T  G
  1   ATGGAGACCGACACCCTGCTGCTCTGGGTCCTCCTGCTGTGGGTGCCTGGCAGCACAGGA
     Signal Peptide
 21   Q  V  Q  L  V  Q  S  G  A  E  V  K  K  P  G  A  T  V  K  I
 61   CAGGTGCAACTGGTGCAGAGCGGCGCCGAGGTCAAGAAACCTGGCGCCACCGTGAAGATC
      VH
 41   S  C  K  V  S  G  F  N  I  K  D  T  Y  I  H  W  V  Q  Q  A
121   AGCTGCAAGGTGTCCGGCTTCAACATCAAGGACACCTACATCCACTGGGTCCAACAAGCC

 61   P  G  K  G  L  E  W  M  G  R  I  D  P  A  N  D  N  T  L  Y
181   CCCGGAAAGGGCCTGGAATGGATGGGCCGGATTGACCCCGCCAACGACAACACCCTCTAT

 81   A  S  K  F  Q  G  R  V  T  I  T  A  D  T  S  T  D  T  A  Y
241   GCCAGCAAGTTCCAGGGCAGGGTCACCATCACCGCCGACACCAGCACCGACACCGCCTAC

101   M  E  L  S  S  L  R  S  E  D  T  A  V  Y  Y  C  A  R  G  Y
301   ATGGAGCTGAGCAGCCTGCGGAGCGAAGACACCGCCGTGTACTACTGCGCCAGGGGCTAC


121   G  Y  Y  V  F  D  H  W  G  Q  G  T  L  V  T  V  S  S  G  G
361   GGCTACTACGTCTTCGACCATTGGGGACAGGGCACCCTCGTGACAGTGTCCAGCGGAGGA
                                                           Linker
141   G  S  G  G  G  G  D  I  Q  M  T  Q  S  P  S  S  L  S  A  S
421   GGATCCGGCGGAGGAGGAGATATCCAGATGACCCAGAGCCCTTCCAGCCTGTCCGCTTCC
                        VL
161   V  G  D  R  V  T  I  T  C  R  T  S  R  S  I  S  Q  Y  L  A
481   GTGGGAGATCGGGTGACCATCACATGCAGGACCTCCAGGTCCATCTCCCAGTACCTGGCC

181   W  Y  Q  Q  K  P  G  K  V  P  K  L  L  I  Y  S  G  S  T  L
541   TGGTACCAACAGAAGCCCGGCAAGGTGCCCAAGCTGCTGATCTACAGCGGCAGCACACTG

201   Q  S  G  V  P  S  R  F  S  G  S  G  S  G  T  D  F  T  L  T
601   CAATCCGGCGTCCCTTCCCGGTTTTCCGGATCCGGATCCGGCACCGACTTCACCCTGACC

221   I  S  S  L  Q  P  E  D  V  A  T  Y  Y  C  Q  Q  H  N  E  N
661   ATCAGCTCCCTGCAACCCGAGGACGTGGCCACCTACTACTGTCAGCAGCACAACGAGAAC

241   P  L  T  F  G  G  G  T  K  V  E  I  K  G  G  C       SEQ ID NO: 15
721   CCCCTCACCTTTGGCGGCGGAACCAAGGTCGAGATCAAGGGCGGCTGC     SEQ ID NO: 11
                                               CYS
```

aagctt SEQ ID NO:76

HinDIII

FIG. 12A Cys-diabodies

**Leader sequence:**
```
atggaaaccgacaccctgctgctgtgggtgctgctgctctgggtcccaggctccaccggt SEQ ID NO: 25
 M   E   T   D   T   L   L   L   W   V   L   L   L   W   V   P   G   S   T   G   SEQ ID NO: 26
```

**Five aa linker:**
```
agtggtggaggaggc  SEQ ID NO:27
 S   G   G   G   G    SEQ ID NO: 28
```

**Eight aa linker:**
```
ggcggagggagtggcggaggcggc      SEQ ID NO: 29
 G   G   G   S   G   G   G   G      SEQ ID NO:30
```

**Cysteine tail:**
```
ggcggctgc  SEQ ID NO: 31
 G   G   C   SEQ ID NO: 32
```

FIG. 12B Minibodies

**Leader sequence:**
```
atggaaaccgacaccctgctgctgtgggtgctgctgctctgggtcccaggctccaccggt  SEQ ID NO: 33
 M   E   T   D   T   L   L   L   W   V   L   L   L   W   V   P   G   S   T   G   SEQ ID NO: 34
```

**Eighteen aa linker:**
```
ggctccacatccggcggaggctctggcggtggatctggcggaggcggctcatcc SEQ ID NO: 35
 G   S   T   S   G   G   G   S   G   G   G   S   G   G   G   G   S   S   SEQ ID NO: 36
```

**IgG1 hinge/linker-CH3 domain:**
```
gagcctaagtcctgcgacaagacccacacctgtccccttgcggcggaggaagcagcgga
 E   P   K   S   C   D   K   T   H   T   C   P   P   C   G   G   G   S   S   G
ggcggatccggtggccagcctcgggagcctcaggtgtacaccctgcctccctcccgggac
 G   G   S   G   G   Q   P   R   E   P   Q   V   Y   T   L   P   P   S   R   D
gagctgaccaagaaccaggtgtccctgacctgtctggtcaagggcttctacccttccgat
 E   L   T   K   N   Q   V   S   L   T   C   L   V   K   G   F   Y   P   S   D
atcgccgtggagtgggagtccaacggccagcctgagaacaactacaagaccacccctcct
 I   A   V   E   W   E   S   N   G   Q   P   E   N   N   Y   K   T   T   P   P
gtgctggactccgacggctccttcttcctgtactccaagctcacagtggataagtcccgg
 V   L   D   S   D   G   S   F   F   L   Y   S   K   L   T   V   D   K   S   R
tggcagcagggcaacgtgttctcctgctccgtgatgcacgaggccctgcacaaccactat
 W   Q   Q   G   N   V   F   S   C   S   V   M   H   E   A   L   H   N   H   Y
acccagaagtccctgtccctgtctcctggcaag   SEQ ID NO: 37
 T   Q   K   S   L   S   L   S   P   G   K     SEQ ID NO: 38
```

**FIG. 12B Continued**

Hinge regions:

Amino acid sequences of IgG hinge regions and variants.

| | Upper | Middle | Lower/extension |
|---|---|---|---|
| IgG1 **(SEQ ID NO: 53)** | EPKSCDKTHT | CPPCP | APELLGGP |
| IgG1v1 **(SEQ ID NO: 54)** | EPKSCDKTHT | CPPCP | GGGSSGGGSG |
| IgG2.NH **(SEQ ID NO: 55)** | ERK | CCVECPPCP | APPVA-GP |
| IgG3 **(SEQ ID NO: 56)** | ELKTPLGDTTHT | CPRCP(EPKSCDTPPPCPRCP)X3 | APELLGGP |
| IgG3v1 **(SEQ ID NO: 57)** | ELKTPLGDTTHT | CPRCP | APELLGGP |
| IgG3v2 **(SEQ ID NO: 58)** | EPKSCDTPPP | CPRCP | APELLGGP |
| IgG4 **(SEQ ID NO: 59)** | ESKYGPP | CPSCP | APEFLGGP |
| IgG4v1 **(SEQ ID NO: 60)** | ESKYGPP | CPPCP | APEFLGGP |
| IgG2.EH **(SEQ ID NO: 79)** | ERK | CCVECPPCP | GGGSSGGGSG |

CH3 Domains:

**Alignment of the hIgG2 CH3 vs hIgG1 CH3 (Amino Acid Sequences):**

```
hIgG2 CH3    GQPREPQVYTLPPSREEMTKNQVSLTCLVKGFYPSDIAVEWESNGQPENNYKTTPPMLDS
hIgG1 CH3    GQPREPQVYTLPPSRDELTKNQVSLTCLVKGFYPSDIAVEWESNGQPENNYKTTPPVLDS
             ***************:*:**************************************:***

hIgG2 CH3    DGSFFLYSKLTVDKSRWQQGNVFSCSVMHEALHNHYTQKSLSLSPGK   (SEQ ID NO: 80)
hIgG1 CH3    DGSFFLYSKLTVDKSRWQQGNVFSCSVMHEALHNHYTQKSLSLSPGK-  (SEQ ID NO: 81)
             ***********************************************
```

FIG. 12C

**Murine VL**

gatgtccagataaaccagtctccatcttttcttgctgcgtctcctggagaaaccattact

D V Q I N Q S P S F L A A S P G E T I T

ataaattgc aggacaagtaggagtattagtcaatatttagcc tggtatcaagagaaacct

I N C R T S R S I S Q Y L A W Y Q E K P

gggaaaactaataagcttcttatctac tctggatccactctgcaatct ggaattccatca

G K T N K L L I Y S G S T L Q S G I P S

aggttcagtggcagtggatctggtacagatttcactctcaccatcagtggcctggagcct

R F S G S G S G T D F T L T I S G L E P

gaagattttgcaatgtattactgt caacagcataatgaaaacccgctcacg ttcggtgct

E D F A M Y Y C Q Q H N E N P L T F G A

gggaccaagctggagctgaag SEQ ID NO: 39

G T K L E L K SEQ ID NO: 40

FIG. 12D

**huVL**

gacgtccagataacccagtctccatcctccctgtctgcatctgtaggagacagagtcacc

D V Q I T Q S P S S L S A S V G D R V T

atcacttgc aggacaagtaggagtattagtcaatatttagcc tggtatcagcagaaacca

I T C R T S R S I S Q Y L A W Y Q Q K P

gggaaagttcctaagctcctgatctat tctggatccactctgcaatct ggagtcccatct

G K V P K L L I Y S G S T L Q S G V P S

cggttcagtggcagtggatctgggacagatttcactctcaccatcagcagcctgcagcct

R F S G S G S G T D F T L T I S S L Q P

gaagatgttgcaacttattactgt caacagcataatgaaaacccgctcacg ttcggcgga

E D V A T Y Y C Q Q H N E N P L T F G G

gggaccaaggtggagatcaaa SEQ ID NO: 41

G T K V E I K SEQ ID NO: 42

FIG. 12E

**Murine VH**

```
gaggtccagctgcagcagtctggggcagagcttgtgaagccaggggcctcagtcaagttg
 E   V   Q   L   Q   Q   S   G   A   E   L   V   K   P   G   A   S   V   K   L
tcctgcacagcttct [ggcttcaacattaaagac] acctatatacacttcgtgaggcagagg
 S   C   T   A   S  [ G   F   N   I   K   D ]  T   Y   I   H   F   V   R   Q   R
cctgaacagggcctggagtggattgga [aggattgatcctgcgaatgataatact] ttatat
 P   E   Q   G   L   E   W   I   G  [ R   I   D   P   A   N   D   N   T ]  L   Y
gcctcaaagttccagggcaaggccactataacagcagacacatcatccaacacagcctac
 A   S   K   F   Q   G   K   A   T   I   T   A   D   T   S   S   N   T   A   Y
atgcacctctgcagcctgacatctggggacactgccgtctattactgtggtaga [ggttat
 M   H   L   C   S   L   T   S   G   D   T   A   V   Y   Y   C   G   R  [ G   Y
ggttactacgtatttgaccac] tggggccaaggcaccactctcacagtctcctca   SEQ ID NO: 43
 G   Y   Y   V   F   D   H ]  W   G   Q   G   T   T   L   T   V   S   S   SEQ ID NO: 44
```

FIG. 12F

**huVH version a (From VH version 1)**

```
gaagtgcagctggtggaaagcggcggcggcctggtgcagccgggcggcagcctgcgcctg
 E   V   Q   L   V   E   S   G   G   G   L   V   Q   P   G   G   S   L   R   L
agctgcgcggcgagc [ggctttaacattaaagat] acctatattcattttgtgcgccaggcg
 S   C   A   A   S  [ G   F   N   I   K   D ]  T   Y   I   H   F   V   R   Q   A
ccgggcaaaggcctggaatggattggc [cgcattgatccggcgaacgataacacc] ctgtat
 P   G   K   G   L   E   W   I   G  [ R   I   D   P   A   N   D   N   T ]  L   Y
gcgagcaaatttcagggcaaagcgaccattagcgcggataccagcaaaaacaccgcgtat
 A   S   K   F   Q   G   K   A   T   I   S   A   D   T   S   K   N   T   A   Y
ctgcagatgaacagcctgcgcgcgggagataccgcggtgtattattgcggccgc [ggctat
 L   Q   M   N   S   L   R   A   G   D   T   A   V   Y   Y   C   G   R  [ G   Y ]
[ggctattatgtgtttgatcat] tggggccagggcaccctggtgaccgtgagcagc   SEQ ID NO: 45
[ G   Y   Y   V   F   D   H ]  W   G   Q   G   T   L   V   T   V   S   S   SEQ ID NO: 46
```

FIG. 12G

**huVH version b (From VH version 1)**

```
gaagtgcagctggtggaaagcggcggcggcctggtgcagccgggcggcagcctgcgcctg
 E  V  Q  L  V  E  S  G  G  G  L  V  Q  P  G  G  S  L  R  L
agctgcgcggcgagc ggctttaacattaaagat acctatattcattttgtgcgccaggcg
 S  C  A  A  S   G  F  N  I  K  D   T  Y  I  H  F  V  R  Q  A
ccgggcaaaggcctggaatggattggc cgcattgatccggcgaacgataacacc ctgtat
 P  G  K  G  L  E  W  I  G   R  I  D  P  A  N  D  N  T   L  Y
gcgagcaaatttcagggcaaagcgaccattagcgcggataccagcaaaaacaccgcgtat
 A  S  K  F  Q  G  K  A  T  I  S  A  D  T  S  K  N  T  A  Y
ctgcagatgaacagcctgcgcgcggaagataccgcggtgtattattgcggccgc ggctat
 L  Q  M  N  S  L  R  A  E  D  T  A  V  Y  Y  C  G  R   G  Y
ggctattatgtgtttgatcat tggggccagggcaccctggtgaccgtgagcagc  SEQ ID NO: 47
 G  Y  Y  V  F  D  H   W  G  Q  G  T  L  V  T  V  S  S   SEQ ID NO: 48
```

FIG. 12H

**huVH version c (From VH version 2)**

```
caggtgcagctggtgcagagcggcgcggaagtgaaaaaaccgggcgcgaccgtgaaaatt
 Q  V  Q  L  V  Q  S  G  A  E  V  K  K  P  G  A  T  V  K  I
agctgcaaagtgagc ggctttaacattaaagat acctatattcattttgtgcagcaggcg
 S  C  K  V  S   G  F  N  I  K  D   T  Y  I  H  F  V  Q  Q  A
ccgggcaaaggcctggaatggattggc cgcattgatccggcgaacgataacacc ctgtat
 P  G  K  G  L  E  W  I  G   R  I  D  P  A  N  D  N  T   L  Y
gcgagcaaatttcagggcaaagcgaccattaccgcggataccagcaccgataccgcgtat
 A  S  K  F  Q  G  K  A  T  I  T  A  D  T  S  T  D  T  A  Y
atggaactgagcagcctgcgcagcggagataccgcggtgtattattgcggccgc ggctat
 M  E  L  S  S  L  R  S  G  D  T  A  V  Y  Y  C  G  R   G  Y
ggctattatgtgtttgatcat tggggccagggcaccctggtgaccgtgagcagc  SEQ ID NO: 49
 G  Y  Y  V  F  D  H   W  G  Q  G  T  L  V  T  V  S  S   SEQ ID NO: 50
```

FIG. 12I

**huVH version d (From VH version 2)**

```
caggtgcagctggtgcagagcggcgcggaagtgaaaaaaccgggcgcgaccgtgaaaatt
 Q  V  Q  L  V  Q  S  G  A  E  V  K  K  P  G  A  T  V  K  I
agctgcaaagtgagc ggctttaacattaaagat acctatattcattttgtgcagcaggcg
 S  C  K  V  S   G  F  N  I  K  D   T  Y  I  H  F  V  Q  Q  A
ccgggcaaaggcctggaatggattggc cgcattgatccggcgaacgataacacc ctgtat
 P  G  K  G  L  E  W  I  G   R  I  D  P  A  N  D  N  T   L  Y
gcgagcaaatttcagggcaaagcgaccattaccgcggataccagcaccgataccgcgtat
 A  S  K  F  Q  G  K  A  T  I  T  A  D  T  S  T  D  T  A  Y
atggaactgagcagcctgcgcagcgaagataccgcggtgtattattgcggccgc ggctat
 M  E  L  S  S  L  R  S  E  D  T  A  V  Y  Y  C  G  R   G  Y
ggctattatgtgtttgatcat tggggccagggcaccctggtgaccgtgagcagc  SEQ ID NO: 51
 G  Y  Y  V  F  D  H   W  G  Q  G  T  L  V  T  V  S      SEQ ID NO: 52
```

# FIGURE 13

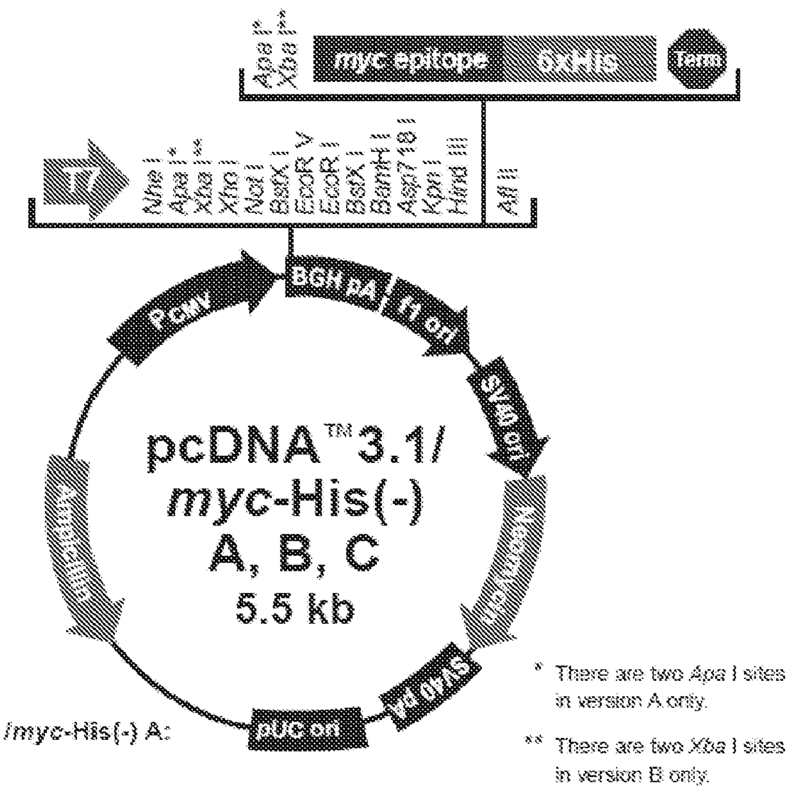


**Comments for pcDNA™3.1/*myc*-His(-) A:**
**5522 nucleotides**

# FIGURE 14

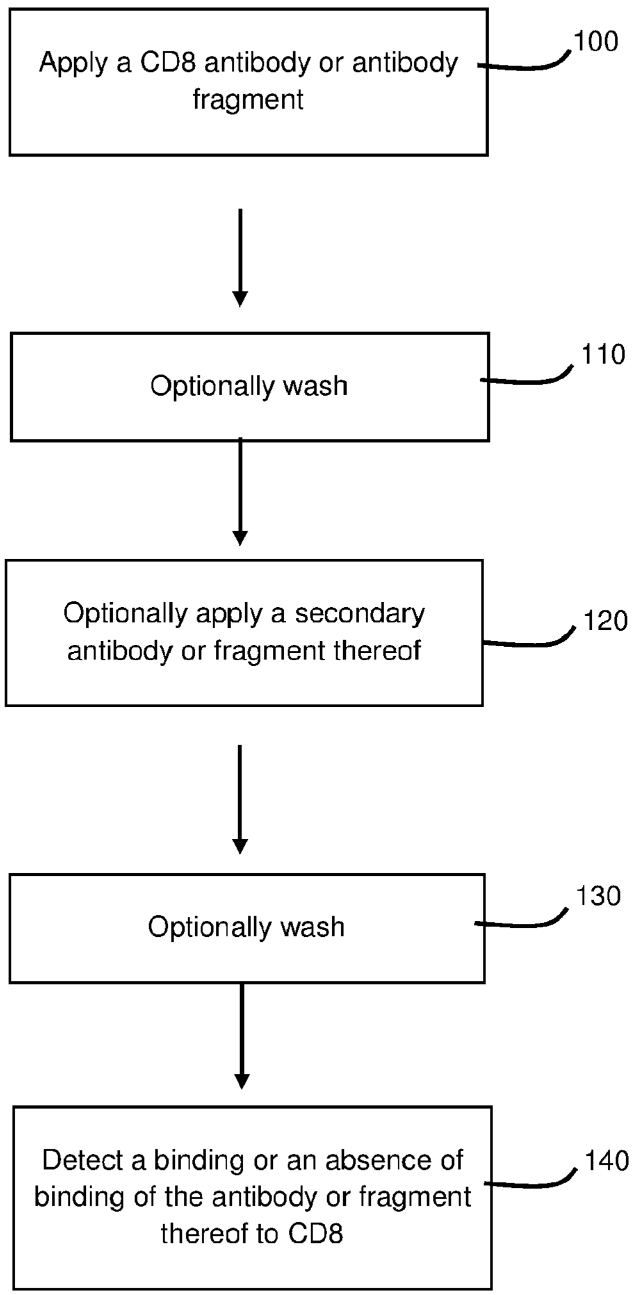
Apply a CD8 antibody or antibody fragment
100
Optionally wash
110
Optionally apply a secondary antibody or fragment thereof
120
Optionally wash
130
Detect a binding or an absence of binding of the antibody or fragment thereof to CD8
140

# FIGURE 15

## IAB22M γ2 EH1 (N- to C-terminal)

Anti-CD8 variable light (VL) sequence
DVQITQSPSSLSASVGDRVTITCRTSRSISQYLAWYQQKPGKVPKLLIYSGSTLQSGVPSRFSGSGSGTDFTLTIS
SLQPEDVATYYCQQHNENPLTFGGGTKVEIK (SEQ ID NO: 82

Linker
GSTSGGGSGGGSGGGGSS (SEQ ID NO: 83

Anti-CD8 variable heavy (VH) sequence
EVQLVESGGGLVQPGGSLRLSCAASGFNIKDTYIHFVRQAPGKGLEWIGRIDPANDNTLYASKFQGKATISADTSK
NTAYLQMNSLRAEDTAVYYCGRGYGYYVFDHWGQGTLVTVSS (SEQ ID NO: 84

Full hinge (EH1)
ERKCCVECPPCPGGGSSGGGSG (SEQ ID NO: 85

Upper hinge
ERK (SEQ ID NO: 86

Core hinge
CCVECPPCP (SEQ ID NO: 87

Lower hinge
GGGSSGGGSG (SEQ ID NO: 88

Human IgG2 CH3
GQPREPQVYTLPPSREEMTKNQVSLTCLVKGFYPSDIAVEWESNGQPENNYKTTPPMLDSDGSFFLYSKLTVDK
SRWQQGNVFSCSVMHEALHNHYTQKSLSLSPGK (SEQ ID NO: 89

# FIGURE 16

## IAB22M $\gamma$2 EH2 (N- to C-terminal)

Anti-CD8 variable light (VL) sequence
DVQITQSPSSLSASVGDRVTITCRTSRSISQYLAWYQQKPGKVPKLLIYSGSTLQSGVPSRFSGSGSGTDFT
LTISSLQPEDVATYYCQQHNENPLTFGGGTKVEIK (SEQ ID NO: 82

Linker
GSTSGGGSGGGSGGGGSS (SEQ ID NO: 83

Anti-CD8 variable heavy (VH) sequence
EVQLVESGGGLVQPGGSLRLSCAASGFNIKDTYIHFVRQAPGKGLEWIGRIDPANDNTLYASKFQGKATISA
DTSKNTAYLQMNSLRAEDTAVYYCGRGYGYYVFDHWGQGTLVTVSS (SEQ ID NO: 84

Full hinge (EH2)
ERKSCVECPPCPGGGSSGGGSG (SEQ ID NO: 90

Upper hinge
ERK (SEQ ID NO: 86

Core hinge
SCVECPPCP (SEQ ID NO: 91

Lower hinge
GGGSSGGGSG (SEQ ID NO: 88

Human IgG2 CH3
GQPREPQVYTLPPSREEMTKNQVSLTCLVKGFYPSDIAVEWESNGQPENNYKTTPPMLDSDGSFFLYSKLT
VDKSRWQQGNVFSCSVMHEALHNHYTQKSLSLSPGK (SEQ ID NO: 89

# FIGURE 17A

## IAB22M γ1 EH1 (N- to C-terminal)

Anti-CD8 variable light (VL) sequence
DVQITQSPSSLSASVGDRVTITCRTSRSISQYLAWYQQKPGKVPKLLIYSGSTLQSGVPSRFSGSGSGTDFTLTISSLQPE
DVATYYCQQHNENPLTFGGGTKVEIK (SEQ ID NO: 82

Linker
GSTSGGGSGGGSGGGGSS (SEQ ID NO: 83

Anti-CD8 variable heavy (VH) sequence
EVQLVESGGGLVQPGGSLRLSCAASGFNIKDTYIHFVRQAPGKGLEWIGRIDPANDNTLYASKFQGKATISADTSKNTAY
LQMNSLRAEDTAVYYCGRGYGYYVFDHWGQGTLVTVSS (SEQ ID NO: 84

Full hinge (EH1)
EPKSCDKTHTCPPCGGGSSGGGSG (SEQ ID NO: 92

Upper hinge
EPKSCDKTHT (SEQ ID NO: 93

Core hinge
CPPC (SEQ ID NO: 94

Lower hinge
GGGSSGGGSG (SEQ ID NO: 88

Human IgG1 CH3 (G1m1 allotype)
GQPREPQVYTLPP<u>SRDEL</u>TKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 95

Human IgG1 CH3 (nG1m1 allotype)
GQPREPQVYTLPP<u>SREEM</u>TKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 96

# FIGURE 17B

## IAB22M $\gamma$2 NH1 (N- to C-terminal)

Anti-CD8 variable light (VL) sequence
DVQITQSPSSLSASVGDRVTITCRTSRSISQYLAWYQQKPGKVPKLLIYSGSTLQSGVPSRFSGSGSGTDFTLTISSL
QPEDVATYYCQQHNENPLTFGGGTKVEIK (SEQ ID NO: 82

Linker
GSTSGGGSGGGSGGGGSS (SEQ ID NO: 83

Anti-CD8 variable heavy (VH) sequence
EVQLVESGGGLVQPGGSLRLSCAASGFNIKDTYIHFVRQAPGKGLEWIGRIDPANDNTLYASKFQGKATISADTSKN
TAYLQMNSLRAEDTAVYYCGRGYGYYVFDHWGQGTLVTVSS (SEQ ID NO: 84

Full hinge (NH1)
ERKCCVECPPCPAPPVAGP (SEQ ID NO: 97

Upper hinge
ERK (SEQ ID NO: 86

Core hinge
CCVECPPCP (SEQ ID NO: 87

Lower hinge
APPVAGP (SEQ ID NO: 98

Human IgG2 CH3
GQPREPQVYTLPPSREEMTKNQVSLTCLVKGFYPSDIAVEWESNGQPENNYKTTPPMLDSDGSFFLYSKLTVDKS
RWQQGNVFSCSVMHEALHNHYTQKSLSLSPGK (SEQ ID NO: 89

# FIGURE 17C

## IAB22M γ2 NH2 (N- to C-terminal)

Anti-CD8 variable light (VL) sequence
DVQITQSPSSLSASVGDRVTITCRTSRSISQYLAWYQQKPGKVPKLLIYSGSTLQSGVPSRFSGSGSGTDFTLTIS
SLQPEDVATYYCQQHNENPLTFGGGTKVEIK (SEQ ID NO: 82

Linker
GSTSGGGSGGGSGGGGSS (SEQ ID NO: 83

Anti-CD8 variable heavy (VH) sequence
EVQLVESGGGLVQPGGSLRLSCAASGFNIKDTYIHFVRQAPGKGLEWIGRIDPANDNTLYASKFQGKATISADTSK
NTAYLQMNSLRAEDTAVYYCGRGYGYYVFDHWGQGTLVTVSS (SEQ ID NO: 84

Full hinge (NH2)
ERKSCVECPPCPAPPVAGP (SEQ ID NO: 99

Upper hinge
ERK (SEQ ID NO: 86

Core hinge
SCVECPPCP (SEQ ID NO: 91

Lower hinge
APPVAGP (SEQ ID NO: 98

Human IgG2 CH3
GQPREPQVYTLPPSREEMTKNQVSLTCLVKGFYPSDIAVEWESNGQPENNYKTTPPMLDSDGSFFLYSKLTVDK
SRWQQGNVFSCSVMHEALHNHYTQKSLSLSPGK (SEQ ID NO: 89

# FIGURE 18

Full hinge N- to C-terminal (SEQ ID NO: 92

E P K S C D K T H T C P P C G G G S S G G G S G

Upper hinge SEQ ID NO: 93 | Core hinge SEQ ID NO: 94 | Lower hinge SEQ ID NO: 88

Full hinge N- to C-terminal (SEQ ID NO: 100

E P K S S D K T H T C P P C P P C G G G S S G G G S G

Upper hinge SEQ ID NO: 101 | Core hinge SEQ ID NO: 102 | Lower hinge SEQ ID NO: 88

Full hinge N- to C-terminal (SEQ ID NO: 120

E P K S S D K T H T C P P C V E C P P C G G G S S G G G S G

Upper hinge SEQ ID NO: 101 | Core hinge SEQ ID NO: 105 | Lower hinge SEQ ID NO: 88

# FIGURE 19A
## IAB22M γ1 EH3 (N- to C-terminal)

Anti-CD8 variable light (VL) sequence
DVQITQSPSSLSASVGDRVTITCRTSRSISQYLAWYQQKPGKVPKLLIYSGSTLQSGVPSRFSGSGSGTDFTLTISSLQPED
VATYYCQQHNENPLTFGGGTKVEIK (SEQ ID NO: 82

Linker
GSTSGGGSGGGSGGGGSS (SEQ ID NO: 83

Anti-CD8 variable heavy (VH) sequence
EVQLVESGGGLVQPGGSLRLSCAASGFNIKDTYIHFVRQAPGKGLEWIGRIDPANDNTLYASKFQGKATISADTSKNTAYLQ
MNSLRAEDTAVYYCGRGYGYYVFDHWGQGTLVTVSS (SEQ ID NO: 84

Full hinge (EH3)
EPKSSDKTHTCPPCPPCGGGSSGGGSG (SEQ ID NO: 100

Upper hinge
EPKSSDKTHT (SEQ ID NO: 101

Core hinge
CPPCPPC (SEQ ID NO: 102

Lower hinge
GGGSSGGGSG (SEQ ID NO: 88

Human IgG1 CH3 (G1m1 allotype)
GQPREPQVYTLPPSRDELTKNQVSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 95

Human IgG1 CH3 (nG1m1 allotype)
GQPREPQVYTLPPSREEMTKNQVSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 96

# FIGURE 19B

## IAB22M γ1 EH5 (N- to C-terminal)

Anti-CD8 variable light (VL) sequence
DVQITQSPSSLSASVGDRVTITCRTSRSISQYLAWYQQKPGKVPKLLIYSGSTLQSGVPSRFSGSGSGTDFTLTISSLQPEDV
ATYYCQQHNENPLTFGGGTKVEIK (SEQ ID NO: 82

Linker
GSTSGGGSGGGSGGGGSS (SEQ ID NO: 83

Anti-CD8 variable heavy (VH) sequence
EVQLVESGGGLVQPGGSLRLSCAASGFNIKDTYIHFVRQAPGKGLEWIGRIDPANDNTLYASKFQGKATISADTSKNTAYLQ
MNSLRAEDTAVYYCGRGYGYYVFDHWGQGTLVTVSS (SEQ ID NO: 84

Full hinge (EH5)
EPKSSDKTHTCPPCPPCPPCGGGSSGGGSG (SEQ ID NO: 103

Upper hinge
EPKSSDKTHT (SEQ ID NO: 101

Core hinge
CPPCPPCPPC (SEQ ID NO: 104

Lower hinge
GGGSSGGGSG (SEQ ID NO: 88

Human IgG1 CH3 (G1m1 allotype)
GQPREPQVYTLPPSRDELTKNQVSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 95

Human IgG1 CH3 (nG1m1 allotype)
GQPREPQVYTLPPSREEMTKNQVSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 96

# FIGURE 19C

Anti-CD8 variable light (VL) sequence
DVQITQSPSSLSASVGDRVTITCRTSRSISQYLAWYQQKPGKVPKLLIYSGSTLQSGVPSRFSGSGSGTDFTLTISSLQPE
DVATYYCQQHNENPLTFGGGTKVEIK (SEQ ID NO: 82

Linker
GSTSGGGSGGGSGGGGSS (SEQ ID NO: 83

Anti-CD8 variable heavy (VH) sequence
EVQLVESGGGLVQPGGSLRLSCAASGFNIKDTYIHFVRQAPGKGLEWIGRIDPANDNTLYASKFQGKATISADTSKNTAYL
QMNSLRAEDTAVYYCGRGYGYYVFDHWGQGTLVTVSS (SEQ ID NO: 84

Full hinge (EH6)
ELKTPLGDTTHTCVECPPCGGGSSGGGSG (SEQ ID NO: 106

Upper hinge
ELKTPLGDTTHT (SEQ ID NO: 107

Core hinge
CVECPPC (SEQ ID NO: 108

Lower hinge
GGGSSGGGSG (SEQ ID NO: 88

Human IgG1 CH3 (G1m1 allotype)
GQPREPQVYTLPPSRDELTKNQVSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 95

Human IgG1 CH3 (nG1m1 allotype)
GQPREPQVYTLPPSREEMTKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 96

# FIGURE 19D

## IAB22M $\gamma3/\gamma1$ EH7 (N- to C-terminal)

Anti-CD8 variable light (VL) sequence
DVQITQSPSSLSASVGDRVTITCRTSRSISQYLAWYQQKPGKVPKLLIYSGSTLQSGVPSRFSGSGSGTDFTLTISSLQPE
DVATYYCQQHNENPLTFGGGTKVEIK (SEQ ID NO: 82

Linker
GSTSGGGSGGGSGGGGSS (SEQ ID NO: 83

Anti-CD8 variable heavy (VH) sequence
EVQLVESGGGLVQPGGSLRLSCAASGFNIKDTYIHFVRQAPGKGLEWIGRIDPANDNTLYASKFQGKATISADTSKNTAY
LQMNSLRAEDTAVYYCGRGYGYYVFDHWGQGTLVTVSS (SEQ ID NO: 84

Full hinge (EH7)
ELKTPLGDTTHTCPPCPPCGGGSSGGGSG (SEQ ID NO: 109

Upper hinge
ELKTPLGDTTHT (SEQ ID NO: 107

Core hinge
CPPCPPC (SEQ ID NO: 102

Lower hinge
GGGSSGGGSG (SEQ ID NO: 88

Human IgG1 CH3 (G1m1 allotype)
GQPREPQVYTLPP<u>SRDEL</u>TKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 95

Human IgG1 CH3 (nG1m1 allotype)
GQPREPQVYTLPP<u>SREEM</u>TKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 96

# FIGURE 19E

## IAB22M γ3/γ1 EH8 (N- to C-terminal)

Anti-CD8 variable light (VL) sequence
DVQITQSPSSLSASVGDRVTITCRTSRSISQYLAWYQQKPGKVPKLLIYSGSTLQSGVPSRFSGSGSGTDFTLTISSLQPED
VATYYCQQHNENPLTFGGGTKVEIK (SEQ ID NO: 82

Linker
GSTSGGGSGGGSGGGGSS (SEQ ID NO: 83

Anti-CD8 variable heavy (VH) sequence
EVQLVESGGGLVQPGGSLRLSCAASGFNIKDTYIHFVRQAPGKGLEWIGRIDPANDNTLYASKFQGKATISADTSKNTAYLQ
MNSLRAEDTAVYYCGRGYGYYVFDHWGQGTLVTVSS (SEQ ID NO: 84

Full hinge (EH8)
ELKTPLGDTTHTCPPCPPCPPCGGGSSGGGSG (SEQ ID NO: 110

Upper hinge
ELKTPLGDTTHT (SEQ ID NO: 107

Core hinge
CPPCPPCPPC (SEQ ID NO: 104

Lower hinge
GGGSSGGGSG (SEQ ID NO: 88

Human IgG1 CH3 (G1m1 allotype)
GQPREPQVYTLPPSRDELTKNQVSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 95

Human IgG1 CH3 (nG1m1 allotype)
GQPREPQVYTLPPSREEMTKNQVSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 96

# FIGURE 20

## IAB22M γ1 EH2 (N- to C-terminal)

Anti-CD8 variable light (VL) sequence
DVQITQSPSSLSASVGDRVTITCRTSRSISQYLAWYQQKPGKVPKLLIYSGSTLQSGVPSRFSGSGSGTDFTLTI
SSLQPEDVATYYCQQHNENPLTFGGGTKVEIK (SEQ ID NO: 82

Linker
GSTSGGGSGGGSGGGGSS (SEQ ID NO: 83

Anti-CD8 variable heavy (VH) sequence
EVQLVESGGGLVQPGGSLRLSCAASGFNIKDTYIHFVRQAPGKGLEWIGRIDPANDNTLYASKFQGKATISADT
SKNTAYLQMNSLRAEDTAVYYCGRGYGYYVFDHWGQGTLVTVSS (SEQ ID NO: 84

Full length (EH2) EPKSSDKTHTCPPCGGGSSGGGSG (SEQ ID NO: 111

Upper hinge
EPKSSDKTHT (SEQ ID NO: 101

Core hinge
CPPC (SEQ ID NO: 94

Lower hinge
GGGSSGGGSG (SEQ ID NO: 88

Human IgG1 CH3 (G1m1 allotype)
GQPREPQVYTLPPSRDELTKNQVSLTCLVKGFYPSDIAVEWESNGQPENNYKTTPPVLDSDGSFFLYSKLTVD
KSRWQQGNVFSCSVMHEALHNHYTQKSLSLSPGK (SEQ ID NO: 95

Human IgG1 CH3 (nG1m1 allotype)
GQPREPQVYTLPPSREEMTKNQVSLTCLVKGFYPSDIAVEWESNGQPENNYKTTPPVLDSDGSFFLYSKLTVD
KSRWQQGNVFSCSVMHEALHNHYTQKSLSLSPGK (SEQ ID NO: 96

# FIGURE 21

IAB22M variable light (VL) and heavy (VH) sequences

VL DVQITQSPSSLSASVGDRVTITCRTSRSISQYLAWYQQKPGKVPKLLIYSGSTL
QSGVPSRFSGSGSGTDFTLTISSLQPEDVATYYCQQHNENPLTFGGGTKVEIK **(SEQ ID NO: 82**

VH EVQLVESGGGLVQPGGSLRLSCAASGFNIKDTYIHFVRQAPGKGLEWIGRIDPA
NDNTLYASKFQGKATISADTSKNTAYLQMNSLRAEDTAVYYCGRGYGYYVFDHW GQGTLVTVSS **(SEQ ID NO: 84**

# FIGURE 22

Linker sequences:

| | | |
|---|---|---|
| GGGGS | 5 aa | (SEQ ID NO: 115 |
| GGGGSGGGGS | 10 aa | (SEQ ID NO: 114 |
| GGGGSGGGGSGGGGS | 15 aa | (SEQ ID NO: 113 |
| GSTSGGGSGGGS | 12 aa | (SEQ ID NO: 112 |
| GSTSGGGSGGGSGGGGSS | 18 aa | (SEQ ID NO: 83 |

# FIGURE 23

**Full hinge:**

| | **Upper** | **Core** | **Lower** |
|---|---|---|---|
| Human IgG1 NH1 (SEQ ID NO: 116 | EPKSCDKTHT (SEQ ID NO: 93 | CPPCP (SEQ ID NO: 122 | APELLGGP (SEQ ID NO: 123 |
| Human IgG1 EH1 (SEQ ID NO: 92 | EPKSCDKTHT (SEQ ID NO: 93 | CPPC (SEQ ID NO: 94 | GGGSSGGGSG (SEQ ID NO: 88 |
| Human IgG1 NH2 (SEQ ID NO: 117 | EPKSSDKTHT (SEQ ID NO: 101 | CPPCP (SEQ ID NO: 122 | APELLGGP (SEQ ID NO: 123 |
| Human IgG1 EH2 (SEQ ID NO: 111 | EPKSSDKTHT (SEQ ID NO: 101 | CPPC (SEQ ID NO: 94 | GGGSSGGGSG (SEQ ID NO: 88 |
| Human IgG1 NH3 (SEQ ID NO: 118 | EPKSSDKTHT (SEQ ID NO: 101 | CPPCPPC (SEQ ID NO: 102 | APELLGGP (SEQ ID NO: 123 |
| Human IgG1 EH3 (SEQ ID NO: 100 | EPKSSDKTHT (SEQ ID NO: 101 | CPPCPPC (SEQ ID NO: 102 | GGGSSGGGSG (SEQ ID NO: 88 |
| Human IgG1 NH4 (SEQ ID NO: 119 | EPKSSDKTHT (SEQ ID NO: 101 | CPPCVECPPC (SEQ ID NO: 105 | APELLGGP (SEQ ID NO: 123 |
| Human IgG1 EH4 (SEQ ID NO: 120 | EPKSSDKTHT (SEQ ID NO: 101 | CPPCVECPPC (SEQ ID NO: 105 | GGGSSGGGSG (SEQ ID NO: 88 |
| Human IgG1 NH5 (SEQ ID NO: 121 | EPKSSDKTHT (SEQ ID NO: 101 | CPPCPPCPPC (SEQ ID NO: 104 | APELLGGP (SEQ ID NO: 123 |
| Human IgG1 EH5 (SEQ ID NO: 103 | EPKSSDKTHT (SEQ ID NO: 101 | CPPCPPCPPC (SEQ ID NO: 104 | GGGSSGGGSG (SEQ ID NO: 88 |

# FIGURE 23 Continued

**Full hinge:**

| | Upper | Core | Lower |
|---|---|---|---|
| Human IgG2 NH1 (SEQ ID NO: 97 | ERK (SEQ ID NO: 86 | CCVECPPCP (SEQ ID NO: 87 | APPVAGP (SEQ ID NO: 98 |
| Human IgG2 EH1 (SEQ ID NO: 85 | ERK (SEQ ID NO: 86 | CCVECPPCP (SEQ ID NO: 87 | GGGSSGGGSG (SEQ ID NO: 88 |
| Human IgG2 NH2 (SEQ ID NO: 99 | ERK (SEQ ID NO: 86 | SCVECPPCP (SEQ ID NO: 91 | APPVAGP (SEQ ID NO: 98 |
| Human IgG2 EH2 (SEQ ID NO: 90 | ERK (SEQ ID NO: 86 | SCVECPPCP (SEQ ID NO: 91 | GGGSSGGGSG (SEQ ID NO: 88 |
| IgG3/IgG1 EH6 (SEQ ID NO: 106 | ELKTPLGDTTHT (SEQ ID NO: 107 | CVECPPC (SEQ ID NO: 108 | GGGSSGGGSG (SEQ ID NO: 88 |
| IgG3/IgG1 EH7 (SEQ ID NO: 109 | ELKTPLGDTTHT (SEQ ID NO: 107 | CPPCPPC (SEQ ID NO: 102 | GGGSSGGGSG (SEQ ID NO: 88 |
| IgG3/IgG1 EH8 (SEQ ID NO: 110 | ELKTPLGDTTHT (SEQ ID NO: 107 | CPPCPPCPPC (SEQ ID NO: 104 | GGGSSGGGSG (SEQ ID NO: 88 |
| IgG4 NH (SEQ ID NO: 124 | ESKYGPP (SEQ ID NO: 126 | CPPCP (SEQ ID NO: 122 | APEFLGGP (SEQ ID NO: 127 |
| IgG4 EH (SEQ ID NO: 125 | ESKYGPP (SEQ ID NO: 126 | CPPCP (SEQ ID NO: 122 | GGGSSGGGSG (SEQ ID NO: 88 |

# FIGURE 24

CH3 domains:

Human IgG1 CH3 (G1m1 allotype)
GQPREPQVYTLPPSRDELTKNQVSLTCLVKGFYPSDIAVEWESNGQPENNYKTTPPVLDSDGSFFLYSKLTVD
KSRWQQGNVFSCSVMHEALHNHYTQKSLSLSPGK (SEQ ID NO: 95

Human IgG1 CH3 (nG1m1 allotype)
GQPREPQVYTLPPSREEMTKNQVSLTCLVKGFYPSDIAVEWESNGQPENNYKTTPPVLDSDGSFFLYSKLTVD
KSRWQQGNVFSCSVMHEALHNHYTQKSLSLSPGK (SEQ ID NO: 96

Human IgG2 CH3 GQPREPQVYTLPPSREEMTKNQVSLTCLVKGFYPSDIAVEWESNGQPENNYKTTPPMLDSDGSFFLYSKLTVD
KSRWQQGNVFSCSVMHEALHNHYTQKSLSLSPGK (SEQ ID NO: 89

Human IgG3 CH3 GQPREPQVYTLPPSREEMTKNQVSLTCLVKGFYPSDIAVEWESSGQPENNYNTTPPMLDSDGSFFLYSKLTVD
KSRWQQGNIFSCSVMHEALHNRFTQKSLSLSPGK (SEQ ID NO: 128

Human IgG4 CH3 GQPREPQVYTLPPSQEEMTKNQVSLTCLVKGFYPSDIAVEWESNGQPENNYKTTPPVLDSDGSFFLYSRLTVD
KSRWQEGNVFSCSVMHEALHNHYTQKSLSLSLGK (SEQ ID NO: 129

# FIGURE 25

Alignment compared IAB22M γ1 EH1(M1) (SEQ ID NO: 130 vs IAB22M γ1 EH3(M1) (SEQ ID NO: 131 , differences shown in box.

```
IAB22M γ1 EH1(M1) METDTLLLWVLLLWVPGSTGDVQITQSPSSLSASVGDRVTITCRTSRSISQYLAWYQQKP 60
IAB22M γ1 EH3(M1) METDTLLLWVLLLWVPGSTGDVQITQSPSSLSASVGDRVTITCRTSRSISQYLAWYQQKP 60
IAB22M γ1 EH1(M1) GKVPKLLIYSGSTLQSGVPSRFSGSGSGTDFTLTISSLQPEDVATYYCQQHNENPLTFGG 120
IAB22M γ1 EH3(M1) GKVPKLLIYSGSTLQSGVPSRFSGSGSGTDFTLTISSLQPEDVATYYCQQHNENPLTFGG 120
IAB22M γ1 EH1(M1) GTKVEIKGSTSGGGSGGGSGGGGSSEVQLVESGGGLVQPGGSLRLSCAASGFNIKDTYIH 180
IAB22M γ1 EH3(M1) GTKVEIKGSTSGGGSGGGSGGGGSSEVQLVESGGGLVQPGGSLRLSCAASGFNIKDTYIH 180
IAB22M γ1 EH1(M1) FVRQAPGKGLEWIGRIDPANDNTLYASKFQGKATISADTSKNTAYLQMNSLRAEDTAVYY 240
IAB22M γ1 EH3(M1) FVRQAPGKGLEWIGRIDPANDNTLYASKFQGKATISADTSKNTAYLQMNSLRAEDTAVYY 240
IAB22M γ1 EH1(M1) CGRGYGYYVFDHWGQGTLVTVSSEPKS [C] DKTHTCP [---] PCGGGSSGGGSGGQPREPQVYT 297
IAB22M γ1 EH3(M1) CGRGYGYYVFDHWGQGTLVTVSSEPKS [S] DKTHTCP [PCP] PCGGGSSGGGSGGQPREPQVYT 300
IAB22M γ1 EH1(M1) LPPSR [DEL] TKNQVSLTCLVKGFYPSDIAVEWESNGQPENNYKTTPPVLDSDGSFFLYSKL 357
IAB22M γ1 EH3(M1) LPPSR [EEM] TKNQVSLTCLVKGFYPSDIAVEWESNGQPENNYKTTPPVLDSDGSFFLYSKL 360
IAB22M γ1 EH1(M1) TVDKSRWQQGNVFSCSVMHEALHNHYTQKSLSLSPG [K] 394 (SEQ ID NO: 130
IAB22M γ1 EH3(M1) TVDKSRWQQGNVFSCSVMHEALHNHYTQKSLSLSPG [-] 396 (SEQ ID NO: 131
```

# FIGURE 26

**IAB22M γ1 EH3(M1), CDR regions are defined by Chothia definition (SEQ ID NO: 131**

atggagaccgataccctgctgctgtgggtgctgctgctgtgggtgcccggctccacaggc

M E T D T L L L W V L L L W V P G S T G

**Signal peptide sequence**

gatgtgcagatcacacagagccctagcagcctgagcgccagcgtgggagatagagtcacc

D V Q I T Q S P S S L S A S V G D R V T

**VL**

Atcacatgc aggaccagcagaagcattagccagtacctggcc tggtaccagcaaaagccc

I T C R T S R S I S Q Y L A W Y Q Q K P

**LCDR1**

Ggcaaggtgcccaagctgctgatctac agcggctccaccctgcagagc ggcgtgcccagc

G K V P K L L I Y S G S T L Q S G V P S

**LCDR2**

agattctccggctccggaagcggcacagactttaccctgaccatctcctccctgcagccc

R F S G S G S G T D F T L T I S S L Q P

gaggatgtcgccacctactactgc cagcagcacaacgaaaacccccctgaca tttggcggc

E D V A T Y Y C Q Q H N E N P L T F G G

**LCDR3**

ggcaccaaggtggagatcaag ggcagcaccagcggtggaggaagtggaggtggaagtgga

G T K V E I K G S T S G G G S G G G S G

# FIGURE 26 CONTINUED

```
ggcaccaaggtggagatcaag ggcagcaccagcggtggaggaagtggaggtggaagtgga
 G  T  K  V  E  I  K   G  S  T  S  G  G  G  S  G  G  G  S  G
                                   Linker

Ggaggcggaagcagc gaggtgcagctggtggagagtggtggaggactggtgcagcccgga
 G  G  G  S  S   E  V  Q  L  V  E  S  G  G  G  L  V  Q  P  G
Linker           VH

Ggcagcctgagactgagctgtgctgcctcc ggattcaatatcaaggacacc tacatccac
 G  S  L  R  L  S  C  A  A  S   G  F  N  I  K  D  T   Y  I  H
                                      HCDR1

ttcgtgagacaggcccccggcaagggactggagtggattggaaggatc gaccccgccaac
 F  V  R  Q  A  P  G  K  G  L  E  W  I  G  R  I   D  P  A  N
                                                      HCDR2

gacaac accctgtacgccagcaaattccagggcaaggccacaatcagcgccgacaccagc
 D  N   T  L  Y  A  S  K  F  Q  G  K  A  T  I  S  A  D  T  S
HCDR2

aagaacaccgcctatctgcagatgaactccctgagagccgaggacacagccgtgtactac
 K  N  T  A  Y  L  Q  M  N  S  L  R  A  E  D  T  A  V  Y
```

# FIGURE 26 CONTINUED

```
tgcggcagg ggctacggctattacgtgttcgaccac tggggccagggcaccctggtgaca
 C  G  R   G  Y  G  Y  Y  V  F  D  H   W  G  Q  G  T  L  V  T
                      HCDR3

gtgagcagc gaacccaagagctccgacaagacccacacc tgtccccttgccctccttgt
 V  S  S   E  P  K  S  S  D  K  T  H  T   C  P  P  C  P  P  C
                  Upper hinge                   Core hinge

ggcggaggaagctccggaggcggaagcggag gacagcctagggagccccaggtgtatacc
 G  G  G  S  S  G  G  G  S  G   G  Q  P  R  E  P  Q  V  Y  T
        Lower hinge             CH3

ctcccccctccagggaagagatgaccaagaaccaggtgagcctgacctgcctcgtgaag
 L  P  P  S  R  E  E  M  T  K  N  Q  V  S  L  T  C  L  V  K

ggcttttatccctccgatatcgccgtggagtgggagagcaacggccagcctgagaacaat
 G  F  Y  P  S  D  I  A  V  E  W  E  S  N  G  Q  P  E  N  N

tacaagaccaccccccctgtgctggactccgatggcagcttcttcctgtattccaagctg
 Y  K  T  T  P  P  V  L  D  S  D  G  S  F  F  L  Y  S  K  L

accgtcgacaagtccaggtggcaacagggcaacgtcttcagctgcagcgtgatgcacgag
 T  V  D  K  S  R  W  Q  Q  G  N  V  F  S  C  S  V  M  H  E

gccctgcacaatcactacacccagaagtccctctccctgagccccggctga (SEQ ID NO: 132
 A  L  H  N  H  Y  T  Q  K  S  L  S  L  S  P  G  Stop (SEQ ID NO: 131
```

# FIGURE 27

**IAB22M γ1 EH5(M1) (SEQ ID NO: 133**

atggagacagacaccctcctcctgtgggtgctgctgctgtgggtgcccggatccaccgga

M E T D T L L L W V L L L W V P G S T G

gacgtgcagatcacacagagccccagctccctgtccgctagcgtgggcgacagagtgacc

D V Q I T Q S P S S L S A S V G D R V T

atcacctgcaggaccagcaggagcatctcccagtacctcgcttggtaccagcagaagcct

I T C R T S R S I S Q Y L A W Y Q Q K P

ggcaaggtgcccaagctgctgatttacagcggatccaccctgcagagcggcgtgcctagc

G K V P K L L I Y S G S T L Q S G V P S

aggtttagcggcagcggatccggaacagacttcaccctgaccatcagcagcctgcagcct

R F S G S G S G T D F T L T I S S L Q P

gaagatgtggccacctactactgtcagcagcacaacgaaaacccctcaccttcggcggc

E D V A T Y Y C Q Q H N E N P L T F G G

ggcacaaaggtggaaatcaagggcagcacctccggaggaggcagcggcggaggcagcgga

G T K V E I K G S T S G G G S G G G S G

ggcggcggctccagcgaagtgcagctggtcgagagcggaggcggactggtgcaacccgga

G G G S S E V Q L V E S G G G L V Q P G

ggaagcctgaggctgagctgtgccgccagcggcttcaacatcaaggacacatacattcac

G S L R L S C A A S G F N I K D T Y I H

tttgtgaggcaggctcctggaaagggcctggagtggatcggcagaatcgaccccgctaac

F V R Q A P G K G L E W I G R I D P A N

gacaacaccctgtacgccagcaagttccagggcaaggccaccatctccgccgacacaagc

# FIGURE 27 CONTINUED

D N T L Y A S K F Q G K A T I S A D T S

aagaataccgcctacctgcagatgaactccctgagggccgaggataccgccgtgtactac

K N T A Y L Q M N S L R A E D T A V Y Y

tgcggcaggggctatggctactacgtgtttgaccactggggccagggcacactggtgaca

C G R G Y G Y Y V F D H W G Q G T L V T

gtgagctccgagcccaagagctccgacaagacacacacctgccctccttgcccccccttgt

V S S E P K S S D K T H T C P P C P P C

cctccctgtggaggaggaagcagcggaggaggaagcggcggacagcccagagagcctcaa

P P C G G G S S G G G S G G Q P R E P Q

gtgtataccctgccccccctccagggaagagatgaccaagaaccaggtgagcctgacatgc

V Y T L P P S R E E M T K N Q V S L T C

ctggtcaaaggcttctaccccagcgatattgctgtggagtgggagagcaacggccagccc

L V K G F Y P S D I A V E W E S N G Q P

gagaacaactacaagaccacaccccccgtcctggatagcgatggcagcttcttcctgtac

E N N Y K T T P P V L D S D G S F F L Y

agcaagctgaccgtggacaagtccaggtggcagcagggcaacgtcttctcctgcagcgtg

S K L T V D K S R W Q Q G N V F S C S V

atgcacgaggctctgcataaccactacacacagaagtccctcagcctgagccctggatga (SEQ ID NO: 136

M H E A L H N H Y T Q K S L S L S P G Stop (SEQ ID NO: 133

# FIGURE 28

**IAB22M γ1 EH7(M1) (SEQ ID NO: 137**

```
atggagacagacaccctcctcctgtgggtgctgctgctgtgggtgcccggatccaccgga
 M  E  T  D  T  L  L  L  W  V  L  L  L  W  V  P  G  S  T  G
gacgtgcagatcacacagagccccagctccctgtccgctagcgtgggcgacagagtgacc
 D  V  Q  I  T  Q  S  P  S  S  L  S  A  S  V  G  D  R  V  T
atcacctgcaggaccagcaggagcatctcccagtacctcgcttggtaccagcagaagcct
 I  T  C  R  T  S  R  S  I  S  Q  Y  L  A  W  Y  Q  Q  K  P
ggcaaggtgcccaagctgctgatttacagcggatccaccctgcagagcggcgtgcctagc
 G  K  V  P  K  L  L  I  Y  S  G  S  T  L  Q  S  G  V  P  S
aggtttagcggcagcggatccggaacagacttcaccctgaccatcagcagcctgcagcct
 R  F  S  G  S  G  S  G  T  D  F  T  L  T  I  S  S  L  Q  P
gaagatgtggccacctactactgtcagcagcacaacgaaaacccctcaccttcggcggc
 E  D  V  A  T  Y  Y  C  Q  Q  H  N  E  N  P  L  T  F  G  G
ggcacaaaggtggaaatcaagggcagcacctccggaggaggcagcggcggaggcagcgga
 G  T  K  V  E  I  K  G  S  T  S  G  G  G  S  G  G  G  S  G
ggcggcggctccagcgaagtgcagctggtcgagagcggaggcggactggtgcaacccgga
 G  G  G  S  S  E  V  Q  L  V  E  S  G  G  G  L  V  Q  P  G
ggaagcctgaggctgagctgtgccgccagcggcttcaacatcaaggacacatacattcac
 G  S  L  R  L  S  C  A  A  S  G  F  N  I  K  D  T  Y  I  H
tttgtgaggcaggctcctggaaagggcctggagtggatcggcagaatcgaccccgctaac
 F  V  R  Q  A  P  G  K  G  L  E  W  I  G  R  I  D  P  A  N
gacaacaccctgtacgccagcaagttccagggcaaggccaccatctccgccgacacaagc
```

# FIGURE 28 CONTINUED

D N T L Y A S K F Q G K A T I S A D T S

aagaataccgcctacctgcagatgaactccctgagggccgaggataccgccgtgtactac

K N T A Y L Q M N S L R A E D T A V Y Y

tgcggcaggggctatggctactacgtgtttgaccactggggccagggcacactggtgaca

C G R G Y G Y Y V F D H W G Q G T L V T

gtgagctccgagctgaagacacctctgggcgacacaacacacacctgccccccttgtcct

V S S E L K T P L G D T T H T C P P C P

ccctgtggaggaggaagcagcggaggaggaagcggcggacagcccagagagcctcaagtg

P C G G G S S G G G S G G Q P R E P Q V

tataccctgccccccctccagggaagagatgaccaagaaccaggtgagcctgacatgcctg

Y T L P P S R E E M T K N Q V S L T C L

gtcaaaggcttctaccccagcgatattgctgtggagtgggagagcaacggccagcccgag

V K G F Y P S D I A V E W E S N G Q P E

Aacaactacaagaccacacccccgtcctggatagcgatggcagcttcttcctgtacagc

N N Y K T T P P V L D S D G S F F L Y S

aagctgaccgtggacaagtccaggtggcagcagggcaacgtcttctcctgcagcgtgatg

K L T V D K S R W Q Q G N V F S C S V M

cacgaggctctgcataaccactacacacagaagtccctcagcctgagccctggatga **(SEQ ID NO: 138)**

H E A L H N H Y T Q K S L S L S P G Stop **(SEQ ID NO: 137)**

# FIGURE 29

IAB22M γ1 EH8(M1) (SEQ ID NO: 139

atggagacagacaccctcctcctgtgggtgctgctgctgtgggtgcccggatccaccgga

M E T D T L L L W V L L L W V P G S T G

gacgtgcagatcacacagagccccagctccctgtccgctagcgtgggcgacagagtgacc

D V Q I T Q S P S S L S A S V G D R V T

atcacctgcaggaccagcaggagcatctcccagtacctcgcttggtaccagcagaagcct

I T C R T S R S I S Q Y L A W Y Q Q K P

ggcaaggtgcccaagctgctgatttacagcggatccaccctgcagagcggcgtgcctagc

G K V P K L L I Y S G S T L Q S G V P S

aggtttagcggcagcggatccggaacagacttcaccctgaccatcagcagcctgcagcct

R F S G S G S G T D F T L T I S S L Q P

gaagatgtggccacctactactgtcagcagcacaacgaaaacccctcaccttcggcggc

E D V A T Y Y C Q Q H N E N P L T F G G

ggcacaaaggtggaaatcaagggcagcacctccggaggaggcagcggcggaggcagcgga

G T K V E I K G S T S G G G S G G G S G

ggcggcggctccagcgaagtgcagctggtcgagagcggaggcggactggtgcaacccgga

G G G S S E V Q L V E S G G G L V Q P G

ggaagcctgaggctgagctgtgccgccagcggcttcaacatcaaggacacatacattcac

G S L R L S C A A S G F N I K D T Y I H

tttgtgaggcaggctcctggaaagggcctggagtggatcggcagaatcgaccccgctaac

F V R Q A P G K G L E W I G R I D P A N

gacaacaccctgtacgccagcaagttccagggcaaggccaccatctccgccgacacaagc

# FIGURE 29 CONTINUED

D N T L Y A S K F Q G K A T I S A D T S

aagaataccgcctacctgcagatgaactccctgagggccgaggataccgccgtgtactac

K N T A Y L Q M N S L R A E D T A V Y Y

tgcggcaggggctatggctactacgtgtttgaccactggggccagggcacactggtgaca

C G R G Y G Y Y V F D H W G Q G T L V T

gtgagctccgagctgaagacacctctgggcgacacaacacacacctgccctccttgcccc

V S S E L K T P L G D T T H T C P P C P

ccttgtcctccctgtggaggaggaagcagcggaggaggaagcggcggacagcccagagag

P C P P C G G G S S G G G S G G Q P R E

cctcaagtgtataccctgccccctccagggaagagatgaccaagaaccaggtgagcctg

P Q V Y T L P P S R E E M T K N Q V S L

acatgcctggtcaaaggcttctaccccagcgatattgctgtggagtgggagagcaacggc

T C L V K G F Y P S D I A V E W E S N G

cagcccgagaacaactacaagaccacacccccgtcctggatagcgatggcagcttcttc

Q P E N N Y K T T P P V L D S D G S F F

ctgtacagcaagctgaccgtggacaagtccaggtggcagcagggcaacgtcttctcctgc

L Y S K L T V D K S R W Q Q G N V F S C

agcgtgatgcacgaggctctgcataaccactacacacagaagtccctcagcctgagccct

S V M H E A L H N H Y T Q K S L S L S P

ggatga (SEQ ID NO: 140

G Stop (SEQ ID NO: 139

# FIGURE 30

**IAB22M γ2 EH2(M1) (SEQ ID NO: 141**

atggaaaccgacacactgctgctgtgggtgctgctgctgtgggtccctggctccaccgga

M E T D T L L L W V L L L W V P G S T G

gacgtccagatcacacagagccccagcagcctgtccgccagcgtgggagacagggtgacc

D V Q I T Q S P S S L S A S V G D R V T

atcacctgcaggaccagcagatccatctcccagtatctggcctggtaccaacagaagccc

I T C R T S R S I S Q Y L A W Y Q Q K P

ggcaaggtccccaaactgctgatctacagcggctccaccctgcagtccggcgtgcctagc

G K V P K L L I Y S G S T L Q S G V P S

aggttctccggcagcggatccggcaccgacttcaccctgaccatcagctccctgcagcct

R F S G S G S G T D F T L T I S S L Q P

gaggacgtggctacctactactgccaacagcacaacgagaacccccctgacctttggaggc

E D V A T Y Y C Q Q H N E N P L T F G G

ggcaccaaggtggaaatcaagggcagcaccagcggcggaggaagcggaggaggatccgga

G T K V E I K G S T S G G G S G G G S G

ggaggcggaagctccgaggtgcagctggtggaaagcggcggcggactggtgcagcctgga

G G G S S E V Q L V E S G G G L V Q P G

ggaagcctcagactgagctgtgccgccagcggattcaacatcaaagacacctacattcat

G S L R L S C A A S G F N I K D T Y I H

ttcgtgagacaggcccccggcaagggcctcgaatggatcggaaggatcgaccccgctaac

F V R Q A P G K G L E W I G R I D P A N

gacaataccctgtacgcctccaagttccagggaaaggccaccatctccgccgatacctcc

# FIGURE 30 CONTINUED

D N T L Y A S K F Q G K A T I S A D T S

aagaacaccgcctacctccagatgaactccctgagggccgaagataccgccgtctactac

K N T A Y L Q M N S L R A E D T A V Y Y

tgtggcaggggctacggctactatgtgttcgatcactggggccaaggaaccctggtgacc

C G R G Y G Y Y V F D H W G Q G T L V T

gtgagcagcgaaaggaagagctgcgtggagtgtcctccttgtcccggcggcggctccagc

V S S E R K S C V E C P P C P G G G S S

ggcggaggctccggcggccagcctagagaacctcaggtgtacaccctccccccctccaga

G G G S G G Q P R E P Q V Y T L P P S R

gaggagatgaccaagaaccaggtgtccctgacctgcctggtgaaaggcttctatcccagc

E E M T K N Q V S L T C L V K G F Y P S

gacatcgccgtggaatgggagtccaacggccagcccgagaacaactacaagaccacccct

D I A V E W E S N G Q P E N N Y K T T P

cccatgctggattccgacggcagcttttttcctgtacagcaagctcaccgtggacaagagc

P M L D S D G S F F L Y S K L T V D K S

agatggcagcagggcaacgtgttcagctgcagcgtgatgcacgaggctctgcataaccac

R W Q Q G N V F S C S V M H E A L H N H

tacacccagaagagcctgtccctgtcccccggatga (SEQ ID NO: 142

Y T Q K S L S L S P G Stop (SEQ ID NO: 141

# FIGURE 31

**IAB22M γ2 EH2(M1)(VH-K67R) (SEQ ID NO: 143**

```
atggagaccgacaccctcctcctgtgggtgctgctgctgtgggtgcctggaagcaccggc
 M  E  T  D  T  L  L  L  W  V  L  L  L  W  V  P  G  S  T  G
gatgtgcagatcacccagagccctagcagcctgtccgcttccgtgggcgacagggtgacc
 D  V  Q  I  T  Q  S  P  S  S  L  S  A  S  V  G  D  R  V  T
atcacctgtaggacctccaggagcatctcccagtacctggcctggtaccagcagaagccc
 I  T  C  R  T  S  R  S  I  S  Q  Y  L  A  W  Y  Q  Q  K  P
ggcaaggtgcccaaactgctcatctactccggcagcacactccagagcggcgtccctagc
 G  K  V  P  K  L  L  I  Y  S  G  S  T  L  Q  S  G  V  P  S
agattcagcggaagcggcagcggcaccgacttcaccctgaccatcagctccctgcagccc
 R  F  S  G  S  G  S  G  T  D  F  T  L  T  I  S  S  L  Q  P
gaggacgtggccacctattactgtcagcagcacaacgaaaacccccctgaccttcggcggc
 E  D  V  A  T  Y  Y  C  Q  Q  H  N  E  N  P  L  T  F  G  G
ggcacaaaagtggagatcaagggcagcaccagcggaggcggatccggcggcggcagcggc
 G  T  K  V  E  I  K  G  S  T  S  G  G  G  S  G  G  G  S  G
ggcggaggatccagcgaagtgcagctggtcgaaagcggaggcggactggtgcagcctgga
 G  G  G  S  S  E  V  Q  L  V  E  S  G  G  G  L  V  Q  P  G
ggaagcctgagactcagctgcgccgcctccggattcaacatcaaggacacctacatccac
 G  S  L  R  L  S  C  A  A  S  G  F  N  I  K  D  T  Y  I  H
ttcgtgaggcaggctcccggcaaaggcctcgagtggattggaaggattgaccccgccaac
 F  V  R  Q  A  P  G  K  G  L  E  W  I  G  R  I  D  P  A  N
gacaacaccctgtacgccagcaagttccaaggaagggccaccatctccgccgacaccagc
```

# FIGURE 31 CONTINUED

D N T L Y A S K F Q G R A T I S A D T S

aagaataccgcctacctgcagatgaactccctgagggctgaggacaccgccgtgtactac

K N T A Y L Q M N S L R A E D T A V Y Y

tgcggcagaggctacggctactacgtgttcgaccactggggacagggcacactggtgaca

C G R G Y G Y Y V F D H W G Q G T L V T

gtgagcagcgagaggaaaagctgcgtggagtgccccccctgccctggcggcggcagctcc

V S S E R K S C V E C P P C P G G G S S

ggcggaggaagcggaggacaacccagggagccccaggtgtacacactccccccctagcagg

G G G S G G Q P R E P Q V Y T L P P S R

gaagagatgaccaagaaccaggtgtccctgacctgcctcgtgaagggattctaccccagc

E E M T K N Q V S L T C L V K G F Y P S

gacattgccgtcgagtgggagagcaacggccagcctgagaacaactacaagacaaccccc

D I A V E W E S N G Q P E N N Y K T T P

Cctatgctcgatagcgatggctccttcttcctgtactccaagctcaccgtcgacaagagc

P M L D S D G S F F L Y S K L T V D K S

aggtggcagcagggcaacgtcttctcctgtagcgtgatgcacgaggctctgcacaaccac

R W Q Q G N V F S C S V M H E A L H N H

tacacccagaagagcctgagcctgagccccggctga (SEQ ID NO: 144

Y T Q K S L S L S P G Stop (SEQ ID NO: 143

# FIGURE 32

IAB22M variable heavy (VH-K67R) sequence
VH
EVQLVESGGGLVQPGGSLRLSCAASGFNIKDTYIHFVRQAPGKGLEWIGRIDP
ANDNTLYASKFQGRATISADTSKNTAYLQMNSLRAEDTAVYYCGRGYGYYVFD
HWGQGTLVTVSS **(SEQ ID NO: 145)**

# FIGURE 33

**T-cell surface glycoprotein CD8 alpha chain, Homo sapiens**

**UniProtKB/SwissProt: P01732 (SEQ ID NO: 134)**

MALPVTALLLPLALLLHAARPSQFRVSPLDRTWNLGETVELKCQVLLSNPTSGCSWLFQPRGAAASPTFL

LYLSQNKPKAAEGLDTQRFSGKRLGDTFVLTLSDFRRENEGYYFCSALSNSIMYFSHFVPVFLPAKPTTT

PAPRPPTPAPTIASQPLSLRPEACRPAAGGAVHTRGLDFACDIYIWAPLAGTCGVLLLSLVITLYCNHRN

RRRVCKCPRPVVKSGDKPSLSARYV **(SEQ ID NO: 134)**

# FIGURE 34

**T-cell surface glycoprotein CD8 beta chain, Homo sapiens**

**UniProtKB/SwissProt: P10966 (SEQ ID NO: 135)**

MRPRLWLLLAAQLTVLHGNSVLQQTPAYIKVQTNKMVMLSCEAKISLSNMRIYWLRQRQAPSSDSHHEFLAL

WDSAKGTIHGEEVEQEKIAVFRDASRFILNLTSVKPEDSGIYFCMIVGSPELTFGKGTQLSVVDFLPTTAQP

TKKSTLKKRVCRLPRPETQKGPLCSPVTLGLLVAGVLVLLVSLGVAMHLCCRRRRARLRFMKQFYK **(SEQ ID NO: 135)**

# FIGURE 35

M1 orientation

| | | | | | |
|---|---|---|---|---|---|
| S | $V_L$ | L | $V_H$ | H | $C_H3$ |

M2 orientation

| | | | | | |
|---|---|---|---|---|---|
| S | $V_H$ | L | $V_L$ | H | $C_H3$ |

S = signal peptide
$V_H$ = heavy chain of variable domain
L = linker
$V_L$ = light chain of variable domain
H = hinge
$C_H3$ = $C_H3$ domain

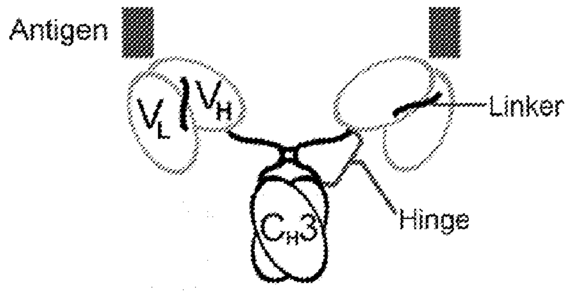

# FIGURE 36

Full hinge N- to C-terminal (SEQ ID NO: 92

E P K S C D K T H T C P P C G G G S S G G G S G

Upper hinge SEQ ID NO: 93 | Core hinge SEQ ID NO: 94 | Lower hinge SEQ ID NO: 88

Full hinge N- to C-terminal (SEQ ID NO: 111

E P K S S D K T H T C P P C G G G S S G G G S G

Upper hinge SEQ ID NO: 101 | Core hinge SEQ ID NO: 94 | Lower hinge SEQ ID NO: 88

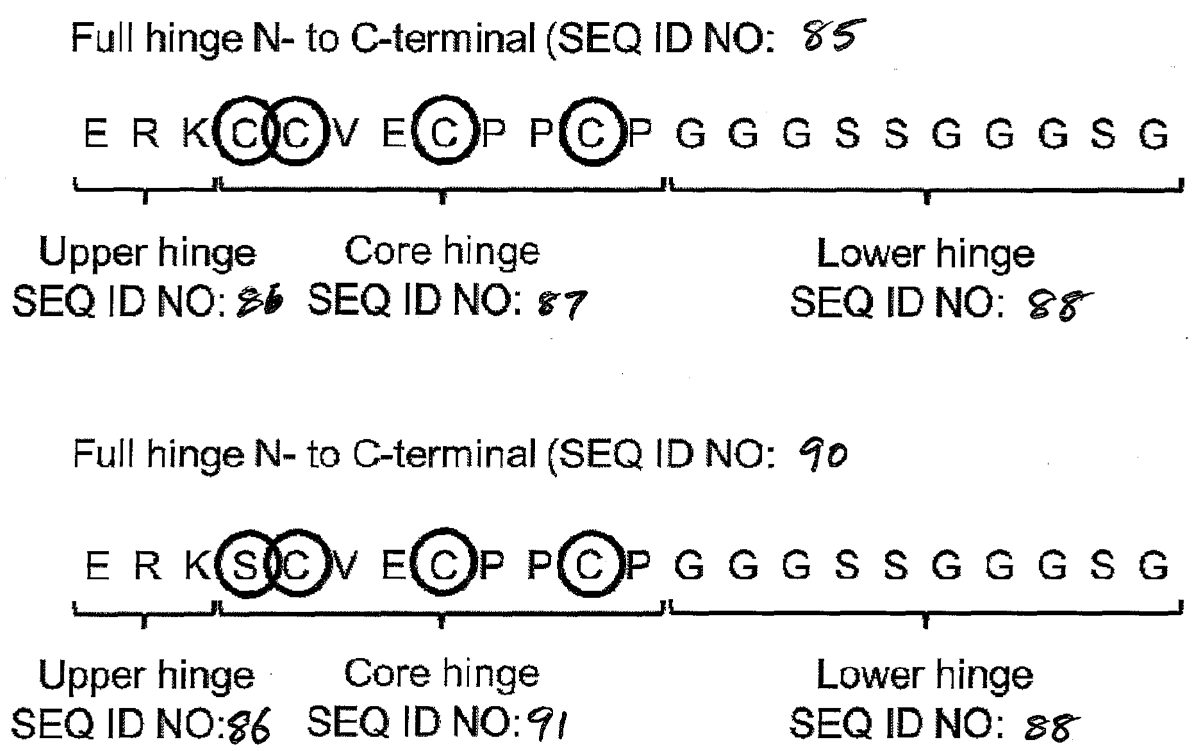
Full hinge N- to C-terminal (SEQ ID NO: 85
E R K C C V E C P P C P G G G S S G G G S G
Upper hinge
SEQ ID NO: 86
Core hinge
SEQ ID NO: 87
Lower hinge
SEQ ID NO: 88
Full hinge N- to C-terminal (SEQ ID NO: 90
E R K S C V E C P P C P G G G S S G G G S G
Upper hinge
SEQ ID NO:86
Core hinge
SEQ ID NO:91
Lower hinge
SEQ ID NO: 88

FIGURE 37

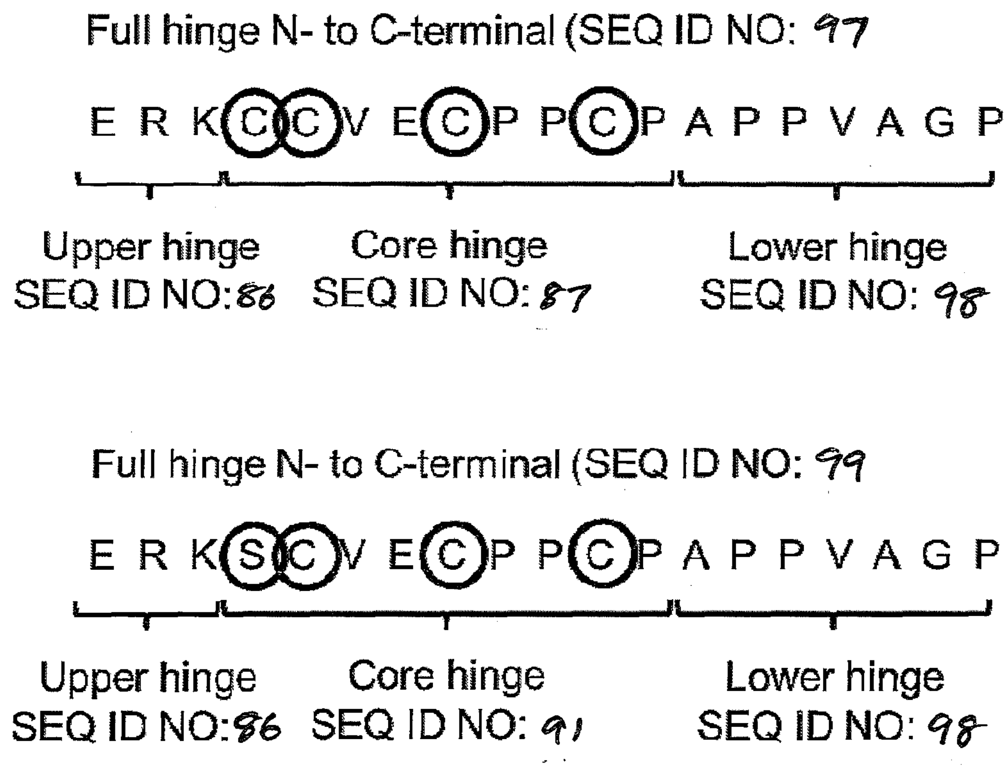
Full hinge N- to C-terminal (SEQ ID NO: 97
E R K C C V E C P P C P A P P V A G P
Upper hinge
SEQ ID NO:86
Core hinge
SEQ ID NO: 87
Lower hinge
SEQ ID NO: 98
Full hinge N- to C-terminal (SEQ ID NO: 99
E R K S C V E C P P C P A P P V A G P
Upper hinge
SEQ ID NO:86
Core hinge
SEQ ID NO: 91
Lower hinge
SEQ ID NO: 98

FIGURE 38

# FIGURE 39

XXXX Light chain CDRs (Chotia definition)

XXXX Heavy chain CDRs (Chotia definition)

XXXX Flexible linkers

XXXX Hinge

XXXX CH3

## IAB22 (CD8): (SEQ ID NO: 147)

DVQITQSPSSLSASVGDRVTITCRTSRSISQYLAWYQQKPGKVPKLLIYSGSTLQSGVPSRFSGSGSGTDFTLTISSLQPEDVATYYCQQHNENPLTFGG
GTKVEIK GSTSGGGSGGGSGGGGSS
EVQLVESGGGLVQPGGSLRLSCAASGFNIKDTYIHFVRQAPGKGLEWIGRIDPANDNTLYASKFQGKATISADTSKNTAYLQMNSLRAEDTAVYYCGRGY
GYYVFDHWGQGTLVTVSS

EPKSSDKTHTCPPCPPCGGGSSGGGSGGQPREPQVYTLPPSREEMTKNQVSLTCLVKGFYPSDIAVEWESNGQPENNYKTTPPVLDSDGSFFLYSKLTVD
KSRWQQGNVFSCSVMHEALHNHYTQKSLSLSPG

FIGURE 40

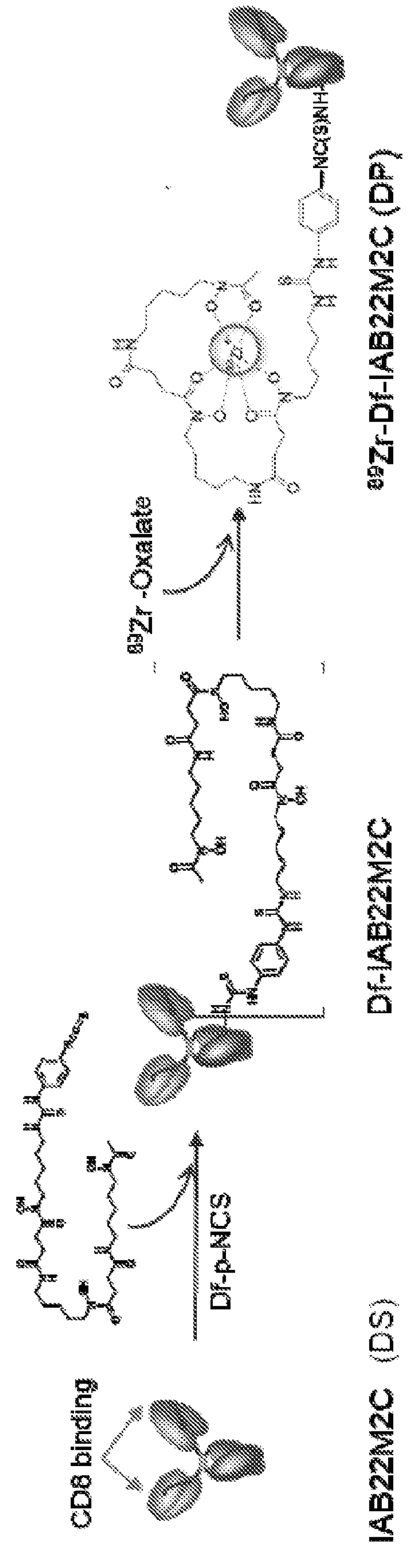
CD8 binding
Df-p-NCS
$^{89}$Zr -Oxalate
IAB22M2C (DS)
Df-IAB22M2C
$^{89}$Zr-Df-IAB22M2C (DP)
NC(S)NH

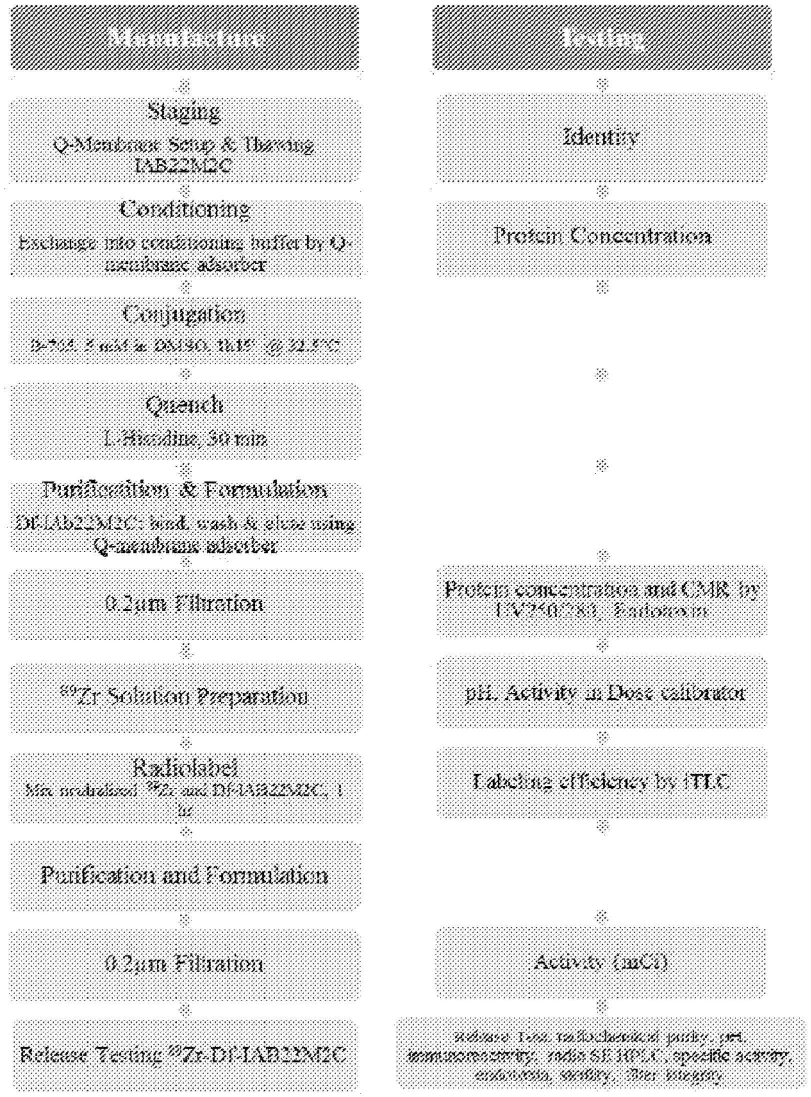
Manufacture
Staging
Q-Membrane Setup & Thawing IAB22M2C
Conditioning
Exchange into conditioning buffer by Q-membrane adsorber
Conjugation
Quench
L-Histidine, 30 min
Purification & Formulation
Df-IAB22M2C: bind, wash & elute using Q-membrane adsorber
0.2µm Filtration
89Zr Solution Preparation
Radiolabel
Mix neutralized 89Zr and Df-IAB22M2C, 1 hr
Purification and Formulation
0.2µm Filtration
Release Testing 89Zr-Df-IAB22M2C
Testing
Identity
Protein Concentration
Protein concentration and CMR by UV280/280, Endotoxin
pH, Activity in Dose calibrator
Labeling efficiency by iTLC
Activity (mCi)

FIGURE 41

# FIGURE 42A

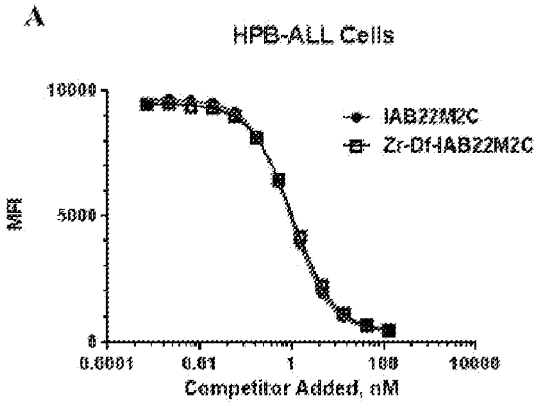


| Competitor | IAB22M2C | Zr-Df-IAB22M2C |
|---|---|---|
| **$IC_{50}$, nM** | 0.9 | 1.1 |

# FIGURE 42B

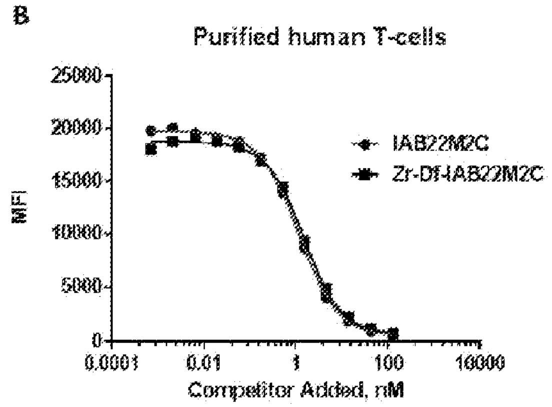


| Competitor | IAB22M2C | Zr-Df-IAB22M2C |
|---|---|---|
| **$IC_{50}$, nM** | 1.2 | 1.5 |

FIGURE 43

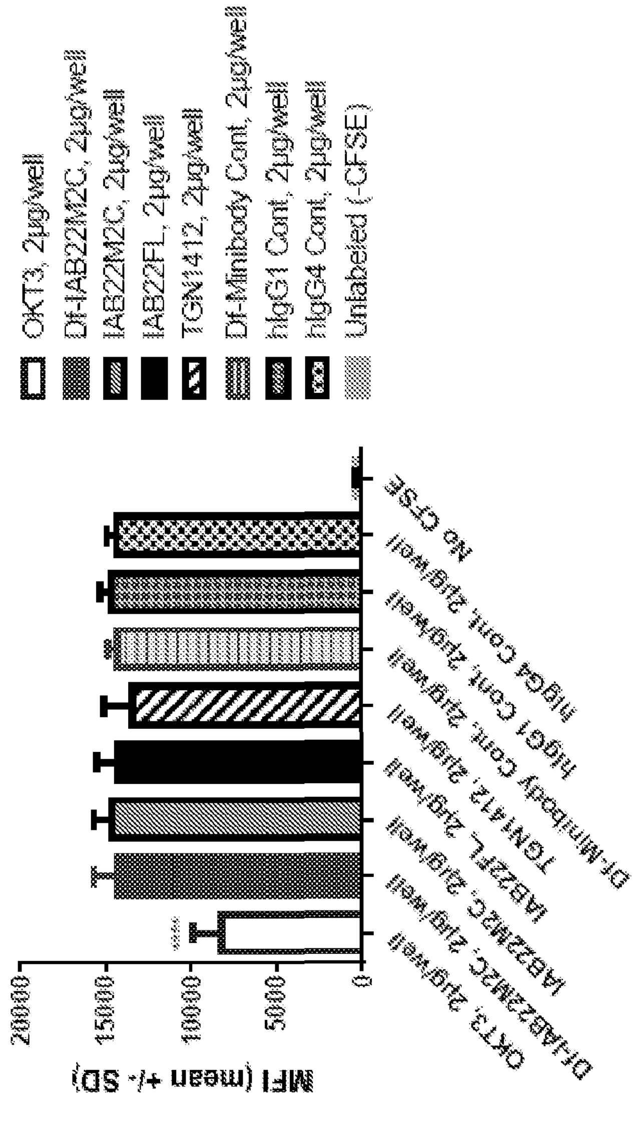
MFI (mean +/- SD)
20000
15000
10000
5000
0
OKT3, 2µg/well
Df-IAB22M2C, 2µg/well
IAB22M2C, 2µg/well
IAB22FL, 2µg/well
TGN1412, 2µg/well
Df-Minibody Cont, 2µg/well
hIgG1 Cont, 2µg/well
hIgG4 Cont, 2µg/well
No CFSE
OKT3, 2µg/well
Df-IAB22M2C, 2µg/well
IAB22M2C, 2µg/well
IAB22FL, 2µg/well
TGN1412, 2µg/well
Df-Minibody Cont, 2µg/well
hIgG1 Cont, 2µg/well
hIgG4 Cont, 2µg/well
Unlabeled (-CFSE)

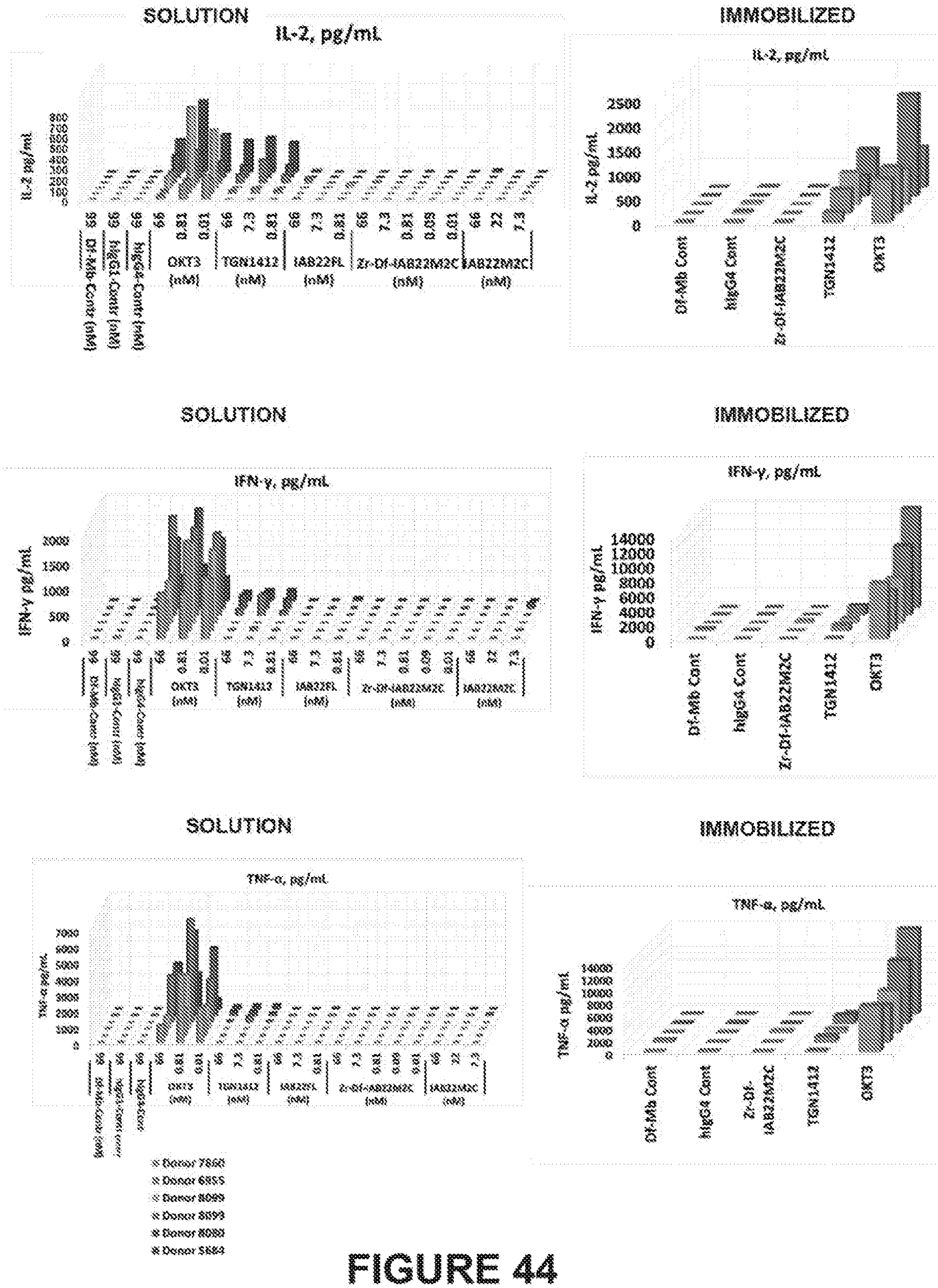


FIGURE 44

SUBSTITUTE SHEET (RULE 26)

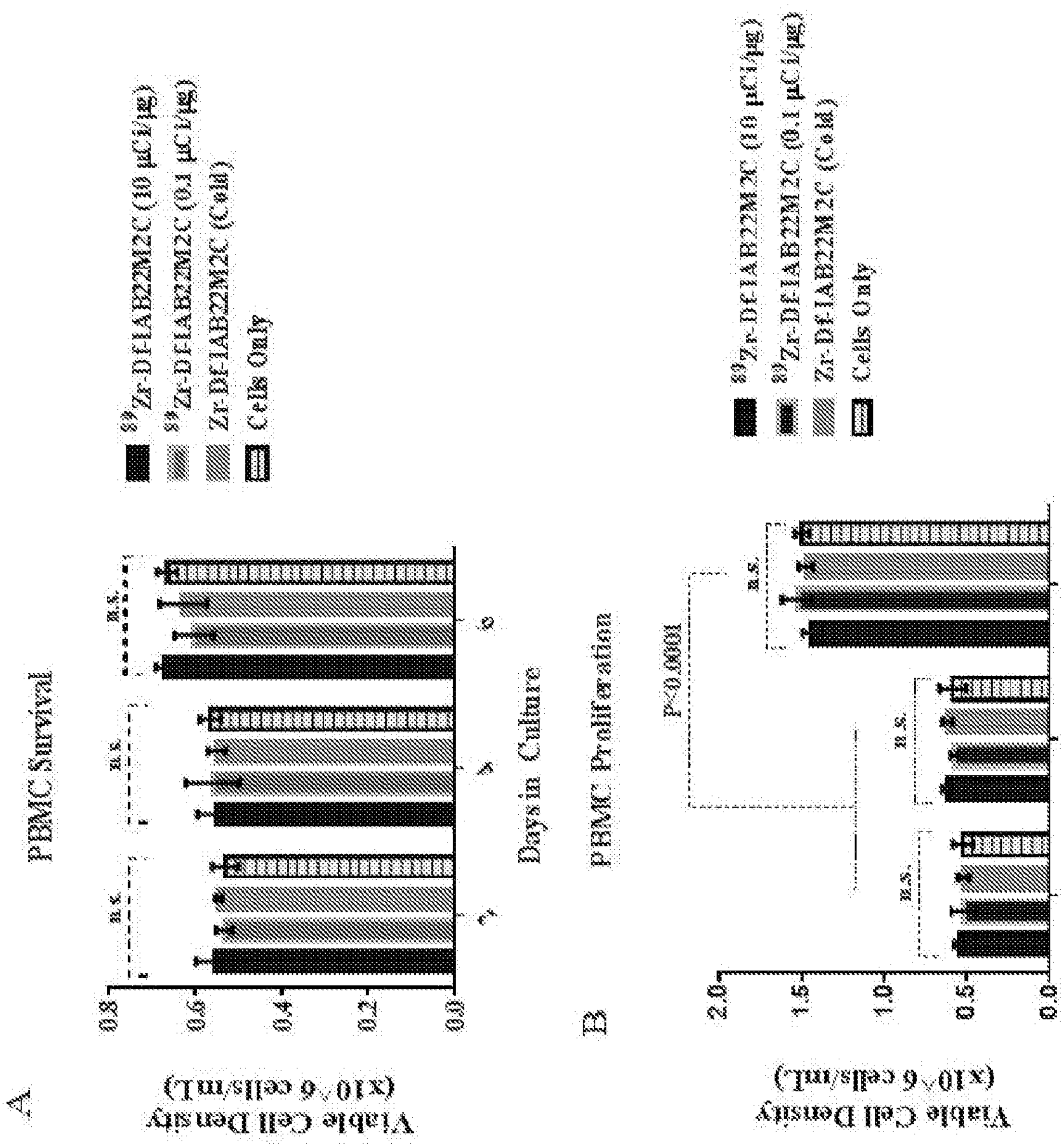
A
PBMC Survival
Viable Cell Density (x10^6 cells/mL)
0.8
0.6
0.4
0.2
0.0
n.s.
Days in Culture
89Zr-DFIAB22M2C (10 μCi/μg)
89Zr-DFIAB22M2C (0.1 μCi/μg)
Zr-DFIAB22M2C (Cold)
Cells Only
B
PBMC Proliferation
Viable Cell Density (x10^6 cells/mL)
2.0
1.5
1.0
0.5
0.0
P<0.0001
n.s.
Days in Culture
89Zr-DFIAB22M2C (10 μCi/μg)
89Zr-DFIAB22M2C (0.1 μCi/μg)
Zr-DFIAB22M2C (Cold)
Cells Only

FIGURE 45

# FIGURE 46

**Table 3**

**Table 5-X:** Predicted pharmacokinetic parameters for an intravenous 20 μg/kg dose of 89Zr-Df-IAB22M2C in humans.

| PK Parameter | Value | Description |
|---|---|---|
| ***Blood*** | | |
| $CL_1$ (mL/h-kg) | 1.51 | Clearance rate from the central compartment (blood) |
| $CL_2$ (mL/h-kg) | 4.17 | Clearance rate from the peripheral compartment (tissues) |
| $V_1$ (mL/kg) | 24.2 | Volume of the central compartment |
| $V_2$ (mL/kg) | 113 | Volume of the peripheral compartment |
| $k_{10}$ (h-1) | 0.0623 | Elimination rate constant from the central compartment |
| $k_{12}$ (h-1) | 0.172 | Distribution rate constant from the central to the peripheral compartment |
| $k_{21}$ (h-1) | 0.0369 | Distribution rate constant from peripheral to the central compartment |
| $t_{1/2\alpha}$(h) | 2.64 | Alpha phase half-life |
| $t_{1/2\beta}$(h) | 78.9 | Beta phase half-life |
| MRT (h) | 90.7 | Mean residence time |
| $V_{ss}$ (mL/kg) | 137 | Volume of distribution at steady state |
| $C_{max}$ (ng/mL) | 825 | Maximum blood concentration |
| ***Tissue (lungs)*** | | |
| $k_{in}$ ($h^{-1}$), low dose | 0.0241 | Distribution rate into the lungs |
| $k_{out}$ ($h^{-1}$) | 0.00202 | Elimination rate out of the lungs |
| $V_{max}$ (ng/h) | 62.5 | Saturable elimination rate out of the lungs |
| $k_m$ (ng) | 646 | Michaelis constant for saturable elimination in the lungs |
| $T_{max}$ (h) | 6.72 | Time to maximum concentration for the 20 μg/kg dose |
| $t_{1/2}$ (h) | 55.6 | Half-life* for the 20 μg/kg dose |
| $C_{max}$ (ng/mL) | 44.7 | Maximum concentration in tissue |

* half-life derived by non-compartmental method

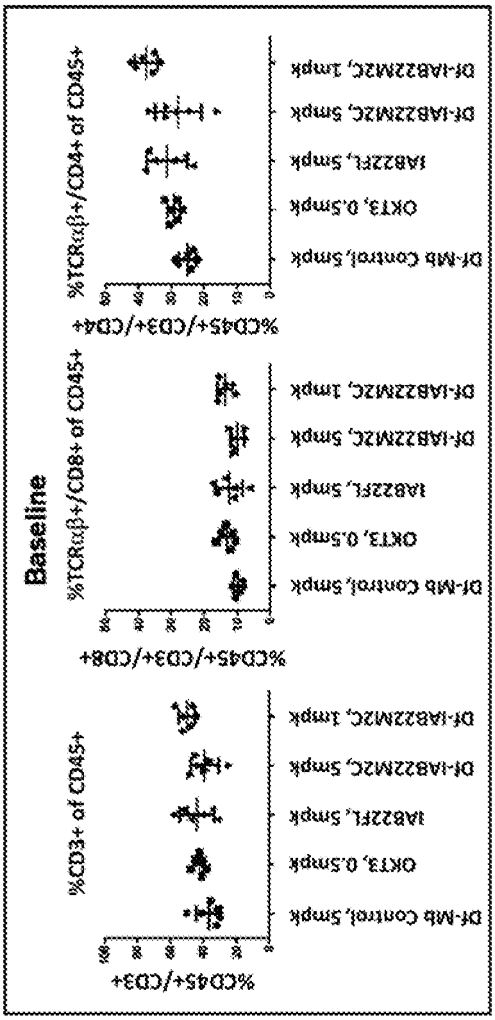


FIGURE 47

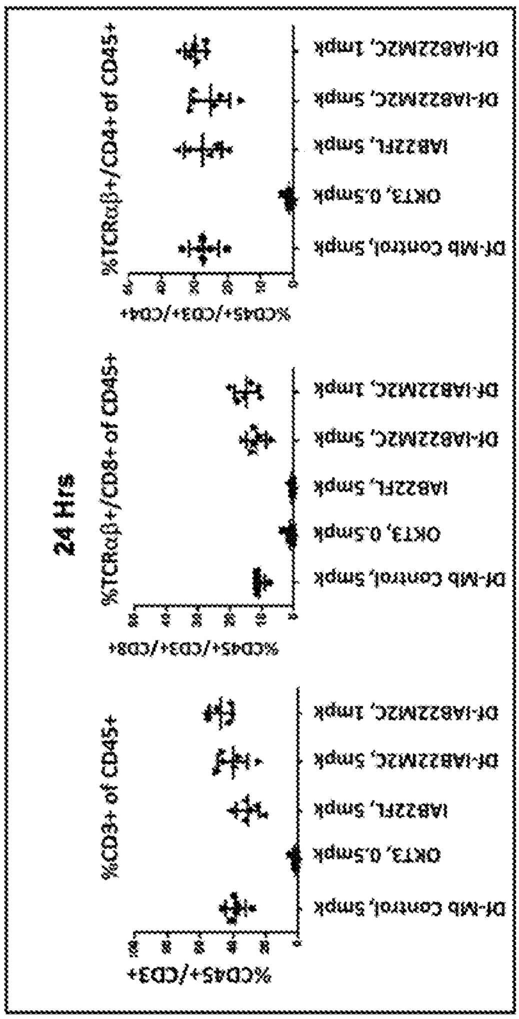


FIGURE 48

FIGURE 49

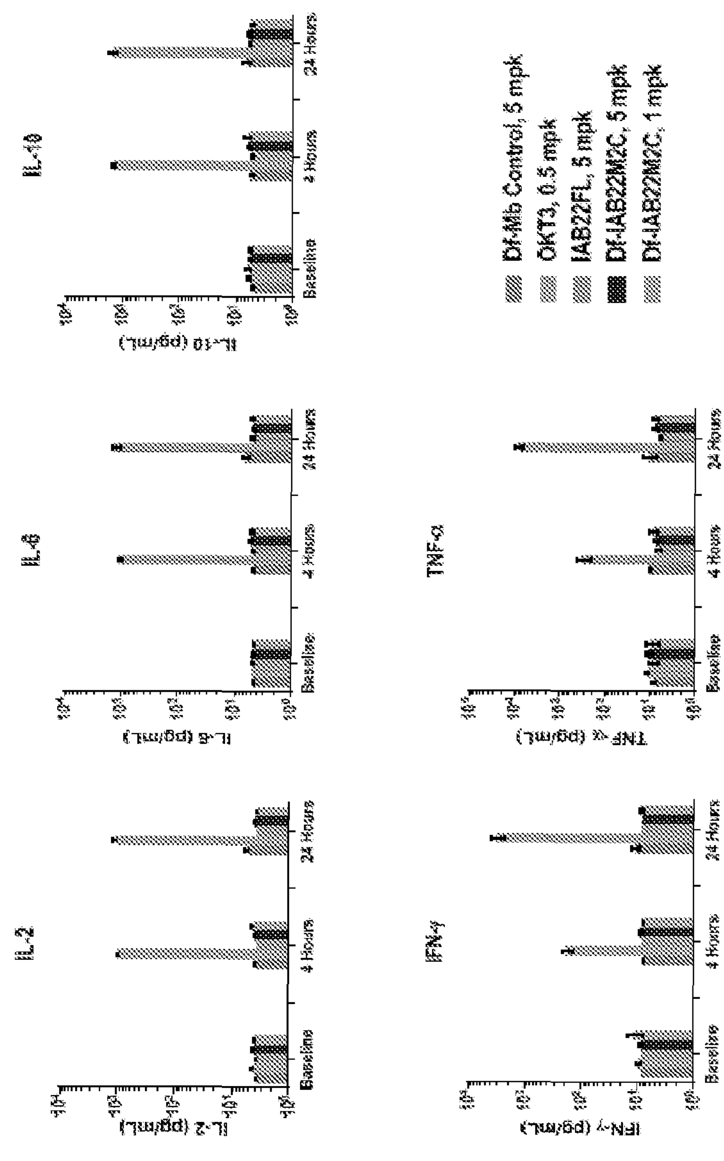

IL-2
IL-6
IL-10
IFN-γ
TNF-α
IL-2 (pg/mL)
IL-6 (pg/mL)
IL-10 (pg/mL)
IFN-γ (pg/mL)
TNF-α (pg/mL)
Baseline
4 Hours
24 Hours
DF-Mb Control, 5 mpk
OKT3, 0.5 mpk
IAB22FL, 5 mpk
DF-IAB22M2C, 5 mpk
DF-IAB22M2C, 1 mpk

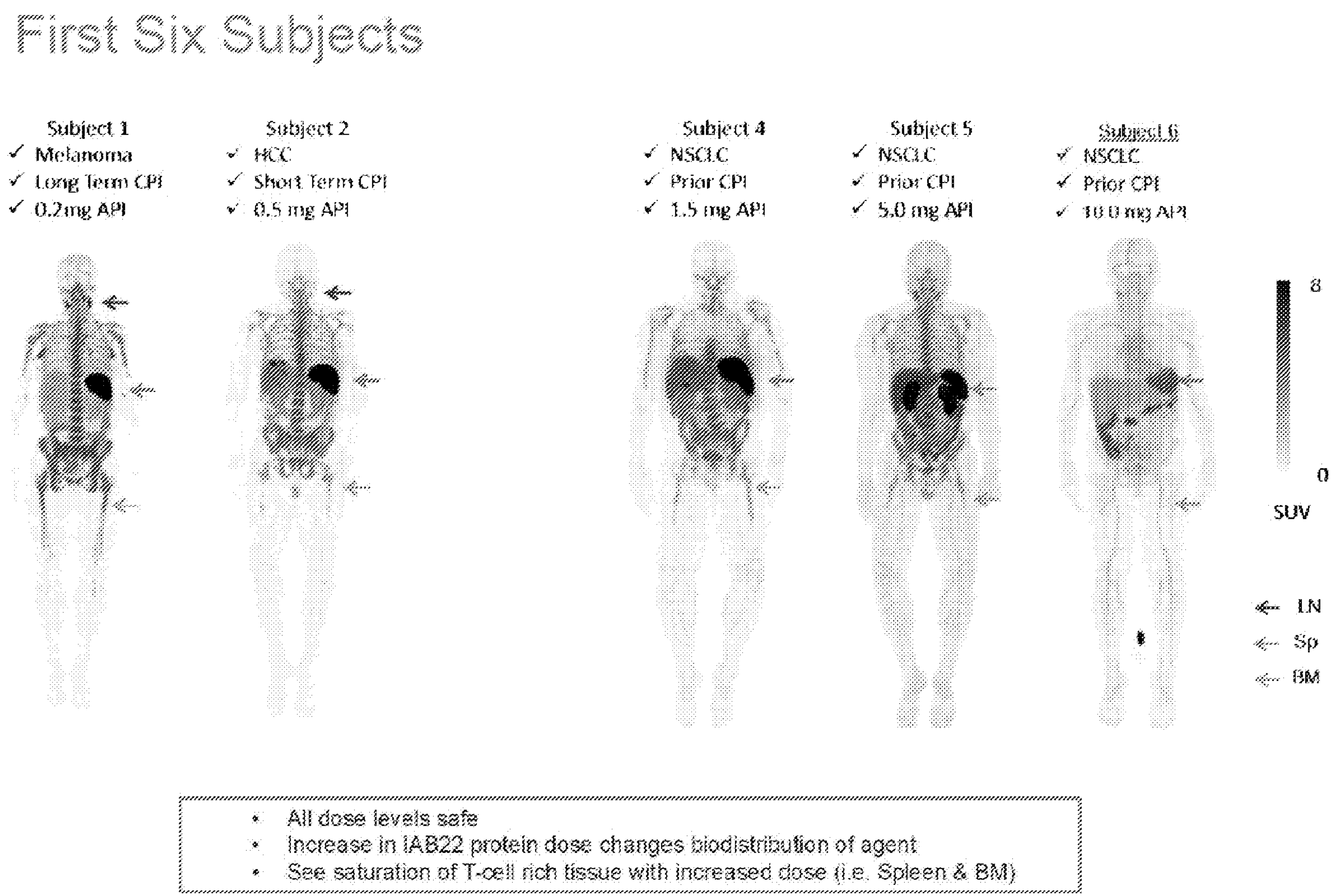
First Six Subjects
Subject 1
✓ Melanoma
✓ Long Term CPI
✓ 0.2mg API
Subject 2
✓ HCC
✓ Short Term CPI
✓ 0.5 mg API
Subject 4
✓ NSCLC
✓ Prior CPI
✓ 1.5 mg API
Subject 5
✓ NSCLC
✓ Prior CPI
✓ 5.0 mg API
Subject 6
✓ NSCLC
✓ Prior CPI
✓ 10.0 mg API
8
0
SUV
LN
Sp
BM
• All dose levels safe
• Increase in IAB22 protein dose changes biodistribution of agent
• See saturation of T-cell rich tissue with increased dose (i.e. Spleen & BM)

FIGRUE 50

**Patient 1 - Subject**: 37 years old, female Metastatic Melanoma, diagnosed
**Treatment history:** Previous Ipilimumab/Nivolumab treatment (2 Years): Pembrolizumab: Ongoing

Note: All images are our $^{89}Zr$-IAB22M2C, except FDG PET/CT in left hand corner.

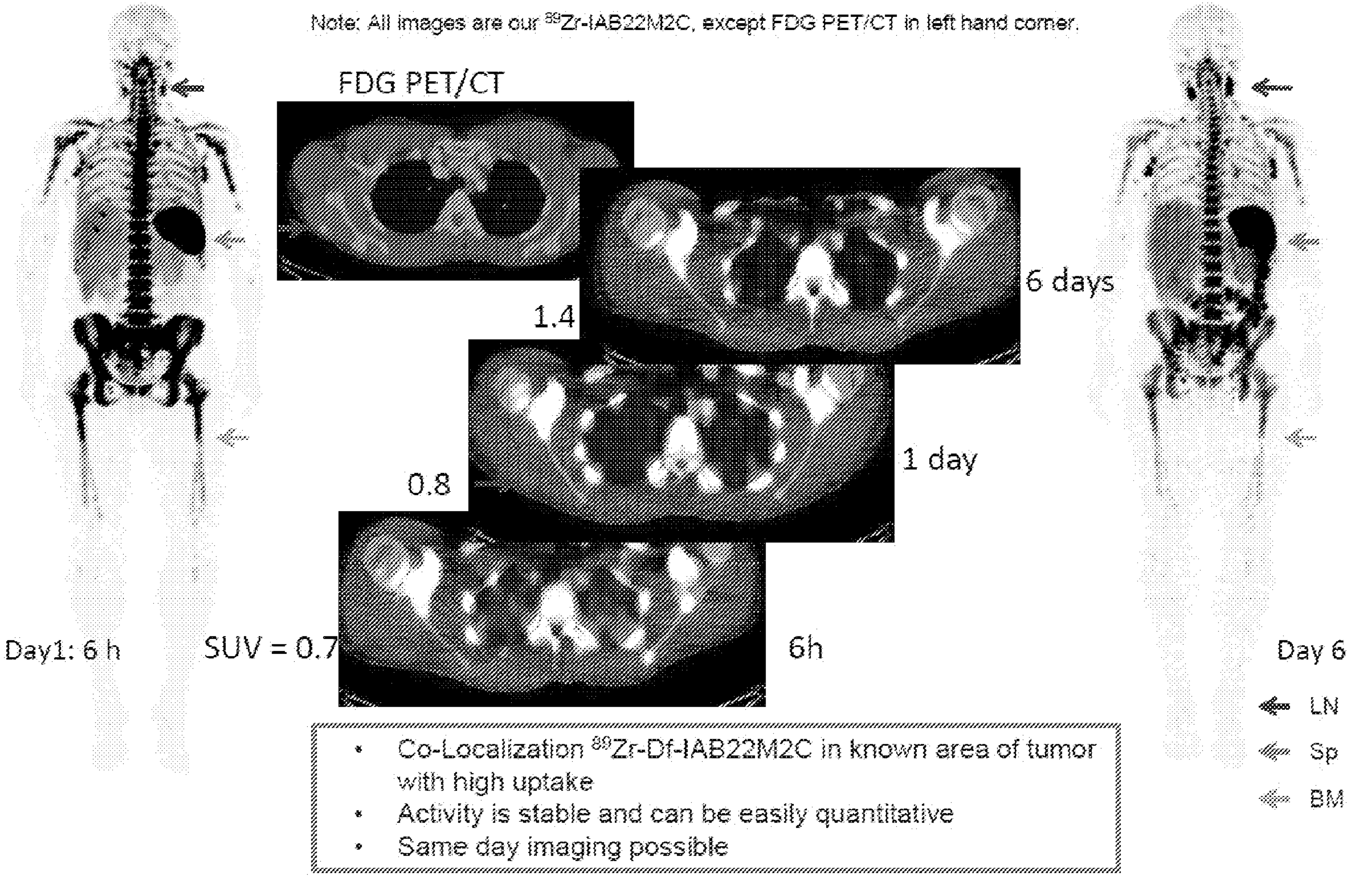


- Co-Localization $^{89}Zr$-Df-IAB22M2C in known area of tumor with high uptake
- Activity is stable and can be easily quantitative
- Same day imaging possible

**FIGRUE 51**

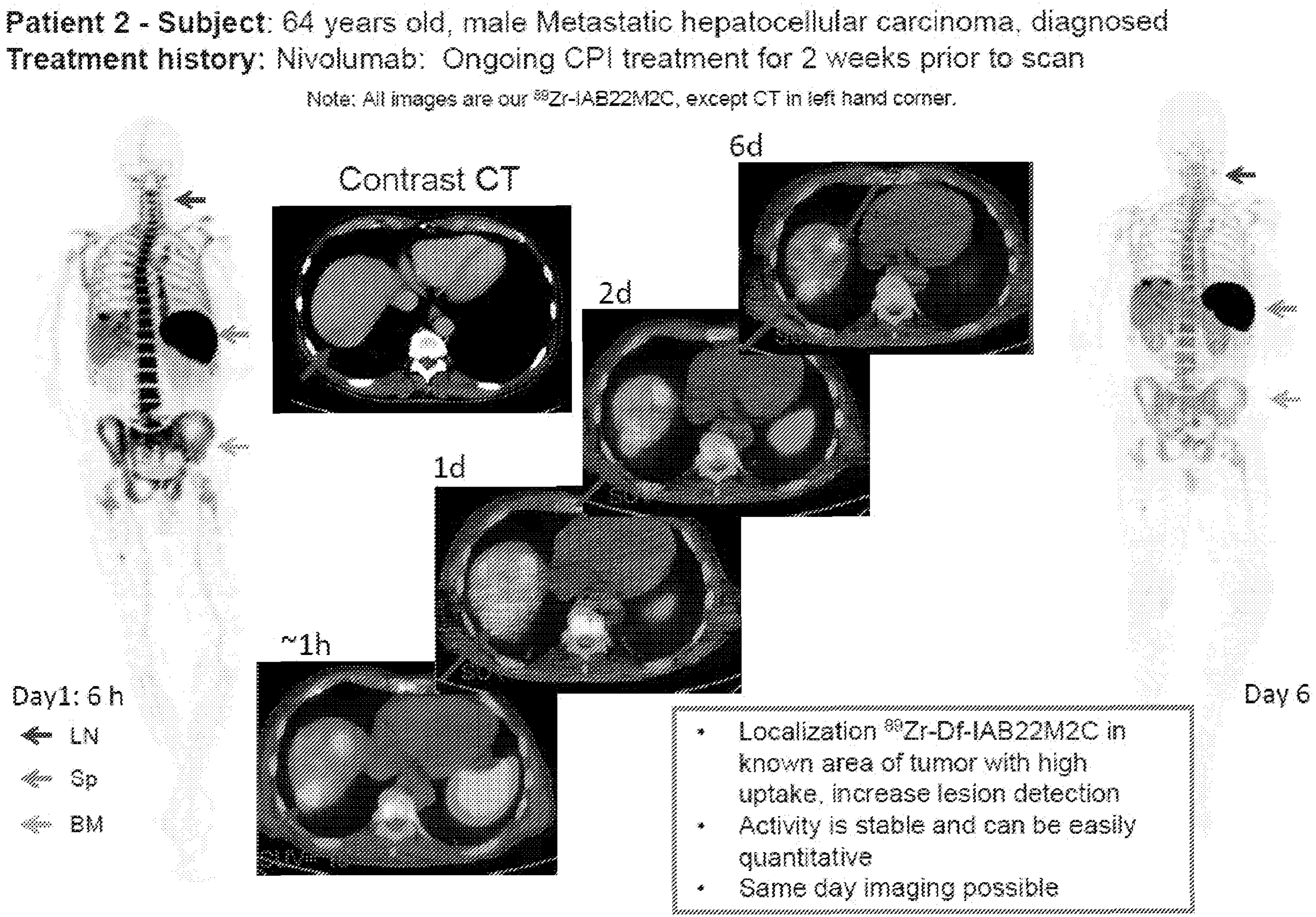

**Patient 2 - Subject**: 64 years old, male Metastatic hepatocellular carcinoma, diagnosed
**Treatment history**: Nivolumab: Ongoing CPI treatment for 2 weeks prior to scan
Note: All images are our $^{89}$Zr-IAB22M2C, except CT in left hand corner.
Contrast CT
6d
2d
1d
~1h
Day1: 6 h
LN
Sp
BM
Day 6
• Localization $^{89}$Zr-Df-IAB22M2C in known area of tumor with high uptake, increase lesion detection
• Activity is stable and can be easily quantitative
• Same day imaging possible


FIGURE 52

# The Other 6 Patients

Subject 4

- ✓ NSCLC
- ✓ Previous CPI
- ✓ 1.5 mg API
- ✓ No visible co-localization

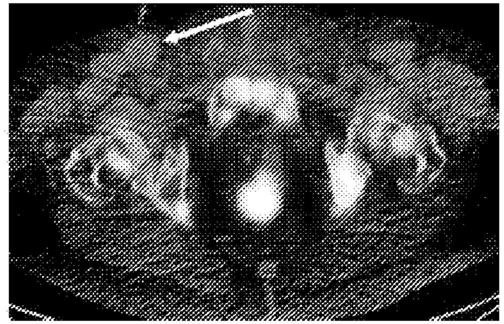

Subject 5

- ✓ NSCLC
- ✓ Previous CPI
- ✓ 5 mg API
- ✓ Regional node uptake, no tumor co-localization

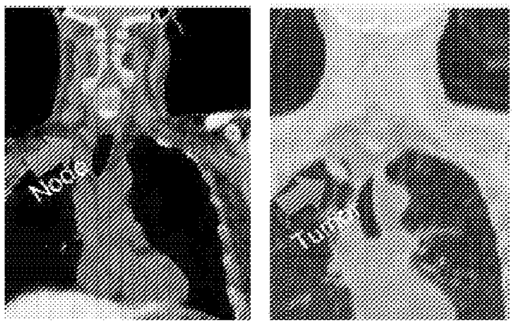


Subject 6

- ✓ NSCLC
- ✓ Previous CPI
- ✓ 10 mg API
- ✓ No visible tumor co-localization

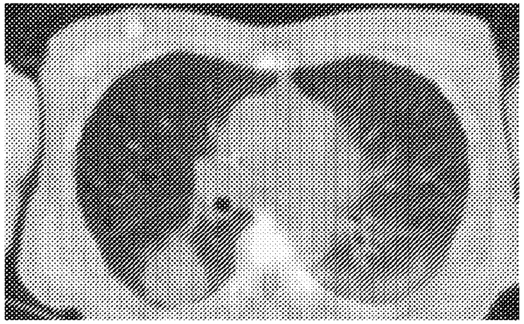

Subject 7

- ✓ Melanoma
- ✓ Previous CPI
- ✓ 1.5 mg API
- ✓ ***Strong*** co-localization in nodal disease

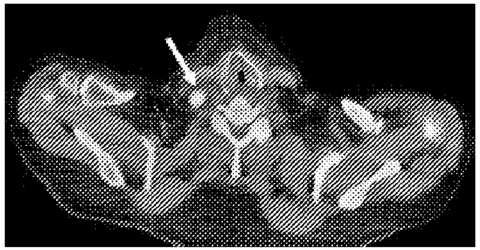

Subject 8

- ✓ NSCLC
- ✓ On CPI
- ✓ 1.5 mg API
- ✓ Questionable tumor co-localization

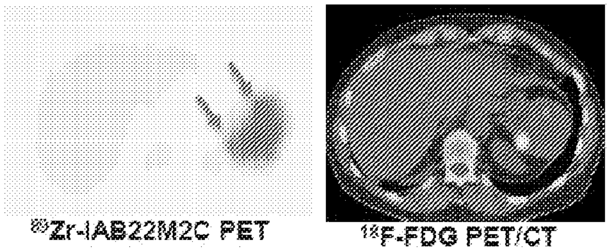

**$^{89}$Zr-IAB22M2C PET** **$^{18}$F-FDG PET/CT**

**FIGURE 53**

Subject 12

Melanoma
On CPI
1.5mg API
Multiple areas of CD8 localization, including potentially non-tumor areas

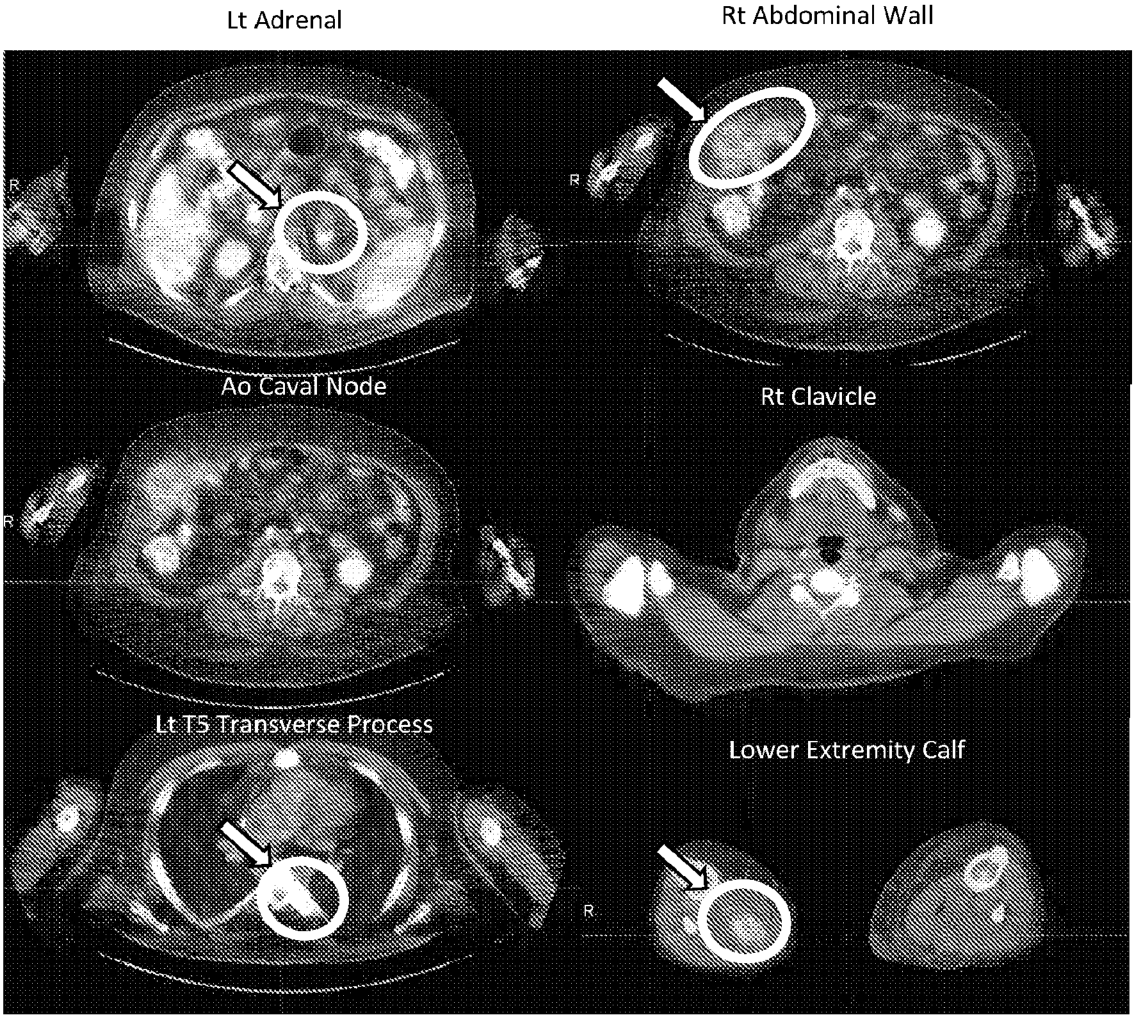


FIGURE 54

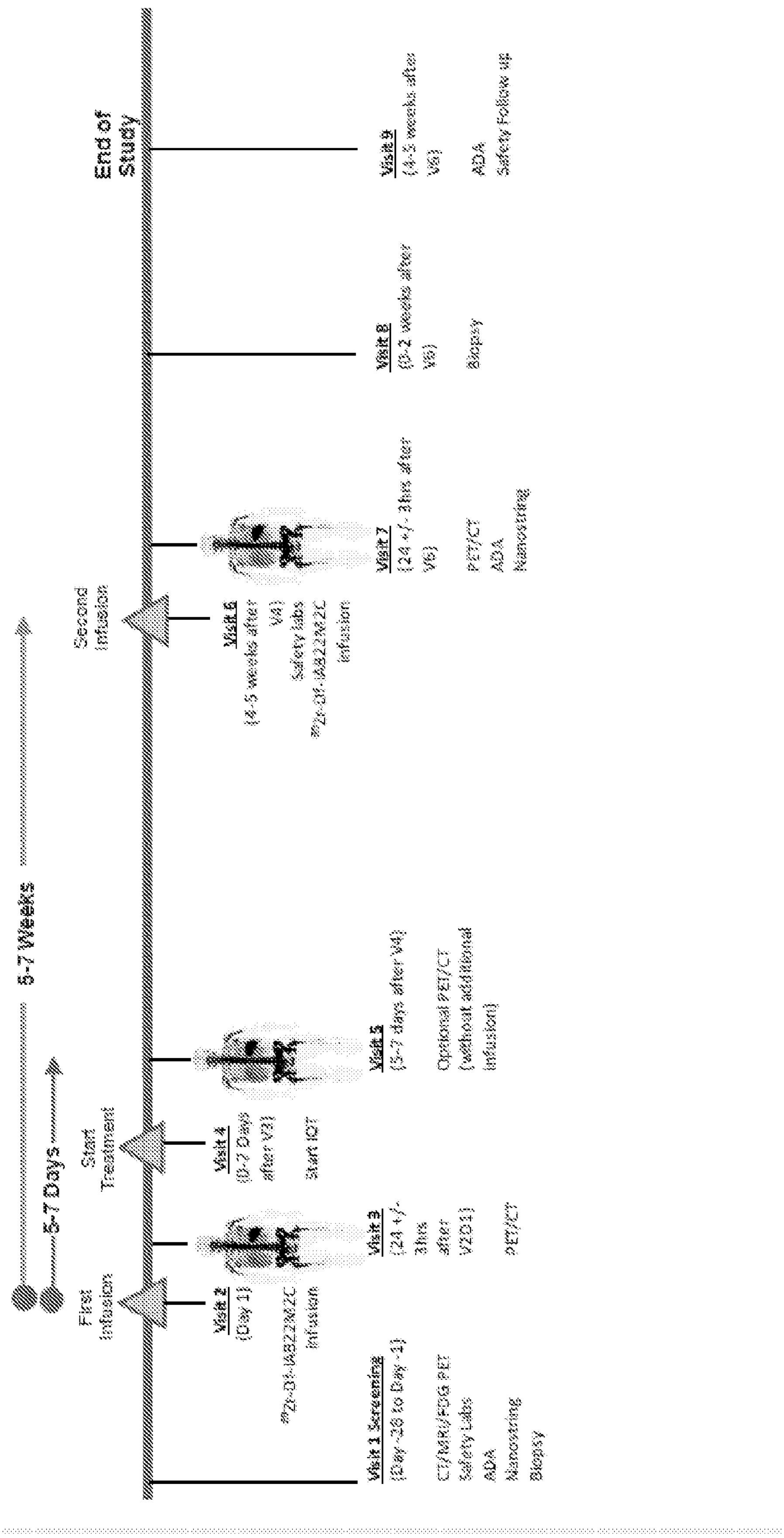
5-7 Weeks
5-7 Days
First Infusion
Start Treatment
Second Infusion
End of Study
Visit 1 Screening
(Day -28 to Day -1)
CT/MRI/FDG PET
Safety Labs
ADA
Nanostring
Biopsy
Visit 2
(Day 1)
89Zr-Df-IAB22M2C infusion
Visit 3
(24 +/- 3hrs after V2D1)
PET/CT
Visit 4
(0-7 Days after V3)
Start IOT
Visit 5
(5-7 days after V4)
Optional PET/CT (without additional infusion)
Visit 6
(4-5 weeks after V4)
Safety labs
89Zr-Df-IAB22M2C infusion
Visit 7
(24 +/- 3hrs after V6)
PET/CT
ADA
Nanostring
Visit 8
(0-2 weeks after V6)
Biopsy
Visit 9
(4-5 weeks after V6)
ADA
Safety Follow up

FIGURE 55

FIG. 56

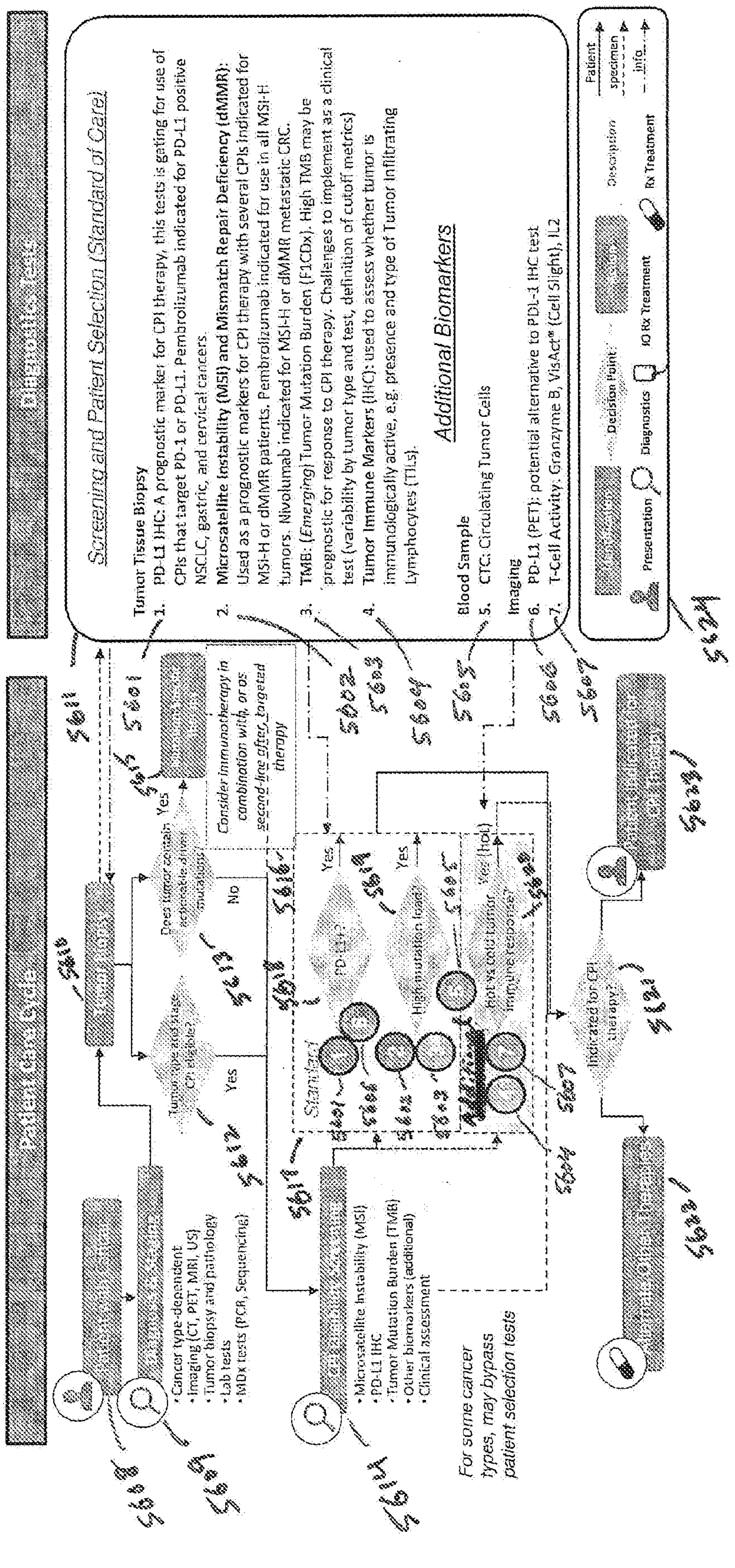
Patient Care Cycle
Cancer type-dependent
Imaging (CT, PET, MRI, US)
Tumor biopsy and pathology
Lab tests
MDx tests (PCR, Sequencing)
Microsatellite Instability (MSI)
PD-L1 IHC
Tumor Mutation Burden (TMB)
Other biomarkers (additional)
Clinical assessment
For some cancer types, may bypass patient selection tests
Tumor type and stage CPI eligible?
Yes
Does tumor contain actionable driver mutations?
No
Yes
Consider immunotherapy in combination with, or as second-line after, targeted therapy
Standard
PD-L1+?
Yes
High mutation load?
Yes
Hot vs cold tumor immune response?
Yes (hot)
Indicated for CPI therapy?
Diagnostics Tests
Screening and Patient Selection (Standard of Care)
Tumor Tissue Biopsy
1. PD-L1 IHC: A prognostic marker for CPI therapy, this tests is gating for use of CPIs that target PD-1 or PD-L1. Pembrolizumab indicated for PD-L1 positive NSCLC, gastric, and cervical cancers.
2. Microsatellite Instability (MSI) and Mismatch Repair Deficiency (dMMR): Used as a prognostic markers for CPI therapy with several CPIs indicated for MSI-H or dMMR patients. Pembrolizumab indicated for use in all MSI-H tumors. Nivolumab indicated for MSI-H or dMMR metastatic CRC.
3. TMB: (Emerging) Tumor Mutation Burden (F1CDx). High TMB may be prognostic for response to CPI therapy. Challenges to implement as a clinical test (variability by tumor type and test, definition of cutoff metrics)
4. Tumor Immune Markers (IHC): used to assess whether tumor is immunologically active, e.g. presence and type of Tumor Infiltrating Lymphocytes (TILs).
Additional Biomarkers
Blood Sample
5. CTC: Circulating Tumor Cells
Imaging
6. PD-L1 (PET): potential alternative to PDL-1 IHC test
7. T-Cell Activity: Granzyme B, VisAct™ (Cell Sight), IL2
Presentation
Diagnostics
Decision Point
IO Rx Treatment
Rx Treatment
Description
Patient
specimen
info

FIG. 57

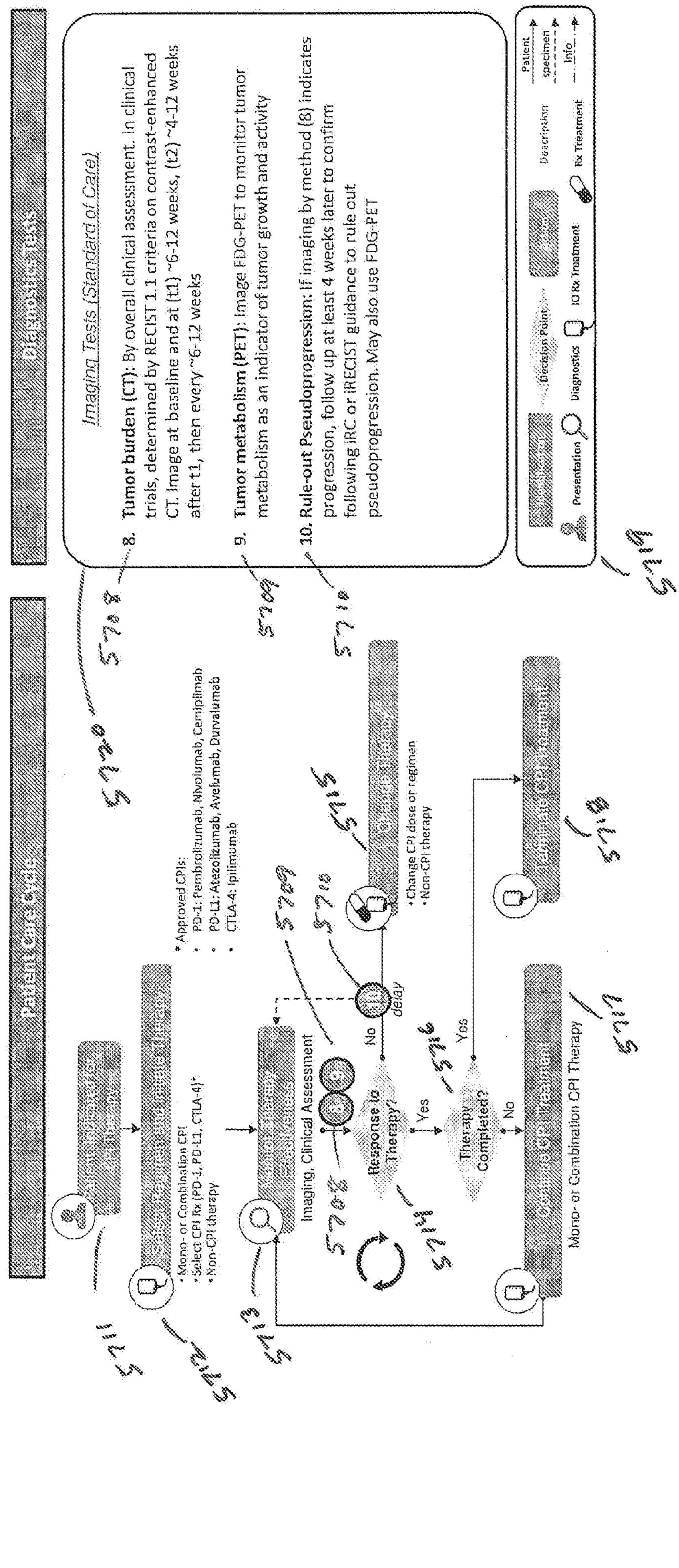
Patient Care Cycle
Diagnostics Tests
• Mono- or Combination CPI
• Select CPI Rx (PD-1, PD-L1, CTLA-4)*
• Non-CPI therapy
* Approved CPIs:
• PD-1: Pembrolizumab, Nivolumab, Cemiplimab
• PD-L1: Atezolizumab, Avelumab, Durvalumab
• CTLA-4: Ipilimumab
Imaging, Clinical Assessment
Response to Therapy?
Yes
No
delay
Therapy Completed?
Yes
No
• Change CPI dose or regimen
• Non-CPI therapy
Mono- or Combination CPI Therapy
Imaging Tests (Standard of Care)
8. Tumor burden (CT): By overall clinical assessment. In clinical trials, determined by RECIST 1.1 criteria on contrast-enhanced CT. Image at baseline and at (t1) ~6-12 weeks, (t2) ~4-12 weeks after t1, then every ~6-12 weeks
9. Tumor metabolism (PET): Image FDG-PET to monitor tumor metabolism as an indicator of tumor growth and activity
10. Rule-out Pseudoprogression: If imaging by method (8) indicates progression, follow up at least 4 weeks later to confirm following iRC or iRECIST guidance to rule out pseudoprogression. May also use FDG-PET
Presentation
Diagnostics
Decision Point
ID Rx Treatment
Rx Treatment
Description
Patient
specimen
Info
5711
5712
5713
5708
5709
5710
5714
5716
5715
5717
5718
5720
5719

FIG. 58

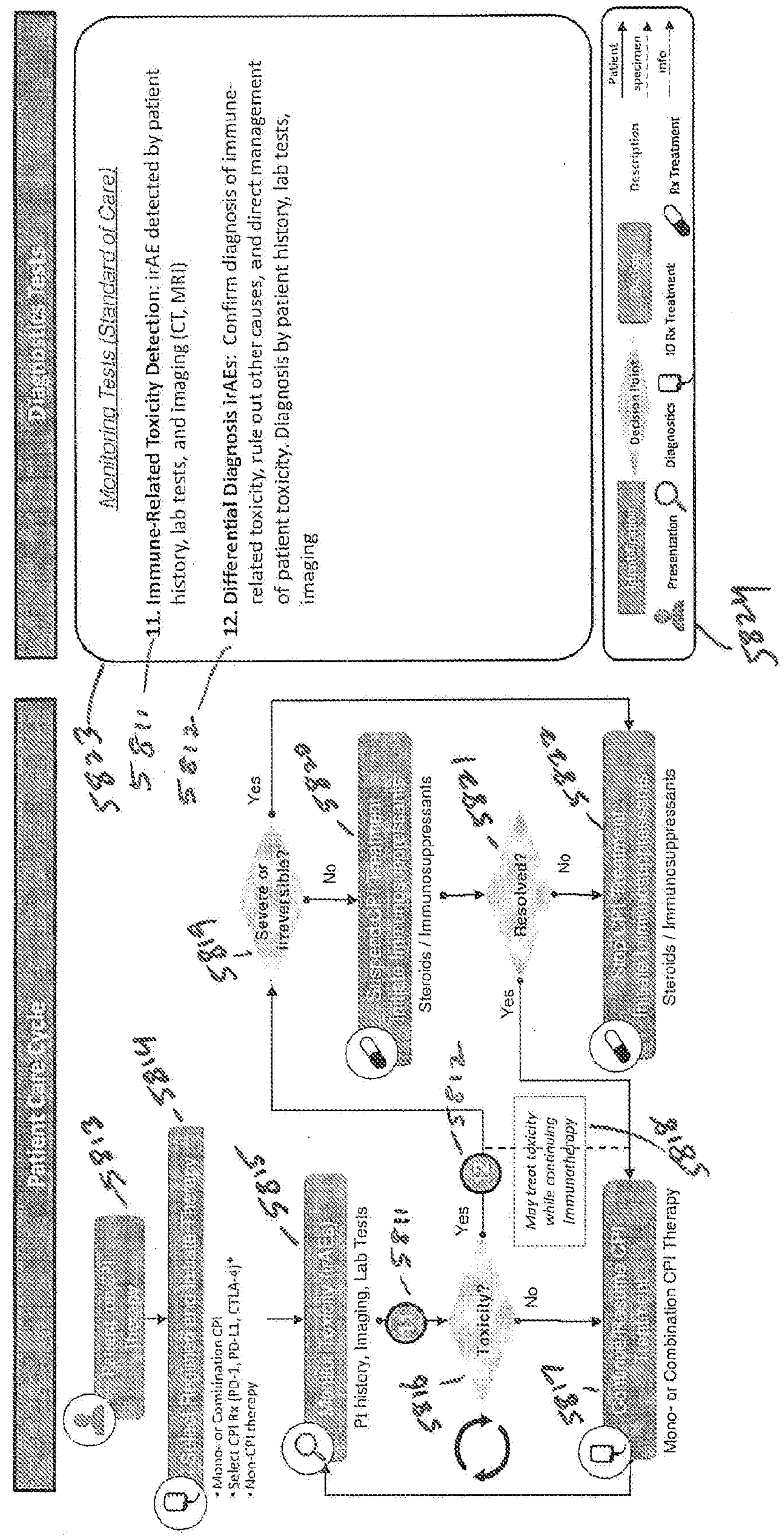
Patient Care Cycle
Diagnostics Tests
Monitoring Tests (Standard of Care)
11. Immune-Related Toxicity Detection: irAE detected by patient history, lab tests, and imaging (CT, MRI)
12. Differential Diagnosis irAEs: Confirm diagnosis of immune-related toxicity, rule out other causes, and direct management of patient toxicity. Diagnosis by patient history, lab tests, imaging
• Mono- or Combination CPI
• Select CPI Rx (PD-1, PD-L1, CTLA-4)*
• Non-CPI therapy
Pt history, Imaging, Lab Tests
Toxicity?
Yes
No
May treat toxicity while continuing Immunotherapy
Mono- or Combination CPI Therapy
Severe or irreversible?
Yes
No
Steroids / Immunosuppressants
Resolved?
Yes
No
Steroids / Immunosuppressants
Presentation
Diagnostics
Decision Point
IO Rx Treatment
Description
Rx Treatment
Patient
specimen
info
5811
5812
5813
5814
5815
5816
5817
5818
5819
5820
5821
5822
5823
5824

FIG. 59

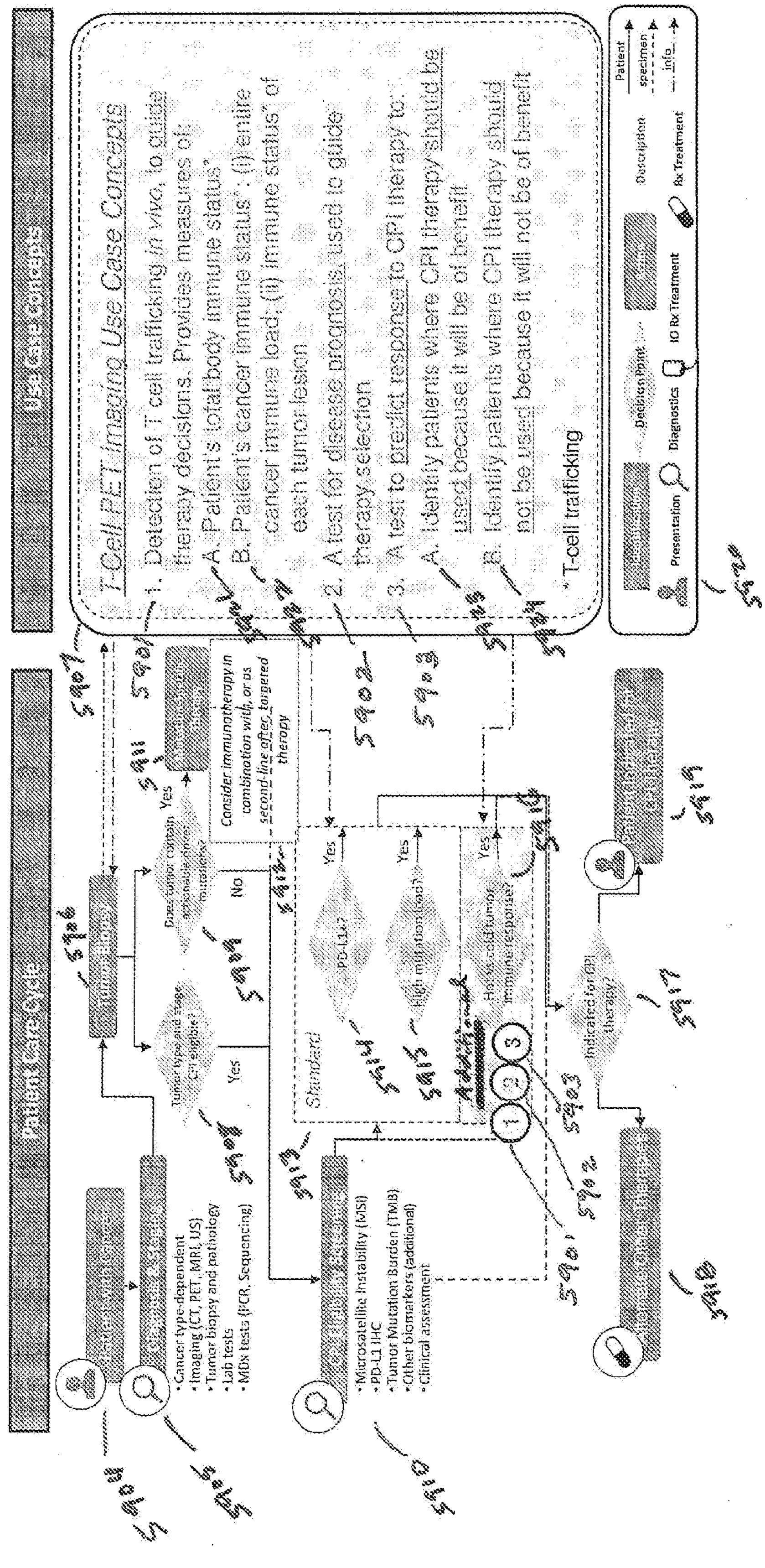
Patient Care Cycle
Use Case Concepts
T-Cell PET Imaging Use Case Concepts
1. Detection of T cell trafficking in vivo, to guide therapy decisions. Provides measures of:
A. "Patient's total body immune status"
B. "Patient's cancer immune status": (i) entire cancer immune load; (ii) immune status* of each tumor lesion
2. A test for disease prognosis, used to guide therapy selection
3. A test to predict response to CPI therapy to:
A. Identify patients where CPI therapy should be used because it will be of benefit
B. Identify patients where CPI therapy should not be used because it will not be of benefit
* T-cell trafficking
• Cancer type-dependent
• Imaging (CT, PET, MRI, US)
• Tumor biopsy and pathology
• Lab tests
• MDx tests (PCR, Sequencing)
Tumor type and stage CT eligible?
Yes
Does tumor contain actionable driver mutations?
No
Yes
Consider immunotherapy in combination with, or as second-line after, targeted therapy
• Microsatellite Instability (MSI)
• PD-L1 IHC
• Tumor Mutation Burden (TMB)
• Other biomarkers (additional)
• Clinical assessment
Standard
PD-L1?
High mutation load?
Has cold tumor immune response?
Indicated for CPI therapy?
Presentation
Diagnostics
Decision Point
IO Rx Treatment
Rx Treatment
Description
Patient
specimen
info
5904
5905
5910
5906
5908
5909
5911
5907
5901
5902
5903
5912
5913
5914
5915
5916
5917
5918
5919
5920
5921
5922
5923
5924

FIG. 60

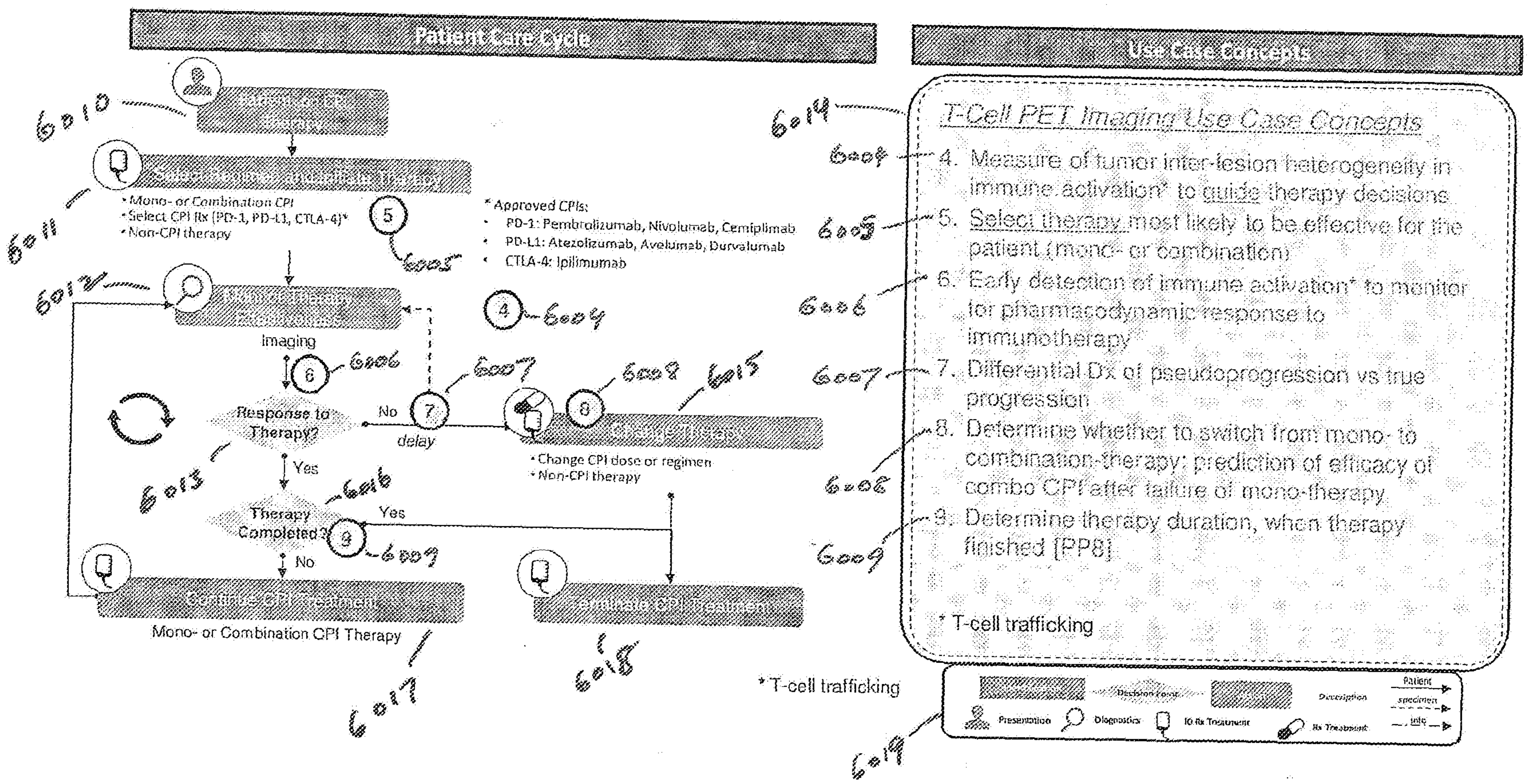
Patient Care Cycle
Use Case Concepts
• Mono- or Combination CPI
• Select CPI Rx (PD-1, PD-L1, CTLA-4)*
• Non-CPI therapy
* Approved CPIs:
• PD-1: Pembrolizumab, Nivolumab, Cemiplimab
• PD-L1: Atezolizumab, Avelumab, Durvalumab
• CTLA-4: Ipilimumab
Imaging
Response to Therapy?
No
delay
Yes
Therapy Completed?
No
• Change CPI dose or regimen
• Non-CPI therapy
Mono- or Combination CPI Therapy
* T-cell trafficking
T-Cell PET Imaging Use Case Concepts
4. Measure of tumor inter-lesion heterogeneity in immune activation* to guide therapy decisions
5. Select therapy most likely to be effective for the patient (mono- or combination)
6. Early detection of immune activation* to monitor for pharmacodynamic response to immunotherapy
7. Differential Dx of pseudoprogression vs true progression
8. Determine whether to switch from mono- to combination-therapy; prediction of efficacy of combo CPI after failure of mono-therapy
9. Determine therapy duration, when therapy finished [PP8]
* T-cell trafficking
Presentation
Diagnostics
IO Rx Treatment
Rx Treatment
Description
Patient
specimen
info
6004
6005
6006
6007
6008
6009
6010
6011
6012
6013
6014
6015
6016
6017
6018
6019

FIG. 61

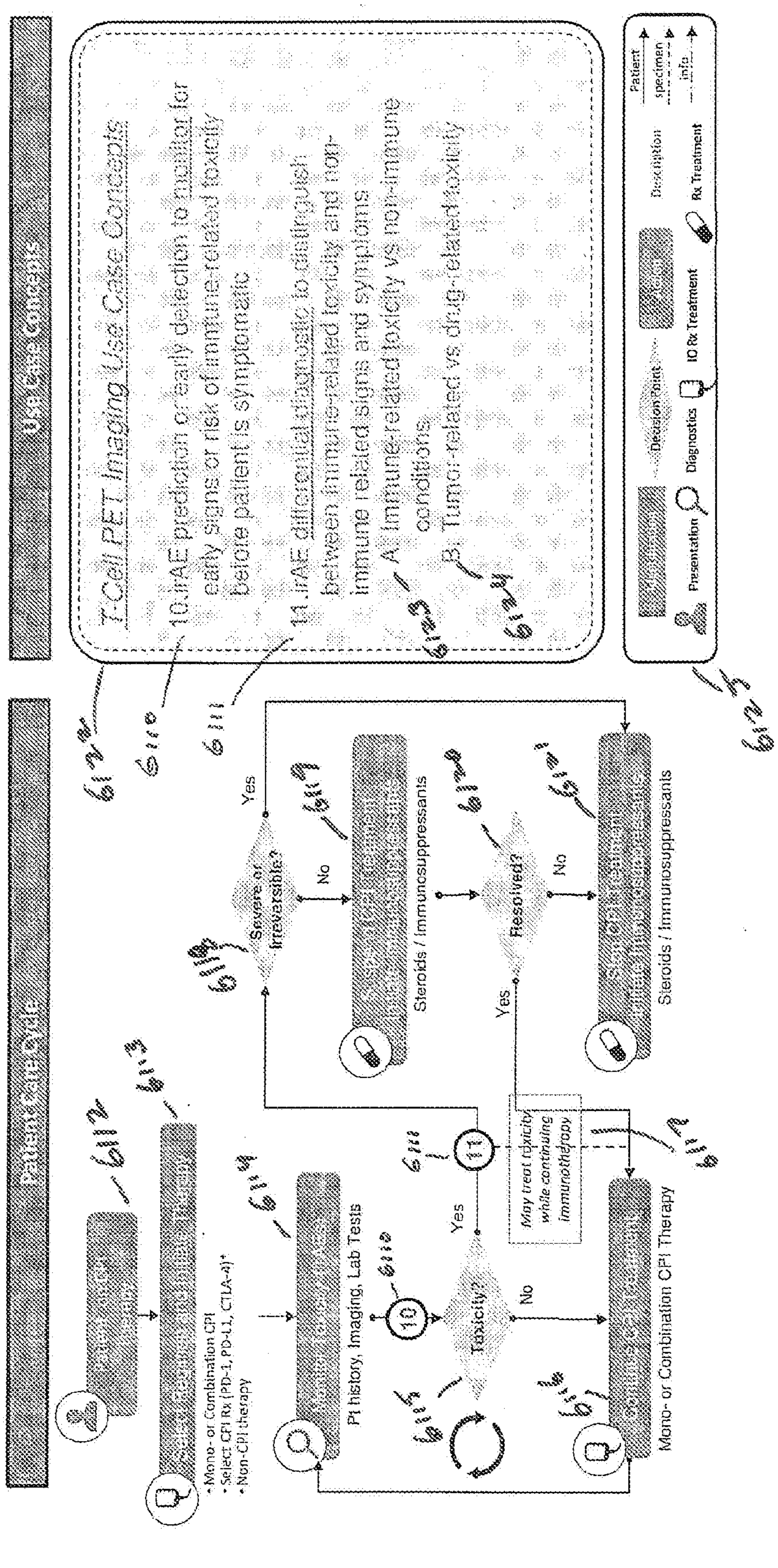
Patient Care Cycle
Mono- or Combination CPI
Select CPI Rx (PD-1, PD-L1, CTLA-4)*
Non-CPI therapy
Pt history, Imaging, Lab Tests
Toxicity?
Yes
No
Mono- or Combination CPI Therapy
May treat toxicity while continuing immunotherapy
Severe or irreversible?
Resolved?
Steroids / Immunosuppressants
Use Case Concepts
T-Cell PET Imaging Use Case Concepts
10. irAE prediction or early detection to monitor for early signs or risk of immune-related toxicity before patient is symptomatic
11. irAE differential diagnostic to distinguish between immune-related toxicity and non-immune related signs and symptoms
A. Immune-related toxicity vs non-immune conditions
B. Tumor-related vs drug-related toxicity
Presentation
Diagnostics
Decision Point
IO Rx Treatment
Rx Treatment
Description
Patient
specimen
info

FIG. 62A

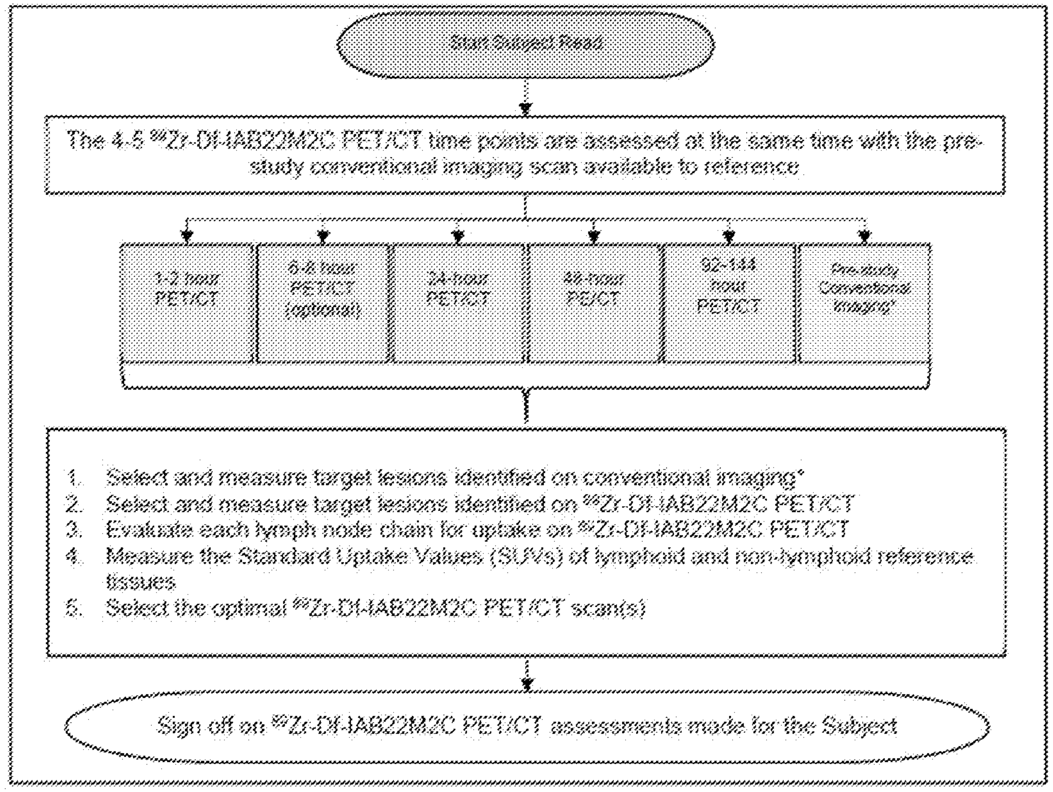


*Conventional imaging = pre-study CT, MRI, and/or 18F-FDG-PET

**FIG. 62B**

**$^{89}$Zr-Df-IAB22M2C Uptake In Reference Tissue In Stage I Subjects: Spleen**

B

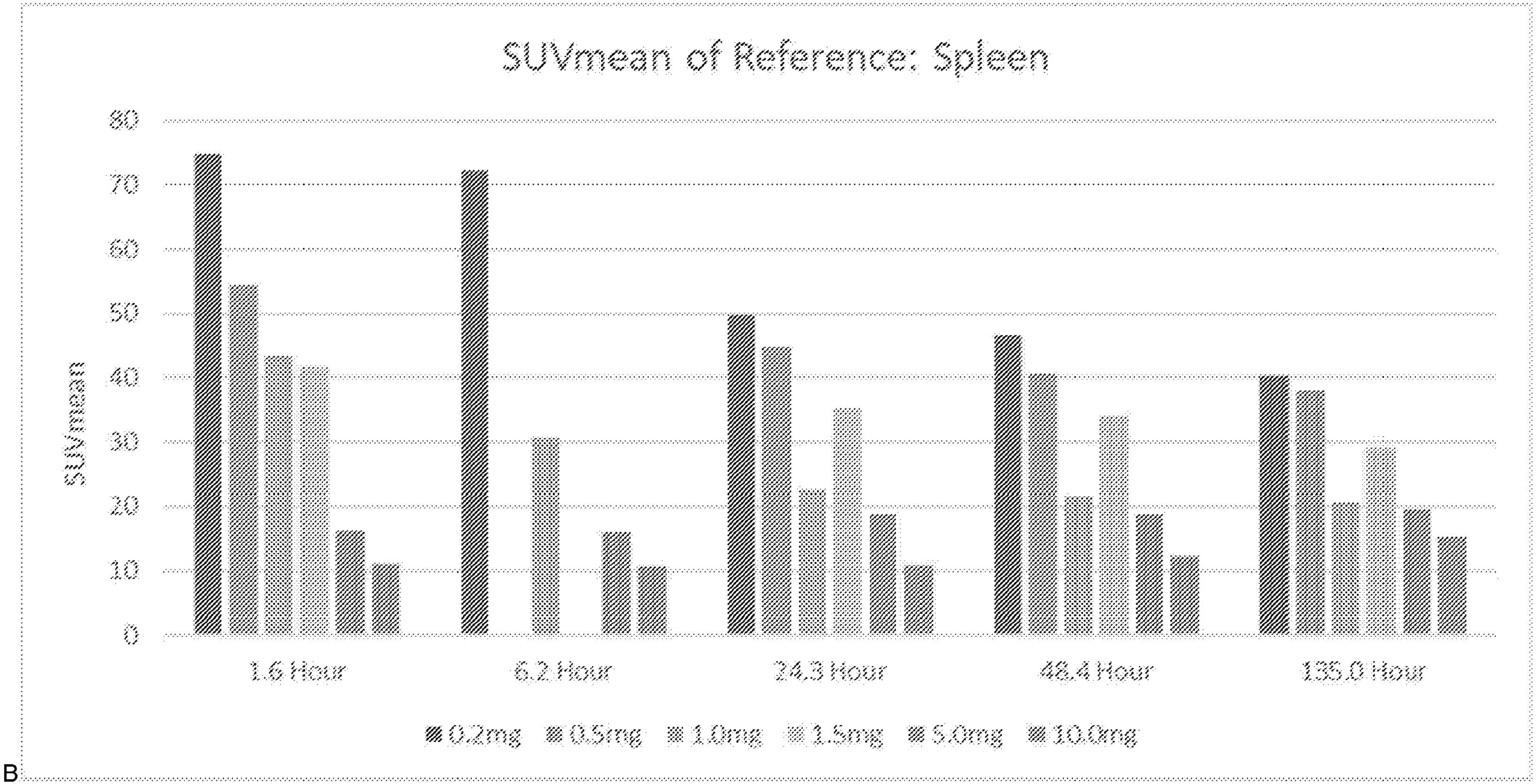

**FIG. 62C: $^{89}$Zr-Df-IAB22M2C Uptake In Reference Tissue In Stage I Subjects: Bone Marrow**

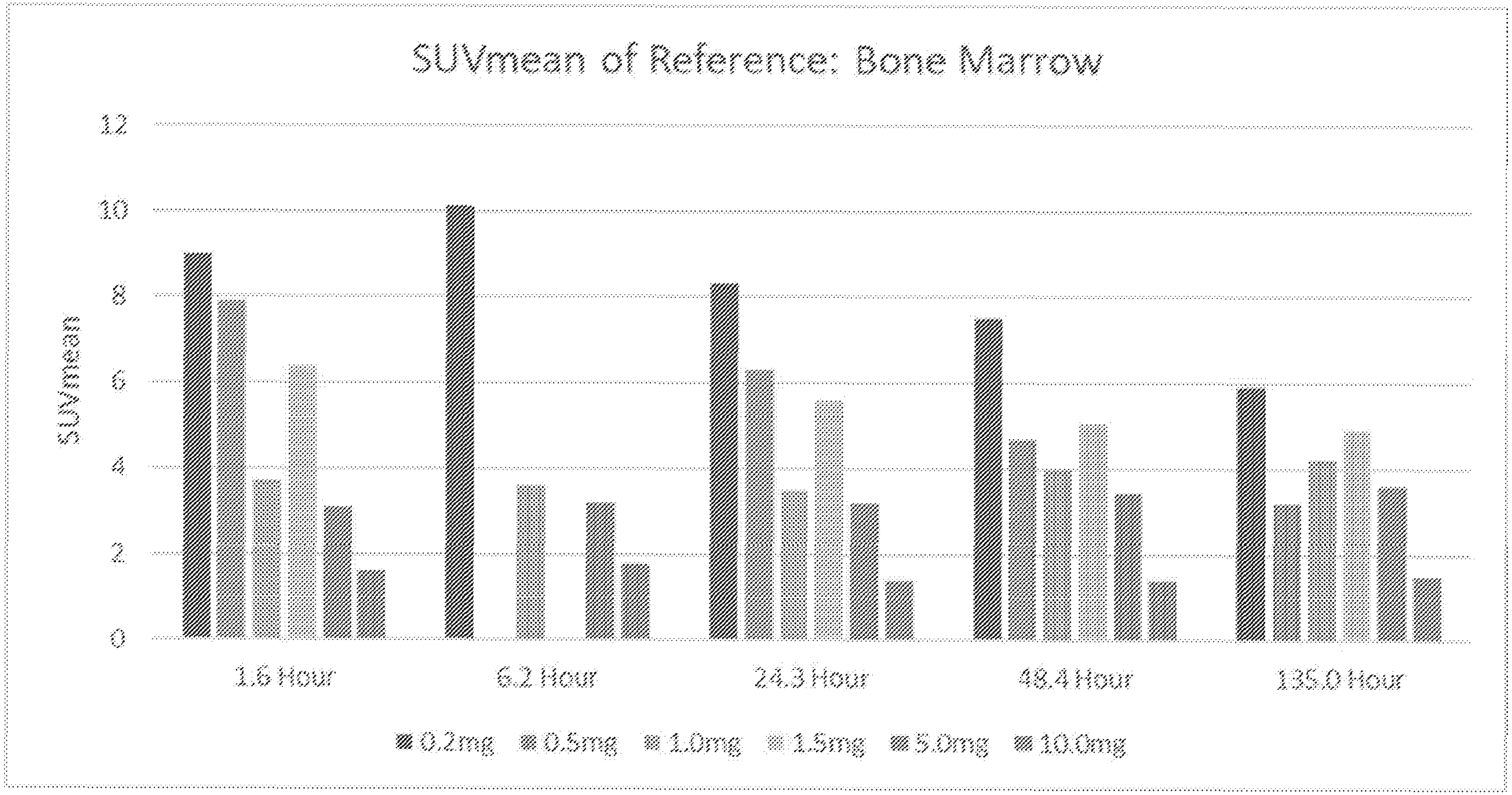

**FIG. 62D: $^{89}$Zr-Df-IAB22M2C Uptake In Reference Tissue In Stage I Subjects: Aorta**

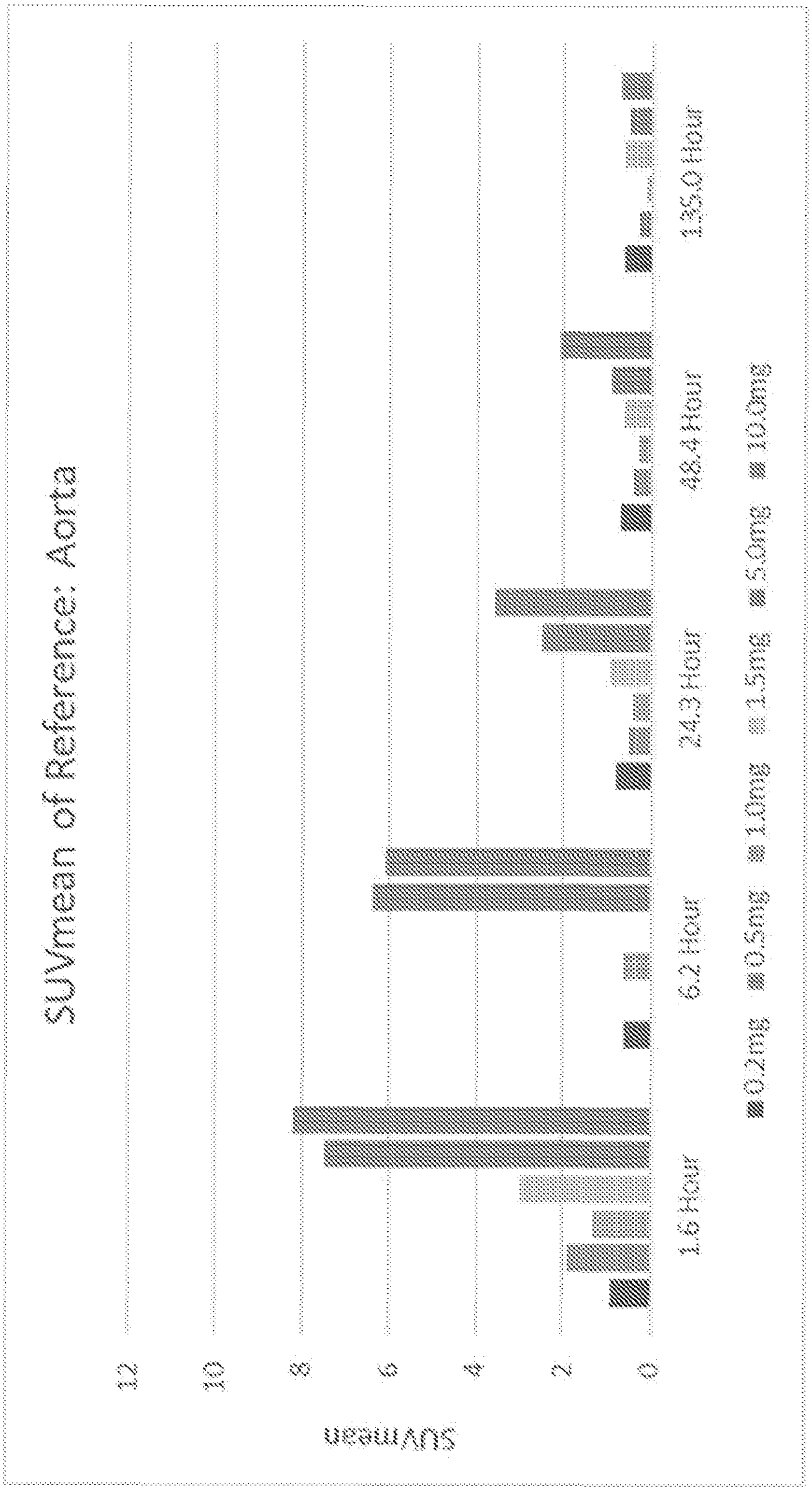

**FIG. 62E: $^{89}$Zr-Df-IAB22M2C Uptake In Reference Tissue In Stage I Subjects: Liver**

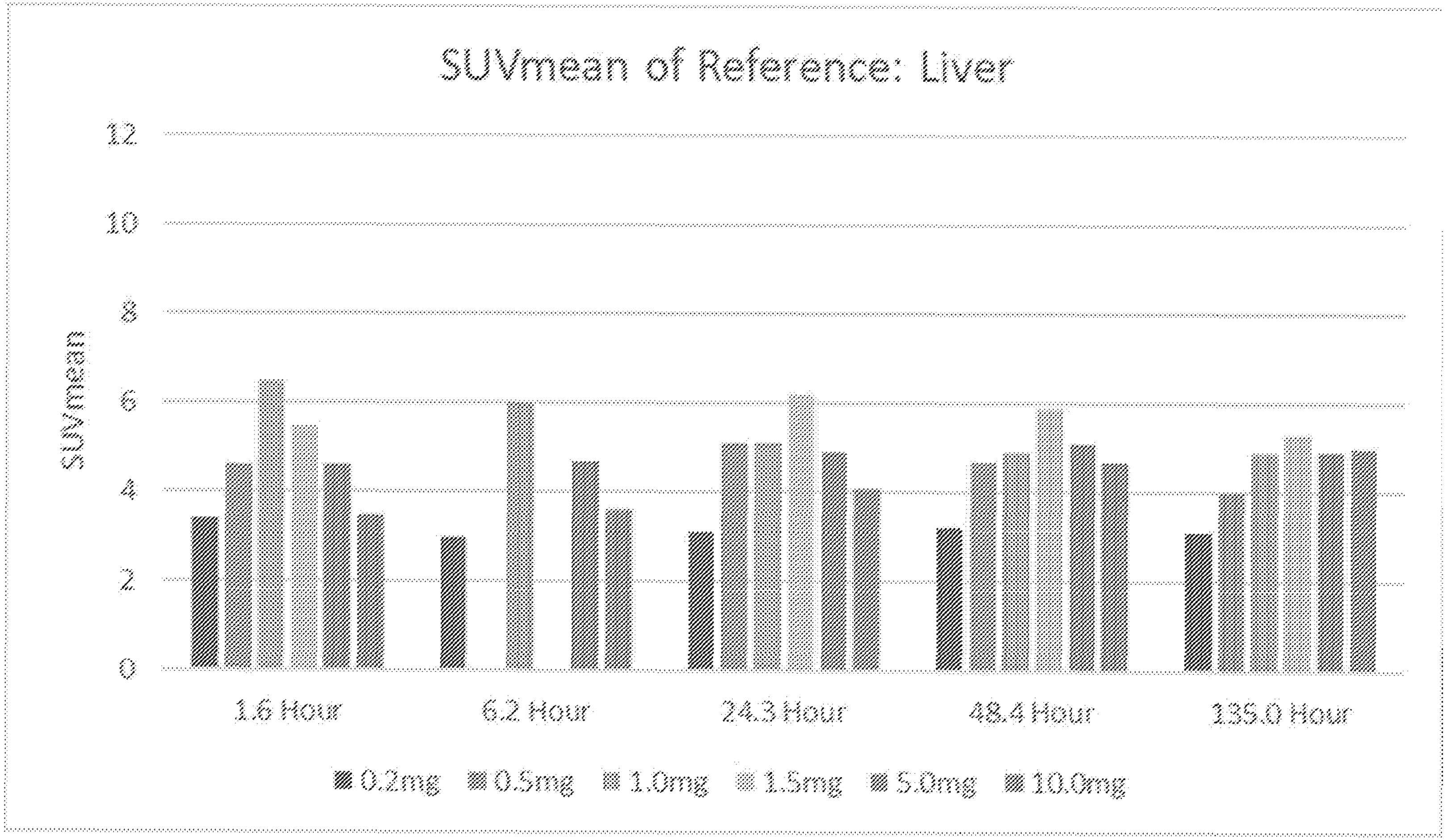

**FIG. 62F: $^{89}Zr$-Df-IAB22M2C Uptake In Reference Tissue In Stage I Subjects: Left Kidney**

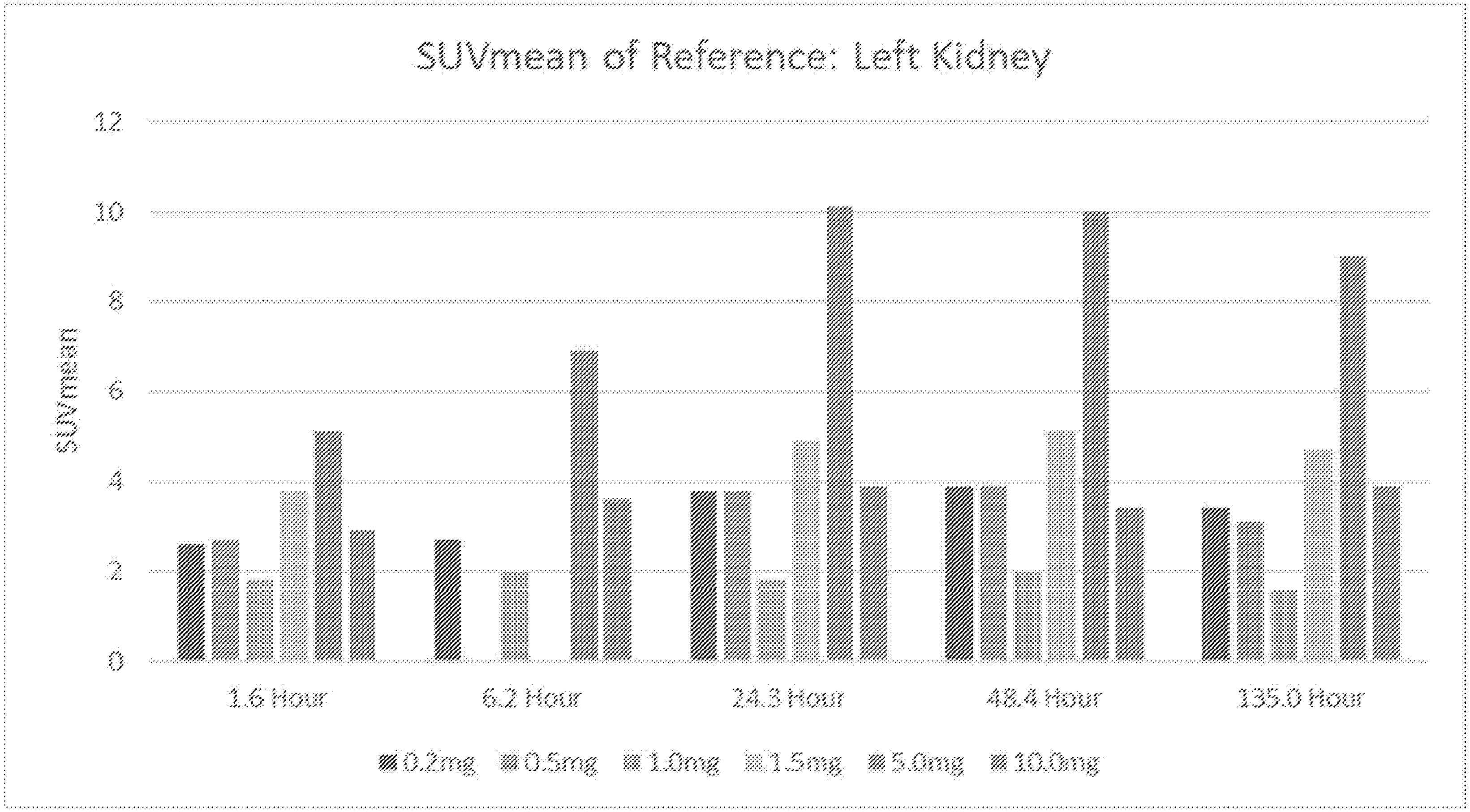

FIG. 62G: $^{89}Zr$-Df-IAB22M2C Uptake In Reference Tissue In Stage I Subjects: Lung

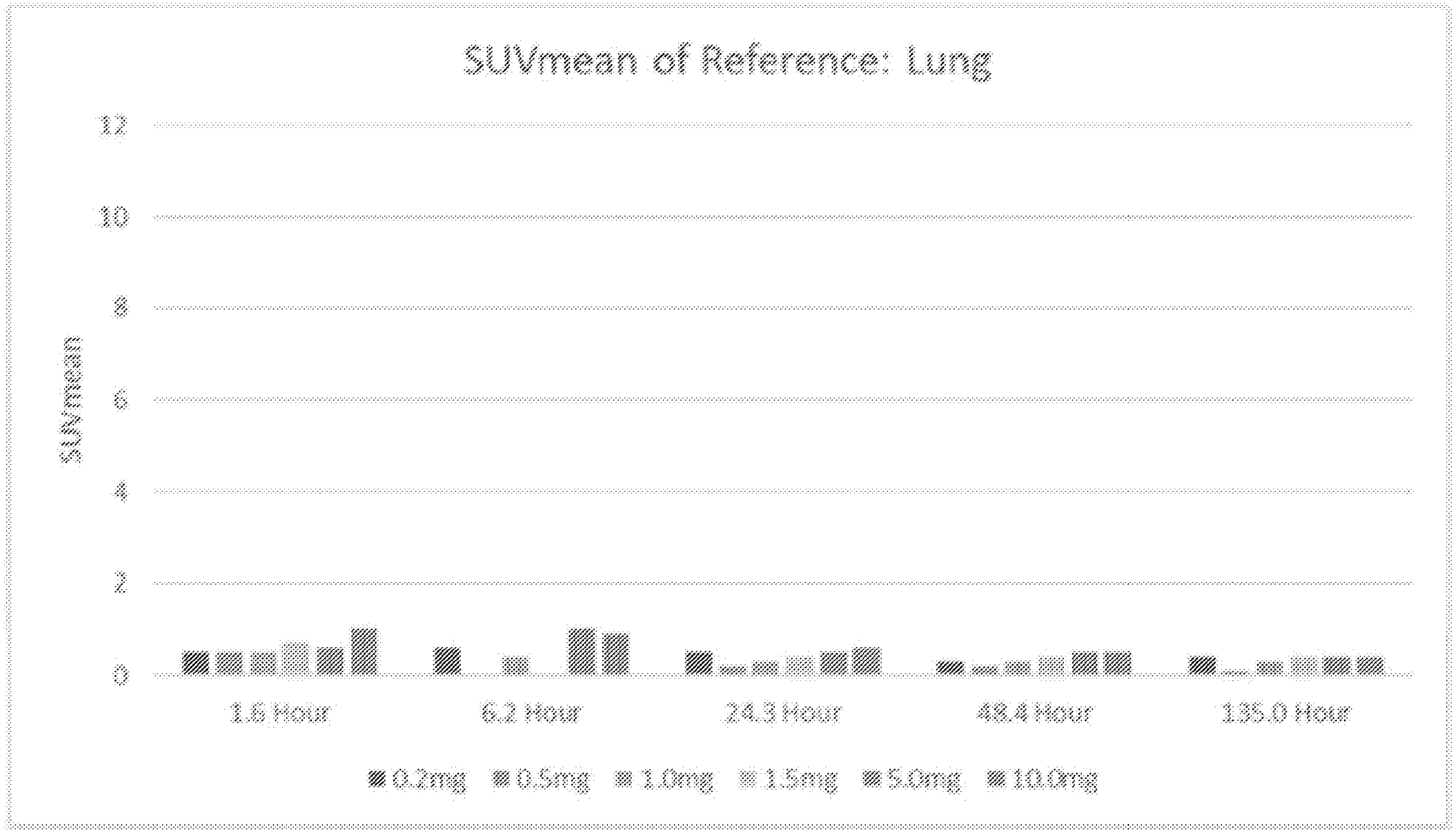

**FIG. 62H: $^{89}Zr$-Df-IAB22M2C Uptake In Reference Tissue**

**In Stage I Subjects: Muscle**

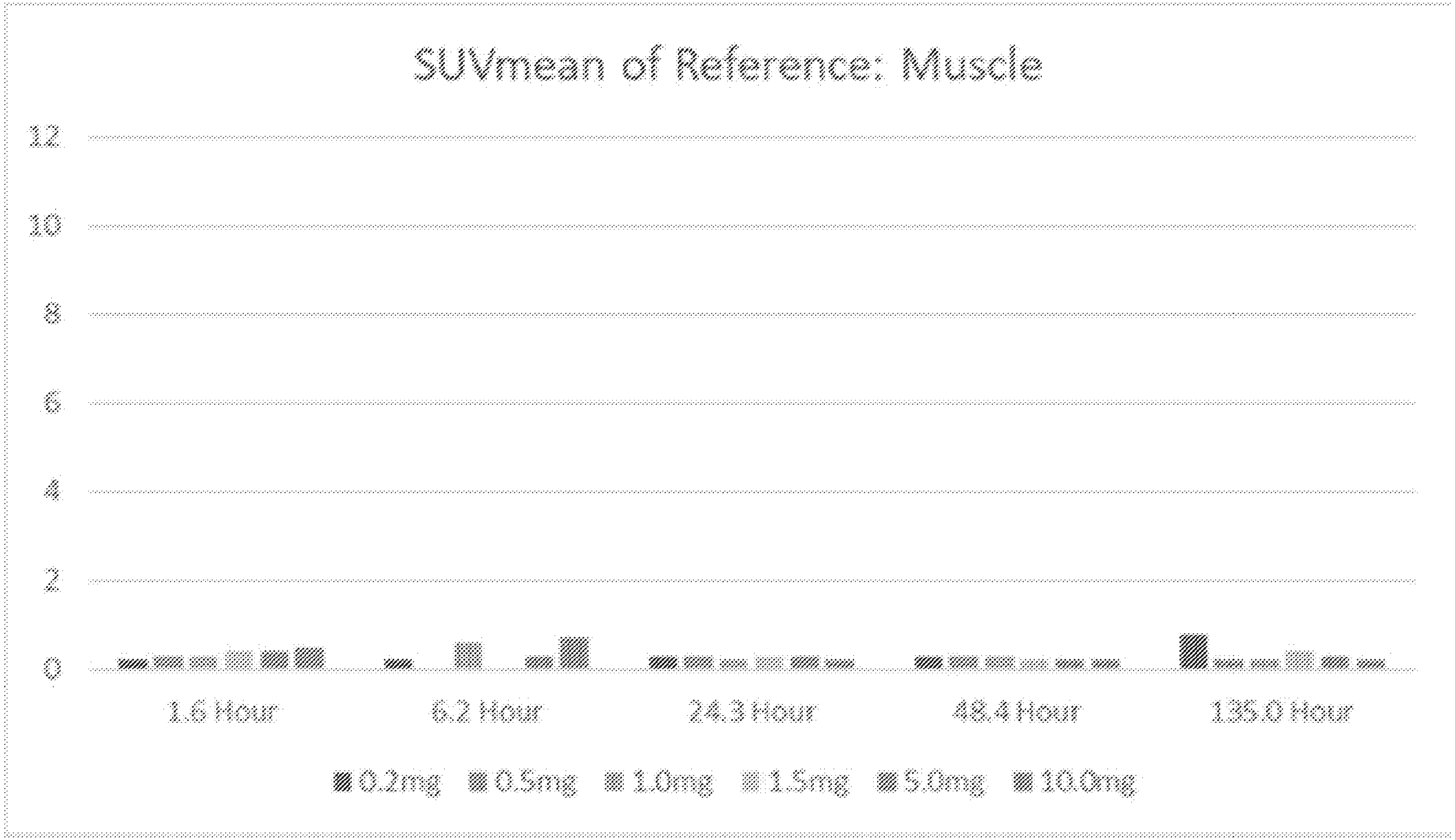

**FIG. 62I: $^{89}$Zr-Df-IAB22M2C Uptake In Cd8+ Til Rich Reference Tissue At 0.5 Mg And 1.5 Mg Active Pharmaceutical Ingredient (API)**

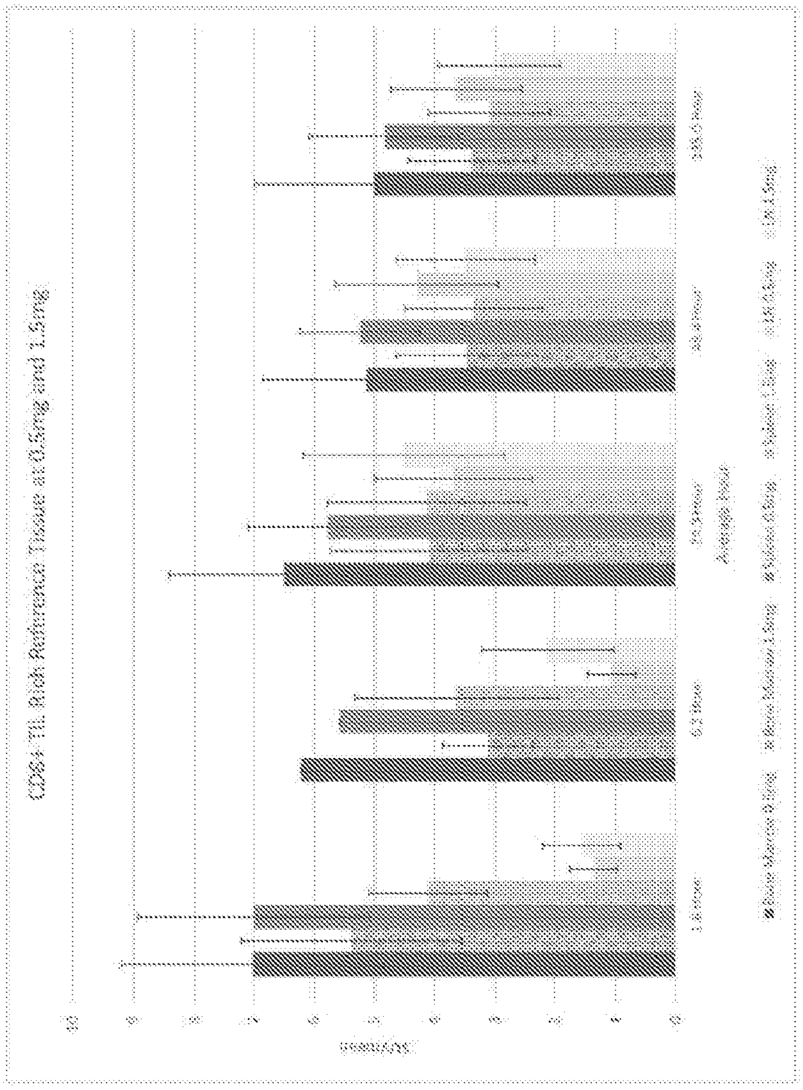

**FIG. 62J: $^{89}$Zr-Df-IAB22M2C Uptake In Non Cd8+ Rich Reference Tissue At 0.5 mg And 1.5 mg API**

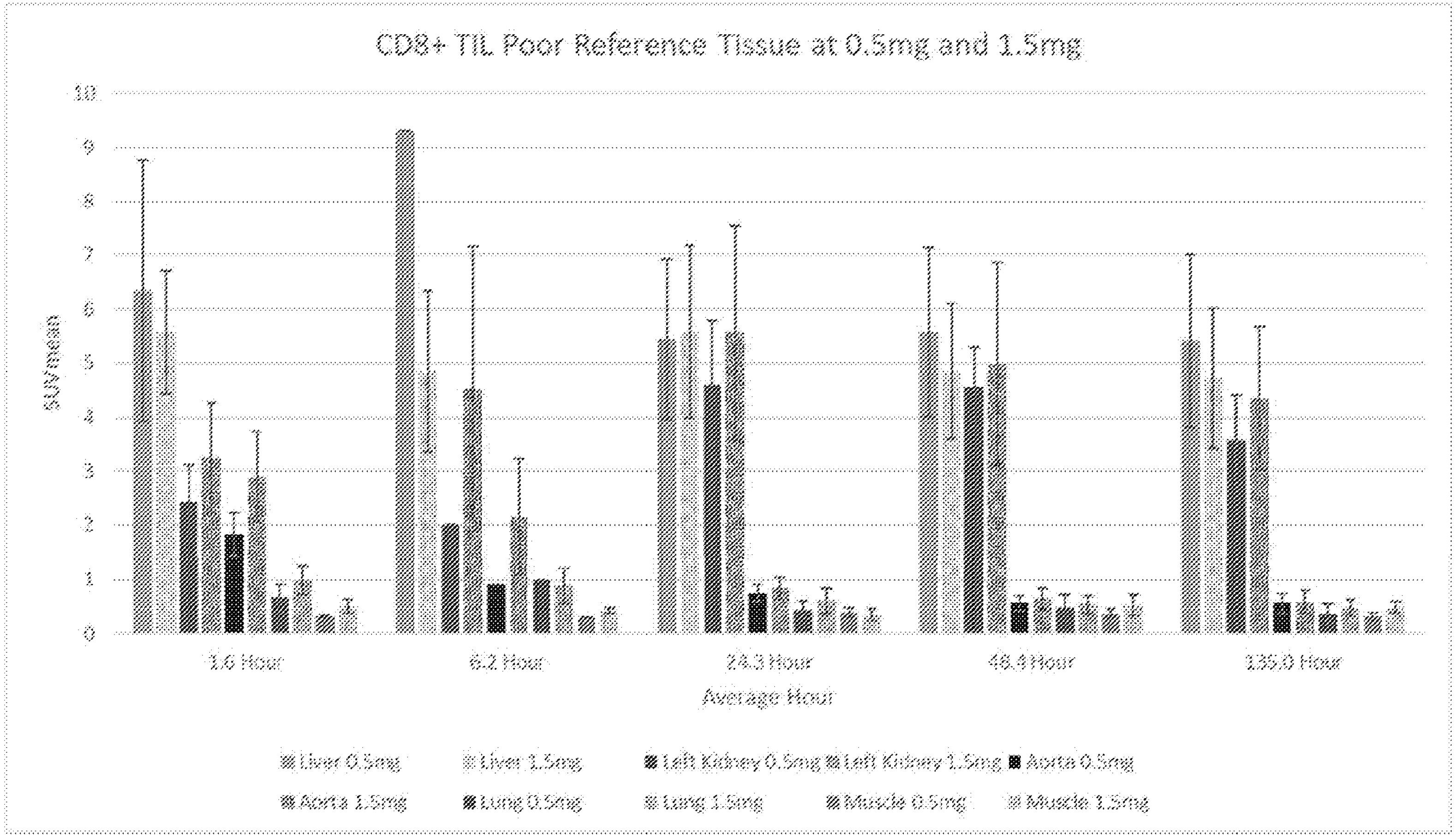

**FIG. 62K: Visualization Of $^{89}Zr$-Df-IAB22M2C Uptake In Normal Appearing Lymph Nodes By Anatomic Region**

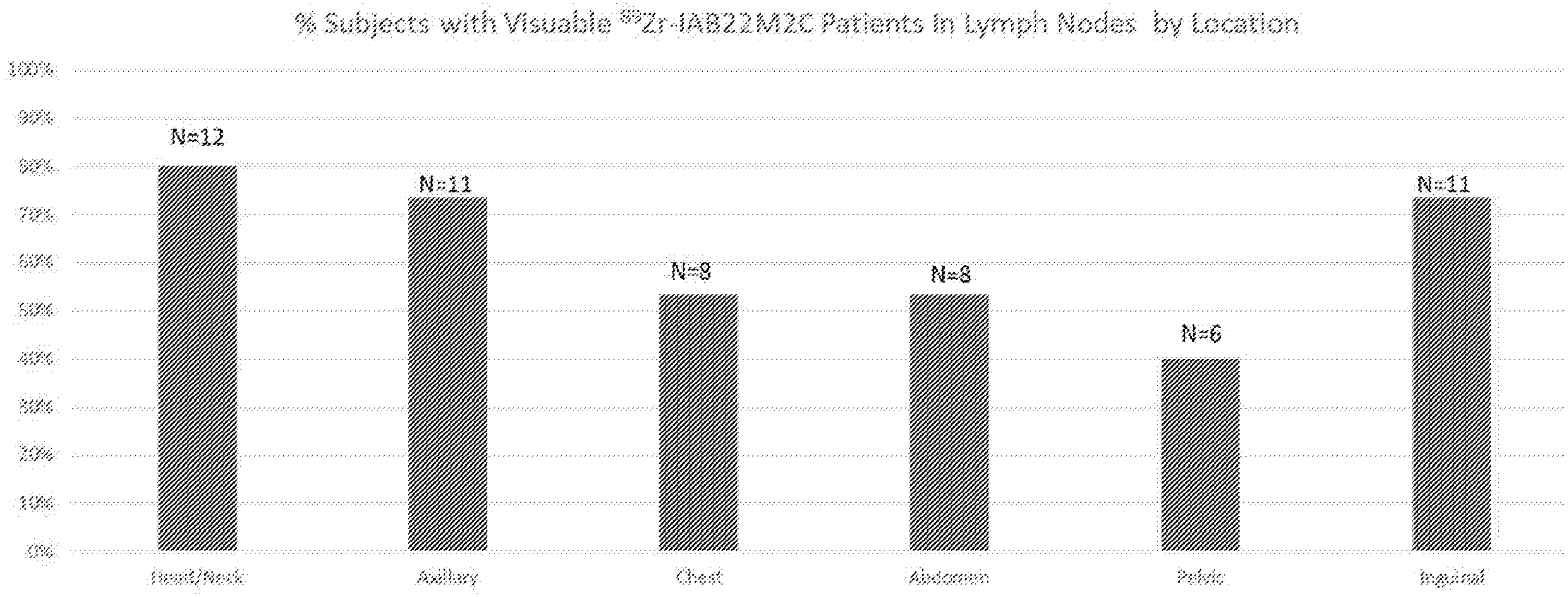

FIG. 62L Distribution of $^{89}Zr$-Df-IAB22M2C SUVmax Over Time for All Tumor Lesions

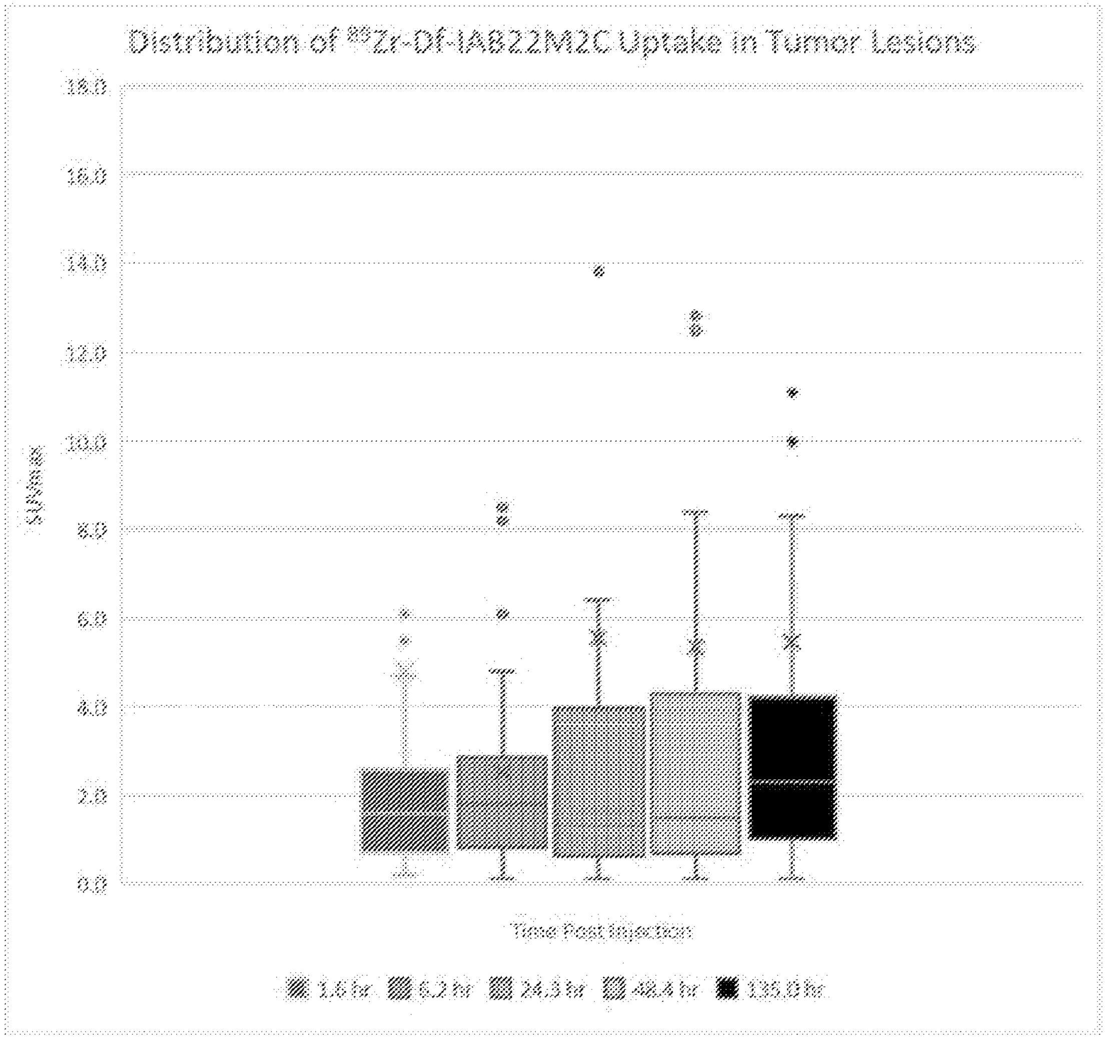


Box and Whisker Plots demonstrating $^{89}Zr$-Df-IAB22M2C uptake in tumor lesions. Box outlines the 1st and 3rd quartile values. The median SUVmax values are indicated by the horizontal line while the mean SUVmax values indicated with an X. Outlier values are indicated by dots.

FIG. 62M Distribution of $^{89}Zr$-Df-IAB22M2C SUVpeak Over Time for All Tumor Lesions

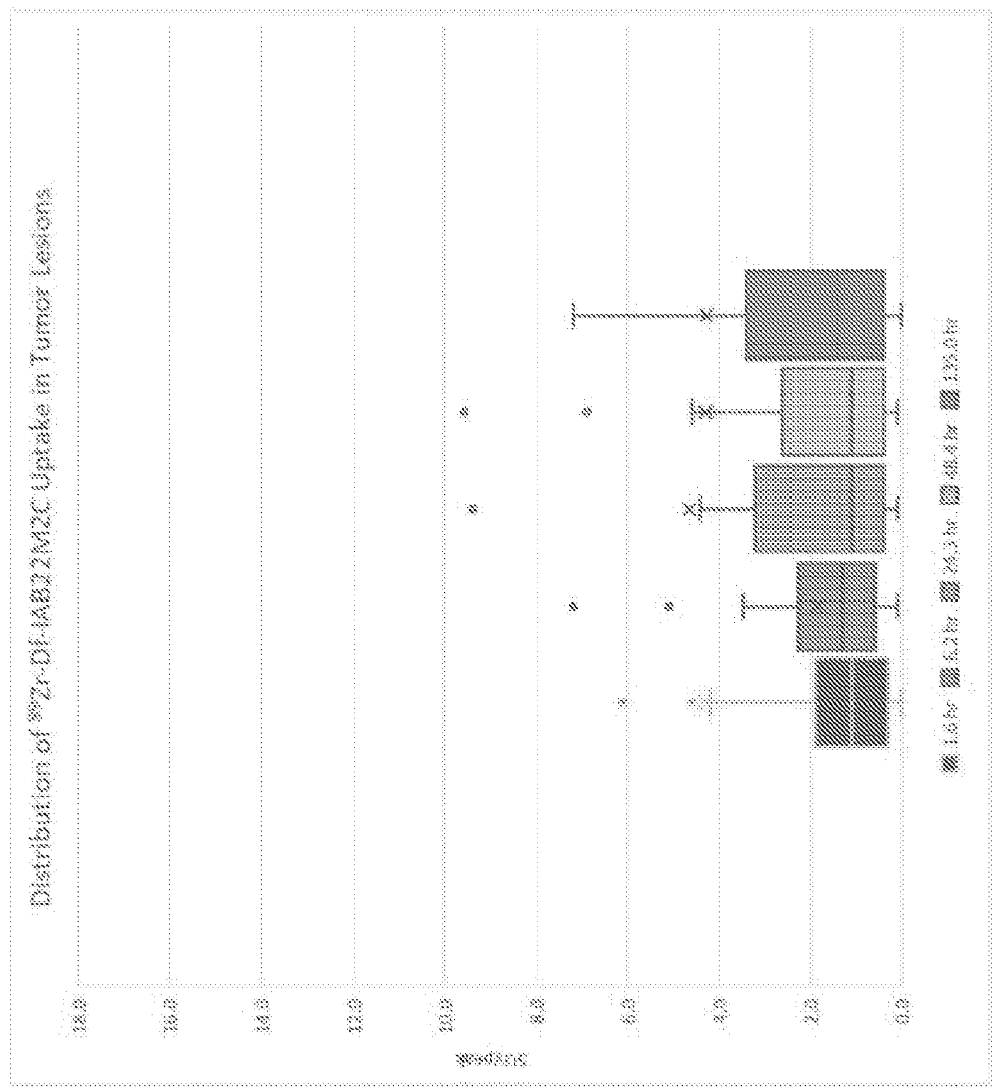


Box and Whisker Plots demonstrating $^{89}Zr$-Df-IAB22M2C uptake in tumor lesions. Box outlines the 1st and 3rd quartile values. The median SUVpeak values are indicated by the horizontal line while the mean SUVpeak values are indicated with an X. Outlier values are indicated by dots.

FIG. 62N

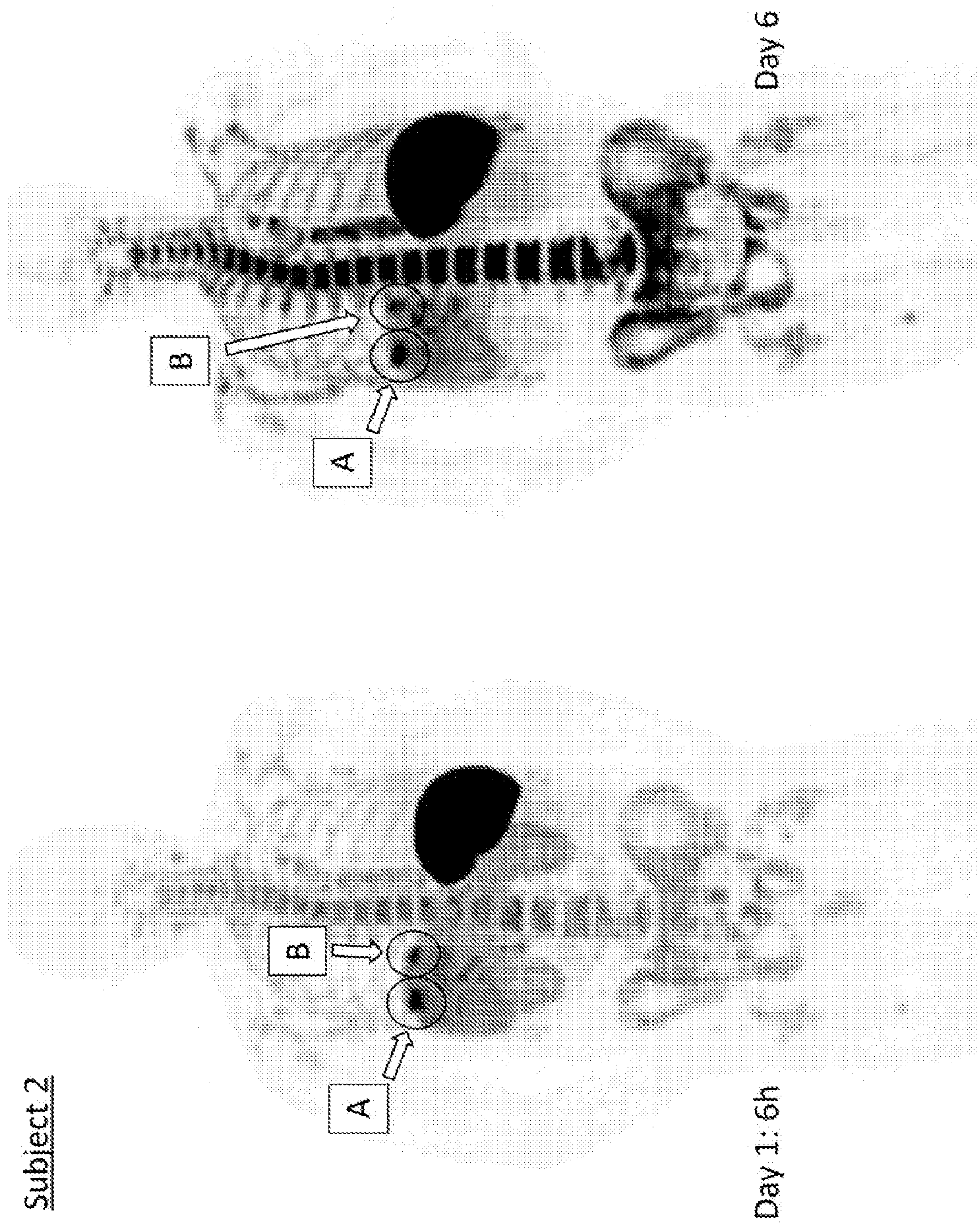

FIG. 63A

**Step By Step Reading Session Workflow**[1]

| | |
|---|---|
| Step 1 | Identify biopsied lesion(s) on biopsy CT scan |
| Step 2 | Identify the biopsied lesion(s) on the $^{89}Zr$-Df-IAB22M2C PET scan |
| Step 3 | Draw a VOI around each biopsied lesion to measure: SUVmax, SUVmean, SUVpeak<br>Assign a location to the VOI to indicate the location of the biopsied lesion |
| Step 4 | Complete a questionnaire for each lesion that includes: approximate lesion size on CTAC, SUV measurements (SUVmean) of uptake in the reference tissues by placing an appropriate size VOI on the the $^{89}Zr$-Df-IAB22M2C PET scan |

1 Note that the results of the qualitative (visual) analysis in step 4 are not included in this report.

**Figure 63B:** Correlation of IHC Measurement with SUVmax from Biopsy Samples at Baseline (A) and On-Treatment (B)

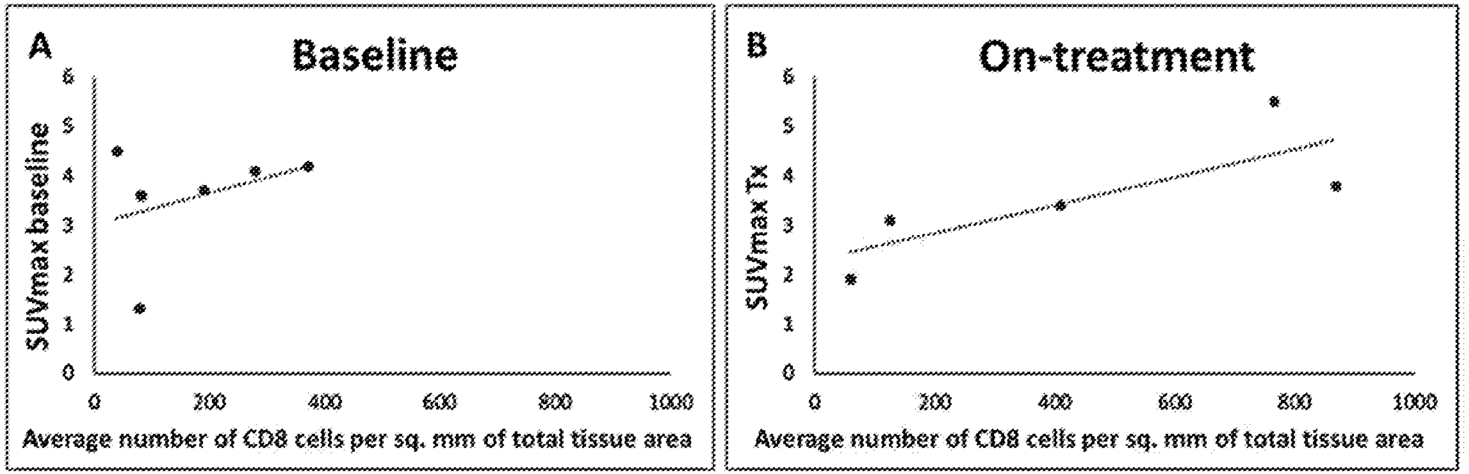

**Figure 63C:** Correlation of IHC Measurement with SUVpeak from Biopsy Samples at Baseline (A) and On-Treatment (B)

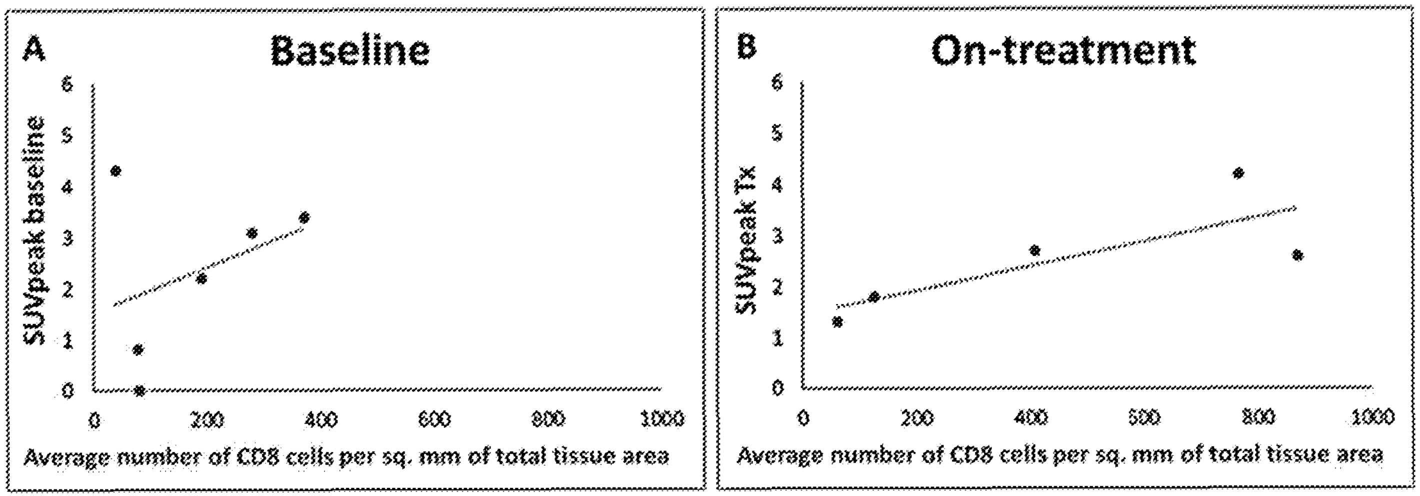

**FIG. 63D: Correlation Of IHC Measurement From Baseline And On-Treatment Combined With SUVmax (A) And SUVpeak (B) From Biopsied Tissue**

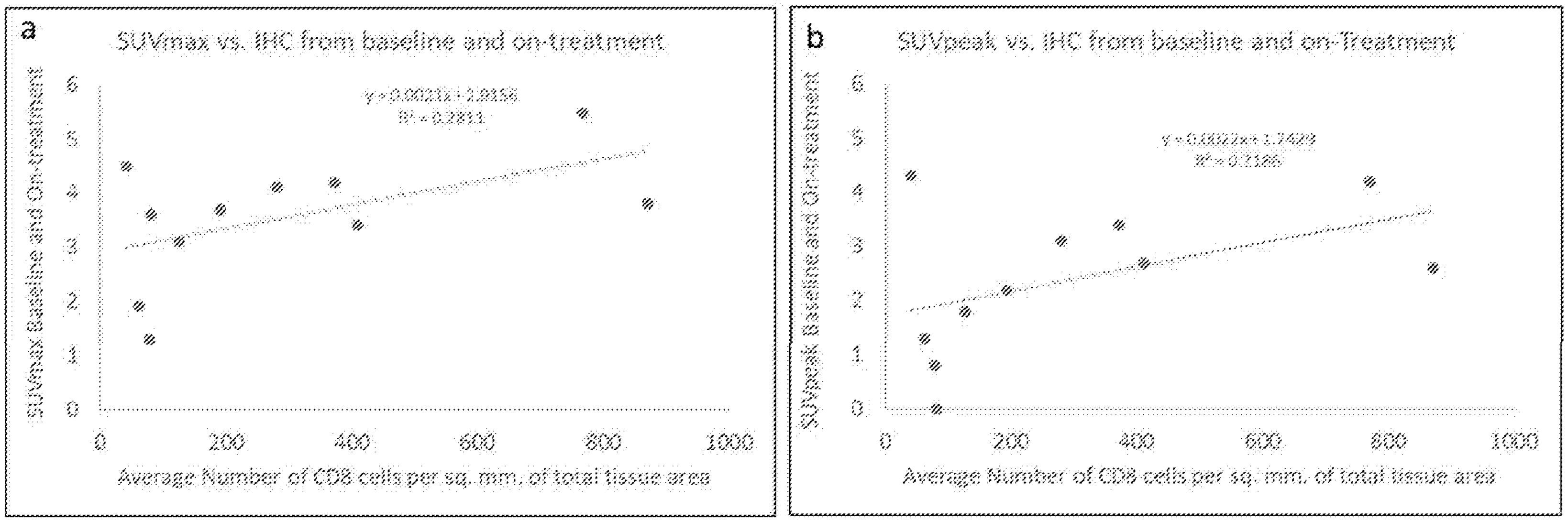

FIG. 64A
Time course: Single Dose of CD8 PET Tracer Multiple Images

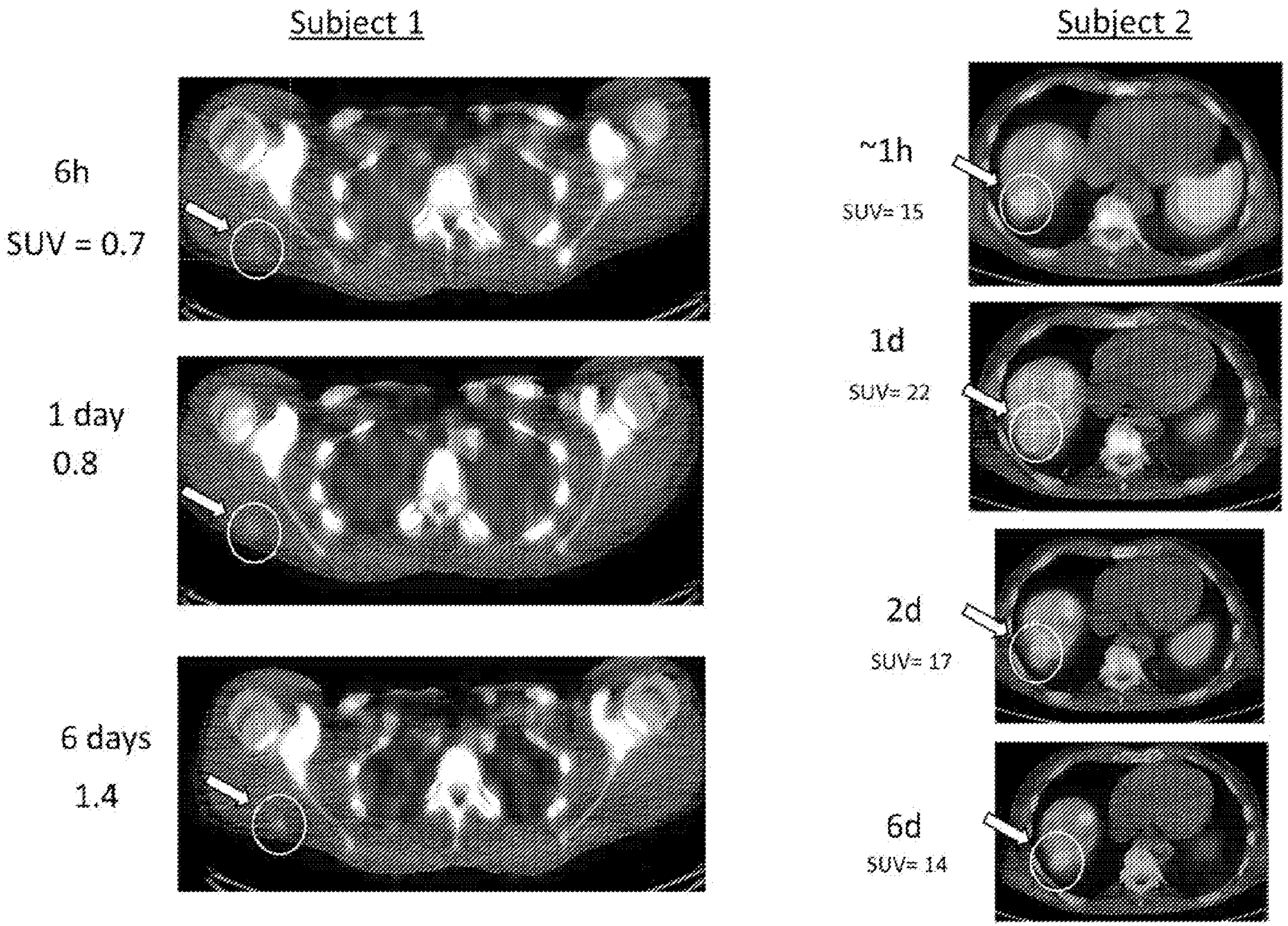

FIG. 64B

Characterization of the tumor micro environment: FDG PET identifies tumors; CD8 PET determines no CD8+ T-cell infiltration

Subject 3

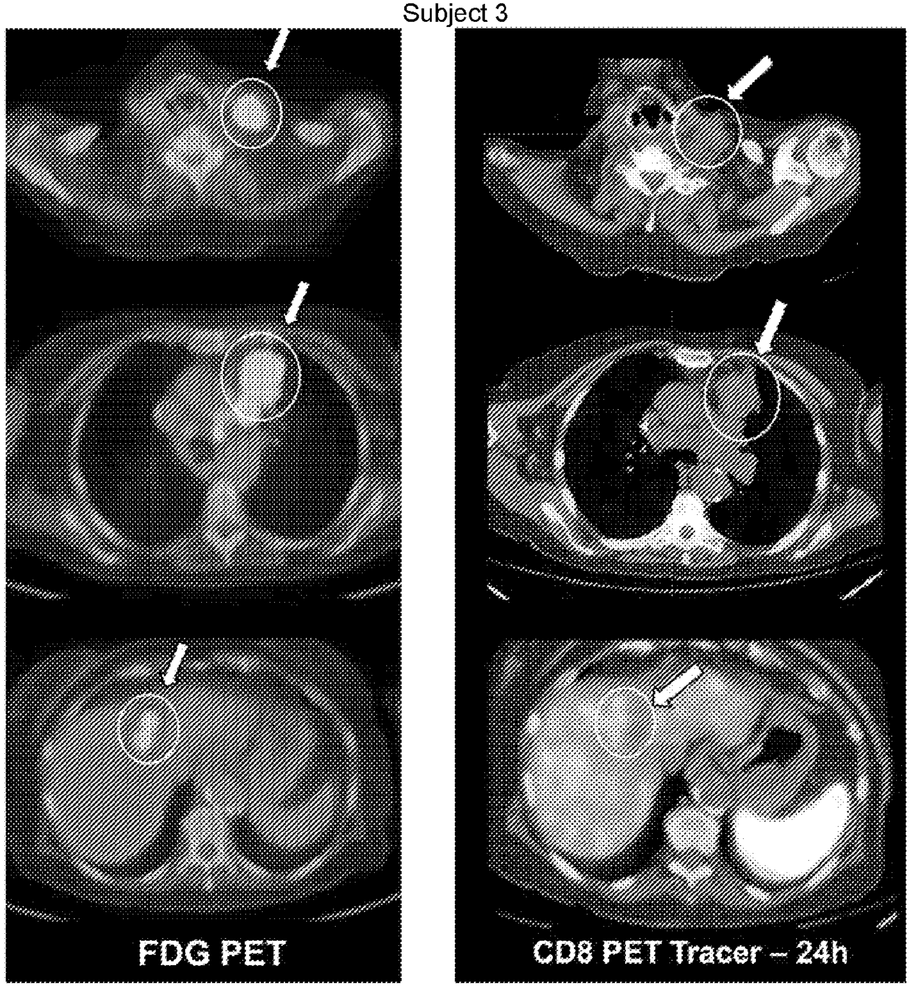

FIG. 64C
CHARACTERIZATION OF THE TUMOR MICROENVIRONMENT

CD8 PET enhances diagnostic understanding of FDG and CT scans

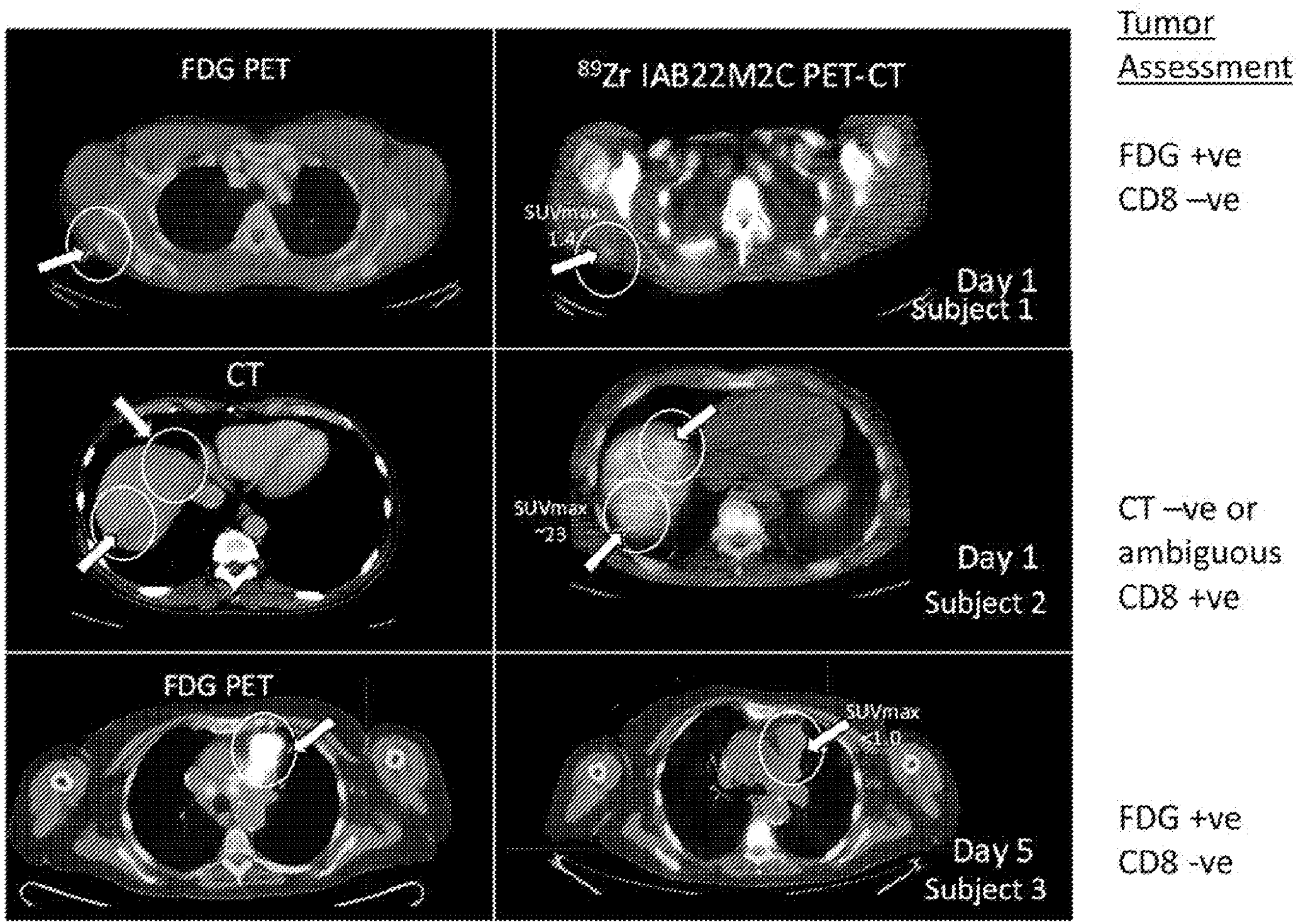

FIG. 64D

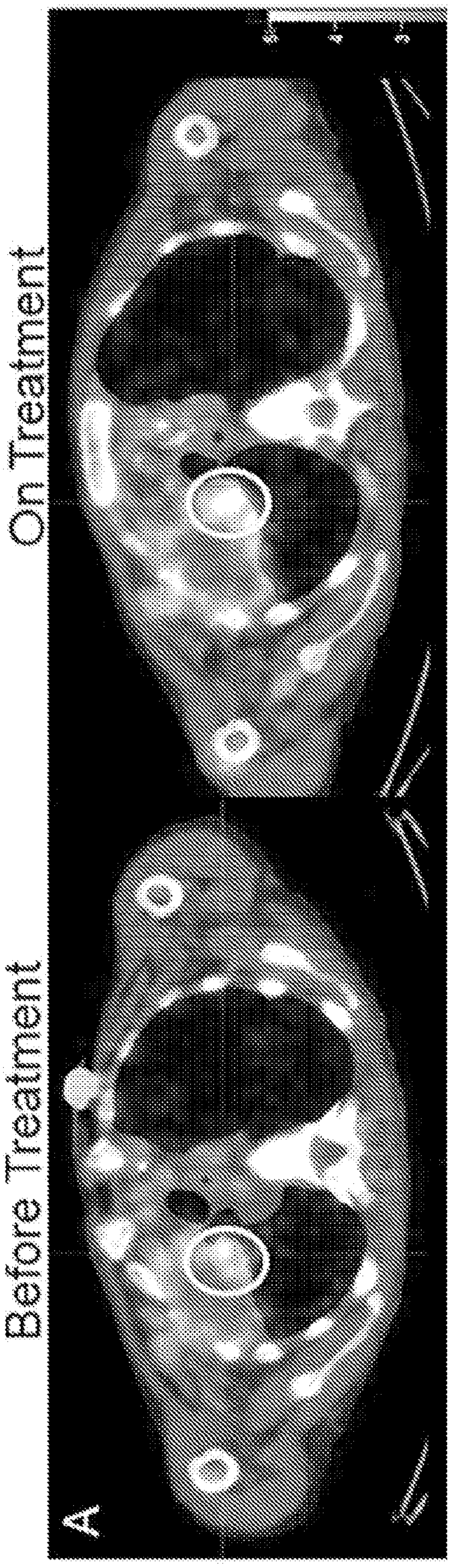

FIG. 64E

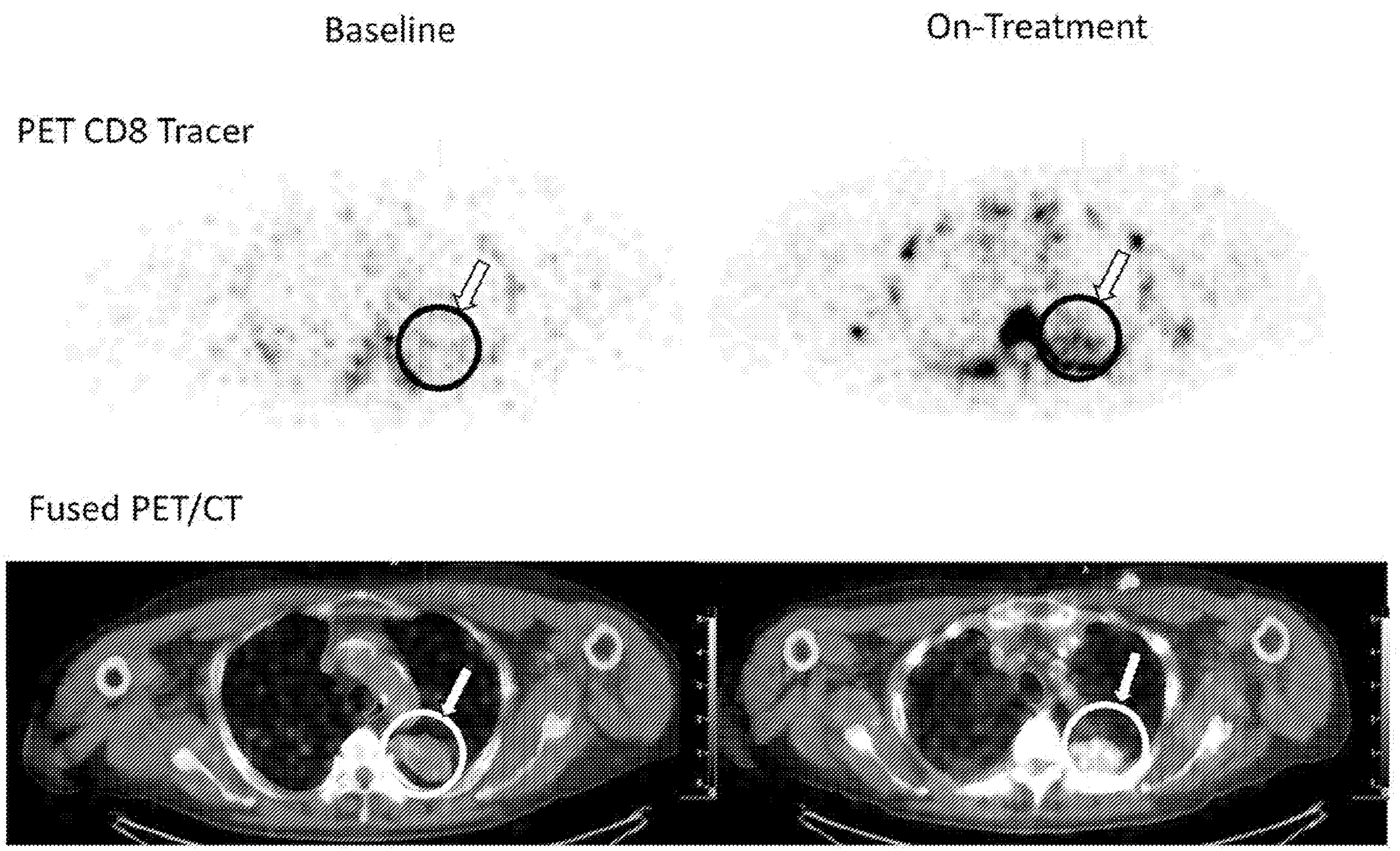

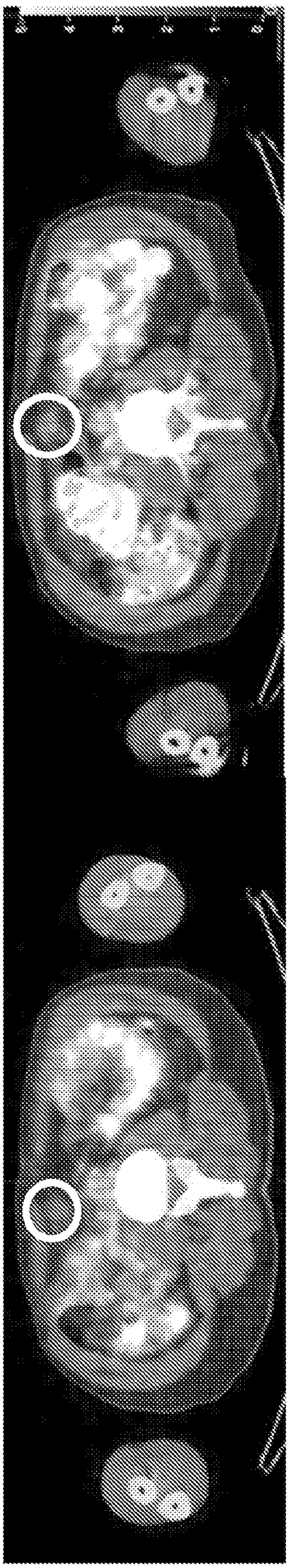

FIG. 64F

Europäisches Patentamt
European Patent Office
Office européen des brevets

# EUROPEAN SEARCH REPORT

Application Number
EP 22 18 2384

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PANDIT-TASKAR NEETA ET AL: "First in Human Phase I Imaging Study With 89Zr-IAB22M2C Anti CD8 Minibody in Patients with Solid Tumors", JOURNAL OF NUCLEAR MEDICINE, vol. 59, no. Suppl 1, 1 May 2018 (2018-05-01), page 596, XP055928037, * the whole document * ----- | 1-15 | INV. A61K51/10 C07K16/28 G01N33/566 G01N33/68 |
| Y | JAUW YVONNE W. S. ET AL: "Immuno-Positron Emission Tomography with Zirconium-89-Labeled Monoclonal Antibodies in Oncology: What Can We Learn from Initial Clinical Trials?", FRONTIERS IN PHARMACOLOGY, vol. 7, 24 May 2016 (2016-05-24), XP055928022, CH ISSN: 1663-9812, DOI: 10.3389/fphar.2016.00131 * e.g. 89Zr-trastuzumab-PET, 89Zr-bevacizumab-PET, 89Zr-fresolimumab-PET, 89Zr-cetuximab-PET * ----- | 13,14 | TECHNICAL FIELDS SEARCHED (IPC) A61K G01N |
| Y | R. TAVARE ET AL: "Immuno-PET of Murine T Cell Reconstitution Postadoptive Stem Cell Transplantation Using Anti-CD4 and Anti-CD8 Cys-Diabodies", THE JOURNAL OF NUCLEAR MEDICINE, vol. 56, no. 8, 7 May 2015 (2015-05-07), pages 1258-1264, XP055514282, US ISSN: 0161-5505, DOI: 10.2967/jnumed.114.153338 * wole document, in particular abstract/conclusions * ----- -/-- | 1-15 | |

The present search report has been drawn up for all claims

| Place of search | Date of completion of the search | Examiner |
|---|---|---|
| The Hague | 26 May 2023 | Schleifenbaum, A |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 22 18 2384

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ADIL I. DAUD ET AL: "Immuno-PET of Murine T Cell Reconstitution Postadoptive Stem Cell Transplantation Using Anti-CD4 and Anti-CD8 Cys-Diabodies", THE JOURNAL OF CLINICAL INVESTIGATION, vol. 126, no. 9, 1 September 2016 (2016-09-01), pages 3447-3452, XP055585410, GB ISSN: 0021-9738, DOI: 10.1172/JCI87324 * wole document, in particular abstract/conclusions * ----- | 1-15 | |
| Y | URYVAEV ANTON ET AL: "The role of tumor-infiltrating lymphocytes (TILs) as a predictive biomarker of response to anti-PD1 therapy in patients with metastatic non-small cell lung cancer or metastatic melanoma", MEDICAL ONCOLOGY, SCIENCE AND TECHNOLOGY LETTERS, NORTHWOOD, GB, vol. 35, no. 3, 31 January 2018 (2018-01-31), pages 1-9, XP036449915, ISSN: 1357-0560, DOI: 10.1007/S12032-018-1080-0 [retrieved on 2018-01-31] * wole document, in particular abstract * ----- | 1-15 | TECHNICAL FIELDS SEARCHED (IPC) |
| Y | S HAN ET AL: "Tumour-infiltrating CD4+ and CD8+ lymphocytes as predictors of clinical outcome in glioma", BRITISH JOURNAL OF CANCER, vol. 110, no. 10, 1 April 2014 (2014-04-01), pages 2560-2568, XP055360649, London ISSN: 0007-0920, DOI: 10.1038/bjc.2014.162 * the whole document * ----- | 1-15 | |

The present search report has been drawn up for all claims

2

| Place of search | Date of completion of the search | Examiner |
|---|---|---|
| The Hague | 26 May 2023 | Schleifenbaum, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62738938 W
- US 62826606
- US 20160024209 A
- WO 2017176769 A
- WO 2012106671 A
- WO 2013033561 A
- WO 2013009869 A
- WO 2014074907 A
- US 4946778 A
- US 4816567 A
- US 20170357015 A
- US 20170153337 A
- US 20150196266 A
- US 20150087974 A
- US 20120318988 A
- US 20090159804 A
- US 5342606 A
- US 5428155 A
- US 5316757 A
- US 5480990 A
- US 5462725 A
- US 5428139 A
- US 5385893 A
- US 5739294 A
- US 5750660 A
- US 5834456 A
- US 4275149 A
- US 4737456 A
- US 6528624 B


### Non-patent literature cited in the description


- *IGNAB047WOSEQLIST.txt,* 27 September 2019
- The Science and Practice of Pharmacy. Univ. of Sciences in Philadelphia (USIP), Lippincott Williams & Wilkins, 2005
- **WU, T. T. ; E. A. KABAT.** An analysis of the sequences of the variable regions of Bence Jones proteins and myeloma light chains and their implications for antibody complementarity. *J. Exp. Med.,* 1970, vol. 132, 211-250
- **KABAT, E. A. ; WU, T. T. ; PERRY, H. ; GOTTESMAN, K. ; FOELLER, C.** Sequences of Proteins of Immunological Interest. 1991
- **CHOTHIA ; LESK.** *J. Mol. Biol.,* 1987, vol. 196, 901-917
- **CHOTHIA et al.** *Nature,* 1989, vol. 342, 877-883
- **CHOTHIA et al.** *J. Mol. Biol.,* 1992, vol. 227, 799-817
- **AL-LAZIKANI et al.** *J. Mol. Biol.,* 1997, vol. 273, 927-748
- **GIUDICELLI, V. ; DUROUX, P. ; GINESTOUX, C. ; FOLCH, G. ; JABADO-MICHALOUD, J. ; CHAUME, D. ; LEFRANC, M.-P.** *IMGT/LIGM-DB, the IMGT® comprehensive database of immunoglobulin and T cell receptor nucleotide sequences Nucl. Acids Res.,* 2006, vol. 34, D781-D784
- **LEFRANC, M.-P. ; POMMIÉ, C. ; RUIZ, M. ; GIUDICELLI, V. ; FOULQUIER, E. ; TRUONG, L. ; THOUVENIN-CONTET, V. ; LEFRANC, G.** IMGT unique numbering for immunoglobulin and T cell receptor variable domains and Ig superfamily V-like domains Dev. *Comp. Immunol.,* 2003, vol. 27, 55-77
- **BROCHET, X. ; LEFRANC, M.-P. ; GIUDICELLI, V.** IMGT/V-QUEST: the highly customized and integrated system for IG and TR standardized V-J and V-D-J sequence analysis. *Nucl. Acids Res,* 2008, vol. 36, W503-508
- **MARTIN et al.** *Proc. Natl. Acad. Sci. USA,* 1989, vol. 86, 9268-9272
- **MACCALLUM et al.** *J. Mol. Biol.,* 1996, vol. 262, 732-745
- **MCCAFFERTY et al.** *Nature,* 1990, vol. 348, 552-554
- **KOHLER ; MILSTEIN.** *Nature,* 1975, vol. 256, 495-497
- **KOZBOR et al.** *Immunology Today,* 1983, vol. 4, 72
- **COLE et al.** Monoclonal Antibodies and Cancer Therapy. Alan R. Liss, Inc, 1985, 77-96
- **SHIXIA WANG.** dvances in the production of human monoclonal antibodies. *Antibody Technology Journal,* 2011, vol. 1, 1-4
- *J Cell Biochem.,* 01 October 2005, vol. 96 (2), 305-13

- **SHARON J ; LIEBMAN MA ; WILLIAMS BR.** Recombinant polyclonal antibodies for cancer therapy. *Drug Discov Today,* July 2006, vol. 11 (13-14), 655-60
- **MARKS et al.** *Biotechnology,* 1992, vol. 10, 779-783
- **JONES et al.** *Nature,* 1986, vol. 321, 522-525
- **RIECHMANN et al.** *Nature,* 1988, vol. 332, 323-327
- **VERHOEYEN et al.** *Science,* 1988, vol. 239, 1534-1536
- **PRESTA.** *Curr. Op. Struct. Biol.,* 1992, vol. 2, 593-596
- **HARLOW ; LANE.** Using Antibodies, A Laboratory Manual. 1998
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **CREIGHTON.** *Proteins,* 1984
- **SMITH ; WATERMAN.** *Adv. Appl. Math.,* 1970, vol. 2, 482c
- **NEEDLEMAN ; WUNSCH.** *J. Mol. Biol.,* 1970, vol. 48, 443
- **PEARSON ; LIPMAN.** *Proc. Nat'1. Acad. Sci. USA,* 1988, vol. 85, 2444
- **AUSUBEL et al.** *Current Protocols in Molecular Biology,* (1995
- **ALTSCHUL et al.** *Nuc. Acids Res.,* 1977, vol. 25, 3389-3402
- **ALTSCHUL et al.** *J. Mol. Biol.,* 1990, vol. 215, 403-410
- **HENIKOFF ; HENIKOFF.** *Proc. Natl. Acad. Sci. USA,* 1989, vol. 89, 10915
- **KARLIN ; ALTSCHUL.** *Proc. Natl. Acad. Sci. USA,* 1993, vol. 90, 5873-5787
- *Agnew, Chem Intl. Ed. Engl.,* 1994, vol. 33, 183-186
- Cell cycle regulation, oncogenes, and antineoplastic drugs. **MURAKAMI et al.** The Molecular Basis of Cancer, Mendelsohn and Israel. WB Saunders, 1995
- **KERR ; CHISOLM.** *The Journal of Immunology,* 2019, vol. 202, 11-19
- **GALON ; BRUNI.** *Nature Reviews Drug Discovery,* 2019, vol. 18, 197-218
- **WOLCHOK JD ; HOOS A ; O'DAY S et al.** Guidelines for the Evaluation of Immune Therapy Activity in Solid Tumors: Immune-Related Response Criteria. *Clin Cancer Res.,* 2009, vol. 15, 7412-7420
- **CHIOU VL ; BUROTTO M.** Pseudoprogression and Immune-Related Response in Solid Tumors. *J Clin Oncol.,* 2015, vol. 33, 3541-3543
- **HALES RK ; BANCHEREAU J ; RIBAS A et al.** Assessing oncologic benefit in clinical trials of immunotherapy agents. *Ann Oncol,* 2010, vol. 21, 1944-1951
- **ROSSELLA GALATI ; ALESSANDRA VERDINA ; GIULIANA FALASCA ; ALBERTO CHERSI.** *Z. Naturforsch,* 2003, vol. 58c, 558-561
- **SHIVELY et al.** *J Nucl Med,* 2007, vol. 48, 170-2
- **SINGH et al.** *Anal. Biochem.,* 2002, vol. 304, 147-15
- **HARLOW E. ; LANE, D.** Using Antibodies: A Laboratory Manual. Cold Springs Harbor Laboratory Press, 1999
- **LUNDBLAD R. L.** Chemical Reagents for Protein Modification. CRC Press, 1991
- Current Protocols in Immunology. Wiley-Interscience, 1991, vol. 1 and 2
- **WU et al.** *Nature Biotechnology,* 2005, vol. 23 (9), 1137-1146
- **AXWORTHY et al.** *Proc. Natl. Acad. Sci. USA,* 2000, vol. 97 (4), 1802-1807
- **LEWIS et al.** *Bioconj. Chem,* 1998, vol. 9, 72-86
- **ALBERT et al.** *Bioorg. Med. Chem. Lett.,* 1998, vol. 8, 1207-1210
- **HNATOWICH et al.** *J. Immunol. Methods,* 1983, vol. 65, 147-157
- **MEARES et al.** *Anal. Biochem.,* 1984, vol. 142, 68-78
- **MIRZADEH et al.** *Bioconjugate Chem.,* 1990, vol. 1, 59-65
- **MEARES et al.** *J. Cancer,* 1990, vol. 1990 (10), 21-26
- **IZARD et al.** *Bioconjugate Chem,* 1992, vol. 3, 346-350
- **NIKULA et al.** *Nucl. Med. Biol.,* 1995, vol. 22, 387-90
- **CAMERA et al.** *Nucl. Med. Biol.,* 1993, vol. 20, 955-62
- **KUKIS et al.** *J. Nucl. Med.,* 1998, vol. 39, 2105-2110
- **VEREL et al.** *J. Nucl. Med.,* 2003, vol. 44, 1663-1670
- **CAMERA et al.** *J. Nucl. Med.,* 1994, vol. 21, 640-646
- **RUEGG et al.** *Cancer Res.,* 1990, vol. 50, 4221-4226
- **LEE et al.** *Cancer Res.,* 2001, vol. 61, 4474-4482
- **MITCHELL et al.** *J. Nucl. Med.,* 2003, vol. 44, 1105-1112
- **KOBAYASHI et al.** *Bioconjugate Chem.,* 1999, vol. 10, 103-111
- **MIEDERER et al.** *J. Nucl. Med.,* 2004, vol. 45, 129-137
- **DENARDO et al.** *Clinical Cancer Research,* 1998, vol. 4, 2483-90

- **BLEND et al.** *Cancer Biotherapy & Radiopharmaceuticals,* 2003, vol. 18, 355-363
- **NIKULA et al.** *J. Nucl. Med.,* 1999, vol. 40, 166-76
- **KOBAYASHI et al.** *J. Nucl. Med.,* 1998, vol. 39, 829-36
- **MARDIROSSIAN et al.** *Nucl. Med. Biol,* 1993, vol. 20, 65-74
- **ROSELLI et al.** *Cancer Biotherapy & Radiopharmaceuticals,* 1999, vol. 14, 209-20
- Methods for the Preparation of Enzyme-Antibody Conjugates for use in Enzyme Immunoassay. **O'SULLIVAN et al.** Methods in Enzym. Academic Press, 1981, vol. 73, 147-166
- **VAN DONGEN G A et al.** Immuno-PET: a navigator in monoclonal antibody development and applications. *Oncologist,* 2007, vol. 12, 1379-89
- **HAUGLAND.** Molecular Probes Handbook of Fluorescent Probes and Research Chemicals. Molecular Probes, Inc, 2003
- **BRINKLEY.** *Bioconjugate Chem.,* 1992, vol. 3, 2
- **GARMAN.** Non-Radioactive Labelling: A Practical Approach. Academic Press, 1997
- **MEANS.** *Bioconjugate Chem.,* 1990, vol. 1, 2
- **GLAZER et al.** Chemical Modification of Proteins. Laboratory Techniques in Biochemistry and Molecular Biology. American Elsevier Publishing Co, 1975
- **LUNDBLAD, R. L. ; NOYES, C. M.** Chemical Reagents for Protein Modification. CRC Press, 1984, vol. I and II
- Chemical Modification of Proteins. **PFLEIDERER, G.** Modern Methods in Protein Chemistry. Walter DeGryter, 1985
- **WONG.** Chemistry of Protein Conjugation and Cross-linking. CRC Press, 1991
- **LEON-RODRIGUEZ et al.** *Chem. Eur. J.,* 2004, vol. 0, 1149-1155
- **LEWIS et al.** *Bioconjugate Chem.,* 2001, vol. 12, 320-324
- **LI et al.** *Bioconjugate Chem.,* 2002, vol. 13, 110-115
- **MIER et al.** *Bioconjugate Chem.,* 2005, vol. 16, 237-240
- Fluorimetric Assays of Proteolytic Enzymes. **KNIGHT, C.** Methods in Enzymology. Academic Press, 1995, vol. 248, 18-34
- **EISENHAUER, E. ; THERASSE, P. ; BOGAERTS, J.** New response evaluation criteria in solid tumors: Revised RECIST guideline (version 1.1). *2009 Eur J Cancer.,* 2008, vol. 45 (97-110
- **HOFFMAN MS. ; HICKS RJ.** How we read Oncologic FDG PET/CT. *Cancer Img.,* 2016, vol. 16 (35
- **BARRINGTON SF. ; KLUGE R.** FDG PET for therapy monitoring in Hodgkin and non-Hodgkin lymphomas. *Eur J Nucl. Med Mol Imaging.,* 2017, vol. 44 (1), 97-110